(19) Europäisches Patentamt
European Patent Office
Office européen des brevets

(11) EP 3 010 893 B1

(12) # EUROPÄISCHE PATENTSCHRIFT

(45) Veröffentlichungstag und Bekanntmachung des Hinweises auf die Patenterteilung:
**02.10.2019 Patentblatt 2019/40**

(21) Anmeldenummer: **14731227.6**

(22) Anmeldetag: **16.06.2014**

(51) Int Cl.:
***C07D 249/12*** (2006.01) ***C07D 257/04*** (2006.01)
***C07D 271/113*** (2006.01) ***C07D 207/38*** (2006.01)
***C07D 207/408*** (2006.01) ***C07D 277/40*** (2006.01)
***C07D 277/42*** (2006.01) ***C07D 405/04*** (2006.01)
***A01N 43/653*** (2006.01) ***A01N 43/713*** (2006.01)
***A01N 43/78*** (2006.01) ***A01N 43/82*** (2006.01)
***A01P 7/02*** (2006.01) ***A01P 7/04*** (2006.01)

(86) Internationale Anmeldenummer:
**PCT/EP2014/062521**

(87) Internationale Veröffentlichungsnummer:
**WO 2014/202510 (24.12.2014 Gazette 2014/52)**

(54) **ARYLSULFID- UND ARYLSULFOXID-DERIVATE ALS AKARIZIDE UND INSEKTIZIDE**
ARYLSULFIDE AND ARYLSULFOXIDE DERIVATIVES AS ACARICIDES AND INSECTICIDES
DÉRIVÉS D’ARYLSULFURE ET D’ARYLSULFOXYDE COMME ACARICIDES ET INSECTICIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.06.2013 EP 13172990**

(43) Veröffentlichungstag der Anmeldung:
**27.04.2016 Patentblatt 2016/17**

(73) Patentinhaber: **Bayer CropScience Aktiengesellschaft**
**40789 Monheim am Rhein (DE)**

(72) Erfinder:
- **ALIG, Bernd**
  **53639 Königswinter (DE)**
- **CEREZO-GALVEZ, Silvia**
  **40764 Langenfeld (DE)**
- **FISCHER, Reiner**
  **40789 Monheim (DE)**
- **KÖHLER, Adeline**
  **40764 Langenfeld (DE)**
- **HAHN, Julia Johanna**
  **40597 Düsseldorf (DE)**
- **BECKER, Angela**
  **40235 Düsseldorf (DE)**
- **ILG, Kerstin**
  **50670 Köln (DE)**
- **VOERSTE, Arnd**
  **50674 Köln (DE)**
- **PORTZ, Daniela**
  **52391 Vettweiss (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 0 597 360** **EP-A1- 1 076 053**
**EP-A1- 2 202 226** **EP-A1- 2 604 118**
**WO-A1-2010/100189** **US-A1- 2009 076 282**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 010 893 B1

## Beschreibung

Die vorliegende Erfindung betrifft Arylsulfid- und Arylsulfoxid-Derivate, deren Verwendung als Akarizide und Insektizide zur Bekämpfung tierischer Schädlinge und Verfahren und Zwischenprodukte zu ihrer Herstellung.

Verschiedene Arylsulfide und Arylsulfoxide sowie deren insektizide und akarizide Wirkung sind bereits aus WO 1999/055668, WO 2007/131680 A, WO 2010/100189 A und JP 2011/42611 bekannt. Strukturell teilweise ähnliche Verbindungen sind ferner aus EP 0597360 A1, US 2009/076282 A1 und EP 2604118 A1 bekannt.

Die gemäß den oben genannten Schriften bereits bekannten Wirkstoffe weisen bei ihrer Anwendung Nachteile auf, beispielsweise dahingehend, dass sie gegebenenfalls keine oder aber eine nur unzureichende insektizide und/oder akarizide Wirkung gegen tierische Schädlinge, insbesondere bei niedrigeren Aufwandmengen, besitzen.

Aufgabe der vorliegenden Erfindung ist es daher, Arylsulfid- und Arylsulfoxid-Derivate bereitzustellen, welche als Insektizide und/oder Akarizide mit einer zufriedenstellenden insektiziden und/oder akariziden Wirkung gegen tierische Schädlinge, insbesondere bei niedrigeren Aufwandmengen, mit einer hohen Selektivität und verbesserten Verträglichkeit in Nutzpflanzenkulturen eingesetzt werden können.

Es wurden nun neue Verbindungen der Formel (I)

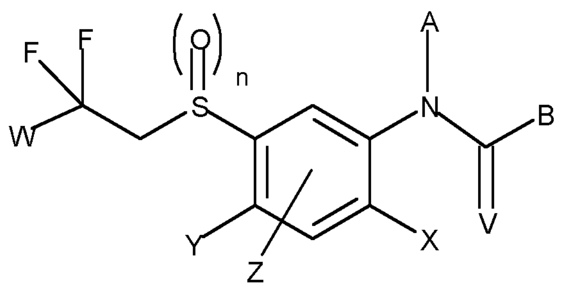

(I)

gefunden,
in welcher

A und B gemeinsam mit den Atomen, an die sie gebunden sind, für eine Substruktur stehen, die ausgewählt ist aus der Gruppe, bestehend aus

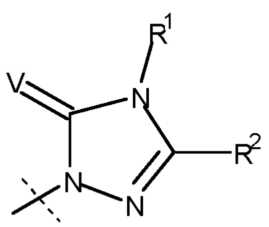
(I-A)

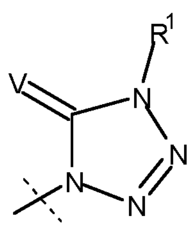
(I-B)

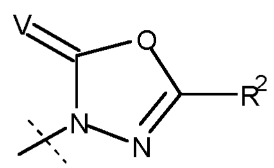
(I-C)

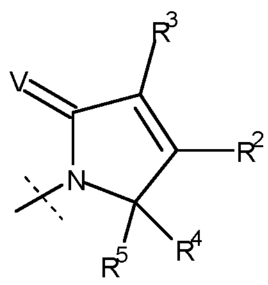
(I-D)

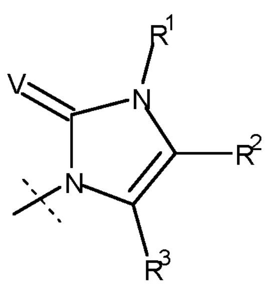
(I-E)

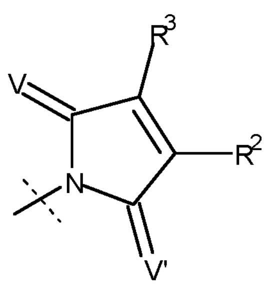
(I-F)

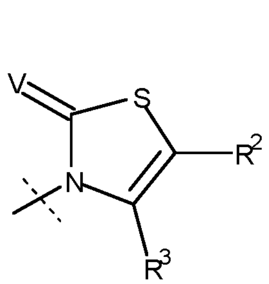
(I-G)

wobei

$R^1$ für Wasserstoff, Cyano oder Nitro steht; oder

für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl steht, wobei die vorgenannten Reste jeweils gesättigt oder ungesättigt und/oder gegebenenfalls substituiert sein können; oder

für gegebenenfalls substituiertes gesättigtes oder ungesättigtes Cycloalkyl, welches gegebenenfalls durch ein oder mehrere Heteroatome unterbrochen sein kann, steht; oder

für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder

für gegebenenfalls substituiertes Phenyl oder gegebenenfalls substituiertes Hetaryl steht; oder

für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder

für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder

für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^2$, $R^3$, $R^4$, und $R^5$, jeweils unabhängig voneinander,

für Wasserstoff, Cyano, Halogen oder Nitro stehen; oder

für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl stehen, wobei die vorgenannten Reste jeweils gesättigt oder ungesättigt und/oder gegebenenfalls substituiert sein können; oder

für gegebenenfalls substituiertes gesättigtes oder ungesättigtes Cycloalkyl, welches gegebenenfalls durch ein oder mehrere Heteroatome unterbrochen sein kann, stehen; oder

für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder

für gegebenenfalls substituiertes Phenyl oder gegebenenfalls substituiertes Hetaryl stehen; oder

für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder

für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder
für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder
$R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten drei- bis achtgliedrigen Ring bilden können; oder
oder $R^2$ für einen gesättigten oder ungesättigten gegebenenfalls durch ein oder mehrere Heteroatome, welche jeweils ausgewählt werden, aus der Gruppe, bestehend aus O, S und N, unterbrochenen Cyclus, der gegebenenfalls substituiert sein kann, steht;

W für Wasserstoff oder Halogen steht;

für den Fall, dass eine Substruktur gemäß einer der Formeln (I-B) bis (I-G) vorliegt,

V und V' jeweils unabhängig voneinander für Sauerstoff, Schwefel oder einen gegebenenfalls substituierten Stickstoff stehen;

und für den Fall, dass eine Substruktur gemäß Formel (I-A) vorliegt,

V für Sauerstoff oder Schwefel steht;

X, Y und Z, jeweils unabhängig voneinander,
für Wasserstoff, Halogen, Hydroxy, Amino, Cyano, Nitro, OCN, SCN, $SF_5$, stehen; oder
für Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfmyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl oder Dialkylaminothiocarbonyl stehen, wobei alle vorgenannten Reste gegebenenfalls substituiert sein können; oder
für Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy, Hetarylthio, Hetarylsulfinyl oder Hetarylsulfonyl stehen, wobei alle vorgenannten Reste gegebenenfalls substituiert sein können; oder
für Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl oder Cycloalkenyl stehen, wobei alle vorgenannten Reste jeweils gegebenenfalls substituiert sein können; oder
für NR'R" stehen,
wobei R' und R" unabhängig voneinander
für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl oder Alkoxycarbonyl stehen; oder
R' und R" gemeinsam mit dem N-Atom, an das sie gebunden sind, einen gegebenenfalls substituierten und gegebenenfalls durch ein oder mehrere Heteroatome, welche unabhängig ausgewählt werden aus der Gruppe, bestehend aus O, S und N, unterbrochenen gesättigten oder ungesättigten fünf- bis achtgliedrigen Ring bilden können; oder

für einen 3- bis 6-gliedrigen gesättigten, teilgesättigten oder aromatischen Ring, der gegebenenfalls ein bis drei Heteroatome, welche unabhängig ausgewählt werden aus der Gruppe, bestehend aus O, S und N, enthalten kann und der gegebenenfalls substituiert sein kann, stehen;

oder X und Z, oder Y und Z, mit den C-Atomen, an die sie gebunden sind, einen 5 oder 6-gliedrigen Ring ausbilden, der gegebenenfalls substituiert ist und gegebenenfalls durch ein oder mehrere Heteroatome, welche unabhängig ausgewählt werden, aus der Gruppe, bestehend aus O, S, N und CO, unterbrochen ist; und

n für die Zahl 0 oder 1 steht.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Bevorzugt handelt es sich bei der in Formel (I) dargestellten Doppelbindung, welche zwischen dem in $\alpha$-Position zum Stickstoff befindlichen Kohlenstoffatom und V ausgebildet wird, um eine nicht im von A und B aufgespannten Fünfring liegende Doppelbindung. Diese Doppelbindung ist somit bevorzugt exocyclisch in Bezug auf den von A und B aufgespannten Ring. Insbesondere sollen durch das Vorliegen dieser Doppelbindung als exocyclische Doppelbindung entsprechende Tautomere, welche eine endocyclische Doppelbindung in Bezug auf den von A und B aufgespannten Fünfring aufweisen, nicht in den von Formel (I) umfassten Verbindungen enthalten sein. Dieser Ausschluss der Tautomere bezieht sich jedoch bevorzugt ausschließlich auf Tautomere der in Formel (I) zwischen dem in $\alpha$-Position zum Stickstoff befindlichen Kohlenstoffatom und V befindlichen Doppelbindung und umfasst somit keine an anderer Stelle im Molekül auftretenden tautomeren Doppelbindungen.

Die Verbindungen der Formel (I) umfassen gegebenenfalls Diastereomere oder Enantiomere.

Die erfindungsgemäßen Verbindungen sind durch die Formel (I) allgemein definiert.

Erfindungsgemäße Verbindungen, in denen $R^1$ bis $R^5$ für Wasserstoff stehen, können in tautomeren Formen vorliegen, die allesamt von der vorliegenden Erfindung umfasst sind.

In einer besonders bevorzugten Ausführungsform der vorliegenden Erfindung weisen die Verbindungen eine Struktur der allgemeinen Formel (I) auf, in welcher

A und B gemeinsam mit den Atomen, an die sie gebunden sind, für eine Substruktur stehen, die ausgewählt ist aus der Gruppe, bestehend aus I-A bis I-G,

$R^1$ für Wasserstoff, Cyano oder Nitro steht; oder

für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl$(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, und $R^5$ unabhängig voneinander
für Wasserstoff, Cyano, Halogen oder Nitro stehen; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl$(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cy-

cloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

oder $R^2$ für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring, steht;

W für Wasserstoff oder Halogen steht;

für den Fall, dass eine Substruktur gemäß einer der Formeln (I-B) bis (I-G) vorliegt,

V und V' jeweils unabhängig voneinander für Sauerstoff, Schwefel oder einen gegebenenfalls substituierten Stickstoff stehen;

und für den Fall, dass eine Substruktur gemäß Formel (I-A) vorliegt,

V für Sauerstoff oder Schwefel steht;

X, Y und Z, jeweils unabhängig voneinander die zuvor genannten Bedeutungen haben; und

n für die Zahl 0 oder 1 steht.

In dieser bevorzugten Ausführungsform ist es besonders bevorzugt, dass

X, Y und Z unabhängig voneinander

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl,

$(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cycloalkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder
für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,

wobei R' und R" unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;
oder X und Z, oder Y und Z, können die folgenden 5 oder 6-gliedrigen Ringe ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

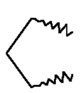 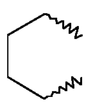 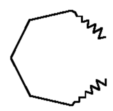 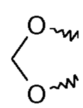 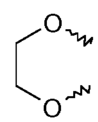 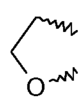 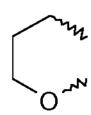

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

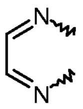 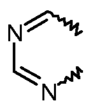 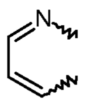 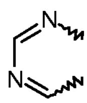 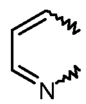 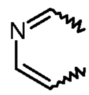

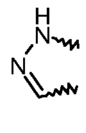 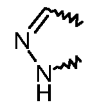 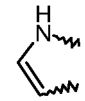 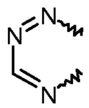 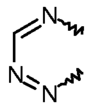 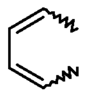

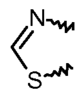 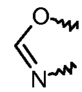 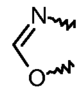

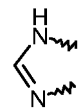 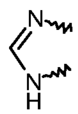 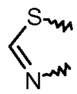 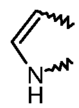 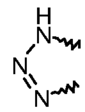 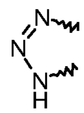

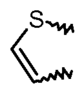 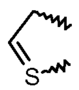 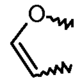 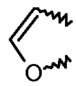

Weitere bevorzugte isolierte Ausführungsformen werden im Folgenden näher beschreiben:

<u>Erste Ausführungsform (I-A):</u>

In einer <u>ersten Ausführungsform</u> der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-A) stehen

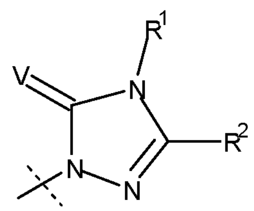

(I-A)

wobei

$R^1$ für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)aklsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, steht, wobei die vorgenannten Reste jeweils gegebenenfalls substituiert sein können und/oder unter der Bedingung, dass im Falle eines ungesättigten Restes, die definierte Mindestanzahl

an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl) aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$ für Wasserstoff, Cyano, Halogen oder Nitro steht; oder
für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl

oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy steht; oder
für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; oder
für einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten dreibis sechsgliedrigen Ring steht,

W für Wasserstoff oder Halogen steht;

V für Sauerstoff oder Schwefel steht;

X, Y und Z unabhängig voneinander für Wasserstoff, Halogen, Hydroxy, Amino, Cyano, Nitro, OCN, SCN, $SF_5$, stehen; oder
für Tri$(C_1-C_6)$alkylsilyl, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxycarbonyl$(C_1-C_6)$alkyl, $(C_1-C_6)$Alkoxy$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, $(C_1-C_6)$Alkoxy, Halogen$(C_1-C_6)$alkoxy, Cyano$(C_1-C_6)$alkoxy, Hydroxycarbonyl$(C_1-C_6)$alkoxy, $(C_1-C_6)$Alkoxycarbonyl$(C_1-C_6)$alkoxy, $(C_1-C_6)$Alkoxy$(C_1-C_6)$alkoxy, $(C_1-C_6)$Alkylhydroxyimino, $(C_1-C_6)$Alkoxyimino, $(C_1-C_6)$Alkyl$(C_1-C_6)$alkoxyimino, Halogen$(C_1-C_6)$alkyl$(C_1-C_6)$alkoxyimino, $(C_1-C_6)$Alkylthio, Halogen$(C_1-C_6)$alkylthio, $(C_1-C_6)$Alkoxy$(C_1-C_6)$alkylthio, $(C_1-C_6)$Alkylthio$(C_1-C_6)$alkyl, $(C_1-C_6)$Alkylsulfinyl, Halogen$(C_1-C_6)$alkylsulfinyl, $(C_1-C_6)$Alkoxy$(C_1-C_6)$alkylsulfinyl, $(C_1-C_6)$Alkylsulfinyl$(C_1-C_6)$alkyl, $(C_1-C_6)$Alkylsulfonyl, Halogen$(C_1-C_6)$alkylsulfonyl, $(C_1-C_6)$Alkoxy$(C_1-C_6)$alkylsulfonyl, $(C_1-C_6)$Alkylsulfonyl$(C_1-C_6)$alkyl, $(C_1-C_6)$Alkylsulfonyloxy, $(C_1-C_6)$Alkylcarbonyl, Halogen$(C_1-C_6)$alkylcarbonyl, Carboxyl, $(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cyclo$(C_1-C_6)$alkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder
für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,

wobei R' und R" unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ringe ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

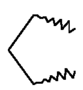 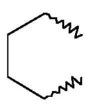 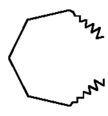 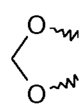 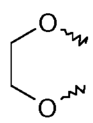 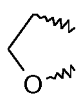 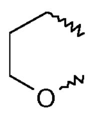

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

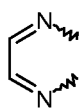 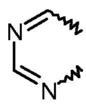 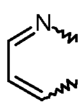 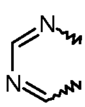 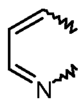 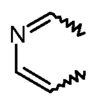

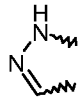
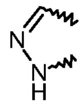
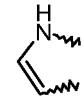
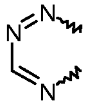
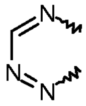
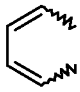
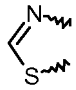
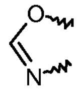
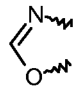
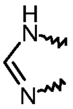
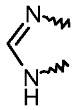
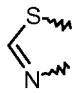
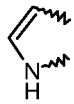
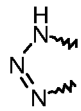
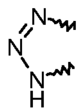
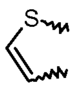
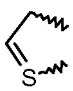
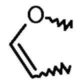
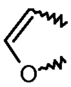

und

n für die Zahl 0 oder 1 steht.

Dabei ist es bevorzugt, dass

$R^1$ für Wasserstoff steht; oder
für $(C_1\text{-}C_6)$Alkyl, $(C_3\text{-}C_6)$Cycloalkyl$(C_1\text{-}C_6)$alkyl, Halogen$(C_2\text{-}C_6)$alkyl, Cyano$(C_1\text{-}C_6)$alkyl, $(C_1\text{-}C_6)$Alkoxy$(C_1\text{-}C_6)$alkyl, $(C_1\text{-}C_6)$Alkoxycarbonyl$(C_1\text{-}C_6)$alkyl, Phenyl$(C_1\text{-}C_6)$alkyl, Hetaryl$(C_1\text{-}C_6)$alkyl, $(C_2\text{-}C_6)$Alkenyl, Halogen$(C_2\text{-}C_6)$Alkenyl, $(C_3\text{-}C_6)$Cycloalkenyl$(C_1\text{-}C_6)$alkyl, $(C_2\text{-}C_6)$Alkinyl, wobei die vorgenannten Reste jeweils gegebenenfalls substituiert sein können, steht; oder
für gegebenenfalls substituiertes $(C_3\text{-}C_6)$Cycloalkyl, $(C_3\text{-}C_6)$Cycloalkenyl, welches gegebenenfalls durch ein oder mehrere Heteroatome aus der Reihe O, S oder N unterbrochen sein kann, steht; oder
für $(C_1\text{-}C_6)$Alkylcarbonyl, Halogen$(C_1\text{-}C_6)$alkylcarbonyl, $(C_1\text{-}C_6)$Alkoxy$(C_1\text{-}C_6)$alkylcarbonyl, $(C_1\text{-}C_6)$Alkoxycarbonyl, Halogen$(C_1\text{-}C_6)$alkoxycarbonyl, Aminocarbonyl, $(C_1\text{-}C_6)$Alkylaminocarbonyl, Di$(C_1\text{-}C_6)$alkylaminocarbonyl, $(C_3\text{-}C_6)$Cycloalkylaminocarbonyl, $(C_1\text{-}C_6)$Alkylaminothiocarbonyl, Di$(C_1\text{-}C_6)$alkylaminothiocarbonyl, $(C_2\text{-}C_6)$Alkenylcarbonyl, $(C_3\text{-}C_6)$Cycloalkenyl$(C_1\text{-}C_6)$alkylcarbonyl, $(C_2\text{-}C_6)$Alkinylcarbonyl oder Aminothiocarbonyl steht, wobei die vorgenannten Reste jeweils gegebenenfalls substituiert sein können oder für Carbonyl oder Carboxyl steht; oder
für einen Phenyl- oder Hetarylring, der gegebenenfalls einfach oder mehrfach, gleich oder verschieden substituiert ist mit Halogen, Cyano, Nitro, Hydroxy, Amino, $(C_1\text{-}C_6)$Alkyl, Halogen$(C_1\text{-}C_6)$Alkyl, $(C_1\text{-}C_6)$Alkoxy, Halogen$(C_1\text{-}C_6)$alkoxy, $(C_1\text{-}C_6)$Alkylsulfinyl, $(C_1\text{-}C_6)$Alkylsulfanyl, $(C_1\text{-}C_6)$Alkylsulfonyl, Halo$(C_1\text{-}C_6)$alkylsulfinyl, Halo$(C_1\text{-}C_6)$alkylsulfanyl, Halo$(C_1\text{-}C_6)$alkylsulfonyl oder gegebenenfalls substituiertes $(C_3\text{-}C_6)$Cycloalkyl steht; oder
für $(C_1\text{-}C_6)$Alkoxy, Halogen$(C_1\text{-}C_6)$alkoxy, $(C_3\text{-}C_6)$Cycloalkyloxy, oder Carbonyloxy steht, wobei die vorgenannten Reste jeweils gegebenenfalls substiuiert sein können, oder für Hydroxy steht; oder
für $(C_1\text{-}C_6)$Alkylamino, Halogen$(C_1\text{-}C_6)$alkylamino, Dihalogen$(C_1\text{-}C_6)$alkylamino, Di$(C_1\text{-}C_6)$alkylamino, $(C_3\text{-}C_6)$Cycloalkylamino, Di$(C_3\text{-}C_6)$cycloalkylamino, $(C_1\text{-}C_6)$Alkylcarbonylamino, $(C_1\text{-}C_6)$Alkoxycarbonylamino, $(C_1\text{-}C_6)$Alkylcarbamoylamino, $(C_1\text{-}C_6)$Alkylsulfonylamino, $(C_2\text{-}C_6)$Alkenylamino, $(C_3\text{-}C_6)$Cycloalkenyl$(C_1\text{-}C_6)$alkylamino, $(C_2\text{-}C_6)$Alkinylamino steht, wobei die vorgenannten Reste jeweils gegebenenfalls substituiert sein können, oder für Amino steht; oder
für $(C_1\text{-}C_6)$Alkylsulfanyl, $(C_1\text{-}C_6)$Alkylsulfinyl, $(C_1\text{-}C_6)$Alkylsulfonyl, Halogen$(C_1\text{-}C_6)$alkylsulfanyl, Halogen$(C_1\text{-}C_6)$alkylsulfinyl, Halogen$(C_1\text{-}C_6)$alkylsulfanyl, $(C_3\text{-}C_6)$Cycloalkyl$(C_1\text{-}C_6)$alkylsulfanyl, $(C_3\text{-}C_6)$Cycloalkyl$(C_1\text{-}C_6)$alkylsulfinyl, $(C_3\text{-}C_6)$Cycloalkyl$(C_1\text{-}C_6)$alkylsulfonyl, Aminosulfonyl, $(C_1\text{-}C_6)$Alkylaminosulfonyl, Di$(C_1\text{-}C_6)$alkylaminosulfonyl oder Arylaminosulfonyl steht, wobei die vorgenannten Reste jeweils gegebenenfalls substituiert sein können oder für Sulfanyl steht; und

$R^2$ für Wasserstoff, Cyano, Halogen oder Nitro steht; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl steht, wobei die vorgenannten Reste jeweils gegebenenfalls substituiert sein können; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder
für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder
für einen Phenyl- oder Hetarylring, der gegebenenfalls einfach oder mehrfach, gleich oder verschieden substituiert ist mit Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl steht; oder
für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können, oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; oder
für einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, steht;

W für Wasserstoff oder Halogen, insbesondere Fluor und Chlor, steht;

V für Sauerstoff oder Schwefel steht;

X, Y und Z, jeweils unabhängig voneinander die zuvor genannten Bedeutungen haben; und

n für die Zahl 0 oder 1 steht.

In einer bevorzugten Ausgestaltung dieser ersten Ausführungsform steht die Substruktur der Formel (I-A) für eine Substruktur, welche ausgewählt wird aus der Gruppe, bestehend aus

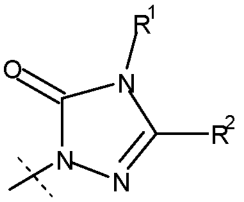

(I-A-1)

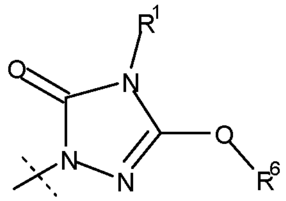

(I-A-2)

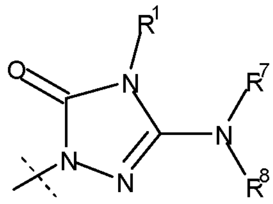

(I-A-3)

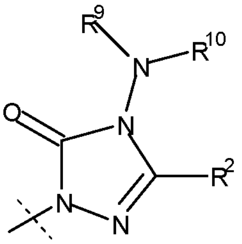

(I-A-4)

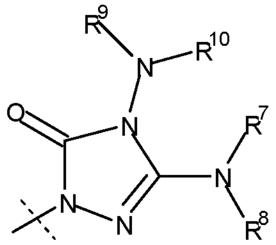

(I-A-5)

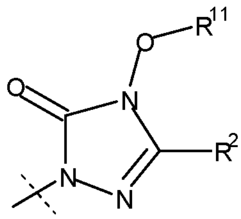

(I-A-6)

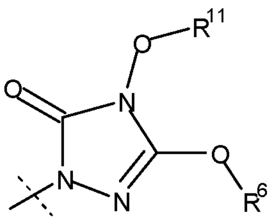

(I-A-7)

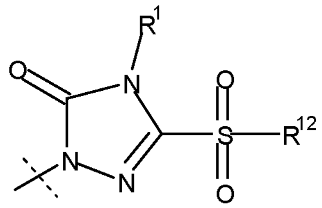

(I-A-8)

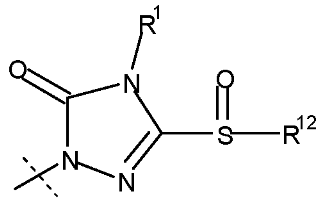

(I-A-9)

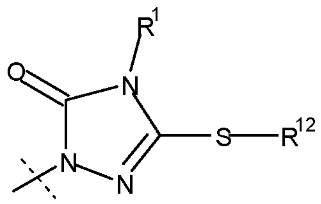

(I-A-10)

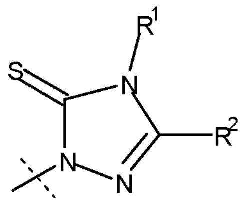

(I-A-11)

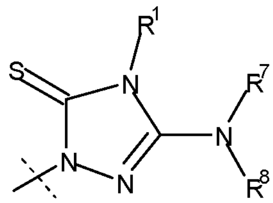

(I-A-12)

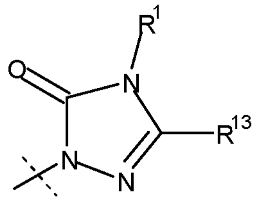

(I-A-13)

wobei

$R^1$ für Wasserstoff steht; oder für $(C_1-C_6)$Alkyl, $(C_3-C_5)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Oxetan, Oxolan, Oxan, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^2$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht; oder
für Carboxyl steht;

$R^6$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^7$ und $R^8$ jeweils unabhängig voneinander für Wasserstoff, $(C_1-C_6)$Alkyl, Halogen$(C_2-C_6)$Alkyl, Cyano$(C_1-C_6)$Alkyl oder $(C_3-C_6)$Cycloalkyl stehen, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können; oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, $(C_1-C_6)$Alkyl, Halogen$(C_2-C_6)$Alkyl, Cyano$(C_1-C_6)$Alkyl, $(C_3-C_6)$Cycloalkyl, oder gemeinsam für $(C_2-C_6)$Alkyliden stehen, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl,

($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano oder Cyclopropyl substituiertes Cyclopropyl substituiert sein können;
oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^{12}$ für ($C_1$-$C_6$)Alkyl, ($C_3$-$C_6$)Cycloalkyl, ($C_2$-$C_6$)Alkenyl, Halogen($C_1$-$C_6$)Alkyl, Cyano($C_1$-$C_6$)Alkyl, Phenyl($C_1$-$C_3$)alkyl oder ($C_3$-$C_6$)Cycloalkyl($C_1$-$C_6$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können;

$R^{13}$ für Halogen steht;

W für Wasserstoff oder Halogen (insbesondere Fluor oder Chlor) steht;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

In einer ebenfalls bevorzugten Ausgestaltung dieser ersten Ausführungsform steht die Substruktur der Formel (I-A) für eine Substruktur, welche ausgewählt wird aus der Gruppe, bestehend aus

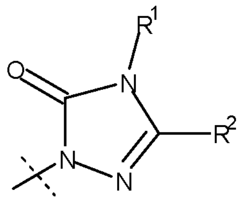

(I-A-1)

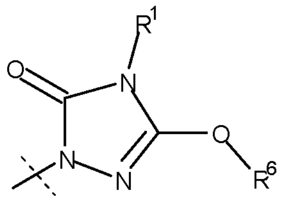

(I-A-2)

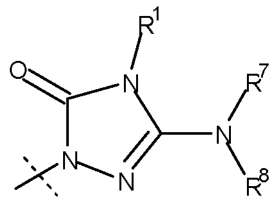

(I-A-3)

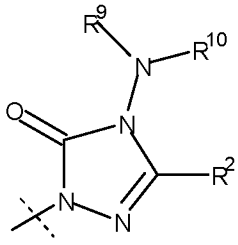

(I-A-4)

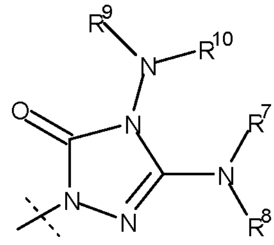

(I-A-5)

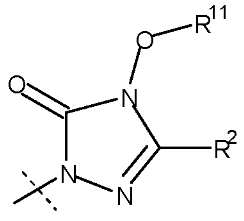

(I-A-6)

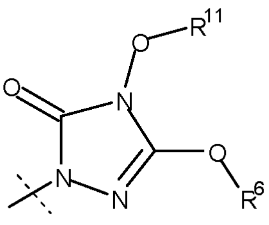

(I-A-7)

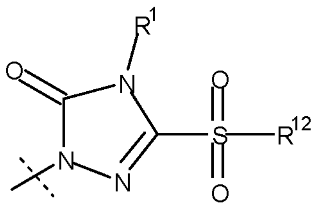

(I-A-8)

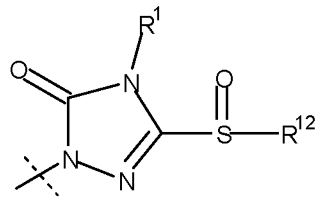

(I-A-9)

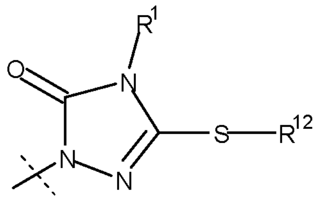

(I-A-10)

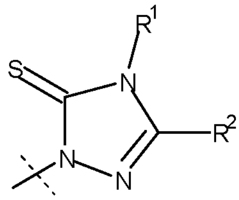

(I-A-11)

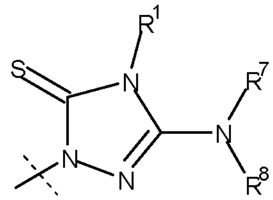

(I-A-12)

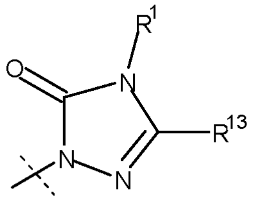

(I-A-13)

wobei

$R^1$ für Wasserstoff steht; oder

für $(C_1-C_6)$Alkyl, $(C_3-C_5)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl,

Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Oxetan, Oxolan, Oxan, Phenyl, Pyridyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^2$ für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht; oder
für Carboxyl steht;

$R^6$ für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^7$und$R^8$ jeweils unabhängig voneinander für Wasserstoff, ($C_1$-$C_6$)Alkyl, Halogen($C_2$-$C_6$)Alkyl, Cyano($C_1$-$C_6$)Alkyl oder ($C_3$-$C_6$)Cycloalkyl stehen, wobei die vorgenannten Reste gegebenenfalls jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano oder Cyclopropyl substituiertes Cyclopropyl substituiert sein können; oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, ($C_1$-$C_6$)Alkyl, Halogen($C_2$-$C_6$)Alkyl, Cyano($C_1$-$C_6$)Alkyl, ($C_3$-$C_6$)Cycloalkyl, oder gemeinsam für ($C_2$-$C_6$)Alkyliden stehen, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder

gegebenenfalls durch Methyl, Fluor, Chlor, Cyano oder Cyclopropyl substituiertes Cyclopropyl substituiert sein können;
oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^{12}$ für ($C_1$-$C_6$)Alkyl, ($C_3$-$C_6$)Cycloalkyl, ($C_2$-$C_6$)Alkenyl, Halogen($C_1$-$C_6$)Alkyl, Cyano($C_1$-$C_6$)Alkyl, Phenyl($C_1$-$C_3$)alkyl oder ($C_3$-$C_6$)Cycloalkyl($C_1$-$C_6$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können;

$R^{13}$ für Halogen steht;

W für Wasserstoff oder Halogen (insbesondere Fluor oder Chlor) steht;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen; oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Bevorzugt steht die Substruktur der Formel (I-A) für eine Substruktur, welche ausgewählt wird aus der Gruppe, bestehend aus

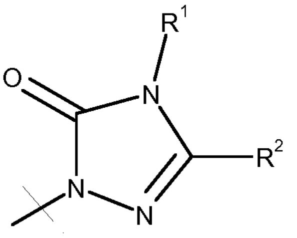

(I-A-1)

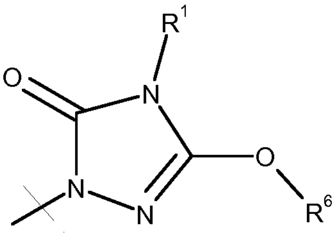

(I-A-2)

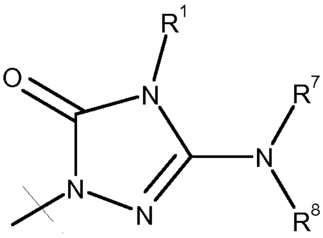

(I-A-3)

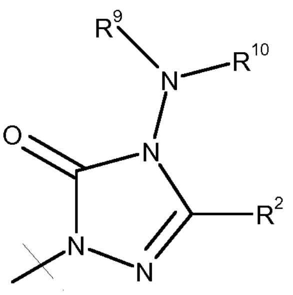

(I-A-4)

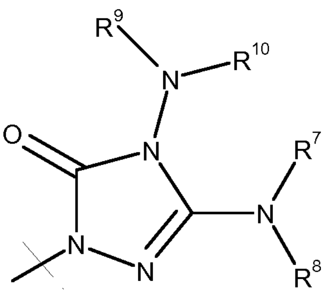

(I-A-5)

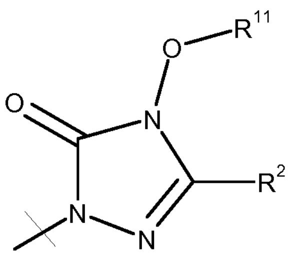

(I-A-6)

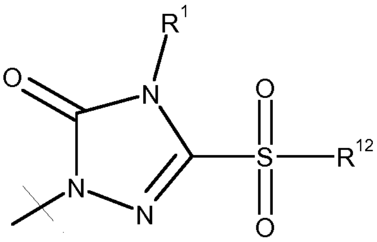

(I-A-8)

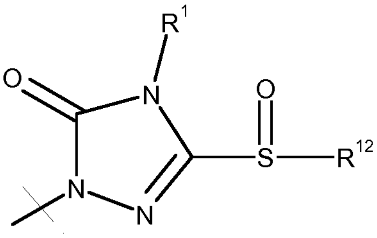

(I-A-9)

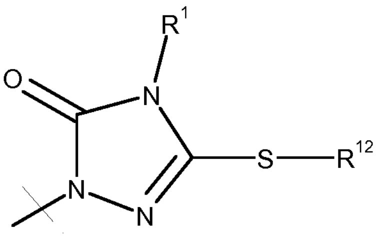

(I-A-10)

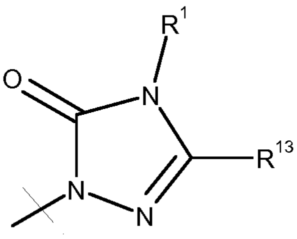

(I-A-13)

wobei

$R^1$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder

gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^2$ für Wasserstoff steht; oder für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht; oder für Carboxyl steht;

$R^6$ für Wasserstoff steht; oder für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^7$und$R^8$ jeweils unabhängig voneinander für Wasserstoff, $(C_1-C_6)$Alkyl, Halogen$(C_2-C_6)$Alkyl, Cyano$(C_1-C_6)$Alkyl oder $(C_3-C_6)$Cycloalkyl stehen, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können; oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, $(C_1-C_6)$Alkyl, Halogen$(C_2-C_6)$Alkyl, Cyano$(C_1-C_6)$Alkyl, $(C_3-C_6)$Cycloalkyl, oder gemeinsam für $(C_2-C_6)$Alkyliden stehen, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können;

oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder
für $(C_1\text{-}C_6)$Alkyl, $(C_3\text{-}C_6)$Cycloalkyl$(C_1\text{-}C_3)$alkyl, $(C_2\text{-}C_6)$Alkenyl, $(C_2\text{-}C_6)$Alkinyl, Halogen$(C_1\text{-}C_6)$alkyl, Cyano$(C_1\text{-}C_6)$Alkyl, $(C_1\text{-}C_6)$Alkoxy$(C_1\text{-}C_6)$alkyl oder Phenyl$(C_1\text{-}C_3)$alkyl steht, wobei die vorgenannten Reste jeweils gegebenenfalls einfach bis dreifach durch Halogen, Cyano, Nitro, Hydroxy, Amino, $(C_1\text{-}C_6)$Alkyl, Halogen$(C_1\text{-}C_6)$Alkyl, $(C_1\text{-}C_6)$Alkoxy, Halogen$(C_1\text{-}C_6)$alkoxy, $(C_1\text{-}C_6)$Alkylsulfinyl, $(C_1\text{-}C_6)$Alkylsulfanyl, $(C_1\text{-}C_6)$Alkylsulfonyl, Halo$(C_1\text{-}C_6)$alkylsulfinyl, Halo$(C_1\text{-}C_6)$alkylsulfanyl, Halo$(C_1\text{-}C_6)$alkylsulfonyl oder gegebenenfalls durch Methyl, Fluor, Chlor, Cyano substituiertes Cyclopropyl substituiert sein können; oder
für $(C_3\text{-}C_6)$Cycloalkyl, $(C_3\text{-}C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1\text{-}C_6)$Alkyl, Halogen$(C_1\text{-}C_6)$Alkyl, $(C_1\text{-}C_6)$Alkoxy, Halogen$(C_1\text{-}C_6)$alkoxy, $(C_1\text{-}C_6)$Alkylsulfinyl, $(C_1\text{-}C_6)$Alkylsulfanyl, $(C_1\text{-}C_6)$Alkylsulfonyl, Halo$(C_1\text{-}C_6)$alkylsulfinyl, Halo$(C_1\text{-}C_6)$alkylsulfanyl, Halo$(C_1\text{-}C_6)$alkylsulfonyl oder gegebenenfalls durch Methyl, Fluor, Chlor, Cyano substituiertes Cyclopropyl substituiert sein können, steht;

$R^{12}$ für $(C_1\text{-}C_6)$Alkyl, $(C_3\text{-}C_6)$Cycloalkyl, $(C_2\text{-}C_6)$Alkenyl, Halogen$(C_1\text{-}C_6)$Alkyl, Cyano$(C_1\text{-}C_6)$Alkyl, Phenyl$(C_1\text{-}C_3)$alkyl oder $(C_3\text{-}C_6)$Cycloalkyl$(C_1\text{-}C_6)$alkyl steht, wobei die vorgenannten Reste jeweils gegebenenfalls einfach bis dreifach durch Halogen, Cyano, Nitro, Hydroxy, Amino, $(C_1\text{-}C_6)$Alkyl, Halogen$(C_1\text{-}C_6)$Alkyl, $(C_1\text{-}C_6)$Alkoxy, Halogen$(C_1\text{-}C_6)$alkoxy, $(C_1\text{-}C_6)$Alkylsulfinyl, $(C_1\text{-}C_6)$Alkylsulfanyl, $(C_1\text{-}C_6)$Alkylsulfonyl, Halo$(C_1\text{-}C_6)$alkylsulfinyl, Halo$(C_1\text{-}C_6)$alkylsulfanyl, Halo$(C_1\text{-}C_6)$alkylsulfonyl oder gegebenenfalls durch Methyl, Fluor, Chlor, Cyano substituiertes Cyclopropyl substituiert sein können;

$R^{13}$ für Halogen steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, $(C_1\text{-}C_4)$Alkyl, $(C_1\text{-}C_4)$Halogenalkyl, $(C_2\text{-}C_4)$Alkenyl, $(C_2\text{-}C_4)$Alkinyl, $(C_1\text{-}C_4)$Alkoxy, $(C_1\text{-}C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Ebenfalls bevorzugt steht die Substruktur der Formel (I-A) für eine Substruktur, welche ausgewählt wird aus der Gruppe, bestehend aus

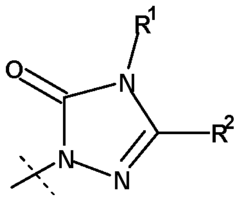

(I-A-1)

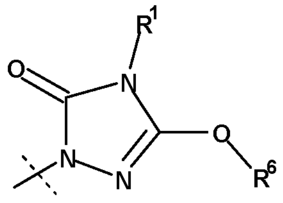

(I-A-2)

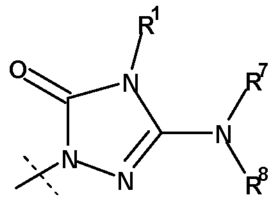

(I-A-3)

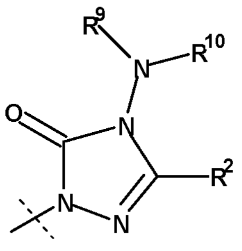

(I-A-4)

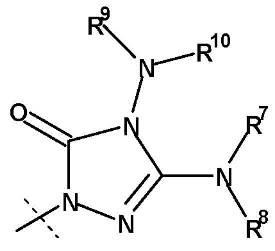

(I-A-5)

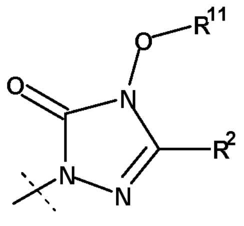

(I-A-6)

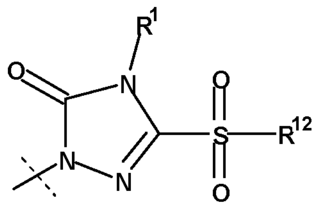

(I-A-8)

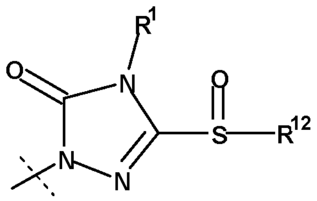

(I-A-9)

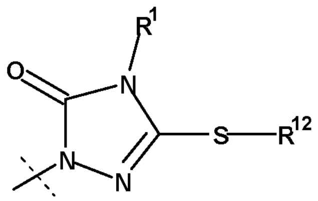

(I-A-10)

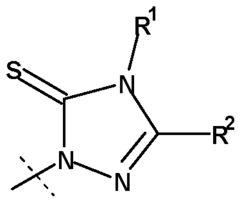

(I-A-11)

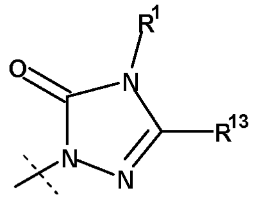

(I-A-13)

wobei

$R^1$ für Wasserstoff steht; oder für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^2$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht; oder
für Carboxyl steht;

$R^6$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^7$und$R^8$ jeweils unabhängig voneinander für Wasserstoff, $(C_1-C_6)$Alkyl, Halogen$(C_2-C_6)$Alkyl, Cyano$(C_1-C_6)$Alkyl oder $(C_3-C_6)$Cycloalkyl, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können;
oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, $(C_1-C_6)$Alkyl, Halogen$(C_2-C_6)$Alkyl, Cyano$(C_1-C_6)$Alkyl, $(C_3-C_6)$Cycloalkyl, oder gemeinsam für $(C_2-C_6)$Alkyliden steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können;
oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cy-

ano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^{12}$ für ($C_1$-$C_6$)Alkyl, ($C_3$-$C_6$)Cycloalkyl, ($C_2$-$C_6$)Alkenyl, Halogen($C_1$-$C_6$)Alkyl, Cyano($C_1$-$C_6$)Alkyl, Phenyl($C_1$-$C_3$)alkyl oder ($C_3$-$C_6$)Cycloalkyl($C_1$-$C_6$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können;

$R^{13}$ für Halogen steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogena!koxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Handelt es sich bei der Substruktur der Formel (I-A) um die Substrukturformel (I-A-1), so ist es bevorzugt, dass

$R^1$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^2$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl,

Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht; oder
für Carboxyl steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, Benzyl, Cyclopropyl, Cyclobutyl, Phenyl oder 4-Methyl-3-(2,2,2-trifluorethylsulfanyl)-phenyl steht;

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl, Phenyl, 3-Fluorphenyl, 2-Fluor-4-chlorphenyl oder COOH steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^1$ für Wasserstoff, Methyl, Ethyl, $CH(CH_3)_2$, $C(CH_3)_3$, $CH_2CF_3$, $CH_2CH_2OCH_3$, Cyclopropyl, Phenyl oder 4-Methyl-3-(2,2,2-trifluorethylsulfanyl)-phenyl steht;

$R^2$ für Wasserstoff, Methyl, Ethyl, $CH_2CH_2CH_3$, $CH_2CH_2CH_2CH_3$, $CH_2CH(CH_3)_2$, $CH(CH_3)CH_2CH_3$, $CH(CH_3)_2$, $C(CH_3)_3$, $CH{=}CHCH_3$, $CF_3$, $CH_2OCH_3$, $CH_2OCH_2CH_3$, $CH_2CH_2OCH_3$, Cyclopropyl, Cyclobutyl, Phenyl, 3-Fluorphenyl, 2-Fluor-4-chlorphenyl oder COOH steht;

W Wasserstoff oder Fluor steht;

X für Wasserstoff, Chlor, Fluor oder Methyl steht;

Y für Chlor, Cyano, Methyl oder Methoxy steht; wobei

X und Y insbesondere für folgende Kombinationen (Y,X) stehen: (CN,F), (Me, F), (Me,H), (Me,Cl), (Me,Me), (Cl, H), ($OCH_3$,H)

Z für Wasserstoff steht; und

n für die Zahl 0 oder 1 steht.

Handelt es sich bei der Substruktur der Formel (I-A) um die Substrukturformel (I-A-1), so ist es ebenfalls bevorzugt, dass

$R^1$ für Wasserstoff steht; oder für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls substituiert sein können; oder für $(C_3-C_6)$Cycloalkyl, $(C_3-C_6)$Cycloalkenyl, Phenyl oder Pyridyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^2$ für Wasserstoff steht; oder für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl, steht, wobei die vorgenannten Reste jeweils gegebenenfalls substituiert sein können; oder für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht; oder für Carboxyl steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es ebenfalls besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, Benzyl, Cyclopropyl, Cyclobutyl, Phenyl, 3-Pyridyl, 4-Fluorphenyl oder 4-Methyl-3-(2,2,2-trifluorethylsulfanyl)-phenyl steht;

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, $CH_2CH_2OCH(CH_3)_2$, Benzyl, Cyclopropyl, Cyclobutyl, Cyclopentyl, Cyclohexyl, Phenyl, 3-Fluorphenyl, 2-Fluor-4-chlorphenyl, oder COOH steht;

W für Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ebenfalls ganz besonders bevorzugt, dass

$R^1$ für Wasserstoff, Methyl, Ethyl, $CH(CH_3)_2$, $C(CH_3)_3$, $CH_2CF_3$, $CH_2CH_2OCH_3$, Cyclopropyl, Phenyl, 3-Pyridyl, 4-Fluorphenyl oder 4-Methyl-3-(2,2,2-trifluorethylsulfanyl)-phenyl steht;

$R^2$ für Wasserstoff, Methyl, Ethyl, $CH_2CH_2CH_3$, $CH_2CH_2CH_2CH_3$, $CH_2CH(CH_3)_2$, $CH(CH_3)CH_2CH_3$, $CH(CH_3)_2$, $C(CH_3)_3$, $CH{=}CHCH_3$, $CF_3$, $CH_2OCH_3$, $CH_2OCH_2CH_3$, $CH_2CH_2OCH_3$, $CH_2CH_2OCH(CH_3)_2$, Cyclopropyl, Cyclobutyl, Cyclopentyl, Cyclohexyl, Phenyl, 3-Fluorphenyl, 2-Fluor-4-chlorphenyl oder COOH steht;

W für Wasserstoff oder Fluor steht;

X für Wasserstoff, Chlor, Fluor oder Methyl steht;

Y für Chlor, Cyano, Methyl oder Methoxy steht; wobei

X und Y insbesondere für folgende Kombinationen (Y,X) stehen: (CN,F), (Me, F), (Me,H), (Me,Cl), (Me,Me), (Cl, H), ($OCH_3$,H)

Z für Wasserstoff steht; und

n für die Zahl 0 oder 1 steht.

Handelt es sich bei der Substruktur der Formel (I-A) um die Substrukturformel (I-A-2), so ist es bevorzugt, dass

$R^1$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^6$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl, Oxetanyl, Oxolanyl, Oxanyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

$R^6$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CH_2C(=CH_2)CH_3$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl, Cyclohexyl, Phenyl oder 3-Tetrahydrofuryl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^1$ für Cyclopropyl, Methyl, Ethyl oder $CH_2CH=CH_2$ steht;

$R^6$ für Phenyl, Cyclohexyl, $CH_2$-Cyclopropyl, Benzyl, 3-Tetrahydrofuryl, Methyl, Ethyl, $CH_2CH_2CH_3$, $CH_2CH_2CH_2CH_3$, $CH_2CH_2OCH_3$, $CH_2CH_2C(=CH_2)CH_3$, $CH_2CH(CH_3)_2$, $CH_2CF_3$, $CH_2C(CH_3)_3$, $CH(CH_3)CH_2CH_3$, $CH(CH_3)_2$ oder $C(CH_3)_3$ steht;

W für Fluor steht;

X für Wasserstoff, Fluor, Chlor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,Me), (Me,H), (Me,Cl), (Cl,Cl), (Cl,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Handelt es sich bei der Substruktur der Formel (I-A) um die Substrukturformel (I-A-2), so ist es ebenfalls bevorzugt, dass

$R^1$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^6$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl, Oxetanyl, Oxolanyl, Oxanyl oder Phenyl, wobei die vorgenannten Reste jeweils einfach bis dreifach durch Halogen, Cyano, Nitro, Hydroxy, Amino, Methyl, Ethyl, Isopropyl,

tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Methoxy, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es ebenfalls besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, $CH_2CH_2CH_2OCH_3$, Benzyl, Cyclopropyl, Cyclobutyl oder Phenyl steht;

$R^6$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CH_2C({=}CH_2)CH_3$, $CH_2CCH$, Cyclopropylmethyl, $CF_3$, $CH_2CF_3$, $CH_2CHF_2$, $CH_2CCl_3$, $CH_2CN$, $CH_2OCH_3$, $CH_2OCH_2CH_3$, $CH_2CH_2OCH_3$, Benzyl, Cyclopropyl, Cyclobutyl, Cyclohexyl, Phenyl oder 3-Tetrahydrofuryl steht;

W für Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ebenfalls ganz besonders bevorzugt, dass

$R^1$ für Cyclopropyl, Methyl, Ethyl, n-Propyl, $CH(CH_3)_2$, $CH_2CH{=}CH_2$, Cyclopropylmethyl, $CH_2CF_3$ oder $CH_2CH_2CH_2OCH_3$ steht;

$R^6$ für Phenyl, Cyclohexyl, $CH_2$-Cyclopropyl, Benzyl, 3-Tetrahydrofuryl, Methyl, Ethyl, $CH_2CH_2CH_3$, $CH_2CH_2CH_2CH_3$, $CH_2CH_2OCH_3$, $CH_2CH_2C({=}CH_2)CH_3$, $CH_2CH(CH_3)_2$, $CH_2CF_3$, $CH_2C(CH_3)_3$, $CH(CH_3)CH_2CH_3$, $CH(CH_3)_2$, $CH_2CCl_3$, $CH_2CH{=}CH_2$ oder $C(CH_3)_3$ steht;

W für Fluor steht;

X für Wasserstoff, Fluor, Chlor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,Me), (Me,H), (Me,Cl), (Cl,Cl), (Cl,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Handelt es sich bei der Substruktur der Formel (I-A) um die Substrukturformel (I-A-3), so ist es bevorzugt, dass

$R^1$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^7$ und $R^8$ jeweils unabhängig voneinander für Wasserstoff, $(C_1-C_6)$Alkyl, Halogen$(C_2-C_6)$Alkyl, Cyano$(C_1-C_6)$Alkyl, oder $(C_3-C_6)$Cycloalkyl stehen, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;
oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

$R^7$ und $R^8$ beide gleichzeitig für Methyl oder Ethyl stehen oder gemeinsam mit dem Stickstoffatom, an das sie gebunden sind, für Morpholin, *N*-Methylpiperazin, 4-Methyl-5-oxo-3-phenoxy-1,2,4-triazol-1-yl), 4-Ethyl-5-oxo-3-phenoxy-1,2,4-triazol-1-yl) oder 4-Cyclopropyl-5-oxo-3-phenoxy-1,2,4-triazol-1-yl), stehen;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, dass

$R^1$ für Cyclopropyl oder Methyl steht;

$R^7$ und $R^8$ beide gleichzeitig für Methyl stehen oder gemeinsam mit dem Stickstoffatom, an das sie gebunden sind, für (4-Methyl-5-oxo-3-phenoxy-1,2,4-triazol-1-yl) stehen;

W für Fluor steht;

X für Wasserstoff oder Fluor steht;

Y für Methyl steht; wobei

X und Y insbesondere für folgende Kombinationen (Y,X) stehen: (Me, F), (Me,H);

Z für Wasserstoff steht; und

n für die Zahl 0 oder 1 steht.

Handelt es sich bei der Substruktur der Formel (I-A) um die Substrukturformel (I-A-4), so ist es bevorzugt, dass

$R^2$ für Wasserstoff steht; oder für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^9$ und $R^{10}$ jeweils unabhängig voneinander für Wasserstoff, ($C_1$-$C_6$)Alkyl, Halogen($C_2$-$C_6$)Alkyl, Cyano($C_1$-$C_6$)alkyl oder ($C_3$-$C_6$)Cycloalkyl stehen, wobei die vorgenannten Reste gegebenenfalls jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder gemeinsam für ($C_1$-$C_6$)Alkyliden, das gegebenenfalls durch Halogen, Cyano, Nitro, Hydroxy, Amino, ($C_1$-$C_3$)Alkyl, Halogen($C_1$-$C_3$)Alkyl, ($C_1$-$C_3$)Alkoxy, Halogen($C_1$-$C_3$)alkoxy oder gegebenenfalls durch Methyl, Fluor, Chlor, Cyano substituiertes Cyclopropyl substituiert sein kann, steht;

W Wasserstoff oder Halogen, insbesondere F oder Cl, stehen;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl steht;

$R^9$ und $R^{10}$ jeweils unabhänging voneinander für Wasserstoff, Methyl oder Ethyl stehen oder gemeinsam mit dem Stickstoffatom, an das sie gebunden sind, für $=C(CH_3)_2$, $=C(CH_3)CH_2\ CH_3$, $=C(CH_3)CH_2CH(CH_3)_2$ stehen;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^2$ für Wasserstoff, Methyl, $CH(CH_3)_2$, $CH_2OCH_3$, Cyclopropyl oder $CF_3$ steht;

$R^9$ und $R^{10}$ jeweils unabhängig voneinander für Wasserstoff oder Methyl stehen oder gemeinsam für $=C(CH_3)CH_2CH(CH_3)_2$ stehen;

W für Fluor steht;

X für Wasserstoff oder Fluor steht;

Y für Methyl steht;

X und Y insbesondere für folgende Kombinationen (Y,X) stehen: (Me, F), (Me,H); und

Z für Wasserstoff steht;

n für die Zahl 0 oder 1 steht.

Handelt es sich bei der Substruktur der Formel (I-A) um die Substrukturformel (I-A-5), so ist es bevorzugt, dass

$R^7$ und $R^8$ jeweils unabhängig voneinander für Wasserstoff, $(C_1-C_6)$Alkyl, Halogen$(C_2-C_6)$Alkyl, Cyano$(C_1-C_6)$alkyl oder $(C_3-C_6)$Cycloalkyl stehen, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

$R^9$ und $R^{10}$ jeweils unabhängig voneinander für Wasserstoff, $(C_1-C_6)$Alkyl, Halogen$(C_2-C_6)$Alkyl, Cyano$(C_1-C_6)$alkyl oder $(C_3-C_6)$Cycloalkyl stehen, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder gemeinsam für $(C_1-C_6)$Alkyliden, das gegebenenfalls durch Halogen, Cyano, Nitro, Hydroxy, Amino, $(C_1-C_3)$Alkyl, Halogen$(C_1-C_3)$Alkyl, $(C_1-C_3)$Alkoxy, Halogen$(C_1-C_3)$alkoxy oder gegebenenfalls durch Methyl, Fluor, Chlor, Cyano substituiertes Cyclopropyl substituiert sein kann, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^7$ und $R^8$ jeweils unabhängig voneinander für Wasserstoff, Methyl, Ethyl, n-Propyl, $CH(CH_3)_2$, n-Butyl, sec-Butyl, $C(CH_3)_3$, $CH_2C(CH_3)_3$ oder $CH_2CH(CH_3)_2$ stehen;
$R^9$ und $R^{10}$ jeweils unabhänging voneinander für Wasserstoff, Methyl oder Ethyl stehen oder gemeinsam mit dem Stickstoffatom, an das sie gebunden sind, für $=C(CH_3)_2$, $=C(CH_3)CH_2$ $CH_3$, $=C(CH_3)CH_2CH(CH_3)_2$ stehen;
W Wasserstoff oder Fluor steht;
X für Wasserstoff, Chlor, Fluor oder Methyl steht;
Y für Fluor, Chlor, Brom, Cyano, Methyl, Methoxy oder Trifluoromethyl steht; wobei
X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);
Z für Wasserstoff steht; und
n für die Zahl 0 oder 1 steht.

Hierbei ist es ganz besonders bevorzugt, dass

$R^7$ und $R^8$ jeweils unabhängig voneinander für Wasserstoff, Methyl, Ethyl oder $CH(CH_3)_2$ stehen; wobei
$R^7$ und $R^8$ insbesondere für folgende Kombinationen ($R^7$,$R^8$) stehen: (Me,Me), (Me,H), (Et,Et), (iPr,H)
$R^9$ und $R^{10}$ jeweils unabhängig voneinander für Wasserstoff oder Methyl stehen; wobei
$R^9$ und $R^{10}$ insbesondere für folgende Kombinationen ($R^9$,$R^{10}$) stehen: (Me,H), (H,H)
W für Fluor steht;
X für Fluor steht;

Y für Methyl steht;

Z für Wasserstoff steht;

n für die Zahl 0 oder 1 steht.

Handelt es sich bei der Substruktur der Formel (I-A) um die Substrukturformel (I-A-6), so ist es bevorzugt, dass

$R^2$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^{11}$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl, Oxetan, Oxolan, Oxan oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl steht;

$R^{11}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl, Phenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^2$ für Methyl steht;

$R^{11}$ für Methyl steht;

W für Fluor steht;

X für Fluor steht;

Y für Methyl steht; wobei

Z für Wasserstoff steht;

n für die Zahl 0 oder 1 steht.

Hierbei ist es ebenfalls ganz besonders bevorzugt, dass

$R^2$ für Methyl oder $CH(CH_3)_2$ steht;

$R^{11}$ für Methyl steht;

W für Fluor steht;

X für Fluor steht;

Y für Methyl steht; wobei

Z für Wasserstoff steht;

n für die Zahl 0 oder 1 steht.

Handelt es sich bei der Substruktur der Formel (I-A) um die Substrukturformel (I-A-7), so ist es bevorzugt, dass

$R^6$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl, steht, wobei die vorgenannten Reste jeweils gegebenenfalls substituiert sein können; oder
für $(C_3-C_6)$Cycloalkyl, $(C_3-C_6)$Cycloalkenyl, Oxetan, Oxolan, Oxan oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, oder gegebenenfalls durch Methyl, Fluor, Chlor, Cyano substituiertes Cyclopropyl substituiert sein können, steht;

$R^{11}$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl, steht, wobei die vorgenannten Reste jeweils gegebenenfalls substituiert sein können; oder
für $(C_3-C_6)$Cycloalkyl, $(C_3-C_6)$Cycloalkenyl, Oxetan, Oxolan, Oxan oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^6$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl steht;

$R^{11}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, dass

$R^6$ für Ethyl steht;

$R^{11}$ für Methyl steht;

W für Fluor steht;

X für Wasserstoff oder Fluor steht;

Y für Methyl steht; wobei

X und Y insbesondere für folgende Kombinationen (Y,X) stehen: (Me, F) oder (Me,H); und

Z für Wasserstoff steht;

n für die Zahl 0 oder 1 steht.

Handelt es sich bei der Substruktur der Formel (I-A) um die Substrukturformel (I-A-8), so ist es bevorzugt, dass

$R^1$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^{12}$ für $(C_1-C_6)$Alkyl, $(C_3-C_6)$Cycloalkyl, $(C_2-C_6)$Alkenyl, Halogen$(C_1-C_6)$Alkyl oder Cyano$(C_1-C_6)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

$R^{12}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CF_3$, $CH_2CF_3$, $CH_2CHF_2$, $CH_2CN$, Cyclopropyl oder Cyclobutyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, dass

$R^1$ für Cyclopropyl steht;
$R^{12}$ für Methyl steht;
W für Fluor steht;
X für Fluor steht;
Y für Methyl steht; wobei
Z für Wasserstoff steht; und
n für die Zahl 0 oder 1 steht.

Hierbei ist es ebenfalls ganz besonders bevorzugt, dass

$R^1$ für Methyl, Ethyl oder Cyclopropyl steht;
$R^{12}$ für Methyl steht;
W für Fluor steht;
X für Fluor steht;
Y für Methyl steht; wobei
Z für Wasserstoff steht; und
n für die Zahl 0 oder 1 steht.

Handelt es sich bei der Substruktur der Formel (I-A) um die Substrukturformel (I-A-9), so ist es bevorzugt, dass

$R^1$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^{12}$ für $(C_1-C_6)$Alkyl, $(C_3-C_6)$Cycloalkyl, $(C_2-C_6)$Alkenyl, Halogen$(C_1-C_6)$Alkyl oder Cyano$(C_1-C_6)$alkyl, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;
$R^{12}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$,

$CH=CH_2$, $CH=CHCH_3$, $CH_2CH=CH_2$, $CF_3$, $CH_2CF_3$, $CH_2CHF_2$, $CH_2CN$, Cyclopropyl oder Cyclobutyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^1$ für Cyclopropyl, Methyl, Ethyl, $CH_2CH=CH_2$ steht;

$R^{12}$ für Methyl, Ethyl, $CH_2CH_2CH_3$, $CH_2$-Cyclopropyl, $CH(CH_3)_2$, Benzyl oder $CH_2CHF_2$ steht;

W für Fluor steht;

X für Fluor steht;

Y für Methyl steht; wobei

Z für Wasserstoff steht; und

n für die Zahl 0 oder 1 steht.

Handelt es sich bei der Substruktur der Formel (I-A) um die Substrukturformel (I-A-10), so ist es bevorzugt, dass

$R^1$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^{12}$ für ($C_1$-$C_6$)Alkyl, ($C_3$-$C_6$)Cycloalkyl, ($C_2$-$C_6$)Alkenyl, Halogen($C_1$-$C_6$)Alkyl oder Cyano($C_1$-$C_6$)alkyl, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

$R^{12}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CF_3$, $CH_2CF_3$, $CH_2CHF_2$, $CH_2CN$, Cyclopropyl oder Cyclobutyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^1$ für Cyclopropyl, Methyl, Ethyl oder $CH_2CH=CH_2$ steht;

$R^{12}$ für Methyl, Ethyl, $CH_2CHF_2$, $CH_2$-Cyclopropyl, $CH_2CH_2CH_3$, $CH_2CH=CH_2$, $CH_2CF_3$, $CH(CH_3)_2$, $CF_3$ oder Benzyl steht;

W für Fluor steht;

X für Fluor steht;

Y für Methyl steht; wobei

Z für Wasserstoff steht; und

n für die Zahl 0 oder 1 steht.

Handelt es sich bei der Substruktur der Formel (I-A) um die Substrukturformel (I-A-11), so ist es bevorzugt, dass

$R^1$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

$R^2$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Methoxy, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, $C(CH_3)_3$, $CH_2CH(CH_3)_2$, $CH_2CH=CH_2$, $CH_2CF_3$, $CH_2CN$, Benzyl, Cyclopropyl, Cyclobutyl oder Phenyl steht;

$R^2$ für Wasserstoff, Methyl, Ethyl, n-Propyl, $CH(CH_3)_2$, n-Butyl, sec-Butyl, $C(CH_3)_3$, $CH_2CH(CH_3)_2$, $CH=CH_2$, $CH=CHCH_3$, $CH_2CH=CH_2$, $CH_2OCH_3$, $CH_2OCH_2CH_3$, $CH_2CH_2OCH_3$, $CH_2CH_2OCH_2CH_3$, $CF_3$, $CH_2CF_3$, $CH_2CHF_2$, $CH_2CN$, Cyclopropyl, Cyclobutyl, Cyclopentyl, Cyclohexyl oder Phenyl steht;

W Wasserstoff oder Fluor steht;
X für Wasserstoff, Chlor, Fluor oder Methyl steht;
Y für Fluor, Chlor, Brom, Cyano, Methyl, Methoxy oder Trifluoromethyl steht; wobei
X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);
Z für Wasserstoff steht; und
n für die Zahl 0 oder 1 steht.

Hierbei ist es ganz besonders bevorzugt, dass

$R^1$ für Cyclopropyl, Ethyl oder $CH(CH_3)_2$ steht;
$R^2$ für Methyl, Ethyl, n-Propyl, n-Butyl, $CH(CH_3)_2$, $CH_2CH(CH_3)_2$, $CH_2OCH_3$, $CH_2OCH_2CH_3$, $CH(CH_3)CH_2CH_3$, $CH=CHCH_3$, Cyclopropyl, Cyclobutyl, Cyclopentyl, Cyclohexyl, 2-Methoxyphenyl oder $CF_3$ steht;
W für Fluor steht;
X für Fluor oder Wasserstoff steht;
Y für Methyl oder Methoxy steht; wobei
X und Y insbesondere für folgende Kombinationen (Y,X) stehen: (Me, F), (MeO,H),
Z für Wasserstoff steht; und
n für die Zahl 0 steht.

Handelt es sich bei der Substruktur der Formel (I-A) um die Substrukturformel (I-A-13), so ist es bevorzugt, dass

$R^1$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^{13}$ für Halogen steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

$R^{13}$ für Fluor, Chlor oder Brom steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

Hierbei ist es ganz besonders bevorzugt, dass

$R^1$ für Cyclopropyl steht;
$R^{13}$ für Brom steht;
W für Fluor steht;
X für Fluor steht;
Y für Methyl steht; wobei
Z für Wasserstoff steht; und
n für die Zahl 0 oder 1 steht.

Hierbei ist es ebenfalls ganz besonders bevorzugt, dass

$R^1$ für Methyl, Ethyl, $CH(CH_3)_2$, $C(CH_3)_3$ oder Cyclopropyl steht;
$R^{13}$ für Brom steht;
W für Fluor steht;
X für Fluor steht;
Y für Methyl steht; wobei
Z für Wasserstoff steht; und
n für die Zahl 0 oder 1 steht.

Zweite Ausführungsform (I-B):

In einer zweiten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-B) stehen

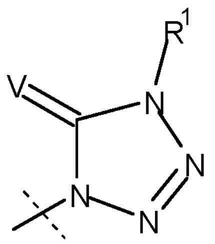

(I-B)

wobei
$R^1$ für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl,

$(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

V für Sauerstoff, Schwefel oder einen gegebenenfalls substituierten Stickstoff stehen;

X, Y und Z unabhängig voneinander für Wasserstoff, Halogen, Hydroxy, Amino, Cyano, Nitro, OCN, SCN, $SF_5$, stehen; oder

für Tri$(C_1-C_6)$alkylsilyl, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxycarbonyl$(C_1-C_6)$alkyl, $(C_1-C_6)$Alkoxy$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, $(C_1-C_6)$Alkoxy, Halogen$(C_1-C_6)$alkoxy, Cyano$(C_1-C_6)$alkoxy, Hydroxycarbonyl$(C_1-C_6)$alkoxy, $(C_1-C_6)$Alkoxycarbonyl$(C_1-C_6)$alkoxy, $(C_1-C_6)$Alkoxy$(C_1-C_6)$alkoxy, $(C_1-C_6)$Alkylhydroxyimino, $(C_1-C_6)$Alkoxyimino, $(C_1-C_6)$Alkyl$(C_1-C_6)$alkoxyimino, Halogen$(C_1-C_6)$alkyl$(C_1-C_6)$alkoxyimino, $(C_1-C_6)$Alkylthio, Halogen$(C_1-C_6)$alkylthio, $(C_1-C_6)$Alkoxy$(C_1-C_6)$alkylthio, $(C_1-C_6)$Alkylthio$(C_1-C_6)$alkyl, $(C_1-C_6)$Alkylsulfinyl, Halogen$(C_1-C_6)$alkylsulfinyl, $(C_1-C_6)$Alkoxy$(C_1-C_6)$alkylsulfinyl, $(C_1-C_6)$Alkylsulfinyl$(C_1-C_6)$alkyl, $(C_1-C_6)$Alkylsulfonyl, Halogen$(C_1-C_6)$alkylsulfonyl, $(C_1-C_6)$Alkoxy$(C_1-C_6)$alkylsulfonyl, $(C_1-C_6)$Alkylsulfonyl$(C_1-C_6)$alkyl, $(C_1-C_6)$Alkylsulfonyloxy, $(C_1-C_6)$Alkylcarbonyl, Halogen$(C_1-C_6)$alkylcarbonyl, Carboxyl, $(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cyclo$(C_1-C_6)$alkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können;

oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R'' unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

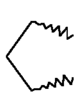 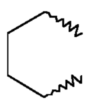 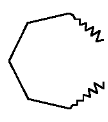 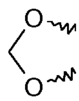 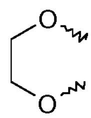 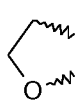 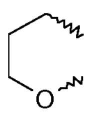

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

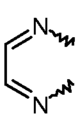 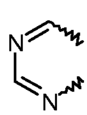 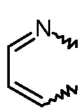 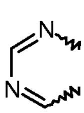 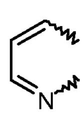 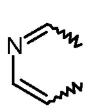

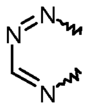 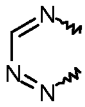 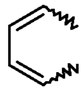 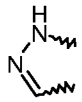 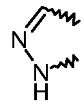 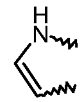 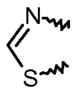 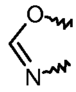 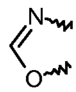 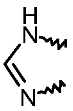 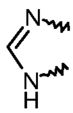 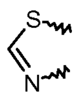 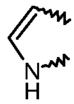 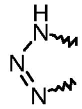 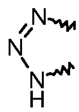 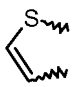 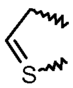 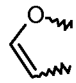 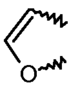

und
n für die Zahl 0 oder 1 steht.

Dabei ist es bevorzugt, dass

$R^1$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

W für Wasserstoff oder Halogen steht;

V für Sauerstoff, Schwefel oder einen gegebenenfalls substituierten Stickstoff steht;

X, Y und Z jeweils unabhängig voneinander die zuvor genannten Beteudungen haben; und

n für die Zahl 0 oder 1 steht.

Bevorzugt steht die Substruktur der Formel (I-B) für eine Substruktur, welche ausgewählt wird aus der Gruppe, bestehend aus

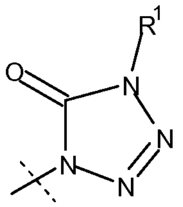

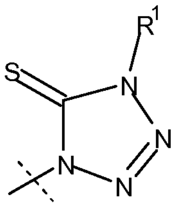

(I-B-1) (I-B-2)

wobei

R[1] für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_2$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder
für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo(C $_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Besonders bevorzugt handelt es sich bei der Substruktur um die Substrukturformel (I-B-1).
Handelt es sich bei der Substruktur der Formel (I-B) um die Substrukturformel (I-B-1), so ist es bevorzugt, dass

R[1] für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_2$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl, Phenyl oder 4-Chlorphenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, dass

$R^1$ für Wasserstoff, Methyl, $CH_2CF_3$, $CH(CH_3)_2$ oder 4-Chlorphenyl steht;
W für Fluor steht;
X für Wasserstoff, Methyl oder Fluor steht;
Y für Methyl steht; wobei
X und Y insbesondere für folgende Kombinationen (Y,X) stehen: (Me, F), (Me, Me), (Me,H);
Z für Wasserstoff steht; und
n für die Zahl 0 oder 1 steht.

Handelt es sich bei der Substruktur der Formel (I-B) um die Substrukturformel (I-B-1), so ist es ebenfalls besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CF_2CF_3$, $CH_2CN$, $CH_2CH_2OCH_3$, Benzyl, Cyclopropyl, Cyclobutyl, Phenyl, 4-Trifluoromethylphenyl oder 4-Chlorphenyl steht;
W Wasserstoff oder Fluor steht;
X für Wasserstoff, Chlor, Fluor oder Methyl steht;
Y für Fluor, Chlor, Brom, Cyano, Methyl, Methoxy oder Trifluoromethyl steht; wobei
X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);
Z für Wasserstoff steht; und
n für die Zahl 0 oder 1 steht.

Hierbei ist es ganz besonders bevorzugt, dass

$R^1$ für Wasserstoff, Methyl, $CH(CH_3)_2$, $CH_2CF_3$, $CH_2CH_2OCH_3$, 4-Trifluoromethylphenyl oder 4-Chlorphenyl steht;
W für Fluor steht;
X für Wasserstoff, Methyl oder Fluor steht;

Y für Methyl steht; wobei
X und Y insbesondere für folgende Kombinationen (Y,X) stehen: (Me, F), (Me, Me), (Me,H);
Z für Wasserstoff steht; und
n für die Zahl 0 oder 1 steht.

Ebenfalls besonders bevorzugt handelt es sich bei der Substruktur um die Substrrukturformel (I-B-2).

Handelt es sich bei der Substruktur der Formel (I-B) um die Substrukturformel (I-B-2), so ist es bevorzugt, dass

$R^1$ für Wasserstoff steht; oder für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Methoxy, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH=CH_2$, Cyclopropylmethyl, $CH_2CF_3$, $CH_2CHF_2$, $CH_2CN$, $CH_2CH_2OCH_3$, Benzyl, Cyclopropyl, Cyclobutyl, Phenyl oder 4-Chlorphenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, dass

$R^1$ für Methyl steht;

W für Fluor steht;

X für Fluor steht;

Y für Methyl steht; wobei

Z für Wasserstoff steht; und

n für die Zahl 0 oder 1 steht.

Dritte Ausführungsform (I-C):

In einer dritten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-C) stehen

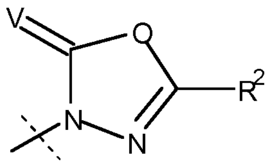

(I-C)

wobei

$R^2$ für Wasserstoff, Cyano, Halogen oder Nitro steht; oder
für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder
für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halo-

gen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten dreibis sechsgliedrigen Ring,

W für Wasserstoff oder Halogen steht;

V für Sauerstoff, Schwefel oder einen gegebenenfalls substituierten Stickstoff stehen;

X, Y und Z unabhängig voneinander

für Wasserstoff, Halogen, Hydroxy, Amino, Cyano, Nitro, OCN, SCN, $SF_5$, stehen; oder für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl(C $_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R" unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;
oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

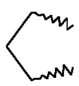 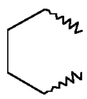 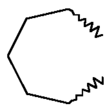 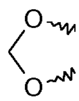 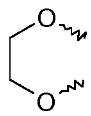 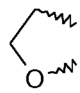 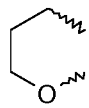

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

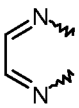 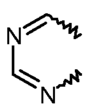 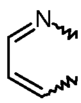 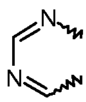 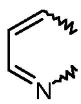 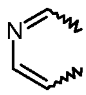

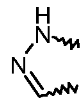 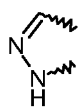 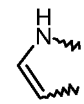

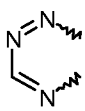 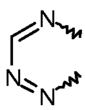 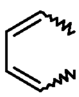

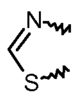 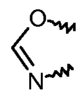 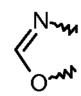

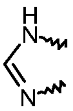 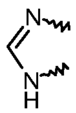 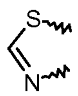

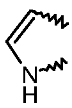 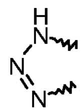 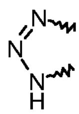

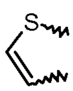 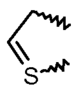 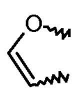 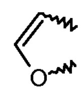

n für die Zahl 0 oder 1 steht.

Dabei ist es bevorzugt, dass

$R^2$ für Wasserstoff, Cyano, Halogen oder Nitro steht; oder
für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl steht, wobei die vorgenannten Reste jeweils gegebenenfalls substituiert sein können; oder
für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, steht; oder
für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl steht, wobei die vorgenannten Reste jeweils gegebenenfalls substituiert sein können oder für Carbonyl oder Carboxyl steht; oder
für gegebenenfalls substituiertes Phenyl oder gegebenenfalls substituiertes Hetaryl steht; oder
für einen Phenyl- oder Hetarylring, der gegebenenfalls einfach oder mehrfach, gleich oder verschieden substituiert ist mit Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl steht; oder
für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy steht; oder
für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino, ($C_1$-$C_6$)Alkoxycarbonylamino, ($C_1$-$C_6$)Alkylcarbamoylamino, ($C_1$-$C_6$)Alkylsulfonylamino, ($C_2$-$C_6$)Alkenylamino, ($C_3$-$C_6$)Cycloalkenyl($C_1$-$C_6$)alkylamino, ($C_2$-$C_6$)Alkinylamino steht, wobei die vorgenannten Reste jeweils gegebenenfalls substituiert sein können oder für Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können oder für Sulfanyl steht; oder
für einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, steht;

W für Wasserstoff, Fluor oder Chlor steht;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

n für die Zahl 0 oder 1 steht.

Bevorzugt steht die Substruktur der Formel (I-C) für die Substruktur

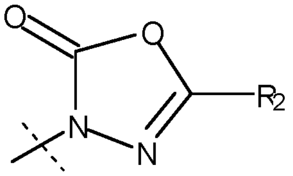

(I-C-1)

wobei

$R^2$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, $(C_1-C_6)$Alkoxy$(C_1-C_6)$alkyl oder Phenyl$(C_1-C_3)$alkyl, steht, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Dabei ist es besonders bevorzugt, wenn

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, wenn

$R^2$ für $CF_3$ steht;

W für Fluor steht;

X für Fluor steht;

Y für Cyano und Methyl steht;

X und Y stehen insbesondere für folgende Kombinationen (Y,X): (CN,F), (Me, F);

Z für Wasserstoff steht; und

n für die Zahl 0 oder 1 steht.

Handelt es sich bei der Substruktur der Formel (I-C) um die Substrukturformel (I-C-1), so ist es ebenfalls bevorzugt, dass

$R^2$ für Wasserstoff steht; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl, Phenyl, Pyridyl, Pyrimidyl, Pyridazinyl, Pyrazinyl, Furyl, Thienyl, Triazinyl, Thiazolyl, Thiadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, steht;

W für Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Methoxy, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Dabei ist es besonders bevorzugt, wenn

$R^2$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH=CH_2$, $CH=CHCH_3$, $CH_2CH=CH_2$, $CH_2CCH$, Cyclopropylmethyl, $CF_3$, $CH_2CF_3$, $CH_2CHF_2$, $CH_2CH_2CH_2Cl$, $CH_2CN$, $CH_2OCH_3$, $CH_2OCH_2CH_3$, $CH_2CH_2OCH_3$, Benzyl, Cyclopropyl, Cyclobutyl, Phenyl, 3-Fluorphenyl, 4-Chlorphenyl, 4-Methoxyphenyl, 4-Nitrophenyl, 2-Thienyl, 2-Furyl oder 4-Pyridyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, wenn

$R^2$ für Wasserstoff, Methyl, Ethyl, n-Propyl, $CH(CH_3)_2$, n-Butyl, $C(CH_3)_3$, $CH_2OCH_3$, $CH_2OCH_2CH_3$, $CH_2CH_2CH_2Cl$,

Benzyl, Cyclopropyl, Phenyl, 3-Fluorphenyl, 4-Chlorphenyl, 4-Methoxyphenyl, 4-Nitrophenyl, 2-Thienyl, 2-Furyl, 4-Pyridyl oder $CF_3$ steht;

W für Fluor steht;

X für Fluor oder Methyl steht;

Y für Cyano oder Methyl steht; insbesondere wobei X und Y für folgende Kombinationen (Y,X) stehen: (CN,F), (Me, F), (Me,Me);

Z für Wasserstoff steht; und

n für die Zahl 0 oder 1 steht.

Vierte Ausführungsform (I-D):

In einer vierten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-D) stehen

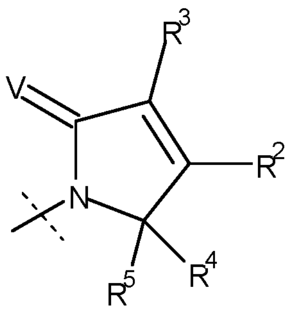

(I-D)

wobei

$R^2$, $R^3$, $R^4$, und $R^5$ unabhängig voneinander für Wasserstoff, Cyano, Halogen oder Nitro stehen; oder für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothio-

carbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder für gegebenenfalls substituiertes Phenyl oder gegebenenfalls substituiertes Hetaryl stehen; oder für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder
$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder
oder $R^2$ für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring, steht;

W für Wasserstoff oder Halogen steht;

V für Sauerstoff, Schwefel oder einen gegebenenfalls substituierten Stickstoff stehen;

W für Wasserstoff oder Halogen steht;

X, Y und Z unabhängig voneinander
für Wasserstoff, Halogen, Hydroxy, Amino, Cyano, Nitro, OCN, SCN, $SF_5$, stehen; oder
für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl,

($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,

wobei R' und R" unabhängig voneinander
für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder
R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;
oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

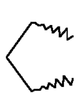 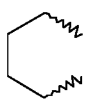 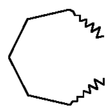 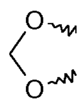 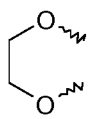 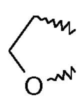 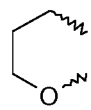

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

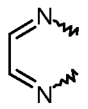 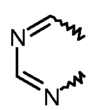 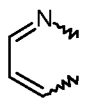 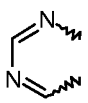 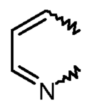 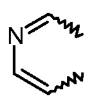

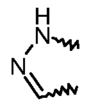 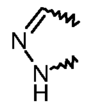 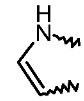

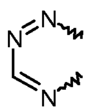 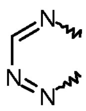 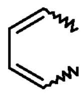

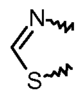 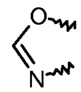 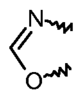

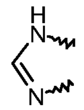 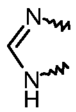 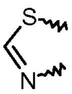

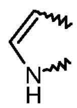 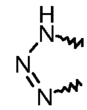 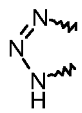

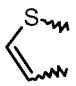 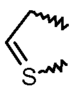 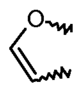 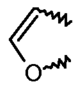

n für die Zahl 0 oder 1 steht.

Dabei ist es bevorzugt, dass

$R^2$, $R^3$, $R^4$, und $R^5$, unabhängig voneinander,

für Wasserstoff, Cyano, Halogen oder Nitro stehen; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, ($C_1$-$C_6$)Alkoxy($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, Phenyl($C_1$-$C_6$)alkyl, Hetaryl($C_1$-$C_6$)alkyl, ($C_2$-$C_6$)Alkenyl, Halogen($C_2$-$C_6$)Alkenyl, ($C_3$-$C_6$)Cycloalkenyl($C_1$-$C_6$)alkyl, ($C_2$-$C_6$)Alkinyl stehen, wobei die vorgenannten Reste jeweils gegebenenfalls substituiert sein können; oder

für gegebenenfalls substituiertes ($C_3$-$C_6$)Cycloalkyl, ($C_3$-$C_6$)Cycloalkenyl, welches gegebenenfalls durch ein oder mehrere Heteroatome aus der Reihe O, S oder N unterbrochen sein kann, stehen; oder

für ($C_1$-$C_6$)Alkylcarbonyl, Halogen($C_1$-$C_6$)alkylcarbonyl, ($C_1$-$C_6$)Alkoxy($C_1$-$C_6$)alkylcarbonyl, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, ($C_1$-$C_6$)Alkylaminothiocarbonyl, Di($C_1$-$C_6$)alkylaminothiocarbonyl, ($C_2$-$C_6$)Alkenylcarbonyl, ($C_3$-$C_6$)Cycloalkenyl($C_1$-$C_6$)alkylcarbonyl, ($C_2$-$C_6$)Alkinylcarbonyl oder Aminothiocarbonyl stehen, wobei die vorgenannten Reste jeweils gegebenenfalls substituiert sein können oder für Carbonyl oder Carboxyl stehen; oder

für einen Phenyl- oder Hetarylring, der gegebenenfalls einfach oder mehrfach, gleich oder verschieden substituiert ist mit Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl stehen; oder

für ($C_1$-$C_6$)Alkoxy, Halogen($C_1$-$C_6$)alkoxy, ($C_1$-$C_6$)Alkoxy($C_1$-$C_6$)alkoxy, ($C_3$-$C_6$)Cycloalkyloxy, ($C_3$-$C_6$)Cycloalkyl($C_1$-$C_6$)alkyloxy, ($C_2$-$C_6$)Alkenyloxy, ($C_3$-$C_6$)Cycloalkenyl($C_1$-$C_6$)alkoxy, ($C_2$-$C_6$)Alkinyloxy oder Carbonyloxy stehen, wobei die vorgenannten Reste jeweils gegebenenfalls substituiert sein können oder für Hydroxy stehen; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_1$-$C_6$)Alkylcarbonylamino,

$(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino stehen, wobei die vorgenannten Reste jeweils gegebenenfalls substituiert sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können oder für Sulfanyl stehen; oder

$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

für einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, steht;

W für Wasserstoff oder Halogen, insbesondere Fluor und Chlor, steht;

V für Sauerstoff, Schwefel oder einen gegebenenfalls substituierten Stickstoff steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

n für die Zahl 0 oder 1 steht.

Bevorzugt steht die Substruktur der Formel (I-D) für eine Substruktur, welche ausgewählt wird aus der Gruppe, bestehend aus

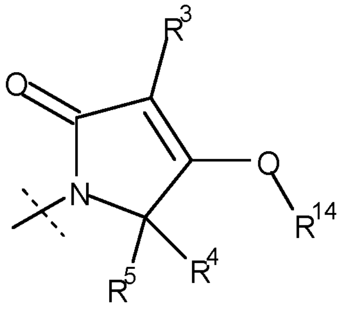

(I-D-1)

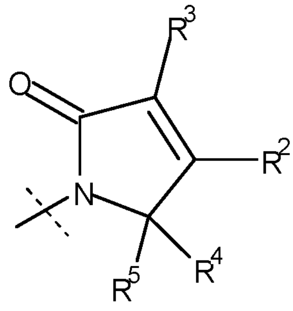

(I-D-2)

wobei

$R^2$, $R^3$, $R^4$, und $R^5$ unabhängig voneinander
für Wasserstoff stehen; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl stehen, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, stehen; oder
für Carboxyl stehen; oder
$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;

$R^{14}$ für $(C_1-C_6)$Alkyl, Halogen$(C_1-C_6)$alkyl, Cyano$(C_1-C_6)$alkyl, $(C_1-C_6)$Alkoxy$(C_1-C_3)$alkyl, Phenyl$(C_1-C_3)$alkyl, $(C_3-C_6)$Cycloalkyl oder $(C_3-C_6)$Cycloalkyl$(C_1-C_3)$alkyl steht, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

n für die Zahl 0 oder 1 steht.

Besonders bevorzugt handelt es sich bei der Substruktur um die Substrrukturformel (I-D-1).
Handelt es sich bei der Substruktur der Formel (I-D) um die Substrukturformel (I-D-1), so ist es bevorzugt, dass

$R^3$, $R^4$ und $R^5$ unabhängig voneinander für Wasserstoff stehen; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl, steht, wobei die vorgenannten

Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht; oder
$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, Methyl, Ethyl, Trifluoromethyl, Difluoromethyl, Trifluoroethyl, Difluoroethyl, Trifluoromethoxy, Difluoromethoxy oder gegebenenfalls durch Halogen, Cyano, Methyl oder Cyclopropyl substituiertes Cyclopropyl substituierten Cyclopropyl-, Cyclobutyl-, Cyclopentyl- oder Cyclohexylring bilden können;

$R^{14}$ für $(C_1-C_6)$Alkyl, Halogen$(C_1-C_6)$alkyl, Cyano$(C_1-C_6)$alkyl, $(C_1-C_6)$Alkoxy$(C_1-C_3)$alkyl, Phenyl$(C_1-C_3)$alkyl, $(C_3-C_6)$Cycloalkyl oder $(C_3-C_6)$Cycloalkyl$(C_1-C_3)$alkyl stehen, wobei die vorgenannten Reste jeweils jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

W für Wasserstoff oder Halogen, insbesondere F und Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^3$, $R^4$ und $R^5$ unabhängig voneinander für Wasserstoff, Methyl, Ethyl, Benzyl, Cyclopropyl, Phenyl stehen; oder

$R^4$ und $R^5$ gemeinsam mit dem Atom, an das sie gebunden sind, einen Cyclopropylring bilden können;

$R^{14}$ für Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, Cyclopropylmethyl, $CH_2CH_2OCH_3$, $CH_2CH_2OCH_2CH_3$, Benzyl, Cyclopropyl, Cyclobutyl oder Cyclopentyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, wenn

$R^3$ für Wasserstoff steht;
$R^4$ für Wasserstoff steht;
$R^5$ für Wasserstoff steht;
$R^{14}$ für Cyclopentyl, $CH_2$-Cyclopropyl, Methyl, Ethyl, $CH_2CH_2OCH_3$, $CH_2CH_2CH_3$, $CH_2CH_2CH_2CH_3$, $CH(CH_3)_2$

oder Benzyl steht;

W für Fluor steht;

X für Wasserstoff, Fluor, Chlor oder Methyl steht;

Y für Cyano, Chlor, $CF_3$ oder $CH_3$ steht; wobei

X und Y insbesondere für folgende Kombinationen (Y,X) stehen: (CN,F), (Me, F), (Me,H), (Me,Cl), (Me,Me), (Cl, H), (Cl, Cl), (Cl,F), ($CF_3$, H);

Z für Wasserstoff steht; und

n für die Zahl 0 oder 1 steht.

Fünfte Ausführungsform (I-E):

In einer fünften Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-E) stehen

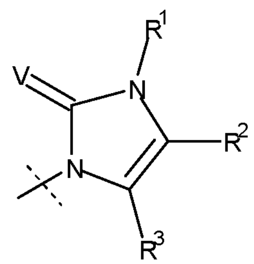

(I-E)

$R^1$ für Wasserstoff, Cyano oder Nitro steht; oder

für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylearbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder

für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Al-

kylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$ und $R^3$ unabhängig voneinander

für Wasserstoff, Cyano, Halogen oder Nitro steht; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylearbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte

Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alky-

laminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten dreibis sechsgliedrigen Ring stehen,

W für Wasserstoff oder Halogen steht;

V für Sauerstoff, Schwefel oder einen gegebenenfalls substituierten Stickstoff stehen;

X, Y und Z unabhängig voneinander

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R" unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen; oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

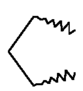 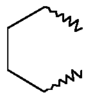 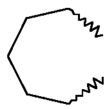 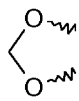 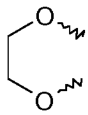 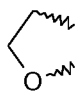 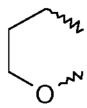

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

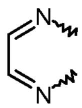 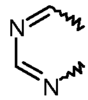 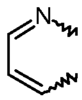 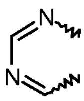 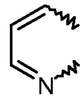 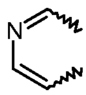

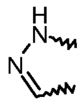 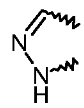 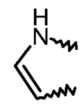

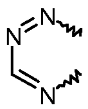 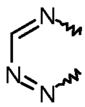 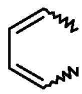

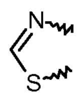 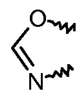 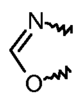

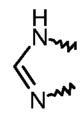 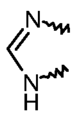 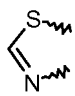

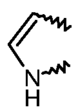 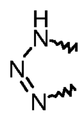 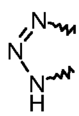

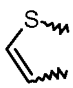 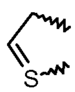 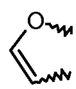 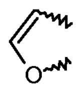

und
n für die Zahl 0 oder 1 steht.

Dabei ist es bevorzugt, dass

$R^1$ für Wasserstoff steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl, wobei die vorgenannten Reste jeweils gegebenenfalls substituiert sein können steht; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, steht; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl steht, wobei die vorgenannten Reste jeweils gegebenenfalls substituiert sein können oder für Carbonyl oder Carboxyl steht; oder

für einen Phenyl- oder Hetarylring, der gegebenenfalls einfach oder mehrfach, gleich oder verschieden substituiert ist mit Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl steht; oder

für $(C_1-C_6)$Alkoxy, Halogen$(C_1-C_6)$alkoxy, $(C_3-C_6)$Cycloalkyloxy, oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können oder für Sulfanyl steht; und

$R^2$ und $R^3$ für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, $(C_1-C_6)$Alkoxy$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl stehen, wobei die vorgenannten Reste jeweils gegebenenfalls substituiert sein können; oder

für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, stehen; oder

für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl stehen, wobei die vorgenannten Reste jeweils gegebenenfalls substituiert sein können oder für Carbonyl oder Carboxyl stehen; oder

für gegebenenfalls substituiertes Phenyl oder gegebenenfalls substituiertes Hetaryl stehen; oder

für einen Phenyl- oder Hetarylring, der gegebenenfalls einfach oder mehrfach, gleich oder verschieden substituiert ist mit Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl stehen; oder

für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino stehen, wobei die vorgenannten Reste jeweils gegebenenfalls substituiert sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können oder für Sulfanyl stehen; oder

für einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, stehen;

W für Wasserstoff oder Halogen, insbesondere Fluor und Chlor, steht;

V für Sauerstoff, Schwefel oder einen gegebenenfalls substituierten Stickstoff steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

n für die Zahl 0 oder 1 steht.

Bevorzugt steht die Substruktur der Formel (I-E) für die Substruktur

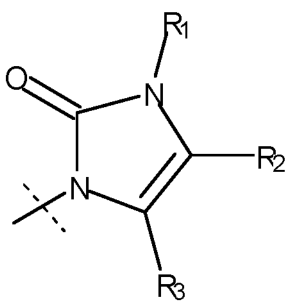

(I-E-1)

wobei

$R^1$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Oxetan, Oxolan, Oxan, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^2$ und $R^3$ unabhängig voneinander für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl stehen, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, stehen;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Hierbei ist es weiter bevorzugt, dass

$R^1$für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

$R^2$ und $R^3$unabhängig voneinander für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, $(C_1-C_6)$Alkoxy$(C_1-C_6)$alkyl oder Phenyl$(C_1-C_3)$alkyl, stehen, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, stehen;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Methoxy, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, wenn

$R^1$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, Benzyl, Cyclopropyl, Cyclobutyl, oder Phenyl steht;

$R^2$ und $R^3$ unabhängig voneinander für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$, Benzyl, Cyclopropyl, Cyclobutyl oder Phenyl stehen;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, wenn

$R^1$ für Ethyl und $C(CH_3)_3$ steht;

$R^2$ und $R^3$ für Wasserstoff stehen;

W für Fluor steht;

X für Fluor steht;

Y für Methyl steht;

Z für Wasserstoff steht; und

n für die Zahl 0 oder 1 steht.

Sechste Ausführungsform (I-F):

In einer sechsten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-F) stehen

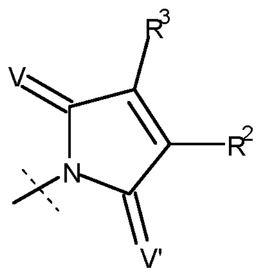

(I-F)

$R^2$ und $R^3$ unabhängig voneinander
für Wasserstoff, Cyano, Halogen oder Nitro steht; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylearbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die

definierte Mindestanzahl an Kohlenstoffatomen 2 beträgt, die Reste gesättigt oder ungesättigt sein können oder für Sulfanyl stehen; oder
für einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten dreibis sechsgliedrigen Ring,

W für Wasserstoff oder Halogen steht;

V und V' für Sauerstoff, Schwefel oder einen gegebenenfalls substituierten Stickstoff stehen;

X, Y und Zunabhängig voneinander für Wasserstoff, Halogen, Hydroxy, Amino, Cyano, Nitro, OCN, SCN, $SF_5$, stehen; oder
für Tri$(C_1-C_6)$alkylsilyl, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxycarbonyl$(C_1-C_6)$alkyl, $(C_1-C_6)$Alkoxy$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, $(C_1-C_6)$Alkoxy, Halogen$(C_1-C_6)$alkoxy, Cyano$(C_1-C_6)$alkoxy, Hydroxycarbonyl$(C_1-C_6)$alkoxy, $(C_1-C_6)$Alkoxycarbonyl$(C_1-C_6)$alkoxy, $(C_1-C_6)$Alkoxy$(C_1-C_6)$alkoxy, $(C_1-C_6)$Alkylhydroxyimino, $(C_1-C_6)$Alkoxyimino, $(C_1-C_6)$Alkyl$(C_1-C_6)$alkoxyimino, Halogen$(C_1-C_6)$alkyl$(C_1-C_6)$alkoxyimino, $(C_1-C_6)$Alkylthio, Halogen$(C_1-C_6)$alkylthio, $(C_1-C_6)$Alkoxy$(C_1-C_6)$alkylthio, $(C_1-C_6)$Alkylthio$(C_1-C_6)$alkyl, $(C_1-C_6)$Alkylsulfinyl, Halogen$(C_1-C_6)$alkylsulfinyl, $(C_1-C_6)$Alkoxy$(C_1-C_6)$alkylsulfinyl, $(C_1-C_6)$Alkylsulfinyl$(C_1-C_6)$alkyl, $(C_1-C_6)$Alkylsulfonyl, Halogen$(C_1-C_6)$alkylsulfonyl, $(C_1-C_6)$Alkoxy$(C_1-C_6)$alkylsulfonyl, $(C_1-C_6)$Alkylsulfonyl$(C_1-C_6)$alkyl, $(C_1-C_6)$Alkylsulfonyloxy, $(C_1-C_6)$Alkylcarbonyl, Halogen$(C_1-C_6)$alkylcarbonyl, Carboxyl, $(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cyclo$(C_1-C_6)$alkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder
für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,

wobei R' und R" unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

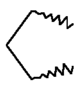 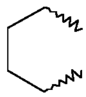 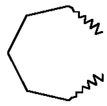 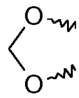 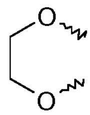 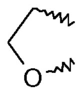 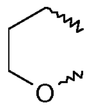

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

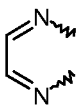 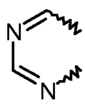 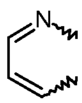 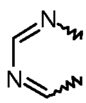 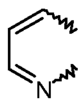 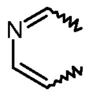

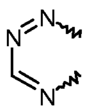 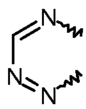 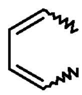 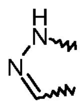 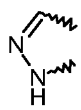 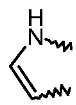

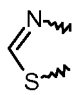 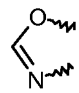 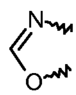 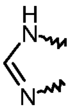 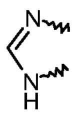 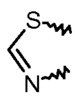

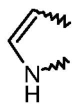 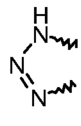 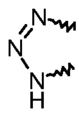

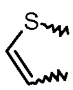 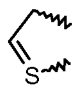 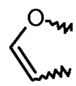 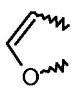

und

n für die Zahl 0 oder 1 steht.

Dabei ist es bevorzugt, dass

$R^2$ und $R^3$, unabhängig voneinander,
für Wasserstoff, Cyano, Halogen oder Nitro stehen; oder
für $(C_1\text{-}C_6)$Alkyl, $(C_3\text{-}C_6)$Cycloalkyl$(C_1\text{-}C_6)$alkyl, Halogen$(C_1\text{-}C_6)$alkyl, Cyano$(C_1\text{-}C_6)$alkyl, $(C_1\text{-}C_6)$Alkoxy$(C_1\text{-}C_6)$alkyl,

$(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, Phenyl$(C_1-C_6)$alkyl, Hetaryl$(C_1-C_6)$alkyl, $(C_2-C_6)$Alkenyl, Halogen$(C_2-C_6)$Alkenyl, $(C_3-C_6)$Cycloalkenyl$(C_1-C_6)$alkyl, $(C_2-C_6)$Alkinyl stehen, wobei die vorgenannten Reste jeweils gegebenenfalls substituiert sein können; oder
für gegebenenfalls substituiertes $(C_3-C_6)$Cycloalkyl, $(C_3-C_6)$Cycloalkenyl, welches gegebenenfalls durch ein oder mehrere Heteroatome aus der Reihe O, S oder N unterbrochen sein kann, stehen; oder
für $(C_1-C_6)$Alkylcarbonyl, Halogen$(C_1-C_6)$alkylcarbonyl, $(C_1-C_6)$Alkoxy$(C_1-C_6)$alkylcarbonyl, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, $(C_1-C_6)$Alkylaminothiocarbonyl, Di$(C_1-C_6)$alkylaminothiocarbonyl, $(C_2-C_6)$Alkenylcarbonyl, $(C_3-C_6)$Cycloalkenyl$(C_1-C_6)$alkylcarbonyl, $(C_2-C_6)$Alkinylcarbonyl oder Aminothiocarbonyl stehen, wobei die vorgenannten Reste jeweils gegebenenfalls substituiert sein können oder für Carbonyl oder Carboxyl stehen; oder
für gegebenenfalls substituiertes Phenyl oder gegebenenfalls substituiertes Hetaryl stehen; oder
für einen Phenyl- oder Hetarylring, der gegebenenfalls einfach oder mehrfach, gleich oder verschieden substituiert ist mit Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl stehen; oder
für $(C_1-C_6)$Alkoxy, Halogen$(C_1-C_6)$alkoxy, $(C_1-C_6)$Alkoxy$(C_1-C_6)$alkoxy, $(C_3-C_6)$Cycloalkyloxy, $(C_3-C_6)$Cycloalkyl$(C_1-C_6)$alkyloxy, $(C_2-C_6)$Alkenyloxy, $(C_3-C_6)$Cycloalkenyl$(C_1-C_6)$alkoxy, $(C_2-C_6)$Alkinyloxy oder Carbonyloxy stehen, wobei die vorgenannten Reste jeweils gegebenenfalls substituiert sein können oder für Hydroxy stehen; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_1-C_6)$Alkylcarbonylamino, $(C_1-C_6)$Alkoxycarbonylamino, $(C_1-C_6)$Alkylcarbamoylamino, $(C_1-C_6)$Alkylsulfonylamino, $(C_2-C_6)$Alkenylamino, $(C_3-C_6)$Cycloalkenyl$(C_1-C_6)$alkylamino, $(C_2-C_6)$Alkinylamino steht, wobei die vorgenannten Reste jeweils gegebenenfalls substituiert sein können oder Amino stehen; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können oder für Sulfanyl stehen; oder
für einen gegebenenfalls gleich oder verschieden durch Halogen, Cyano, Nitro, Hydroxy, Amino, Methyl, Ethyl, Trifluormethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, stehen;

| | |
|---|---|
| W | für Wasserstoff, Fluor oder Chlor steht; |
| V und V' | jeweils unabhängig voneinander, für Sauerstoff, Schwefel oder einen gegebenenfalls substituierten Stickstoff stehen; |
| X, Y und Z | unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;<br>oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;<br>oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; |
| n | für die Zahl 0 oder 1 steht. |

Bevorzugt steht die Substruktur der Formel (I-F) für die Substruktur

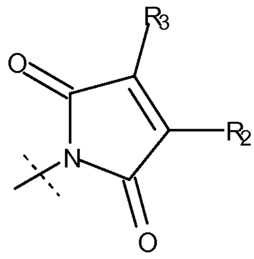

(I-F-1)

wobei

$R^2$ und $R^3$ unabhängig voneinander für Wasserstoff oder Halogen stehen; oder für $(C_1\text{-}C_6)$Alkyl, $(C_3\text{-}C_6)$Cycloalkyl$(C_1\text{-}C_6)$alkyl, $(C_2\text{-}C_6)$Alkenyl, $(C_2\text{-}C_6)$Alkinyl, Halogen$(C_1\text{-}C_6)$alkyl, Cyano$(C_1\text{-}C_6)$alkyl, $(C_1\text{-}C_6)$Alkoxy$(C_1\text{-}C_6)$alkyl oder Phenyl$(C_1\text{-}C_3)$alkyl, stehen, wobei die vorgenannten Reste jeweils gegebenenfalls substituiert sein können; oder für $(C_3\text{-}C_6)$Cycloalkyl, $(C_3\text{-}C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1\text{-}C_3)$alkylsulfanyl oder gegebenenfalls durch Methyl, Fluor, Chlor, Cyano substituiertes Cyclopropyl substituiert sein können, stehen;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^2$ für Wasserstoff, Fluor, Chlor, Brom, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$. $CH_2CH_2OCH_3$, Benzyl, Cyclopropyl, Cyclobutyl oder Phenyl steht;

$R^3$ für Wasserstoff, Fluor, Chlor, Brom, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$. $CH_2CH_2OCH_3$, Benzyl, Cyclopropyl, Cyclobutyl oder Phenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), $(CF_3,H)$;

Z für Wasserstoff steht; und

n für die Zahl 0 oder 1 steht.

Hierbei ist es ganz besonders bevorzugt, dass

$R^2$ für Chlor, Brom oder Methyl steht;

$R^3$ für Wasserstoff, Chlor oder Methyl steht;

W für Fluor steht;

X für Fluor oder Methyl steht;

Y für Methyl steht; wobei

X und Y insbesondere für folgende Kombinationen (Y,X) stehen: (Me, F), (Me,Me);

Z für Wasserstoff steht; und

n für die Zahl 0 oder 1 steht.

Siebte Ausführungsform (I-G):

In einer siebten Ausführungsform der vorliegenden Erfindung weisen die erfindungsgemäßen Verbindungen eine Struktur der allgemeinen Formel (I) auf, in welcher

A und B gemeinsam mit den Atomen, an die sie gebunden sind, für eine Substruktur der Formel (I-G) stehen

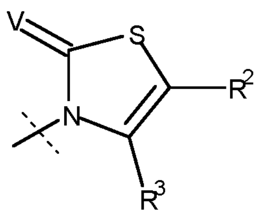

(I-G)

$R^2$ und $R^3$ unabhängig voneinander
für Wasserstoff, Cyano, Halogen oder Nitro steht; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten dreibis sechsgliedrigen Ring,

W für Wasserstoff oder Halogen steht;

V für Sauerstoff, Schwefel oder einen gegebenenfalls substituierten Stickstoff stehen;

X, Y und Z unabhängig voneinander für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cyclo($C_1$-$C_6$)alkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,

wobei R' und R" unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

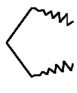 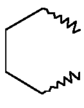 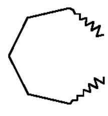 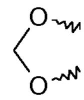 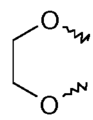 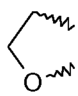 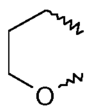

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

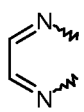 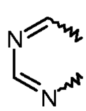 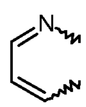 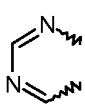 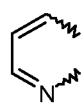 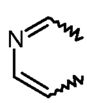

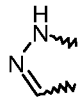
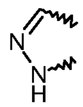
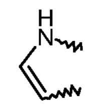
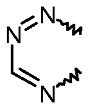
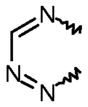
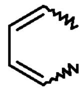
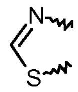
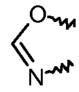
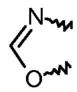
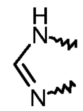
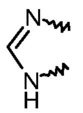
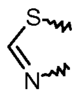
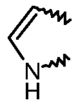
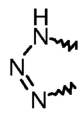
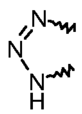
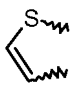
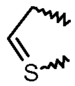
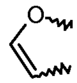
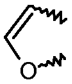

und

n für die Zahl 0 oder 1 steht.

Dabei ist es bevorzugt, dass

$R^2$ und $R^3$, unabhängig voneinander,
für Wasserstoff, Cyano, Halogen oder Nitro steht; oder
für $(C_1$-$C_6)$Alkyl, $(C_3$-$C_6)$Cycloalkyl$(C_1$-$C_6)$alkyl, Halogen$(C_1$-$C_6)$alkyl, Cyano$(C_1$-$C_6)$alkyl, $(C_1$-$C_6)$Alkoxy$(C_1$-$C_6)$alkyl, $(C_1$-$C_6)$Alkoxycarbonyl$(C_1$-$C_6)$alkyl, $(C_1$-$C_6)$Alkylsulfanyl$(C_1$-$C_6)$alkyl, $(C_1$-$C_6)$Alkylsulfinyl$(C_1$-$C_6)$alkyl, Phenyl$(C_1$-$C_6)$alkyl, Hetaryl$(C_1$-$C_6)$alkyl, $(C_2$-$C_6)$Alkenyl, Halogen$(C_2$-$C_6)$Alkenyl, $(C_3$-$C_6)$Cycloalkenyl$(C_1$-$C_6)$alkyl, $(C_2$-$C_6)$Alkinyl stehen, wobei die vorgenannten Reste jeweils gegebenenfalls substituiert sein können; oder
für gegebenenfalls substituiertes $(C_3$-$C_6)$Cycloalkyl, $(C_3$-$C_6)$Cycloalkenyl, welches gegebenenfalls durch ein oder mehrere Heteroatome aus der Reihe O, S oder N unterbrochen sein kann, stehen; oder
für $(C_1$-$C_6)$Alkylcarbonyl, Halogen$(C_1$-$C_6)$alkylcarbonyl, $(C_1$-$C_6)$Alkoxy$(C_1$-$C_6)$alkylcarbonyl, $(C_1$-$C_6)$Alkoxycarbonyl, Halogen$(C_1$-$C_6)$alkoxycarbonyl, Aminocarbonyl, $(C_1$-$C_6)$Alkylaminocarbonyl, Di$(C_1$-$C_6)$alkylaminocarbonyl, $(C_3$-$C_6)$Cycloalkylaminocarbonyl, $(C_1$-$C_6)$Alkylaminothiocarbonyl, Di$(C_1$-$C_6)$alkylaminothiocarbonyl, $(C_2$-$C_6)$Alkenylcarbonyl, $(C_3$-$C_6)$Cycloalkenyl$(C_1$-$C_6)$alkylcarbonyl, $(C_2$-$C_6)$Alkinylcarbonyl oder Aminothiocarbonyl stehen, wobei die vorgenannten Reste jeweils gegebenenfalls substituiert sein können oder für Carbonyl oder Carboxyl stehen; oder
für einen Phenyl- oder Hetarylring, der gegebenenfalls einfach oder mehrfach, gleich oder verschieden substituiert ist mit Halogen, Cyano, Nitro, Hydroxy, Amino, $(C_1$-$C_6)$Alkyl, Halogen$(C_1$-$C_6)$Alkyl, $(C_1$-$C_6)$Alkoxy, Halogen$(C_1$-$C_6)$alkoxy, $(C_1$-$C_6)$Alkylsulfinyl, $(C_1$-$C_6)$Alkylsulfanyl, $(C_1$-$C_6)$Alkylsulfonyl, Halo$(C_1$-$C_6)$alkylsulfinyl, Halo$(C_1$-$C_6)$alkylsulfanyl, Halo$(C_1$-$C_6)$alkylsulfonyl oder gegebenenfalls substituiertes $(C_3$-$C_6)$Cycloalkyl stehen; oder
für $(C_1$-$C_6)$Alkoxy, Halogen$(C_1$-$C_6)$alkoxy, $(C_1$-$C_6)$Alkoxy$(C_1$-$C_6)$alkoxy, $(C_3$-$C_6)$Cycloalkyloxy, $(C_3$-$C_6)$Cycloalkyl$(C_1$-$C_6)$alkyloxy, $(C_2$-$C_6)$Alkenyloxy, $(C_3$-$C_6)$Cycloalkenyl$(C_1$-$C_6)$alkoxy, $(C_2$-$C_6)$Alkinyloxy oder Carbonyloxy stehen, wobei die vorgenannten Reste jeweils gegebenenfalls substituiert sein können oder für Hydroxy stehen; oder
für $(C_1$-$C_6)$Alkylamino, Halogen$(C_1$-$C_6)$alkylamino, Dihalogen$(C_1$-$C_6)$alkylamino, Di$(C_1$-$C_6)$alkylamino, $(C_3$-$C_6)$Cycloalkylamino, Di$(C_3$-$C_6)$cycloalkylamino, $(C_1$-$C_6)$Alkylcarbonylamino, , $(C_1$-$C_6)$Alkoxycarbonylamino, $(C_1$-$C_6)$Alkylcarbamoylamino, $(C_1$-$C_6)$Alkylsulfonylamino, $(C_2$-$C_6)$Alkenylamino, $(C_3$-$C_6)$Cycloalkenyl$(C_1$-$C_6)$alkylamino, $(C_2$-$C_6)$Alkinylamino stehen, wobei die vorgenannten Reste jeweils gegebenenfalls substituiert sein können oder für Amino stehen; oder
für $(C_1$-$C_6)$Alkylsulfanyl, $(C_1$-$C_6)$Alkylsulfinyl, $(C_1$-$C_6)$Alkylsulfonyl, Halogen$(C_1$-$C_6)$alkylsulfanyl, Halogen$(C_1$-$C_6)$alkylsulfinyl, Halogen$(C_1$-$C_6)$alkylsulfanyl, $(C_3$-$C_6)$Cycloalkyl$(C_1$-$C_6)$alkylsulfanyl, $(C_3$-$C_6)$Cy-

cloalkyl($C_1$-$C_6$)alkylsulfinyl, ($C_3$-$C_6$)Cycloalkyl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können oder für Sulfanyl stehen; oder
für einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten dreibis sechsgliedrigen Ring, stehen;

W für Wasserstoff, Fluor oder Chlor steht;

V für Sauerstoff, Schwefel oder einen gegebenenfalls substituierten Stickstoff stehen;

X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

n für die Zahl 0 oder 1 steht.

Bevorzugt steht die Substruktur der Formel (I-G) für die Substruktur

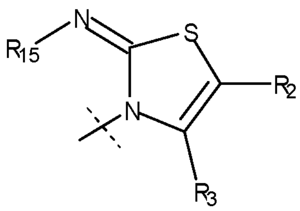

(I-G-1)

wobei

$R^2$ und $R^3$ unabhängig voneinander für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl stehen, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder
für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, stehen;

$R^{15}$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Oxetan, Oxolan, Oxan, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

Hierbei sind insbesondere auch die E- und Z-Isomere der Substruktur der Formel (I-G) eingeschlossen.
Handelt es sich bei der Substruktur der Formel (I-G) um die Substrukturformel (I-G-1), so ist es bevorzugt, dass

$R^2$ und $R^3$ unabhängig voneinander für Wasserstoff stehen; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl, stehen, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, stehen;

$R^{15}$ für Wasserstoff steht; oder
für $(C_1-C_6)$Alkyl, $(C_3-C_6)$Cycloalkyl$(C_1-C_6)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl, wobei die vorgenannten Reste jeweils gegebenenfalls substituiert sein können; oder
für $(C_3-C_6)$Cycloalkyl, $(C_3-C_6)$Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1-C_3)$alkylsulfanyl oder gegebenenfalls durch Methyl, Fluor, Chlor, Cyano substituiertes Cyclopropyl substituiert sein können, steht;

W Wasserstoff oder Halogen, insbesondere F oder Cl, steht;

X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;

Z für Wasserstoff steht; und

n für die Zahl 0 oder 1 steht.

Hierbei ist es besonders bevorzugt, dass

$R^2$ für Wasserstoff, Fluor, Chlor, Brom, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$, $CH_2CH_2OCH_3$. Benzyl, Cyclopropyl, Cyclobutyl oder Phenyl steht;

$R^3$ für Wasserstoff, Fluor, Chlor, Brom, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH{=}CH_2$, $CH{=}CHCH_3$, $CH_2CH{=}CH_2$, $CH_2CCH$, Cyclopropylmethyl, $CF_3$, $CH_2CF_3$, $CH_2CHF_2$, $CH_2CN$, $CH_2OCH_3$, $CH_2OCH_2CH_3$. $CH_2CH_2OCH_3$, Benzyl, Cyclopropyl, Cyclobutyl oder Phenyl steht;

$R^{15}$ für Wasserstoff, Methyl, Ethyl, n-Propyl, $CH(CH_3)_2$, n-Butyl, sec-Butyl, $C(CH_3)_3$, $CH_2C(CH_3)_3$, $CH_2CH(CH_3)_2$, $CH_2CH{=}CH_2$, Cyclopropylmethyl, $CH_2CF_3$, $CH_2CHF_2$, $CH_2CN$, $CH_2CH_2OCH_3$, Benzyl, Cyclopropyl, Cyclobutyl oder Phenyl steht;

W Wasserstoff oder Fluor steht;

X für Wasserstoff, Chlor, Fluor oder Methyl steht;

Y für Fluor, Chlor, Brom, Cyano, Methyl, Methoxy oder Trifluoromethyl steht; wobei

X und Y insbesondere für folgende Kombinationen (Y,X) stehen: (Me, F), (Me,H), (Me,Cl), (Me,Me), (Cl,Cl), (Cl,F), (CN,F), (CN,H), (MeO,F), (MeO,H), (Cl,H), (Br,H), (Br,F), (F,F), ($CF_3$,H);

Z für Wasserstoff steht; und

n für die Zahl 0 oder 1 steht.

Hierbei ist es ganz besonders bevorzugt, wenn

$R^2$ für Methyl steht;

$R^3$ für Methyl steht;

$R^{15}$ für $CH_2CF_3$ steht;

W für Fluor steht;

X für Fluor steht;

Y für Methyl steht; wobei

Z für Wasserstoff steht; und

n für die Zahl 0 oder 1 steht.

Hierbei ist es ebenfalls ganz besonders bevorzugt, wenn

$R^2$ für Wasserstoff oder Methyl steht;

$R^3$ für Wasserstoff oder Methyl steht;

$R^{15}$ für $CH_2CF_3$ steht;

W für Fluor steht;

X für Fluor steht;

Y für Methyl steht; wobei

Z für Wasserstoff steht; und

n für die Zahl 0 oder 1 steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl, Alkinyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im Rahmen der vorliegenden Erfindung steht Halogen für Fluor, Chlor, Brom und Jod, besonders bevorzugt für Fluor, Chlor und Brom, und ganz besonders bevorzugt für Fluor und Chlor.

Ferner steht Alkyl für geradkettiges oder verzweigtes $C_1$- bis $C_8$-Alkyl, bevorzugt geradkettiges oder verzweigtes $C_1$- bis $C_6$-Alkyl, weiter bevorzugt geradkettiges oder verzweigtes $C_1$- bis $C_4$-Alkyl, insbesondere Methyl und Ethyl.

Alkoxy steht für geradkettiges oder verzweigtes $C_1$- bis $C_8$-Alkoxy, bevorzugt geradkettiges oder verzweigtes $C_1$- bis $C_6$-Alkoxy, weiter bevorzugt geradkettiges oder verzweigtes $C_1$- bis $C_4$-Alkoxy, insbesondere Methoxy.

Halogenalkyl und Halogenalkoxy ergeben sich aus substituierten Alkyl- bzw. Alkoxyresten gemäß der vorstehenden Definition.

Alkylreste in Cycloalkyl, Alkoxycarbonyl, Alkylthioalkyl, Alkylsulfinylalkyl, Phenylalkyl, Hetarylalkyl und Alkylsulfonylalkyl ergeben sich ebenfalls aus der vorstehenden Definition von Alkyl.

## Herstellverfahren

Die Verbindungen der allgemeinen Formel (I) lassen sich in Verbindungen mit n=0 (Ia) und n=1 (Ib) unterteilen und können prinzipiell nach den allgemeinen Verfahren V1, V2, V3 und V3' hergestellt werden.

Unter **Verfahren V1** lassen sich alle Methoden zusammenfassen, die -meistens in einem mehrstufigen Prozesseinen Aufbau des 5-gliedrigen Ringes ermöglichen, insbesondere ausgehend aus den Anilinen der allgemeinen Formel (IVa), nach folgendem Schema,

VERFAHREN V1

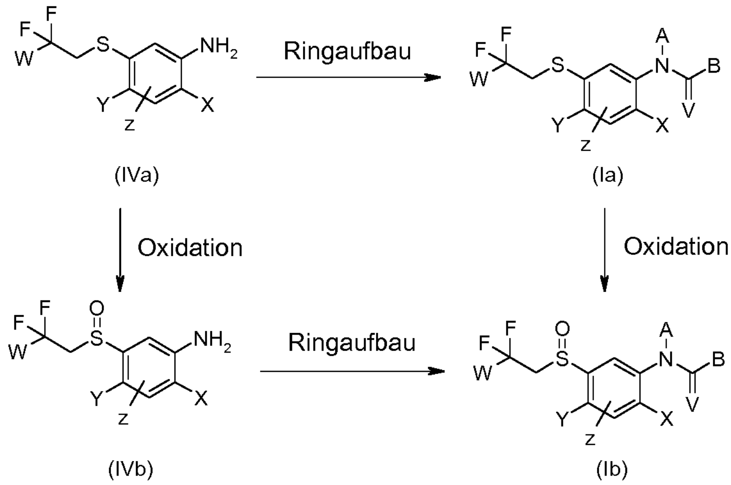

(IVa) (Ia) (IVb) (Ib)

wobei X, Y, Z, A, B und V die oben angegebene Bedeutung haben.

Durch Oxidation der Thioether der allgemeinen Formel (Ia) nach literaturbekannten Methoden können die Sulfoxide der allgemeinen Formel (Ib) erhalten werden.

Alternativ können einige der in Verfahren V1 beschriebenen Methoden auch ausgehend von Sulfoxiden der allgemeinen Formel (IVb) zu den Sulfoxiden der allgemeinen Formel (Ib) angewandt werden. Die Sulfoxide der Formel (IVb) können aus den Sulfiden (IVa) nach literaturbekannten Methoden hergestellt werden.

Verfahren V1 eignet sich insbesondere zur Darstellung der Ausführungsformen I-A bis I-G.

Alternativ können die Verbindungen der allgemeinen Formel (Ia) nach **Verfahren V2** hergestellt werden, nach folgendem Schema,

VERFAHREN V2

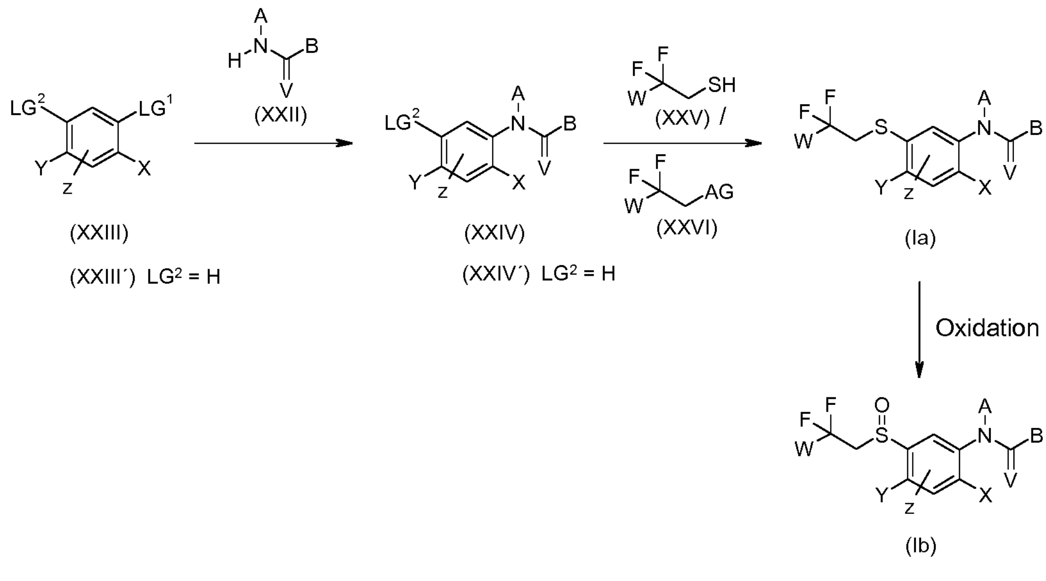

(XXII) (XXV) (XXVI)
(XXIII) (XXIII´) $LG^2$ = H
(XXIV) (XXIV´) $LG^2$ = H
(Ia) (Ib)

wobei Z, A, B, V und W die oben angegebene Bedeutung haben, X steht für Wasserstoff oder eine elektronenziehende Gruppe (insbesondere Nitro, Chlor, Fluor, Cyano), Y stellt elektronenziehende Substituenten (insbesondere Nitro, Chlor, Fluor, Cyano) dar, $LG^1$ steht für typische Abgangsgruppen in nucleophilen Substitutionsreaktionen (insbesondere Fluorid, Chlorid) und $LG^2$ kann für Wasserstoff oder für typische Abgangsgruppen in nucleophilen Substitutionsreaktionen (insbesondere Fluorid, Chlorid) stehen.

Die Umsetzung von Verbindungen der allgemeinen Formel (XXIII) mit heterocyclischen Verbindungen der allgemeinen Formel (XXII), meistens unter basischen Reaktionsbedingungen wie zum Beispiel in US 6906006 und DE 19500439 für Triazolinone, WO 2010/0119194 für Hydantoine, DE 4431218 für Pyrimidin(thi)one, WO 2009/012275

und WO 2008/155034 für Pyridone oder DE 19528305 für Uracile beschrieben, liefert die Verbindungen der allgemeinen Formel (XXIV). Durch erneute nukleophile aromatische Substitution mit Thiolen der allgemeinen Formel (XXV) können die Thioether der Formel (Ia) hergestellt werden. Geeignete Reaktionsbedingungen für solche Reaktionen werden in WO 2007/131680 und WO 2008/086226 beschrieben.

Durch Oxidation der Thioether der allgemeinen Formel (Ia) nach literaturbekannten Methoden können die Sulfoxide der allgemeinen Formel (Ib) erhalten werden.

Verbindungen der Formel (XXIII) in denen $LG^2$ für Wasserstoff steht werden als (XXIII') genannt und können auf ähnlicher Weise wie oben dargestellt mit Verbindungen der Formel (XXII) umgesetzt werden, in diesem Fall zu Verbindungen der Formel (XXIV').

Verbindungen der Formel (XXIV') sind zum Teil kommerziell erhältlich.

Die Verbindungen der Formel (XXIV') können in einem mehrstufigen Prozess zu den erfindungsgemäßen Verbindungen (Ia) umgesetzt werden. Zu den benötigten Schritten gehören Chlorsulfonierung, Reduktion und Alkylierung mit Haloalkylelektrophilen der Formel (XXVI), alle möglich nach literaturbekannten Methoden. Die Chlorsulfonierung der Verbindungen (XXIV') mit Chlorsulfonsäure liefert die entsprechenden Sulfonylchloride und diese können in deren Disulfide mit literaturbekannten Methoden wie zum Beispiel Eisen in Salzsäure oder Iodid überführt werden. Die Umsetzung der Disulfide mit Haloalkylelektrophilen der Formel (XXVI) liefert die Sulfide (Ia).

Durch Oxidation der Thioether der allgemeinen Formel (Ia) nach literaturbekannten Methoden können die Sulfoxide der allgemeinen Formel (Ib) erhalten werden.

Verfahren V2 eignet sich insbesondere zur Darstellung der Ausführungsformen I-A bis I-G, bei denen X für Wasserstoff oder elektronenziehende Substituenten (insbesondere Nitro, Chlorid, Fluorid, Cyano) und Y für elektronenziehende Substituenten (insbesondere Nitro, Chlorid, Fluorid, Cyano) stehen.

Alternativ können die Verbindungen der allgemeinen Formel (Ia) nach **Verfahren V3 und V3'** hergestellt werden, wie im folgendem Schema dargestellt,

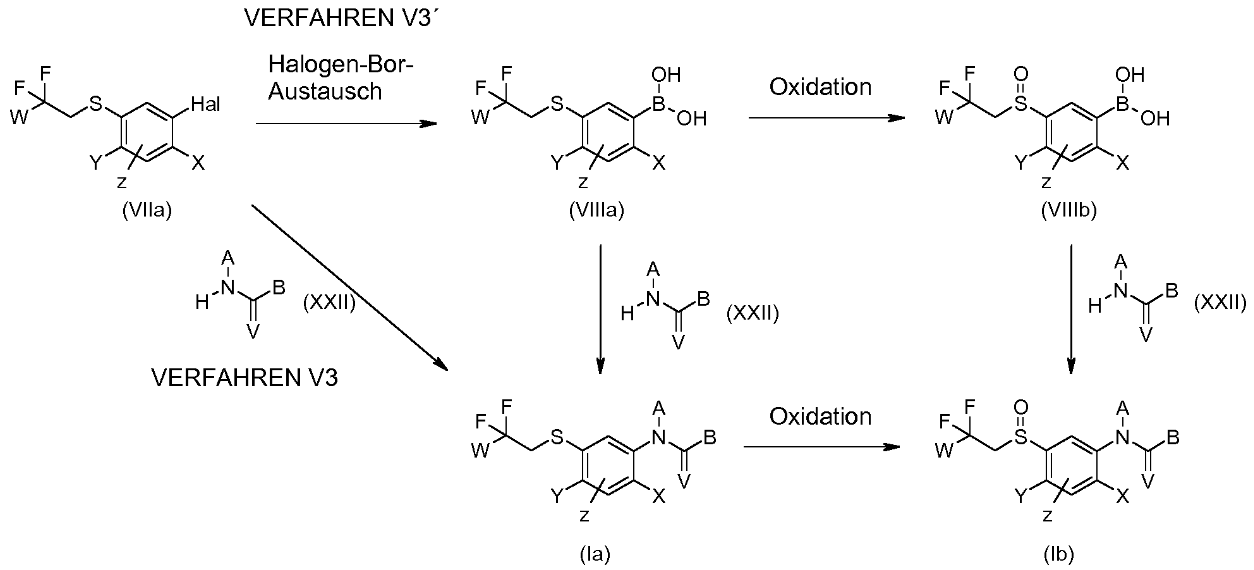


wobei X, Y, Z, W, A, B und V die oben angegebene Bedeutung haben und Hal steht für Halogen (bevorzugt Chlor, Brom, Iod).

Laut Verfahren V3 können Verbindungen der allgemeinen Formel (Ia) nach literaturbekannten Methoden durch Umsetzung von Arylhalogeniden der allgemeinen Formel (VIIa) mit heterocyclischen Verbindungen der allgemeinen Formel (XXII) hergestellt werden. Bevorzugt findet die Umsetzung durch Übergangsmetall-Katalyse oder -Vermittlung statt. Beispielhafte Reaktionsbedingungen sind in der Literatur zahlreich bekannt, zum Beispiel in WO 2006/117657 A1, in US 2010/99725 A1, in WO 2010/47956 A1, in Chem. Pharm. Bull. 1997, Vol. 45, No. 4, 719-721, in J. Amer. Chem. Soc. 2003, Vol. 125, No. 37, 11253-11258 oder auch in Bull. Korean Chem. Soc. 2010, Vol. 31, No. 8, 2143-2146. Bevorzugt werden Kupfer oder Kupfersalze, zum Beispiel Kupfer(I)-iodid, Kupfer(I)-oxid, Kupfer(I)-triflat oder Kupfer(II)-triflat, als Katalysator verwendet, häufig in Gegenwart eines Liganden, zum Beispiel Diamin-Liganden wie *N,N'*-Dimethylethylendiamin, *N,N*-Dimethylethylendiamin oder *trans-N,N'*-Dimethyl-1,2-cyclohexandiamin. Eine Übersicht findet sich zum Beispiel in Chem. Sci. 2010, Vol. 1, 13-31. Alternativ können 1,3-Diketone, wie z. B. 2,4-Pentandion, 2,2,6,6-Tetramethyl-3,5-heptandion oder Dibenzoylmethan, Aminosäuren, wie z.B. L-Prolin oder Glycin oder andere Verbindungen wie 8-Hydroxychinolin (Tetrahedron Lett. 2009, Vol. 50, 7293-7296), Dibenzylidenaceton, Bipyridin oder

Phenanthrolin Verwendung finden. In der Regel wird die Umsetzung unter Anwesenheit einer Base, häufig Carbonat- oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid durchgeführt. Desweiteren können Additive wie z. B. Kaliumiodid, Cäsiumfluorid oder andere Salze Einsatz finden.

Alternativ lassen sich derartige Umsetzungen unter Palladiumkatalyse durchführen, etwa durch Einsatz von Katalysatoren wie zum Beispiel Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl, 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid.

Verbindungen der allgemeinen Formel (Ia) können alternativ nach Verfahren V3' durch Umsetzung von Boronsäuren der allgemeinen Formel (VIIIa) mit heterocyclischen Verbindungen der allgemeinen Formel (XXII) hergestellt werden.

In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel mit Molekularsieb) statt. Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N,N*-Dimethylformamid, Tetrahydrofuran, Dioxan, Acetonitril, Essigester oder Toluol verwendet. In der Literatur werden zahlreiche Beispiele beschrieben, unter anderem in Bioorg. Med. Chem. Lett. 2010, Vol. 20, No. 13, 3920-3924, in Proc. Natl. Acad. Sci. USA 2011, Vol. 108, No. 17, 6781-6786, in Chem. Eur. J. 2009, Vol. 15, No. 29, 7044-7047, in Synlett 2004, Vol. 6, 1095-97, in J. Org. Chem. 2005, 70, 4, 1486-1489, in Tetrahedron Lett. 1998, Vol. 39, 2933-2936, in WO 2005/85226 A1 oder in WO 2008/62905 A2. Zusammenfassende Übersichten finden sich zum Beispiel in Synthesis 2011, No. 6, 829-856 oder in Tetrahedron 2012, Vol. 68, 7735-7754. Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Durch Oxidation der Thioether der allgemeinen Formel (Ia) nach literaturbekannten Methoden können die Sulfoxide der allgemeinen Formel (Ib) erhalten werden. Alternativ kann die oxidative übergangsmetallvermittelte Kohlenstoff-Stickstoff-Kupplung zu Arylsulfoxiden der allgemeinen Formel (Ib) ausgehend von Boronsäure-Sulfoxiden der allgemeinen Formel (VIIIb), welche durch Oxidation der Boronsäuren (VIIIa), zum Beispiel mit Natriumperiodat, oder analoger Derivate zugänglich sind, ermöglicht werden.

Verfahren V3 und V3' sind insbesondere zur Darstellung der Ausführungsformen I-A, I-B, I-C, I-D und I-F geeignet.

Verfahren Va1 bis Va5 eignen sich zur Darstellung der Ausführungsform I-A der Verbindungen der Formel (I).

**Verfahren Va1 und Va2 (I-A-1, I-A-2, I-A-3, I-A-4, I-A-6, I-A-8, I-A-10)**

Die 2-Aryltriazolin-3-one der allgemeinen Formel (I-A-1, I-A-2, I-A-3, I-A-4, I-A-6, I-A-8, I-A-10) können in (I-A-1a, I-A-2a, I-A-3a, I-A-4a, I-A-6a, I-A-8a, I-A-10a) (für n=0) und (I-A-1b, I-A-2b, I-A-3b, I-A-4b, I-A-6b, I-A-8b, I-A-10b) (für n=1) unterteilt und beispielsweise nach **Verfahren Va1** hergestellt werden, wie im folgenden Schema,

VERFAHREN Va1

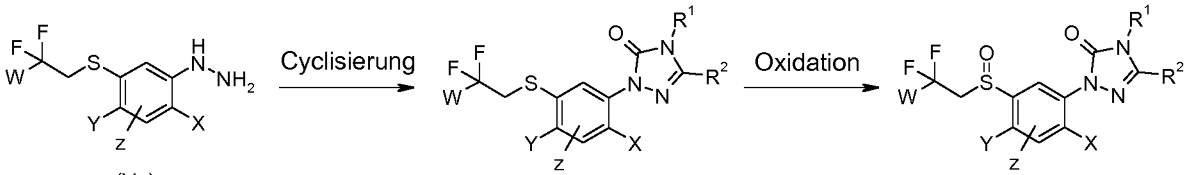


(Va)

(I-A-1b, I-A-2a, I-A-3a, I-A-4a, I-A-6a, I-A-8a, I-A-10a)

(I-A-1b, I-A-2b, I-A-3b, I-A-4b, I-A-6b, I-A-8b, I-A-10b)

wobei X, Y, Z, W, $R^1$ und $R^2$ die oben angegebene Bedeutung haben.

Verbindungen der allgemeinen Formel (I-A-1a, I-A-2a, I-A-3a, I-A-4a, I-A-6a, I-A-8a, I-A-10a) können ausgehend von Hydrazinen der allgemeinen Formel (Va) in mehrstufigen Prozessen hergestellt werden.

Die Darstellung der 2-Aryltriazolin-3-one der allgemeinen Formel (I-A-1a) ist zum Beispiel durch Kondensation von Hydrazinen der allgemeinen Formel (Va) mit geeigneten $\alpha$-Dicarbonylverbindungen oder deren Surrogaten und anschließender Cyclisierung möglich. Zahlreiche Vorschriften finden sich in der Literatur, zum Beispiel mit $\alpha$-Ketosäuren/Diphenylphosphorylazid ($R^1$ = H, $R^2$ = H, Alkyl, Aryl: Synth. Comm.1986, 16, 2, 163-167; $R^1$ = H, $R^2$ = H: Eur.

J. Med. Chem. 2011, 46, 10, 5039-5045; $R^1$ = H, $R^2$ = Aryl: J. Med. Chem. 2007, 50, 3, 528-542), mit Imidoylchloriden/Chlorameisensäureestern ($R^1$ = Aryl, $R^2$ = Haloalkyl: Tetrahedron Lett. 1990, 31, 19, 2717-2718), mit Alkyl-*N*-(alkoxycarbonyl)alkylimidoaten/Triethylamin ($R^1$ = H, Alkyl, Aryl, Benzyl, $R^2$ = Alkyl, Cycloalkyl: J. Med. Chem. 1993, 36, 2558-2568), mit Bromcyan/Schwefelwasserstoff/Methyliodid/Trichlortrifluoraceton ($R^1$ = H, $R^2$ = Haloalkyl: J. Heterocycl. Chem. 1983, 20, 1533-1537), mit *N*-Alkoxycarbonylthioamiden ($R^1$ = H, $R^2$ = Alkyl, Aryl, Heteroaryl: J. Org. Chem. 1976, 41, 20, 3233-3237).

Ferner lassen sich 2-Aryltriazolin-3-one der allgemeinen Formel (I-A-2a) durch Kondensation von Hydrazinen der allgemeinen Formel (Va) mit Carbamoylharnstoff zu Urazolen und anschließender Alkylierung zum Beispiel mit Diazomethan herstellen, wie zum Beispiel in J. Phys. Org. Chem. 1993, 6, 601-608 oder in Chem. Ber. 1903, 36, 3139-3154 ($R^1$ = Methyl, $R^2$ = Methoxy) beschrieben.

Die Synthese von 2-Aryltriazolin-3-onen der allgemeinen Formel (I-A-3a) wird zum Beispiel in Liebigs Ann. 1959, 627, 162-165 ausgehend von Arylsemicarbaziden mit Bromcyan beschrieben ($R^1$ = Aryl, $R^2$ = Amino). Synthese von 3-Alkoxycarbonylamino-1,2,4-triazolinonen ($R^1$ = Methyl, $R^2$ = $N(CH_3)CO_2CH_3$) ist gemäß Ind. J. Chem. Sect. B 1980, 19, 9, 805-809, möglich.

Zur Darstellung von 4-Amino-1,2,4-triazolin-3-onen der allgemeinen Formel (I-A-4a) sind ebenfalls Literaturvorschriften bekannt. Die Kondensation zweier Hydrazon-Derivate mit anschließender Cyclisierung zum Beispiel mit Phosgen führt gemäß Liebigs Ann. 1982, 5, 994-1000 zu 4-Amino-1,2,4-triazolin-3-onen ($R^1$ = Amino, $R^2$ = H, COOAlkyl). Eine weitere Synthese von 4-Amino-1,2,4-triazolin-3-onen ($R^1$ = $N(CO_2Alkyl')Alkyl$, $R^2$ = Aryl, Heteroaryl) ausgehend von Aldehyd-Hydrazonen, die wiederum aus Hydrazinen der allgemeinen Formel (Va) zugängig sind, in einer Kaskadenreaktion wird in Tetrahedron 2010, 66, 13, 2427-2432 bzw. in J. Computational Chem. 2012, 33, 7, 715-722, beschrieben. Desweiteren können 4-Amino-1,2,4-triazolin-3-one ($R^1$ = Amino, $NHC(O)CH_3$, $R^2$ = Alkyl) aus den entsprechenden 1,3,4-Oxadiazol-2(3H)-onen, die ebenfalls in einem mehrstufigen Prozess aus Hydrazinen der allgemeinen Formel (Va) zugängig sind, hergestellt werden, wie zum Beispiel in J. Chem. Res. Synopses 2003, 5, 275-278 beschrieben.

In Arch. Pharm. 1987, 320, 11, 1167-1173 wird die Synthese von 4-Hydroxy-1,2,4-triazolin-5-onen der allgemeinen Formel (I-A-6a) beschrieben ($R^1$ = OH, $R^2$ = Alkyl).

Über die Herstellung von 2-Aryltriazolin-3-onen der allgemeinen Formel (I-A-8a) wird zum Beispiel in J. Amer. Chem. Soc. 1915, 37, 183-189 und J. Amer. Chem. Soc. 1917, 39, 950-961 ($R^1$=Alkyl, $R^2$=$SO_2CH_3$) berichtet.

Kondensation von Hydrazinen der allgemeinen Formel (Va) mit Dialkyl-*N*-carbomethoxyimidodithiocarbonaten ($R^1$ = H, $R^2$ = S-Alkyl: Z. Chem. 1980, 20, 10, 371-372) führt zu den entsprechenden 5-(Alkylsulfanyl)-2-aryl-2,4-dihydro-3H-1,2,4-triazol-3-onen der allgemeinen Formel (I-A-10a). Diese können nach literaturbekannten Methoden durch Oxidation in die entsprechenden 5-(Alkylsulfinyl)-2-aryl-2,4-dihydro-3H-1,2,4-triazol-3-onen der allgemeinen Formel (I-A-9a) überführt werden.

Die 2-Aryltriazolin-3-one der allgemeinen Formel (I-A-1) können alternativ nach **Verfahren Va2** hergestellt werden, wie im folgenden Schema dargestellt,

VERFAHREN Va2

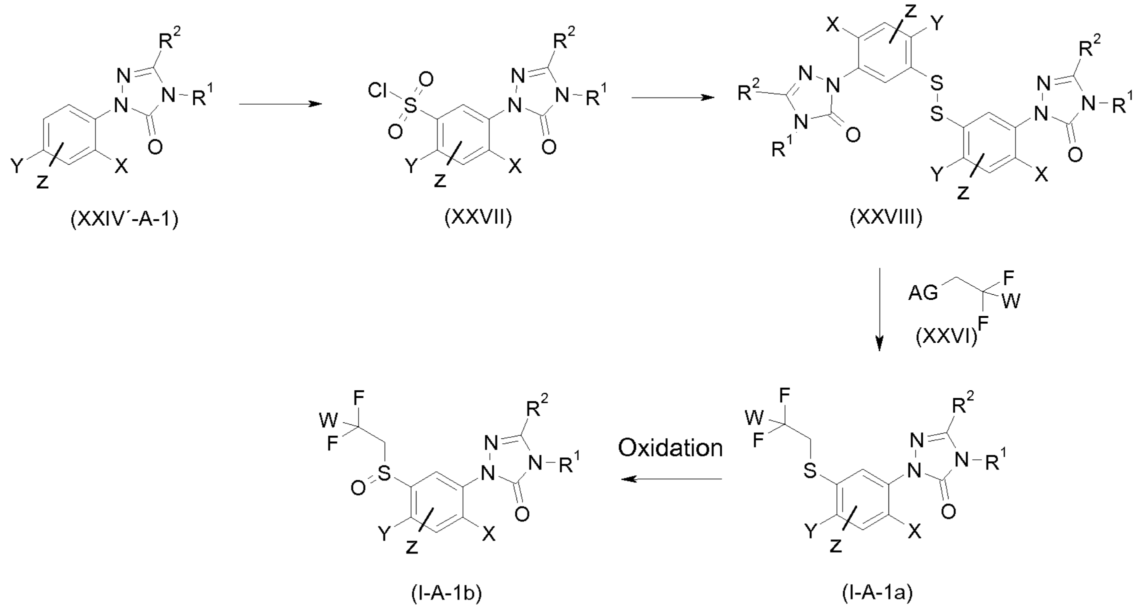


(XXIV´-A-1) (XXVII) (XXVIII)

(XXVI)

(I-A-1b) (I-A-1a)

wobei X, Y, Z, W, $R^1$ und $R^2$ die oben angegebene Bedeutung haben und AG steht für eine Abgangsgruppe wie zum

Beispiel Chlor, Brom, Iod, Tosylat, Mesylat oder Triflat.

In der Ausführungsform (I-A-1) der vorliegenden Erfindung erfolgt die Herstellung der Verbindungen der allgemeinen Formel (XXVII) durch Chlorsulfonierung der Aryltriazolinone (XXIV'-A-1) mit Chlorsulfonsäure, insbesondere nach den in den Herstellbeispielen genannten Bedingungen.

Die als Ausgangsverbindungen benötigten 2-Aryl-2,4-dihydro-3H-1,2,4-triazol-3-one (XXIV'-A-1) können nach Literaturmethoden hergestellt werden, beispielsweise analog EP 301946, EP 597360, WO 2002012203, WO 2009088025 oder J. Chem. Soc. (1954), 3319-3324 oder Chem. Ber. (1958), 91 2578-2580.

Die Reduktion der Sulfonylchloride (XXVII) in die Disulfide (XXVIII) ist mit literaturbekannten Methoden wie zum Beispiel Eisen in Salzsäure oder Iodid möglich, insbesondere nach den in den Herstellbeispielen genannten Bedingungen. Die Umsetzung der Disulfide (XXVIII) mit Halogenalkylelektrophilen der Formel (XXVI) liefert die Verbindungen der Formel (I-A-1a).

Die Thioether (I-A-1a) können in die entsprechenden Sulfoxide (I-A-1b) nach hinlänglich bekannten Verfahren durch Umsetzung mit Oxidationsmitteln überführt werden.

**Verfahren Va3 und Va4 (I-A-12)**

Die 2-Aryl-1,2,4-triazolin-3-thione der allgemeinen Formel (I-A-12), wo $R^2$ für eine Dimethylaminogruppe steht, können in (I-A-12a) (für n=0) und (I-A-12b) (für n=1) unterteilt und beispielsweise nach **Verfahren Va3** hergestellt werden, wie im folgenden Schema,

VERFAHREN Va3

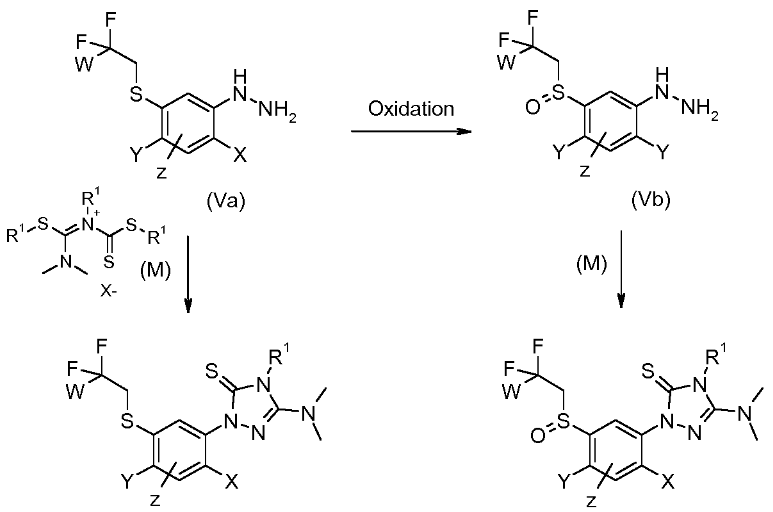

(Va) (Vb)

(I-A-12a) (I-A-12b)

wobei W, X, Y, Z, A und $R^1$ die oben angegebenen Bedeutungen haben.

Hydrazine der Formel (Va) bzw. (Vb) können zu den Triazolinthionen der Formel (I-A-12a) bzw. (I-A-12b) umgesetzt werden, zum Beispiel nach M. Yokoyama in J. Chem. Soc. (Perkin Trans. 1), 1982, 1059-1062, in dem sie mit Methylimmonium -Salzen der Formel (M) in Ethanol unter Hitzeeinwirkung versetzt werden. Diese Methylimmonium -Salze lassen sich, wie von M. Yokoyama beschrieben, aus Dialkylthioharnstoffen mit Schwefelkohlenstoff und einem Überschuss eines Alkylierungsmittels herstellen.

Die 2-Aryl-1,2,4-triazolin-3-thione der allgemeinen Formel (I-A-12), in denen $R^2$ für eine monosubstituierte Aminogruppe steht, können alternativ nach **Verfahren Va4** hergestellt werden, wie im folgenden Schema,

VERFAHREN Va4

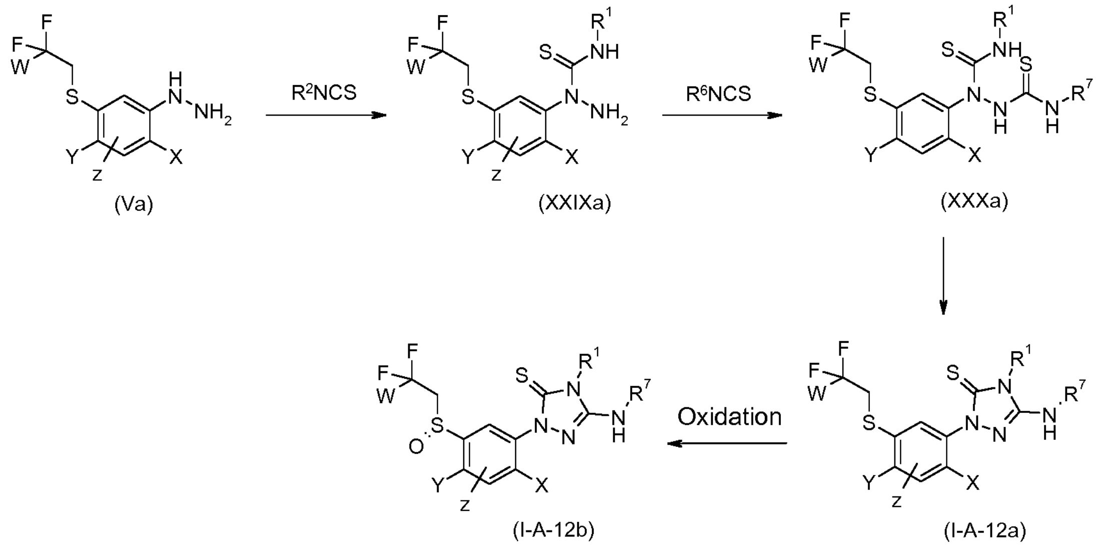


wobei W, X, Y, Z, A, $R^1$ und $R^7$ die oben angegebenen Bedeutungen haben .

Die Hydrazine der Formel (Va) können mit Isothiocyanaten zu Thiosemicarbaziden der Formel (XXIXa) umgesetzt werden, wie zum Beispiel nach M. Yokomoto in J. Med. Chem. 2012, 55, 7772-7785, in einem Alkohol als Lösungsmittel. Die Thiosemicarbazide (XXIXa) können mit geeigneten Isothiocyanaten zu Bisthioharnstoffen der Formel (XXXa) umgesetzt werden, beispielsweise wenn die Reaktion in Toluol oder Acetonitril nach K. Schulze in J. Het. Chem. 1995, 32, 275-281, durchgeführt wird. Anschließend kann die Cyclisierung zu den erwünschten Triazolin-3-thionen der Formel (Ia) erfolgen, zum Beispiel durch Reaktion mit 0,5 Äquivalenten Natriumethanolat im Ethanol, wie von K. Schulze beschrieben.

Die Thioether (I-A-12a) können anschließend in die entsprechenden Sulfoxide (I-A-12b) nach literaturbekannten Verfahren durch Umsetzung mit Oxidationsmitteln überführt werden.

**Verfahren Va5 (I-A-11)**

Die 2,4-Dihydro-3H-1,2,4-triazol-3-thione der allgemeinen Formel (I-A-11) können in (I-A-11a) (für n=0) und (I-A-11b) (für n=1) unterteilt und beispielsweise nach **Verfahren Va5** hergestellt werden, wie im folgenden Schema,

VERFAHREN Va5

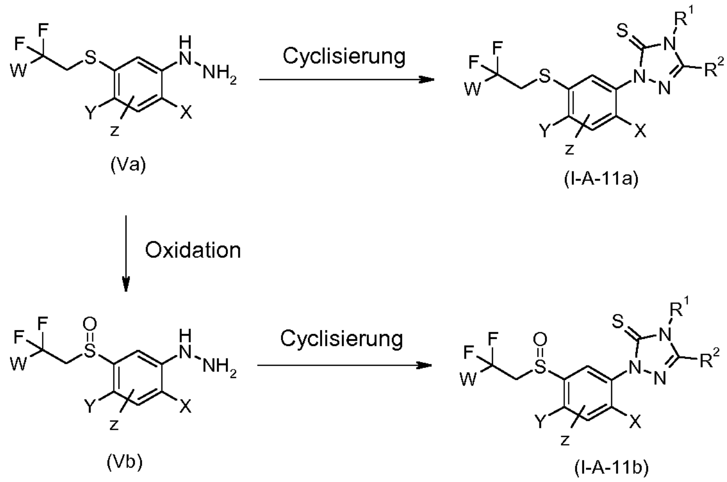


wobei X, Y, Z, W, $R^1$ und $R^2$ die oben angegebene Bedeutung haben.

2,4-Dihydro-3H-1,2,4-triazol-3-thione der allgemeinen Formel (I-A-11) können ausgehend von Hydrazinen der allgemeinen Formel (Va) in häufig mehrstufigen Prozessen hergestellt werden. Zahlreiche Vorschriften finden sich in der Literatur, zum Beispiel Kondensation mit Isothiocyanaten und anschließender Zyklisierung zum Beispiel mit Ameisensäure oder Orthoestern wie etwa in J. Heterocycl. Chem. 1998, 35, 29-32 ($R^1$=Hetaryl, $R^2$=H, Alkyl), mit Säurechloriden oder Säureanhydriden wie zum Beispiel in J. Chem. Soc. 1959, 3789-3794 ($R^1$=Alkyl, $R^2$=Alkyl) oder in Helv. Chim. Acta 1996, 79, 61-83 ($R^1$=Aryl, $R^2$=Haloalkyl) beschrieben.

Die Bildung von Thiosemicarbazonen aus Hydrazinen der allgemeinen Formel (Va) und anschließende oxidative Cyclisierung mit Eisen(III)-chlorid oder Kupferperchlorat wird in J. Heterocycl. Chem. 1999, 36, 667-674 ($R^1$=Aryl, $R^2$=Aryl) oder in J. Heterocycl. Chem. 1991, 28, 1421-1427 ($R^1$=Aryl, $R^2$=Alkyl) beschrieben.

Durch Reaktion von Thiosemicarbaziden, die aus den entsprechenden Hydrazinen (Va) zugängig sind, mit Ketonen und anschließender Kohlenwasserstoff-Eliminierung nach Tetrahedron Lett. 1989, 30, 2369-2370 ($R^1$=Alkyl, Alkenyl, $R^2$=Alkyl) ist die Synthese von 2,4-Dihydro-3H-1,2,4-triazol-3-thione ebenfalls möglich.

Verfahren Vb1 bis Vb4 eignen sich zur Darstellung der Ausführungsform I-B der Verbindungen der Formel (I).

**Verfahren Vb1 und Vb2 (I-B-1)**

Die Tetrazolinone der allgemeinen Formel (I-B-1) können in (I-B-1a) (für n=0) und (I-B-1b) (für n=1) unterteilt und beispielsweise nach **Verfahren Vb1** hergestellt werden, wie im folgenden Schema,

VERFAHREN Vb1

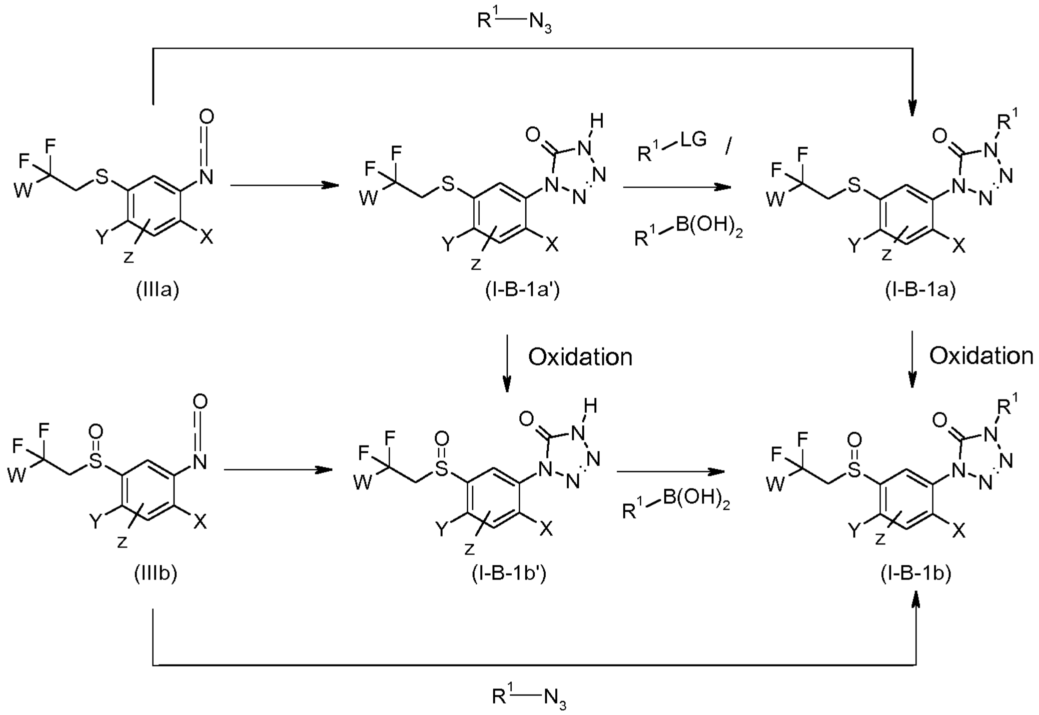


wobei X, Y, Z, W und $R^1$ die oben angegebene Bedeutung haben und LG steht für eine klassische Abgangsgruppe wie Iodid, Bromid, Chlorid, Tosylat, Mesylat, Methylsulfat oder Triflat.

Tetrazolinone der allgemeinen Formel (I-B-1a') bzw. (I-B-1b') können durch Cycloaddition von Isocyanaten der allgemeinen Formel (IIIa) bzw. (IIIb), die aus den entsprechenden Anilinen der allgemeinen Formel (IVa) darstellbar sind, z.B. nach JP 2011/042611, mit Aziden (wie zum Beispiel Trimethylsilylazid oder Natriumazid) gegebenenfalls in Anwesenheit von Lewis-Säuren (wie zum Beispiel Aluminiumchlorid) hergestellt werden. Literaturbeispiele sind unter anderen in J. Org. Chem. 2011, 76, 216-222, in US2011/130415 A1, in J. Org. Chem. 1980, 45, 25, 5130-5136, in J. Am. Chem. Soc. 1959, 81, 12, 3076-3079, in J. Heterocycl. Chem. 2007, 44, 4, 937-943 und in Tetrahedron 1975, 31, 7, 765-775 zu finden.

Die *N*-Alkylierung oder *N*-Arylierung der Tetrazolinone (I-B-1a') mit entsprechenden Alkylierungsmitteln oder Arylierungsmitteln führt zu *N*-alkylierten oder *N*-arylierten Tetrazolinonen der allgemeinen Formel (I-B-1a). Beispiele von *N*-Alkylierung sind in Bioorg. Med. Chem. Lett. 1999, 9, 1251-1254 beschrieben. Die *N*-Arylierung kann beispielsweise mit Arylboronsäuren, wie zum Beispiel nach Bioorg. Med. Chem. Lett. 2010, 20, 3920-3924, erfolgen oder alternativ durch Oxidation der entsprechenden *N*-cyclohexenylierten Tetrazolinone (I-B-1a) bzw. (I-B-1b) mit Oxidationmitteln, wie zum Beispiel Dichlor-dicyan-1,4-benzochinon (DDQ), wie in Chem. Ber. 1985, 118, 526-540 beschrieben. Diese Oxidation an Tetrazolinonen der Formel (I-B-1a) kann teilweise zu den erfindungsgemäßen Verbindungen der Formel (I-B-1b) führen.

Die Tetrazolinone der allgemeinen Formel (I-B-1b') können analog *N*-aryliert werden, zum Beispiel mit Arylboronsäuren nach Bioorg. Med. Chem. Lett. 2010, 20, 3920-3924.

Die Thioether (I-B-1a) und (I-B-1a') können anschließend in die entsprechenden Sulfoxide (I-B-1b) und (I-B-1b') nach bekannten Verfahren durch Umsetzung mit Oxidationsmitteln überführt werden.

Alternativ kann die Cycloaddition ausgehend von Isocyanaten der allgemeinen Formel (IIIa) bzw. (IIIb) direkt mit entsprechenden Alkyl- oder Arylaziden, die größtenteils kommerziell verfügbar sind, zu den entsprechenden *N*-funktionalisierten Tetrazolinonen (I-B-1a) bzw. (I-B-1b) stattfinden (zum Beispiel nach J. Fluor. Chem. 2008, 129, 11, 1073-1075 oder nach Tetrahedron Lett. 1999, 40, 37, 6739-6744).

Alternativ können die Tetrazolinone der allgemeinen Formel (I-B-1) nach **Verfahren Vb2** hergestellt werden, wie im folgenden Schema,

VERFAHREN Vb2

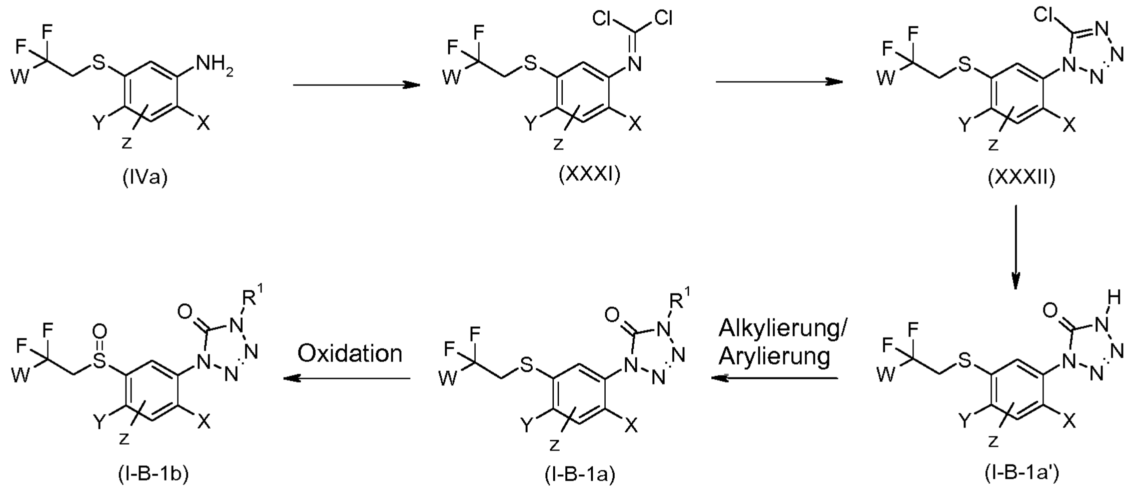

(IVa) (XXXI) (XXXII)
(I-B-1b) (I-B-1a) (I-B-1a')

wobei X, Y, Z, W und $R^1$ die oben angegebene Bedeutung haben.

Tetrazolinone der allgemeinen Formel (I-B-1a') lassen sich alternativ durch 1,3-dipolare Cycloaddition von Isocyaniddichloriden der Formel (XXXI) mit Natriumazid zum 5-Chlortetrazol (XXXII) und anschließender basischer Hydrolyse herstellen, wie zum Beispiel in J. Mol. Struct. 2009, 933, 38-45 beschrieben. Isocyaniddihalogenide lassen sich unter anderem durch Chlorierung aus Isocyanaten der allgemeinen Formel (IIIa, A=O), Isothiocyanaten der allgemeinen Formel (IIIa, A=S) oder Formamiden der allgemeinen Formel (Xa, PG=CHO) darstellen, wie zum Beispiel in Angew. Chem. 1967, 79, 663-680, in Synth. Commun. 1997, 27, 2645-2650 oder in J. Amer. Chem. Soc. 1922, 44, 2896-2903 beschrieben.

Die benötigten Syntheseintermediate (IIIa) (Isocyanate für V=O, Isothiocyanate für V=S) und Formamide (Xa, PG=CHO) sind literaturbekannt oder können aus den Anilinen der allgemeinen Formel (IVa) nach literaturbekannten Methoden hergestellt werden. Isocyanate und Isothiocyanate der allgemeinen Formel (IIIa) sind aus JP2011/042611 bekannt oder können nach literaturbekannten Methoden hergestellt werden, insbesondere nach den in den Herstellbeispielen genannten Bedingungen. Formamide der allgemeinen Formel (Xa) können nach literaturbekannten Methoden aus Anilinen hergestellt werden, zum Beispiel nach J. Het. Chem. 1968, 5, 165-177; J. Med. Chem. 2001, 44(12), 1972-1985; Tetrahedron 2002, 58, 2101-2116.

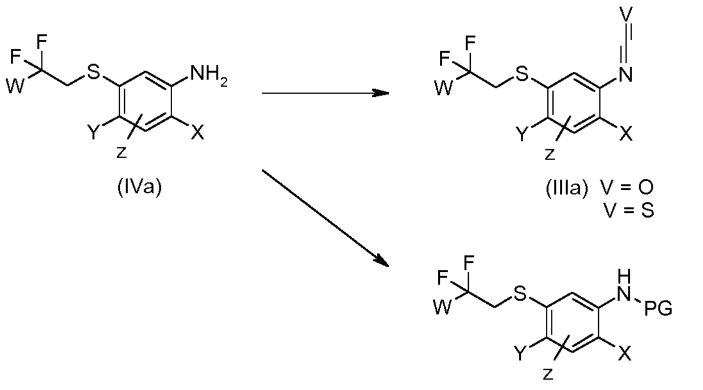

(IVa)

(IIIa) V = O
V = S

(Xa) PG = CHO

Tetrazolinone der allgemeinen Formel (I-B-1a') können anschließend durch *N*-Funktionalisierung wie in Verfahren Vb1 beschrieben zu den Tetrazolinonen der allgemeinen Formel (I-B-1a) führen.

Durch Oxidation der Thioether der allgemeinen Formel (I-B-1a) nach literaturbekannten Methoden können die Sulfoxide der allgemeinen Formel (I-B-1b) erhalten werden.

**<u>Verfahren Vb3 und Vb4</u> (I-B-2)**

Die Tetrazolinthione der allgemeinen Formel (I-B-2) können in (I-B-2a) (für n=0) und (I-B-2b) (für n=1) unterteilt und beispielsweise nach **Verfahren Vb3** hergestellt werden, wie im folgenden Schema,

VERFAHREN Vb3

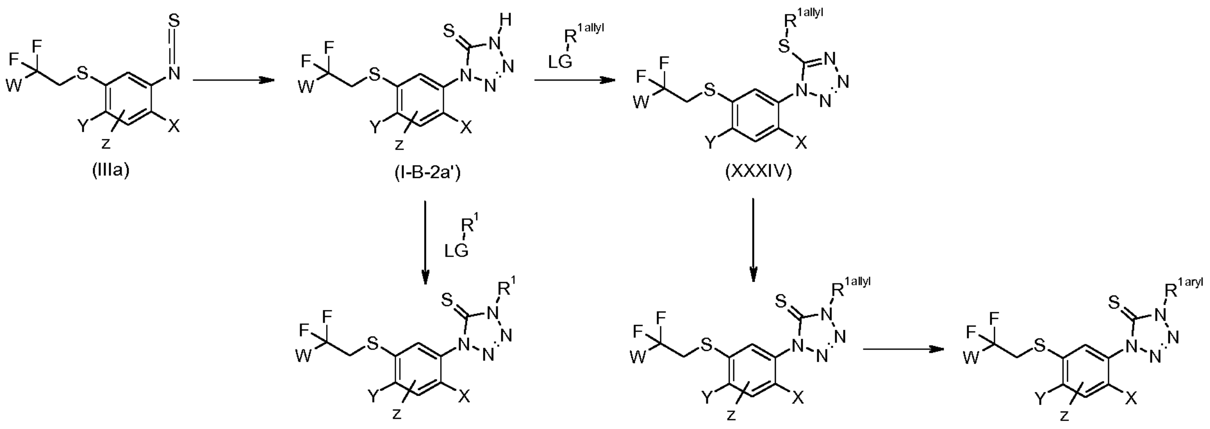


(IIIa) (I-B-2a') (XXXIV)

(I-B-2a) (I-B-2a) (I-B-2a)

wobei X, Y, Z, W und $R^1$ die oben angegebene Bedeutung haben, $R^{1allyl}$ steht spezifisch für $R^1$ Reste allylischer Natur und $R^{1aryl}$ steht spezifisch für $R^1$ Reste arylischer Natur, nach den obenen genannten Restedefinitionen.

Am Stickstoff unsubstituierte Tetrazolinthione der allgemeinen Formel (I-B-2a') können nach Verfahren Vb3 durch Cycloaddition von Isothiocyanaten der allgemeinen Formel (IIIa) mit Aziden, wie zum Beispiel Natriumazid, hergestellt werden. Die Cycloaddition kann unter neutralen oder basischen Reaktionsbedingungen durchgeführt werden, wie zum Beispiel in Can. J. Chem. 1957, 37, 101-105 oder in Bull. Kor. Chem. Soc. 2012, 33, 55-59 beschrieben. Alternativ sind Azid-Derivate von Nickel-, Antimon-, Germanium- oder Arsen-Verbindungen einsetzbar (zum Beispiel Inorg. Chem. 1987, 26, 2969-2974; J. Fluor. Chem. 2003, 122, 2, 165-170; Synthesis and Reactivity in Inorganic and Metal-Organic Chemistry 1999, 29, 1579-1592; Synthesis and Reactivity in Inorganic and Metal-Organic Chemistry 1984, 14, 477-484). Anschließende *N*-Alkylierung zu Tetrazolinthionen der allgemeinen Formel (I-B-2a) ist nach literaturbekannten Methoden möglich, zum Beispiel mit α,β-ungesättigten Aldehyden in Aza-Michael-Reaktionen zu *N*-(Carbonylalkyl)-Tetrazolinthionen (Org. Lett. 2011, 13, 336-339), mit Acrylnitrilen in Michael-Additionen zu *N*-(Cyanoalkyl)-Tetrazolinthionen (Russ. J. Appl. Chem. 1996, 69, 1841-1848), mit Aldehyden/Aminen in Mannich-Reaktionen zu *N*-(Aminoalkyl)-Tetrazolinthionen (Heteroatom Chem. 2007, 18, 637-643), mit Formaldehyd zu *N*-(Hydroxyalkyl)-Tetrazolinthionen (Monatsh. Chem. 1995, 126, 1035-1044) oder durch Alkylierung unter themodynamischen Bedingungen mit Halomethyluracilen (J. Org. Chem. 1985, 50, 980-987). Auch S-Alkylierung mit allylischen $R^{1allyl}$ zu

Verbindungen der Formel (XXXIV) mit anschließender Claisen-Umlagerung ist in Chem. Ber. 1985, 118, 526-540 zur Synthese von Tetrazolinthione der allgemeinen Formel (I-B-2a) beschrieben, wobei LG für eine klassische Abgangsgruppen wie Iodid, Bromid, Chlorid, Tosylat, Mesylat, Methylsulfat oder Triflat steht. Sofern die Alkylierung mit $R^{1allyl}$=Cyclohex-2-en-1-yl durchgeführt wird, ist eine anschließende Oxidation mit Oxidationsmitteln wie zum Beispiel Dichlordicyan-1,4-benzochinon (DDQ) zum *N*-Aryl-substituierten Tetrazolinthione der allgemeinen Formel (I-B-2a) möglich.

Tetrazolinthione der allgemeinen Formel (I-B-2a) bzw. (I-B-2b) lassen sich alternativ nach **Verfahren Vb4** durch Schwefelung der Tetrazolinone der allgemeinen Formel (I-B-1a) bzw. (I-B-1b), beispielsweise mit Phosphor(V)-sulfid oder Lawessons Reagenz, herstellen, wie zum Beispiel in J. Mol. Struct. 2009, 933, 38-45 oder in Bioorg. Med. Chem. Lett. 2010, 20, 3920-3924 beschrieben, und in folgendem Schema dargestellt,

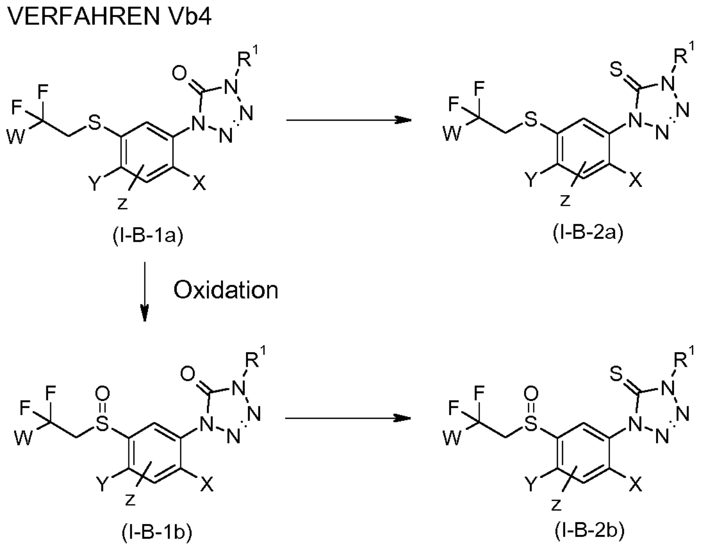


wobei X, Y, Z, $R^1$ und W die oben angegebene Bedeutung haben.

Verfahren Vc1 eignet sich zur Darstellung der Ausführungsform I-C der Verbindungen der Formel (I).

**Verfahren Vc1 (I-C-1)**

Die 1,3,4-Oxadiazol-2(3H)-one der allgemeinen Formel (I-C-1) können in (I-C-1a) (für n=0) und (I-C-1b) (für n=1) unterteilt und beispielsweise nach **Verfahren Vc1** hergestellt werden, wie im folgenden Schema

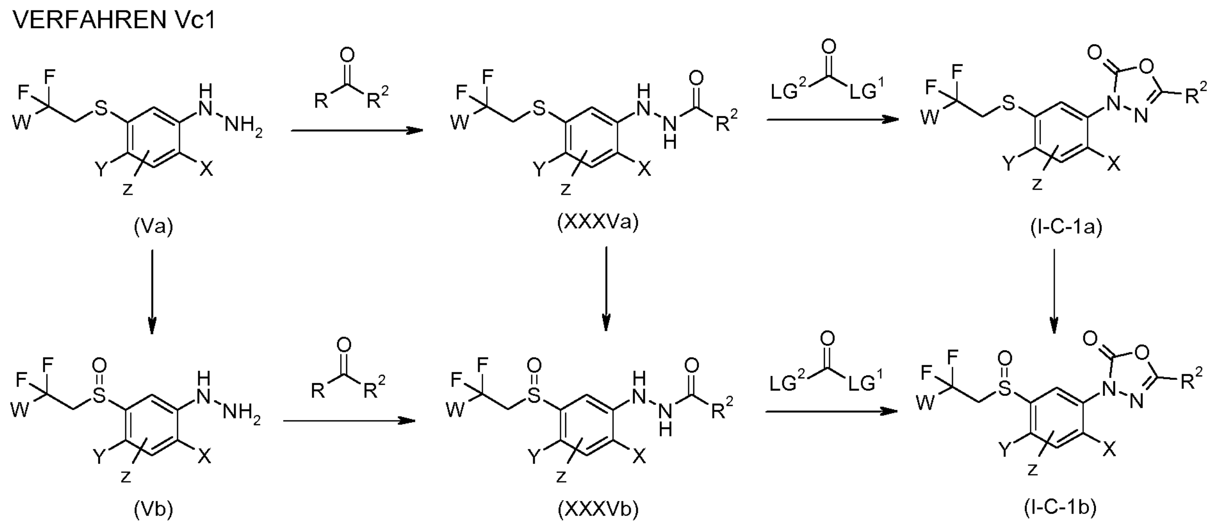


wobei X, Y, Z, W und $R^2$ die oben angegebene Bedeutung haben, R für Halogen (insbesondere Chlor) oder Alkylcarboxyl (insbesondere Isopropylcarboxyl) und $LG^1$ und $LG^2$ unabhängig voneinander stehen für Abgangsgruppen wie Chlorid, Alkoxid, Phenoxid, Imidazol, Trichlormethyl oder 1-Hydroxypyrrolidin-2,5-dion.

1,3,4-Oxadiazol-2(3H)-one der allgemeinen Formel (I-C-1a) können durch Acylierung von Hydrazinen der allgemeinen Formel (Va) mit Säurehalogeniden oder gemischten Anhydriden (die aus Chlorameisensäureestern -wie z.B. Chlorameisensäureisopropylester- und Säuren darstellbar sind) zu den entsprechenden Hydraziden (XXXVa) und anschließender Cyclisierung mit C1-Bausteinen wie zum Beispiel 1,1'-Carbonyldiimidazol, Phosgen, Diphosgen, Triphosgen, Diphenylcarbonat oder Chlorameisensäure-*N*-hydroxydicarboximidestern, hergestellt werden. Literaturbeispiele finden sich zum Beispiel in J. Med. Chem. 2007, 50, 528-542, in J. Heterocycl. Chem. 1982, 19, 823-828, Bioorg. Med. Chem. 2001, 9, 1307-1323, in J. Agric. Food Chem. 2010, 58, 2643-2651 oder in Pharmazie 1990, 45, 138-139.

Durch Oxidation der Thioether der allgemeinen Formel (I-C-1a) nach literaturbekannten Methoden können die Sulfoxide der allgemeinen Formel (I-C-1b) erhalten werden. Alternativ können die in Verfahren Vc1 beschriebenen Reaktionen auch ausgehend von Sulfoxiden der allgemeinen Formel (Vb) direkt zu Sulfoxiden der allgemeinen Formel (I-C-1b) erfolgen.

Verfahren Vd1 bis Vd4 eignen sich zur Darstellung der Ausführungsform I-D der Verbindungen der Formel (I).

## Verfahren Vd1, Vd2 und Vd3 (I-D-1)

4-Alkoxy-1,5-dihydro-2H-pyrrol-2-one der allgemeinen Formel (I-D-1) können in (I-D-1a) (für n=0) und (I-D-1b) (für n=1) unterteilt und beispielsweise nach Verfahren Vd1 hergestellt werden, wie im folgenden Schema

VERFAHREN Vd1

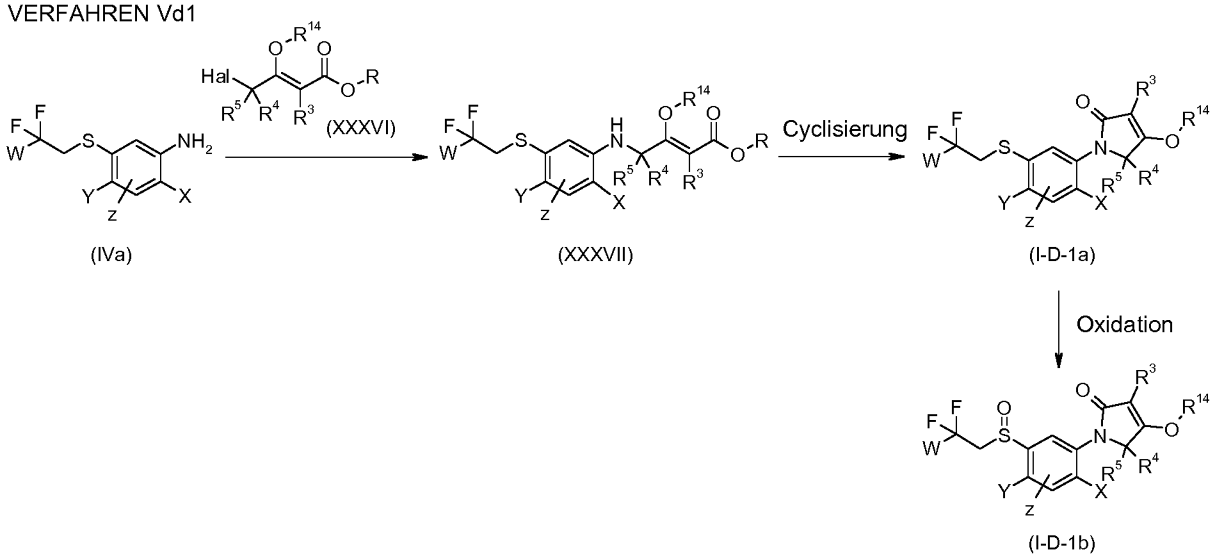


wobei X, Y, Z, W, $R^3$, $R^4$, $R^5$ und $R^{14}$ die oben angegebene Bedeutung haben, Hal steht für Halogen (insbesondere Chlor, Brom, Iod) und R steht für ggf. substituiertes Alkyl (insbesondere Methyl, Ethyl).

Durch die Alkylierung von Anilinen der allgemeinen Formel (IVa) mit 4-Halo-3-alkoxyalkenylsäureestern der Formel (XXXVI) (zum Teil kommerziell erhältlich oder nach literaturbekannten Methoden darstellbar, siehe Synthesis 1992, 391-394) können Verbindungen der Formel (XXXVII) hergestellt werden. Die säurekatalysierte Cyclisierung aus den alkylierten Anilinen (XXXVII) führt zu den erfindungsgemäßen Pyrrolinonen der allgemeinen Formel (I-D-1a), wie zum Beispiel in J. Med. Chem. 2006, 49, 1855-1866, in Tetrahedron Lett. 1984, 25, 1871-1874 oder in Jordan J. Chem. 2010, 5, 13-21 für $R^3 = R^4 = R^5$ = Wasserstoff beschrieben.

Durch Oxidation der Thioether der allgemeinen Formel (I-C-1a) nach literaturbekannten Methoden können die Sulfoxide der allgemeinen Formel (I-C-1b) erhalten werden.

Alternativ können 4-Alkoxy-1,5-dihydro-2H-pyrrol-2-one der allgemeinen Formel (I-D-1a), in denen $R^4$ und $R^5$ für Wasserstoff stehen, nach **Verfahren Vd2** hergestellt werden, nach folgendem Schema

VERFAHREN Vd2

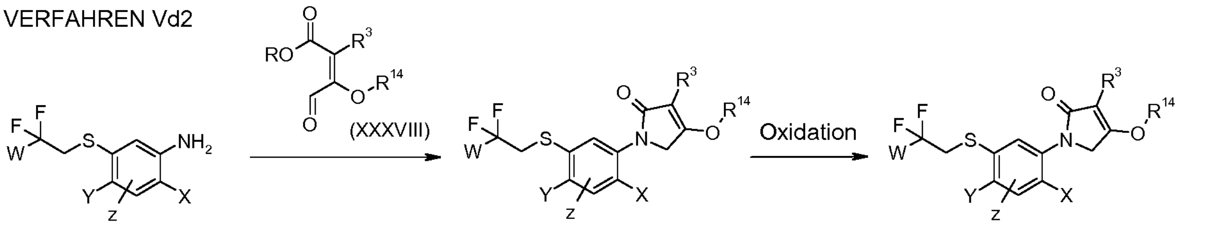


(IVa) (I-D-1a) (I-D-1b)

wobei X, Y, Z, W, $R^3$ und $R^{14}$ die oben angegebene Bedeutung haben und R für ggf. substituiertes Alkyl (insbesondere Methyl, Ethyl) steht.

Durch Umsetzung von Anilinen der allgemeinen Formel (IVa) mit Alkyl-3-(Alkyloxy)-4-oxobut-2-enoaten der Formel (XXXVIII) in einer Reaktionsfolge aus reduktiver Aminierung und Cyclisierung können die erfindungsgemäßen Verbindungen der Formel (I-D-1b) hergestellt werden, wie beispielsweise in Synthesis 2002, 869-874 beschrieben.

Funktionalisierungen der 4-Alkoxy-Position in Verbindungen der allgemeinen Formel (I-D-1a'), wo $R^{14}$ für Methyl steht, können nach **Verfahren Vd3** durchgeführt werden, wie im folgenden Schema,

VERFAHREN Vd3

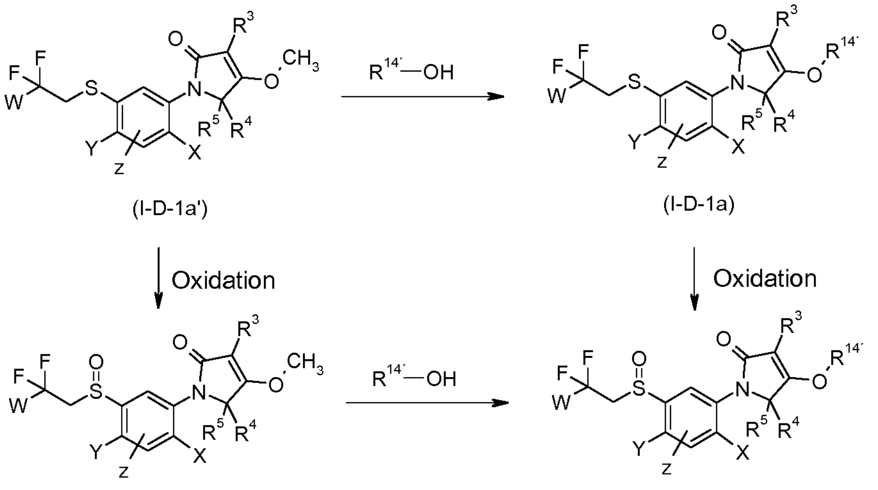


(I-D-1a') (I-D-1a) (I-D-1b') (I-D-1b)

wobei X, Y, Z, W, $R^3$, $R^4$ und $R^5$ die oben angegebene Bedeutung haben und $R^{14'}$ steht für die oben angegebenen Restedefinitionen für $R^{14}$ außer Methyl.

Die Einführung von unterschiedlichen Resten $R^{14'}$ kann zum Beispiel durch Alkoholyse mit Alkoholen $R^{14'}$ OH unter Säurekatalyse und unter Anwesenheit von Molekularsieb ausgehend von Methylestern der allgemeinen Formel (I-D-1a') erfolgen, wie zum Beispiel in J. Org. Chem. 2008, 73, 2345-2356 für $R^3$ = Wasserstoff beschrieben ist.

Durch Oxidation der Thioether der allgemeinen Formel (I-D-1a) nach literaturbekannten Methoden können die Sulfoxide der allgemeinen Formel (I-D-1b) erhalten werden. Alternativ können die in Verfahren Vd3 beschriebenen Reaktionen auch ausgehend von Sulfoxiden der allgemeinen Formel (I-D-1b') direkt zu Sulfoxiden der allgemeinen Formel (I-D-1b) erfolgen.

**Verfahren Vd4 (I-D-2)**

Die 1-Aryl-1,5-dihydro-2H-pyrrol-2-one der Formel (I-D-2b), wo $R^4$ und $R^5$ für Wasserstoff stehen, können beispielsweise nach **Verfahren Vd4** hergestellt werden, wie im folgenden Schema gezeigt,

VERFAHREN Vd4

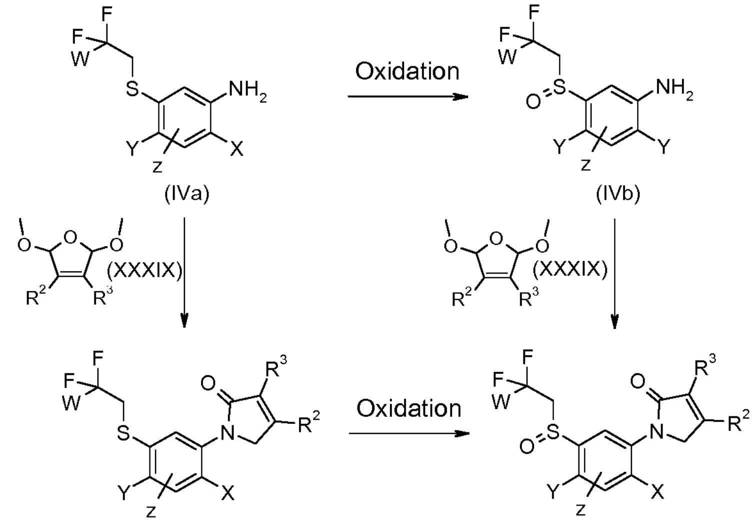


(IVa) (IVb) (I-D-2a) (I-D-2b)

wobei X, Y, Z, W, $R^2$ und $R^3$ die oben angegebene Bedeutung haben.

Aniline der Formel (IVa) bzw. (IVa) können entsprechend der Literaturvorschrift aus J. Med. Chem. 2006, 49, 6015-6026, durch die Umsetzung mit 2,5-Dimethoxy-2,5-dihydrofuranen der Formel (XXXIX) zu den Sulfiden bzw. Sulfoxiden der Formel (I-D-2a) bzw. (I-D-2b) umgesetzt werden.

Die Thioether (I-D-2a) können nach bekannten Verfahren in die entsprechenden Sulfoxide (I-D-2b) überführt werden.

Als Cyclisierungsreagenzien eignen sich auch 2,4-Dibrombuttersäurebromid oder 2,4-Dichlorbuttersäurechlorid (analog JP 46037339).

1-Aryl-1,5-dihydro-2H-pyrrol-2-one der Formel (I-D-2a) bzw. (I-D-2b) mit komplexerem Substituentenmuster im 5-Ring sind nach zahlreichen weiteren Verfahren zugänglich.

Eine Methode zur Herstellung von 4-Aryl-substituierten 1-Aryl-1,5-dihydro-2H-pyrrol-2-onen der Formel (I-D-2a) bzw. (I-D-2b) (mit $R^2$ = Aryl) besteht in der Umsetzung von Anilinen der Formel (IVa) bzw. (IVb) mit Estern der allgemeinen Struktur (XL-Ar) (beispielsweise beschrieben in JP 6145142).

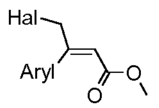

(XL-Ar)

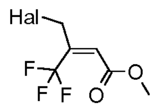

($XL-CF_3$)

Ein Verfahren zur Herstellung von 4-Aryl-substituierten 1,5-Dihydro-2H-pyrrol-2-onen der Formel (I-D-2a) bzw. (I-D-2b) (mit $R^2$ = Aryl) durch die Umsetzung von Anilinen der Formel (IVa) bzw. (IVb) mit Estern der allgemeinen Struktur (XL-Ar) ist beschrieben in J. Med. Chem. 2006, 49, 1855-1866 oder in Bioscience, Biotechnology, and Biochemistry 1992, 56(7), 1164-1154.

Durch die analogen Umsetzungen der Aniline mit den Trifluormethylderivaten ($XL-CF_3$) erhält man Gemische der 1-Aryl-1,5-dihydro-2H-pyrrol-2-one der Formel (I-D-2a) bzw. (I-D-2b) (mit $R^2$ = $CF_3$) und den entsprechenden Doppelbindungsisomeren 1-Aryl-1,3-dihydro-2H-pyrrol-2-onen (z. B. Chem. Pharm. Bull. 1985, 33(9), 4026-4029).

Verfahren Ve1 eignet sich zur Darstellung der Ausführungsform I-E der Verbindungen der Formel (I).

<u>Verfahren Ve1</u> **(I-E-1)**

Die 1-Aryl-1,3-dihydro-2H-imidazol-2-one der Formel (I-E-1) können in (I-E-1a) (für n=0) und (I-E-1b) (für n=1) unterteilt und beispielsweise nach **Verfahren Ve1** hergestellt werden, wie im folgenden Schema

VERFAHREN Ve1

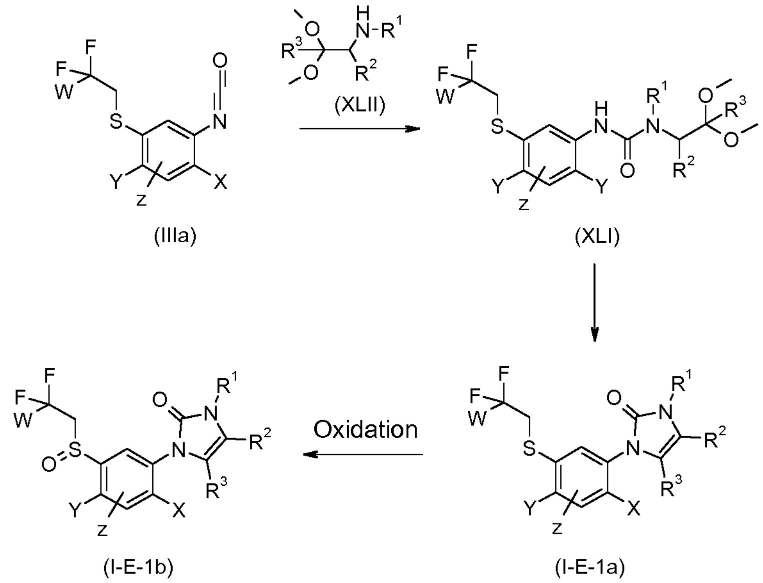


(IIIa) (XLI) (I-E-1b) (I-E-1a)

wobei X, Y, Z, W, $R^1$, $R^2$ und $R^3$ die oben angegebene Bedeutung haben.

Isocyanate der Formel (IIIa) können durch die Umsetzung mit 2,2-Dialkoxyethylaminen der Formel (XLII) zu den Harnstoffen der Formel (XLI) umgesetzt werden. Die anschließende saure Hydrolyse liefert die 1-Aryl-1,3-dihydro-2H-imidazol-2-one der Formel (I-E-1a). Es ist möglich, die Sulfide der Formel (I-E-1a) mit $R^1$ = H nachträglich durch geeignete Alkylierungsreagenzien der allgemeinen Formel $R^2$-AG (wo AG für eine Abgangsgruppe wie zum Beispiel Chlor, Brom, Tosylat, Mesylat oder Triflat steht) nach hinreichend bekannten Verfahren zu alkylieren. Diese Synthesen sind z.B. beschrieben in Can. J. Chem. 2004, 82, 1649-1661. Weitere Synthesevarianten sind beschrieben in US 3133079 oder WO 2004/011438.

Durch die Umsetzung von entsprechenden Carbamaten gelingt ebenfalls die Synthese von 1-Aryl-1,3-dihydro-2H-imidazol-2-onen, in Analogie zu US 4279637. Das im J. Med. Chem. 1966, 9 (6),858-860 beschrieben Verfahren verwendet als Ausgangsverbindungen die entsprechenden Aniline der Formel (IVa).

Die Thioether (I-E-1a) können in die entsprechenden Sulfoxide (I-E-1b) nach hinlänglich bekannten Verfahren durch Umsetzung mit Oxidationsmitteln umgesetzt werden.

Verfahren Vfl eignet sich zur Darstellung der Ausführungsform I-F der Verbindungen der Formel (I).

**Verfahren Vf1 (I-F-1)**

1-Aryl-1H-pyrrol-2,5-dione der allgemeinen Formel (I-F-1) können in (I-F-1a) (für n=0) und (I-F-1b) (für n=1) unterteilt und beispielsweise nach **Verfahren Vf1** hergestellt werden, wie im folgenden Schema,

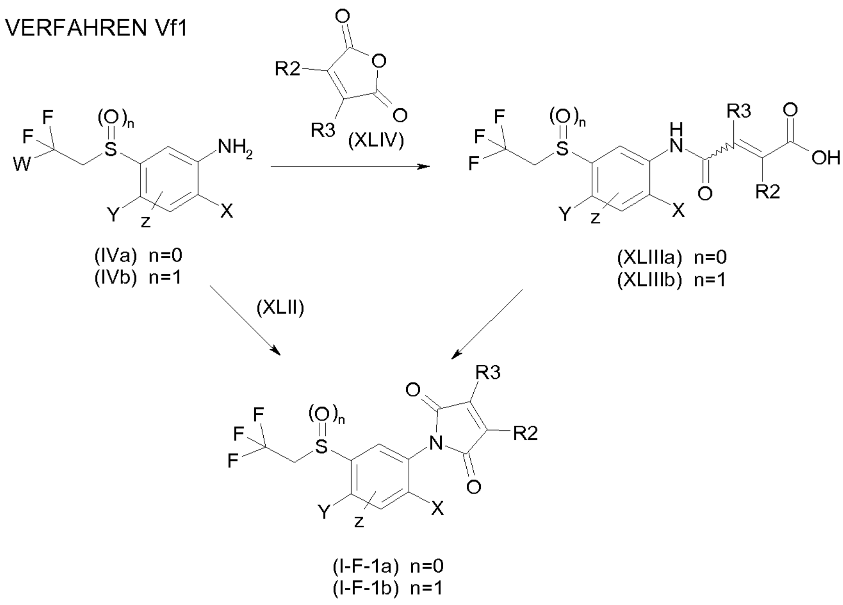


wobei X, Y, Z, W, $R^2$ und $R^3$ die oben angegebene Bedeutung haben.

Die Herstellung der Verbindungen der allgemeinen Formel (I-F-1a) bzwl. (I-F-1b) kann dadurch erfolgen, dass man Aniline der Formel (IV) mit Furan-2,5-dionen der Formel (XLIV) in die entsprechenden 4-Aryl-4-oxobut-2-ensäuren (XLIIIa) bzw. (XLIIIb) überführt und diese anschliessend in Gegenwart eines Kondensationsmittels in die erfindungsgemäßen Verbindungen der Formel (I-F-1a) bzw. (I-F-1b) cyclisiert, z.B. gemäß Angewandte Makromolekulare Chemie 1988, 157, 59-78 oder US 3853912.

Als Kondensationsmittel sind vor allem wasserentziehende Chemikalien geeignet. Hierzu gehören vorzugsweise Säureanhydride und Säurehalogenide, wie beispielsweise Acetanhydrid, Propionsäureanhydrid, Phosphor-(V)-oxid, Phosphorylchlorid, Phosphorylbromid, Phosphortrichlorid, Phosphortribromid, Thionylchlorid, Oxalylchlorid, Phosgen, Diphosgen, Ameisensäuremethylester, Ameisensäureethylester, sowie Carbodiimide, wie beispielsweise N,N'-Dicyclohexylcarbodiimid (DCC) oder 1-Ethyl-3-(3-dimethylaminopropyl)carbodiimid Hydrochlorid (EDC-HCl). Weitere bekannte Kondensationsreagenzien sind Triphenylphosphin/Tetrachlormethan, 4-(4,6-Dimethoxy[1.3.5]triazin-2-yl)-4-methyl-morpholiniumchlorid-Hydrat oder Hydroxybenzotriazol (HOBt). Insbesondere die Kombination von 1-Ethyl-3-(3-dimethylaminopropyl)carbodiimid Hydrochlorid (EDC-HCl) und Hydroxybenzotriazol (HOBt) sei hier aufgeführt.

Alternativ - wie in der EP 260228 beschrieben - lassen sich die 1-Aryl-1H-pyrrol-2,5-dione der Formel (I-F-1a) bzw. (I-F-1b) auch direkt durch die Umsetzung von Anilinen der Formel (IVa) bzw. (IVb) mit Furan-2,5-dionen der Formel (XLII) in Gegenwart von Alkylcarbonsäuren, beispielsweise Essigsäure, darstellen. Die Verbindungen der allgemeinen Formel (XLIV) sind kommerziell erhältlich, literaturbekannt oder können nach literaturbekannten Methoden hergestellt werden.

Die Thioether (XLIIIa) oder (I-F-IIIa) können in die entsprechenden Sulfoxide (XLIb) oder (I-F-1b) nach hinlänglich bekannten Verfahren durch Umsetzung mit Oxidationsmitteln umgesetzt werden.

Die erfindungsgemäßen Verbindungen der allgemeinen Formel (XLI) und (I-F-1) werden gegebenenfalls unter Einsatz eines oder mehrerer Verdünnungsmittel hergestellt. Als Verdünnungsmittel kommen vor allem organische Lösungsmittel in Betracht, wie beispielsweise Alkylalkohole, Dialkylether, Toluol, Xylol, Chlorbenzol, Dichlorbenzol, Petrolether, Hexan, Cyclohexan, Dichlormethan, Chloroform, Tetrachlormethan, Tetrahydrofuran, Dioxan, Acetonitril, Dimethylformamid, Ameisensäure, Essigsäure und Propionsäure.

Die Reaktionstemperaturen können bei der Durchführung des Verfahrens I-F in einem größeren Bereich variiert werden. Im Allgemeinen arbeitet man bei Temperaturen zwischen 0 °C und 150 °C, vorzugsweise zwischen 10 °C und 120 °C.

Verfahren Vg1 eignet sich zur Darstellung der Ausführungsform I-G der Verbindungen der Formel (I).

**Verfahren Vg1 (I-G-1)**

Die 2-Alkylimino-3-aryl-1,3-oxazole (V=O) und 2-Alkylimino-3-aryl-1,3-thiazole (V=S) der allgemeinen Formel (I) können in Verbindungen der Formel (I-G-1a) (für n=0) und (I-G-1b) (für n=1) unterteilt und beispielsweise nach **Verfahren Vg1** hergestellt werden, wie im folgenden Schema,

VERFAHREN Vg1

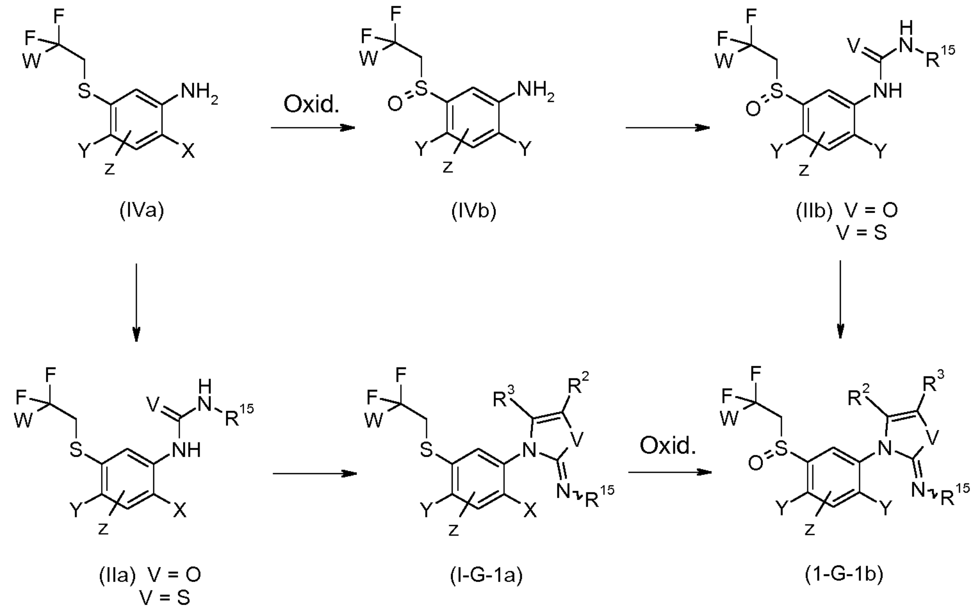


(IVa) (IVb) (IIb) V = O V = S

(IIa) V = O V = S (I-G-1a) (1-G-1b)

wobei W, X, Y, Z, $R^2$, $R^3$ und $R^{15}$ die oben angegebenen Bedeutungen haben und V steht für Sauerstoff oder Schwefel.

Aniline der Formel (IVa) bzw. (IVb) können zu den Harnstoffen (V=O) und Thioharnstoffen (S) der Formel (IIa) bzw. (IIb) nach literaturbekannten Methoden umgesetzt werden, zum Beispiel nach JP 2011/042611, indem sie mit Isocyanaten (für V=O) und Isothiocyanaten (für V=S) gegebenenfalls in Anwesenheit einer Base und gegebenfalls in Anwesenheit eines organischen Lösungsmittels versetzt werden oder in dem sie nach allgemein bekannten Methoden in deren Isocyanaten (V=O) und Isothiocyanaten (V=S) umgewandelt und diese mit Aminen zu den Harnstoffen oder Thioharnstoffen umgesetzt werden.

Aus den Harnstoffen (V=O) und Thioharnstoffen (V=S) der allgemeinen Formel (IIa) bzw. (IIb) kann die Synthese der 2-Alkylimino-3-aryl-1,3-oxazole (V=O) und -thiazole (V=S) der allgemeinen Formel (I-G-1a) bzw. (I-G-1b) erfolgen, zum Beispiel durch Cycloacylierung mit einem adäquaten Halocarbonylderivaten in einem inerten Lösungsmittel, meistens bei Temperaturen höher als 100 °C. Geeignete Halocarbonylderivaten sind zum Beispiel 2-Chlor-1,1-diethoxyethan für $R^2$ =$R^3$ = H nach dem Patent US 4079144, 3-Brom-2-butanon für $R^2$ = $R^3$ = Methyl.

Bei der Durchführung der erfindungsgemäßen Verfahren kann gegebenenfalls jede für diese Reaktionen geeignete, handelsübliche Mikrowellenapparatur verwendet werden (z.B. Anton Paar Monowave 300, CEM Discover S, Biotage Initiator 60).

Thionierung:

Ein weiteres allgemeines Verfahren zur Herstellung der erfindungsgemäßen Verbindungen der allgemeinen Formel (Ia) oder (Ib), in denen V für Schwefel steht, besteht in der Umwandlung der Carbonylgruppe entsprechender Vorstufen in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Pyridin, Xylol oder Cumol. Diese Variante ist in zahlreichen Publikationen beschrieben, z.B. in J. Amer. Chem. Soc. 1956, 1938-1941, Chem. Pharm. Bull. 1962, 10, 647-652, US 3007927, DE 2554866 oder WO 2000026194.

Oxidation:

Verbindungen der allgemeinen Formel (Ib) lassen sich durch Oxidation nach literaturbekannten Verfahren aus Verbindungen der allgemeinen Formel (Ia) herstellen, beispielsweise durch ein Oxidationsmittel in einem geeignetem Lösungs- und Verdünnungsmittel. Als Oxidationsmittel eignen sich zum Beispiel verdünnte Salpetersäure, Wasserstoffperoxid und Peroxycarbonsäuren, wie etwa *meta*-Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum

Beispiel mit Titanium und Vanadium als meistbenutzten Katalysatorquellen, in Form von Ti(O$^i$Pr$_4$) und VO(acac)$_2$, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nichtmetall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC-Säule getrennt werden.

Alternativ können Verbindungen der allgemeinen Formel (Ib) durch ähnliche wie die hier genannten Methoden in einer anderen Reihenfolge hergestellt werden.

<u>**Erläuterung der Ausgangsstoffe und Zwischenprodukte**</u>

***Aniline der allgemeinen Formel (IVa) und (IVb)***

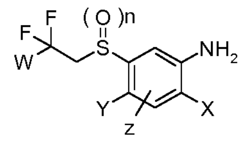

(IVa) n=0
(IVb) n=1

Aniline der Formel (IVa) sind teilweise literaturbekannt, z.B. aus JP 2007/284356, oder können anhand literaturbekannter Verfahren synthetisiert werden, insbesondere nach den in den Herstellbeispielen genannten Bedingungen.

Die Verbindungen der Formel (IVb) sind neu und lassen sich durch Oxidation, insbesondere nach den in den Herstellbeispielen genannten Bedingungen, herstellen.

Die Aniline der allgemeinen Formel (IVa) können vorzugsweise wie im folgenden Schema hergestellt werden,

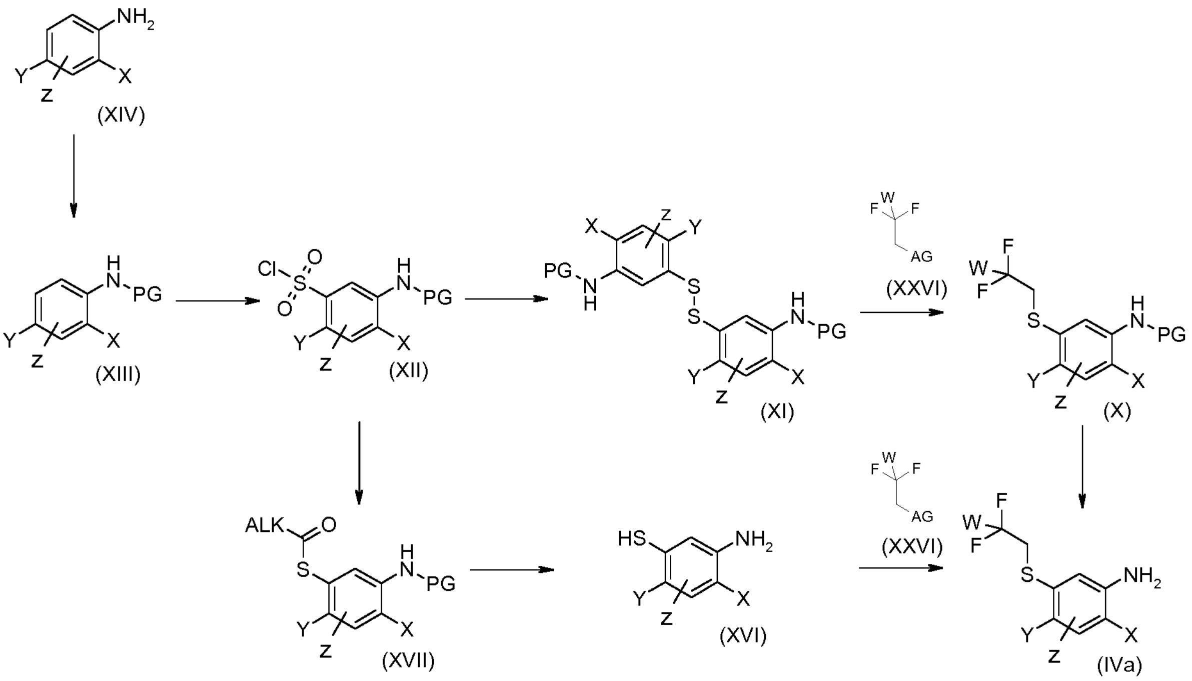

(XIV) (XIII) (XII) (XI) (XXVI) (X) (XVII) (XVI) (XXVI) (IVa)

,

wobei X, Y, Z und W die oben angegebenen Bedeutungen haben, AG für eine Abgangsgruppe und PG für eine Schutzgruppe steht.

Aniline der Formel (XIV) sind entweder kommerziell erhältlich oder können durch bekannte Methoden hergestellt werden. Sie können mit einer geeigneten Schutzgruppe, wie z.B. einer Acetylgruppe, zu Verbindungen der Formel (XIII) geschützt werden. Beispielsweise in Gegenwart von Säuren, Säureanhydriden oder Säurechloriden können die Aniline

(XIV) in die entsprechenden Anilide (XIII) überführt werden. Die Chlorsulfonierung der geschützen Aniline (XIII) mit Chlorsulfonsäure liefert die entsprechenden Sulfonylchloride (XII). Die Reduktion der Sulfonylchloride (XII) in die Disulfide (XI) ist mit literaturbekannten Methoden wie zum Beispiel Eisen in Salzsäure oder Iodid möglich. Die Umsetzung der Disulfide (XI) mit Haloalkylelektrophilen der Formel (XXVI), wobei AG für eine Abgangsgruppe wie zum Beispiel Chlor, Brom, Tosylat, Mesylat oder Triflat steht, liefert die Sulfide (X). Die Schutzgruppe kann durch geeignete literaturbekannte Methoden abgespalten werden, so dass man Aniline der Formel (IVa) erhält.

Anstatt der Reduktion zum Disulfid (XI), kann das Sulfonylchlorid (XII) mit einem geeigneten Reduktionsmittel wie zum Beispiel Iod/Phosphor zum Alkylthioat (XVII) reduziert und anschließend durch eine geeignete Methode, wie zum Beispiel die Umsetzung mit Kaliumhydroxidlösung, zu Thiolen der Formel (XVI) entschützt werden. Die Umsetzung der Thiole (XVI) mit Haloalkylelektrophilen der Formel (XXVI) liefert die Sulfide (IVa).

Die Verbindungen der Formel (X), (XI), (XII), (XIII), (XVI) und (XVII) sind neu und lassen sich insbesondere nach den in den Herstellbeispielen genannten Bedingungen herstellen.

Ebenfalls bevorzugt können die Thioether der Formel (IVa) alternativ nach folgendem Schema hergestellt werden,

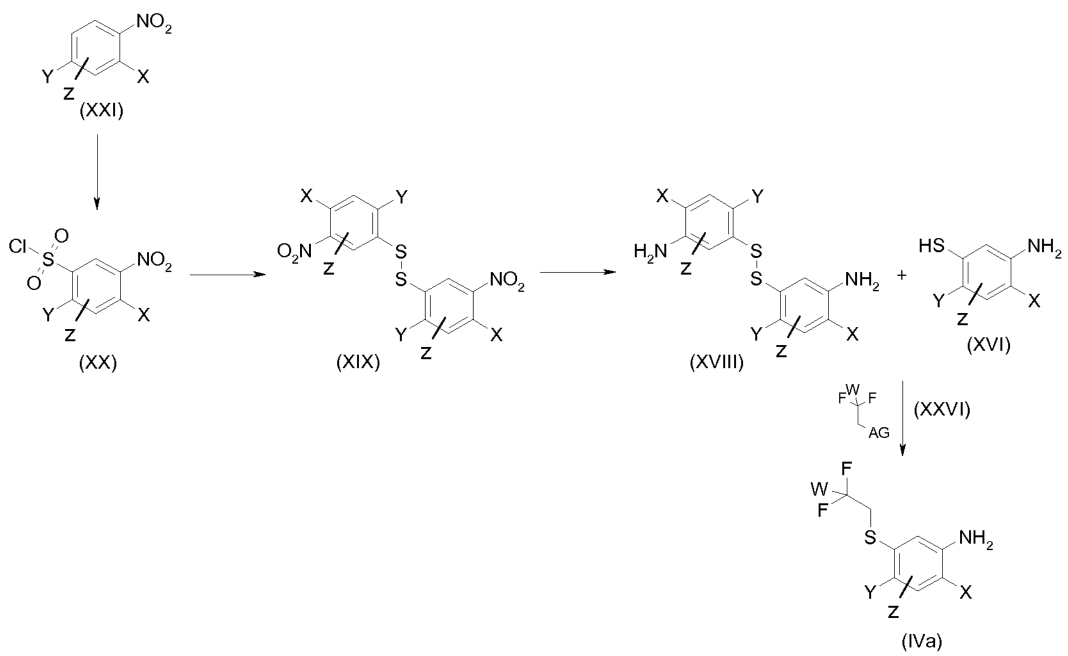


wobei X, Y, Z und W die oben angegebenen Bedeutungen haben, AG für eine Abgangsgruppe und PG für eine Schutzgruppe steht.

Die Chlorsulfonierung der Nitroaromaten der Formel (XXI) mit Chlorsulfonsäure liefert die entsprechenden Sulfonylchloride (XX). Die Reduktion der Sulfonylchloride (XX) in die Bis(nitroaryl)disulfide (XIX) ist mit literaturbekannten Methoden, wie zum Beispiel Iodid, möglich. Die Reduktion der Disulfide (XXI) in die Disulfanediyldianiline (XIX), die teilweise als Gemisch mit den entsprechenden Aminoarylthiolen (XVI) entstehen, ist mit allgemein bekannten Reduktionsmittel, wie zum Beispiel Wasserstoff, gegebenenfalls mit Hilfe heterogener Katalysatoren, wie zum Beispiel Raney-Nickel, Platin auf Aktivkohle oder Palladium auf Aktivkohle, möglich. Die Umsetzung der Disulfide (XVIII) bzw. Thiophenole (XVI) mit Haloalkylelektrophilen der Formel (XXVI), wobei AG für eine Abgangsgruppe wie zum Beispiel Chlor, Brom, Iod, Tosylat, Mesylat oder Triflat steht, liefert die 3-[(2,2,2-Trifluorethyl)sulfanyl]aniline der Formel (IVa).

Die Verbindungen der Formel (XVI), (XVIII), (XIX) und (XX) sind neu und lassen sich insbesondere nach den in den Herstellbeispielen genannten Bedingungen herstellen.

***Halogenide der allgemeinen Formel (VIIa)***

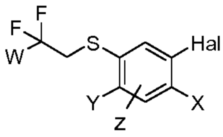

(VIIa)

in welcher X, Y, Z und W die oben angegebenen Bedeutungen haben und Hal für Chlor, Brom oder Iod steht, sind literaturbekannt aus WO 2007/034755, JP 2007/081019, JP 2007/284385, JP 2008/260706, JP 2008/308448, JP 2009/023910 oder WO 2012/176856 oder können anhand literaturbekannter Verfahren synthetisiert werden, die gegebenenfalls geringfügig modifiziert sein können, insbesondere wie in den konkreten Synthesebeispielen beschrieben.

Als Ausgangsstoffe für die Synthese der Iodide der allgemeinen Formel (VIIa) eignen sich Bromide derselben Formel, zum Beispiel in Halogenaustauschreaktionen nach literaturbekannten Methoden gegebenenfalls unter Metallkatalyse (s. H. Suzuki, Chem. Let. 1985, 3, 411-412; S. L. Buchwald, J. Amer. Chem. Soc. 2002, 124 (50), 14844-14845), insbesondere nach den in den Synthesebeispielen genannten Bedingungen. Ebenso ist die Synthese möglich ausgehend von Anilinen der Formel (IVa) unter Sandmeyers Reaktionsbedingungen, wie von E. B. Merkushev in Synthesis 1988, 12, 923-937, beschrieben.

***Boronsäuren der allgemeinen Formel (VIIIa) und (VIIIb)***

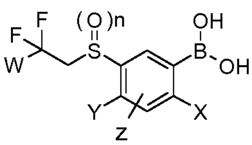

(VIIIa) n=0
(VIIIb) n=1

in welcher X, Y, Z und W die oben angegebenen Bedeutungen haben, sind literaturbekannt, z.B. aus WO2007/034755, JP2007/284385, JP2009/023910 und WO2012/176856 oder können anhand literaturbekannter Verfahren synthetisiert werden, insbesondere wie in den konkreten Synthesebeispielen beschrieben.

***Hydrazine der allgemeinen Formel (Va)***

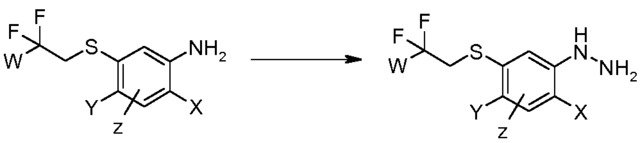

(IVa) (Va)

Hydrazine der allgemeinen Formel (Va) sind teilweise literaturbekannt, zum Beispiel aus EP 1803712 A1 und WO 2006043635, oder können anhand literaturbekannter Verfahren synthetisiert werden, wie zum Beispiel in J. Med. Chem. 2003, 46, 4405-4418 beschrieben.

***Isocyanate der Formel (IIIa)***

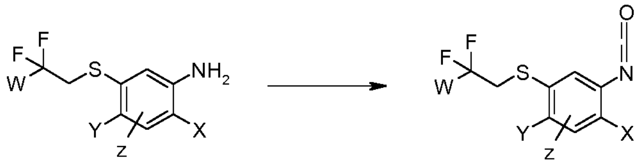

(IVa) (IIIa)

Isocyanate der allgemeinen Formel (IIIa) sind teilweise literaturbekannt, zum Beispiel aus JP 2011/042611 A, oder können anhand literaturbekannter Verfahren synthetisiert werden, wie zum Beispiel in EP 1183229 B1, in J. Amer. Chem. Soc. 1940, 62, 2965-2966, in Angew. Chem. 1995, 107, 2746-2749 oder in US2010/160388 A1 beschrieben, insbesondere wie in den konkreten Synthesebeispielen beschrieben.

***Heterocyclischen Verbindungen der Formel (XXII)***

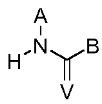

(XXII)

in welcher A und B die vorstehende Bedeutung aufweisen sind kommerziell erhältlich, literaturbekannt oder können anhand literaturbekannter Verfahren synthetisiert werden. Als Beispiele werden die unterschiedlichen heterocyclischen Verbindungen in ihre Subklassen unterteilt und dort genannt.

***2,4-Dihydro-3H-1,2,4-triazol-3-one der Formel (XXII-A)***

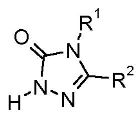

(XXII-A)

2,4-Dihydro-3H-1,2,4-triazol-3-one der allgemeinen Formel (XXII-A) sind kommerziell erhältlich, literaturbekannt oder können anhand literaturbekannter Verfahren (analog der unten zitierten Literaturstellen) synthetisiert werden. Als Beispiele seien folgende Verbindungen genannt: 2,4-Dihydro-3H-1,2,4-triazol-3 -on (kommerziell erhältlich), 4-Cyclopropyl-5-methyl-2,4-dihydro-3H-1,2,4-triazol-3-on (kommerziell erhältlich), 5-Methyl-2,4-dihydro-3H-1,2,4-triazol-3-on (siehe zum Beispiel Chem. Ber. 1965, 98, 3025-3034), 4-Cyclopropyl-5-isopropyl-2,4-dihydro-3H-1,2,4-triazol-3-on (siehe zum Beispiel EP 422469 A2), 4-Methyl-5-phenyl-2,4-dihydro-3H-1,2,4-triazol-3-on (siehe zum Beispiel J. Med. Chem. 1990, 33, 2772-2777; Synthesis 1987, 10, 912-914), 4-Ethyl-5-(trifluormethyl)-2,4-dihydro-3H-1,2,4-triazol-3-on (siehe zum Beispiel EP 657437 A1), 4-Allyl-5-methoxy-2,4-dihydro-3H-1,2,4-triazol-3-on (siehe zum Beispiel EP 507171 A1), 4-Methyl-5-(2,2,2-trifluorethoxy)-2,4-dihydro-3H-1,2,4-triazol-3-on (siehe zum Beispiel DE 19508118 A1), 4-Methyl-5-(tetrahydrofuran-3-yloxy)-2,4-dihydro-3H-1,2,4-triazol-3-on (siehe zum Beispiel DE 19525973 A1), 4-Cyclopropyl-5-(dimethylamino)-2,4-dihydro-3H-1,2,4-triazol-3-on (siehe zum Beispiel EP 422469 A2), 4-Amino-5-(methoxymethyl)-2,4-dihydro-3H-1,2,4-triazol-3-on (siehe zum Beispiel EP 422469 A2), 4-(Dimethylamino)-5-(trifluormethyl)-2,4-dihydro-3H-1,2,4-triazol-3-on (siehe zum Beispiel DE 4339412 A1; EP 726258 A1), 4-Amino-5-(dimethylamino)-2,4-dihydro-3H-1,2,4-triazol-3-on (siehe zum Beispiel EP 415196 A2), 4-Methoxy-5-methyl-2,4-dihydro-3H-1,2,4-triazol-3-on (siehe zum Beispiel EP 422469 A2; DE 4239296 A1), 5-Ethoxy-4-methoxy-2,4-dihydro-3H-1,2,4-triazol-3-on (siehe zum Beispiel EP 534266 A1), 4-Methyl-5-(methylsulfanyl)-2,4-dihydro-3H-1,2,4-triazol-3-on (kommerziell erhältlich), 4-Cyclopropyl-5-(methylsulfanyl)-2,4-dihydro-3H-1,2,4-triazol-3-on (siehe zum Beispiel DE 3936623 A1; US 5599944 A), 4-Methyl-5-[(trifluormethyl)sulfanyl]-2,4-dihydro-3H-1,2,4-triazol-3-on (siehe zum Beispiel DE 19508119 A1), 4-Ethyl-5-(methylsulfanyl)-2,4-dihydro-3H-1,2,4-triazol-3-on (siehe zum Beispiel Tetrahedron 2001, 57, 2003-2010), 5-Brom-4-cyclopropyl-2,4-dihydro-3H-1,2,4-triazol-3-on (siehe zum Beispiel EP 425948 A2).

***1,4-Dihydro-5H-tetrazol-5-one der allgemeinen Formel (XXII-B)***

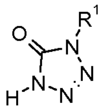

(XXII-B)

1,4-Dihydro-5H-tetrazol-5-one der allgemeinen Formel (XXII-B) sind kommerziell erhältlich, literaturbekannt oder können anhand literaturbekannter Verfahren synthetisiert werden. Als Beispiele seien genannt: 1,4-Dihydro-5H-tetrazol-5-on (kommerziell erhältlich), 1-(2,2,2-Trifluorethyl)-1,4-dihydro-5H-tetrazol-5-on (siehe zum Beispiel EP 711761 A1; EP 643049 A1); 1-(4-Chlorphenyl)-1,4-dihydro-5H-tetrazol-5-on (siehe zum Beispiel J. Org. Chem. 1980, 45, 5130-5136; Chem. Lett. 2011, 40, 1149-1151; J. Heterocycl. Chem. 2007, 44, 937-943), 1-Methyl-1,4-dihydro-5H-tetrazol-5-on (siehe zum Beispiel US5502204 A1; J. Amer. Chem. Soc. 1959, 81, 3076-3079; US 20110130415), 1-Isopropyl-1,4-dihydro-5H-tetrazol-5-on (siehe zum Beispiel EP 643049 A1; EP 638561 A1; J. Med. Chem. 1986, 29, 2290-2297).

***1,3,4-Oxadiazol-2(3H)-one der Formel (XXII-C)***

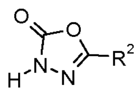

(XXII-C)

1,3,4-Oxadiazol-2(3H)-one der allgemeinen Formel (XXII-C) sind kommerziell erhältlich, literaturbekannt oder können anhand literaturbekannter Verfahren synthetisiert werden. Als Beispiel sei genannt: 5-(Trifluormethyl)-1,3,4-oxadiazol-2(3H)-on (kommerziell erhältlich).

***1,5-Dihydro-2H-pyrrol-2-one der Formel (XXII-D)***

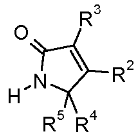

(XXII-D)

1,5-Dihydro-2H-pyrrol-2-one der allgemeinen Formel (XXII-D) sind kommerziell erhältlich, literaturbekannt oder können anhand literaturbekannter Verfahren synthetisiert werden. Als Beispiel sei genannt: 4-Methoxy-1,5-dihydro-2H-pyrrol-2-on (kommerziell erhältlich).

***Verbindungen der allgemeinen Formel (XXIII) und (XXIII)***

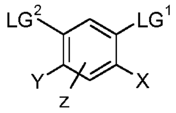

(XXIII)

(XXIII´) $LG^2$ = H

in welcher Z die oben angegebene Bedeutung hat, X steht für Wasserstoff oder eine elektronenziehende Gruppe (insbesondere Nitro, Chlor, Fluor, Cyano), Y stellt elektronenziehende Substituenten (insbesondere Nitro, Chlor, Fluor, Cyano) dar, $LG^1$ steht für typische Abgangsgruppen in nucleophilen Substitutionsreaktionen (insbesondere Fluorid, Chlorid) und $LG^2$ kann für Wasserstoff oder für typische Abgangsgruppen in nucleophilen Substitutionsreaktionen (insbesondere Fluorid, Chlorid) stehen, sind kommerziell erhältlich, literaturbekannt oder lassen sich nach literaturbekannten Methoden herstellen.

Als Beispiele seien folgende Verbindungen genannt, die kommerziell erhältlich sind: 2,4,5-Trifluorbenzonitril (Y=CN, X=$LG^1$=$LG^2$=F, Z=H), 2,4-Difluorobenzonitril (Y=CN, X=Z=H, $LG^1$=$LG^2$=F), 3,4-Difluorbenzonitril (Y=CN, X=$LG^1$=F, $LG^2$=Z=H).

***Verbindungen der allgemeinen Formel (XXIV) und (XXIV)***

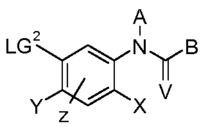

(XXIV)

(XXIV´) $LG^2$ = H

in welcher A, B, V und Z die oben angegebene Bedeutung haben, X steht für Wasserstoff oder eine elektronziehende Gruppe (insbesondere Nitro, Chlorid, Fluorid, Cyano), Y stellt elektronenziehende Substituenten (insbesondere Nitro, Chlorid, Fluorid, Cyano) dar und $LG^2$ kann für Wasserstoff oder für typische Abgangsgruppen in nucleophilen Substitutionsreaktionen (insbesondere Fluorid, Chlorid) stehen, sind kommerziell erhältlich, literaturbekannt oder lassen sich nach literaturbekannten Methoden herstellen, insbesondere wie in den konkreten Synthesebeispielen beschrieben.

Als Beispiele seien folgende Verbindungen genannt: 4-[4-Cyclopropyl-5-oxo-3-(trifluormethyl)-4,5-dihydro-1H-1,2,4-triazol-1-yl]-2,5-difluorbenzonitril (Y=CN, X=$LG^2$=F, Z=H) (s. Synthesebeispiele), Ethyl-4-methyl-5-oxo-1-phenyl-4,5-dihydro-1H-1,2,4-triazol-3-carboxylat (Y=CN, X=F, $LG^2$=Z=H) (kommerziell erhältlich), Methyl-1-(2-fluor-4-methylphenyl)-4-isopropyl-5-oxo-4,5-dihydro-1H-1,2,4-triazol-3-carboxylat (Synthese nach DE 2725148).

***Thiole der allgemeinen Formel (XXV)***

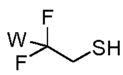

(XXV)

in welcher W die oben angegebenen Bedeutungen haben sind kommerziell erhältlich, literaturbekannt oder anhand literaturbekannter Verfahren synthetisiert werden.

Als Beispiele seien folgende Thiole genannt: 2,2,2-Trifluorethanthiol (W=F) (kommerziell erhältlich), 2,2-Difluorethanthiol (W=H) (Synthese nach J. Amer. Chem. Soc. 1963, 85, 749-754), 2-Chlor-2,2-difluorethanthiol (W=Cl) (Synthese nach Phosp., Sulf., Sil. and rel. Elem. 1996, 119, 161-168).

***Elektrophile der allgemeinen Formel (XXVI)***

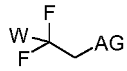

(XXVI)

in welcher W die oben angegebenen Bedeutungen hat und AG steht für Halogen (insbesondere Chlor, Iod) sind kommerziell erhältlich, literaturbekannt oder anhand literaturbekannter Verfahren synthetisiert werden.

Als Beispiele seien folgende Elektrophile genannt, die kommerziell erhältlich sind: 2-Chlor-1,1,1-trifluorethan (W=F, AG=Cl), 2-Chlor-1,1-difluorethan (W=H, AG=Cl), 2-Brom-1,1,1-trifluorethan (W=F, AG=Br), 2-Brom-1,1-difluorethan (W=H, AG=Br), 2-Iod-1,1,1-trifluorethan (W=F, AG=I), 2-Iod-1,1-difluorethan (W=H, AG=I), 2,2,2-Trifluorethylmethansulfonat (W=F, AG=-$SO_2$Me), 2,2,2-Trifluorethyltriflat (W=F, AG=-$SO_2CF_3$), 2,2,2-Trifluorethyltosylat (W=F, AG=-$SO_2(4\text{-}CH_3C_6H_4)$).

***Furan-2,5-dione der allgemeinen Formel (XLIV)***

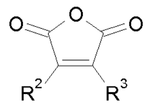

(XLIV)

sind literaturbekannt oder können anhand literaturbekannter Verfahren synthetisiert werden.

Als Beispiele seien folgende Furandione genannt, die kommerziell erhältlich sind: 3-Bromfuran-2,5-dion, 3,4-Dichlorfuran-2,5-dion, 3-Methylfuran-2,5-dion, 3,4-Dimethylfuran-2,5-dion.

**Verwendung**

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen, wobei Verfahren und Verwendungen in Verfahren zur chirurgischen oder therapeutischen Behandlung des menschlichen oder tierischen Körpers und Diagnostizierverfahren, die am menschlichen oder tierischen Körper vorgenommen werden, ausgenommen sind. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus

spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp.

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp.

Aus der Ordnung der Hymenoptera z.B. Athalia spp., Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp.

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp.

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp.

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z.B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z.B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanolalkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z.B. Dioctylsulfosuccinat oder Hydroxypropyl-guar Polymere und/oder Humectants wie z.B. Glycerin und/oder Dünger wie beispielsweise Ammonium-, Kalium- oder Phosphorenthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen

oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die gegebenenfalls auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffe in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z.B. hohen oder niedrigen Temperaturen, gegen Trockenheit oder gegen erhöhten Wasser- bzw. Bodensalzgehalt. Auch lässt sich das Blüh- und Fruchtverhalten verbessern, die Keimfähigkeit und Bewurzelung optimieren die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die Folgenden:

**Insektizide/Akarizide/Nematizide**

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.

(2) GABA-gesteuerte Chlorid-Kanal-Antagonisten, wie beispielsweise
Cyclodien-organochlorine, z.B. Chlordane und Endosulfan; oder
Phenylpyrazole (Fiprole), z.B. Ethiprole und Fipronil.

(3) Natrium-Kanal-Modulatoren / Spannungsabhängige Natrium-Kanal-Blocker, wie beispielsweise
Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
DDT; oder Methoxychlor.

(4) Nikotinerge Acetylcholin-Rezeptor (nAChR) Agonisten, wie beispielsweise
Neonikotinoide, z.B. Acetamiprid, Clothianidin, Dinotefuran, Imidacloprid, Nitenpyram, Thiacloprid und Thiamethoxam; oder
Nikotin.

(5) Nikotinerge Acetylcholin-Rezeptor (nAChR) allosterische Aktivatoren, wie beispielsweise
Spinosine, z.B. Spinetoram und Spinosad.

(6) Chlorid-Kanal-Aktivatoren, wie beispielsweise
Avermectine/Milbemycine, z.B. Abamectin, Emamectin-benzoat, Lepimectin und Milbemectin.

(7) Juvenilhormon-Imitatoren, wie beispielsweise
Juvenilhormon-Analoge, z.B. Hydroprene, Kinoprene und Methoprene; oder
Fenoxycarb; oder Pyriproxyfen.

(8) Wirkstoffe mit unbekannten oder nicht spezifischen Wirkmechanismen, wie beispielsweise Alkylhalide, z.B. Methylbromid und andere Alkylhalide; oder
Chloropicrin; oder Sulfurylfluorid; oder Borax; oder Brechweinstein.

(9) Selektive Fraßhemmer, z.B. Pymetrozine; oder Flonicamid.

(10) Milbenwachstumsinhibitoren, z.B. Clofentezine, Hexythiazox und Diflovidazin; oder
Etoxazole.

(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.

(12) Inhibitoren der oxidativen Phosphorylierung, ATP-Disruptoren, wie beispielsweise Diafenthiuron; oder Organozinnverbindungen, z.B. Azocyclotin, Cyhexatin und Fenbutatin-oxid; oder
Propargite; oder Tetradifon.

(13) Entkoppler der oxidativen Phoshorylierung durch Unterbrechung des H-Protongradienten, wie beispielsweise Chlorfenapyr, DNOC und Sulfluramid.

(14) Nikotinerge Acetylcholin-Rezeptor-Antagonisten, wie beispielsweise Bensultap, Cartap-hydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsstörende Wirkstoffe, Dipteran, wie beispielsweise Cyromazine.

(18) Ecdyson-Rezeptor Agonisten, wie beispielsweise Chromafenozide, Halofenozide, Methoxyfenozide und Tebufenozide.

(19) Oktopaminerge Agonisten, wie beispielsweise Amitraz.

(20) Komplex-III-Elektronentransportinhibitoren, wie beispielsweise Hydramethylnon; oder Acequinocyl; oder Fluacrypyrim.

(21) Komplex-I-Elektronentransportinhibitoren, beispielsweise
METI-Akarizide, z.B. Fenazaquin, Fenpyroximate, Pyrimidifen, Pyridaben, Tebufenpyrad und Tolfenpyrad; oder
Rotenone (Derris).

(22) Spannungsabhängige Natriumkanal-Blocker, z.B. Indoxacarb; oder Metaflumizone.

(23) Inhibitoren der Acetyl-CoA-Carboxylase, wie beispielsweise
Tetron- und Tetramsäurederivate, z.B. Spirodiclofen, Spiromesifen und Spirotetramat.

(24) Komplex-IV-Elektronentransportinhibitoren, wie beispielsweise
Phosphine, z.B. Aluminiumphosphid, Calciumphosphid, Phosphin und Zinkphosphid; oder
Cyanid.

(25) Komplex-II-Elektronentransportinhibitoren, wie beispielsweise Cyenopyrafen.

(28) Ryanodinrezeptor-Effektoren, wie beispielsweise
Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-$\lambda^4$-sulfanyliden}cyanamid (bekannt aus WO2007/149134) und seine Diastereomere {[(1R)-1-(6-Chlorpyridin-3-yl)ethyl](methyl)oxido-$\lambda^4$-sulfanyliden}cyanamid (A) und {[(1S)-1-(6-Chlorpyridin-3-yl)ethyl](methyl)oxido-$\lambda^4$-sulfanyliden}cyanamid (B) (ebenfalls bekannt aus WO2007/149134) sowie Sulfoxaflor und seine Diastereomere [(R)-Methyl(oxido){(1R)-1-[6-(trifluormethyl)pyridin-3-yl]ethyl}-$\lambda^4$-sulfanyliden]cyanamid (A1) und [(S)-Methyl(oxido){(1S)-1-[6-(trifluormethyl)pyridin-3-yl]ethyl}-$\lambda^4$-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-$\lambda^4$-sulfanyliden]cyanamid (B1) und [(S)-Methyl(oxido){(1R)-1-[6-(trifluormethyl)pyridin-3-yl]ethyl}-$\lambda^4$-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233).

**Fungizide**

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb

(88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).

(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus WO2010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.

(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).

(4) Inhibitoren der Mitose und Zellteilung, wie beispielsweise (4.1) Benomyl (17804-35-2), (4.2) Carbendazim

(10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).

(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).

(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).

(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus WO2005070917).

(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).

(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).

(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).

(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus WO2005042474).

(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (4148343-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).

(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).

(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).

(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus WO2003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus WO2005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus WO2009094442), (15.75) 5-Fluor-2-[(4-methylbenzyl)oxy]pyrimidin-4-amin (1174376-25-0) (bekannt aus WO2009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl} -2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.

(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino} oxy)methyl]pyridin-2-yl} carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet

worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops

spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec., Tryptodendron spec., Apate monachus, Bostrychus capucins, Heterobostrychus brunneus, Sinoxylon spec., Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

## Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC-MS (Liquid Chromatography Mass Spectrometry) und/oder GC-MS (Gas Chromatography-Mass Spectrometry) charakterisiert.

Die Bestimmung der logP Werte erfolgte analog OECD Guideline 117 (EC Directive 92/69/EEC) durch HPLC

(High Performance Liquid Chromatography) an reversed-phase Säulen (C 18), mit nachfolgenden Methoden:

[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10% Acetonitril bis 95 % Acetonitril.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker II Avance 400, ausgestattet mit einem 1,7 mm TCI-Probenkopf, gemessen. In Einzelfällen wurden die NMR Spektren mit einem Bruker Avance II 600 bestimmt.

Die NMR-Daten ausgewählter Beispiele werden in klassischer Form ($\delta$-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), m (Multiplett), breit (für breite Signale). Als Lösungsmittel wurden $CD_3CN$, $CDCl_3$ oder D6-DMSO verwendet, wobei als Referenz Tetramethylsilan (0.00 ppm) eingesetzt wurde.

Die GC-MS-Spektren wurden mit einem Agilent 6890 GC, HP 5973 MSD an Dimethylsilikonphase bestimmt, mit einem Temperaturgradient von 50 °C bis 320 °C. GC-MS-Indices werden als Kovats-Indices mit Lösung einer homologen Reihe von n-Alkanen (mit geradzahliger Anzahl von 8 bis 38 Kohlenstoffatomen) bestimmt.

Die Enantiomere wurden aus dem Racemat gewonnen, in dem sie präparativ mittels HPLC über einer chiralen Säule (ChiralCel OJ-H z.B. 5nm 250 x4.6 mm) mit Laufmittel Heptan / Methanol / Ethanol (95:2.5:2.5) getrennt wurden.

## Herstellbeispiele:

**Herstellbeispiel 1**: 2-{2-Fluor-4-methyl-5-[(2,2,2-trifluorethyl)sulfanyl]phenyl} -4-isopropyl-2,4-dihydro-3H-1,2,4-triazol-3-on (Bsp.Nr.4)

Stufe 1: *1-{2-Fluor-4-methyl-5-[(2,2,2-trifluorethyl)sulfanyl]phenyl}-4-isopropyl-5-oxo-4,5-dihydro-1H-1,2,4-triazol-3-carbonsäure*

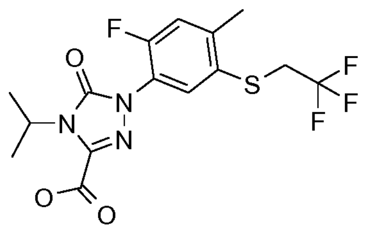

1,50 g (2,31 mmol) Dimethyl-1,1'-[disulfanediylbis(6-fluor-4-methylbenzol-3,1-diyl)]bis(4-isopropyl-5-oxo-4,5-dihydro-1H-1,2,4-triazol-3-carboxylat) (XXVIII-1) werden in 30 ml *N,N*-Dimethylformamid vorgelegt und mit 0,83 g Natrium-Dithionit, 1,9 g Kaliumcarbonat und 0,7 g Natriumbiphosphat und 15 ml Wasser versetzt und anschließend 3 h bei 60°C gerührt. Nach dem Abkühlen werden 1,70 g (8,1 mmol) 1,1,1-Trifluor-2-iodethan dazugegeben und weitere 12 h bei 70°C gerührt. Nach Entfernen des Lösungsmittels unter vermindertem Druck wird der verbleibende Rückstand mit konzentrierter Salzsäure angesäuert und der entstandene Niederschlag abgesaugt. Es werden 570 mg (86% Reinheit, 63% der Theorie) Produkt als grauer Feststoff erhalten.
logP[a]: 2,16

Stufe 2: *2-{2-Fluor-4-methyl-5-[(2,2,2-trifluorethyl)sulfanyl]phenyl}-4-isopropyl-2,4-dihydro-3H-1,2,4-triazol-3-on* (Bsp.Nr.4)

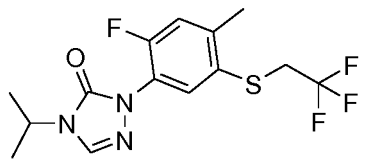

300 mg (0,76 mmol) 1-{2-Fluor-4-methyl-5-[(2,2,2-trifluorethyl)sulfanyl]phenyl}-4-isopropyl-5-oxo-4,5-dihydro-1H-1,2,4-triazol-3-carbonsäure werden in 5 ml Xylol 18 h unter Rückfluss erhitzt. Nach Entfernen des Lösungsmittels unter vermindertem Druck erhält man 260 mg (89% Reinheit, 99% der Theorie) Produkt als beigen Feststoff.
logP[a]: 2,82; logP[b]: 2,97; 1H-NMR (D6-DMSO, 400MHz) δ ppm: 8,36(s,1H), 7,73-7,71(m,1H), 7,41-7,38(m,1H), 4,17(sept,1H), 3,99-3,91(q,2H), 2,43(s,3H), 1,38-1,36(d,6H)

**Herstellbeispiel 2:** 2-{2-Fluor-4-methyl-5-[(2,2,2-trifluorethyl)sulfinyl]phenyl}-4-isopropyl-2,4-dihydro-3H-1,2,4-triazol-3-on (Bsp.Nr.6)

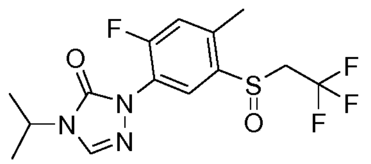

Zu einer Lösung aus 26 mg (0,07 mmol) 2-{2-Fluor-4-methyl-5-[(2,2,2-trifluorethyl)sulfanyl]phenyl}-4-isopropyl-2,4-dihydro-3H-1,2,4-triazol-3-on in 20 ml Trichlormethan und 20 mg Puffer-Lösung pH 7 ($KH_2PO_4$/$Na_2HPO_4$) werden bei 0-4 °C portionsweise 22 mg (70%ig, 0,1 mmol) *meta*-Chlorperbenzoesäure (ca. 77%ig) dazugegeben und das Reaktionsgemisch wird 24 h bei RT gerührt. Nach Versetzung mit einer 33%igen wäßrigen Bisulfit-Lösung wird das Gemisch zweimal mit Essigester extrahiert. Die vereinigten organischen Phasen werden mit Wasser gewaschen, über Natriumsulfat getrocknet und filtriert. Nach Entfernen des Lösungsmittels unter vermindertem Druck werden 18 mg (86% Reinheit, 66% der Theorie) Sulfoxid erhalten.
logP[a]: 1,88; logP[b]: 2,02; 1H-NMR (D6-DMSO, 400MHz) δ ppm: 8,42(s,1H), 7,99-7,97(m,1H), 7,53-7,50(m,1H), 4,26-4,02(m,3H), 2,42(s,3H), 1,39-1,37(d,6H)

**Herstellbeispiel 3:** 2-{2-Fluor-4-methyl-5-[(2,2,2-trifluorethyl)sulfanyl]phenyl}-4,5-dimethyl-2,4-dihydro-3H-1,2,4-triazol-3-on (Bsp.Nr.10)

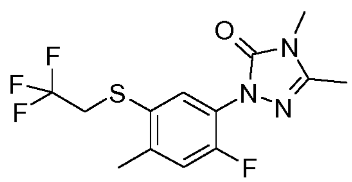

454,6 mg (1,5 mmol) 5-Brom-4-fluor-2-methylphenyl-2,2,2-trifluorethylsulfid, 203,6 mg (1,8 mmol) 4,5-Dimethyl-2,4-dihydro-3H-1,2,4-triazol-3-on (Synthese z.B. Bull. Soc. Chim. Fr. 1975,1191-1194), 28,6 mg (0,15 mmol) Kupfer(I)-iodid, 42,7 mg (0,30 mmol) *trans-N,N'*-Dimethylcyclohexan-1,2-diamin (racemisch), 74,7 mg (0,45 mmol) Kaliumiodid sowie 621,9 mg (4,50 mmol) Kaliumcarbonat werden in 3 mL entgastem, trockenem Dioxan 2 d bei 115 °C gerührt. Nach Abkühlen auf Raumtemperatur wird die Reaktionsmischung mit Essigester verdünnt und mit Essigester über Kieselgel filtriert. Nach säulenchromatographischer Reinigung an Kieselgel mittels MPLC mit Cyclohexan/Essigester als Laufmittel erhält man 136 mg (100% Reinheit, 27% der Theorie) der Titelverbindung als farbloses Öl.
logP[a]: 2,42; logP[b]: 2,38; 1H-NMR (D6-DMSO, 400MHz) δ ppm: 7,68(d,1H), 7,38(d,1H), 3,93(q,2H), 3,20(s,3H), 2,43(s,3H), 2,25(s,3H)

**Herstellbeispiel 4:** 2-{2-Fluor-4-methyl-5-[(2,2,2-trifluorethyl)sulfinyl]phenyl}-4,5-dimethyl-2,4-dihydro-3H-1,2,4-triazol-3-on (Bsp.Nr.11)

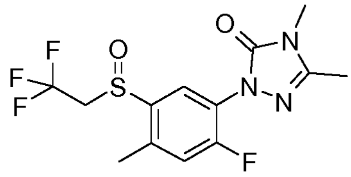

Zu einer Lösung aus 95,0 mg (0,28 mmol) 2-{2-Fluor-4-methyl-5-[(2,2,2-trifluorethyl)sulfanyl]phenyl}-4,5-dimethyl-2,4-dihydro-3H-1,2,4-triazol-3-on in 10 mL Dichlormethan werden 63,5 mg (0,28 mmol) *meta*-Chlorperbenzoesäure (ca. 77%ig) gegeben. Die Reaktionsmischung wird über Nacht bei Raumtemperatur gerührt, mit gesättigter wässriger Natriumbicarbonatlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 70 mg (100% Reinheit, 70% der Theorie) der Titelverbindung als farbloses Öl.
logP[a]: 1,57; logP[b]: 1,50; 1H-NMR (D6-DMSO, 400MHz) δ ppm: 7,94(d,1H), 7,49(d,1H), 4,28-4,16(m,1H), 4,10-3,98(m,1H), 3,21(s,3H), 2,41(s,3H), 2,27(s,3H)

**Herstellbeispiel 5:** 2-{2-Fluor-4-methyl-5-[(2,2,2-trifluorethyl)sulfanyl]phenyl} -4-methyl-5-(trifluormethyl)-2,4-dihydro-3H-1,2,4-triazol-3-on (Bsp.Nr.9)

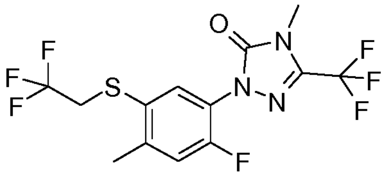

Herstellung nach Verfahren V3: 454,6 mg (1,5 mmol) 5-Brom-4-fluor-2-methylphenyl-2,2,2-trifluorethylsulfid, 300,8 mg (1,8 mmol) 4-Methyl-5-(trifluormethyl)-2,4-dihydro-3H-1,2,4-triazol-3-on (Synthese nach DE 4339412), 28,6 mg (0,15 mmol) Kupfer(I)-iodid, 42,7 mg (0,30 mmol) *trans-N,N'*-Dimethylcyclohexan-1,2-diamin (racemisch), 74,7 mg (0,45 mmol) Kaliumiodid sowie 621,9 mg (4,50 mmol) Kaliumcarbonat werden in 3 mL entgastem, trockenem Dioxan unter Mikrowellenbestrahlung (Anton-Paar, 2-5 mL-Gefäß) 3h bei 160 °C gerührt. Nach Abkühlen auf Raumtemperatur wird die Reaktionsmischung mit Essigester verdünnt und mit Essigester über Kieselgel filtriert. Nach säulenchromatographischer Reinigung an Kieselgel mittels MPLC mit Cyclohexan/Essigester als Laufmittel erhält man 70 mg (100% Reinheit, 12% der Theorie) der Titelverbindung als gelbes Öl.

Herstellung nach Verfahren V3': 268,0 mg (1,0 mmol) {2-Fluor-4-methyl-5-[(2,2,2-trifluorethyl)sulfanyl]phenyl}boronsäure, 334 mg (2,00 mmol) 4-Methyl-5-(trifluormethyl)-2,4-dihydro-3H-1,2,4-triazol-3-on, 272 mg (1,5 mmol) Kupfer(II)-acetat, 158 mg (2,0 mmol) Pyridin sowie 0,5 g aktiviertes 3A-Molsieb werden in 5 mL trockenem Dichlormethan 3 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 98 mg (87% Reinheit, 22% der Theorie) der Titelverbindung als farbloses Öl.
logP[a]: 3,58; logP[b]: 3,53; 1H-NMR (D6-DMSO, 400MHz) δ ppm: 7,79(d,1H), 7,47(d,1H), 3,95(q,2H), 3,38(s,3H), 2,45(s,3H)

**Herstellbeispiel 6:** 2-{2-Fluor-4-methyl-5-[(2,2,2-trifluorethyl)sulfinyl]phenyl} -4-methyl-5-(trifluormethyl)-2,4-dihydro-3H-1,2,4-triazol-3-on (Bsp.Nr.12)

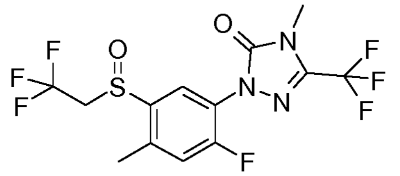

Zu einer Lösung aus 70,0 mg (0,18 mmol) 2-{2-Fluor-4-methyl-5-[(2,2,2-trifluorethyl)sulfanyl]phenyl}-4-methyl-5-(trifluormethyl)-2,4-dihydro-3H-1,2,4-triazol-3-on in 10 mL Dichlormethan werden 40,3 mg (0,18 mmol) *meta*-Chlorperbenzoesäure (ca. 77%ig) gegeben. Die Reaktionsmischung wird über Nacht bei Raumtemperatur gerührt, mit gesättigter wässriger Natriumbicarbonatlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 51 mg (95% Reinheit, 65% der Theorie) der Titelverbindung als farblosen Feststoff.

logP[a]: 2,52; logP[b]: 2,46; 1H-NMR (D6-DMSO, 400MHz) δ ppm: 8,04(d,1H), 7,58(d,1H), 4,32-4,20(m,1H), 4,09-3,98(m,1H), 3,38(s,3H), 2,44(s,3H)

**Herstellbeispiel 7**: 4-[4-Cyclopropyl-5-oxo-3-(trifluormethyl)-4,5-dihydro-1H-1,2,4-triazol-1-yl]-5-fluor-2-[(2,2,2-trifluorethyl)sulfmyl]benzonitril (Bsp.Nr.47)

Stufe 1: *4-[4-Cyclopropyl-5-oxo-3-(trifluormethyl)-4,5-dihydro-1H-1,2,4-triazol-1-yl]-5-fluor-2-[(2,2,2-trifluorethyl)sulfanyl]benzonitril*

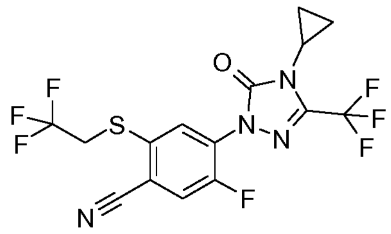

Zu einer Lösung von 64 mg (1,6 mmol) Natriumhydrid (60%ig in Mineralöl) in 25 mL *N,N*-Dimethylformamid werden bei 5-10 °C 116 mg (1,6 mmol) 2,2,2-Trifluorethylthiol gegeben. Nach 30-minütigem Rühren bei Raumtemperatur wird diese Lösung zu einer auf -15 °C gekühlten Lösung von 0,5 g (1,5 mmol) 4-[4-Cyclopropyl-5-oxo-3-(trifluormethyl)-4,5-dihydro-1H-1,2,4-triazol-1-yl]-2,5-difluorbenzonitril (XXIV-1) in 25 mL N,N-Dimethylformamid getropft und 2 h bei -20 °C bis -10 °C gerührt. Die Reaktionsmischung wird in Wasser gegossen, mit gesättigter wässriger Ammoniumchloridlösung neutralisiert und mit Essigester extrahiert. Die vereinten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Nach säulenchromatographischer Reinigung an RP(C-18)-Kieselgel mittels MPLC mit Wasser/Acetonitril als Laufmittel erhält man 47 mg (82% Reinheit, 6% der Theorie) der Titelverbindung als hellgelben Feststoff.

Stufe 2: *4-[4-Cyclopropyl-5-oxo-3-(trifluormethyl)-4,5-dihydro-1H-1,2,4-triazol-1-yl]-5-fluor-2-[(2,2,2-trifluorethyl)sulfinyl]benzonitril (Bsp.Nr.47)*

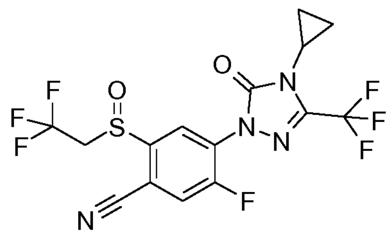

Zu einer Lösung aus 37,0 mg (0,09 mmol) 4-[4-Cyclopropyl-5-oxo-3-(trifluormethyl)-4,5-dihydro-1H-1,2,4-triazol-1-yl]-5-fluor-2-[(2,2,2-trifluorethyl)sulfanyl]benzonitril in 10 mL Dichlormethan werden bei 0 °C 19,5 mg (0,09 mmol) *meta*-Chlorperbenzoesäure (ca. 77%ig) gegeben. Die Reaktionsmischung wird 2 h bei 0 °C und über Nacht bei Raumtemperatur gerührt, dann mit Natronlauge (ca. 0.25 M) versetzt und mit Dichlormethan extrahiert. Die vereinten organischen Phasen werden über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 7 mg (92% Reinheit, 17% der Theorie) der Titelverbindung als farblosen Feststoff.
logP[a]: 2,92; logP[b]: 2,84; 1H-NMR (D6-DMSO, 400MHz) $\delta$ ppm: 8,46(d,1H), 8,24(d,1H), 4,52-4,40(m,1H), 4,33-4,22(m,1H), 3,10-3,05(m,1H), 1,15-1,02(m,4H)

**Herstellbeispiel 8:** 5-Methoxy-4-methyl-2-{3-[(2,2,2-trifluorethyl)sulfanyl]-4-(trifluormethyl)phenyl}-2,4-dihydro-3H-1,2,4-triazol-3-on (Bsp.Nr.95)

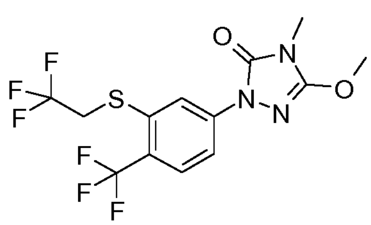

678 mg (2,0 mmol) 5-Brom-2-(trifluormethyl)phenyl-2,2,2-trifluorethylsulfid, 309,9 mg (2,4 mmol) 5-Methoxy-4-methyl-2,4-dihydro-3H-1,2,4-triazol-3-on (Synthese z.B. nach US 5599945), 38,1 mg (0,2 mmol) Kupfer(I)-iodid, 56,9 mg (0,4 mmol) *trans-N,N'*-Dimethylcyclohexan-1,2-diamin (racemisch), 99,6 mg (0,6 mmol) Kaliumiodid sowie 829,2 mg (6,0 mmol) Kaliumcarbonat werden in 8 mL entgastem, trockenem Dioxan über Nacht bei 120 °C gerührt. Nach Abkühlen auf Raumtemperatur wird die Reaktionsmischung mit Essigester verdünnt, mit Essigester über Kieselgel filtriert und unter vermindertem Druck vom Lösungsmittel befreit. Nach säulenchromatographischer Reinigung an RP(C-18)-Kieselgel mittels MPLC mit Wasser/Acetonitril als Laufmittel erhält man 86 mg (92% Reinheit, 10% der Theorie) der Titelverbindung als gelbes Öl.
logP[a]: 3,74; logP[b]: 3,64; 1H-NMR (D6-DMSO, 400MHz) δ ppm: 8,34(d,1H), 7,94-7,92(m,1H), 7,87-7,84(m,1H), 4,11-4,04(m,5H), 3,11(s,3H)

**Herstellbeispiel 9:** 5-Methoxy-4-methyl-2-{3-[(2,2,2-trifluorethyl)sulfinyl]-4-(trifluormethyl)phenyl}-2,4-dihydro-3H-1,2,4-triazol-3-on (Bsp.Nr.96)

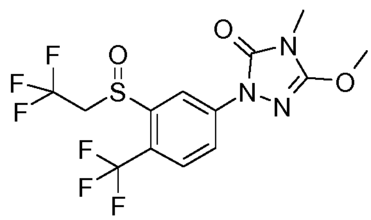

Zu einer Lösung aus 65,0 mg (0,17 mmol) 5-Methoxy-4-methyl-2-{3-[(2,2,2-trifluorethyl)sulfanyl]-4-(trifluormethyl)phenyl}-2,4-dihydro-3H-1,2,4-triazol-3-on in 10 mL Dichlormethan werden bei 0 °C 33,8 mg (0,15 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhält man 43 mg (99% Reinheit, 63% der Theorie) der Titelverbindung als farblosen Feststoff.
logP[a]: 2,89; logP[b]: 2,83; 1H-NMR (D6-DMSO, 400MHz) δ ppm: 8,70(d,1H), 8,32-8,29(m,1H), 8,03(d,1H), 4,15(q,2H), 4,09(s,3H), 3,13(s,3H)

**Herstellbeispiel 10:** 5-Cyclopropyl-4-(dimethylamino)-2-{2-fluor-4-methyl-5-[(2,2,2-trifluorethyl)sulfanyl]phenyl}-2,4-dihydro-3H-1,2,4-triazol-3-on (Bsp.Nr.195)

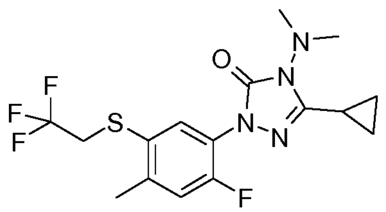

303,1 mg (1,0 mmol) 5-Brom-4-fluor-2-methylphenyl-2,2,2-trifluorethylsulfid, 201,8 mg (1,2 mmol) 5-Cyclopropyl-4-(dimethylamino)-2,4-dihydro-3H-1,2,4-triazol-3-on (Synthese nach US 5516749), 38,1 mg (0,20 mmol) Kupfer(I)-iodid, 35,3 mg (0,40 mmol) *N,N'*-Dimethylethylendiamin sowie 424,5 mg (2,0 mmol) Kaliumphosphat werden in 3 mL entgastem, trockenem Dioxan über Nacht bei 110 °C gerührt. Nach Abkühlen auf Raumtemperatur wird die Reaktionsmischung mit Dichlormethan verdünnt, sukzessive mit Wasser und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 105 mg (99% Reinheit, 27% der Theorie) der Titelverbindung als farbloses Öl.
logP[a]: 3,75; logP[b]: 3,73; 1H-NMR (D6-DMSO, 400MHz) δ ppm: 7,67(d,1H), 7,37(d,1H), 3,94(q,2H), 2,95(s,6H), 2,43(s,3H), 2,04-1,97(m,1H), 0,99-0,93(m,2H), 0,91-0,85(m,2H)

**Herstellbeispiel 11:** 5-Cyclopropyl-4-(dimethylamino)-2-{2-fluor-4-methyl-5-[(2,2,2-trifluorethyl)sulfinyl]phenyl}-2,4-dihydro-3H-1,2,4-triazol-3-on (Bsp.Nr.197)

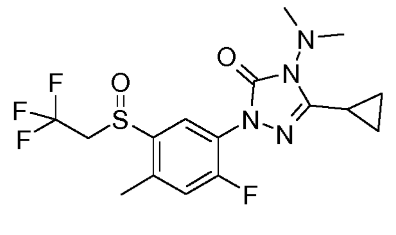

Zu einer Lösung aus 75,0 mg (0,19 mmol) 5-Cyclopropyl-4-(dimethylamino)-2-{2-fluor-4-methyl-5-[(2,2,2-trifluorethyl)sulfanyl]phenyl}-2,4-dihydro-3H-1,2,4-triazol-3-on in 15 mL Dichlormethan werden 38,7 mg (0,17 mmol) *meta*-Chlorperbenzoesäure (ca. 77%ig) gegeben. Die Reaktionsmischung wird über Nacht bei Raumtemperatur gerührt, sukzessive mit 40%iger wässriger Natriumbisulfit-Lösung und gesättigter wässriger Natriumbicarbonatlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 50 mg (100% Reinheit, 64% der Theorie) der Titelverbindung als farbloses Öl.
logP[a]: 2,60; logP[b]: 2,59; 1H-NMR (D6-DMSO, 400MHz) δ ppm: 7,91(d,1H), 7,49(d,1H), 4,27-4,00(m,2H), 2,96(s,3H), 2,50(s,6H), 2,40(s,3H), 2,06-1,98(m,1H), 1,00-0,95(m,2H), 0,94-0,87(m,2H)

**Herstellbeispiel 12:** 5-(Ethylsulfanyl)-2-{2-fluor-4-methyl-5-[(2,2,2-trifluorethyl)sulfanyl]phenyl}-4-methyl-2,4-dihydro-3H-1,2,4-triazol-3-on (Bsp.Nr.273)

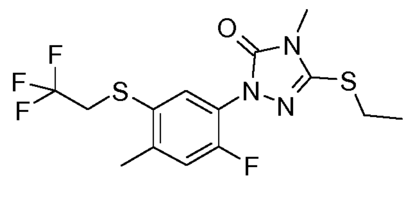

454,6 mg (1,5 mmol) 5-Brom-4-fluor-2-methylphenyl-2,2,2-trifluorethylsulfid, 286,6 mg (1,8 mmol) 5-(Ethylsulfanyl)-4-methyl-2,4-dihydro-3H-1,2,4-triazol-3-on (Synthese z.B. nach DE 2250572), 28,6 mg (0,15 mmol) Kupfer(I)-iodid, 42,7 mg (0,30 mmol) *trans-N,N'*-Dimethylcyclohexan-1,2-diamin (racemisch), 74,7 mg (0,45 mmol) Kaliumiodid sowie 621,9 mg (4,50 mmol) Kaliumcarbonat werden in 3 mL entgastem, trockenem Dioxan über Nacht bei 110 °C gerührt. Nach Abkühlen auf Raumtemperatur wird die Reaktionsmischung mit Essigester verdünnt und mit Essigester über Kieselgel filtriert. Nach säulenchromatographischer Reinigung an Kieselgel mittels MPLC mit Cyclohexan/Essigester als Laufmittel erhält man 470 mg (100% Reinheit, 82% der Theorie) der Titelverbindung als farbloses Öl.
logP[a]: 3,51; logP[b]: 3,44; 1H-NMR (D6-DMSO, 400MHz) δ ppm: 7,72(d,1H), 7,40(d,1H), 3,95(q,2H), 3,18(s,3H), 3,11(q,2H), 2,43(s,3H), 1,34(t,3H)

**Herstellbeispiel 13:** 5-(Ethylsulfanyl)-2-{2-fluor-4-methyl-5-[(2,2,2-trifluorethyl)sulfinyl]phenyl}-4-methyl-2,4-dihydro-3H-1,2,4-triazol-3-on (Bsp.Nr.274), 5-(Ethylsulfinyl)-2-{2-fluor-4-methyl-5-[(2,2,2-trifluorethyl)sulfanyl]phenyl}-4-methyl-2,4-dihydro-3H-1,2,4-triazol-3-on (Bsp.Nr.243) und 5-(Ethylsulfinyl)-2-{2-fluor-4-methyl-5-[(2,2,2-trifluorethyl)sulfinyl]phenyl}-4-methyl-2,4-dihydro-3H-1,2,4-triazol-3-on (Bsp.Nr.244)

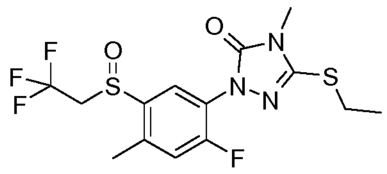 + 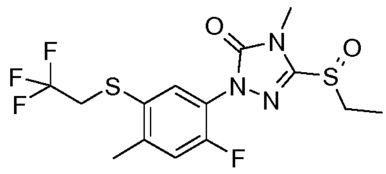 + 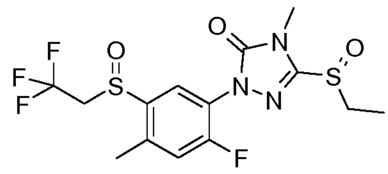

Zu einer Lösung aus 235 mg (0,62 mmol) 5-(Ethylsulfanyl)-2-{2-fluor-4-methyl-5-[(2,2,2-trifluorethyl)sulfanyl]phenyl}-4-methyl-2,4-dihydro-3H-1,2,4-triazol-3-on in 19 mL Dichlormethan werden bei 0 °C 165,7 mg (0,74 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhält man 93 mg (100% Reinheit, 38% der Theorie) 5-(Ethylsulfanyl)-2-{2-fluor-4-methyl-5-[(2,2,2-trifluorethyl)sulfinyl]phenyl}-4-methyl-2,4-dihydro-3H-1,2,4-triazol-3-on, 12 mg (95% Reinheit, 5% der Theorie) 5-(Ethylsulfinyl)-2-{2-fluor-4-methyl-5-[(2,2,2-trifluorethyl)sulfanyl]phenyl}-4-methyl-2,4-dihydro-3H-1,2,4-triazol-3-on und 102 mg (100% Reinheit, 40% der Theorie) 5-(Ethylsulfinyl)-2-{2-fluor-4-methyl-5-[(2,2,2-trifluorethyl)sulfinyl]phenyl}-4-methyl-2,4-dihydro-3H-1,2,4-triazol-3-on jeweils als farblose Öle, die bei Raumtemperatur langsam zu farblosen Feststoffen kristallisieren.

5-(Ethylsulfanyl)-2-{2-fluor-4-methyl-5-[(2,2,2-trifluorethyl)sulfinyl]phenyl}-4-methyl-2,4-dihydro-3H-1,2,4-triazol-3-on (Bsp.Nr.274): logP[a]: 2,42; logP[b]: 2,36; 1H-NMR (D6-DMSO, 400MHz) δ ppm: 7,98(d,1H), 7,52(d,1H), 4,26-4,20(m,1H), 4,09-4,03(m,1H), 3,19(s,3H), 3,12(q,2H), 2,42(s,3H), 1,34(t,3H) 5-(Ethylsulfinyl)-2-{2-fluor-4-methyl-5-[(2,2,2-trifluorethyl)sulfanyl]phenyl}-4-methyl-2,4-dihydro-3H-1,2,4-triazol-3-on (Bsp.Nr.243): logP[a]: 2,55; logP[b]: 2,51; 1H-NMR (D6-DMSO, 400MHz) δ ppm: 7,77(d,1H), 7,45(d,1H), 3,96(q,2H), 3,45(s,3H), 3,43-3,35(m,2H), 2,45(s,3H), 1,27(t,3H)
5-(Ethylsulfinyl)-2-{2-fluor-4-methyl-5-[(2,2,2-trifluorethyl)sulfinyl]phenyl}-4-methyl-2,4-dihydro-3H-1,2,4-triazol-3-on (Bsp.Nr.244): logP[a]: 1,69; logP[b]: 1,65; 1H-NMR (D6-DMSO, 400MHz) δ ppm: 8,02(d,1H), 7,56(d,1H), 4,28-4,19(m,1H), 4,08-4,00(m,1H), 3,45(s,3H), 3,43-3,34(m,2H), 2,43(s,3H), 1,29-1,25(m,3H)

**Herstellbeispiel 14:** 1-(4-Chlorphenyl)-4-{2-fluor-4-methyl-5-[(2,2,2-trifluorethyl)sulfanyl]phenyl}-1,4-dihydro-5H-tetrazol-5-on (Bsp.Nr.306)

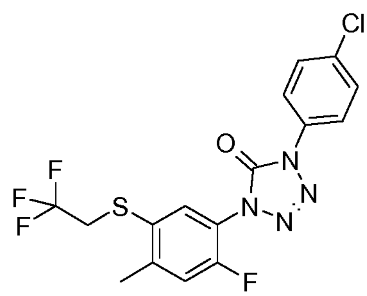

268,0 mg (1,0 mmol) {2-Fluor-4-methyl-5-[(2,2,2-trifluorethyl)sulfanyl]phenyl}boronsäure, 272 mg (2,00 mmol) 1-(4-Chlorphenyl)-1,4-dihydro-5H-tetrazol-5-on (z.B. nach J. Org. Chem. 1980, 45, 5130-5136), 272 mg (1,5 mmol) Kupfer(II)-acetat, 158 mg (2,0 mmol) Pyridin sowie 0,5 g aktiviertes 3A-Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 109 mg (98% Reinheit, 26% der Theorie) der Titelverbindung als farblosen Feststoff.
logP[a]: 4,75; logP[b]: 4,59; 1H-NMR (D6-DMSO, 400MHz) δ ppm: 7,98-7,93(m,3H), 7,72-7,69(m,2H), 7,59(d,1H), 3,98(q,2H), s, 3H unter DMSO-Signal

**Herstellbeispiel 15:** 1-(4-Chlorphenyl)-4-{2-fluor-4-methyl-5-[(2,2,2-trifluorethyl)sulfinyl]phenyl}-1,4-dihydro-5H-tetrazol-5-on (Bsp.Nr.307)

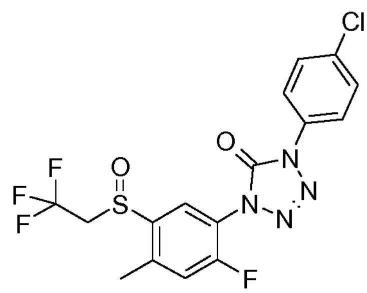

Zu einer Lösung aus 70,0 mg (0,17 mmol) 1-(4-Chlorphenyl)-4-{2-fluor-4-methyl-5-[(2,2,2-trifluorethyl)sulfanyl]phenyl}-1,4-dihydro-5H-tetrazol-5-on in 10 mL Dichlormethan werden bei 0 °C 37,5 mg (0,17 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhält man 55 mg (100% Reinheit, 76% der Theorie) der Titelverbindung als farblosen Feststoff.
logP[a]: 3,53; logP[b]: 3,49; 1H-NMR (D6-DMSO, 400MHz) δ ppm: 8,25(d,1H), 7,96-7,92(m,2H), 7,72-7,68(m,3H), 4,35-4,26(m,1H), 4,08-3,96(m,1H), 2,48(s,3H)

**Herstellbeispiel 16:** 1-{2-Fluor-4-methyl-5-[(2,2,2-trifluorethyl)sulfanyl]phenyl}-1,4-dihydro-5H-tetrazol-5-on (Bsp.Nr.299)

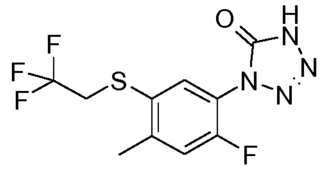

Eine Lösung aus 2,00 g (7,5 mmol) 4-Fluor-5-isocyanato-2-methylphenyl-2,2,2-trifluorethylsulfid in 10 mL Toluol wird bei Raumtemperatur mit 3,48 g (30,2 mmol) Trimethylsilylazid versetzt und über Nacht bei 100 °C gerührt. Nach Abkühlen auf 0 °C wird überschüssiges Trimethylsilylazid im Vakuum in eine Vorlage mit Natriumhydroxid und Ethanol evaporiert und anschließend mit Natriumnitritlösung vorsichtig versetzt. Der Rückstand wird in Essigester aufgenommen und mit gesättigter wässriger AmmoniumchloridLösung und verdünnter Salzsäure gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 869 mg (96% Reinheit, 36% der Theorie) der Titelverbindung als hellbraunen Feststoff.
logP[a]: 2,45; logP[b]: 0,90; 1H-NMR (D6-DMSO, 400MHz) δ ppm: 14,78(s,1H), 7,89(d,1H), 7,52(d,1H), 4,00(q,2H), 2,46(s,3H)

**Herstellbeispiel 17:** 1-{2-Fluor-4-methyl-5-[(2,2,2-trifluorethyl)sulfinyl]phenyl}-1,4-dihydro-5H-tetrazol-5-on (Bsp.Nr.303)

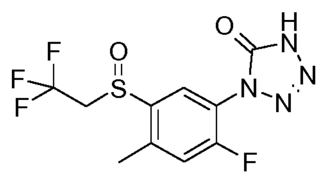

Zu einer Lösung aus 100 mg (0,32 mmol) 1-{2-Fluor-4-methyl-5-[(2,2,2-trifluorethyl)sulfanyl]phenyl}-1,4-dihydro-5H-tetrazol-5-on in 3 mL Acetonitril werden bei 0 °C 2,7 mg (0,008 mmol) Natriumwolframat(VI)-dihydrat und 34 mg (0,36 mmol) 3%ige wässrige Wasserstoffperoxidlösung gegeben. Die Reaktionsmischung wird über Nacht bei 0 °C -

Raumtemperatur gerührt, mit 40%iger wässriger Natriumbisulfitlösung (15 mL) versetzt und mit Dichlormethan extrahiert. Die vereinten organischen Phasen werden mit gesättigter wässriger Ammoniumchloridlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 91 mg (95% Reinheit, 82% der Theorie) der Titelverbindung als beigen Feststoff.
logP[a]: 1,59; logP[b]: 0,37; 1H-NMR (D6-DMSO, 400MHz) δ ppm: 8,13(d,1H), 7,64(d,1H), 4,30-4,21(m,1H), 4,12-4,01(m,1H), 2,46(s,3H)

**Herstellbeispiel 18:** 1-{2-Fluor-4-methyl-5-[(2,2,2-trifluorethyl)sulfanyl]phenyl}-4-methyl-1,4-dihydro-5H-tetrazol-5-on (Bsp.Nr.302)

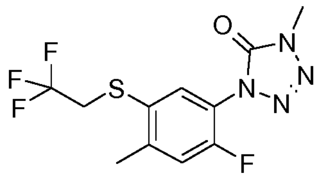

Zu einer Lösung von 183,5 mg (0,6 mmol) 1-{2-Fluor-4-methyl-5-[(2,2,2-trifluorethyl)sulfanyl]phenyl}-1,4-dihydro-5H-tetrazol-5-on in 3 mL N,N-Dimethylformamid werden bei 5 °C 138,2 mg (1,8 mmol) Kaliumcarbonat und 141,9 mg Methyliodid gegeben. Die Reaktionsmischung wird 1 h bei 5 °C-Raumtemperatur gerührt, dann auf eine Kieselgel-Kartusche gegeben und säulenchromatographisch an Kieselgel mittels MPLC mit Cyclohexan/Essigester als Laufmittel gereinigt. Man erhält 176 mg (95% Reinheit, 86% der Theorie) der Titelverbindung als gelbliches Öl.
logP[a]: 2,89; logP[b]: 2,86; 1H-NMR (D6-DMSO, 400MHz) δ ppm: 7,88(d,1H), 7,54(d,1H), 3,99(q,2H), 3,63(s,3H), 2,46(s,3H)

**Herstellbeispiel 19:** 1-{2-Fluor-4-methyl-5-[(2,2,2-trifluorethyl)sulfinyl]phenyl}-4-methyl-1,4-dihydro-5H-tetrazol-5-on (Bsp.Nr.301)

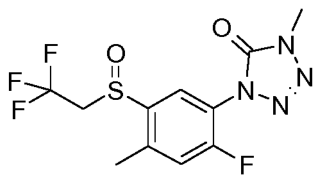

Zu einer Lösung aus 88,9 mg (0,28 mmol) 1-{2-Fluor-4-methyl-5-[(2,2,2-trifluorethyl)sulfanyl]phenyl}-4-methyl-1,4-dihydro-5H-tetrazol-5-on in 10 mL Dichlormethan werden bei 0 °C 61,8 mg (0,28 mmol) *meta*-Chlorperbenzoesäure (ca. 77%ig) gegeben. Die Reaktionsmischung wird 1 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhält man 76,5 mg (95% Reinheit, 78% der Theorie) der Titelverbindung als farbloses Öl.
logP[a]: 1,87; logP[b]: 1,82; 1H-NMR (D6-DMSO, 400MHz) δ ppm: 8,13(d,1H), 7,65(d,1H), 4,32-4,22(m,1H), 4,08-4,01(m,1H), 3,61(s,3H), 2,46(s,3H)

**Herstellbeispiel 20:** 1-{2-Fluor-4-methyl-5-[(2,2,2-trifluorethyl)sulfanyl]phenyl}-4-methoxy-1,5-dihydro-2H-pyrrol-2-on (Bsp.Nr.310)

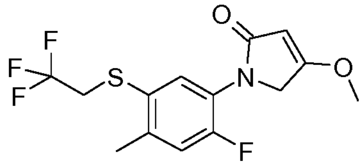

400,0 mg (1,3 mmol) 5-Brom-4-fluor-2-methylphenyl-2,2,2-trifluorethylsulfid, 182,1 mg (1,6 mmol) 4-Methoxy-3-pyrrolin-2-on, 16,3 mg (0,09 mmol) Kupfer(I)-iodid, 14,5 mg (0,17 mmol) *N,N'*-Dimethylethylendiamin sowie 364,8 mg (2,6 mmol) Kaliumcarbonat werden in 2 mL entgastem, trockenem Toluol über Nacht bei 115 °C gerührt. Nach Abkühlen auf Raumtemperatur werden 91,0 mg (0,81 mmol) 4-Methoxy-3-pyrrolin-2-on, 8,2 mg (0,04 mmol) Kupfer(I)-iodid und 7,9 mg (0,09 mmol) *N,N'*-Dimethylethylendiamin nachgesetzt und erneut über Nacht bei 115 °C gerührt. Nach Abkühlen auf Raumtemperatur wird die Reaktionsmischung mit Essigester/Cyclohexan (1:1) über Kieselgel filtriert und unter vermindertem Druck vom Lösungsmittel befreit. Nach säulenchromatographischer Reinigung an Kieselgel mittels MPLC mit Cyclohexan/Essigester als Laufmittel erhält man 155 mg (100% Reinheit, 35% der Theorie) der Titelverbindung als farblosen Feststoff.
logP[a]: 2,87; logP[b]: 2,81; 1H-NMR (D6-DMSO, 400MHz) $\delta$ ppm: 7,73(d,1H), 7,26(d,1H), 5,34(s,1H), 4,42(s,2H), 3,90(q,2H), 3,85(s,3H), 2,38(s,3H)

**Herstellbeispiel 21:** 1-{2-Fluor-4-methyl-5-[(2,2,2-trifluorethyl)sulfinyl]phenyl}-4-methoxy-1,5-dihydro-2H-pyrrol-2-on (Bsp.Nr.311)

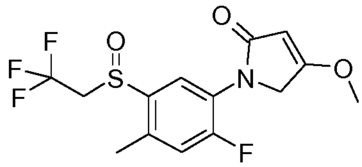

Zu einer Lösung aus 50 mg (0,15 mmol) 1-{2-Fluor-4-methyl-5-[(2,2,2-trifluorethyl)sulfanyl]phenyl}-4-methoxy-1,5-dihydro-2H-pyrrol-2-on und 10 mL Dichlormethan werden bei 0 °C 33,4 mg (0,15 mmol) *meta*-Chlorperbenzoesäure (ca. 77%ig) gegeben. Die Reaktionsmischung wird 2 h bei 0 °C gerührt, sukzessive mit 40%iger wässriger Natriumhydrogensulfitlösung und gesättigter wässriger Natriumbicarbonatlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 44 mg (99% Reinheit, 83% der Theorie) der Titelverbindung als farbloses Öl.
logP[a]: 1,83; logP[b]: 1,84; 1H-NMR (D6-DMSO, 400MHz) $\delta$ ppm: 8,06(d,1H), 7,37(d,1H), 5,38(s,1H), 4,48-4,44(m,2H), 4,20-4,13(m,1H), 4,06-3,94(m,1H), 3,86(s,3H), 2,38(s,3H)

**Herstellbeispiel 22:** 4-(Cyclopentyloxy)-1-{2-fluor-4-methyl-5-[(2,2,2-trifluorethyl)sulfanyl]phenyl}-1,5-dihydro-2H-pyrrol-2-on (Bsp.Nr.329)

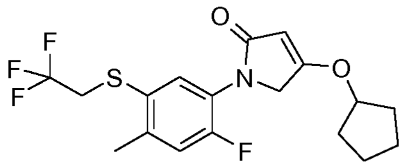

150,0 mg (0,45 mmol) 1-{2-Fluor-4-methyl-5-[(2,2,2-trifluorethyl)sulfanyl]phenyl}-4-methoxy-1,5-dihydro-2H-pyrrol-2-on, 231,2 mg (2,68 mmol) Cyclopentanol und 4,3 mg (0,05 mmol) Methansulfonsäure werden in 3 mL Toluol gelöst, mit aktiviertem 4A-Molsieb versetzt und über Nacht bei 110 °C gerührt. Nach Abkühlen auf Raumtemperatur wird die Reaktionsmischung mit Toluol über Kieselgel filtriert und unter vermindertem Druck vom Lösungsmittel befreit. Nach säulenchromatographischer Reinigung an Kieselgel mittels MPLC mit Cyclohexan/Essigester als Laufmittel erhält

man 81,2 mg (100% Reinheit, 47% der Theorie) der Titelverbindung als braunes Öl.
logP[a]: 4,20; logP[b]: 4,06; 1H-NMR (D6-DMSO, 400MHz) δ ppm: 7,72(d,1H), 7,71(d,1H), 5,28(s,1H), 4,80-4,76(m,1H), 4,37(s,2H), 3,91(q,2H), 2,37(s,3H), 1,96-1,89(m,2H), 1,80-1,58(m,6H)

**Herstellbeispiel 23:** 4-(Cyclopentyloxy)-1-{2-fluor-4-methyl-5-[(2,2,2-trifluorethyl)sulfinyl]phenyl}-1,5-dihydro-2H-pyrrol-2-on (Bsp.Nr.336)

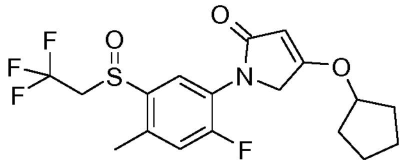

Zu einer Lösung aus 65 mg (0,17 mmol) 4-(Cyclopentyloxy)-1-{2-fluor-4-methyl-5-[(2,2,2-trifluorethyl)sulfanyl]phenyl}-1,5-dihydro-2H-pyrrol-2-on in 4 mL Dichlormethan werden bei 0 °C 40,3 mg (0,17 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 64,7 mg (99% Reinheit, 95% der Theorie) der Titelverbindung als gelben Feststoff.
logP[a]: 2,97; logP[b]: 2,91; 1H-NMR (D6-DMSO, 400MHz) δ ppm: 8,07(d,1H), 7,36(d,1H), 5,33(s,1H), 4,81-4,77(m,1H), 4,04(s,2H), 4,19-4,10(m,1H), 4,00-3,91(m,1H), 2,37(s,3H), 1,98-1,90(m,2H), 1,81-1,76(m,2H), 1,73-1,58(m,4H)

**Herstellbeispiel 24:** 1-{2-Fluor-4-methyl-5-[(2,2,2-trifluorethyl)sulfanyl]phenyl}-3,4-dimethyl-1H-pyrrol-2,5-dion (Bsp.Nr.339)

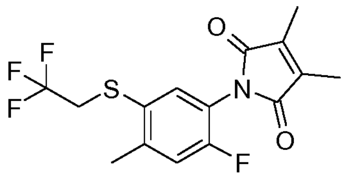

100 mg (0,42 mmol) 2-Fluor-4-methyl-5-[(2,2,2-trifluorethyl)sulfanyl]anilin werden in 15ml Essigsäure mit 60 mg (0,48 mmol) 3,4-Dimethylfuran-2,5-dion 4 h unter Rückfluss erhitzt . Nach der Zugabe von 50 ml Wasser wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck werden 100 mg Rohprodukt erhalten. Nach säulenchromatographischer Aufreinigung mittels MPLC über RP(C-18) mit Wasser/Acetonitril erhält man 30 mg (100 % Reinheit, 20,7 % der Theorie) Produkt als weiße, wachsähnliche Substanz.
logP[a]: 3,60; logP[b]: 3,61; 1H-NMR (D6-DMSO, 400MHz) δ ppm: 7,63-7,62(m,1H), 7,41-7,38(m,1H), 3,95-3,88(q,2H), 2,43(s,3H), 2,00(s,6H)

**Herstellbeispiel 25:** 2,2,2-Trifluor-N-[3-{2-fluor-4-methyl-5-[(2,2,2-trifluorethyl)sulfanyl]phenyl}-4,5-dimethyl-1,3-thiazol-2(3H)-yliden]ethanamin (Bsp.Nr.343)

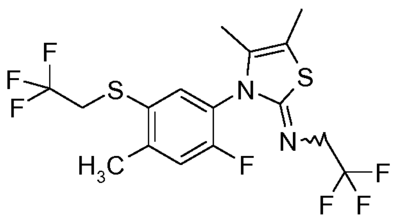

100 mg (0,26 mmol) 1-{2-Fluor-4-methyl-5-[(2,2,2-trifluorethyl)sulfanyl]phenyl}-3-(2,2,2-trifluorethyl)thioharnstoff werden in 1 ml Toluol vorgelegt und mit 77 mg (0,51 mmol) 3-Brom-2-butanon versetzt und 4 h bei Rückfluss gerührt. Nach Abkühlen wird das Reaktionsgemisch unter Vakuum von Lösungsmittel befreit. Der Rückstand wird mittels MPLC über RP(C-18) mit Wasser/Acetonitril aufgereinigt. Die isolierte Fraktion wird mittels MPLC über RP(C-18) mit Wasser/Acetonitril/0,1% Ameisensäure aufgereinigt. So werden 14 mg (100% Reinheit, 14% d. Th.) der Titelverbindung isoliert.

logP[a]: 3,02; logP[b]: 4,91; 1H-NMR (D6-DMSO, 400MHz) δ ppm: 7,63(d,1H), 7,37 (d,1H), 4,08-4,02(m,1H), 3,95-3,90 (m,1H), 3,64-3,59(m,2H), 2,41(s,3H), 2,11(s,3H), 1,71(s,3H)

**Herstellbeispiel 26:** 2,2,2-Trifluor-N-[3-{2-fluor-4-methyl-5-[(2,2,2-trifluorethyl)sulfinyl]phenyl}-4,5-dimethyl-1,3-thiazol-2(3H)-yliden]ethanamin (Bsp.Nr.342)

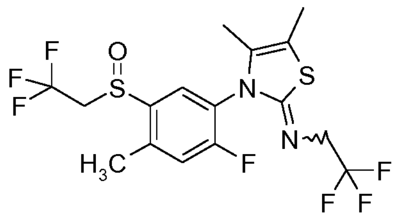

180 mg (0,45 mmol) 1-{2-Fluor-4-methyl-5-[(2,2,2-trifluorethyl)sulfinyl]phenyl}-3-(2,2,2-trifluorethyl)thioharnstoff werden in 2 ml Toluol vorgelegt und mit 137 mg (0,91 mmol) 3-Brom-2-butanon versetzt und 8 h bei Rückfluss gerührt. Nach Abkühlen wird das Reaktionsgemisch unter Vakuum vom Lösungsmittel befreit. Nach säulenchromatographischer Aufreinigung mittels MPLC über RP(C-18) mit Wasser/Acetonitril/0,1% Ameisensäure werden 45 mg (100% Reinheit, 22% d. Th.) der Titelverbindung isoliert.

logP[a]: 2,07; logP[b] 3,67; 1H-NMR (D6-DMSO, 400MHz) δ ppm: 7,81(m,1H), 7,52(d,1H), 4,36-4,17(m,2H), 3,89-3,56(m, 2H), 2,45-2,44(m,3H), 2,13-2,12(m,3H), 1,77-1,74(m,3H)

**Herstellbeispiel 27**: 5-Cyclopentyl-4-cyclopropyl-2-{2-fluor-4-methyl-5-[(2,2,2-trifluorethyl)sulfanyl]phenyl}-2,4-dihydro-3H-1,2,4-triazol-3-on (Bsp. Nr. 358)

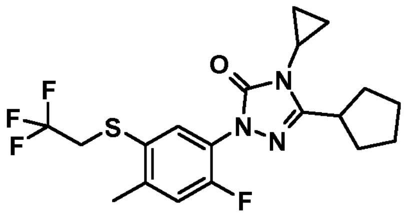

454,6 mg (1,5 mmol) 5-Brom-4-fluor-2-methylphenyl-2,2,2-trifluorethylsulfid, 304,0 mg (1,58 mmol) 5-Cyclopentyl-4-cyclopropyl-2,4-dihydro-3H-1,2,4-triazol-3-on, 28,6 mg (0,15 mmol) Kupfer(I)-iodid, 42,7 mg (0,30 mmol) *trans-N,N'*-Dimethylcyclohexan-1,2-diamin (racemisch), 74,7 mg (0,45 mmol) Kaliumiodid sowie 621,9 mg (4,50 mmol) Kaliumcarbonat werden in 3 mL entgastem, trockenem Dioxan über Nacht bei 110 °C gerührt. Nach Abkühlen auf Raumtemperatur wird die Reaktionsmischung mit Essigester verdünnt, mit Essigester über Kieselgel filtriert und konzentriert. Nach säulenchromatographischer Reinigung an Kieselgel mittels MPLC mit Cyclohexan/Essigester als Laufmittel erhält man 502 mg (99% Reinheit, 80% der Theorie) der Titelverbindung als gelbes Öl.

logP[a]: 4.18; logP[b]: 4.20; $^1$H-NMR (D$^6$-DMSO, 400MHz) δ ppm: 7,66(d,1H), 7,36(d,1H), 3,92(q,2H), 3,31-3,23(m,1H), 2,91-2,86(m,1H), 2,42(s,3H), 2,08-2,00(m,2H), 1,86-1,77(m,2H), 1,75-1,60(m,4H), 1,02-0,98(m,4H)

**Herstellbeispiel 28:** 2-{2-Fluor-4-methyl-5-[(2,2,2-trifluorethyl)sulfanyl]phenyl} -4-methyl-5-(methylsulfonyl)-2,4-dihydro-3H-1,2,4-triazol-3-on (Bsp. Nr. 420)

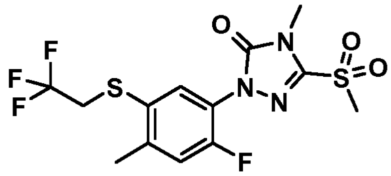

268,0 mg (1,0 mmol) {2-Fluor-4-methyl-5-[(2,2,2-trifluorethyl)sulfanyl]phenyl}boronsäure, 354 mg (2,00 mmol) 4-Methyl-5-(methylsulfonyl)-2,4-dihydro-3H-1,2,4-triazol-3-on, 272 mg (1,5 mmol) Kupfer(II)-acetat, 158 mg (2,0 mmol) Pyridin sowie 0,5 g aktiviertes 3Å-Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 41 mg (100% Reinheit, 10% der Theorie) der Titelverbindung als farblosen Feststoff.

logP[a]: 2.70; logP[b]: 2.77; $^1$H-NMR (D$^6$-DMSO, 400MHz) $\delta$ ppm: 7,80(d,1H), 7,48(d,1H), 3,95(q,2H), 3,57(s,3H), 3,47(s,3H), 2,46(s,3H)

**Herstellbeispiel 29:** 2-{2-Fluor-4-methyl-5-[(2,2,2-trifluorethyl)sulfinyl]phenyl} -4-methyl-5-(methylsulfonyl)-2,4-dihydro-3H-1,2,4-triazol-3-on (Bsp. Nr. 421)

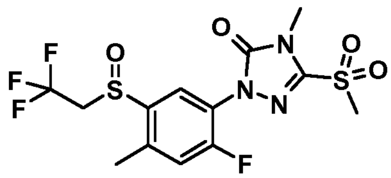

Zu einer Lösung von 30,0 mg (0,075 mmol) 2-{2-Fluor-4-methyl-5-[(2,2,2-trifluorethyl)sulfanyl]phenyl}-4-methyl-5-(methylsulfonyl)-2,4-dihydro-3H-1,2,4-triazol-3-on in 10 mL Dichlormethan werden bei 0 °C 17,7 mg (0,079 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 29 mg (96% Reinheit, 89% der Theorie) der Titelverbindung als farblosen Feststoff.

logP[a]: 1.88; logP[b]: 1.84; $^1$H-NMR (D$^6$-DMSO, 400MHz) $\delta$ ppm: 8,05(d,1H), 7,59(d,1H), 4,30-4,24(m,1H), 4,06-4,00(m,1H), 3,57(s,3H), 3,47(s,3H), 2,44(s,3H)

**Herstellbeispiel 30:** 5-Brom-2-{2-fluor-4-methyl-5-[(2,2,2-trifluorethyl)sulfanyl]phenyl}-4-isopropyl-2,4-dihydro-3H-1,2,4-triazol-3-on (Bsp. Nr. 439)

Stufe 1: *5-Brom-4-isopropyl-2,4-dihydro-3H-1,2,4-triazol-3-on*

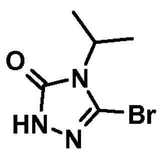

Eine Lösung von 412 mg (10,3 mmol) Natriumhydroxid und 936 mg (7,4 mmol) 4-Isopropyl-2,4-dihydro-3H-1,2,4-triazol-3-on in Wasser wird mit 1,18 g (7,4 mmol) Brom versetzt und über Nacht bei Raumtemperatur gerührt. Der ausgefallene gelbe Feststoff wird filtriert, mit Wasser gewaschen und auf einer Tonplatte getrocknet. Der erhaltene Feststoff wird in Natriumhydrogensulfit-Lösung verrührt, filtriert, mit Wasser gewaschen und erneut auf einer Tonplatte getrocknet. Man erhält 722 mg (89% Reinheit, 42% der Theorie) der Titelverbindung als gelblichen Feststoff.

logP[a]: 0,86; logP[b]: 0,83; $^1$H-NMR (D$^6$-DMSO, 400MHz) $\delta$ ppm: 12,03(s,1H), 4,27-4,21(m,1H), 1,40(d,6H)

Stufe 2: *5-Brom-2-{2-fluor-4-methyl-5-[(2,2,2-trifluorethyl)sulfanyl]phenyl}-4-isopropyl-2,4-dihydro-3H-1,2,4-triazol-3-on (Bsp. Nr. 439)*

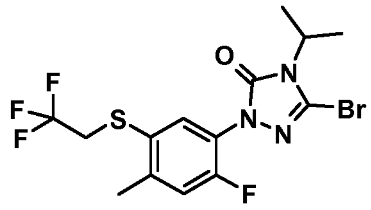

402,0 mg (1,5 mmol) {2-Fluor-4-methyl-5-[(2,2,2-trifluorethyl)sulfanyl]phenyl}boronsäure, 309 mg (1,5 mmol) 5-Brom-4-isopropyl-2,4-dihydro-3H-1,2,4-triazol-3-on, 409 mg (2,25 mmol) Kupfer(II)-acetat, 237 mg (3,0 mmol) Pyridin sowie 1,0 g aktiviertes 3Å-Molsieb werden in 5 mL trockenem Dichlormethan 4 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 38 mg (97% Reinheit, 6% der Theorie) der Titelverbindung als farbloses Öl.
logP[a]: 3.83; logP[b]: 3.80; $^1$H-NMR (D$^6$-DMSO, 400MHz) $\delta$ ppm: 7,75(d,1H), 7,41(d,1H), 4,43-4,31(m,1H), 3,96(q,2H), 2,43(s,3H), 1,47(d,6H)

**Herstellbeispiel 31:** 5-Brom-2-{2-fluor-4-methyl-5-[(2,2,2-trifluorethyl)sulfinyl]phenyl}-4-isopropyl-2,4-dihydro-3H-1,2,4-triazol-3-on (Bsp. Nr. 442)

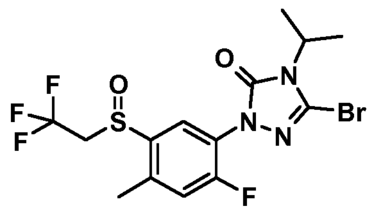

Zu einer Lösung von 28,0 mg (0,065 mmol) 5-Brom-2-{2-fluor-4-methyl-5-[(2,2,2-trifluorethyl)sulfanyl]phenyl}-4-isopropyl-2,4-dihydro-3H-1,2,4-triazol-3-on in 10 mL Dichlormethan werden bei 0 °C 14,7 mg (0,065 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 30 mg (98% Reinheit, 100% der Theorie) der Titelverbindung als farbloses Öl.
logP[a]: 2.68; logP[b]: 2.62; $^1$H-NMR (D$^6$-DMSO, 400MHz) $\delta$ ppm: 8,00(d,1H), 7,53(d,1H), 4,40-4,31(m,1H), 4,30-4,19(m,1H), 4,12-4,00(m,1H), 2,42(s,3H), 1,49-1,47(m,6H)

**Herstellbeispiel 32:** 1-{2-Fluor-4-methyl-5-[(2,2,2-trifluorethyl)sulfanyl]phenyl}-4-methyl-1,4-dihydro-5H-tetrazol-5-thion (Bsp. Nr. 447)

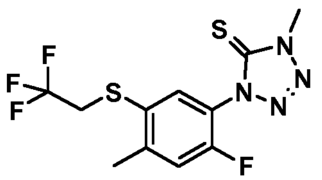

Zu einer Lösung von 100,0 mg (0,31 mmol) 1-{2-Fluor-4-methyl-5-[(2,2,2-trifluorethyl)sulfanyl]phenyl}-4-methyl-1,4-dihydro-5H-tetrazol-5-on in 10 mL Toluol werden 377 mg (0,93 mmol) 2,4-Bis(4-methoxyphenyl)-1,3,2,4-dithiadiphosphetan-2,4-disulfid (Lawessons Reagenz) gegeben. Die Reaktionsmischung wird über Nacht unter Argon zum

Rückfluss erhitzt, dann konzentriert. Nach säulenchromatographischer Reinigung an Kieselgel mittels MPLC mit Cyclohexan/Essigester als Laufmittel erhält man 73 mg (94% Reinheit, 65% der Theorie) der Titelverbindung als farblosen Feststoff.
logP[a]: 3.51; logP[b]: 3.49; $^1$H-NMR (D$^6$-DMSO, 400MHz) δ ppm: 7,96(d,1H), 7,58(d,1H), 3,99(q,2H), 3,93(s,3H), 2,49(s,3H)

**Herstellbeispiel 33:** 3-{2-Fluor-4-methyl-5-[(2,2,2-trifluorethyl)sulfanyl]phenyl}-5-methyl-1,3,4-oxadiazol-2(3H)-on (Bsp. Nr. 448)

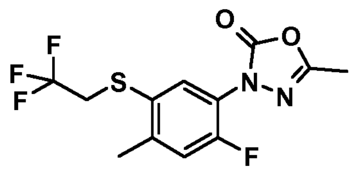

268,0 mg (1,0 mmol) {2-Fluor-4-methyl-5-[(2,2,2-trifluorethyl)sulfanyl]phenyl}boronsäure, 200 mg (2,00 mmol) 5-Methyl-1,3,4-oxadiazol-2(3H)-on, 272 mg (1,5 mmol) Kupfer(II)-acetat, 158 mg (2,0 mmol) Pyridin sowie 0,5 g aktiviertes 3Å-Molsieb werden in 5 mL trockenem Dichlormethan 3 d bei Raumtemperatur gerührt. Die Reaktionsmischung wird auf Kieselgur adsorbiert und säulenchromatographisch an Kieselgel mittels MPLC mit Cyclohexan/Essigester als Laufmittel gereinigt. Man erhält 56 mg (98% Reinheit, 17% der Theorie) der Titelverbindung als farbloses Öl.
logP[a]: 3.11; logP[b]: 3.09; $^1$H-NMR (D$^6$-DMSO, 400MHz) δ ppm: 7,80(d,1H), 7,45(d,1H), 3,95(q,2H), 2,44(s,3H), 2,34(s,3H)

**Herstellbeispiel 34:** 3-{2-Fluor-4-methyl-5-[(2,2,2-trifluorethyl)sulfinyl]phenyl}-5-methyl-1,3,4-oxadiazol-2(3H)-on (Bsp. Nr. 451)

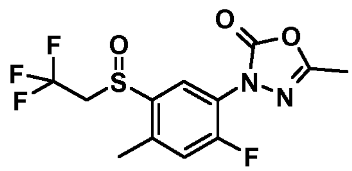

Zu einer Lösung von 36,0 mg (0,112 mmol) 3-{2-Fluor-4-methyl-5-[(2,2,2-trifluorethyl)sulfanyl]phenyl}-5-methyl-1,3,4-oxadiazol-2(3H)-on in 10 mL Dichlormethan werden bei 0 °C 26,3 mg (0,117 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Man erhält 40 mg (100% Reinheit, 100% der Theorie) der Titelverbindung als farblosen Feststoff.
logP[a]: 1.99; logP[b]: 2.02; $^1$H-NMR (D$^6$-DMSO, 400MHz) δ ppm: 8,05(d,1H), 7,56(d,1H), 4,31-4,19(m,1H), 4,07-3,94(m,1H), 2,42(s,3H), 2,35(s,3H)

**Herstellbeispiel 35:** 1-Ethyl-3-{2-fluor-4-methyl-5-[(2,2,2-trifluorethyl)sulfanyl]phenyl}-1,3-dihydro-2H-imidazol-2-on (Bsp. Nr. 486)

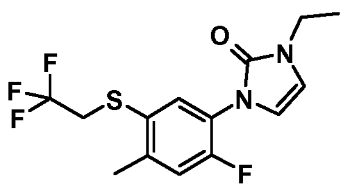

454,6 mg (1,5 mmol) 5-Brom-4-fluor-2-methylphenyl-2,2,2-trifluorethylsulfid, 185,0 mg (1,65 mmol) 1-Ethyl-1,3-dihydro-2H-imidazol-2-on, 28,6 mg (0,15 mmol) Kupfer(I)-iodid, 42,7mg (0,30 mmol) *trans-N,N'*-Dimethylcyclohexan-1,2-diamin (racemisch), 74,7 mg (0,45 mmol) Kaliumiodid sowie 621,9 mg (4,50 mmol) Kaliumcarbonat werden in 2,4 mL entgastem, trockenem Dioxan über Nacht bei 110 °C gerührt. Nach Abkühlen auf Raumtemperatur wird die Reaktionsmischung mit Essigester verdünnt und mit Essigester über Kieselgel filtriert. Nach säulenchromatographischer Reinigung an Kieselgel mittels MPLC mit Cyclohexan/Essigester als Laufmittel erhält man 167 mg (100% Reinheit, 33% der Theorie) der Titelverbindung als farbloses Öl.
logP[a]: 2.84; logP[b]: 2.79; $^1$H-NMR ($D^6$-DMSO, 400MHz) δ ppm: 7,72(d,1H), 7,36(d,1H), 6,77(d,1H), 6,73-6,72(m,1H), 3,94(q,2H), 3,60(q,2H), 2,41(s,3H), 1,22(t,3H)

**Herstellbeispiel 36:** 1-Ethyl-3-{2-fluor-4-methyl-5-[(2,2,2-trifluorethyl)sulfinyl]phenyl}-1,3-dihydro-2H-imidazol-2-on (Bsp. Nr. 487)

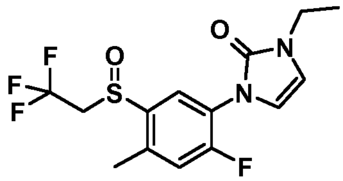

Zu einer Lösung von 84,0 mg (0,25 mmol) 1-Ethyl-3-{2-fluor-4-methyl-5-[(2,2,2-trifluorethyl)sulfanyl]phenyl}-1,3-dihydro-2H-imidazol-2-on in 10 mL Dichlormethan werden bei 0 °C 59,1 mg (0,26 mmol) *meta*-Chlorperbenzoesäure (ca. 77%ig) gegeben. Die Reaktionsmischung wird 2 h bei 0 °C gerührt, mit 1M Natronlauge gewaschen und unter vermindertem Druck vom Lösungsmittel befreit. Nach säulenchromatographischer Reinigung an Kieselgel mittels MPLC mit Cyclohexan/Essigester als Laufmittel erhält man 45 mg (95% Reinheit, 49% der Theorie) der Titelverbindung als farbloses Öl.
logP[a]: 1.83; logP[b]: 1.81; $^1$H-NMR ($D^6$-DMSO, 400MHz) δ ppm: 7,99(d,1H), 7,48(d,1H), 6,83-6,80(m,2H), 4,22-4,11(m,1H), 4,11-4,02(m,1H), 3,61(q,2H), 2,41(s,3H), 1,23(t,3H)

**Herstellbeispiel 37:** 5-Cyclopentyl-4-cyclopropyl-2-{2-fluor-4-methyl-5-[(2,2,2-trifluorethyl)sulfanyl]-phenyl}-2,4-dihydro-3H-1,2,4-triazol-3-thion (Bsp. Nr. 497)

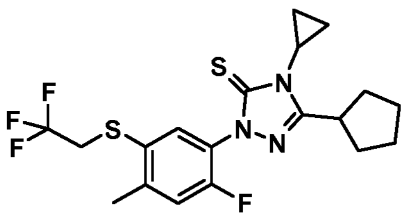

Zu einer Lösung von 100,0 mg (0,24 mmol) 5-Cyclopentyl-4-cyclopropyl-2-{2-fluor-4-methyl-5-[(2,2,2-trifluorethyl)sulfanyl]phenyl}-2,4-dihydro-3H-1,2,4-triazol-3-on in 10 mL Toluol werden 292 mg (0,72 mmol) 2,4-Bis(4-methoxyphenyl)-1,3,2,4-dithiadiphosphetan-2,4-disulfid (Lawessons Reagenz) gegeben. Die Reaktionsmischung wird über Nacht unter Argon zum Rückfluss erhitzt, dann mit Essigester verdünnt, über Kieselgel filtriert und konzentriert. Nach säulenchromatographischer Reinigung an Kieselgel mittels MPLC mit Cyclohexan/Essigester als Laufmittel erhält man 51 mg (98% Reinheit, 48% der Theorie) der Titelverbindung als farblosen Feststoff.
logP[a]: 4.68; logP[b]: 4.57; $^1$H-NMR ($D^6$-DMSO, 400MHz) δ ppm: 7,74(d,1H), 7,42(d,1H), 3,95(q,2H), 3,49-3,3,39(m,1H), 3,11-3,02(m,1H), 2,45(s,3H), 2,15-2,05(m,2H), 1,89-1,75(m,2H), 1,73-1,60(m,4H), 1,20-1,11(m,4H)

**Synthese von Anilinen der Formel (IVa), (IVb) und Zwischenstufen (X), (XI), (XII), (XIII), (XVI), (XVII), (XVIII), (XIX) und (XX)**

**2,2,2-Trifluor-*N*-(2-fluor-4-methylphenyl)acetamid (XIII-1)**

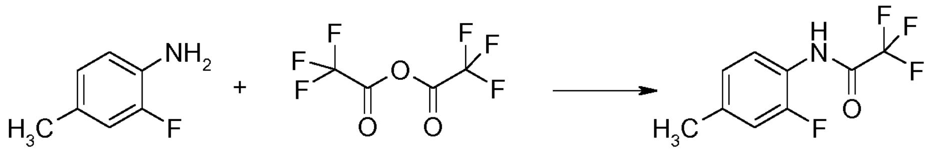

(XIV-1) (XIII-1)

27,5 g 2-Fluor-4-methylanilin werden bei 0 °C in 300 mL Dichlormethan vorgelegt, 26,7 g Triethylamin werden zugegeben und anschließend werden 50,8 g Trifluoressigsäureanhydrid zugetropft. Es wird 2 h bei 0 °C nachgerührt und anschließend einrotiert. Der Rückstand wird in Wasser aufgenommen und zweimal mit Ethylacetat extrahiert. Die vereinigten organischen Phasen werden über Natriumsulfat getrocknet, filtriert, und das Lösemittel wird im Vakuum entfernt. Es werden 49,0 g (100% d.Th.) der Titelverbindung erhalten.
logP[a]: 2,40

Analog erhalten wurde:

***N*-(4-Chlor-2-fluorphenyl)-2,2,2-trifluoracetamid (XIII-2)**

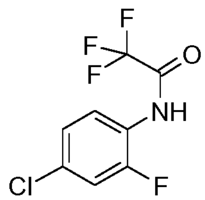

(XIII-2)

logP[a]: 2,53; logP[b]: 2,40; 1H-NMR (D6-DMSO, 400MHz) $\delta$ ppm: 11.29(s,1H), 7,62(dd,1H), 7,55(dd,1H), 7,37(dd,1H)

**4-Fluor-2-methyl-5-[(trifluoracetyl)amino]benzolsulfonylchlorid (XII-1)**

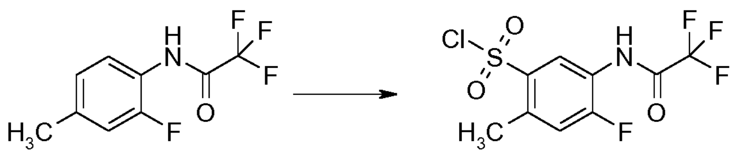

(XIII-1) (XII-1)

258 g Chlorsulfonsäure werden vorgelegt und bei Raumtemperatur werden 49 g 2,2,2-Trifluor-N-(2-fluor-4-methylphenyl)acetamid in Portionen zugesetzt. Bei Raumtemperatur wird noch 16 h nachgerührt. Die Mischung wird unter Rühren auf Eis gegeben und zweimal mit Ethylacetat extrahiert. Die vereinigten organischen Phasen werden über Natriumsulfat getrocknet, filtriert, und das Lösemittel wird im Vakuum entfernt. Es werden 70,8 g des Chlorsulfonyls (XII-1) erhalten. Das Rohprodukt wird sofort weiter umgesetzt.

***N,N'***-**[Disulfanediylbis(6-fluor-4-methylbenzol-3,1-diyl)]bis(2,2,2-trifluoracetamid) (XI-1)**

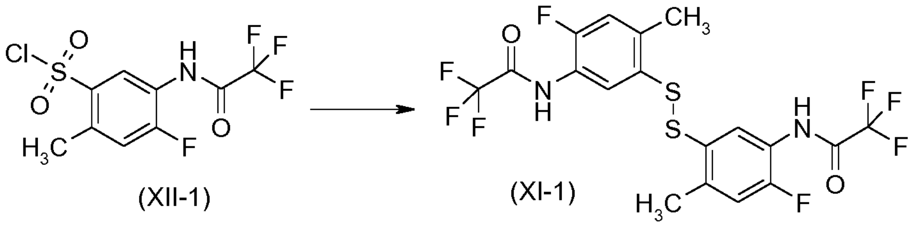

(XII-1) (XI-1)

298,8 g Natriumiodid werden in 1000 mL Trifluoressigsäure gelöst und 70,8 g 4-Fluor-2-methyl-5-[(trifluoracetyl)amino]benzolsulfonylchlorid werden bei Raumtemperatur zugegeben. Es wird 16 h bei Raumtemperatur nachgerührt und anschließend das Lösemittel im Vakuum entfernt. Der Rückstand wird mit Wasser verrührt und abgesaugt. Es werden 62,3 g (86% d.Th.) der Titelverbindung als Feststoff erhalten.
logP[a]: 4,41

Analog erhalten wurde:

***N,N'***-**[Disulfanediylbis(4-chlor-6-fluorbenzol-3,1-diyl)]bis(2,2,2-trifluoracetamid) (XI-2)**

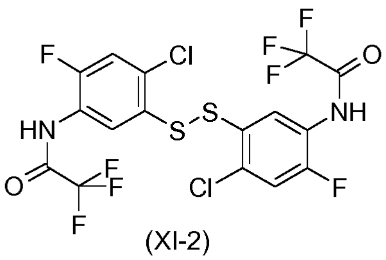

(XI-2)

logP[a]: 4,60; logP[b]: 3,82; 1H-NMR (D6-DMSO,400MHz) $\delta$ ppm: 11,44(s,2H), 7,95(d,2H), 7,83(d,2H)

**2,2,2-Trifluor-*N*-{2-fluor-4-methyl-5-[(2,2,2-trifluorethyl)sulfanyl]phenyl}acetamid (X-1)**

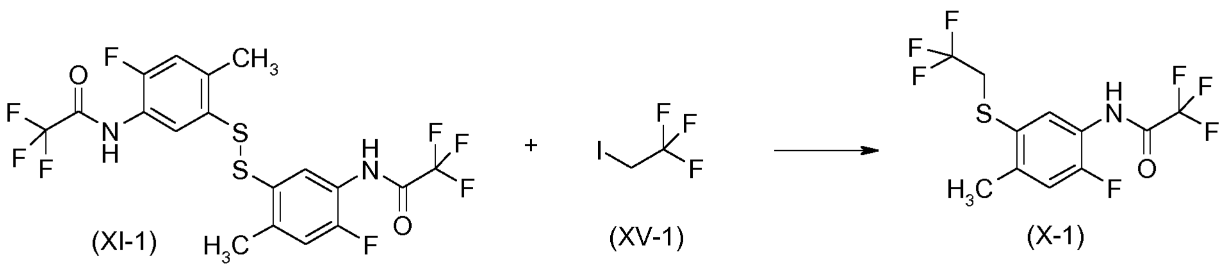

(XI-1) (XV-1) (X-1)

3,4 g *N,N'*-[Disulfanediylbis(6-fluor-4-methylbenzol-3,-diyl)]bis(2,2,2-trifluoracetamid) werden in 150 ml *N,N*-Dimethylformamid gelöst und mit 1,86 g Kaliumcarbonat, 3,11 g 1,1,1-Trifluoriodethan, 2,39 g Rongalit und einigen Tropfen Wasser versetzt. Die Reaktionsmischung wird 16 h bei Raumtemperatur gerührt. Die Hauptmenge des *N,N*-Dimethylformamids wird im Vakuum abdestilliert. Der Rückstand wird in Wasser aufgenommen und zweimal mit Ethylacetat extrahiert. Die vereinigten organischen Phasen werden über Natriumsulfat getrocknet, filtriert, und anschließend wird das Lösemittel im Vakuum entfernt. Es werden 4,48 g (90% d.Th.) der Titelverbindung erhalten.
logP[a]: 3,31

Analog erhalten wurde:

***N*-{4-Chlor-2-fluor-5-[(2,2,2-trifluorethyl)sulfanyl]phenyl}-2,2,2-trifluoracetamid (X-2)**

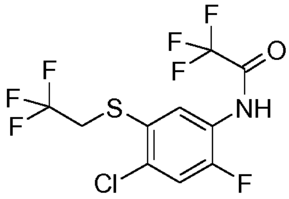

(X-2)

logP[a]: 3,34; logP[b]: 3,14; 1H-NMR (D6-DMSO,400MHz) δ ppm: 11,47(bs,1H), 7,85(d,1H), 7,76(d,1H), 4,09(q,2H)

**4-Chlor-2-fluor-5-[(2,2,2-trifluorethyl)sulfanyl]anilin (IVa-2)**

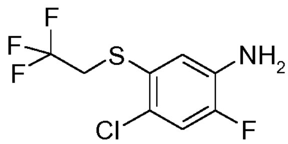

11,0 g (30,9 mmol) *N*-{4-Chlor-2-fluor-5-[(2,2,2-trifluorethyl)sulfanyl]phenyl}-2,2,2-trifluoracetamid in 150 ml Dioxan werden vorsichtig zu einer Lösung aus 10,3 ml (186 mmol) Schwefelsäure (96%ig) in 100 ml Wasser zu gegeben. Anschließend wird das Reaktionsgemisch über Nacht refluxiert. Nach Abkühlen wird die Lösung mit einer gesättigten Natriumhydrogencarbonatlösung und etwas Natriumcarbonat auf pH 7 gebracht und dreimal mit Essigester extrahiert. Die vereinten organischen Phasen werden mit einer gesättigten Natriumchloridlösung gewaschen, über Natriumsulfat getrocknet, filtriert und eingeengt. Der Rückstand enthält 8,27 g (96% Reinheit, 99% d.Th.) der Titelverbindung als schwarzes Öl/Feststoff-Gemisch.
logP[a]: 3,02; logP[b]: 3,00; 1H-NMR (D6-DMSO, 400MHz) δ ppm: 7,27(d,1H), 7,04(d,1H), 5,46(bs,2H), 3,85(q,2H)

Analog erhalten wurde:

**4-Brom-2-fluor-5-[(2,2,2-trifluorethyl)sulfanyl]anilin (IVa-14)**

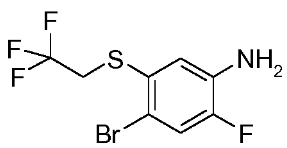

logP[a]: 3,15; logP[b]: 3,12; 1H-NMR (D6-DMSO, 400MHz) δ ppm: 7,37(d,1H), 7,05(d,1H), 5,48(breit,2H), 3,86(q,2H)

**S-(5-Acetamido-4-fluor-2-methylphenyl)ethanthioat (XVII-1)**

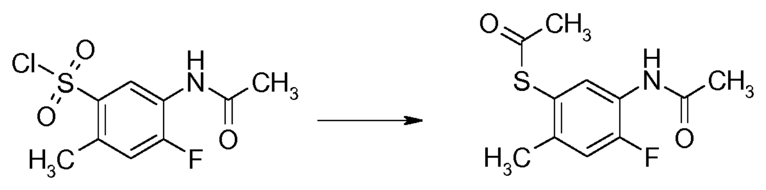

(XII-1) (XVII-1)

99,3 g 5-Acetamido-4-fluor-2-methylbenzolsulfonylchlorid werden in 700 mL Eisessig suspendiert, mit 0,9 g Iod und 38,7 g rotem Phosphor versetzt und 5 h bei Rückfluß nachgerührt. Nach Abkühlen wird der Feststoff abfiltriert und das Filtrat einrotiert. Der Rückstand wird mit Wasser verrührt und abgesaugt. 57,6 g (67% d.Th.) der Titelverbindung werden als Feststoff erhalten.
logP[a]: 1,78

**5-Amino-4-fluor-2-methylbenzolthiol (XVI-1)**

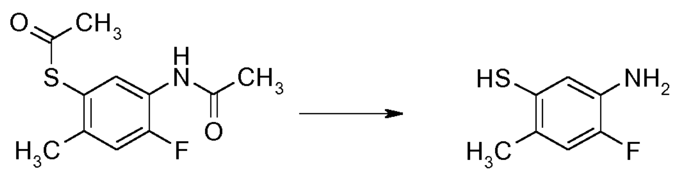

(XVII-1) (XVI-1)

57,4 g S-(5-Acetamido-4-fluor-2-methylphenyl)ethanthioat werden in 750 mL Wasser und 96,6 g Kaliumhydroxid gelöst. Die Reaktionsmischung wird 16 h bei Rückfluß gekocht. Nach Abkühlen wird die Lösung mit Salzsäure auf pH 2-3 gestellt und der ausgefallene Feststoff abgesaugt. 35,8 g (94% d.Th.) der Titelverbindung werden als Feststoff erhalten.
logP[a]: 3,70

**2-Fluor-4-methyl-5-[(2,2,2-trifluorethyl)sulfinyl]anilin (IVb-1)**

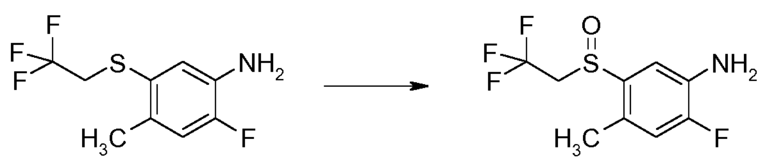

(IVa-1) (IVb-1)

5,00 g (0,21 mmol) 2-Fluor-4-methyl-5-[(2,2,2-trifluorethyl)sulfanyl]anilin werden bei 0-4 °C in 100 ml Dichlormethan vorgelegt, 6,18 g (0,25 mmol) *meta*-Chlorperbenzoesäure werden dazu gegeben und das Reaktionsgemisch wird 2 h bei Raumtemperatur nachgerührt. Anschließend mit einer 33%igen Natriumthiosulfatlösung versetzt und zweimal mit Dichlormethan ausgeschüttelt. Die vereinigten organischen Phasen werden mit einer gesättigten Natriumcarbonatlösung gewaschen, über Natriumsulfat getrocknet, filtriert, und das Lösemittel wird im Vakuum entfernt. Der Rückstand enthält 5,10 g (90% Reinheit, 86% d.Th.) der Titelverbindung als braunes Öl.
logP[a]: 1,77; logP[b]: 1,72; 1H-NMR (D6-DMSO, 400MHz) δ ppm: 7,26(d,1H), 7,02(d,1H), 5,45(bs,2H), 4,08-3,95(m,1H), 3,88-3,75(m,1H), 2,19(s,3H)

**Synthese von Bromiden der Formel (VIIa)**

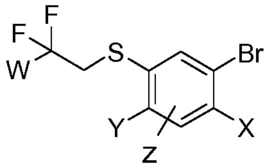

(VIIa)

**5-Brom-2-methylphenyl-2,2,2-trifluorethylsulfid (VIIa-7)**

Stufe 1: *5-Brom-2-methylbenzolsulfonylchlorid*

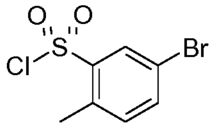

69,50 g (406,3 mmol) 4-Bromtoluol werden in 250 ml Dichlormethan vorgelegt und bei -5 - 5 °C tropfenweise mit 175,33 g (1,50 mol) Chlorsulfonsäure versetzt. Die Reaktionsmischung wird über Nacht unter Rühren auf Raumtemperatur gebracht, mit 1000 mL Eiswasser versetzt und mit Dichlormethan extrahiert. Die vereinigten organischen Phasen werden mit gesättigter wässriger Kochsalzlösung gewaschen, über Magnesiumsulfat getrocknet, filtriert und unter vermindertem Druck vom Lösungsmittel befreit. Man erhält 89,54 g (95% Reinheit, 78% der Theorie) der Titelverbindung als gelbe Flüssigkeit, die ohne weitere Aufreinigung weiter umgesetzt wird.
logP[a]: 3,73; logP[b]: 3,74; GC-MS: EI-Masse (m/z): 270 (1Cl,1Br) $[M]^+$; $^1$H-NMR ($CDCl_3$, 400 MHz): 8,19(d,1H), 7,73-7,72(m,1H), 7,31(d,1H), 2,73(s,3H); $^1$H-NMR ($CD_3CN$, 400 MHz): 8,18(d,1H), 7,88-7,85(m,1H), 7,46(d,1H), 2,71(s,3H)

Stufe 2: *4-Brom-2-[(3-brom-4-methylphenyl)disulfanyl]-1-methylbenzol*

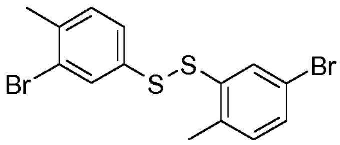

25,00 g (92,7 mmol) 5-Brom-2-methylbenzolsulfonylchlorid werden mit 145,7 g wässriger Iodwasserstoffsäure (57%ig, 649,2 mmol) unter kräftigem Rühren versetzt. Die Reaktionsmischung wird 3 Tage bei Raumtemperatur gerührt, dann mit 40%iger wässriger Natriumhydrogensulfitlösung versetzt. Der Feststoff wird abgesaugt, gründlich mit Wasser gewaschen und über Nacht auf einer Tonplatte getrocknet.

Man erhält 19,50 g (95% Reinheit, 99% der Theorie) der Titelverbindung als gelben Feststoff, der ohne weitere Aufreinigung weiter umgesetzt wird.
logP[a]: >7,36; logP[b]: >7,36; GC-MS: EI-Masse (m/z): 404 (2Br) $[M]^+$
1H-NMR (D6-DMSO): 7,56(d,2H), 7,46-7,43(m,2H), 7,26(d,2H), 2,35(s,6H)

Stufe 3: *5-Brom-2-methylphenyl-2,2,2-trifluorethylsulfid* (VIIa-7)

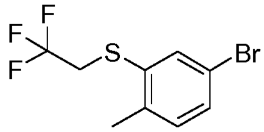

(VIIa-7)

27,70 g (68,5 mmol) 4-Brom-2-[(3-brom-4-methylphenyl)disulfanyl]-1-methylbenzol werden in 350 mL *N,N*-Dimethylformamid vorgelegt, mit 23,86 g (137,1 mmol) Natriumdithionit, 18,66 g (137,1 mmol) Rongalit® sowie 18,94 g (137,1 mmol) Kaliumcarbonat versetzt und auf 0 °C gekühlt. 31,65 g (150,8 mmol) 1,1,1-Trifluor-2-iodethan in 20 mL *N,N*-Dimethylformamid werden bei 0 °C tropfenweise zugegeben. Die Reaktionsmischung wird über Nacht unter Rühren auf Raumtemperatur gebracht, mit 500 mL Wasser versetzt und mit *tert*-Butyl-methylether extrahiert. Die vereinigten organischen Phasen werden mit Wasser, gesättigter wässriger Natriumbicarbonatlösung und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 30,01 g (93% Reinheit, 71% der Theorie) der Titelverbindung als farblose Flüssigkeit.
logP[a]: 4,29; logP[b]: 4,26; GC-MS: EI-Masse (m/z): 286 (1Br) $[M]^+$
1H-NMR (D6-DMSO, 400MHz) $\delta$ ppm: 7,70(d,1H), 7,39-7,36(m,1H), 7,21(d,1H), 4,09(q,2H), 2,30(s,3H)

Analog erhalten wurden:

**4-Brom-1-methoxy-2-[(2,2,2-trifluorethyl)sulfanyl]benzol (VIIa-8)**

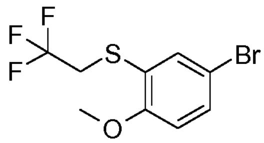

(VIIa-8)

logP[a]: 3,69; logP[b]: 3,81; GC-MS: EI-Masse (m/z): 302 (1Br) $[M]^+$
1H-NMR (D6-DMSO, 400MHz) $\delta$ ppm: 7,59(d,1H), 7,45-7,42(m,1H), 7,00(d,1H), 4,02(q,2H), 3,85(s,3H)

**1,4-Dibrom-2-fluor-5-[(2,2,2-trifluorethyl)sulfanyl]benzol (VIIa-14)**

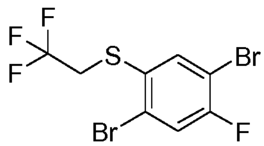

logP[a]: 4,39; logP[b]: 4,36; GC-MS: EI-Masse (m/z): 368 (2Br) $[M]^+$; $^1$H-NMR (D6-DMSO, 400MHz): 8,00(d,1H), 7,87(d,1H), 4,21(q,2H)

**4-Brom-2-[(2,2,2-trifluorethyl)sulfanyl]benzonitril (VIIa-9)**

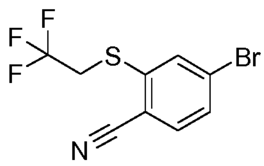

(VIIa-9)

24,0 g (86,4 mmol) Natriumhydrid (60%ig in Mineralöl) werden in 300 ml *N,N*-Dimethylformamid vorgelegt und bei 0 °C tropfenweise mit 10,0g (86,4 mmol) 2,2,2-Trifluorethanthiol versetzt. Die Reaktionsmischung wird bei 0 °C tropfenweise zu einer Lösung aus 14,4 g (72,0 mmol) 4-Brom-2-fluorbenzonitril in 100 mL *N,N*-Dimethylformamid gegeben und über Nacht unter Rühren auf Raumtemperatur gebracht. Die Reaktionsmischung wird in Wasser gegossen, mit gesättigter wässriger Ammoniumchloridlösung neutralisiert und mit *tert*-Butyl-methylether extrahiert. Die vereinigten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Der Rückstand wird mit Petrolether verrührt, abfiltriert und aus Diethylether umkristallisiert. Man erhält 17,6 g (99% Reinheit, 82% der Theorie) der Titelverbindung als farblosen Feststoff.
logP[a]: 3,21; logP[b]: 3,16; 1H-NMR (D6-DMSO, 400MHz) $\delta$ ppm: 8,12(d,1H), 7,83(d,1H), 7,72-7,69(m,1H), 4,33(q,2H)

Analog erhalten wurde:

**4-Brom-2-[(2,2,2-trifluorethyl)sulfanyl]-1-(trifluormethyl)benzol (VIIa-10)**

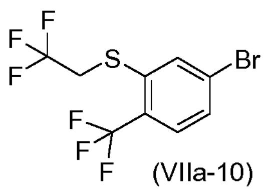

(VIIa-10)

logP[a]: 4,22; logP[b]: 4,20; $^1$H-NMR (D6-DMSO, 400MHz): 8,18(s,1H), 7,73-7,68(m,2H), 4,30(q,2H)

**4-Brom-5-fluor-2-[(2,2,2-trifluorethyl)sulfanyl]benzonitril (VIIa-18)**

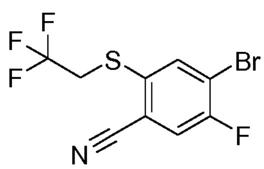

logP[a]: 3,32; logP[b]: 3,26; GC-MS: EI-Masse (m/z): 315 $[M]^+$; $^1$H-NMR (D6-DMSO, 400MHz): 8,29(d,1H), 8,12(d,1H), 4,26(q,2H)

**Synthese von Iodiden der Formel (VIIa)**

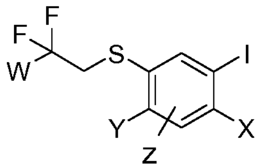

(VIIa)

**1-Fluor-2-iod-5-methyl-4-[(2,2,2-trifluorethyl)sulfanyl]benzol (VIIa-1-I)**

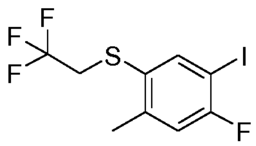

(VIIa-1-I)

10,0 g (33,0 mmol) 5-Brom-2-methylphenyl-2,2,2-trifluorethylsulfid, 9,89 g (66,0 mmol) Natriumiodid, 314 mg (1,65 mmol) Kupfer(I)-iodid und 469 mg (3,3 mmol) *trans-N,N'*-Dimethylcyclohexan-1,2-diamin (racemisch) wurden in 33 mL entgastem Dioxan über Nacht bei 110 °C gerührt. Erneut wurden 9,89 g (66,0 mmol) Natriumiodid, 314 mg (1,65 mmol) Kupfer(I)-iodid und 234 mg (1,65 mmol) *trans-N,N'*-Dimethylcyclohexan-1,2-diamin (racemisch) zugesetzt und über Nacht bei 110 °C gerührt. Erneut wurden 9,89 g (66,0 mmol) Natriumiodid und 314 mg (1,65 mmol) Kupfer(I)-iodid sowie 20 mL Dioxan zugesetzt und über Nacht bei 110 °C gerührt. Nach Abkühlen auf Raumtemperatur wurde mit Essigester verdünnt, über Kieselgur filtriert und konzentriert. Nach säulenchromatographischer Reinigung an Kieselgel mittels MPLC mit Cyclohexan/Essigester als Laufmittel erhält man 8,77 g (97% Reinheit, 74% der Theorie) der Titelverbindung als farbloses Öl.
logP[a]: 4,44; logP[b]: 4,44; GC-MS: EI-Masse (m/z): 350 $[M]^+$
1H-NMR (D6-DMSO, 400MHz): 7,97(d,1H), 7,24(d,1H), 3,96(q,2H), 2,35(s,3H)

Analog erhalten wurde:

**4-Iod-1-methyl-2-[(2,2,2-trifluorethyl)sulfanyl]benzol (VIIa-7-I)**

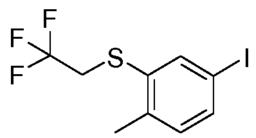

(VIIa-7-I)

logP[a]: 4,49; logP[b]: 4,48; GC-MS: EI-Masse (m/z): 332 $[M]^+$
1H-NMR (D6-DMSO, 400 MHz): 7,83(d,1H), 7,55-7,53(m,1H), 7,05(d,1H), 4,05(q,2H), 2,30(s,3H)

**Synthese von Boronsäuren der Formel (VIII)**

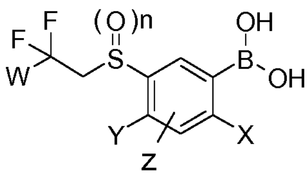

(VIII)

**{4-Methyl-3-[(2,2,2-trifluorethyl)sulfanyl]phenyl}boronsäure (VIIIa-7)**

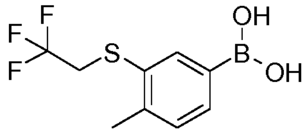

(VIIIa-7)

Stufe 1: *4,4,5,5-Tetramethyl-2-{4-methyl-3-[(2,2,2-trifluorethyl)sulfanyl]phenyl}-1,3,2-dioxaborolan*

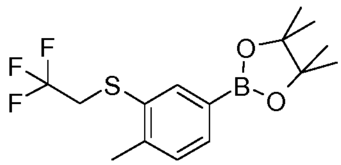

15,0 g (52,6 mmol) 5-Brom-2-methylphenyl-2,2,2-trifluorethylsulfid, 14,7 g (57,9 mmol) 4,4,4',4',5,5,5',5'-Octamethyl-2,2'-bi-1,2,3-dioxaborolan, 10,3 g (105,2 mmol) Kaliumacetat und 2,15 g (2,63 mmol) 1,1'-Bis(diphenylphosphino)ferrocen-palladium(II)dichlorid-Methylenchlorid-Addukt werden in 78 ml entgastem trockenen Dioxan vorgelegt und 40 min unter Mikrowellenbestrahlung (Anton Paar Multiwave) bei 160 °C gerührt. Die Reaktionsmischung wird mit Essigester über Kieselgel filtriert und unter vermindertem Druck vom Lösungsmittel befreit. Nach säulenchromatographischer Reinigung an Kieselgel mittels MPLC mit Cyclohexan/Essigester als Laufmittel erhält man 14,07 g (90% Reinheit, 72% der Theorie) der Titelverbindung als grünes Öl.
logP[a]: 3,73; logP[b]: 3,74; ESI-Masse (m/z): 333 $[M+1]^+$; GC-MS: EI-Masse (m/z): 332 $[M]^+$
1H-NMR(D6-DMSO, 400MHz) $\delta$ ppm: 7,77(s,1H), 7,52(d,1H), 7,30(d,1H), 3,87(q,2H), 2,42(s,3H), 1,29(s,12H)

Stufe 2: *{4-Methyl-3-[(2,2,2-trifluorethyl)sulfanyl]phenyl}boronsäure (VIIIa-7) und {4-Methyl-3-[(2,2,2-trifluorethyl)sulfinyl]phenyl}boronsäure (VIIIb-7)*

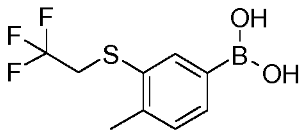

(VIIIa-7)

+

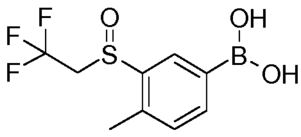

(VIIIb-7)

730 mg (2,2 mmol) 4,4,5,5-Tetramethyl-2-{4-methyl-3-[(2,2,2-trifluorethyl)sulfanyl]phenyl}-1,3,2-dioxaborolan werden in 20 ml Aceton und 20 mL Wasser vorgelegt und bei 0 °C mit 381 mg (4,9 mmol) Ammoniumacetat sowie 1,06 g (4,9 mmol) Natriumperiodat versetzt. Die Reaktionsmischung wird über Nacht bei Raumtemperatur gerührt und anschließend unter vermindertem Druck von Aceton befreit. Die saure wässrige Phase wird mit Essigester extrahiert, die vereinigten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 105 mg (96% Reinheit, 18% der Theorie) {4-Methyl-3-[(2,2,2-trifluorethyl)sulfanyl]phenyl}boronsäure sowie 138 mg (97% Reinheit, 23% der Theorie) {4-Methyl-3-[(2,2,2-trifluorethyl)sulfinyl]phenyl}boronsäure als farblose Feststoffe.

{4-Methyl-3-[(2,2,2-trifluorethyl)sulfanyl]phenyl}boronsäure (VIIIa-7)

logP[a]: 2,30; logP[b]: 2,24; ESI-Masse (m/z): pos.[a]: 251 $[M+1]^+$, neg.[b]: 249 $[M-1]^-$
1H-NMR (D6-DMSO, 400 MHz): 8,08(s,2H), 7,92(s,1H), 7,61-7,59(m,1H), 7,23(d,1H), 3,90(q,2H), 2,38(s,3H)

{4-Methyl-3-[(2,2,2-trifluorethyl)sulfinyl]phenyl}boronsäure (VIIIb-7)

logP[a]: 1,41; logP[b]: 1,36; ESI-Masse (m/z): pos.[a]: 267 $[M+1]^+$; 1H-NMR (D6-DMSO, 400 MHz): 8,31(s,1H), 8,24(s,2H), 7,89-7,87(m,1H), 7,31(d,1H), 4,12-4,02(m,1H), 3,94-3,82(m,1H), 2,39(s,3H)

Analog erhalten wurden:

**2-{4-Chlor-3-[(2,2,2-trifluorethyl)sulfanyl]phenyl}-4,4,5,5-tetramethyl-1,3,2-dioxaborolan**

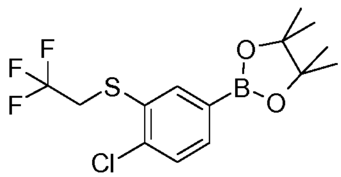

logP[a]: 5,27; logP[b]: 5,16; ESI-Masse (m/z): pos.[a]: 353 $[M+1]^+$; GC-MS: EI-Masse (m/z): $352[M]^+$; 1H-NMR (D6-DMSO, 400MHz): 7,87(d,1H), 7,62-7,60(m,1H), 7,50(d,1H), 3,71(q,2H), 1,33(s,12H)

**{4-Chlor-3-[(2,2,2-trifluorethyl)sulfanyl]phenyl}boronsäure (VIIIa-3)**

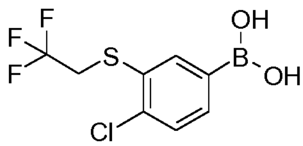

(VIIIa-3)

logP[a]: 2,40; logP[b]: 2,41; ESI-Masse (m/z): neg.[a]: 315 $[M+HCOO^-]^-$, 1H-NMR (D6-DMSO, 400MHz): 8,30(breit,2H), 8,00(d,1H), 7,67-7,64(m,1H), 7,49(d,1H), 4,07(q,2H)

**2-{2,4-Dichlor-5-[(2,2,2-trifluorethyl)sulfanyl]phenyl}-4,4,5,5-tetramethyl-1,3,2-dioxaborolan**

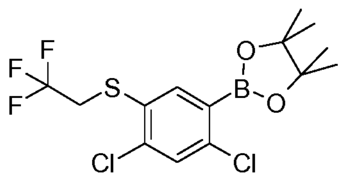

logP[a]: 5,68; logP[b]: 5,31; ESI-Masse (m/z): pos.[a]: 387 $[M+1]^+$; GC-MS: EI-Masse (m/z): $386[M]^+$; 1H-NMR (D6-DMSO, 400MHz): 7,79(s,1H), 7,72(s,1H), 4,08(q,2H), 1,32(s,12H)

**{2,4-Dichlor-5-[(2,2,2-trifluorethyl)sulfanyl]phenyl}boronsäure (VIIIa-6)**

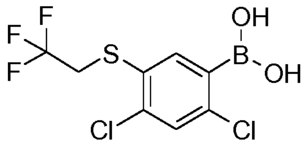

(VIIIa-6)

logP[a]: 2,61; logP[b]: 2,52; ESI-Masse (m/z): neg.[a]: 348 $[M+HCOO^-]^-$, 1H-NMR (D6-DMSO, 400MHz): 8,50(breit,2H), 7,68(s,1H), 7,60(s,1H), 4,11(q,2H)

**2-{4-Methoxy-3-[(2,2,2-trifluorethyl)sulfanyl]phenyl}-4,4,5,5-tetramethyl-1,3,2-dioxaborolan**

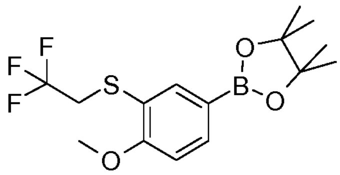

logP[a]: 4,54; logP[b]: 4,46; ESI-Masse (m/z): pos.[a]: 349 $[M+1]^+$; GC-MS: EI-Masse (m/z): 348$[M]^+$; 1H-NMR (D6-DMSO, 400MHz): 7,67(d,1H), 7,64-7,62(m,1H), 7,07(d,1H), 3,89(s,3H), 3,83(q,2H), 1,29(s,12H)

**{4-Methoxy-3-[(2,2,2-trifluorethyl)sulfanyl]phenyl}boronsäure (VIIIa-8)**

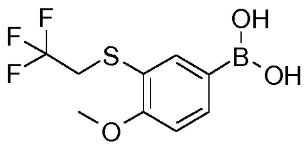

(VIIIa-8)

logP[a]: 1,94; logP[b]: 1,93; ESI-Masse (m/z): neg.[a]: 311 $[M+HCOO^-]^-$, $^1$H-NMR (D6-DMSO, 400MHz): 7,98(breit,2H), 7,86(d,1H), 7,74-7,71(m,1H), 7,02(d,1H), 3,87(s,3H), 3,84(q,2H)

**{2,4-Dimethyl-5-[(2,2,2-trifluorethyl)sulfanyl]phenyl}boronsäure (VIIIa-11)**

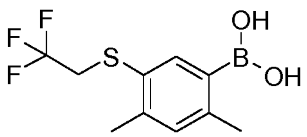

(VIIIa-11)

logP[a]: 2,58; logP[b]: 2,57; ESI-Masse (m/z): neg.[a]: 309 $[M+HCOO^-]^-$, $^1$H-NMR (D6-DMSO, 400MHz): 8,02(breit,2H), 7,61(s,1H), 7,03(s,1H), 3,81(q,2H), 2,36(s,3H),2,34(s,3H)

**{2-Fluor-4-methyl-5-[(2,2,2-trifluorethyl)sulfinyl]phenyl}boronsäure (VIIIb-1)**

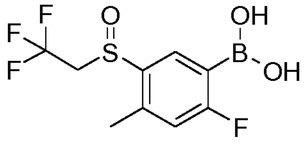

(VIIIb-1)

300 mg (1,12 mmol) {2-Fluor-4-methyl-5-[(2,2,2-trifluorethyl)sulfanyl]phenyl}boronsäure werden in Acetonitril gelöst, mit 396,4 g (1,12 mmol) Selectfluor® versetzt und über Nacht bei Raumtemperatur gerührt. Weitere 39,8 mg (0,12 mmol) Selectfluor® werden zugegeben und die Reaktionsmischung wird 2 h gerührt, dann mit Essigester verdünnt und mit IN Salzsäure gewaschen. Die organische Phase wird über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 102 mg (97% Reinheit, 31% der Theorie) der Titelverbindung als farbloses Öl, das bei Lagerung langsam zu einem farblosen Feststoff kristallisiert.
logP[a]: 1,38; logP[b]: 1,11; ESI-Masse (m/z): pos.[a]: 285 $[M+1]^+$, neg.[a]: 329 $[M+HCOO^-]^-$
$^1$H-NMR (D6-DMSO, 400MHz): 8,36(s,2H), 8,08(d,1H), 7,15(d,1H), 4,14-3,92(m,2H), 2,39(s,3H)

Analog erhalten wurden:

**{4-Chlor-3-[(2,2,2-trifluorethyl)sulfinyl]phenyl}boronsäure (VIIIb-3)**

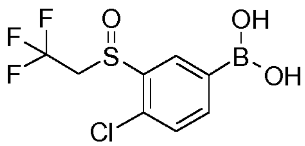

(VIIIb-3)

logP[a]: 1,74; logP[b]: 1,68; ESI-Masse (m/z): pos.[a]: 287 $[M+1]^+$; $^1$H-NMR (D6-DMSO, 400MHz): 8,45(breit,2H), 8,33(d,1H), 8,01-7,99(m,1H), 7,62(d,1H), 4,24-4,12(m,1H), 4,08-3,96(m,1H)

**{2,4-Dichlor-5-[(2,2,2-trifluorethyl)sulfinyl]phenyl}boronsäure (VIIIb-6)**

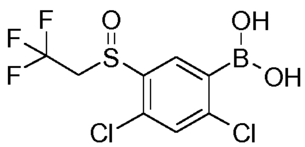

(VIIIb-6)

logP[a]: 1,86; logP[b]: 1,51; ESI-Masse (m/z): pos.[a]: 321 $[M+1]^+$; $^1$H-NMR (D6-DMSO, 400MHz): 8,64(breit,2H), 7,92(s,1H), 7,79(s,1H), 4,29-4,04(m,2H)

**{4-Methoxy-3-[(2,2,2-trifluorethyl)sulfanyl]phenyl}boronsäure (VIIIb-8)**

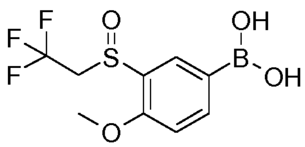

(VIIIb-8)

logP[a]: 1,36; logP[b]: 1,37; ESI-Masse (m/z): pos.[a]: 283 $[M+1]^+$; $^1$H-NMR (D6-DMSO, 400MHz): 8,15(s,1H), 8,13(s,2H), 8,02-8,00(m,1H), 7,17(d,1H), 4,08-3,99(m,1H), 3,90(s,3H), 3,87-3,78(m,1H)

**{2,4-Dimethyl-5-[(2,2,2-trifluorethyl)sulfinyl]phenyl}boronsäure (VIIIb-11)**

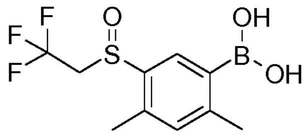

(VIIIb-11)

logP[a]: 1,61; logP[b]: 1,61; ESI-Masse (m/z): pos.[a]: 281 $[M+1]^+$; $^1$H-NMR (D6-DMSO, 400MHz): 8,15(s,2H), 7,96(s,1H), 7,09(s,1H), 4,05-3,85(m,2H), 2,44(s,3H), 2,33(s,3H)

**Synthese von Thioharnstoffen der Formel (IIa) und (IIb)**

**1-{2-Fluor-4-methyl-5-[(2,2,2-trifluorethyl)sulfanyl]phenyl}-3-(2,2,2-trifluorethyl)thioharnstoff (IIa-1)**

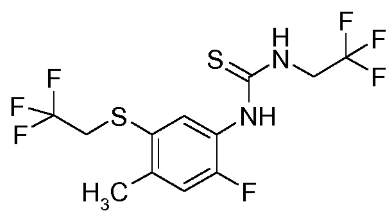

1,00 g (4,18 mmol) 2-Fluor-4-methyl-5-[(2,2,2-trifluorethyl)sulfanyl]anilin wird in 5 ml Dichlormethan vorgelegt und dazu werden 0,006 ml (0,042 mmol) Triethylamin gegeben. Nach der Zugabe von 0,59 g (4,18 mmol) 1,1,1-Trifluor-2-isothiocyanatoethan wird das Reaktionsgemisch über Nacht bei Raumtemperatur gerührt. Unter Vakuum wird das Lösungsmittel entfert, der Rückstand wird mit etwas Toluol verrührt und der unlösliche Teil wird abgesaugt und getrocknet. Es werden 0,31 g (100% Reinheit, 20% d.Th.) der Titelverbindung als weißer Feststoff erhalten. Das Filtrat wird unter Vakuum von Lösungsmittel befreit. Der Rückstand aus 1,30 g enthält die Titelverbindung mit einer Reinheit von 77%. logP[a]: 3,32; logP[b]: 3,24; 1H-NMR (D6-DMSO, 400MHz) □ ppm: 9,62(bs,1H), 8,34(bs,1H), 7,76(d,1H), 7,26(d,1H), 4,46-4,40(m,2H), 3,87(q,2H), 2,38(s,3H)

Analog erhalten wurde:

**1-{2-Fluor-4-methyl-5-[(2,2,2-trifluorethyl)sulfinyl]phenyl}-3-(2,2,2-trifluorethyl)thioharnstoff (IIb-1)**

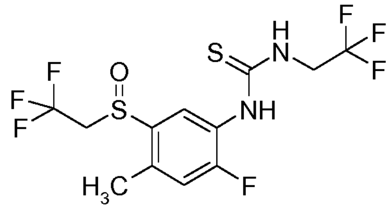

logP[a]: 2,34; logP[b]: 2,3; 1H-NMR (D6-DMSO, 400MHz) □ ppm: 9,75(bs,1H), 8,50(bs,1H), 8,12(bd,1H), 7,36(d,1H), 4,52-4,40(m,1H), 4,21-4,15(m,1H), 4,05-3,95(m,1H), 2,36(s,3H)

**Synthese von Isocyanaten der Formel (IIIa)**

**4-Fluor-5-isocyanato-2-methylphenyl-2,2,2-trifluorethylsulfid (IIIa-1)**

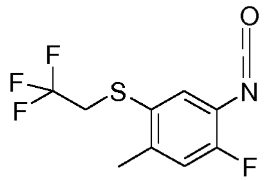

Eine Lösung aus 5,00 g (20,9 mmol) 2-Fluor-4-methyl-5-[(2,2,2-trifluorethyl)sulfanyl]anilin in 50 mL Toluol wird bei Raumtemperatur mit 3,10 g (15,68 mmol) Trichlormethylchlorocarbonat (Diphosgen) versetzt und 2 h an einer Apparatur mit Ammoniak/iso-Propanol-Mischung enthaltenen Waschflaschen zum Rückfluss erhitzt. Toluol wird über einen Wasserabscheider abdestilliert, frisches Toluol wird zugegeben und der Vorgang wird mehrfach wiederholt. Nach Trocknung im Vakuum erhält man 5,15 g (90% Reinheit, 84% der Theorie) der Titelverbindung als gelbes Öl.
GC-MS EI-Masse (m/z): 265 $[M]^+$, GC-MS-Index 1392; 1H-NMR (CDC13, 400MHz) □ ppm: 7,24(d,1H), 7,03(d,1H), 3,31(q,2H), 2,45(s,3H)

Analog erhalten wurden:

**2-Chlor-5-isocyanatophenyl-2,2,2-trifluorethylsulfid (IIIa-3)**

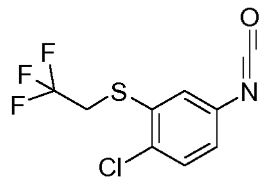

GC-MS EI-Masse (m/z): 267 $[M]^+$, GC-MS-Index 1452

**5-Isocyanato-2,4-dimethylphenyl-2,2,2-trifluorethylsulfid (IIIa-11)**

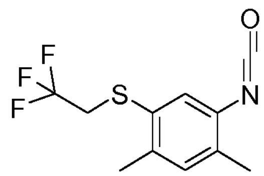

GC-MS EI-Masse (m/z): 261 $[M]^+$, GC-MS-Index 1500; 1H-NMR (CDC13, 400MHz) □ ppm: 7,19(s,1H), 7,05(s,1H), 3,34(q,2H), 2,40(s,3H), 2,28(s,3H)

**Synthese von Verbindungen der allgemeinen Formel (XXIV)**

**4-[4-Cyclopropyl-5-oxo-3-(trifluormethyl)-4,5-dihydro-1H-1,2,4-triazol-1-yl]-2,5-difluorbenzonitril (XXIV-1)**

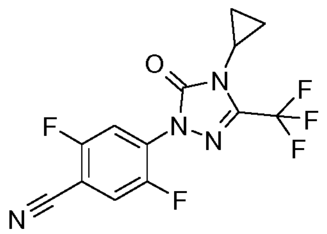

Zu einer Lösung von 202 mg (5,0 mmol) Natriumhydrid (60%ig in Mineralöl) in 30 mL N,N-Dimethylformamid werden bei 0 °C 974 mg (5,0 mmol) 4-Cyclopropyl-5-(trifluormethyl)-2,4-dihydro-3H-1,2,4-triazol-3-on (hergestellt nach EP 657437 A1; DE 4339412 A1) gegeben. Nach 30-minütigem Rühren bei 0 °C werden 660 mg (4.2 mmol) 2,4,5-Trifluorbenzonitril zugegeben. Die Reaktionsmischung wird 3 d bei Raumtemperatur gerührt, dann in Wasser gegossen, neutralisiert und mit *tert*-Butylmethylether extrahiert. Die vereinten organischen Phasen werden sukzessive mit Wasser und gesättigter wässriger Kochsalzlösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 539 mg (100% Reinheit, 39% der Theorie) der Titelverbindung als gelben Feststoff.
logP[a]: 2,91; logP[a]: 2,90; 1H-NMR(D6-DMSO): 8,29(dd,1H), 7,84(dd,1H), 3,10-3,04(m,1H), 1,23-1,01(m,4H)

**Synthese von Verbindungen der allgemeinen Formel (XXVII) und (XXVIII)**

**Methyl-1-[5-(chlorsulfonyl)-2-fluor-4-methylphenyl]-4-isopropyl-5-oxo-4,5-dihydro-1H-1,2,4-triazol-3-carboxylat (XXVII-1)**

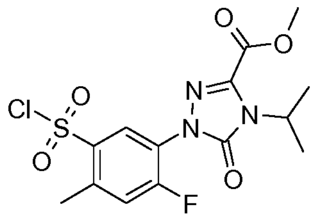

330 mg (1,13 mmol) Methyl-1-(2-fluor-4-methylphenyl)-4-isopropyl-5-oxo-4,5-dihydro-1H-1,2,4-triazol-3-carboxylat (Synthese nach DE2725148) werden in 30 ml Dichlormethan portionsweise mit 5 g (42,91 mmol) Chlorsulfonsäure versetzt und 12 h unter Rückfluss gerührt. Nach dem Abkühlen wird Eiswasser zugetropft und mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck werden 350 mg (85% Reinheit nach 1H-NMR, 79 % der Theorie) Produkt erhalten.
1H-NMR(D6-DMSO): 7,84-7,82(m,1H), 7,27-7,25(m,1H), 4,99-4,95(sept,1H), 3,89(s,3H), 2,57(s,3H), 1,48-1,47(d,6H)

**Dimethyl-1,1'-[disulfanediylbis(6-fluor-4-methylbenzol-3,1-diyl)]bis(4-isopropyl-5-oxo-4,5-dihydro-1H-1,2,4-triazol-3-carboxylat) (XXVIII-1)**

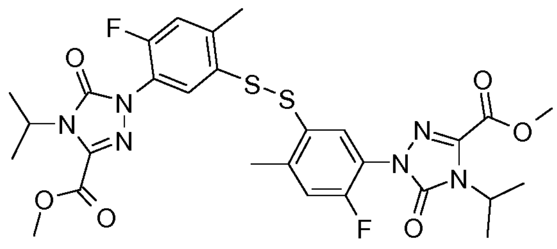

5,50 g (14,04 mmol) Methyl-1-[5-(chlorsulfonyl)-2-fluor-4-methylphenyl]-4-isopropyl-5-oxo-4,5-dihydro-1H-1,2,4-triazol-3-carboxylat werden mit 4,10 g (73,42 mmol) Eisenpulver in 200 ml Ethanol und 7,6 g konzentrierter Salzsäure 12 h unter Rückfluss erhitzt. Nach dem Entfernen des Lösungsmittels unter vermindertem Druck wird der Rückstand mit Wasser verrührt und abgesaugt und man erhält 4,50 g Rohprodukt (49% der Theorie) als hellbraunen Feststoff.
logP[a]: 4,11

Gemäß den zuvor beschriebenen Verfahren wurden die folgenden Verbindungen der allgemeinen Formel (I) hergestellt:

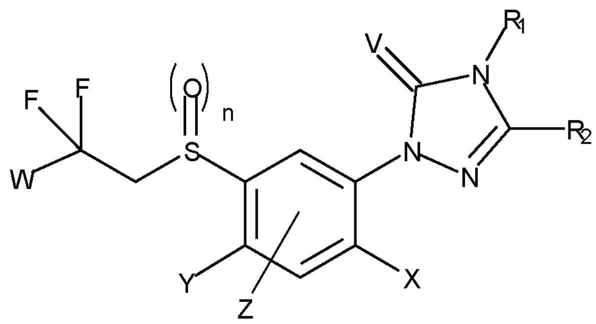

(I) mit (I-A)

(Verbindung der allgemeinen Formel (I) mit (I-A) mit Z = H)

| Beispiel-Nr. | $R^1$ | $R^2$ | W | n | Y | X | V |
|---|---|---|---|---|---|---|---|
| 1 | $CH_3$ | $CH_3$ | F | 1 | CN | F | O |
| 2 | H | $CH_3$ | F | 0 | CN | F | O |
| 3 | $CH(CH_3)_2$ | COOH | H | 0 | $CH_3$ | F | O |
| 4 | $CH(CH_3)_2$ | H | F | 0 | $CH_3$ | F | O |
| 5 | $CH(CH_3)_2$ | H | H | 0 | $CH_3$ | F | O |
| 6 | $CH(CH_3)_2$ | H | F | 1 | $CH_3$ | F | O |
| 7 | H | H | F | 0 | $CH_3$ | F | O |
| 8 | $CH(CH_3)_2$ | H | H | 1 | $CH_3$ | F | O |
| 9 | $CH_3$ | $CF_3$ | F | 0 | $CH_3$ | F | O |
| 10 | $CH_3$ | $CH_3$ | F | 0 | $CH_3$ | F | O |
| 11 | $CH_3$ | $CH_3$ | F | 1 | $CH_3$ | F | O |
| 12 | $CH_3$ | $CF_3$ | F | 1 | $CH_3$ | F | O |
| 13 | $CH_3$ | 2-Fluor-4-chlorphenyl | F | 0 | $OCH_3$ | H | O |
| 14 | $CH_3$ | $CH_3$ | F | 0 | $CH_3$ | H | O |
| 15 | $CH_3$ | Phenyl | F | 0 | $CH_3$ | F | O |
| 16 | $CH_3$ | 2-Fluor-4-chlorphenyl | F | 0 | $CH_3$ | H | O |
| 17 | Cyclopropyl | $CH_3$ | F | 0 | $CH_3$ | H | O |
| 18 | Cyclopropyl | $CH(CH_3)_2$ | F | 0 | $CH_3$ | H | O |
| 19 | Cyclopropyl | $CH_2OCH_3$ | F | 0 | $CH_3$ | H | O |
| 20 | Cyclopropyl | H | F | 0 | $CH_3$ | H | O |
| 21 | $CH_3$ | $CF_3$ | F | 0 | $CH_3$ | H | O |
| 22 | $C(CH_3)_3$ | $CF_3$ | F | 0 | $CH_3$ | H | O |
| 23 | $CH_3$ | Phenyl | F | 1 | $CH_3$ | F | O |
| 24 | $CH_3$ | Phenyl | F | 1 | $CH_3$ | $CH_3$ | O |
| 25 | $CH_3$ | 2-Fluor-4-chlorphenyl | F | 1 | $CH_3$ | H | O |
| 26 | Cyclopropyl | $CH_3$ | F | 1 | $CH_3$ | H | O |

(fortgesetzt)

| Beispiel-Nr. | $R^1$ | $R^2$ | W | n | Y | X | V |
|---|---|---|---|---|---|---|---|
| 27 | Cyclopropyl | $CH_2OCH_3$ | F | 1 | $CH_3$ | H | O |
| 28 | Cyclopropyl | H | F | 1 | $CH_3$ | H | O |
| 29 | $CH_3$ | $CF_3$ | F | 1 | $CH_3$ | H | O |
| 30 | $C(CH_3)_3$ | $CF_3$ | F | 1 | $CH_3$ | H | O |
| 31 | 4-Methyl-3-(2,2,2-trifluorethy lsulfanyl)-phenyl | $CH_3$ | F | 0 | $CH_3$ | H | O |
| 32 | Cyclopropyl | $CF_3$ | F | 0 | $CH_3$ | H | O |
| 33 | Cyclopropyl | $CH(CH_3)_2$ | F | 0 | Cl | H | O |
| 34 | Cyclopropyl | $CF_3$ | F | 1 | $CH_3$ | H | O |
| 35 | Phenyl | $CF_3$ | F | 0 | $CH_3$ | H | O |
| 36 | Phenyl | $CF_3$ | F | 0 | $CH_3$ | F | O |
| 37 | Phenyl | $CF_3$ | F | 1 | $CH_3$ | F | O |
| 38 | $CH_3$ | $CF_3$ | F | 0 | CN | F | O |
| 39 | $CH_3$ | 2-Fluor-4-chlorphenyl | F | 1 | $CH_3$ | F | O |
| 40 | $CH_3$ | 2-Fluor-4-chlorphenyl | F | 0 | $CH_3$ | F | O |
| 41 | Cyclopropyl | $CH_3$ | F | 1 | $CH_3$ | F | O |
| 42 | Cyclopropyl | $CH_3$ | F | 0 | $CH_3$ | F | O |
| 43 | Cyclopropyl | $CH(CH_3)_2$ | F | 1 | $CH_3$ | F | O |
| 44 | Cyclopropyl | $CH(CH_3)_2$ | F | 0 | $CH_3$ | F | O |
| 45 | Cyclopropyl | $CH_2OCH_3$ | F | 1 | $CH_3$ | F | O |
| 46 | Cyclopropyl | $CH_2OCH_3$ | F | 0 | $CH_3$ | F | O |
| 47 | Cyclopropyl | $CF_3$ | F | 1 | CN | F | O |
| 48 | Cyclopropyl | $CF_3$ | F | 0 | $CH_3$ | $CH_3$ | O |
| 49 | $C(CH_3)_3$ | $CF_3$ | F | 0 | $CH_3$ | $CH_3$ | O |
| 50 | $CH_2CH_3$ | $CF_3$ | F | 0 | $CH_3$ | Cl | O |
| 51 | Cyclopropyl | $CF_3$ | F | 0 | Cl | H | O |
| 52 | Cyclopropyl | $CF_3$ | F | 0 | $OCH_3$ | H | O |
| 53 | Cyclopropyl | $CF_3$ | F | 1 | $OCH_3$ | H | O |
| 54 | $CH_2CH_3$ | $CF_3$ | F | 0 | $CH_3$ | H | O |
| 55 | $CH_2CF_3$ | $CF_3$ | F | 0 | $CH_3$ | F | O |
| 56 | $CH_2CF_3$ | $CF_3$ | F | 1 | $CH_3$ | F | O |
| 57 | $CH_2CH_3$ | $CF_3$ | F | 0 | $CH_3$ | F | O |
| 58 | $CH_2CH_3$ | $CF_3$ | F | 1 | $CH_3$ | F | O |
| 59 | Cyclopropyl | $CF_3$ | F | 0 | $CH_3$ | F | O |
| 60 | Cyclopropyl | $CF_3$ | F | 1 | $CH_3$ | F | O |
| 61 | Cyclopropyl | H | F | 0 | $CH_3$ | F | O |
| 62 | Cyclopropyl | H | F | 1 | $CH_3$ | F | O |
| 63 | Cyclopropyl | $C(CH_3)_3$ | F | 0 | $CH_3$ | F | O |
| 64 | Cyclopropyl | $C(CH_3)_3$ | F | 1 | $CH_3$ | F | O |

(fortgesetzt)

| Beispiel-Nr. | $R^1$ | $R^2$ | W | n | Y | X | V |
|---|---|---|---|---|---|---|---|
| 65 | Cyclopropyl | Cyclopropyl | F | 0 | $CH_3$ | F | O |
| 66 | Cyclopropyl | Cyclopropyl | F | 1 | $CH_3$ | F | O |
| 67 | $C(CH_3)_3$ | $CF_3$ | F | 0 | $CH_3$ | F | O |
| 68 | $C(CH_3)_3$ | $CF_3$ | F | 1 | $CH_3$ | F | O |
| 69 | Cyclopropyl | 3-Fluorphenyl | F | 0 | $CH_3$ | F | O |
| 70 | Cyclopropyl | 3-Fluorphenyl | F | 1 | $CH_3$ | F | O |
| 71 | Cyclopropyl | $CH_2CH_2CH_2CH_3$ | F | 0 | $CH_3$ | F | O |
| 72 | Cyclopropyl | $CH_2CH_2CH_2CH_3$ | F | 1 | $CH_3$ | F | O |
| 73 | Cyclopropyl | $CH_2OCH_2CH_3$ | F | 0 | $CH_3$ | F | O |
| 74 | Cyclopropyl | $CH_2OCH_2CH_3$ | F | 1 | $CH_3$ | F | O |
| 75 | Cyclopropyl | $CH_2CH_2OCH_3$ | F | 0 | $CH_3$ | F | O |
| 76 | Cyclopropyl | $CH_2CH_2OCH_3$ | F | 1 | $CH_3$ | F | O |
| 77 | Cyclopropyl | $CH_2CH_3$ | F | 0 | $CH_3$ | F | O |
| 78 | Cyclopropyl | $CH_2CH_3$ | F | 1 | $CH_3$ | F | O |
| 79 | Cyclopropyl | $CH_2CH_2CH_3$ | F | 0 | $CH_3$ | F | O |
| 80 | Cyclopropyl | $CH_2CH_2CH_3$ | F | 1 | $CH_3$ | F | O |
| 81 | Cyclopropyl | $CH=CHCH_3$ | F | 0 | $CH_3$ | F | O |
| 82 | Cyclopropyl | $CH=CHCH_3$ | F | 1 | $CH_3$ | F | O |
| 83 | Cyclopropyl | $CH_2CH(CH_3)_2$ | F | 0 | $CH_3$ | F | O |
| 84 | Cyclopropyl | $CH_2CH(CH_3)_2$ | F | 1 | $CH_3$ | F | O |
| 85 | Cyclopropyl | $CH(CH_3)CH_2CH_3$ | F | 0 | $CH_3$ | F | O |
| 86 | Cyclopropyl | $CH(CH_3)CH_2CH_3$ | F | 1 | $CH_3$ | F | O |
| 87 | Cyclopropyl | Cyclobutyl | F | 0 | $CH_3$ | F | O |
| 88 | Cyclopropyl | Cyclobutyl | F | 1 | $CH_3$ | F | O |
| 89 | $CH_2CH_2OCH_3$ | $CF_3$ | F | 0 | $CH_3$ | F | O |
| 90 | Cyclopropyl | $OCH_3$ | F | 0 | $CH_3$ | H | O |
| 91 | Cyclopropyl | $OCH(CH_3)_2$ | F | 0 | $CH_3$ | H | O |
| 92 | Cyclopropyl | $OCH_2CF_3$ | F | 0 | $CH_3$ | H | O |
| 93 | $CH_3$ | $OCH_3$ | F | 0 | $CH_3$ | F | O |
| 94 | $CH_3$ | $OCH_3$ | F | 1 | $CH_3$ | F | O |
| 95 | $CH_3$ | $OCH_3$ | F | 0 | $CF_3$ | H | O |
| 96 | $CH_3$ | $OCH_3$ | F | 1 | $CF_3$ | H | O |
| 97 | $CH_3$ | $OCH_3$ | F | 1 | Cl | Cl | O |
| 98 | $CH_3$ | $OCH_3$ | F | 1 | Cl | F | O |
| 99 | $CH_3$ | $OCH_3$ | F | 1 | Cl | H | O |
| 100 | Cyclopropyl | $OCH(CH_3)_2$ | F | 1 | $CH_3$ | F | O |
| 101 | Cyclopropyl | $OCH(CH_3)_2$ | F | 0 | $CH_3$ | F | O |
| 102 | Cyclopropyl | $OCH_2CF_3$ | F | 1 | $CH_3$ | F | O |
| 103 | Cyclopropyl | $OCH_2CF_3$ | F | 0 | $CH_3$ | F | O |
| 104 | Cyclopropyl | $OCH_3$ | F | 1 | $CH_3$ | F | O |

(fortgesetzt)

| Beispiel-Nr. | $R^1$ | $R^2$ | W | n | Y | X | V |
|---|---|---|---|---|---|---|---|
| 105 | Cyclopropyl | $OCH_3$ | F | 0 | $CH_3$ | F | O |
| 106 | Cyclopropyl | $OCH_3$ | F | 1 | Cl | Cl | O |
| 107 | Cyclopropyl | $OCH_3$ | F | 0 | Cl | Cl | O |
| 108 | Cyclopropyl | $OCH_3$ | F | 0 | Cl | H | O |
| 109 | Cyclopropyl | $OCH_3$ | F | 1 | $OCH_3$ | H | O |
| 110 | Cyclopropyl | $OCH_3$ | F | 1 | Br | H | O |
| 111 | Cyclopropyl | $OCH_3$ | F | 0 | Br | H | O |
| 112 | Cyclopropyl | $OCH_2CH_2CH_3$ | F | 1 | $CH_3$ | F | O |
| 113 | Cyclopropyl | $OCH_2CH_2CH_3$ | F | 0 | $CH_3$ | F | O |
| 114 | Cyclopropyl | $OCH_3$ | F | 0 | Cl | F | O |
| 115 | Cyclopropyl | $OCH_3$ | F | 1 | Cl | F | O |
| 116 | Cyclopropyl | $OCH_3$ | F | 0 | $CH_3$ | Cl | O |
| 117 | Cyclopropyl | $OCH_3$ | F | 1 | $CH_3$ | Cl | O |
| 118 | Cyclopropyl | $OCH_3$ | F | 1 | CN | H | O |
| 119 | Cyclopropyl | $OCH_3$ | F | 1 | $CH_3$ | $CH_3$ | O |
| 120 | Cyclopropyl | $OCH_2CH_3$ | F | 0 | $CH_3$ | F | O |
| 121 | Cyclopropyl | $OCH_2CH_3$ | F | 1 | $CH_3$ | F | O |
| 122 | Cyclopropyl | $OCH_2CH_3$ | F | 0 | $CH_3$ | H | O |
| 123 | Cyclopropyl | $OCH_2CH_2CH_3$ | F | 0 | $CH_3$ | H | O |
| 124 | Cyclopropyl | $OCH_2CH_2CH_2CH_3$ | F | 1 | $CH_3$ | F | O |
| 125 | Cyclopropyl | $OCH_2CH_2CH_2CH_3$ | F | 0 | $CH_3$ | F | O |
| 126 | Cyclopropyl | $OCH_2CH_2CH_2CH_3$ | F | 0 | $CH_3$ | H | O |
| 127 | $CH_3$ | $OCH_2CH_2CH_3$ | F | 0 | $CH_3$ | F | O |
| 128 | $CH_3$ | $OCH_2CH_2CH_2CH_3$ | F | 0 | $CH_3$ | F | O |
| 129 | $CH_3$ | $OCH_2CH_3$ | F | 0 | $CH_3$ | F | O |
| 130 | $CH_3$ | $OCH_2CH_3$ | F | 0 | $CH_3$ | H | O |
| 131 | $CH_3$ | $OCH_2CH_3$ | F | 1 | $CH_3$ | F | O |
| 132 | $CH_3$ | $OCH_2CH_2CH_3$ | F | 1 | $CH_3$ | F | O |
| 133 | $CH_3$ | $OCH_2CH_2CH_3$ | F | 1 | $CH_3$ | H | O |
| 134 | Cyclopropyl | $OCH_2$-Cyclopropyl | F | 0 | $CH_3$ | F | O |
| 135 | Cyclopropyl | $OCH_2$-Cyclopropyl | F | 0 | $CH_3$ | H | O |
| 136 | Cyclopropyl | $OCH_2CH_2C(=CH_2)C$ H3 | F | 0 | $CH_3$ | F | O |
| 137 | Cyclopropyl | $OCH_2CH_2C(=CH_2)C$ H3 | F | 1 | $CH_3$ | F | O |
| 138 | Cyclopropyl | $OCH_2CH_2C(=CH_2)C$ H3 | F | 0 | $CH_3$ | H | O |
| 139 | Cyclopropyl | $OCH_2CH_2C(=CH_2)C$ H3 | F | 1 | $CH_3$ | H | O |
| 140 | Cyclopropyl | $OCH(CH_3)CH_2CH_3$ | F | 0 | $CH_3$ | F | O |
| 141 | Cyclopropyl | $OCH(CH_3)CH_2CH_3$ | F | 1 | $CH_3$ | F | O |
| 142 | Cyclopropyl | $OCH(CH_3)CH_2CH_3$ | F | 0 | $CH_3$ | H | O |
| 143 | $CH_3$ | $OCH_2$-Cyclopropyl | F | 0 | $CH_3$ | F | O |

(fortgesetzt)

| Beispiel-Nr. | $R^1$ | $R^2$ | W | n | Y | X | V |
|---|---|---|---|---|---|---|---|
| 144 | Cyclopropyl | $OCH_3$ | F | 0 | $CF_3$ | H | O |
| 145 | $CH_3$ | $OCH_2$-Cyclopropyl | F | 0 | $CH_3$ | H | O |
| 146 | $CH_3$ | $OCH_2CH_2CH_2CH_3$ | F | 1 | $CH_3$ | F | O |
| 147 | Cyclopropyl | $OCH_3$ | F | 1 | $CF_3$ | H | O |
| 148 | $CH_3$ | $OCH_2CH(CH_3)_2$ | F | 0 | $CH_3$ | F | O |
| 149 | $CH_3$ | $OCH_2CH_2OCH_3$ | F | 0 | $CH_3$ | F | O |
| 150 | $CH_3$ | $OCH_2C(CH_3)_3$ | F | 0 | $CH_3$ | F | O |
| 151 | $CH_3$ | $OCH(CH_3)_2$ | F | 0 | $CH_3$ | F | O |
| 152 | $CH_3$ | $OCH_2CH(CH_3)_2$ | F | 1 | $CH_3$ | F | O |
| 153 | $CH_3$ | O-Cyclohexyl | F | 0 | $CH_3$ | F | O |
| 154 | $CH_3$ | $OC(CH_3)_3$ | F | 0 | $CH_3$ | H | O |
| 155 | $CH_3$ | O-Cyclohexyl | F | 0 | $CH_3$ | H | O |
| 156 | $CH_3$ | O-3-Tetrahydrofuryl | F | 0 | $CH_3$ | H | O |
| 157 | $CH_3$ | $OC(CH_3)_3$ | F | 0 | $CH_3$ | F | O |
| 158 | $CH_3$ | O-Phenyl | F | 0 | $CH_3$ | F | O |
| 159 | $CH_3$ | O-Phenyl | F | 1 | $CH_3$ | F | O |
| 160 | $CH_3$ | $OCH_2CF_3$ | F | 1 | $CH_3$ | F | O |
| 161 | $CH_3$ | 0-3 -Tetrahydrofuryl | F | 1 | $CH_3$ | F | O |
| 162 | $CH_3$ | $OCH_2C(CH_3)_3$ | F | 1 | $CH_3$ | H | O |
| 163 | $CH_3$ | $OCH(CH_3)_2$ | F | 1 | $CH_3$ | H | O |
| 164 | $CH_3$ | O-Cyclohexyl | F | 1 | $CH_3$ | F | O |
| 165 | $CH_3$ | $OCH_2C(CH_3)_3$ | F | 1 | $CH_3$ | F | O |
| 166 | $CH_3$ | $OCH(CH_3)_2$ | F | 1 | $CH_3$ | F | O |
| 167 | $CH_3$ | $OCH_2CH(CH_3)_2$ | F | 1 | $CH_3$ | H | O |
| 168 | $CH_3$ | $OCH_3$ | F | 0 | Br | H | O |
| 169 | $CH_3$ | $OCH_3$ | F | 1 | F | F | O |
| 170 | Cyclopropyl | $OCH_3$ | F | 1 | F | F | O |
| 171 | Cyclopropyl | $OCH_3$ | F | 0 | F | F | O |
| 172 | Cyclopropyl | $OCH_3$ | F | 1 | Br | F | O |
| 173 | Cyclopropyl | $OCH_3$ | F | 0 | Br | F | O |
| 174 | $CH_3$ | $OCH_3$ | F | 1 | Br | F | O |
| 175 | $CH_3$ | $OCH_3$ | F | 0 | Br | F | O |
| 176 | $CH_2CH=CH_2$ | $OCH_3$ | F | 0 | $CH_3$ | H | O |
| 177 | $CH_3$ | O-Benzyl | F | 1 | $CH_3$ | F | O |
| 178 | $CH_3$ | O-Benzyl | F | 1 | $CH_3$ | H | O |
| 179 | $CH_2CH=CH_2$ | $OCH_3$ | F | 1 | $CH_3$ | H | O |
| 180 | $CH_2CH_3$ | $OCH_3$ | F | 0 | $CH_3$ | F | O |
| 181 | $CH_2CH_3$ | $OCH_3$ | F | 1 | $CH_3$ | F | O |
| 182 | $CH_2CH=CH_2$ | $OCH_3$ | F | 0 | $CH_3$ | F | O |
| 183 | $CH_2CH=CH_2$ | $OCH_3$ | F | 1 | $CH_3$ | F | O |
| 184 | $CH_3$ | $OC(CH_3)_3$ | F | 1 | $CH_3$ | F | O |

(fortgesetzt)

| Beispiel-Nr. | $R^1$ | $R^2$ | W | n | Y | X | V |
|---|---|---|---|---|---|---|---|
| 185 | Cyclopropyl | $OCH_2CF_3$ | F | 0 | Cl | Cl | O |
| 186 | Cyclopropyl | $OCH_2CF_3$ | F | 0 | Cl | F | O |
| 187 | Cyclopropyl | $OCH_2CF_3$ | F | 0 | $CH_3$ | Cl | O |
| 188 | Cyclopropyl | $OCH_2CF_3$ | F | 0 | $OCH_3$ | F | O |
| 189 | Cyclopropyl | $OCH_2CF_3$ | F | 0 | $CF_3$ | H | O |
| 190 | $CH_3$ | $N(CH_3)_2$ | F | 0 | $CH_3$ | F | O |
| 191 | $CH_3$ | $N(CH_3)_2$ | F | 1 | $CH_3$ | F | O |
| 192 | $CH_3$ | (4-methyl-5-oxo-3-phenoxy-1,2,4-triazol-1-yl) | F | 0 | $CH_3$ | H | O |
| 193 | Cyclopropyl | $N(CH_3)_2$ | F | 0 | $CH_3$ | F | O |
| 194 | Cyclopropyl | $N(CH_3)_2$ | F | 1 | $CH_3$ | F | O |
| 195 | $N(CH_3)_2$ | Cyclopropyl | F | 0 | $CH_3$ | F | O |
| 196 | $NH_2$ | Cyclopropyl | F | 0 | $CH_3$ | F | O |
| 197 | $N(CH_3)_2$ | Cyclopropyl | F | 1 | $CH_3$ | F | O |
| 198 | $NH_2$ | $CH_2OCH_3$ | F | 0 | $CH_3$ | F | O |
| 199 | $NHCH_3$ | Cyclopropyl | F | 0 | $CH_3$ | F | O |
| 200 | $NH_2$ | $CH_3$ | F | 0 | $CH_3$ | F | O |
| 201 | $N(CH_3)_2$ | $CH_3$ | F | 0 | $CH_3$ | F | O |
| 202 | $N=C(CH_3)CH_2CH(C H_3)_2$ | $CH_3$ | F | 0 | $CH_3$ | F | O |
| 203 | $NHCH_3$ | H | F | 0 | $CH_3$ | F | O |
| 204 | $NH_2$ | $CH(CH_3)_2$ | F | 1 | $CH_3$ | F | O |
| 205 | $NH_2$ | $CF_3$ | F | 0 | $CH_3$ | F | O |
| 206 | $NH_2$ | Cyclopropyl | F | 1 | $CH_3$ | F | O |
| 207 | $NH_2$ | $CH_2OCH_3$ | F | 1 | $CH_3$ | F | O |
| 208 | $NHCH_3$ | Cyclopropyl | F | 1 | $CH_3$ | F | O |
| 209 | $NH_2$ | $CH_3$ | F | 1 | $CH_3$ | F | O |
| 210 | $N(CH_3)_2$ | $CH_3$ | F | 1 | $CH_3$ | F | O |
| 211 | $NH_2$ | H | F | 0 | $CH_3$ | F | O |
| 212 | $NH_2$ | $CF_3$ | F | 0 | $CH_3$ | H | O |
| 213 | $N(CH_3)_2$ | $CF_3$ | F | 1 | $CH_3$ | F | O |
| 214 | $N(CH_3)_2$ | $CF_3$ | F | 0 | $CH_3$ | F | O |
| 215 | $NH_2$ | $CF_3$ | F | 1 | $CH_3$ | F | O |
| 216 | $NHCH_3$ | $N(CH_3)_2$ | F | 0 | $CH_3$ | F | O |
| 217 | $NHCH_3$ | $N(CH_3)_2$ | F | 1 | $CH_3$ | F | O |
| 218 | $NH_2$ | $NHCH_3$ | F | 0 | $CH_3$ | F | O |
| 219 | $NH_2$ | $NHCH_3$ | F | 1 | $CH_3$ | F | O |
| 220 | $NH_2$ | $N(CH_3)_2$ | F | 0 | $CH_3$ | F | O |
| 221 | $NH_2$ | $N(CH_3)_2$ | F | 1 | $CH_3$ | F | O |
| 222 | $NH_2$ | $N(CH_2CH_3)_2$ | F | 0 | $CH_3$ | F | O |

(fortgesetzt)

| Beispiel-Nr. | $R^1$ | $R^2$ | W | n | Y | X | V |
|---|---|---|---|---|---|---|---|
| 223 | $NH_2$ | $N(CH_2CH_3)_2$ | F | 1 | $CH_3$ | F | O |
| 224 | $NH_2$ | $NHCH(CH_3)_2$ | F | 0 | $CH_3$ | F | O |
| 225 | $NH_2$ | $NHCH(CH_3)_2$ | F | 1 | $CH_3$ | F | O |
| 226 | $OCH_3$ | $CH_3$ | F | 1 | $CH_3$ | F | O |
| 227 | $OCH_3$ | $OCH_2CH_3$ | F | 0 | $CH_3$ | H | O |
| 228 | Cyclopropyl | $S(=O)_2CH_3$ | F | 1 | $CH_3$ | F | O |
| 229 | Cyclopropyl | $S(=O)CH_2CHF_2$ | F | 1 | $CH_3$ | F | O |
| 230 | Cyclopropyl | $S(=O)CH_3$ | F | 0 | $CH_3$ | F | O |
| 231 | Cyclopropyl | $S(=O)CH_3$ | F | 1 | $CH_3$ | F | O |
| 232 | $CH_3$ | $S(=O)CH_3$ | F | 0 | $CH_3$ | F | O |
| 233 | $CH_3$ | $S(=O)CH_3$ | F | 1 | $CH_3$ | F | O |
| 234 | Cyclopropyl | $S(=O)CH_2CH_3$ | F | 0 | $CH_3$ | F | O |
| 235 | Cyclopropyl | $S(=O)CH_2CH_3$ | F | 1 | $CH_3$ | F | O |
| 236 | Cyclopropyl | $S(=O)CH_2CH_2CH_3$ | F | 0 | $CH_3$ | F | O |
| 237 | Cyclopropyl | $S(=O)CH_2CH_2CH_3$ | F | 1 | $CH_3$ | F | O |
| 238 | Cyclopropyl | $S(=O)CH(CH_3)_2$ | F | 0 | $CH_3$ | F | O |
| 239 | Cyclopropyl | $S(=O)CH(CH_3)_2$ | F | 1 | $CH_3$ | F | O |
| 240 | $CH_2CH_3$ | $S(=O)CH_3$ | F | 0 | $CH_3$ | F | O |
| 241 | $CH_2CH_3$ | $S(=O)CH_3$ | F | 1 | $CH_3$ | F | O |
| 242 | $CH_2CH=CH_2$ | $S(=O)CH_3$ | F | 1 | $CH_3$ | F | O |
| 243 | $CH_3$ | $S(=O)CH_2CH_3$ | F | 0 | $CH_3$ | F | O |
| 244 | $CH_3$ | $S(=O)CH_2CH_3$ | F | 1 | $CH_3$ | F | O |
| 245 | $CH_3$ | $S(=O)CH(CH_3)_2$ | F | 1 | $CH_3$ | F | O |
| 246 | $CH_3$ | $S(=O)CH_2CH_2CH_3$ | F | 1 | $CH_3$ | F | O |
| 247 | $CH_3$ | $S(=O)CH_2$-Cyclopropyl | F | 0 | $CH_3$ | F | O |
| 248 | $CH_3$ | $S(=O)CH_2$-Cyclopropyl | F | 1 | $CH_3$ | F | O |
| 249 | $CH_2CH=CH_2$ | $S(=O)CH(CH_3)_2$ | F | 1 | $CH_3$ | F | O |
| 250 | $CH_3$ | S(=O)-Benzyl | F | 1 | $CH_3$ | F | O |
| 251 | $CH_2CH_3$ | $S(=O)CH_2CH_2CH_3$ | F | 1 | $CH_3$ | F | O |
| 252 | $CH_2CH_3$ | $S(=O)CH_2CH_3$ | F | 1 | $CH_3$ | F | O |
| 253 | $CH_2CH_3$ | $S(=O)CH_2CH_3$ | F | 0 | $CH_3$ | F | O |
| 254 | Cyclopropyl | $SCH_2CF_3$ | F | 0 | $CH_3$ | F | O |
| 255 | Cyclopropyl | $SCH_2CF_3$ | F | 1 | $CH_3$ | F | O |
| 256 | Cyclopropyl | $SCH_2CHF_2$ | F | 1 | $CH_3$ | F | O |
| 257 | Cyclopropyl | $SCH_3$ | F | 1 | $CH_3$ | F | O |
| 258 | Cyclopropyl | $SCH_2CHF_2$ | F | 0 | $CH_3$ | F | O |
| 259 | Cyclopropyl | $SCH_3$ | F | 0 | $CH_3$ | F | O |
| 260 | $CH_3$ | $SCH_3$ | F | 1 | $CH_3$ | F | O |
| 261 | Cyclopropyl | $SCH_2CH_3$ | F | 0 | $CH_3$ | F | O |
| 262 | Cyclopropyl | $SCH_2CH_3$ | F | 1 | $CH_3$ | F | O |

(fortgesetzt)

| Beispiel-Nr. | $R^1$ | $R^2$ | W | n | Y | X | V |
|---|---|---|---|---|---|---|---|
| 263 | Cyclopropyl | $SCF_3$ | F | 0 | $CH_3$ | F | O |
| 264 | Cyclopropyl | $SCF_3$ | F | 1 | $CH_3$ | F | O |
| 265 | Cyclopropyl | $SCH_2CH_2CH_3$ | F | 0 | $CH_3$ | F | O |
| 266 | Cyclopropyl | $SCH_2CH_2CH_3$ | F | 1 | $CH_3$ | F | O |
| 267 | Cyclopropyl | $SCH(CH_3)_2$ | F | 1 | $CH_3$ | F | O |
| 268 | Cyclopropyl | $SCH(CH_3)_2$ | F | 0 | $CH_3$ | F | O |
| 269 | $CH_2CH_3$ | $SCH_3$ | F | 0 | $CH_3$ | F | O |
| 270 | $CH_2CH_3$ | $SCH_3$ | F | 1 | $CH_3$ | F | O |
| 271 | $CH_2CH=CH_2$ | $SCH_3$ | F | 0 | $CH_3$ | F | O |
| 272 | $CH_2CH=CH_2$ | $SCH_3$ | F | 1 | $CH_3$ | F | O |
| 273 | $CH_3$ | $SCH_2CH_3$ | F | 0 | $CH_3$ | F | O |
| 274 | $CH_3$ | $SCH_2CH_3$ | F | 1 | $CH_3$ | F | O |
| 275 | $CH_3$ | $SCH(CH_3)_2$ | F | 1 | $CH_3$ | F | O |
| 276 | $CH_3$ | $SCH(CH_3)_2$ | F | 0 | $CH_3$ | F | O |
| 277 | $CH_3$ | $SCH_2CH_2CH_3$ | F | 0 | $CH_3$ | F | O |
| 278 | $CH_3$ | $SCH_2CH_2CH_3$ | F | 1 | $CH_3$ | F | O |
| 279 | $CH_3$ | $SCH_2$-Cyclopropyl | F | 0 | $CH_3$ | F | O |
| 280 | $CH_3$ | $SCH_2$-Cyclopropyl | F | 1 | $CH_3$ | F | O |
| 281 | $CH_3$ | $SCH_2CF_3$ | F | 0 | $CH_3$ | F | O |
| 282 | $CH_3$ | $SCH_2CF_3$ | F | 1 | $CH_3$ | F | O |
| 283 | $CH_3$ | $SCF_3$ | F | 0 | $CH_3$ | F | O |
| 284 | $CH_3$ | $SCF_3$ | F | 1 | $CH_3$ | F | O |
| 285 | $CH_2CH=CH_2$ | $SCH(CH_3)_2$ | F | 1 | $CH_3$ | F | O |
| 286 | $CH_3$ | S-Benzyl | F | 0 | $CH_3$ | F | O |
| 287 | $CH_3$ | S-Benzyl | F | 1 | $CH_3$ | F | O |
| 288 | $CH_2CH_3$ | $SCH_2CH_2CH_3$ | F | 1 | $CH_3$ | F | O |
| 289 | $CH_3$ | $SCH_2CHF_2$ | F | 0 | $CH_3$ | F | O |
| 290 | $CH_3$ | $SCH_2CH=CH_2$ | F | 0 | $CH_3$ | F | O |
| 291 | $CH_3$ | $SCH_2CH=CH_2$ | F | 1 | $CH_3$ | F | O |
| 292 | $CH_2CH_3$ | $SCH_2CH_3$ | F | 0 | $CH_3$ | F | O |
| 293 | $CH_2CH_3$ | $SCH_2CH_3$ | F | 1 | $CH_3$ | F | O |
| 294 | Cyclopropyl | Br | F | 0 | $CH_3$ | F | O |
| 295 | Cyclopropyl | Br | F | 1 | $CH_3$ | F | O |

(Verbindung der allgemeinen Formel (I) mit (I-A) mit Z = H und W = F)

| Beispiel-Nr. | $R^1$ | $R^2$ | n | Y | X | V |
|---|---|---|---|---|---|---|
| 345 | $CH_2CH{=}CH_2$ | $CF_3$ | 0 | $CH_3$ | F | O |
| 346 | $CH_3$ | Cyclopropyl | 0 | $CH_3$ | F | O |
| 347 | $CH(CH_3)_2$ | Cyclopropyl | 0 | $CH_3$ | F | O |
| 348 | 3-Pyridyl | $CH_2CH_3$ | 0 | $CH_3$ | F | O |
| 349 | 4-Fluorphenyl | H | 0 | $CH_3$ | F | O |
| 350 | Phenyl | $CH_3$ | 0 | $CH_3$ | F | O |
| 351 | Phenyl | H | 0 | $CH_3$ | F | O |
| 352 | $CH_3$ | Cyclopropyl | 1 | $CH_3$ | F | O |
| 353 | $CH(CH_3)_2$ | Cyclopropyl | 1 | $CH_3$ | F | O |
| 354 | 3-Pyridyl | $CH_2CH_3$ | 1 | $CH_3$ | F | O |
| 355 | 4-Fluorphenyl | H | 1 | $CH_3$ | F | O |
| 356 | Phenyl | $CH_3$ | 1 | $CH_3$ | F | O |
| 357 | Phenyl | H | 1 | $CH_3$ | F | O |
| 358 | Cyclopropyl | Cyclopentyl | 0 | $CH_3$ | F | O |
| 359 | Cyclopropyl | $CH_2CH_2OCH(CH_3)_2$ | 0 | $CH_3$ | F | O |
| 360 | Cyclopropyl | Cyclopentyl | 1 | $CH_3$ | F | O |
| 361 | Cyclopropyl | Cyclohexyl | 1 | $CH_3$ | F | O |
| 362 | Cyclopropyl | $CH_2CH_2OCH(CH_3)_2$ | 1 | $CH_3$ | F | O |
| 363 | $CH(CH_3)_2$ | $CF_3$ | 1 | $CH_3$ | F | O |
| 364 | $CH_2CH{=}CH_2$ | $CF_3$ | 1 | $CH_3$ | F | O |
| 365 | $CH_2CH_2CH_2CH_3$ | $CF_3$ | 1 | $CH_3$ | F | O |
| 366 | Cyclopropyl | 2-Methoxyphenyl | 0 | $CH_3$ | F | O |
| 367 | $C(CH_3)_3$ | H | 0 | $CH_3$ | F | O |
| 368 | Benzyl | H | 0 | $CH_3$ | F | O |
| 369 | $C(CH_3)_3$ | H | 1 | $CH_3$ | F | O |
| 370 | Benzyl | H | 1 | $CH_3$ | F | O |
| 371 | $C(CH_3)_3$ | H | 0 | $CH_3$ | $CH_3$ | O |
| 372 | $C(CH_3)_3$ | H | 1 | $CH_3$ | $CH_3$ | O |
| 373 | Benzyl | H | 0 | $CH_3$ | $CH_3$ | O |
| 374 | Benzyl | H | 1 | $CH_3$ | $CH_3$ | O |
| 375 | Cyclopropyl | $OCH_2CH{=}CH_2$ | 0 | $CH_3$ | F | O |
| 376 | $CH_3$ | $OCH_2CCl_3$ | 0 | $CH_3$ | F | O |
| 377 | Cyclopropyl | $OCH_2CF_3$ | 0 | $CH_3$ | $CH_3$ | O |
| 378 | Cyclopropyl | $OCH_2CF_3$ | 0 | Br | H | O |
| 379 | Cyclopropyl | $OCH_3$ | 1 | $CH_3$ | F | O |
| 380 | Cyclopropyl | $OCH_3$ | 1 | $CH_3$ | F | O |
| 381 | Cyclopropyl | $OCH_2CF_3$ | 1 | $CH_3$ | $CH_3$ | O |
| 382 | Cyclopropyl | $OCH_2CF_3$ | 1 | Cl | F | O |

(fortgesetzt)

| Beispiel-Nr. | $R^1$ | $R^2$ | n | Y | X | V |
|---|---|---|---|---|---|---|
| 383 | Cyclopropyl | $OCH_2CF_3$ | 1 | Br | F | O |
| 384 | Cyclopropyl | $OCH_2CF_3$ | 1 | Cl | $CH_3$ | O |
| 385 | Cyclopropyl | $OCH_2CF_3$ | 1 | $OCH_3$ | F | O |
| 386 | Cyclopropyl | $OCH_2C(CH_3)_3$ | 0 | $CH_3$ | F | O |
| 387 | Cyclopropyl | $OCH_2C(CH_3)_3$ | 1 | $CH_3$ | F | O |
| 388 | $CH_2CH_2CH_2OCH_3$ | $OCH_3$ | 0 | $CH_3$ | F | O |
| 389 | $CH_2CH_2CH_3$ | $OCH_3$ | 0 | $CH_3$ | F | O |
| 390 | $CH_2$-Cyclopropyl | $OCH_3$ | 0 | $CH_3$ | F | O |
| 391 | Cyclopropyl | $OCH_2CH=CH_2$ | 1 | $CH_3$ | F | O |
| 392 | $CH_3$ | $OCH_2CCl_3$ | 1 | $CH_3$ | F | O |
| 393 | $CH_2CF_3$ | $OCH_3$ | 0 | $CH_3$ | F | O |
| 394 | Cyclopropyl | $OCH_2C(CH_3)_3$ | 0 | Cl | F | O |
| 395 | Cyclopropyl | $OCH_2C(CH_3)_3$ | 0 | $OCH_3$ | F | O |
| 396 | Cyclopropyl | $OCH_2C(CH_3)_3$ | 0 | $CH_3$ | Cl | O |
| 397 | Cyclopropyl | $OCH_2C(CH_3)_3$ | 0 | Cl | Cl | O |
| 398 | $CH_2CH_3$ | $OCH_2CF_3$ | 0 | $CH_3$ | F | O |
| 399 | $CH_2CH=CH_2$ | $OCH_2CF_3$ | 0 | $CH_3$ | F | O |
| 400 | $CH_2CH_3$ | $OCH_2CH_2CH_3$ | 0 | $CH_3$ | F | O |
| 401 | $CH_2CH_3$ | $OCH_2CH_3$ | 0 | $CH_3$ | F | O |
| 402 | Cyclopropyl | $OCH_2C(CH_3)_3$ | 1 | Cl | F | O |
| 403 | Cyclopropyl | $OCH_2C(CH_3)_3$ | 1 | $OCH_3$ | F | O |
| 404 | Cyclopropyl | $OCH_2C(CH_3)_3$ | 1 | $CH_3$ | Cl | O |
| 405 | Cyclopropyl | $OCH_2C(CH_3)_3$ | 1 | Cl | Cl | O |
| 406 | $CH_2CH_3$ | $OCH_2CF_3$ | 1 | $CH_3$ | F | O |
| 407 | $CH_2CH=CH_2$ | $OCH_2CF_3$ | 1 | $CH_3$ | F | O |
| 408 | $CH_2CH_3$ | $OCH_2CH_2CH_3$ | 1 | $CH_3$ | F | O |
| 409 | $CH_2CH_3$ | $OCH_2CH_3$ | 1 | $CH_3$ | F | O |
| 410 | Cyclopropyl | $OCH_2$Phenyl | 0 | $CH_3$ | F | O |
| 411 | $CH_2CH_2CH_3$ | $OCH_3$ | 1 | $CH_3$ | F | O |
| 412 | $CH_2$-Cyclopropyl | $OCH_3$ | 1 | $CH_3$ | F | O |
| 413 | $CH(CH_3)_2$ | $OCH_3$ | 1 | $CH_3$ | F | O |
| 414 | Cyclopropyl | $OCH_2$Phenyl | 1 | $CH_3$ | F | O |
| 415 | $CH_2CF_3$ | $OCH_3$ | 1 | $CH_3$ | F | O |
| 416 | Cyclopropyl | $N(CH_3)_2$ | 0 | $CH_3$ | F | O |
| 417 | $NH_2$ | $CH(CH_3)_2$ | 2 | $CH_3$ | F | O |
| 418 | $OCH_3$ | $CH(CH_3)_2$ | 0 | $CH_3$ | F | O |
| 419 | $OCH_3$ | $CH(CH_3)_2$ | 1 | $CH_3$ | F | O |

(fortgesetzt)

| Beispiel-Nr. | $R^1$ | $R^2$ | n | Y | X | V |
|---|---|---|---|---|---|---|
| 420 | $CH_3$ | $SO_2CH_3$ | 0 | $CH_3$ | F | O |
| 421 | $CH_3$ | $SO_2CH_3$ | 1 | $CH_3$ | F | O |
| 422 | $CH_2CH_3$ | $SO_2CH_3$ | 1 | $CH_3$ | F | O |
| 423 | $CH_2CH=CH_2$ | $SOCH_2CH_2CH_3$ | 1 | $CH_3$ | F | O |
| 424 | $CH_2CH=CH_2$ | $SOCH_2CH_3$ | 1 | $CH_3$ | F | O |
| 425 | Cyclopropyl | $SCH_2CH=CH_2$ | 0 | $CH_3$ | F | O |
| 426 | $CH_2CH_3$ | $SCH_2CH_2CH_3$ | 0 | $CH_3$ | F | O |
| 427 | $CH_2CH=CH_2$ | $SCH_2CH_2CH_3$ | 0 | $CH_3$ | F | O |
| 428 | $CH_2CH=CH_2$ | $SCH_2CH_3$ | 0 | $CH_3$ | F | O |
| 429 | $CH_2CH=CH_2$ | $SCH_2CH=CH_2$ | 0 | $CH_3$ | F | O |
| 430 | $CH_2CH=CH_2$ | $SCH_2CH_2CH_3$ | 1 | $CH_3$ | F | O |
| 431 | $CH_2CH=CH_2$ | $SCH_2CH_3$ | 1 | $CH_3$ | F | O |
| 432 | $CH_2CH=CH_2$ | $SCH_2CH=CH_2$ | 1 | $CH_3$ | F | O |
| 433 | $CH_3$ | Br | 1 | $CH_3$ | F | O |
| 434 | $CH_2CH_3$ | Cl | 0 | $CH_3$ | F | O |
| 435 | $CH_3$ | Cl | 0 | $CH_3$ | F | O |
| 436 | $CH_2CH_3$ | Cl | 1 | $CH_3$ | F | O |
| 437 | $CH_3$ | Cl | 1 | $CH_3$ | F | O |
| 438 | $C(CH_3)_3$ | Br | 0 | $CH_3$ | F | O |
| 439 | $CH(CH_3)_2$ | Br | 0 | $CH_3$ | F | O |
| 440 | Benzyl | Br | 0 | $CH_3$ | F | O |
| 441 | $C(CH_3)_3$ | Br | 1 | $CH_3$ | F | O |
| 442 | $CH(CH_3)_2$ | Br | 1 | $CH_3$ | F | O |
| 443 | Benzyl | Br | 1 | $CH_3$ | F | O |
| 492 | Cyclopropyl | $CH_2OCH_3$ | 0 | $CH_3$ | F | S |
| 493 | Cyclopropyl | $CH_2CH(CH_3)_2$ | 0 | $CH_3$ | F | S |
| 494 | Cyclopropyl | $CH(CH_3)_2$ | 0 | $CH_3$ | F | S |
| 495 | Cyclopropyl | $CH_2CH_2CH_2CH_3$ | 0 | $CH_3$ | F | S |
| 496 | Cyclopropyl | Cyclohexyl | 0 | $CH_3$ | F | S |
| 497 | Cyclopropyl | Cyclopentyl | 0 | $CH_3$ | F | S |
| 498 | CH(CH3)2 | Cyclopropyl | 0 | $CH_3$ | F | S |
| 499 | Cyclopropyl | $CH(CH_3)CH_2CH_3$ | 0 | $CH_3$ | F | S |
| 500 | Cyclopropyl | $CH_2OCH_2CH_3$ | 0 | $CH_3$ | F | S |
| 501 | Cyclopropyl | $CH=CHCH_3$ | 0 | $CH_3$ | F | S |
| 502 | Cyclopropyl | $CH_2CH_3$ | 0 | $CH_3$ | F | S |
| 503 | Cyclopropyl | $CH_2CH_2CH_3$ | 0 | $CH_3$ | F | S |
| 504 | Cyclopropyl | Cyclopropyl | 0 | $CH_3$ | F | S |
| 505 | Cyclopropyl | Cyclobutyl | 0 | $CH_3$ | F | S |

(fortgesetzt)

| Beispiel-Nr. | $R^1$ | $R^2$ | n | Y | X | V |
|---|---|---|---|---|---|---|
| 506 | Cyclopropyl | 2-Methoxyphenyl | 0 | $CH_3$ | F | S |
| 507 | $CH_2CH_3$ | $CF_3$ | 0 | OCH3 | H | S |
| 508 | $CH_2CF_3$ | $OCH_3$ | 1 | $CH_3$ | F | O |
| 509 | $CH_2CF_3$ | $OCH_3$ | 1 | $CH_3$ | F | O |

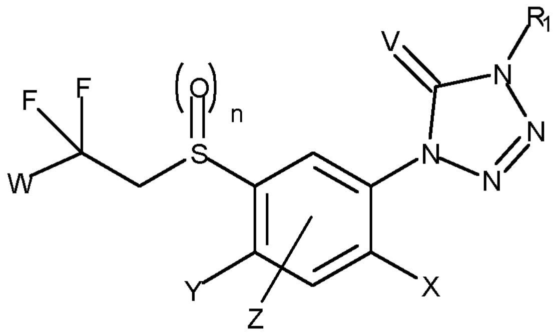

(I) mit (I-B)

(Verbindung der allgemeinen Formel (I) mit (I-B) mit Z = H)

| Beispiel-Nr. | $R^1$ | W | n | Y | X | V |
|---|---|---|---|---|---|---|
| 296 | $CH_3$ | F | 0 | $CH_3$ | H | O |
| 297 | $CH_3$ | F | 1 | $CH_3$ | $CH_3$ | O |
| 298 | $CH(CH_3)_2$ | F | 0 | $CH_3$ | $CH_3$ | O |
| 299 | H | F | 0 | $CH_3$ | F | O |
| 300 | $CH(CH_3)_2$ | F | 1 | $CH_3$ | $CH_3$ | O |
| 301 | $CH_3$ | F | 1 | $CH_3$ | F | O |
| 302 | $CH_3$ | F | 0 | $CH_3$ | F | O |
| 303 | H | F | 1 | $CH_3$ | F | O |
| 304 | $CH_2CF_3$ | F | 0 | $CH_3$ | F | O |
| 305 | $CH_2CF_3$ | F | 1 | $CH_3$ | F | O |
| 306 | 4-Chlorphenyl | F | 0 | $CH_3$ | F | O |
| 307 | 4-Chlorphenyl | F | 1 | $CH_3$ | F | O |

(Verbindung der allgemeinen Formel (I) mit (I-B) mit Z = H und W = F)

| Beispiel-Nr. | $R^1$ | n | Y | X | V |
|---|---|---|---|---|---|
| 444 | $CH_2CF_2CF_3$ | 0 | $CH_3$ | F | O |
| 445 | 4-Trifluormethylphenyl | 1 | $CH_3$ | F | O |
| 446 | $CH_2CF_2CF_3$ | 1 | $CH_3$ | F | O |
| 447 | $CH_3$ | 0 | $CH_3$ | F | S |

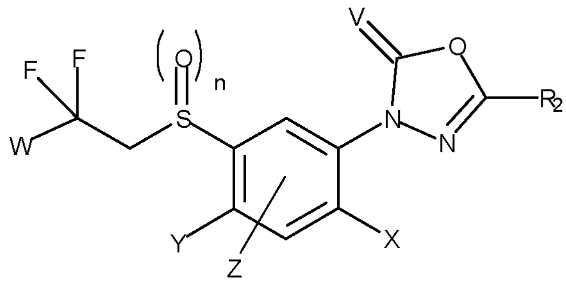

(I) mit (I-C)

(Verbindung der allgemeinen Formel (I) mit (I-C) mit Z = H)

| Beispiel-Nr. | $R^2$ | W | n | Y | X | V |
|---|---|---|---|---|---|---|
| 308 | $CF_3$ | F | 0 | CN | F | O |
| 309 | $CF_3$ | F | 1 | CN | F | O |

(Verbindung der allgemeinen Formel (I) mit (I-C) mit Z = H und W = F)

| Beispiel-Nr. | $R^2$ | n | Y | X | V |
|---|---|---|---|---|---|
| 448 | $CH_3$ | 0 | $CH_3$ | F | O |
| 449 | $C(CH_3)_3$ | 0 | $CH_3$ | F | O |
| 450 | $CH_2OCH_3$ | 0 | $CH_3$ | F | O |
| 451 | $CH_3$ | 1 | $CH_3$ | F | O |
| 452 | $C(CH_3)_3$ | 1 | $CH_3$ | F | O |
| 453 | $CH_2OCH_3$ | 1 | $CH_3$ | F | O |
| 454 | H | 0 | $CH_3$ | F | O |
| 455 | $CH_2CH_3$ | 0 | $CH_3$ | F | O |
| 456 | Cyclopropyl | 0 | $CH_3$ | F | O |
| 457 | Phenyl | 0 | $CH_3$ | F | O |
| 458 | 4-Chlorphenyl | 0 | $CH_3$ | F | O |
| 459 | 4-Pyridyl | 0 | $CH_3$ | F | O |
| 460 | $CH_2CH_2CH_3$ | 0 | $CH_3$ | F | O |
| 461 | $CH_2CH_2CH_2CH_3$ | 0 | $CH_3$ | F | O |
| 462 | $CH(CH_3)_2$ | 0 | $CH_3$ | F | O |
| 463 | $CH_2OCH_2CH_3$ | 0 | $CH_3$ | F | O |
| 464 | 3-Fluorphenyl | 0 | $CH_3$ | F | O |
| 465 | Cyclopropyl | 1 | $CH_3$ | F | O |
| 466 | 4-Methoxyphenyl | 0 | $CH_3$ | F | O |
| 467 | 4-Nitrophenyl | 0 | $CH_3$ | F | O |
| 468 | 2-Thienyl | 0 | $CH_3$ | F | O |
| 469 | 2-Furyl | 0 | $CH_3$ | F | O |

(fortgesetzt)

| | | | | | |
|---|---|---|---|---|---|
| 470 | $CH_2CH_2CH_3$ | 1 | $CH_3$ | F | O |
| 471 | $CH_2CH_2CH_2CH_3$ | 1 | $CH_3$ | F | O |
| 472 | $CH(CH_3)_2$ | 1 | $CH_3$ | F | O |
| 473 | $CH_2OCH_2CH_3$ | 1 | $CH_3$ | F | O |
| 474 | 4-Methoxyphenyl | 1 | $CH_3$ | F | O |
| 475 | 4-Nitrophenyl | 1 | $CH_3$ | F | O |
| 476 | 2-Thienyl | 1 | $CH_3$ | F | O |
| 477 | 2-Furyl | 1 | $CH_3$ | F | O |
| 478 | Benzyl | 1 | $CH_3$ | F | O |
| 479 | $CH_2CH_2CH_2Cl$ | 0 | $CH_3$ | F | O |
| 480 | $CH_2CH_2CH_2Cl$ | 1 | $CH_3$ | F | O |
| 481 | H | 1 | $CH_3$ | F | O |
| 482 | Cyclopropyl | 0 | $CH_3$ | $CH_3$ | O |
| 483 | $C(CH_3)_3$ | 0 | $CH_3$ | $CH_3$ | O |
| 484 | Cyclopropyl | 1 | $CH_3$ | $CH_3$ | O |
| 485 | $C(CH_3)_3$ | 1 | $CH_3$ | $CH_3$ | O |

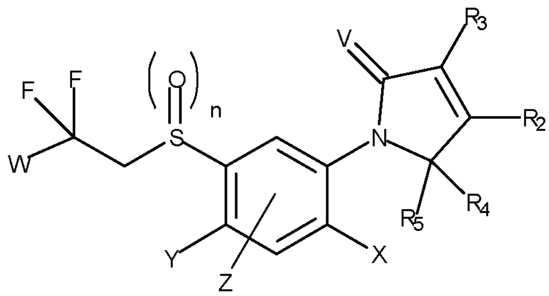

(I) mit (I-D)

(Verbindung der allgemeinen Formel (I) mit (I-D) mt Z = H)

| Beispiel-Nr. | $R^2$ | W | n | Y | X | V | $R^3$ | $R^4$ | $R^5$ |
|---|---|---|---|---|---|---|---|---|---|
| 310 | $OCH_3$ | F | 0 | $CH_3$ | F | O | H | H | H |
| 311 | $OCH_3$ | F | 1 | $CH_3$ | F | O | H | H | H |
| 312 | $OCH_3$ | F | 0 | $CH_3$ | $CH_3$ | O | H | H | H |
| 313 | $OCH_3$ | F | 1 | $CH_3$ | $CH_3$ | O | H | H | H |
| 314 | $OCH_3$ | F | 1 | Cl | Cl | O | H | H | H |
| 315 | $OCH_3$ | F | 1 | Cl | H | O | H | H | H |
| 316 | $OCH_3$ | F | 0 | $CH_3$ | H | O | H | H | H |
| 317 | $OCH_3$ | F | 1 | $CH_3$ | H | O | H | H | H |
| 318 | $OCH_3$ | F | 1 | $CH_3$ | Cl | O | H | H | H |
| 319 | $OCH_3$ | F | 0 | $CH_3$ | Cl | O | H | H | H |
| 320 | $OCH_3$ | F | 0 | CN | H | O | H | H | H |

(fortgesetzt)

| Beispiel-Nr. | $R^2$ | W | n | Y | X | V | $R^3$ | $R^4$ | $R^5$ |
|---|---|---|---|---|---|---|---|---|---|
| 321 | $OCH_3$ | F | 1 | $CF_3$ | H | O | H | H | H |
| 322 | $OCH_3$ | F | 0 | $CF_3$ | H | O | H | H | H |
| 323 | O-Benzyl | F | 1 | $CH_3$ | F | O | H | H | H |
| 324 | $OCH_3$ | F | 1 | Cl | F | O | H | H | H |
| 325 | $OCH(CH_3)_2$ | F | 1 | $CH_3$ | F | O | H | H | H |
| 326 | $OCH_2CH_2CH_2CH_3$ | F | 1 | $CH_3$ | F | O | H | H | H |
| 327 | $OCH_3$ | F | 0 | Cl | F | O | H | H | H |
| 328 | $OCH_2CH_2OCH_3$ | F | 0 | $CH_3$ | F | O | H | H | H |
| 329 | O-Cyclopentyl | F | 0 | $CH_3$ | F | O | H | H | H |
| 330 | $OCH_2CH_3$ | F | 0 | $CH_3$ | F | O | H | H | H |
| 331 | $OCH_2$-Cyclopropyl | F | 0 | $CH_3$ | F | O | H | H | H |
| 332 | $OCH_2$-Cyclopropyl | F | 1 | $CH_3$ | F | O | H | H | H |
| 333 | $OCH_2CH_2CH_3$ | F | 1 | $CH_3$ | F | O | H | H | H |
| 334 | $OCH_2CH_3$ | F | 1 | $CH_3$ | F | O | H | H | H |
| 335 | $OCH_2CH_2OCH_3$ | F | 1 | $CH_3$ | F | O | H | H | H |
| 336 | O-Cyclopentyl | F | 1 | $CH_3$ | F | O | H | H | H |

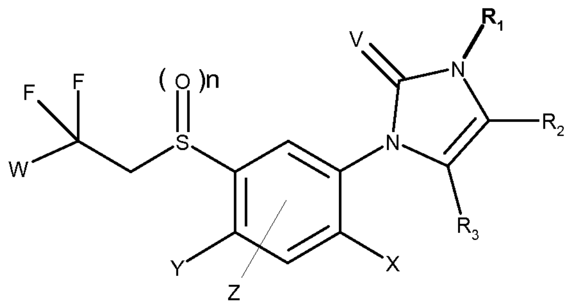

(I) mit (I-E)

(Verbindung der allgemeinen Formel (I) mit (I-E) mit Z = H und W = F)

| Beispiel-Nr. | $R^1$ | $R^2$ | $R^3$ | n | Y | X | V |
|---|---|---|---|---|---|---|---|
| 486 | $CH_2CH_3$ | H | H | 0 | $CH_3$ | F | O |
| 487 | $CH_2CH_3$ | H | H | 1 | $CH_3$ | F | O |
| 488 | $C(CH_3)_3$ | H | H | 0 | $CH_3$ | F | O |
| 489 | $C(CH_3)_3$ | H | H | 1 | $CH_3$ | F | O |

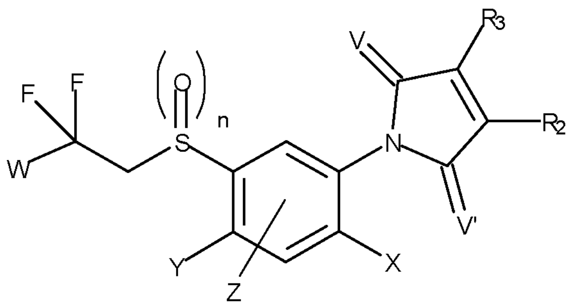

(I) mit (I-F)

(Verbindung der allgemeinen Formel (I) mit (I-F) mit Z = H)

| Beispiel-Nr. | $R^2$ | W | n | Y | X | V | $R^3$ | V' |
|---|---|---|---|---|---|---|---|---|
| 337 | Br | F | 0 | $CH_3$ | F | O | H | O |
| 338 | Cl | F | 0 | $CH_3$ | F | O | Cl | O |
| 339 | $CH_3$ | F | 0 | $CH_3$ | F | O | $CH_3$ | O |
| 340 | $CH_3$ | F | 0 | $CH_3$ | F | O | H | O |
| 341 | $CH_3$ | F | 1 | $CH_3$ | F | O | $CH_3$ | O |
| 342 | $CH_3$ | F | 0 | $CH_3$ | $CH_3$ | O | $CH_3$ | O |

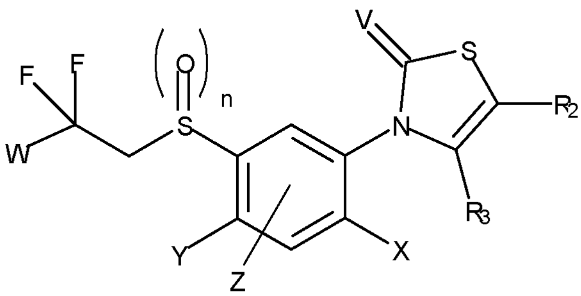

(I) mit (I-G)

(Verbindung der allgemeinen Formel (I) mit (I-G) mit Z = H)
für V = $NR^{15}$

| Beispiel-Nr. | $R^2$ | W | n | Y | X | V | $R^3$ | $R^{15}$ |
|---|---|---|---|---|---|---|---|---|
| 343 | $CH_3$ | F | 0 | $CH_3$ | F | N | $CH_3$ | $CH_2CF_3$ |
| 344 | $CH_3$ | F | 1 | $CH_3$ | F | N | $CH_3$ | $CH_2CF_3$ |

(Verbindung der allgemeinen Formel (I) mit (I-G) mit Z = H und W = F)
für V = $NR^{15}$

| Beispiel-Nr. | $R^2$ | n | Y | X | V | $R^3$ | $R^{15}$ |
|---|---|---|---|---|---|---|---|
| 490 | H | 0 | $CH_3$ | F | N | H | $CH_2CF_3$ |
| 491 | H | 1 | $CH_3$ | F | N | H | $CH_2CF_3$ |

**Spektroskopische Daten ausgewählter Beispiele:**

| Bsp.-Nr. | logP[a] | logP[b] | $^1$H-NMR(D6-DMSO, 400MHz) δ ppm |
|---|---|---|---|
| 1 | 1,61 | 1,59 | 8,37(d,1H), 8,28(d,1H), 4,49-4,26(m,2H), 3,23(s,3H), 2,31(s,3H) |
| 2 | 1,88 | 1,74 | 11,99(s,1H), 8,14(d,1H), 8,07(d,1H), 4,16(q,2H), 2,18(s,3H) |
| 3 | 1,95 | 2,50 | 7,70-7,68(m,1H), 7,41(m,1H), 6,20(tt,1H), 5,05-4,98(sept,1H), 3,44(dt,2H), 2,33(s,3H), 1,48-1,46(d,6H) |
| 4 | 2,82 | 2,97 | 8,36(s,1H), 7,73-7,71(m,1H), 7,41-7,38(m,1H), 4,17(sept,1H), 3,99-3,91(q,2H), 2,43(s,3H), 1,38-1,36(d,6H) |
| 5 | 2,55 | 2,50 | 8,35(s,1H), 7,64-7,62(m,1H), 7,38-7,35(m,1H), 6,19(tt,1H), 4,20-4,13(sept,1H), 3,51-3,42(dt,2H), 2,41(s,3H), 1,38-1,36(d,6H) |
| 6 | 1,88 | 2,02 | 8,42(s,1H), 7,99-7,97(m,1H), 7,53-7,50(m,1H), 4,26-4,02(m,3H), 2,42(s,3H), 1,39-1,37(d,6H) |
| 7 | 2,00 | 2,16 | 11,89(s,1H), 8,10(s,1H), 7,71-7,70(m,1H), 7,40-7,37(m,1H), 3,99-3,91(q,2H), 2,43(s,3H) |
| 8 | 1,58 | 1,60 | 8,40(s,1H), 7,95-7,93(m,1H), 7,50-7,47(m,1H), 6,44(tt,1H), 4,21-4,14(sept,1H), 3,73-3,65(m,1H), 3,54-3,41(m,1H), 2,39(s,3H), 1,39-1,37(d,6H) |
| 9 | 3,58 | 3,53 | 7,79(d,1H), 7,47(d,1H), 3,95(q,2H), 3,38(s,3H), 2,45(s,3H) |
| 10 | 2,42 | 2,38 | 7,68(d,1H), 7,38(d,1H), 3,93(q,2H), 3,20(s,3H), 2,43(s,3H), 2,25(s,3H) |
| 11 | 1,57 | 1,50 | 7,94(d,1H), 7,49(d,1H), 4,28-4,16(m,1H), 4,10-3,98(m,1H), 3,21(s,3H), 2,41(s,3H), 2,27(s,3H) |
| 12 | 2,52 | 2,46 | 8,04(d,1H), 7,58(d,1H), 4,32-4,20(m,1H), 4,09-3,98(m,1H), 3,38(s,3H), 2,44(s,3H) |
| 13 | 4,07 | 3,99 | 7,98(d,1H), 7,85-7,81(m,1H), 7,80-7,75(m,2H), 7,57-7,53(m,1H), 7,19(d,1H), 3,89(s,3H), 3,89(q,2H), 3,19(s,3H) |
| 14 | 2,81 | 2,77 | 8,05(d,1H), 7,76-7,72(m,1H), 7,34(d,1H), 3,89(q,2H), 3,20(s,3H), 2,38(s,3H), 2,28(s,3H) |
| 15 | 3,53 | 3,46 | 7,82-7,76(m,3H), 7,61-7,56(m,3H), 7,44(d,1H), 3,96(q,2H), 3,37(s,3H), 2,45(s,3H) |
| 16 | 4,60 | 4,52 | 8,10(d,1H), 7,80-7,74(m,3H), 7,58-7,54(m,1H), 7,39(d,1H), 3,93(q,2H), 3,19(s,3H), 2,40(s,3H) |
| 17 | 3,28 | 3,22 | 8,03(d,1H), 7,73-7,69(m,1H), 7,33(d,1H), 3,88(q,2H), 2,90-2,83(m,1H), 2,37(s,3H), 2,32(s,3H), 0,99-0,94(m,4H) |
| 18 | 4,29 | 4,22 | 8,07(d,1H), 7,72-7,68(m,1H), 7,33(d,1H), 3,88(q,2H), 3,20-3,11(m,1H), 2,94-2,88(m,1H), 2,38(s,3H), 1,30(d,6H), 1,02-0,97(m,4H) |
| 19 | 3,38 | 3,31 | 8,04(d,1H), 7,73-7,69(m,1H), 7,36(d,1H), 4,47(s,2H), 3,90(q,2H), 3,36(s,3H), 2,92-2,85(m,1H), 2,38(s,3H), 1,07-1,00(m,2H), 0,99-0,92(m,2H) |
| 20 | 3,02 | 2,96 | 8,20(s,1H), 8,03(d,1H), 7,75-7,71(m,1H), 7,35(d,1H), 3,92(q,2H), 3,08-3,00(m,1H), 2,38(s,3H), 0,95-0,91(m,4H) |
| 21 | 3,96 | 3,90 | 8,02(d,1H), 7,67-7,70(m,1H), 7,42(d,1H), 3,94(q,2H), 3,36(s,3H), 2,41(s,3H) |
| 22 | 5,30 | 5,24 | 8,00(d,1H), 7,67-7,70(m,1H), 7,42(d,1H), 3,94(q,2H), 2,41(s,3H), 1,67(s,9H) |
| 23 | 2,48 | 2,43 | 8.06(d,1H), 7,78-7,80(m,2H), 7,53-7,61(m,4H), 4,19-4,28(m,1H), 4,02-4,13(m,1H), 3,37(s, 3H), 2,44(s,3H) |
| 24 | 2,59 | 2,53 | 7,85(s,1H), 7,78-7,80(m,2H), 7,57-7,61(m,3H), 7,40(s,1H), 4,13-4,22(m,1H), 3,96-4,05(m,1H), 3,38(s,3H), 2,39(s,3H), 2,34(s,3H) |
| 25 | 3,30 | 3,26 | 8.41(d,1H), 8,12-8,14(m,1H), 7,77-7,81(m,2H), 7,55-7,57(m,1H), 7,49(d,1H), 4,11-4,21(m,1H), 3,96-4,05(m,1H), 3,21(s,3H), 2,38(s,3H) |

(fortgesetzt)

| Bsp.-Nr. | logP[a] | logP[b] | $^1$H-NMR(D6-DMSO, 400MHz) δ ppm |
|---|---|---|---|
| 26 | 2,13 | 2,12 | 8,34(d,1H), 8,02-8,05(m,1H), 7,42(d,1H), 4,11-4,18(m,1H), 3,90-3,97(m,1H), 2,88-2,91(m,1H), 2,35(s,3H), 2,34(s,3H), 0,97-0,99(m,4H) |
| 27 | 2,22 | 2,21 | 8,35(d,1H), 8,04-8,06(m,1H), 7,44(d,1H), 4,50(s,2H), 4,10-4,19(m,1H), 3,93-4,02(m,1H), 3,37(s,3H), 2,88-2,93(m,1H), 2,35(s,3H), 0,94-1,07(m,4H) |
| 28 | 1,94 | 1,92 | 8,38(d,1H), 8,25(s,1H), 8,02-8,05(m,1H), 7,45(d,1H), 4,10-4,19(m,1H), 3,92-4,01(m,1H), 3,03-3,09(m,1H), 2,36(s,3H), 0,93-0,95(m,4H) |
| 29 | 2,70 | 2,68 | 8,33(d,1H), 8,04-8,06(m,1H), 7,51(d,1H), 4,13-4,23(m,1H), 3,96-4,07(m,1H), 3,38(s,3H), 2,38(s,3H) |
| 30 | 3,87 | 3,82 | 8,37(d,1H), 7,99-8,01(m,1H), 7,51(d,1H), 4,15-4,19(m,1H), 3,98-4,05(m,1H), 2,39(s,3H), 1,68(s,9H) |
| 31 | 4,88 | 4,80 | 8,09(d,1H), 7,81-7,77(m,1H), 7,72(d,1H), 7,44(d,1H), 7,38(d,1H), 7,35-7,31(m,1H), 4,08(q,2H), 3,90(q,2H), 2,41(s,3H), 2,40(s,3H), 2,15(s,3H) |
| 32 | 4,35 | 4,30 | 7,99(d,1H), 7,65-7,68(m,1H), 7,41(d,1H), 3,89-3,96(q,2H), 3,01-3,07(m,1H), 2,40(s,3H), 1,00-1,09(m,4H) |
| 33 | 4,42 | 4,47 | 8,19(d,1H), 7,78-7,81(m,1H), 7,60(d,1H), 4,00-4,08(q,2H), 3,13-19(m,1H), 2,89-2,94(m,1H), 1,31(d,6H), 0,99-1,01(m,4H) |
| 34 | 3,10 | 3,01 | 8,31(d,1H), 8,02-8,00(m,1H), 7,50(d,1H), 4,22-4,13(m,1H), 4,05-3,96(m,1H), 3,08-3,03(m,1H), 2,38(s,3H), 1,13-1,01(m,4H) |
| 35 | 4,63 | 4,55 | 8,08(d,1H), 7,76-7,74(m,1H), 7,63-7,59(m,5H), 7,45(d,1H), 3,95(q,2H), 2,43(s,3H) |
| 36 | 4,33 | 4,24 | 7,90(d,1H), 7,61(s,5H), 7,51(d,1H), 3,95(q,2H), 2,48(s,3H) |
| 37 | 3,29 | 3,25 | 8,16(d,1H), 7,65-7,60(m,6H), 4,33-4,24(m,1H), 4,08-3,96(m,1H), 2,45(s,3H) |
| 38 | 3,09 | 3,08 | 8,27(d,1H), 8,14(d,1H), 4,17(q,2H), 3,32(s,3H) |
| 39 | 2,97 | 2,91 | 8,05(d,1H), 7,80-7,75(m,2H), 7,57-7,54(m,2H), 4,28-4,19(m,1H), 4,13-4,02(m,1H), 3,21(s,3H), 2,43(s,3H) |
| 40 | 4,06 | 3,96 | 7,81-7,74(m,3H), 7,56-7,53(m,1H), 7,44(d,1H), 3,96(q,2H), 3,21(s,3H), 2,45(s,3H) |
| 41 | 1,85 | 1,79 | 7,93(d,1H), 7,48(d,1H), 4,25-4,19(m,1H), 4,06-3,99(m,1H), 2,91-2,88(m,1H), 2,40(s,3H), 2,32(s,3H), 0,99-0,95(m,4H) |
| 42 | 2,76 | 2,73 | 7,66(d,1H), 7,37(d,1H), 3,92(q,2H), 2,90-2,87(m,1H), 2,42(s,3H), 2,30(s,3H), 0,97-0,95(m,4H) |
| 43 | 2,52 | 2,47 | 7,92(d,1H), 7,48(d,1H), 4,24-4,12(m,1H), 4,10-4,00(m,1H), 3,21-3,14(m,1H), 2,97-2,92(m,1H), 2,40(s,3H), 1,30-1,28(m,6H), 1,04-0,99(m,4H) |
| 44 | 3,59 | 3,52 | 7,67(d,1H), 7,37(d,1H), 3,92(q,2H), 3,19-3,12(m,1H), 2,94-2,91(m,1H), 2,43(s,3H), 1,27(d,6H), 1,00-0,98(m,4H) |
| 45 | 1,99 | 1,96 | 7,96(d,1H), 7,51(d,1H), 4,47(s,2H), 4,26-4,17(m,1H), 4,10-4,01(m,1H), 3,36(s,3H), 2,94-2,88(m,1H), 2,41(s,3H), 1,06-1,02(m,2H), 1,00-0,93(m,2H) |
| 46 | 2,91 | 2,90 | 7,70(d,1H), 7,39(d,1H), 4,44(s,2H), 3,94(q,2H), 3,36(s,3H), 2,92-2,87(m,1H), 2,43(s,3H), 1,05-0,93(m,4H) |
| 47 | 2,92 | 2,84 | 8,46(d,1H), 8,24(d,1H), 4,52-4,40(m,1H), 4,33-4,22(m,1H), 3,10-3,05(m,1H), 1,15-1,02(m,4H) |
| 48 | 4,13 | 4,09 | 7,54(s,1H), 7,30(s,1H), 3,90(q,2H), 3,10-3,04(m,1H), 2,40(s,3H), 2,14(s,3H), 1,11-1,01(s,4H) |
| 49 | 4,95 | 4,88 | 7,59(s,1H), 7,31(s,1H), 3,95(q,2H), 2,40(s,3H), 2,11(s,3H), 1,67(s,9H) |
| 50 | 4,14 | 4,07 | 7,85(s,1H), 7,66(s,1H), 4,07(q,2H), 3,85(q,2H), 2,42(s,3H), 1,29(t,3H) |

(fortgesetzt)

| Bsp.-Nr. | logP[a] | logP[b] | $^1$H-NMR(D6-DMSO, 400MHz) δ ppm |
|---|---|---|---|
| 51 | 4,43 | 4,39 | 8,12(d,1H), 7,78-7,76(m,1), 7,71-7,65(m,1H), 4,09(q,2H), 3,07-3,02(m,1H), 1,12-1,01(m,4H) |
| 52 | 3,84 | 3,79 | 7,86(d,1H), 7,72-7,69(m,1H), 7,18(d,1H), 3,93-3,83(m,5H), 3,05-3,01(m,1H), 1,11-0,99(m,4H) |
| 53 | 3,03 | 2,93 | 8,10(d,1H), 8,05-8,02(m,1H), 7,35(d,1H), 4,20-4,10(m,1H), 3,98-3,90(m,1H), 3,92(s,3H), 3,07-3,02(m,1H), 1,12-1,00(m,4H) |
| 54 | 4,38 | 4,32 | 8,02(d,1H), 7,70-7,68(m,1H), 7,41(d,1H), 3,95(q,2H), 3,83(q,2H), 2,41(s,3H), 1,28(t,3H) |
| 55 | 4,22 | 4,11 | 7,84(d,1H), 7,50(d,1H), 4,79(q,2H), 3,97(q,2H), 2,46(s,3H) |
| 56 | 3,20 | 3,09 | 8,10(d,1H), 7,61(d,1H), 4,78(q,2H), 4,32-4,20(m,1H), 4,11-3,99(m,1H), 2,45(s,3H) |
| 57 | 3,96 | 3,88 | 7,83(d,1H), 7,47(d,1H), 3,96(q,2H), 3,84(q,2H), 2,46(s,3H), 1,29(t,3H) |
| 58 | 2,88 | 2,77 | 8,07(d,1H), 7,58(d,1H), 4,31-4,20(m,1H), 4,10-3,98(m,1H), 3,84(q,2H), 2,44(s,3H), 1,29(t,3H) |
| 59 | 3,97 | 3,91 | 7,77(d,1H), 7,46(d,1H), 3,93(q,2H), 3,11-3,05(m,1H), 2,45(s,3H), 1,11-1,01(m,4H) |
| 60 | 2,93 | 2,82 | 8,02(d,1H), 7,57(d,1H), 4,31-4,21(m,1H), 4,05-3,95(m,1H), 3,10-3,0(m,1H), 2,43(s,3H), 1,10-1,02(m,4H) |
| 61 | 2,62 | 2,59 | 8,19(s,1H), 7,70(d,1H), 7,39(d,1H), 3,94(q,2H), 3,07-3,02(m,1H), 2,43(s,3H), 0,94-0,92(m,4H) |
| 62 | 1,69 | 1,69 | 8,24(s,1H), 7,96(d,1H), 7,51(d,1H), 4,26-4,17(m,1H), 4,10-4,00(m,1H), 3,08-3,03(m,1H), 2,41(s,3H), 0,94-0,92(m,4H) |
| 63 | 4,17 | 4,11 | 7,68(d,1H), 7,37(d,1H), 3,92(q,2H), 3,10-3,03(m,1H), 2,43(s,3H), 1,43(s,9H), 1,27-1,22(m,2H), 1,08-1,02(m,2H) |
| 64 | 2,95 | 2,97 | 7,92(d,1H), 7,48(d,1H), 4,27-4,15(m,1H), 4,12-4,00(m,1H), 3,10-3,04(m,1H), 2,40(s,3H), 1,44(s,9H), 1,28-1,24(m,2H), 1,08-1,02(m,2H) |
| 65 | 3,39 | 3,33 | 7,63(d,1H), 7,35(d,1H), 3,92(q,2H), 2,99-2,94(m,1H), 2,42(s,3H), 2,09-2,02(m,1H), 1,02-0,98(m,6H), 0,91-0,87(m,2H) |
| 66 | 2,31 | 2,33 | 7,89(d,1H), 7,46(d,1H), 4,26-4,14(m,1H), 4,09-3,98(m,1H), 3,01-2,95(m,1H), 2,39(s,3H), 2,11-2,03(m,1H), 1,05-0,98(m,6H), 0,95-0,88(m,2H) |
| 67 | 4,78 | 4,66 | 7,82(d,1H), 7,46(d,1H), 3,97(q,2H), 2,45(s,3H), 1,66(s,9H) |
| 68 | 3,54 | 3,49 | 8,07(d,1H), 7,57(d,1H), 4,26-4,22(m,1H), 4,09-4,02(m,1H), 2,44(s,3H), 1,67(s,9H) |
| 69 | 3,98 | 3,89 | 7,79(d,1H), 7,71-7,69(m,2H), 7,64-7,59(m,1H), 7,47-7,42(m,2H), 3,95(q,2H), 3,32-3,27(m,1H), 2,45(s,3H), 0,95-0,90(m,2H), 0,68-0,64(m,2H) |
| 70 | 2,94 | 2,87 | 8,04(d,1H), 7,71(d,2H), 7,65-7,59(m,1H), 7,54(d,1H), 7,47-7,42(m,1H), 4,28-4,19(m,1H), 4,11-4,02(m,1H), 3,31-3,26(m,1H), 2,43(s,3H), 0,95-0,89(m,2H), 0,72-0,66(m,2H) |
| 71 | 4,05 | 3,96 | 7,66(d,1H), 7,37(d,1H), 3,92(q,2H), 2,91-2,85(m,1H), 2,70-2,66(m,2H), 2,43(s,3H), 1,71-1,64(m,2H), 1,46-1,37(m,2H), 0,98-0,95(m,4H), 0,93(t,3H) |
| 72 | 2,94 | 2,88 | 7,92(d,1H), 7,48(d,1H), 4,25-4,16(m,1H), 4,10-4,01(m,1H), 2,93-2,87(m,1H), 2,70(t,2H), 2,40(s,3H), 1,73-1,65(m,2H), 1,47-1,38(m,2H), 1,01-0,92(m,7H) |
| 73 | 3,33 | 3,27 | 7,70(d,1H), 7,39(d,1H), 4,47(s,2H), 3,94(q,2H), 3,56(q,2H), 2,93-2,88(m,1H), 2,43(s,3H), 1,17(t,3H), 1,06-1,00(m,2H), 0,99-0,93(m,2H) |
| 74 | 2,31 | 2,25 | 7,96(d,1H), 7,50(d,1H), 4,49(s,2H), 4,26-4,17(m,1H), 4,11-4,00(m,1H), 3,57(q,2H), 2,95-2,89(m,1H), 2,41(s,3H), 1,19-1,15(m,3H), 1,07-0,93(m,4H) |

(fortgesetzt)

| Bsp.-Nr. | logP[a] | logP[b] | $^1$H-NMR(D6-DMSO, 400MHz) δ ppm |
|---|---|---|---|
| 75 | 2,97 | 2,89 | 7,66(d,1H), 7,38(d,1H), 3,92(q,2H), 3,71(t,2H), 3,29(s,3H), 2,96(t,2H), 2,91-2,86(m,1H), 2,43(s,3H), 0,99-0,96(m,4H) |
| 76 | 2,03 | 1,97 | 7,92(d,1H), 7,49(d,1H), 4,25-4,16(m,1H), 4,11-4,02(m,1H), 3,72(t,2H), 3,29(s,3H), 2,97(t,2H), 2,93-2,88(m,1H), 2,40(s,3H), 1,02-0,98(m,4H) |
| 77 | 3,24 | 3,18 | 7,67(d,1H), 7,37(d,1H), 3,92(q,2H), 2,91-2,86(m,1H), 2,72(q,2H), 2,43(s,3H), 1,23(t,3H), 0,99-0,95(m,4H) |
| 78 | 2,20 | 2,15 | 7,92(d,1H), 7,48(d,1H), 4,25-4,16(m,1H), 4,10-4,00(m,1H), 2,92-2,87(m,lH), 2,72(q,2H), 2,40(s,3H), 1,24(t,3H), 1,01-0,96(m,4H) |
| 79 | 3,64 | 3,56 | 7,66(d,1H), 7,37(d,1H), 3,92(q,2H), 2,90-2,85(m,1H), 2,68-2,64(m,2H), 2,43(s,3H), 1,76-1,67(m,2H), 1,02-0,96(m,7H) |
| 80 | 2,56 | 2,49 | 7,92(d,1H), 7,48(d,1H), 4,25-4,15(m,1H), 4,10-4,00(m,1H), 2,92-2,87(m,1H), 2,68(t,2H), 2,40(s,3H), 1,78-1,69(m,2H), 1,02-0,97(m,7H) |
| 81 | 3,55 | 3,49 | 7,70(d,1H), 7,38(d,1H), 6,73-6,64(m,1H), 6,42-6,64(m,1H), 3,93(q,2H), 2,95-2,90(m,1H), 2,43(s,3H), 1,94-1,92(m,3H), 1,06-1,01(m,2H), 0,93-0,89(m,2H) |
| 82 | 2,48 | 2,42 | 7,96(d,1H), 7,49(d,1H), 6,75-6,66(m,1H), 6,43-6,37(m,1H), 4,25-4,16(m,1H), 4,10-3,98(m,1H), 2,97-2,91(m,1H), 2,41(s,3H), 1,95-1,93(m,3H), 1,06-0,99(m,2H), 0,95-0,91(m,2H) |
| 83 | 3,99 | 3,93 | 7,66(d,1H), 7,37(d,1H), 3,92(q,2H), 2,88-2,84(m,1H), 2,56(d,2H), 2,43(s,3H), 2,18-2,11(m,1H), 1,01-0,97(m,10H) |
| 84 | 2,86 | 2,80 | 7,93(d,1H), 7,48(d,1H), 4,25-4,18(m,1H), 4,08-4,01(m,1H), 2,90-2,86(m,1H), 2,55(d,2H), 2,40(s,3H), 2,20-2,13(m,1H), 1,02-0,98(m,10H) |
| 85 | 4,01 | 3,94 | 7,67(d,1H), 7,37(d,1H), 3,92(q,2H), 3,02-2,97(m,1H), 2,93-2,88(m,1H), 2,43(s,3H), 1,83-1,76(m,1H), 1,61-1,54(m,1H), 1,24(d,3H), 1,03-0,91(m,7H) |
| 86 | 2,86 | 2,79 | 7,92(d,1H), 7,48(d,1H), 4,24-4,18(m,1H), 4,10-4,02(m,1H), 3,04-2,99(m,1H), 2,95-2,89(m,1H), 2,41(s,3H), 1,85-1,77(m,1H), 1,63-1,55(m,1H), 1,27-1,24(m,3H), 1,03-0,91(m,7H) |
| 87 | 3,85 | 3,77 | 7,69(d,1H), 7,38(d,1H), 3,93(q,2H), 3,74-3,65(m,1H), 2,83-2,77(m,1H), 2,43(s,3H), 2,35-2,27(m,4H), 2,12-2,00(m,1H), 1,91-1,82(m,1H), 0,96-0,90(m,4H) |
| 88 | 2,72 | 2,66 | 7,94(d,1H), 7,48(d,1H), 4,25-4,16(m,1H), 4,12-4,00(m,1H), 3,74-3,67(m,1H), 2,84-2,79(m,1H), 2,41(s,3H), 2,37-2,30(m,4H), 2,11-2,03(m,1H), 1,91-1,86(m,1H), 0,94(d,4H) |
| 89 | 3,80 | 3,73 | 7,83(d,1H), 7,48(d,1H), 4,01-3,94(m,4H), 3,61(t,2H), 3,27(s,3H), 2,45(s,3H) |
| 90 | 3,74 | 3,67 | 8,02(d,1H), 7,68-7,65(m,1H), 7,32(d,1H), 4,02(s,3H), 3,87(q,2H), 2,79-2,75(m,1H), 2,37(s,3H), 0,93-0,87(m,4H) |
| 91 | 4,59 | 4,54 | 7,99(d,1H), 7,66-7,63(m,1H), 7,31(d,1H), 5,08-5,01(m,1H), 3,87(q,2H), 2,76-2,74(m,1H), 2,36(s,3H), 1,41(d,6H), 0,92-0,90(m,4H) |
| 92 | 4,29 | 4,20 | 8,00(d,1H), 7,68-7,66(m,1H), 7,34(d,1H), 5,11(q,2H), 3,89(q,2H), 2,86-2,81(m,1H), 2,37(s,3H), 0,95-0,93(m,4H) |
| 93 | 2,78 | 2,73 | 7,70(d,1H), 7,38(d,1H), 3,97(s,3H), 3,92(q,2H), 3,10(s,3H), 2,43(s,3H) |
| 94 | 1,77 | 1,77 | 7,96(d,1H), 7,48(d,1H), 4,25-4,19(m,1H), 4,07-4,00(m,1H), 3,99(s,3H), 3,11(s,3H), 2,40(s,3H) |
| 95 | 3,74 | 3,64 | 8,34(d,1H), 7,94-7,92(m,1H), 7,87-7,84(m,1H), 4,11-4,04(m,5H), 3,11(s,3H) |
| 96 | 2,89 | 2,83 | 8,70(d,1H), 8,32-8,29(m,1H), 8,03(d,1H), 4,15(q,2H), 4,09(s,3H), 3,13(s,3H) |
| 97 | 2,26 | 2,24 | 8,11(s,1H), 7,94(s,1H), 4,37-4,28(m,1H), 4,20-4,13(m,1H), 3,99(s,3H), 3,11(s,3H) |

(fortgesetzt)

| Bsp.-Nr. | logP[a] | logP[b] | $^1$H-NMR(D6-DMSO, 400MHz) δ ppm |
|---|---|---|---|
| 98 | 2,12 | 2,10 | 8,01(d,1H), 7,96(d,1H), 4,34-4,28(m,1H), 4,18-4,12(m,1H), 4,00(s,3H), 3,11(s,3H) |
| 99 | 2,54 | 2,52 | 8,35(d,1H), 8,17-8,14(m,1H), 7,72(d,1H), 4,26-4,09(m,2H), 4,07(s,3H), 3,11(s,3H) |
| 100 | 2,82 | 2,79 | 7,94(d,1H), 7,46(d,1H), 4,99-4,93(m,1H), 4,24-4,15(m,1H), 4,09-4,00(m,1H), 2,81-2,75(m,1H), 2,39(s,3H), 1,40-1,37(m,6H), 0,92-0,91 (m,4H) |
| 101 | 3,93 | 3,85 | 7,68(d,1H), 7,35(d,1H), 4,96-4,90(m,1H), 3,92(q,2H), 2,78-2,75(m,1H), 2,49(s,3H), 1,38(d,6H), 0,92-0,90(m,4H) |
| 102 | 2,83 | 2,77 | 7,95(d,1H), 7,49(d,1H), 5,08-5,01(m,2H), 4,26-4,17(m,1H), 4,06-3,94(m,1H), 2,89-2,84(m,1H), 2,40(s,3H), 0,96-0,93(m,4H) |
| 103 | 3,80 | 3,72 | 7,69(d,1H), 7,38(d,1H), 5,02(q,2H), 3,91(q,2H), 2,88-2,83(m,1H), 2,43(s,3H), 0,96-0,90(m,4H) |
| 104 | 2,13 | 2,08 | 7,95(d,1H), 7,47(d,1H), 4,25-4,18(m,1H), 4,06-4,00(m,1H), 3,97(s,3H), 2,82-2,77(m,1H), 2,40(s,3H), 0,94-0,91(m,4H) |
| 105 | 3,16 | 3,11 | 7,69(d,1H), 7,36(d,1H), 3,95(s,3H), 3,91(q,2H), 2,80-2,78(m,1H), 2,42(s,3H), 0,92-0,89(m,4H) |
| 106 | 2,65 | 2,61 | 8,09(s,1H), 7,94(s,1H), 4,40-4,28(m,1H), 4,21-4,09(m,1H), 3,97(s,3H), 2,85-2,78(m,1H), 0,94-0,91(m,4H) |
| 107 | 3,46 | 3,42 | 7,91(s,1H), 7,83(s,1H), 4,19(q,2H), 3,95(s,3H), 2,82-2,78(m,1H), 0,94-0,89(m,4H) |
| 108 | 3,91 | 3,84 | 8,13(d,1H), 7,77-7,74(m,1H), 7,58(d,1H), 4,07-3,99(m,5H), 2,79-2,75(m,1H), 0,94-0,90(m,4H) |
| 109 | 2,34 | 2,30 | 8,11(d,1H), 8,05-8,02(m,1H), 7,28(d,1H), 4,13-4,04(m,1H), 4,03(s,3H), 3,95-3,90(m,1H), 3,89(s,3H), 2,80-2,76(m,1H), 0,94-0,91 (m,4H) |
| 110 | 3,00 | 2,95 | 8,35(d,1H), 8,07-8,04(m,1H), 7,84(d,1H), 4,21-4,03(m,2H), 4,05(s,3H), 2,81-2,77(m,1H), 0,95-0,92(m,4H) |
| 111 | 4,01 | 3,93 | 8,12(d,1H), 7,72(d,1H), 7,67-7,64(m,1H), 4,08-4,00(m,5H), 2,79-2,75(m,1H), 0,93-0,90(m,4H) |
| 112 | 2,83 | 2,79 | 7,94(d,1H), 7,46(d,1H), 4,25(t,2H), 4,24-4,18(m,1H), 4,06-4,02(m,1H), 2,82-2,80(m,1H), 2,40(s,3H), 1,80-1,75(m,2H), 0,99(t,3H), 0,94-0,92(m,4H) |
| 113 | 4,00 | 3,92 | 7,68(d,1H), 7,35(d,1H), 4,23(t,2H), 3,91(q,2H), 2,82-2,77(m,1H), 2,42(s,3H), 1,82-1,72(m,2H), 0,98(t,3H), 0,93-0,91(m,4H) |
| 114 | 3,32 | 3,27 | 7,84(d,1H), 7,78(d,1H), 4,08(q,2H), 3,97(s,3H), 2,81-2,78(m,1H), 0,93-0,91(m,4H) |
| 115 | 2,45 | 2,43 | 8,00(d,1H), 7,95(d,1H), 4,34-4,24(m,1H), 4,20-4,10(m,1H), 3,99(s,3H), 2,83-2,77(m,1H), 0,94-0,91(m,4H) |
| 116 | 3,20 | 3,30 | 7,68(s,1H), 7,56(s,1H), 4,04(q,2H), 3,94(s,3H), 2,81-2,78(m,1H), 2,39(s,3H), 0,94-0,89(m,4H) |
| 117 | 2,28 | 2,30 | 7,90(s,1H), 7,69(s,1H), 4,28-4,22(m,1H), 4,08-4,01(m,1H), 3,96(s,3H), 2,82-2,80(m,1H), 2,40(s,3H), 0,93-0,91(m,4H) |
| 118 | 2,47 | 2,43 | 8,50(d,1H), 8,24-8,21(m,1H), 8,12(d,1H), 4,40-4,20(m,2H), 4,07(s,3H), 2,85-2,78(m,1H), 0,96-0,93(m,4H) |
| 119 | 2,21 | 2,17 | 7,76(s,1H), 7,33(s,1H), 4,19-4,12(m,1H), 3,98-3,92(m,1H), 3,96(s,3H), 2,82-2,78(m,1H), 2,36(s,3H), 2,27(s,3H), 0,94-0,90(m,4H) |
| 120 | 3,58 | 3,44 | 7,68(d,1H), 7,35(d,1H), 4,32(q,2H), 3,91(q,2H), 2,81-2,76(m,1H), 2,42(s,3H), 1,37(t,3H), 0,92-0,87(m,4H) |
| 121 | 2,47 | 2,43 | 7,94(d,1H), 7,46(d,1H), 4,35(q,2H), 4,24-4,18(m,1H), 4,06-3,99(m,1H), 2,81-2,78(m,1H), 2,39(s,3H), 1,38(t,3H), 0,94-0,91 (m,4H) |

(fortgesetzt)

| Bsp.-Nr. | logP[a] | logP[b] | $^1$H-NMR(D6-DMSO, 400MHz) δ ppm |
|---|---|---|---|
| 122 | 4,19 | 4,04 | 8,00(d,1H), 7,67-7,64(m,1H), 7,31(d,1H), 4,40(q,2H), 3,87(q,2H), 2,80-2,74(m,1H), 2,37(s,3H), 1,38(t,3H), 0,94-0,88(m,4H) |
| 123 | 4,66 | 4,57 | 8,00(d,1H), 7,67-7,64(m,1H), 7,31(d,1H), 4,31(t,2H), 3,87(q,2H), 2,81-2,75(m,1H), 2,37(s,3H), 1,84-1,76(m,2H), 1,00(t,3H), 0,93-0,91 (m,4H) |
| 124 | 3,24 | 3,20 | 7,94(d,1H), 7,46(d,1H), 4,29(t,2H), 4,24-4,15(m,1H), 4,08-3,99(m,1H), 2,83-2,77(m,1H), 2,39(s,3H), 1,78-1,71(m,2H), 1,49-1,40(m,2H), 0,96-0,92(m,7H) |
| 125 | 4,41 | 4,35 | 7,68(d,1H), 7,35(d,1H), 4,27(t,2H), 3,91(q,2H), 2,82-2,76(m,1H), 2,42(s,3H), 1,77-1,70(m,2H), 1,48-1,39(m,2H), 0,96-0,89(m,7H) |
| 126 | 5,11 | 5,02 | 8,00(d,1H), 7,67-7,64(m,1H), 7,31(d,1H), 4,35(t,2H), 3,87(q,2H), 2,80-2,74(m,1H), 2,36(s,3H), 1,80-1,73(m,2H), 1,50-1,41(m,2H), 0,97-0,90(m,7H) |
| 127 | 3,61 | 3,57 | 7,69(d,1H), 7,37(d,1H), 4,25(t,2H), 3,92(q,2H), 3,10(s,3H), 2,43(s,3H), 1,80-1,73(m,2H), 0,97(q,3H) |
| 128 | 4,11 | 3,95 | 7,68(d,1H),7,37(d,1H), 4,29(t,2H), 3,92(q,2H), 3,09(s,3H), 2,43(s,3H), 1,77-1,70(m,2H), 1,46-1,40(m,2H), 0,93(t,3H) |
| 129 | 3,19 | 3,14 | 7,69(d,1H), 7,37(d,1H), 4,35(q,2H), 3,92(q,2H), 3,09(s,3H), 2,42(s,3H), 1,38(t,3H) |
| 130 | 3,77 | 3,74 | 8,02(d,1H), 7,69-7,66(m,1H), 7,32(d,1H), 4,42(q,2H), 3,88(q,2H), 3,08(s,3H), 2,37(s,3H), 1,40(t,3H) |
| 131 | 2,13 | 2,09 | 7,95(d,1H), 7,47(d,1H), 4,27(t,2H), 4,25-4,20(m,1H), 4,04-4,01(m,1H), 3,11(s,3H), 2,40(s,3H), 1,78(q,2H), 0,98(t,3H) |
| 132 | 2,52 | 2,47 | 7,95(d,1H), 7,47(d,1H), 4,27(t,2H), 4,25-4,20(m,1H), 4,04-4,01(m,1H), 3,11(s,3H), 2,40(s,3H), 1,78(q,2H), 0,98(t,3H) |
| 133 | 2,93 | 2,86 | 8,30(d,1H), 8,02-8,00(m,1H), 7,41(d,1H), 4,35(t,2H), 4,17-4,11(m,1H), 3,98-3,92(m,1H), 3,11(s,3H), 2,40(s,3H), 1,84-1,76(m,2H), 1,00(t,3H) |
| 134 | 3,99 | 3,90 | 7,67(d,1H), 7,35(d,1H), 4,12(d,2H), 3,91(q,2H), 2,84-2,78(m,1H), 2,42(s,3H), 1,33-1,27(m,1H), 0,94-0,93(m,4H), 0,62-0,60(m,2H), 0,42-0,38(m,2H) |
| 135 | 4,60 | 4,56 | 7,99(d,1H), 7,66-7,64(m,1H), 7,31(d,1H), 4,20(d,2H), 3,87(q,2H), 2,82-2,77(m,1H), 2,36(s,3H), 1,36-1,30(m,1H), 0,95-0,92(m,4H), 0,64-0,61(m,2H), 0,42-0,41(m,2H) |
| 136 | 4,37 | 4,33 | 7,68(d,1H), 7,36(d,1H), 4,84-4,80(m,2H), 4,38(t,2H), 3,91(q,2H), 2,80-2,74(m,1H), 2,49-2,47(m,2H), 2,42(s,3H), 1,77(s,3H), 0,90-0,85(m,4H) |
| 137 | 3,25 | 3,17 | 7,94(d,1H), 7,46(d,1H), 4,85-4,81(m,2H), 4,40(t,2H), 4,24-4,15(m,1H), 4,08-3,96(m,1H), 2,81-2,75(m,1H), 2,50-2,47(m,2H), 2,40(s,3H), 1,78(s,3H), 0,93-0,89(m,4H) |
| 138 | 5,01 | 4,96 | 8,00(d,1H), 7,68-7,65(m,1H), 7,32(d,1H), 4,85-4,82(m,2H), 4,46(t,2H), 3,87(q,2H), 2,78-2,73(m,1H), 2,53-2,52(m,2H), 2,33(s,3H), 1,79(s,3H), 0,93-0,88(m,4H) |
| 139 | 3,67 | 3,60 | 8,30(d,1H), 7,99-7,96(m,1H), 7,41(d,1H), 4,85-4,82(m,2H), 4,48(t,2H), 4,17-4,10(m,1H), 3,97-3,90(m,1H), 2,79-2,76(m,1H), 2,53-2,52(m,2H), 2,34(s,3H), 1,79(s,3H), 0,93-0,89(m,4H) |
| 140 | 4,36 | 4,30 | 7,68(d,1H), 7,35(d,1H), 4,78-4,74(m,1H), 3,92(q,2H), 2,80-2,75(m,1H), 2,42(s,3H), 1,76-1,67(m,2H), 1,35(d,3H), 0,96-0,89(m,7H) |
| 141 | 3,18 | 3,12 | 7,94(d,1H), 7,46(d,1H), 4,81-4,77(m,1H), 4,24-4,15(m,1H), 4,09-4,00(m,1H), 2,82-2,76(m,1H), 2,39(s,3H), 1,76-1,70(m,2H), 1,36(t,3H), 0,98-0,91(m,7H) |
| 142 | 5,01 | 4,96 | 7,99(d,1H), 7,66-7,63(m,1H), 7,31(d,1H), 4,90-4,85(m,1H), 3,88(q,2H), 2,79-2,73(m,1H), 2,36(s,3H), 1,78-1,70(m,2H), 1,39(d,3H), 0,96(t,3H), 0,92-0,91(m,4H) |

(fortgesetzt)

| Bsp.-Nr. | logP[a] | logP[b] | $^1$H-NMR(D6-DMSO, 400MHz) δ ppm |
|---|---|---|---|
| 143 | 3,66 | 3,58 | 7,69(d,1H), 7,36(d,1H), 4,14(d,2H), 3,91(q,2H), 3,11(s,3H), 2,43(s,3H), 1,35-1,24(m,1H), 0,63-0,56(m,2H), 0,43-0,35(m,2H) |
| 144 | 4,14 | 3,85 | 8,33(d,1H), 7,93-1,90(m,1H), 7,84(d,1H), 4,10-4,03(m,5H), 2,81-2,78(m,lH), 0,95-0,92(m,4H) |
| 145 | 4,20 | 4,19 | 8,01(d,1H), 7,68-7,65(m,1H), 7,32(d,1H), 4,22(d,2H), 3,88(q,2H), 3,10(s,3H), 2,37(s,3H), 1,36-1,28(m,1H), 0,65-0,59(m,2H), 0,47-0,40(m,2H) |
| 146 | 2,90 | 2,85 | 7,95(d,1H), 7,45(d,1H), 4,31(t,2H), 4,25-4,15(m,1H), 4,07-4,00(m,1H), 3,10(s,3H), 2,40(s,3H), 1,78-1,71(m,2H), 1,48-1,41(m,2H), 0,94(t,3H) |
| 147 | 3,27 | 3,19 | 8,71(d,1H), 8,28-8,26(m,1H), 8,02(d,1H), 4,17-4,10(m,2H), 4,08(s,3H), 2,84-2,80(m,1H), 0,97-0,93(m,4H) |
| 148 | 4,03 | 3,92 | 7,69(d,1H), 7,37(d,1H), 4,07(d,2H), 3,92(q,2H), 3,11(s,3H), 2,43(s,3H), 2,12-2,05(m,1H), 0,98(d,6H) |
| 149 | 2,83 | 2,76 | 7,69(d,1H), 7,37(d,1H), 4,42-4,40(m,2H), 3,92(q,2H), 3,70-3,68(m,2H), 3,33(s,3H), 3,10(s,3H), 2,43(s,3H) |
| 150 | 4,35 | 4,29 | 7,69(d,1H), 7,37(d,1H), 3,97(s,2H), 3,92(q,2H), 3,13(s,3H), 2,43(s,3H), 1,00(s,9H) |
| 151 | 3,61 | 3,53 | 7,69(d,1H), 7,36(d,1H), 4,99-4,92(m,1H), 3,92(q,2H), 3,07(s,3H), 2,43(s,3H), 1,39(d,6H) |
| 152 | 2,90 | 2,83 | 7,95(d,1H), 7,47(d,1H), 4,29-4,12(m,1H), 4,09(d,2H), 4,07-3,99(m,1H), 3,12(s,3H), 2,40(s,3H), 2,12-2,06(m,1H), 0,99(d,6H) |
| 153 | 4,50 | 4,44 | 7,68(d,1H), 7,36(d,1H), 4,76-4,72(m,1H), 3,92(q,2H), 3,09(s,3H), 2,41(s,3H), 2,00-1,97(m,2H), 1,74-1,70(m,2H), 1,64-1,56(m,2H), 1,49-1,41(m,1H), 1,25-1,40(m,3H) |
| 154 | 4,55 | 4,45 | 7,98(d,1H), 7,69-7,66(m,1H), 7,32(d,1H), 3,90(q,2H), 3,05(s,3H), 2,36(s,3H), 1,59(s,9H) |
| 155 | 5,22 | 5,17 | 8,01(d,1H), 7,66-7,63(m,1H), 7,31(d,1H), 4,87-4,83(m,1H), 3,89(q,2H), 3,08(s,3H), 2,36(s,3H), 2,09-2,01(m,2H), 1,76-1,71(m,2H), 1,68-1,58(m,2H), 1,53-1,45(m,1H), 1,43-1,34(m,3H) |
| 156 | 3,27 | 3,27 | 8,01(d,1H), 7,68-7,65(m,1H), 7,32(d,1H), 5,43-5,42(m,1H), 3,98(d,1H), 3,93-3,85(m,4H), 3,80-3,74(m,1H), 3,07(s,3H), 2,37(s,3H), 2,32-2,25(m,1H), 2,20-2,09(m,1H) |
| 157 | 3,98 | 3,88 | 7,68(d,1H), 7,36(d,1H), 3,92(q,2H), 3,05(s,3H), 2,42(s,3H), 1,55(s,9H) |
| 158 | 3,70 | 3,66 | 7,66(d,1H), 7,49-7,42(m,4H), 7,36(d,1H), 7,32-7,28(m,1H), 3,90(q,2H), 3,25(s,3H), 2,41 (s,3H) |
| 159 | 2,70 | 2,65 | 7,89(d,1H), 7,51-7,43(m,5H), 7,33-7,28(m,1H), 4,23-4,14(m,1H), 4,08-3,99(m,1H), 3,26(s,3H), 2,39(s,3H) |
| 160 | 2,52 | 2,38 | 7,96(d,1H), 7,50(d,1H), 5,11-5,04(m,2H), 4,26-4,20(m,1H), 4,05-3,98(m,1H), 3,15(s,3H), 2,41(s,3H) |
| 161 | 1,89 | 1,84 | 7,95(d,1H), 7,48(d,1H), 5,37-5,35(m,1H), 4,25-4,03(m,1H), 4,02-3,95(m,2H), 3,91-3,81(m,2H), 3,78-3,73(m,1H), 3,09(s,3H), 2,40(s,3H), 2,27-2,15(m,2H) |
| 162 | 3,67 | 3,57 | 8,29(d,1H), 8,03-8,01(m,1H), 7,41(d,1H), 4,14-4,11(m,1H), 4,07(s,2H), 3,99-3,94(m,1H), 3,14(s,3H), 2,34(s,3H), 1,03(s,9H) |
| 163 | 2,87 | 2,80 | 8,30(d,1H), 8,01-7,98(m,1H), 7,41(d,1H), 5,13-5,06(m,1H), 4,17-4,10(m,1H), 3,99-3,92(m,1H), 3,08(s,3H), 2,34(s,3H), 1,43-1,40(m,6H) |

(fortgesetzt)

| Bsp.-Nr. | logP[a] | logP[b] | $^1$H-NMR(D6-DMSO, 400MHz) δ ppm |
|---|---|---|---|
| 164 | 3,29 | 3,22 | 7,93(d,1H), 7,47(d,1H), 4,79-4,75(m,1H), 4,24-4,16(m,1H), 4,08-4,02(m,1H), 3,10(s,3H), 2,40(s,3H), 2,01-1,98(m,2H), 1,73-1,59(m,4H), 1,51-1,48(m,1H), 1,42-1,26(m,3H) |
| 165 | 3,21 | 3,15 | 7,94(d,1H), 7,47(d,1H), 4,20-4,18(m,1H), 4,07-4,04(m,1H), 3,99(s,2H), 3,14(s,3H), 2,40(s,3H), 1,01(s,9H) |
| 166 | 2,48 | 2,44 | 7,95(d,1H), 7,47(d,1H), 5,01-4,95(m,1H), 4,24-4,18(m,1H), 4,08-4,01(m,1H), 3,08(s,3H), 2,40(s,3H), 1,40-1,38(m,6H) |
| 167 | 3,31 | 3,26 | 8,29(d,1H), 8,02-8,00(m,1H), 7,41(d,1H), 4,16(d,2H), 4,15-4,11(m,1H), 3,98-3,92(m,1H), 3,12(s,3H), 2,34(s,3H), 2,14-2,08(m,1H), 1,00(d,6H) |
| 168 | 3,60 | 3,54 | 8,13(d,1H), 7,73(d,1H), 7,68-7,66(m,1H), 4,05(s,3H), 4,04(q,2H), 3,09(s,3H) |
| 169 | 1,81 | 1,77 | 7,95(t,1H), 7,81(t,1H), 4,39-4,28(m,2H), 3,99(s,3H), 3,11(s,3H) |
| 170 | 2,15 | 2,11 | 7,94(t,1H), 7,80(t,1H), 4,41-4,27(m,2H), 3,98(s,3H), 2,82-2,79(m,1H), 0,94-0,91(m,4H) |
| 171 | 2,97 | 2,92 | 7,83(t,1H), 7,62(t,1H), 3,96(q,2H), 3,96(s,3H), 2,81-2,78(m,1H), 0,92-0,91 (m,4H) |
| 172 | 2,57 | 2,53 | 8,05(d,1H), 7,99(d,1H), 4,31-4,20(m,1H), 4,15-4,03(m,1H), 3,99(s,3H), 2,87-2,79(m,1H), 0,94-0,91(m,4H) |
| 173 | 3,43 | 3,36 | 7,89(d,1H), 7,82(d,1H), 4,08(q,2H), 3,97(s,3H), 2,81-2,78(m,1H), 0,93-0,91(m,4H) |
| 174 | 2,21 | 2,15 | 8,07(d,1H), 8,00(d,1H), 4,29-4,23(m,1H), 4,14-4,07(m,1H), 4,00(s,3H), 3,11(s,3H) |
| 175 | 3,04 | 2,98 | 7,90(d,1H), 7,83(d,1H), 4,09(q,2H), 3,98(s,3H), 3,10(s,3H) |
| 176 | 3,87 | 3,81 | 8,04(d,1H), 7,70-7,67(m,1H), 7,34(d,1H), 5,92-5,84(m,1H), 5,20-5,10(m,2H), 4,18(d,2H), 4,04(s,3H), 3,89(q,2H), 2,38(s,3H) |
| 177 | 2,92 | 2,85 | 7,98(d,1H), 7,55-7,48(m,3H), 7,46-7,40(m,3H), 5,37(s,2H), 4,26-4,20(m,1H), 4,07-4,01(m,1H), 3,12(s,3H), 2,41(s,3H) |
| 178 | 3,23 | 3,18 | 8,34(d,1H), 8,03-8,01(m,1H), 7,57-7,55(m,2H), 7,46-7,40(m,4H), 5,45(s,2H), 4,19-4,12(m,1H), 3,98-3,92(m,1H), 3,12(s,3H), 2,35(s,3H) |
| 179 | 2,61 | 2,57 | 8,33(d,1H), 8,03-8,00(m,1H), 7,41(d,1H), 5,93-5,85(m,1H), 5,21-5,11(m,2H), 4,20(d,2H), 4,18-4,08(m,1H), 4,06(s,3H), 4,00-3,94(m,1H), 2,35(s,3H) |
| 180 | 3,16 | 3,10 | 7,72(d,1H), 7,37(d,1H), 3,97(s,3H), 3,93(q,2H), 3,59(q,2H), 2,43(s,3H), 1,20(t,3H) |
| 181 | 2,11 | 2,07 | 7,97(d,1H), 7,48(d,1H), 4,25-4,18(m,1H), 4,09-4,02(m,1H), 4,00(s,3H), 3,60(q,2H), 2,41(s,3H), 1,21(t,3H) |
| 182 | 3,30 | 3,24 | 7,73(d,1H), 7,38(d,1H), 5,93-5,85(m,1H), 5,22-5,19(m,1H), 5,15-5,11(m,1H), 4,19-4,17(m,2H), 3,97(s,3H), 3,93(q,2H), 2,43(s,3H) |
| 183 | 2,24 | 2,21 | 7,99(d,1H), 7,49(d,1H), 5,95-5,83(m,1H), 5,22-5,19(m,1H), 5,16-5,11(m,1H), 4,25-4,15(m,3H), 4,10-4,04(m,1H), 3,99(s,3H), 2,41(s,3H) |
| 184 | 2,82 | 2,76 | 7,96(d,1H), 7,47(d,1H), 4,20-4,18(m,1H), 4,10-4,05(m,1H), 3,06(s,3H), 2,40(s,3H), 1,56(s,9H) |
| 185 | 4,14 | 4,10 | 7,93(s,1H), 7,84(s,1H), 5,03(q,2H), 4,17(q,2H), 2,90-2,85(m,1H), 0,98-0,89(m,4H) |
| 186 | 3,96 | 3,89 | 7,85(d,1H), 7,81(d,1H), 5,04(q,2H), 4,07(q,2H), 2,90-2,83(m,1H), 0,99-0,89(m,4H) |
| 187 | 3,98 | 3,93 | 7,70(s,1H), 7,57(d,1H), 5,02(q,2H), 4,02(q,2H), 2,90-2,84(m,1H), 2,40(s,3H), 1,00-0,90(m,4H) |
| 188 | 3,49 | 3,46 | 7,58(d,1H), 7,20(d,1H), 5,01(q,2H), 3,92(s,3H), 3,86(q,2H), 2,87-2,82(m,1H), 0,97-0,90(m,4H) |

(fortgesetzt)

| Bsp.-Nr. | logP[a] | logP[b] | $^1$H-NMR(D6-DMSO, 400MHz) δ ppm |
|---|---|---|---|
| 189 | 4,55 | 4,48 | 8,30(d,1H), 7,96-7,93(m,1H), 7,86(d,1H), 5,17(q,2H), 4,10(q,2H), 2,88-2,83(m,1H), 0,98-0,94(m,4H) |
| 190 | 2,84 | 2,81 | 7,68(d,1H), 7,35(d,1H), 3,92(q,2H), 3,20(s,3H), 2,78(s,6H), 2,42(s,3H) |
| 191 | 1,86 | 1,84 | 7,94(d,1H), 7,46(d,1H), 4,21-4,17(m,1H), 4,09-4,02(m,1H), 3,21(s,3H), 2,80(s,6H), 2,40(s,3H) |
| 192 | 3,76 | 3,68 | 7,98(d,1H), 7,67-7,64(m,1H), 7,52-7,46(m,4H), 7,38-7,31(m,2H), 3,91(q,2H), 3,28(s,3H), 3,21(s,3H), 2,38(s,3H) |
| 193 | 3,20 | 3,15 | 7,67(d,1H), 7,34(d,1H), 3,91(q,2H), 2,95-2,93(m,1H), 2,85(s,6H), 2,42(s,3H), 1,02-0,99(m,2H), 0,93-0,90(m,2H) |
| 194 | 2,17 | 2,12 | 7,93(d,1H), 7,44(d,1H), 4,25-4,14(m,1H), 4,10-4,00(m,1H), 2,99-2,92(m,1H), 2,87(s,6H), 2,39(s,3H), 1,05-1,00(m,2H), 0,98-0,90(m,2H) |
| 195 | 3,75 | 3,73 | 7,67(d,1H), 7,37(d,1H), 3,94(q,2H), 2,95(s,6H), 2,43(s,3H), 2,04-1,97(m,1H), 0,99-0,93(m,2H), 0,91-0,85(m,2H) |
| 196 | 2,67 | 2,61 | 7,63(d,1H), 7,37(d,1H), 5,47(breit,2H), 3,93(q,2H), 2,43(s,3H), 2,09-2,01(m,1H), 1,01-0,95(m,2H), 0,94-0,89(m,2H) |
| 197 | 2,60 | 2,59 | 7,91(d,1H), 7,49(d,1H), 4,27-4,00(m,2H), 2,96(s,3H), 2,50(s,6H), 2,40(s,3H), 2,06-1,98(m,1H), 1,00-0,95(m,2H), 0,94-0,87(m,2H) |
| 198 | 2,28 | 2,22 | 7,70(d,1H), 7,41(s,1H), 5,49(s,2H), 4,38(s,2H), 3,96(q,2H), 3,32(s,3H), 2,44(s,3H) |
| 199 | 3,15 | 3,07 | 7,67(d,1H), 7,37(d,1H), 6,19(q,1H), 3,95(q,2H), 2,71(d,3H), 2,42(s,3H), 2,06-1,99(m,1H), 1,02-0,95(m,2H), 0,94-0,88(m,2H) |
| 200 | 2,18 | 2,13 | 7,66(d,1H), 7,39(d,1H), 5,41(s,2H), 3,94(q,2H), 2,43(s,3H), 2,21(s,3H) |
| 201 | 3,17 | 3,14 | 7,69(d,1H), 7,39(d,1H), 3,94(q,2H), 2,92(s,3H), 2,43(s,3H), 2,20(s,6H) |
| 202 | 4,06 | 3,98 | 7,75(d,1H), 7,40(d,1H), 3,96(q,2H), 2,43(s,3H), 2,37(d,2H), 2,17(s,3H), 2,12-2,02(m,1H), 1,98(s,3H), 0,96(d,6H) |
| 203 | 2,32 | 2,27 | 8,29(s,1H), 7,71(d,1H), 7,41(d,1H), 6,24(q,1H), 3,97(q,2H), 2,71(d,3H), 2,42(s,3H) |
| 204 | 1,96 | 1,95 | 7,92(d,1H), 7,50(d,1H), 5,46(s,2H), 4,27-4,05(m,2H), 3,11-3,02(m,1H), 2,41(s,3H), 1,28-1,22(m,6H) |
| 205 | 3,15 | 3,08 | 7,79(d,1H), 7,47(d,1H), 5,85(breit,2H), 3,98(q,2H), 2,45(s,3H) |
| 206 | 1,75 | 1,72 | 7,89(d,1H), 7,48(d,1H), 5,49(breit,2H), 4,25-4,00(m,2H), 2,40(s,3H), 2,12-2,03(m,1H), 1,04-0,98(m,2H), 0,97-0,90(m,2H) |
| 207 | 1,47 | 1,45 | 7,96(d,1H), 7,53(d,1H), 5,50(breit,2H), 4,40(s,2H), 4,30-4,16(m,1H), 4,14-4,02(m,1H), 3,35(s,3H), 2,42(s,3H) |
| 208 | 2,09 | 2,05 | 7,91(d,1H), 7,49(d,1H), 6,22(q,1H), 4,27-4,01(m,2H), 2,73(d,3H), 2,41(s,3H), 2,09-2,00(m,1H), 1,03-0,97(m,2H), 0,96-0,89(m,2H) |
| 209 | 1,36 | 1,34 | 7,93(d,1H), 7,50(d,1H), 5,43(s,2H), 4,29-4,16(m,1H), 4,12-4,00(m,1H), 2,41(s,3H), 2,23(s,3H) |
| 210 | 2,09 | 2,09 | 7,95(d,1H), 7,50(d,1H), 4,29-4,17(m,1H), 4,11-4,00(m,1H), 2,93(s,3H), 2,41(s,3H), 2,22(s,6H) |
| 211 | 2,02 | 2,01 | 8,23(s,1H), 7,68(d,1H), 7,41(d,1H), 5,58(breit,2H), 3,96(q,2H), 2,43(s,3H) |
| 212 | 3,38 | 3,37 | 8,00(d,1H), 7,70-7,67(m,1H), 7,42(d,1H), 5,79(breit,2H), 3,95(q,2H), 2,41(s,3H) |
| 213 | 3,16 | 3,09 | 8,08(d,1H), 7,58(d,1H), 4,30-4,23(m,1H), 4,04-3,98(m,1H), 2,97(s,6H), 2,44(s,3H) |
| 214 | 4,24 | 4,15 | 7,82(d,1H), 7,48(d,1H), 3,95(q,2H), 2,97(s,6H), 2,46(s,3H) |

(fortgesetzt)

| Bsp.-Nr. | logP[a] | logP[b] | $^1$H-NMR(D6-DMSO, 400MHz) δ ppm |
|---|---|---|---|
| 215 | 2,19 | 2,17 | 8,03(d,1H), 7,59(d,1H), 5,83(breit,2H), 4,31-4,20(m,1H), 4,15-4,03(m,1H), 2,44(s,3H) |
| 216 | 3,03 | 2,99 | 7,68(d,1H), 7,35(d,1H), 6,08(q,1H), 3,93(q,2H), 2,90(s,6H), 2,67(d,3H), 2,42(s,3H) |
| 217 | 2,02 | 1,98 | 7,95(d,1H), 7,46(d,1H), 6,11(q,1H), 4,25-4,00(m,2H), 2,92(s,6H), 2,68(d,3H), 2,40(s,3H) |
| 218 | 2,06 | 2,04 | 7,64(d,1H), 7,32(d,1H), 6,19-6,15(m,1H), 5,27(breit,2H), 3,90(q,2H), 2,69(d,3H), 2,42(s,3H) |
| 219 | 1,32 | 1,29 | 7,93(d,1H), 7,42(d,1H), 6,25(q,1H), 5,31(breit,2H), 4,22-4,12(m,1H), 4,09-4,00(m,1H), 2,71(d,3H), 2,39(s,3H) |
| 220 | 2,51 | 2,48 | 7,65(d,1H), 7,35(d,1H), 5,37(breit,2H), 3,92(q,2H), 2,89(s,6H), 2,43(s,3H) |
| 221 | 1,63 | 1,60 | 7,93(d,1H), 7,46(d,1H), 5,40(breit,2H), 4,26-4,12(m,1H), 4,11-4,00(m,1H), 2,91(s,6H), 2,40(s,3H) |
| 222 | 3,22 | 3,17 | 7,64(d,1H), 7,34(d,1H), 5,34(breit,2H), 3,91(q,2H), 3,36(q,4H), 2,42(s,3H), 1,10(t,6H) |
| 223 | 2,23 | 2,18 | 7,92(d,1H), 7,45(d,1H), 5,37(breit,2H), 4,25-4,12(m,1H), 4,11-4,00(m,1H), 3,38(q,4H), 2,39(s,3H), 1,12(t,6H) |
| 224 | 2,74 | 2,71 | 7,63(d,1H), 7,32(d,1H), 5,84(d,1H), 5,26(breit,2H), 3,90(q,2H), 3,69-3,63(m,1H), 2,42(s,3H), 1,18(d,6H) |
| 225 | 1,87 | 1,83 | 7,92(d,1H), 7,42(d,1H), 5,93(d,1H), 5,30(breit,2H), 4,24-4,13(m,1H), 4,11-4,00(m,1H), 3,72-3,67(m,1H), 2,39(s,3H), 1,18(t,6H) |
| 226 | 1,76 | 1,74 | 7,97(d,1H), 7,52(d,1H), 4,30-4,19(m,1H), 4,10-4,00(m,1H), 4,04(s,3H), 2,42(s,3H), 2,35(s,3H) |
| 227 | 3,76 | 3,70 | 7,96(d,1H), 7,66-7,62(m,1H), 7,35(d,1H), 4,48(q,2H), 3,99(s,3H), 3,90(q,2H), 2,38(s,3H), 1,43(t,3H) |
| 228 | 2,12 | 2,08 | 8,03(d,1H), 7,57(d,1H), 4,30-4,23(m,1H), 4,06-3,97(m,1H), 3,59(s,3H), 3,14-3,11(m,1H), 2,43(s,3H), 1,21-1,16(m,2H), 1,07-1,03(m,2H) |
| 229 | 2,10 | 2,07 | 8,02-8,00(m,1H), 7,57(d,1H), 6,77-6,48(m,1H), 4,32-4,20(m,1H), 4,19-3,96(m,3H), 3,11-3,06(m,1H), 2,43(s,3H), 1,20-1,04(m,4H) |
| 230 | 2,45 | 2,40 | 7,76(d,1H), 7,45(d,1H), 3,95(q,2H), 3,10(s,3H), 3,09-3,05(m,1H), 2,45(s,3H), 1,11-1,05(m,4H) |
| 231 | 1,61 | 1,58 | 8,02-7,99(m,1H), 7,56(d,1H), 4,29-4,19(m,1H), 4,08-4,01(m,1H), 3,12(s,3H), 3,10-3,05(m,1H), 2,43(s,3H), 1,19-1,05(m,4H) |
| 232 | 2,25 | 2,21 | 7,78(d,1H), 7,45(d,1H), 3,96(q,2H), 3,47(s,3H), 3,14(s,3H), 2,45(s,3H) |
| 233 | 1,47 | 1,44 | 8,03-8,01(m,1H), 7,57(d,1H), 4,29-4,22(m,1H), 4,08-4,02(m,1H), 3,47(d,3H), 3,15(s,3H), 2,43(s,3H) |
| 234 | 2,68 | 2,65 | 7,75(d,1H), 7,44(d,1H), 3,95(q,2H), 3,44-3,33(m,2H), 3,09-3,04(m,1H), 2,45(s,3H), 1,29(t,3H), 1,15-1,03(m,4H) |
| 235 | 1,85 | 1,80 | 8,01-7,99(m,1H), 7,55(d,1H), 4,28-4,19(m,1H), 4,07-4,00(m,1H), 3,45-4,35(m,2H), 3,09-3,04(m,1H), 2,43(s,3H), 1,32-1,28(m,3H), 1,19-1,04(m,4H) |
| 236 | 3,05 | 2,98 | 7,75(d,1H), 7,60(d,1H), 3,95(q,2H), 3,39-3,34(m,2H), 3,10-3,04(m,1H), 2,45(s,3H), 1,83-1,74(m,2H), 1,16-1,03(m,7H) |
| 237 | 2,14 | 2,08 | 8,00(d,1H), 7,56(d,1H), 4,29-4,19(m,1H), 4,07-4,00(m,1H), 3,40-3,34(m,2H), 3,10-3,05(m,1H), 2,43(s,3H), 1,82-1,75(m,2H), 1,19-1,03(m,7H) |

(fortgesetzt)

| Bsp.-Nr. | logP[a] | logP[b] | $^1$H-NMR(D6-DMSO, 400MHz) δ ppm |
|---|---|---|---|
| 238 | 2,99 | 2,92 | 7,75(d,1H), 7,44(d,1H), 3,94(q,2H), 3,76-3,69(m,1H), 3,09-3,04(m,1H), 2,45(s,3H), 1,33(d,3H), 1,30(d,3H), 1,141,04(m,4H) |
| 239 | 2,08 | 2,03 | 8,01(d,1H), 7,55(d,1H), 4,28-4,19(m,1H), 4,06-4,00(m,1H), 3,75-3,68(m,1H), 3,09-3,04(m,1H), 2,43(s,3H), 1,34-1,30(m,6H), 1,13-1,02m,4H) |
| 240 | 2,53 | 2,46 | 7,80(d,1H), 7,45(d,1H), 4,01-3,92(m,4H), 3,14(s,3H), 2,45(s,3H), 1,33(t,3H) |
| 241 | 1,65 | 1,61 | 8,05-8,02(m,1H), 7,57(d,1H), 4,28-4,22(m,1H), 4,11-4,02(m,1H), 3,98-3,93(m,2H), 3,15(s,3H), 2,44(s,3H), 1,33(t,3H) |
| 242 | 1,79 | 1,75 | 8,06(d,1H), 7,57(d,1H), 6,05-5,95(m,1H), 5,31-5,22(m,2H), 4,60-4,50(m,2H), 4,29-4,19(m,1H), 4,10-4,02(m,1H), 3,13(s,3H), 2,44(s,3H) |
| 243 | 2,55 | 2,51 | 7,77(d,1H), 7,45(d,1H), 3,96(q,2H), 3,45(s,3H), 3,43-3,35(m,2H), 2,45(s,3H), 1,27(t,3H) |
| 244 | 1,69 | 1,65 | 8,02(d,1H), 7,56(d,1H), 4,28-4,19(m,1H), 4,08-4,00(m,1H), 3,45(s,3H), 3,43-3,34(m,2H), 2,43(s,3H), 1,29-1,25(m,3H) |
| 245 | 1,94 | 1,90 | 8,03(d,1H), 7,56(d,1H), 4,28-4,19(m,1H), 4,07-4,00(m,1H), 3,68-3,61(m,1H), 3,44(s,3H), 2,43(s,3H), 1,36-1,34(m,3H), 1,26(d,3H) |
| 246 | 1,99 | 1,95 | 8,02(d,1H), 7,56(d,1H), 4,29-4,19(m,1H), 4,08-4,01(m,1H), 3,46(s,3H), 3,43-3,35(m,2H), 2,43(s,3H), 1,78-1,73(m,2H), 1,06(t,3H) |
| 247 | 2,94 | 2,85 | 7,77(d,1H), 7,45(d,1H), 3,96(q,2H), 3,47(s,3H), 3,46-3,41(m,2H), 2,45(s,3H), 1,06-1,02(m,1H), 0,65-0,58(m,2H), 0,49-0,34(m,2H) |
| 248 | 2,03 | 1,98 | 8,01(d,1H), 7,56(d,1H), 4,28-4,19(m,1H), 4,08-4,00(m,1H), 3,47(s,3H), 3,45-3,41(m,2H), 2,43(s,3H), 1,07-1,02(m,1H), 0,66-0,60(m,2H), 0,49-0,35(m,2H) |
| 249 | 2,32 | 2,26 | 8,07(d,1H), 7,57(d,1H), 6,03-5,94(m,1H), 5,29-5,19(m,2H), 4,54(d,2H), 4,28-4,19(m,1H), 4,09-4,02(m,1H), 3,64-3,57(m,1H), 2,44(s,3H), 1,40-1,33(m,3H), 1,26(d,3H) |
| 250 | 2,34 | 2,29 | 7,95-7,92(m,1H), 7,56(d,1H), 7,39-7.33(m,5H), 4,76-4,69(m,2H), 4,29-4,20(m,1H), 4,08-4,00(m,1H), 3,25(d,3H), 2,43(s,3H) |
| 251 | 2,22 | 2,18 | 8,04(d,1H), 7,57(d,1H), 4,28-4,19(m,1H), 4,10-4,03(m,1H), 3,95(q,2H), 3,45-3,35(m,2H), 2,43(s,3H), 1,80-1,74(m,2H), 1,32(t,3H), 1,06(t,3H) |
| 252 | 1,90 | 1,87 | 8,04(d,1H), 7,56(d,1H), 4,28-4,19(m,1H), 4,10-4,00(m,1H), 3,94(q,2H), 3,45-3,35(m,2H), 2,43(s,3H), 1,34-1,25(m,6H) |
| 253 | 2,84 | 2,81 | 7,80(d,1H), 7,45(d,1H), 4,07-3,91(m,4H), 3,45-3,34(m,2H), 2,45(s,3H), 1,37-1,23(m,6H) |
| 254 | 3,89 | 3,84 | 7,68(d,1H), 7,40(d,1H), 4,17(q,2H), 3,93(q,2H), 2,96-2,90(m,1H), 2,43(s,3H), 1,05-0,95(m,4H) |
| 255 | 2,93 | 2,89 | 7,95(d,1H), 7,51(d,1H), 4,26-4,14(m,1H), 4,17(q,2H), 4,07-3,95(m,1H), 2,96-2,94(m,1H), 2,41(s,3H), 1,02-1,00(m,4H) |
| 256 | 2,73 | 2,69 | 7,78(d,1H), 7,51(d,1H), 6,48-6,18(m,1H), 4,30-4,17(m,1H), 4,08-3,96(m,1H), 3,71-3,62(m,2H), 2,93-2,88(m,1H), 2,41(s,3H), 1,04-0,96(m,4H) |
| 257 | 2,45 | 2,40 | 7,96(d,1H), 7,50(d,1H), 4,26-4,16(m,1H), 4,10-4,01(m,1H), 2,89-2,84(m,1H), 2,53(s,3H), 2,41(s,3H), 0,99-0,97(m,4H) |
| 258 | 3,71 | 3,75 | 7,71(d,1H), 7,39(d,1H), 6,49-6,18(m,1H), 3,94(q,2H), 3,70-3,61(m,2H), 2,92-2,86(m,1H), 2,42(s,3H), 1,04-0,94(m,4H) |
| 259 | 3,56 | 3,60 | 7,71(d,1H), 7,39(d,1H), 3,94(q,2H), 2,87-2,82(m,1H), 2,52(s,3H), 2,43(s,3H), 1,01-0,93(m,4H) |

(fortgesetzt)

| Bsp.-Nr. | logP[a] | logP[b] | $^1$H-NMR(D6-DMSO, 400MHz) δ ppm |
|---|---|---|---|
| 260 | 2,07 | 2,03 | 7,98(d,1H), 7,51(d,1H), 4,26-4,20(m,1H), 4,09-4,02(m,1H), 3,19(s,3H), 2,58(s,3H), 2,42(s,3H) |
| 261 | 3,99 | 4,03 | 7,70(d,1H), 7,38(d,1H), 3,94(q,2H), 3,11(q,2H), 2,86-2,81(m,1H), 2,42(s,3H), 1,36(t,3H), 1,01-0,93(m,4H) |
| 262 | 2,81 | 2,76 | 7,96(d,1H), 7,50(d,1H), 4,26-4,16(m,1H), 4,10-4,01(m,1H), 3,12(q,2H), 2,88-2,82(m,1H), 2,41(s,3H), 1,36(t,3H), 0,98-0,94(m,4H |
| 263 | 4,08 | 4,01 | 7,73(d,1H), 7,45(d,1H), 3,96(q,2H), 2,97-2,92(m,1H), 2,45(s,3H), 1,12-1,02(m,4H) |
| 264 | 3,05 | 2,98 | 7,99(d,1H), 7,57(d,1H), 4,28-4,19(m,1H), 4,14-4,04(m,1H), 2,98-2,93(m,1H), 2,43(s,3H), 1,13-1,02(m,4H) |
| 265 | 4,41 | 4,32 | 7,69(d,1H), 7,38(d,1H), 3,94(q,2H), 3,08(t,2H), 2,86-2,81(m,1H), 2,42(s,3H), 1,78-1,69(m,2H), 1,01-0,93(m,7H) |
| 266 | 3,24 | 3,17 | 7,97(d,1H), 7,50(d,1H), 4,25-4,21(m,1H), 4,06-4,02(m,1H), 3,11(t,2H), 2,88-2,85(m,1H), 2,42(s,3H), 1,78-1,72(m,2H), 1,01-0,97(m,7H) |
| 267 | 3,19 | 3,12 | 7,97(d,1H), 7,50(d,1H), 4,26-4,17(m,1H), 4,09-4,00(m,1H), 3,80-3,73(m,1H), 2,87-2,82(m,1H), 2,41(s,3H), 1,43-1,40(m,6H), 0,98-0,96(m,4H) |
| 268 | 4,37 | 4,28 | 7,71(d,1H), 7,39(d,1H), 3,94(q,2H), 3,78-3,71(m,1H), 2,86-2,79(m,1H), 2,43(s,3H), 1,41(d,6H), 0,99-0,94(m,4H) |
| 269 | 3,53 | 3,44 | 7,75(d,1H), 7,40(d,1H), 3,96(q,2H), 3,65(q,2H), 2,57(s,3H), 2,43(s,3H), 1,23(s,3H) |
| 270 | 2,39 | 2,33 | 7,99(d,1H), 7,51(d,1H), 4,26-4,19(m,1H), 4,11-4,02(m,1H), 3,66(q,2H), 2,59(s,3H), 2,42(s,3H), 1,24(t,3H) |
| 271 | 3,61 | 3,58 | 7,76(d,1H), 7,41(d,1H), 5,93-5,94(m,1H), 5,27-2,24(m,1H), 5,18-5,14(m,1H), 4,25(d,2H), 3,96(q,2H), 2,55(s,3H), 2,44(s,3H) |
| 272 | 2,54 | 2,48 | 8,01(d,1H), 7,52(d,1H), 5,93-5,86(m,1H), 5,27-5,24(m,1H), 5,19-5,14(m,1H), 4,27(d,2H), 4,23-4,20(m,1H), 4,11-4,05(m,1H), 2,56(s,3H), 2,42(s,3H) |
| 273 | 3,51 | 3,44 | 7,72(d,1H), 7,40(d,1H), 3,95(q,2H), 3,18(s,3H), 3,11(q,2H), 2,43(s,3H), 1,34(t,3H) |
| 274 | 2,42 | 2,36 | 7,98(d,1H), 7,52(d,1H), 4,26-4,20(m,1H), 4,09-4,03(m,1H), 3,19(s,3H), 3,12(q,2H), 2,42(s,3H), 1,34(t,3H) |
| 275 | 2,73 | 2,68 | 7,99(d,1H), 7,52(d,1H), 4,27-4,20(m,1H), 4,09-4,02(m,1H), 3,72-3,66(m,1H), 3,21(s,3H), 2,42(s,3H), 1,40-1,37(m,6H) |
| 276 | 3,86 | 3,80 | 7,73(d,1H), 7,41(d,1H), 3,95(q,2H), 3,71-3,64(m,1H), 3,20(s,3H), 2,43(s,3H), 1,37(d,6H) |
| 277 | 3,90 | 3,86 | 7,71(d,1H), 7,40(d,1H), 3,95(q,2H), 3,19(s,3H), 3,08(t,2H), 2,43(s,3H), 1,74-1,67(m,2H), 0,97(t,3H) |
| 278 | 2,80 | 2,74 | 7,97(d,1H), 7,51(d,1H), 4,26-4,20(m,1H), 4,09-4,02(m,1H), 3,20(s,3H), 3,10(t,2H), 2,42(s,3H), 1,74-1,67(m,2H), 0,98(t,3H) |
| 279 | 3,91 | 3,83 | 7,71(d,1H), 7,40(d,1H), 3,97(q,2H), 3,20(s,3H), 3,06(d,2H), 2,43(s,3H), 1,20-1,14(m,1H), 0,58-0,51(m,2H), 0,32-0,26(m,2H) |
| 280 | 2,82 | 2,75 | 7,97(d,1H), 7,52(d,1H), 4,26-4,20(m,1H), 4,09-4,02(m,1H), 3,21(s,3H), 3,07(d,2H), 2,42(s,3H), 1,39-1,32(m,1H), 0,59-0,55(m,2H), 0,33-0,29(m,2H) |
| 281 | 3,53 | 3,45 | 7,69(d,1H), 7,42(d,1H), 4,12(q,2H), 3,94(q,2H), 3,26(s,3H), 2,44(s,3H) |
| 282 | 2,57 | 2,52 | 7,96(d,1H), 7,53(d,1H), 4,29-4,17(m,1H), 4,12(q,2H), 4,11-3,99(m,1H), 3,27(s,3H), 2,42(s,3H) |
| 283 | 3,71 | 3,64 | 7,75(d,1H), 7,47(d,1H), 3,97(q,2H), 2,45(s,3H) |

(fortgesetzt)

| Bsp.-Nr. | logP[a] | logP[b] | $^1$H-NMR(D6-DMSO, 400MHz) δ ppm |
|---|---|---|---|
| 284 | 2,65 | 2,60 | 8,00(d,1H), 7,58(d,1H), 4,32-4,19(m,1H), 4,16-4,04(m,1H), 3,32(s,3H), 2,44(s,3H) |
| 285 | 3,24 | 3,17 | 8,03(d,1H), 7,53(d,1H), 5,92-5,85(m,1H), 5,25-5,22(m,1H), 5,14-5,10(m,1H), 4,29-4,28(m,2H), 4,28-4,20(m,1H), 4,17-4,05(m,1H), 3,74-3,61(m,1H), 2,43(s,3H), 1,40-4,35(m,6H) |
| 286 | 4,12 | 4,03 | 7,67(d,1H), 7,43-7,38(m,3H), 7,35-7,27(m,3H), 4,33(s,2H), 3,95(q,2H), 3,09(s,3H), 2,44(s,3H) |
| 287 | 3,08 | 3,02 | 7,97(d,1H), 7,53(d,1H), 7,42-7,27(m,5H), 4,35(s,2H), 4,28-4,22(m,1H), 4,07-4,00(m,1H), 3,10(s,3H), 2,42(s,3H) |
| 288 | 3,18 | 3,13 | 7,99(d,1H), 7,48(d,1H), 4,26-4,17(m,1H), 4,10-4,01(m,1H), 3,67(q,2H), 3,12(t,2H), 2,42(s,3H), 1,77-1,68(m,2H), 1,23(t,3H), 0,98(t,3H) |
| 289 | 3,30 | 3,25 | 7,72(d,1H), 7,41(d,1H), 6,46-6,18(m,1H), 3,94(q,2H), 3,68-3,59(m,2H), 3,23(s,3H), 2,43(s,3H) |
| 290 | 3,62 | 3,56 | 7,70(d,1H), 7,41(d,1H), 5,98-5,90(m,1H), 5,30-5,25(m,1H), 5,17-5,14(m,1H), 3,95(q,2H), 3,76(d,2H), 3,20(s,3H), 2,43(s,3H) |
| 291 | 2,55 | 2,51 | 7,97(d,1H), 7,52(d,1H), 6,00-5,90(m,1H), 5,31-5,26(m,1H), 5,18-5,15(m,1H), 4,27-4,20(m,1H), 4,09-4,02(m,1H), 3,77(d,2H), 3,21(s,3H), 2,42(s,3H) |
| 292 | 3,91 | 3,86 | 7,74(d,1H), 7,40(d,1H), 3,96(q,2H), 3,65(q,2H), 3,13(q,2H), 2,43(s,3H), 1,35(t,3H), 1,22(t,3H) |
| 293 | 2,76 | 2,72 | 8,00(d,1H), 7,51(d,1H), 4,25-4,16(m,1H), 4,13-4,04(m,1H), 3,66(q,2H), 3,14(q,2H), 2,42(s,3H), 1,35(t,3H), 1,23(t,3H) |
| 294 | 3,48 | 3,38 | 7,71(d,1H), 7,41(d,1H), 3,94(q,2H), 2,96-2,91(m,1H), 2,43(s,3H), 1,04-1,00(m,4H) |
| 295 | 2,39 | 2,34 | 7,96(d,1H), 7,52(d,1H), 4,27-4,18(m,1H), 4,07-3,98(m,1H), 2,96-2,91(m,1H), 2,41(s,3H), 1,05-1,00(m,4H) |
| 296 | 3,07 | 3,04 | 7,98(d,1H), 7,69-7,66(m,1H), 7,47(d,1H), 4,02(q,2H), 3,62(s,3H), 2,42(s,3H) |
| 297 | 1,96 | 1,96 | 7,88(s,1H), 7,48(s,1H), 4,26-4,19(m,1H), 4,02-3,96(m,1H), 3,63(s,3H), 2,41(s,3H), 2,27(s,3H) |
| 298 | 3,81 | 3,78 | 7,66(s,1H), 7,36(s,1H), 4,51-4,43(m,1H), 3,98(q,2H), 2,41(s,3H), 2,13(s,3H), 1,47(d,6H) |
| 299 | 2,45 | 0,90 | 14,78(s,1H), 7,89(d,1H), 7,52(d,1H), 4,00(q,2H), 2,46(s,3H) |
| 300 | 2,63 | 2,61 | 7,91(s,1H), 7,48(s,1H), 4,51-4,44(m,1H), 4,31-4,16(m,1H), 4,12-3,96(m,1H), 2,42(s,3H), 2,26(s,3H), 1,48(d,6H) |
| 301 | 1,87 | 1,82 | 8,13(d,1H), 7,65(d,1H), 4,32-4,22(m,1H), 4,08-4,01(m,1H), 3,61(s,3H), 2,46(s,3H) |
| 302 | 2,89 | 2,86 | 7,88(d,1H), 7,54(d,1H), 3,99(q,2H), 3,63(s,3H), 2,46(s,3H) |
| 303 | 1,59 | 0,37 | 8,13(d,1H), 7,64(d,1H), 4,30-4,21(m,1H), 4,12-4,01(m,1H), 2,46(s,3H) |
| 304 | 3,61 | 3,48 | 7,95(d,1H), 7,56(d,1H), 5,14(q,2H), 4,01(q,2H), 2,47(s,3H) |
| 305 | 2,57 | 2,51 | 8,20(d,1H), 7,67(d,1H), 5,12(q,2H), 4,32-4,23(m,1H), 4,11-4,00(m,1H), 2,47(s,3H) |
| 306 | 4,75 | 4,59 | 7,98-7,93(m,3H), 7,72-7,69(m,2H), 7,59(d,1H), 3,98(q,2H), s,3H unter DMSO-Signal |
| 307 | 3,53 | 3,49 | 8,25(d,1H), 7,96-7,92(m,2H), 7,72-7,68(m,3H), 4,35-4,26(m,1H), 4,08-3,96(m,1H), 2,48(s,3H) |
| 308 | 3,47 | 3,40 | 8,32(d,1H), 8,10(d,1H), 4,14(q,2H) |
| 309 | 2,86 | 2,80 | 8,53(d,1H), 8,30(d,1H), 4,55-4,43(m,1H), 4,32-4,20(m,1H) |
| 310 | 2,87 | 2,81 | 7,73(d,1H), 7,26(d,1H), 5,34(s,1H), 4,42(s,2H), 3,90(q,2H), 3,85(s,3H), 2,38(s,3H) |

(fortgesetzt)

| Bsp.-Nr. | logP[a] | logP[b] | $^1$H-NMR(D6-DMSO, 400MHz) δ ppm |
|---|---|---|---|
| 311 | 1,83 | 1,84 | 8,06(d,1H), 7,37(d,1H), 5,38(s,1H), 4,48-4,44(m,2H), 4,20-4,13(m,1H), 4,06-3,94(m,1H), 3,86(s,3H), 2,38(s,3H) |
| 312 | 2,88 | 2,81 | 7,42(s,1H), 7,16(s,1H), 5,28(s,1H), 4,36(s,2H), 3,93(q,2H), 3,84(s,3H), 2,32(s,3H), 2,08(s,3H) |
| 313 | 1,83 | 1,83 | 7,70(s,1H), 7,27(s,1H), 5,31(s,1H), 4,44-4,33(m,2H), 4,14-3,93(m,2H), 3,85(s,3H), 2,35(s,3H), 2,18(s,3H) |
| 314 | 2,24 | 2,23 | 8,03(s,1H), 7,93(s,1H), 5,39(s,1H), 4,48-4,38(m,2H), 4,33-4,24(m,1H), 4,14-4,05(m,1H), 3,87(s,3H) |
| 315 | 2,24 | 2,23 | 8,36(d,1H), 7,84-8,81(m,1H), 7,60(d,1H), 5,41(s,1H), 4,62-4,52(m,2H), 4,20-4,00(m,2H), 3,87(s,3H) |
| 316 | 2,98 | 2,95 | 7,78(d,1H), 7,58-7,55(m,1H), 7,21(d,1H), 5,32(s,1H), 4,49(s,2H), 3,92(q,2H), 3,85(s,3H), 2,32(s,3H) |
| 317 | 1,90 | 1,90 | 8,22(d,1H), 7,77-7,74(m,1H), 7,31(d,1H), 5,37(s,1H), 4,54(q,2H), 4,11-4,02(m,1H), 3,96-3,90(m,1H), 3,86(s,3H), 2,32(s,3H) |
| 318 | 1,99 | 1,94 | 7,87(s,1H), 7,63(s,1H), 5,37(s,1H), 4,38(s,2H), 4,24-4,17(m,1H), 4,04-3,98(m,1H), 3,87(s,3H), 2,38(s,3H) |
| 319 | 3,02 | 2,98 | 7,62(s,1H), 7,48(s,1H), 5,33(s,1H), 4,33(s,2H), 4,03(q,2H), 3,86(s,3H), 2,35(s,3H) |
| 320 | 2,45 | 2,43 | 7,99(d,1H), 7,91-7,88(m,1H), 7,82(d,1H), 5,42(s,1H), 4,58(s,2H), 4,16(q,2H), 3,88(s,3H) |
| 321 | 2,61 | 2,55 | 8,56(d,1H), 8,12-8,09(m,1H), 7,89(d,1H), 5,46(s,1H), 4,74-4,59(m,2H), 4,19-4,02(m,2H), 3,90(s,3H) |
| 322 | 3,31 | 3,25 | 7,97-7,93(m,2H), 7,71(d,1H), 5,41(s,1H), 4,60(s,2H), 4,13(q,2H), 3,88(s,3H) |
| 323 | 2,87 | 2,81 | 8,07(d,1H), 7,50-7,36(m,6H), 5,49(s,1H), 5,17(s,2H), 4,56-4,46(m,2H), 4,20-4,10(m,1H), 4,04-3,94(m,1H), 2,38(s,3H) |
| 324 | 2,13 | 2,11 | 8,21(d,1H), 7,85(d,1H), 5,42(s,1H), 4,57-4,47(m,2H), 4,28-4,22(m,1H), 4,11-4,05(m,1H), 3,87(s,3H) |
| 325 | 2,46 | 2,43 | 8,07(d,1H), 7,37(d,1H), 5,37(s,1H), 4,60-4,53(m,1H), 4,45-4,35(m,2H), 4,19-4,13(m,1H), 4,00-3,89(m,1H), 2,37(s,3H),1,32(d,6H) |
| 326 | 2,91 | 2,88 | 8,07(d,1H), 7,37(d,1H), 5,37(s,1H), 4,50-4,40(m,2H), 4,20-4,4,10(m,1H), 4,07(t,2H), 4,03-3,91(m,1H), 2,38(s,3H), 1,74-1,67(m,2H), 1,46-1,37(m,2H), 0,93(t,3H) |
| 327 | 2,97 | 2,92 | 7,90(d,1H), 7,67(d,1H), 5,37(s,1H), 4,48(s,2H), 4,07(q,2H), 3,86(s,3H) |
| 328 | 2,86 | 2,82 | 7,72(d,1H), 7,25(d,1H), 5,35(s,1H), 4,43(s,2H), 4,19-4,17(m,2H), 3,91(q,2H), 3,67-3,64(m,2H), 3,30(s,3H), 2,37(s,3H) |
| 329 | 4,20 | 4,06 | 7,72(d,1H), 7,71(d,1H), 5,28(s,1H), 4,80-4,76(m,1H), 4,37(s,2H), 3,91(q,2H), 2,37(s,3H), 1,96-1,89(m,2H), 1,80-1,58(m,6H) |
| 330 | 3,29 | 3,21 | 7,72(d,1H), 7,25(d,1H), 4,41(s,2H), 4,11(q,2H), 3,91(q,3H), 2,37(s,3H), 1,34(t,3H) |
| 331 | 3,64 | 3,58 | 7,72(d,1H), 7,25(d,1H), 5,29(s,1H), 4,42(s,2H), 3,95-3,87(m,4H), 2,37(s,3H), 1,25-1,21(m,1H), 0,61-0,58(m,2H), 0,37-0,34(m,2H) |
| 332 | 2,55 | 2,50 | 8,06(d,1H), 7,37(d,1H), 5,34(s,1H), 4,48-4,44(m,2H), 4,20-4,11(m,1H), 4,03-3,91(m,3H), 2,38(s,3H), 1,26-1,22(m,1H), 0,61-0,58(m,2H), 0,37-0,33(m,2H) |
| 333 | 2,57 | 2,52 | 8,07(d,1H), 7,37(d,1H), 5,36(s,1H), 4,57-4,55(m,2H), 4,20-4,13(m,1H), 4,03(t,2H), 4,01-3,94(m,1H), 2,38(s,3H), 1,79-1,70(m,2H), 0,96(t,3H) |

(fortgesetzt)

| Bsp.-Nr. | logP[a] | logP[b] | $^1$H-NMR(D6-DMSO, 400MHz) δ ppm |
|---|---|---|---|
| 334 | 2,19 | 2,14 | 8,07(d,1H), 7,37(d,1H), 5,36(s,1H), 4,45(d,2H), 4,20-4,10(m,3H), 4,03-3,94(m,1H), 2,37(s,3H), 1,35(t,3H) |
| 335 | 1,92 | 1,86 | 8,06(d,1H), 7,37(d,1H), 5,40(s,1H), 4,49-4,45(m,2H), 4,22-4,18(m,3H), 4,02-3,91(m,1H), 3,67-3,65(m,2H), 3,30(s,3H), 2,38(s,3H) |
| 336 | 2,97 | 2,91 | 8,07(d,1H), 7,36(d,1H), 5,33(s,1H), 4,81-4,77(m,1H), 4,04(s,2H), 4,19-4,10(m,1H), 4,00-3,91(m,1H), 2,37(s,3H), 1,98-1,90(m,2H), 1,81-1,76(m,2H), 1,73-1,58(m,4H) |
| 337 | 3,91 | 3,50 | 7,72(s,1H), 7,71-7,69(m,1H), 7,44-7,42(m,1H), 3,93-3,86(q,2H), 2,44(s,3H) |
| 338 | 3,99 | 4,00 | 7,71-7,70(m,1H), 7,47-7,45(m,1H), 3,89-3,84(q,2H), 2,46(s,3H) |
| 339 | 3,60 | 3,61 | 7,63-7,62(m,1H), 7,41-7,38(m,1H), 3,95-3,88(q,2H), 2,43(s,3H), 2,00(s,6H) |
| 340 | 3,37 | 3,39 | 7,65-7,63(m,1H), 7,41-7,39(m,1H), 6,90-6,88(q,1H), 3,95-3,88(q,2H), 2,43(s,3H), 2,10(d,3H) |
| 341 | 2,52 | 2,49 | 7,90-7,88(m,1H), 7,53-7,50(m,1H), 4,30-4,24(m,1H), 3,97-3,90(m,1H), 2,43(s,3H), 2,01(s,6H) |
| 342 | 3,73 | 3,70 | 7,39(s,1H), 7,26(s,1H), 3,93-3,85(q,2H), 2,37(s,3H), 2,01(s,3H), 1,99(s,6H) |
| 343 | 3,02 | 4,91 | 7,63(d,1H), 7,37(d,1H), 4,08-4,02(m,1H), 3,95-3,90(m,1H), 3,64-3,59(m,2H), 2,41(s,3H), 2,11(s,3H), 1,71(s,3H) |
| 344 | 2,17 | 3,68 | 7,81(m,1H), 7,52(d,1H), 4,36-4,17(m,2H), 3,89-3,56(m,2H), 2,45-2,44(m,3H), 2,13-2,12(m,3H), 1,77-1,74(m,3H) |
| 345 | 4,02 | 4,04 | 7,85(d,1H), 7,48(d,1H), 5,99-5,89(m,1H), 5,29-5,18(m,2H), 4,44(d,2H), 3,97(q,2H), 2,46(s,3H) |
| 346 | 2,97 | 2,92 | 7,65(d,1H), 7,37(d,1H), 3,92(q,2H), 3,30(s,3H), 2,42(s,3H), 2,00-1,93(m,1H), 0,99-0,95(m,2H), 0,91-0,85(m,2H) |
| 347 | 3,70 | 3,63 | 7,67(d,1H), 7,36(d,1H), 4,50-4,43(m,1H), 3,93(q,2H), 2,42(s,3H), 2,05-1,99(m,1H), 1,49(d,6H), 0,99-0,94(m,2H), 0,89-0,85(m,2H) |
| 348 | 2,91 | 2,87 | 8,77(d,1H), 8,73-8,71(m,1H), 8,06-8,03(m,1H), 7,81(d,1H), 7,66-7,63(m,1H), 7,44(d,1H), 3,95(q,2H), 2,56(q,2H), 2,46(s,3H), 1,09(t,3H) |
| 349 | 3,48 | 3,40 | 8,73(s,1H), 7,82-7,75(m,3H), 7,46-7,40(m,3H), 3,97(q,2H), 2,45(s,3H) |
| 350 | 3,40 | 3,34 | 7,80(d,1H), 7,60-7,50(m,5H), 7,42(d,1H), 3,96(q,2H), 2,45(s,3H), 2,17(s,3H) |
| 351 | 3,43 | 3,36 | 8,76(s,1H), 7,82(d,1H), 7,74(d,2H), 7,56(t,2H), 7,46-7,41(m,2H), 3,98(q,2H), 2,45(s,3H) |
| 352 | 1,99 | 1,96 | 7,91(d,1H), 7,48(d,1H), 4,24-4,15(m,1H), 4,11-4,02(m,1H), 3,32(s,3H), 2,40(s,3H), 2,02-1,95(m,1H), 1,01-0,95(m,2H), 0,91-0,87(m,2H) |
| 353 | 2,58 | 2,55 | 7,92(d,1H), 7,47(d,1H), 4,52-4,45(m,1H), 4,23-4,17(m,1H), 4,14-4,04(m,1H), 2,40(s,3H), 2,07-2,01(m,1H), 1,51-1,48(m,6H), 1,00-0,88(m,4H) |
| 354 | 1,98 | 1,97 | 8,79(d,1H), 8,73-8,72(m,1H), 8,07(d,2H), 7,66-7,63(m,1H), 7,55(d,1H), 4,30-4,20(m,1H), 4,12-4,00(m,1H), 2,56(q,2H), 2,43(s,3H), 1,10(t,3H) |
| 355 | 2,45 | 2,41 | 8,76(s,1H), 8,07(d,1H), 7,80-7,75(m,2H), 7,56(d,1H), 7,45-7,40(m,2H), 4,30-4,20(m,1H), 4,12-4,00(m,1H), 2,44(s,3H) |
| 356 | 2,38 | 2,35 | 8,06(d,1H), 7,61-7,51(m,6H), 4,29-4,22(m,1H), 4,09-4,02(m,1H), 2,43(s,3H), 2,18(s,3H) |
| 357 | 2,38 | 2,36 | 8,80(s,1H), 8,08(d,1H), 7,75-7,73(m,2H), 7,59-7,55(m,3H), 7,46-7,41(m,1H), 4,29-4,20(m,1H), 4,13-4,01(m,1H), 2,44(s,3H) |

(fortgesetzt)

| Bsp.-Nr. | logP[a] | logP[b] | $^{1}$H-NMR(D6-DMSO, 400MHz) δ ppm |
|---|---|---|---|
| 358 | 4,18 | 4,20 | 7,66(d,1H), 7,36(d,1H), 3,92(q,2H), 3,31-3,23(m,1H), 2,91-2,86(m,1H), 2,42(s,3H), 2,08-2,00(m,2H), 1,86-1,77(m,2H), 1,75-1,60(m,4H), 1,02-0,98(m,4H) |
| 359 | 3,63 | 3,66 | 7,65(d,1H), 7,38(d,1H), 3,92(q,2H), 3,74(t,2H), 3,64-3,57(m,1H), 2,93-2,87(m,3H), 2,43(s,3H), 1,09(d,6H), 0,98-0,96(m,4H) |
| 360 | 3,06 | 3,03 | 7,92(d,1H), 7,47(d,1H), 4,24-4,15(m,1H), 4,11-4,02(m,1H), 3,31-2,25(m,1H), 2,93-2,88(m,1H), 2,40(s,3H), 2,08-2,01(m,2H), 1,88-1,79(m,2H), 1,75-1,61(m,4H), 1,05-0,96(m,4H) |
| 361 | 3,34 | 3,31 | 7,91(d,1H), 7,47(d,1H), 4,24-4,15(m,1H), 4,12-4,03(m,1H), 2,95-2,83(m,2H), 2,40(s,3H), 2,04(m,2H), 1,81-1,78(m,2H), 1,70-1,67(m,1H), 1,49-1,22(m,5H), 1,01-0,99(m,4H) |
| 362 | 2,63 | 2,61 | 7,92(d,1H), 7,48(d,1H), 4,25-4,16(m,1H), 4,09-4,00(m,1H), 3,75(t,2H), 3,64-3,58(m,1H), 2,95-2,88(m,3H), 2,40(s,3H), 1,09(d,6H), 1,02-0,98(m,4H) |
| 363 | 3,22 | 3,18 | 8,08(d,1H), 7,57(d,1H), 4,38-4,31(m,1H), 4,29-4,20(m,1H), 4,10-4,01(m,1H), 2,44(s,3H), 1,53-1,50(m,6H) |
| 364 | 3,00 | 2,96 | 8,10(d,1H), 7,59(d,1H), 6,00-5,90(m,1H), 5,29-5,20(m,2H), 4,44(d,2H), 4,30-4,21(m,1H), 4,10-4,01(m,1H), 2,44(s,3H) |
| 365 | 3,61 | 3,54 | 8,08(d,1H), 7,58(d,1H), 4,29-4,20(m,1H), 4,10-3,98(m,1H), 3,78(t,2H), 2,44(s,3H), 1,72-1,64(m,2H), 1,41-1,31(m,2H), 0,92(t,3H) |
| 366 | 3,77 | 3,71 | 7,77(d,1H), 7,61-7,57(m,1H), 7,46-7,40(m,2H), 7,23(d,1H), 7,12-7,08(m,1H), 3,96(q,2H), 3,89(s,3H), 2,95-2,91(m,1H), 2,44(s,3H), 0,72-0,67(m,2H), 0,55-0,51(m,2H) |
| 367 | 3,27 | 3,23 | 8,22(s,1H), 7,70(d,1H), 7,39(d,1H), 3,94(t,2H), 2,43(s,3H), 1,54(s,9H) |
| 368 | 3,38 | 3,34 | 8,36(s,1H), 7,74(d,1H), 7,42-7,32(m,6H), 4,87(s,2H), 3,96(q,2H), 2,43(s,3H) |
| 369 | 2,21 | 2,16 | 8,27(s,1H), 7,97(d,1H), 7,50(d,1H), 4,29-4,16(m,1H), 4,14-4,02(m,1H), 2,42(s,3H), 1,55(s,9H) |
| 370 | 2,35 | 2,36 | 8,41(s,1H), 8,00(d,1H), 7,51(d,1H), 7,43-7,32(m,5H), 4,89(s,2H), 4,25-4,16(m,1H), 4,13-4,04(m,1H), 2,42(s,3H) |
| 371 | 3,46 | 3,39 | 8,16(s,1H), 7,46(s,1H), 7,26(s,1H), 3,96-3,88(m,2H), 2,39(s,3H), 2,11(s,3H), 1,54(s,9H) |
| 372 | 2,34 | 2,28 | 8,22(s,1H), 7,75(s,1H), 7,36(s,1H), 4,16-4,13(m,1H), 4,04-4,01(m,1H), 2,37(s,3H), 2,24(s,3H), 1,55(s,9H) |
| 373 | 3,55 | 3,47 | 8,30(s,1H), 7,50(s,1H), 7,42-7,32(m,5H), 7,27(s,1H), 4,88(s,2H), 3,92(q,2H), 2,39(s,3H), 2,13(s,3H) |
| 374 | 2,50 | 2,47 | 8,35(s,1H), 7,78(s,1H), 7,43-7,32(m,6H), 4,89(s,2H), 4,20-4,10(m,1H), 4,07-3,97(m,1H), 2,38(s,3H), 2,25(s,3H) |
| 375 | 3,74 | 3,70 | 7,68(d,1H), 7,36(d,1H), 6,13-6,03(m,1H), 5,52-5,46(m,1H), 5,37-5,32(m,1H), 4,82-4,79(m,2H), 3,91(q,2H), 2,84-2,78(m,1H), 2,42(s,3H), 0,95-0,90(m,4H) |
| 376 | 4,23 | 4,18 | 7,72(d,1H), 7,39(d,1H), 5,14(s,2H), 3,93(q,2H), 3,19(s,3H), 2,43(s,3H) |
| 377 | 4,01 | 3,92 | 7,48(s,1H), 7,25(s,1H), 5,01(q,2H), 3,88(q,2H), 2,89-2,82(m,1H), 2,38(s,3H), 2,16(s,3H), 0,98-0,90(m,4H) |
| 378 | 4,43 | 4,48 | 7,69(d,1H), 7,38(d,1H), 5,02(q,2H), 3,91(q,2H), 2,88-2,83(m,1H), 2,43(s,3H), 0,96-0,90(m,4H) |
| 379 | 2,14 | 2,09 | 7,95(d,1H), 7,47(d,1H), 4,25-4,18(m,1H), 4,06-4,00(m,1H), 3,97(s,3H), 2,82-2,77(m,1H), 2,40(s,3H), 0,94-0,91(m,4H) |

(fortgesetzt)

| Bsp.-Nr. | logP[a] | logP[b] | $^1$H-NMR(D6-DMSO, 400MHz) δ ppm |
|---|---|---|---|
| 380 | 2,14 | 2,09 | 7,95(d,1H), 7,47(d,1H), 4,25-4,18(m,1H), 4,06-4,00(m,1H), 3,97(s,3H), 2,82-2,77(m,1H), 2,40(s,3H), 0,94-0,91(m,4H) |
| 381 | 2,88 | 2,94 | 7,77(s,1H), 7,35(d,1H), 5,06-4,99(m,2H), 4,20-4,11(m,1H), 3,96-3,86(m,1H), 2,89-2,84(m,1H), 2,36(s,3H), 2,27(s,3H), 0,95-0,94(m,4H) |
| 382 | 3,22 | 3,13 | 8,01-7,97(m,2H), 5,09-5,02(m,2H), 4,35-4,26(m,1H), 4,14-4,02(m,1H), 2,89-2,84(m,1H), 0,96-0,94(m,4H) |
| 383 | 3,27 | 3,26 | 8,08(d,1H), 7,99(d,1H), 5,05(q,2H), 4,31-4,24(m,1H), 4,09-4,03(m,1H), 2,88-2,85(m,1H), 0,96-0,94(m,4H) |
| 384 | 3,06 | 2,97 | 7,92(s,1H), 7,71(s,1H), 5,06-4,99(m,2H), 4,29-4,20(m,1H), 4,07-3,96(m,1H), 2,90-2,85(m,1H), 2,40(s,3H), 0,96-0,92(m,4H) |
| 385 | 2,80 | 2,74 | 7,72(d,1H), 7,40(d,1H), 5,02(q,2H), 4,23-4,16(m,1H), 4,06-3,91(m,1H), 3,92(s,3H), 2,88-2,83(m,1H), 0,94-0,93(m,4H) |
| 386 | 4,77 | 4,71 | 7,68(d,1H), 7,36(d,1H), 3,94(s,2H), 3,91(q,2H), 2,86-2,79(m,1H), 2,42(s,3H), 1,01(s,9H), 0,96-0,90(m,4H) |
| 387 | 3,60 | 3,52 | 7,93(d,1H), 7,46(d,1H), 4,24-4,14(m,1H), 4,10-3,99(m,1H), 3,96(s,2H), 2,89-2,80(m,1H), 2,40(s,3H), 1,02(s,9H), 0,97-0,92(m,4H) |
| 388 | 3,16 | 3,15 | 7,71(d,1H), 7,37(d,1H), 3,97(s,3H), 3,92(q,2H), 3,60(t,2H), 3,35(t,2H), 3,22(s,3H), 2,43(s,3H), 1,86-1,80(m,2H) |
| 389 | 3,53 | 3,46 | 7,72(d,1H), 7,37(d,1H), 3,97(s,3H), 3,93(q,2H), 3,51(t,2H), 2,43(s,3H), 1,67-1,58(m,2H), 0,88(t,3H) |
| 390 | 3,64 | 3,58 | 7,73(d,1H), 7,38(d,1H), 3,98(s,3H), 3,94(q,2H), 3,42(d,2H), 2,43(s,3H), 1,16-1,08(m,1H), 0,54-0,48(m,2H), 0,36-0,30(m,2H) |
| 391 | 2,68 | 2,65 | 7,94(d,1H), 7,47(d,1H), 6,13-6,03(m,1H), 5,51-5,46(m,1H), 5,36-5,33(m,1H), 4,83-4,81(m,2H), 4,24-4,15(m,1H), 4,08-3,99(m,1H), 2,85-2,80(m,1H), 2,40(s,3H), 0,94-0,91(m,4H) |
| 392 | 3,13 | 3,09 | 7,97(d,1H), 7,50(d,1H), 5,19-5,13(m,2H), 4,26-4,20(m,1H), 4,07-4,01(m,1H), 3,20(s,3H), 2,41(s,3H) |
| 393 | 3,50 | 3,47 | 7,75(d,1H), 7,41(d,1H), 4,49(q,2H), 4,01(s,3H), 3,95(q,2H), 2,42(s,3H) |
| 394 | 4,95 | 4,89 | 7,84(d,1H), 7,78(d,1H), 4,07(q,2H), 3,95(s,2H), 2,87-2,79(m,1H), 1,01(s,9H), 0,96-0,92(m,4H) |
| 395 | 4,38 | 4,32 | 7,56(d,1H), 7,18(d,1H), 3,95-3,80(m,7H), 2,84-2,77(m,1H), 1,01(s,9H), 0,96-0,90(m,4H) |
| 396 | 4,92 | 4,85 | 7,68(s,1H), 7,55(s,1H), 4,02(q,2H), 3,92(s,2H), 2,86-2,81(m,1H), 2,39(s,3H), 1,01(s,9H), 0,96-0,92(m,4H) |
| 397 | 5,12 | 5,04 | 7,91(s,1H), 7,83(s,1H), 4,17(q,2H), 3,93(s,2H), 2,87-2,81(m,1H), 1,01(s,9H), 0,99-0,88(m,4H) |
| 398 | 3,84 | 3,79 | 7,72(d,1H), 7,39(d,1H), 5,07(q,2H), 3,93(q,2H), 3,63(q,2H), 2,43(s,3H), 1,23(t,3H) |
| 399 | 3,94 | 3,89 | 7,74(d,1H), 7,40(d,1H), 5,95-5,85(m,1H), 5,25-5,22(m,1H), 5,17-5,13(m,1H), 5,06(q,2H), 4,23-4,22(m,2H), 3,93(q,2H), 2,44(s,3H) |
| 400 | 4,06 | 3,96 | 7,71(d,1H), 7,36(d,1H), 4,26(t,2H), 3,93(q,2H), 3,59(q,2H), 2,43(s,3H), 1,82-1,73(m,2H), 1,21(t,3H), 0,97(t,3H) |
| 401 | 3,62 | 3,53 | 7,71(d,1H), 7,37(d,1H), 4,35(q,2H), 3,93(q,2H), 3,58(q,2H), 2,43(s,3H), 1,38(t,3H), 1,20(t,3H) |

(fortgesetzt)

| Bsp.-Nr. | logP[a] | logP[b] | $^1$H-NMR(D6-DMSO, 400MHz) δ ppm |
|---|---|---|---|
| 402 | 4,13 | 3,98 | 7,98(d,1H), 7,94(d,1H), 4,33-4,22(m,1H), 4,19-4,08(m,1H), 3,97(s,2H), 2,87-2,79(m,1H), 1,02(s,9H), 0,97-0,90(m,4H) |
| 403 | 3,50 | 3,44 | 7,70(d,1H), 7,37(d,1H), 4,22-4,11(m,1H), 4,02-3,91(m,1H), 3,94(s,2H), 3,93(s,3H), 2,86-2,79(m,1H), 1,01(s,9H), 0,96-0,92(m,4H) |
| 404 | 3,79 | 3,71 | 7,90(s,1H), 7,68(s,1H), 4,31-4,20(m,1H), 4,11-4,00(m,1H), 3,94(s,2H), 2,89-2,80(m,1H), 2,40(s,3H), 1,01(s,9H), 0,97-0,92(m,4H) |
| 405 | 4,36 | 4,18 | 8,09(s,1H), 7,93(s,1H), 4,37-4,27(m,1H), 4,20-4,10(m,1H), 3,95(s,2H), 2,87-2,80(m,1H), 1,02(s,9H), 0,98-0,91(m,4H) |
| 406 | 2,82 | 2,78 | 7,98(d,1H), 7,50(d,1H), 5,13-5,05(m,2H), 4,27-4,15(m,1H), 4,10-3,98(m,1H), 3,64(q,2H), 2,41(s,3H), 1,24(t,3H) |
| 407 | 2,94 | 2,90 | 8,00(d,1H), 7,51(d,1H), 5,97-5,74(m,1H), 5,26-5,22(m,1H), 5,18-5,13(m,1H), 5,12-5,04(m,2H), 4,27-4,16(m,3H), 4,11-4,00(m,1H), 2,42(s,3H) |
| 408 | 2,86 | 2,81 | 7,96(d,1H), 7,47(d,1H), 4,28(t,2H), 4,25-4,14(m,1H), 4,12-4,02(m,1H), 3,60(q,2H), 2,40(s,3H), 1,83-1,73(m,2H), 1,22(t,3H), 0,98(t,3H) |
| 409 | 2,44 | 2,44 | 7,96(d,1H), 7,47(d,1H), 4,37(q,2H), 4,24-4,15(m,1H), 4,11-4,02(m,1H), 3,59(q,2H), 2,40(s,3H), 1,38(t,3H), 1,21(t,3H) |
| 410 | 4,32 | 4,25 | 7,69(d,1H), 7,53-7,51(m,2H), 7,46-7,36(m,4H), 5,33(s,2H), 3,92(q,2H), 2,84-2,79(m,1H), 2,43(s,3H), 0,95-0,88(m,4H) |
| 411 | 2,44 | 2,41 | 7,97(d,1H), 7,48(d,1H), 4,30-4,15(m,1H), 4,15-4,02(m,1H), 3,99(s,3H), 3,52(t,2H), 2,41(s,3H), 1,70-1,59(m,2H), 0,88(t,3H) |
| 412 | 2,54 | 2,52 | 7,98(d,1H), 7,49(d,1H), 4,39-4,15(m,1H), 4,15-4,01(m,1H), 4,01(s,3H), 3,44(d,2H), 2,41(s,3H), 1,19-1,09(m,1H), 0,55-0,49(m,2H), 0,45-39(m,2H) |
| 413 | 2,44 | 2,40 | 7,97(d,1H), 7,48(d,1H), 4,30-4,15(m,2H), 4,15-4,02(m,1H), 3,99(s,3H), 2,40(s,3H), 1,39(d,6H) |
| 414 | 3,24 | 3,19 | 7,97(d,1H), 7,58-7,39(m,6H), 5,35(s,2H), 4,26-4,16(m,1H), 4,08-3,99(m,1H), 2,85-2,80(m,1H), 2,40(s,3H), 0,98-0,88(m,4H) |
| 415 | 2,48 | 2,45 | 8,00(d,1H), 7,51(d,1H), 4,50(q,2H), 4,30-4,15(m,1H), 4,15-4,03(m,1H), 4,03(s,3H), 2,42(s,3H) |
| 416 | 3,86 | 3,80 | 8,04(d,1H), 7,70-7,68(m,1H), 7,30(d,1H), 3,87(q,2H), 2,95-2,91(m,1H), 2,90(s,6H), 2,36(s,3H), 1,04-1,00(m,2H), 0,95-0,90(m,2H) |
| 417 | 2,25 | 2,21 | 8,02(d,1H), 7,67(d,1H), 5,45(s,2H), 4,98(q,2H), 3,10-3,20(m,1H), 2,69(s,3H), 1,25(d,6H) |
| 418 | 3,52 | 3,49 | 7,73(d,1H), 7,41(d,1H), 4,06(s,3H), 3,95(q,2H), 3,11-3,04(m,1H), 2,44(s,3H), 1,27(d,6H) |
| 419 | 2,46 | 2,42 | 7,97(d,1H), 7,52(d,1H), 4,26-4,19(m,1H), 4,11-4,05(m,1H), 4,07(s,3H), 3,13-3,06(m,1H), 2,42(s,3H), 1,30-1,28(m,6H) |
| 420 | 2,70 | 2,77 | 7,80(d,1H), 7,48(d,1H), 3,95(q,2H), 3,57(s,3H), 3,47(s,3H), 2,46(s,3H) |
| 421 | 1,88 | 1,84 | 8,05(d,1H), 7,59(d,1H), 4,30-4,24(m,1H), 4,06-4,00(m,1H), 3,57(s,3H), 3,47(s,3H), 2,44(s,3H) |
| 422 | 2,12 | 2,11 | 8,08(d,1H), 7,59(d,1H), 4,29-4,23(m,1H), 4,08-4,01(m,1H), 3,96(q,2H), 3,60(s,3H), 2,44(s,3H), 1,31(t,3H) |
| 423 | 2,34 | 2,37 | 8,06(d,1H), 7,57(d,1H), 6,04-5,95(m,1H), 5,30-5,28(m,1H), 5,25-5,21(m,1H), 4,56-4,53(m,2H), 4,28-4,19(m,1H), 4,10-4,03(m,1H), 3,42-3,27(m,2H), 2,44(s,3H), 1,77-1,72(m,2H), 1,04(t,3H) |

(fortgesetzt)

| Bsp.-Nr. | logP[a] | logP[b] | $^1$H-NMR(D6-DMSO, 400MHz) δ ppm |
|---|---|---|---|
| 424 | 2,05 | 2,04 | 8,07(d,1H), 7,57(d,1H), 6,04-5,95(m,1H), 5,30-5,27(m,1H), 5,25-5,20(m,1H), 4,56-4,48(m,2H), 4,30-4,18(m,1H), 4,12-4,00(m,1H), 3,45-3,30(m,2H), 2,44(s,3H), 1,29-1,25(m,3H) |
| 425 | 4,10 | 4,01 | 7,69(d,1H), 7,39(d,1H), 6,03-5,93(m,1H), 5,35-5,31(m,1H), 5,19-5,16(m,1H), 3,94(q,2H), 3,78(d,2H), 2,88-2,83(m,1H), 2,43(s,3H), 1,01-0,92(m,4H) |
| 426 | 4,37 | 4,29 | 7,73(d,1H), 7,40(d,1H), 3,96(q,2H), 3,66(q,2H), 3,10(t,2H), 2,43(s,3H), 1,77-1,68(m,2H), 1,22(t,3H), 0,97(t,3H) |
| 427 | 4,45 | 4,37 | 7,75(d,1H), 7,41(d,1H), 5,93-5,84(m,1H), 5,26-5,23(m,1H), 5,16-5,11(m,1H), 4,26-4,25(m,2H), 3,96(q,2H), 3,08(t,2H), 2,43(s,3H), 1,75-1,66(m,2H), 0,96(t,3H) |
| 428 | 4,03 | 3,96 | 7,76(d,1H), 7,41(d,1H), 5,93-5,84(m,1H), 5,26-5,23(m,1H), 5,16-5,11(m,1H), 4,26-4,25(m,2H), 3,96(q,2H), 3,10(q,2H), 2,44(s,3H), 1,33(t,3H) |
| 429 | 4,13 | 4,07 | 7,74(d,1H), 7,41(d,1H), 5,99-5,87(m,2H), 5,25-5,23(m,2H), 5,17-5,11(m,2H), 4,28-4,26(m,2H), 3,96(q,2H), 3,76(d,2H), 2,44(s,3H) |
| 430 | 3,18 | 3,33 | 8,01(d,1H), 7,52(d,1H), 5,95-5,86(m,1H), 5,26-5,23(m,1H), 5,16-5,12(m,1H), 4,28-4,27(m,2H), 4,27-4,17(m,1H), 4,14-4,03(m,1H), 3,09(t,2H), 2,42(s,3H), 1,75-1,66(m,2H), 0,96(t,3H) |
| 431 | 2,83 | 2,92 | 8,02(d,1H), 7,52(d,1H), 5,95-5,85(m,1H), 5,26-5,23(m,1H), 5,16-5,12(m,1H), 4,29-4,26(m,2H), 4,26-4,17(m,1H), 4,14-4,05(m,1H), 3,12(q,2H), 2,42(s,3H), 1,34(t,3H) |
| 432 | 2,94 | 3,05 | 8,01(d,1H), 7,53(d,1H), 5,99-5,87(m,2H), 5,27-5,23(m,2H), 5,18-5,12(m,2H), 4,29-4,28(m,2H), 4,28-4,17(m,1H), 4,13-4,04(m,1H), 3,78(d,2H), 2,42(s,3H) |
| 433 | 2,02 | 1,99 | 7,98(d,1H), 7,53(d,1H), 4,27-4,18(m,1H), 4,10-4,01(m,1H), 3,25(s,3H), 2,42(s,3H) |
| 434 | 3,45 | 3,40 | 7,76(d,1H), 7,43(d,1H), 3,96(q,2H), 3,75(q,2H), 2,44(s,3H), 1,25(t,3H) |
| 435 | 3,07 | 3,04 | 7,73(d,1H), 7,43(d,1H), 3,95(q,2H), 3,26(s,3H), 2,44(s,3H) |
| 436 | 2,34 | 2,30 | 8,01(d,1H), 7,54(d,1H), 4,31-4,16(m,1H), 4,16-3,98(m,1H), 4,04(q,2H), 2,42(s,3H), 1,26(t,3H) |
| 437 | 2,00 | 1,98 | 7,99(d,1H), 7,54(d,1H), 4,31-4,19(m,1H), 4,10-3,98(m,1H), 3,26(s,3H), 2,42(s,3H) |
| 438 | 4,24 | 4,17 | 7,74(d,1H), 7,41(d,1H), 3,96(q,2H), 2,43(s,3H), 1,70(s,9H) |
| 439 | 3,83 | 3,80 | 7,75(d,1H), 7,41(d,1H), 4,43-4,31(m,1H), 3,96(q,2H), 2,43(s,3H), 1,47(d,6H) |
| 440 | 4,18 | 4,13 | 7,81(d,1H), 7,48-7,39(m,4H), 7,38-7,31(m,2H), 4,93(s,2H), 3,97(q,2H), 2,44(s,3H) |
| 441 | 3,02 | 2,98 | 7,98(d,1H), 7,52(d,1H), 4,31-4,18(m,1H), 4,15-4,00(m,1H), 2,42(s,3H), 1,71(s,9H) |
| 442 | 2,68 | 2,62 | 8,00(d,1H), 7,53(d,1H), 4,40-4,31(m,1H), 4,30-4,19(m,1H), 4,12-4,00(m,1H), 2,42(s,3H), 1,49-1,47(m,6H) |
| 443 | 3,08 | 3,00 | 8,05(d,1H), 7,55(d,1H), 7,44-7,40(m,2H), 7,36-7,33(m,3H), 5,76(s,2H), 4,31-4,19(m,1H), 4,18-3,98(m,1H), 2,43(s,3H) |
| 444 | 4,09 | 4,04 | 7,95(d,1H), 7,56(d,1H), 5,18(t,2H), 4,02(q,2H), 2,47(s,3H) |
| 445 | 3,75 | 3,72 | 8,23(d,1H), 8,18(d,2H), 8,02(d,2H), 7,71(d,1H), 4,39-4,27(m,1H), 4,08-3,96(m,1H), 2,48(s,3H) |
| 446 | 3,05 | 3,01 | 8,19(d,1H), 7,68(d,1H), 5,16(t,2H), 4,32-4,23(m,1H), 4,13-4,01(m,1H), 2,47(s,3H) |
| 447 | 3,51 | 3,49 | 7,96(d,1H), 7,58(d,1H), 3,99(q,2H), 3,93(s,3H), 2,49(s,3H) |
| 448 | 3,11 | 3,09 | 7,80(d,1H), 7,45(d,1H), 3,95(q,2H), 2,44(s,3H), 2,34(s,3H) |
| 449 | 4,30 | 4,25 | 7,84(d,1H), 7,45(d,1H), 3,94(q,2H), 2,44(s,3H), 1,31(s,9H) |
| 450 | 3,18 | 3,15 | 7,85(d,1H), 7,47(d,1H), 4,45(s,2H), 3,95(q,2H), 3,39(s,3H), 2,45(s,3H) |

(fortgesetzt)

| Bsp.-Nr. | logP[a] | logP[b] | $^1$H-NMR(D6-DMSO, 400MHz) δ ppm |
|---|---|---|---|
| 451 | 1,99 | 2,02 | 8,05(d,1H), 7,56(d,1H), 4,31-4,19(m,1H), 4,07-3,94(m,1H), 2,42(s,3H), 2,35(s,3H) |
| 452 | 3,10 | 3,09 | 8,08(d,1H), 7,56(d,1H), 4,31-4,20(m,1H), 4,10-3,98(m,1H), 2,42(s,3H), 1,32(s,9H) |
| 453 | 2,08 | 2,10 | 8,11(d,1H), 7,58(d,1H), 4,46(s,2H), 4,30-4,21(m,1H), 4,06-3,97(m,1H), 3,39(s,3H), 2,43(s,3H) |
| 454 | 2,85 | 2,83 | 8,69(s,1H), 7,83(d,1H), 7,47(d,1H), 3,96(q,2H), 2,44(s,3H) |
| 455 | 3,53 | 3,46 | 7,81(d,1H), 7,45(d,1H), 3,95(q,2H), 2,69(q,2H), 2,44(s,3H), 1,20(s,3H) |
| 456 | 3,62 | 3,55 | 7,80(d,1H), 7,44(d,1H), 3,95(q,2H), 2,43(s,3H), 2,09-2,02(m,1H), 1,11-1,04(m,2H), 1,02-0,98(m,2H) |
| 457 | 4,35 | 4,29 | 7,93-7,88(m,3H), 7,67-7,58(m,3H), 7,50(d,1H), 3,96(q,2H), 2,47(s,3H) |
| 458 | 4,85 | 4,78 | 7,92-7,89(m,3H), 7,68(d,2H), 7,50(d,1H), 3,95(q,2H), 2,47(s,3H) |
| 459 | 3,20 | 3,20 | 8,83-8,82(m,2H), 7,92(d,1H), 7,83-7,81(m,2H), 7,52(d,1H), 3,95(q,2H), 2,47(s,3H) |
| 460 | 3,94 | 3,92 | 7,82(d,1H), 7,45(d,1H), 3,95(q,2H), 2,67(t,2H), 2,44(s,3H), 1,72-1,61(m,2H), 0,98(t,3H) |
| 461 | 4,35 | 4,33 | 7,81(d,1H), 7,45(d,1H), 3,95(q,2H), 2,67(t,2H), 2,44(s,3H), 1,70-1,60(m,2H), 1,45-1,35(m,2H), 0,91(t,3H) |
| 462 | 3,93 | 3,84 | 7,82(d,1H), 7,45(d,1H), 3,95(q,2H), 3,05-2,95(m,1H), 2,44(s,3H), 1,25(d,6H) |
| 463 | 3,53 | 3,52 | 7,85(d,1H), 7,47(d,1H), 4,48(s,2H), 3,95(q,2H), 3,59(q,2H), 2,44(s,3H), 1,16(t,3H) |
| 464 | 4,44 | 4,34 | 7,92(d,1H), 7,75(d,1H), 7,70-7,64(m,2H), 7,54-7,49(m,2H), 3,95(q,2H), 2,47(s,3H) |
| 465 | 2,50 | 2,48 | 8,08(d,1H), 7,55(d,1H), 4,28-4,19(m,1H), 4,06-3,94(m,1H), 2,42(s,3H), 2,09-2,03(m,1H), 1,11-1,05(m,2H), 1,05-0,99(m,2H) |
| 466 | 4,35 | 4,27 | 7,91(d,1H), 7,84-7,81(m,2H), 7,49(d,1H), 7,16-7,13(m,2H), 3,96(q,2H), 3,86(s,3H), 2,46(s,3H) |
| 467 | 4,23 | 4,11 | 8,43-8,40(m,2H), 8,16-8,13(m,2H), 7,93(d,1H), 7,52(d,1H), 3,95(q,2H), 2,48(s,3H) |
| 468 | 4,13 | 4,09 | 7,97-7,86(m,1H), 7,90(d,1H), 7,80-7,79(m,1H), 7,49(d,1H), 7,30-7,28(m,1H), 3,95(q,2H), 2,46(s,3H) |
| 469 | 3,74 | 3,65 | 8,06(s,1H), 7,89(d,1H), 7,49(d,1H), 7,34-7,33(m,1H), 6,81-6,80(m,1H), 3,95(q,2H), 2,46(s,3H) |
| 470 | 2,77 | 2,63 | 8,07(d,1H), 7,56(d,1H), 4,32-4,20(m,1H), 4,09-3,95(m,1H), 2,65(t,2H), 2,42(s,3H), 1,75-1,65(m,2H), 0,99(t,3H) |
| 471 | 3,17 | 3,11 | 8,06(d,1H), 7,56(d,1H), 4,31-4,19(m,1H), 4,10-3,98(m,1H), 2,67(t,2H), 2,42(s,3H), 1,70-1,60(m,2H), 1,45-1,35(m,2H), 0,92(t,3H) |
| 472 | 2,75 | 2,71 | 8,07(d,1H), 7,56(d,1H), 4,31-4,20(m,1H), 4,08-3,98(m,1H), 3,07-2,98(m,1H), 2,42(s,3H), 1,26(d,6H) |
| 473 | 2,45 | 2,40 | 8,10(d,1H), 7,58(d,1H), 4,49(s,2H), 4,31-4,20(m,1H), 4,09-3,95(m,1H), 3,60(q,2H), 2,43(s,3H), 1,17(t,3H) |
| 474 | 3,21 | 3,18 | 8,17(d,1H), 7,85-7,82(m,2H), 7,60(d,1H), 7,16-7,13(m,2H), 4,35-4,21(m,1H), 4,10-3,98(m,1H), 3,86(s,3H), 2,45(s,3H) |
| 475 | 3,13 | 3,10 | 8,44-8,40(m,2H), 8,20-8,13(m,3H), 7,63(d,1H), 4,38-4,22(m,1H), 4,10-3,98(m,1H), 2,45(s,3H) |
| 476 | 2,96 | 2,93 | 8,15(d,1H), 7,98-7,97(m,1H), 7,80-7,79(m,1H), 7,60(d,1H), 7,31-7,29(m,1H), 4,35-4,22(m,1H), 4,10-3,99(m,1H), 2,44(s,3H) |
| 477 | 2,66 | 2,62 | 8,15(d,1H), 8,07-8,06(m,1H), 7,60(d,1H), 7,35-7,34(m,1H), 6,81-6,80(m,1H), 4,33-4,22(m,1H), 4,09-3,92(m,1H), 2,44(s,3H) |

(fortgesetzt)

| Bsp.-Nr. | logP[a] | logP[b] | $^1$H-NMR(D6-DMSO, 400MHz) δ ppm |
|---|---|---|---|
| 478 | 3,09 | 3,06 | 8,08(d,1H), 7,56(d,1H), 7,39(d,4H), 7,35-7,29(m,1H), 4,30-4,19(m,1H), 4,10(s,2H), 4,09-3,95(m,1H), 2,42(s,3H) |
| 479 | 3,83 | 3,82 | 7,81(d,1H), 7,46(d,1H), 3,94(q,2H), 3,77(t,2H), 2,82(t,2H), 2,44(s,3H), 2,15-2,08(m,2H) |
| 480 | 2,76 | 2,76 | 8,07(d,1H), 7,56(d,1H), 4,31-4,19(m,1H), 4,08-3,95(m,1H), 3,78(t,2H), 2,82(t,2H), 2,42(s,3H), 2,18-2,09(m,2H) |
| 481 | 1,80 | 1,79 | 8,71(s,1H), 8,09(d,1H), 7,58(d,1H), 4,31-4,19(m,1H), 4,09-3,92(m,1H), 2,42(s,3H) |
| 482 | 3,83 | 3,78 | 7,60(s,1H), 7,28(s,1H), 3,93(q,2H), 2,38(s,3H), 2,18(s,3H), 2,09-2,00(m,1H), 1,11-1,02(m,2H), 1,02-0,92(m,2H) |
| 483 | 4,52 | 4,47 | 7,63(s,1H), 7,29(s,1H), 3,93(q,2H), 2,39(s,3H), 2,18(s,3H), 1,31(s,9H) |
| 484 | 2,66 | 2,59 | 7,87(s,1H), 7,39(s,1H), 4,28-4,11(m,1H), 4,02-3,89(m,1H), 2,37(s,3H), 2,29(s,3H), 2,09-1,99(m,1H), 1,10-1,01(m,2H), 1,00-0,95(m,2H) |
| 485 | 3,27 | 3,20 | 7,90(s,1H), 7,40(s,1H), 4,25-4,11(m,1H), 4,05-3,92(m,1H), 2,38(s,3H), 2,30(s,3H), 1,32(s,9H) |
| 486 | 2,84 | 2,79 | 7,72(d,1H), 7,36(d,1H), 6,77(d,1H), 6,73-6,72(m,1H), 3,94(q,2H), 3,60(q,2H), 2,41(s,3H), 1,22(t,3H) |
| 487 | 1,83 | 1,81 | 7,99(d,1H), 7,48(d,1H), 6,83-6,80(m,2H), 4,22-4,11(m,1H), 4,11-4,02(m,1H), 3,61(q,2H), 2,41(s,3H), 1,23(t,3H) |
| 488 | 3,69 | 3,63 | 7,70(d,1H), 7,35(d,1H), 6,74(d,1H), 6,68(t,1H), 3,99-3,90(m,2H), 2,42(s,3H), 1,49(s,9H) |
| 489 | 2,50 | 2,47 | 8,00(d,1H), 7,47(d,1H), 6,79-6,77(m,2H), 4,17-4,07(m,2H), 2,40(s,3H), 1,50(s,9H) |
| 490 | 3,66 | 4,29 | 7,70(d,1H), 7,37(d,1H), 7,11(d,1H), 6,50(d,1H), 3,95(q,2H),3,65(q,2H), 2,41(s,3H) |
| 491 | 2,46 | 3,10 | 7,93(d,1H), 7,49(d,1H), 7,18(d,1H), 6,56(d,1H), 4,28-3,92(m,2H), 3,68(q,2H), 2,43(s,3H) |
| 492 | 3,49 | 3,43 | 7,78(d,1H), 7,45(d,1H), 4,60(s,2H), 3,96(q,2H), 3,37(s,3H), 3,09-3,05(m,1H), 2,46(s,3H), 1,20-1,15(m,2H), 1,13-1,09(m,2H) |
| 493 | 4,44 | 4,35 | 7,74(d,1H), 7,43(d,1H), 3,94(q,2H), 3,07-3,05(m,1H), 2,71(d,2H), 2,45(s,3H), 2,19-2,15(m,1H), 1,15-1,14(m,4H), 1,00(d,6H) |
| 494 | 4,1 | 4,02 | 7,74(d,1H), 7,43(d,1H), 3,95(q,2H), 3,36-3,29(m,1H), 3,13-3,10(m,1H), 2,46(s,3H), 1,30(s,6H), 1,17-1,15(m,4H) |
| 495 | 4,52 | 4,43 | 7,73(d,1H), 7,43(d,1H), 3,95(q,2H), 3,10-3,04(m,1H), 2,83(t,2H), 2,45(s,3H), 1,74-1,67(m,2H), 1,46-1,37(m,2H), 1,17-1,12(m,4H), 0,93(t,3H) |
| 496 | 5,00 | 4,88 | 7,74(d,1H), 7,42(d,1H), 3,95(q,2H), 3,12-3,07(m,1H), 3,05-2,99(m,1H), 2,45(s,3H), 2,08-2,02(m,2H), 1,82-1,75(m,2H), 1,71-1,62(m,1H), 1,45-1,30(m,4H), 1,29-1,20(m,1H), 1,17-1,16(m,4H) |
| 497 | 4,68 | 4,57 | 7,74(d,1H), 7,42(d,1H), 3,95(q,2H), 3,49-3,3,39(m,1H), 3,11-3,02(m,1H), 2,45(s,3H), 2,15-2,05(m,2H), 1,89-1,75(m,2H), 1,73-1,60(m,4H), 1,20-1,11(m,4H) |
| 498 | 4,24 | 4,14 | 7,75(d,1H), 7,42(d,1H), 5,20-5,10(m,1H), 3,96(q,2H), 2,45(s,3H), 2,28-2,18(m,1H), 1,59(d,6H), 1,10-1,01(m,2H), 1,00-0,95(m,2H) |
| 499 | 4,46 | 4,39 | 7,75(d,1H), 7,43(d,1H), 3,95(q,2H), 3,21-3,164(m,1H), 3,12-3,06(m,1H), 2,46(s,3H), 1,85-1,78(m,1H), 1,64-1,57(m,1H), 1,27(d,3H), 1,20-1,11(m,4H), 0,92(t,3H) |
| 500 | 3,82 | 3,77 | 7,78(d,1H), 7,44(d,1H), 4,62(s,2H), 3,96(q,2H), 3,58(q,2H), 3,11-3,04(m,1H), 2,45(s,3H), 1,23-1,19(m,2H), 1,17(t,3H), 1,13-1,08(m,2H) |

(fortgesetzt)

| Bsp.-Nr. | logP[a] | logP[b] | $^1$H-NMR(D6-DMSO, 400MHz) δ ppm |
|---|---|---|---|
| 501 | 4,00 | 3,94 | |
| 502 | 3,69 | 3,65 | 7,73(d,1H), 7,43(d,1H), 3,94(q,2H), 3,10-3,04(m,1H), 2,86(q,2H), 2,46(s,3H), 1,26(t,3H), 1,17-1,08(m,4H) |
| 503 | 4,09 | 4,04 | 7,73(d,1H), 7,43(d,1H), 3,95(q,2H), 3,10-3,04(m,1H), 2,83-2,78(m,2H), 2,45(s,3H), 1,80-1,70(m,2H), 1,17-1,11(m,4H), 1,00(t,3H) |
| 504 | 3,89 | 3,82 | 7,71(d,1H), 7,41(d,1H), 3,94(q,2H), 3,20-3,10(m,1H), 2,44(s,3H), 2,21-2,11(m,1H), 1,21-1,12(m,4H), 1,11-1,03(m,2H), 0,98-0,89(m,2H) |
| 505 | 4,32 | 4,25 | 7,76(d,1H), 7,43(d,1H), 3,95(q,2H), 3,90-3,80(m,1H), 3,05-2,98(m,1H), 2,46(s,3H), 2,40-2,30(m,4H), 2,15-2,01(m,1H), 1,95-1,82(m,1H), 1,11-1,09(m,4H) |
| 506 | 4,23 | 4,18 | 7,86(d,1H), 7,65-7,61(m,1H), 7,49-7,45(m,2H), 7,26(d,1H), 7,14-7,10(m,1H), 3,98(q,2H), 3,91(s,3H), 3,16-3,12(m,1H), 2,47(s,3H), 0,84-0,82(m,2H), 0,61-0,51(m,2H) |
| 507 | 4,24 | 4,17 | 7,88(d,1H), 7,73-7,70(m,1H), 7,23(d,1H), 4,17(q,2H), 3,94(q,2H), 3,94(s,3H), 1,34(t,3H) |
| 508 | 2,49 | 2,43 | 8,00(d,1H), 7,51(d,1H), 4,50(q,2H), 4,30-4,15(m,1H), 4,15-4,03(m,1H), 4,03(s,3H), 2,42(s,3H) |
| 509 | 2,49 | 2,43 | 8,00(d,1H), 7,51(d,1H), 4,50(q,2H), 4,30-4,15(m,1H), 4,15-4,03(m,1H), 4,03(s,3H), 2,42(s,3H) |

Die Bestimmung der Drehwerte erfolgte an einem Perkin Elmer 341, Seriennummer 9123, bei einer Wellenlänge von 589 nm und einer Temperatur von 20 °C, nach der nachfolgender Formel:

$$\left(spezifischerDrehwert\alpha\right)_D^{°C} = \frac{Drehwinkel\alpha * L\ sungsvolumen(ml)}{Küverttenlänge(dm) * Einwaage(g)}$$

Die unten stehenden spezifischen Drehwerte sind als Durchschnitt aus 5 unterschiedlichen Messungen zu verstehen:

| | |
|---|---|
| 379 | -103,2 in $CHCl_3$ (c=0,009) |
| 380 | 103,9 in $CHCl_3$ (c=0,009) |
| 508 | 86,7 in $CHCl_3$ (c=0,009) |
| 509 | -87,6 in $CHCl_3$ (c=0,009) |

## Anwendungsbeispiele

### Boophilus microplus -Injektionstest (BOOPMI Inj)

Lösungsmittel: Dimethylsulfoxid

Zur Herstellung einer zweckmäßigen Wirkstoffzu bereitung vermischt man 10 mg Wirkstoff mit 0,5 ml Lösungsmittel und verdünnt das Konzentrat mit Lösungsmittel auf die gewünschte Konzentration.

1μl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt.

Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: 5, 36, 37, 38, 45, 60, 92, 99, 101, 102, 103, 104, 105, 106, 115, 116, 117, 119, 157, 160, 215, 228, 231, 241, 245, 253, 257, 270, 295, 301, 302, 305, 311, 321, 381, 384, 385, 390, 392, 393, 406, 411, 415, 423, 439, 442, 504

**Lucilia cuprina - Test (LUCICU)**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 80% bei einer Aufwandmenge von 100ppm: 4

**Myzus persicae - Sprühtest (MYZUPE)**

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 500g/ha: 311, 320, 423

Bei diesem Test zeigen z. B. die folgenden Verbindungen der Herstellungsbeispiele Wirkung von 90% bei einer Aufwandmenge von 100g/ha: 411

**Phaedon cochleariae - Sprühtest (PHAECO)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden;

0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha:2, 8, 10, 11, 93, 106, 109, 176, 179, 183, 219, 223, 225, 303, 311, 320, 321, 322, 328, 329, 330, 349, 351, 376, 376, 390, 392, 392, 411, 447, 490, 504, 506

Bei diesem Test zeigen z. B. die folgenden Verbindungen der Herstellungsbeispiele Wirkung von 83% bei einer Aufwandmenge von 500g/ha:1, 94, 95, 154, 209, 272, 331, 332, 336, 350, 355, 394,435, 437, 502

Bei diesem Test zeigen z. B. die folgenden Verbindungen der Herstellungsbeispiele Wirkung von 83% bei einer Aufwandmenge von 100g/ha: 98

**Spodoptera frugiperda - Sprühtest (SPODFR)**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: 411, 504

**Tetranychus urticae - Sprühtest, OP-resistent (TETRUR)**

| | | |
|---|---|---|
| Lösungsmittel: | 78,0 | GewichtsteileAceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: 5, 9, 11, 12, 15, 17, 18, 19, 21, 29, 32, 36, 37, 38, 40, 43, 44, 45, 48, 50, 59, 61, 63, 64, 65, 66, 68, 74, 75,76, 77, 78, 79, 80, 82, 83, 84, 85, 86, 87, 88, 89, 90, 91, 92, 98, 100, 101, 102, 103, 104, 105, 106, 114, 115, 120, 121, 122, 123, 127, 132, 140, 146, 149, 150, 151, 153, 154, 155, 157, 158, 159, 160, 165, 170, 171, 172, 173, 180, 182, 183, 184, 189, 193, 194, 195, 196, 197, 198, 199, 200, 201, 202, 203, 204, 205, 206, 207, 208, 209, 210, 211, 215, 216, 221, 225, 226, 230, 231, 233, 234, 235, 237, 238, 239, 240, 241,242, 243, 244, 245, 246, 247, 248, 250, 251, 252, 253, 255, 257, 259, 260, 261, 262, 264, 265, 266, 267, 268, 269, 270, 271, 272, 273, 274, 275, 276, 277, 278, 279, 280, 281, 282, 283, 284, 285, 286, 287, 288, 289, 290, 291, 292, 294, 295, 298, 300, 301, 302, 304, 305, 306, 307, 310, 311, 314, 324, 325, 343, 344, 345, 347, 350, 351, 353, 356, 358, 359, 361, 363, 365, 366, 367, 368, 369, 370, 371, 372, 375, 376, 377, 380, 382, 383, 384, 386, 390, 391, 393, 394, 395, 396, 397, 398, 400, 401, 403, 404, 408, 410, 411, 412, 414, 417, 419, 421, 422, 425, 426, 428, 433, 434, 435, 436, 438, 439, 440, 441, 442, 444, 447, 448, 450, 452, 453, 455, 456, 458, 460, 462, 464, 466, 467, 468, 470, 472, 475, 478, 479, 480, 482, 483, 484, 485, 486, 488, 490, 491, 492, 493, 494, 495, 498, 500, 503, 504, 505, 508

Bei diesem Test zeigen z. B. die folgenden Verbindungen der Herstellungsbeispiele eine Wirkung von 90% bei einer Aufwandmenge von 500g/ha: 3, 4, 10, 14, 16, 20, 22, 23, 24, 25, 28, 30, 33, 34, 35, 39, 41, 42, 46, 49, 51, 52, 54, 55, 57, 58, 60, 67, 70, 71, 72, 73, 93, 97, 99, 112, 113, 116, 117, 119, 125, 126, 128, 129, 130, 131, 133, 134, 136, 137, 141, 143, 144, 148, 152, 162, 164, 166, 169, 176, 178, 179, 185, 187, 190, 191, 192, 212, 213, 217, 218, 219, 220, 222, 223, 224, 228, 229, 232, 236, 249, 254, 256, 258, 293, 296, 302, 312, 313, 315, 316, 317, 318, 326, 327, 328, 329, 330, 331, 332, 335,336, 337, 338, 339, 340, 341, 342, 346, 348, 349, 352, 355, 357, 360, 362, 364, 374, 378, 379, 381, 387, 388, 389, 392, 399, 402, 405, 406, 407, 409, 413, 415, 416, 418, 420, 423, 424, 427, 429, 430, 431, 437, 446, 449, 451, 454, 457, 459, 461, 463, 465, 469, 474, 487, 489, 496, 497, 499, 501, 502, 506, 507

Bei diesem Test zeigen z. B. die folgenden Verbindungen der Herstellungsbeispiele eine Wirkung von 80% bei einer Aufwandmenge von 500g/ha: 6

Bei diesem Test zeigen z. B. die folgenden Verbindungen der Herstellungsbeispiele eine Wirkung von 100% bei einer Aufwandmenge von 100g/ha: 62, 69, 175, 177,263

Bei diesem Test zeigen z. B. die folgenden Verbindungen der Herstellungsbeispiele eine Wirkung von 90% bei einer Aufwandmenge von 100g/ha: 56, 124, 188, 476

Bei diesem Test zeigen z. B. die folgenden Verbindungen der Herstellungsbeispiele eine Wirkung von 90% bei einer Aufwandmenge von 20g/ha: 334

**Meloidogyne incognita- Test (MELGIN)**

Lösungsmittel: 125,0 Gewichtsteile Aceton

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100% bei einer Aufwandmenge von 20ppm : 7, 40, 61, 102, 103, 110, 111, 116, 117, 118, 145, 160, 202, 304, 305, 415, 446, 491

Bei diesem Test zeigen z. B. die folgenden Verbindungen der Herstellungsbeispiele Wirkung von 90% bei einer Aufwandmenge von 20ppm: 4, 39, 99, 104, 105, 106, 107, 108, 115, 135, 138, 139, 140, 142, 146, 150, 213, 214, 323, 331, 332, 444, 502

**Patentansprüche**

1. Verbindungen der Formel (I)

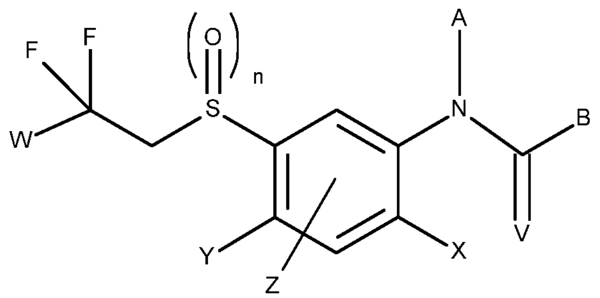

(I)

in welcher

A und B gemeinsam mit den Atomen, an die sie gebunden sind, für eine Substruktur stehen, die ausgewählt ist aus der Gruppe, bestehend aus

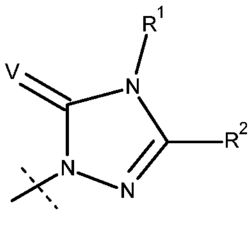
(I-A)

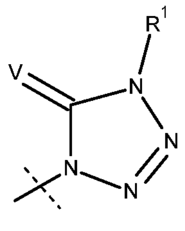
(I-B)

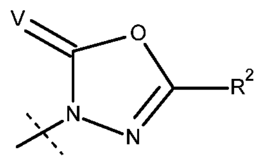
(I-C)

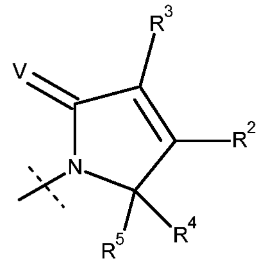
(I-D)

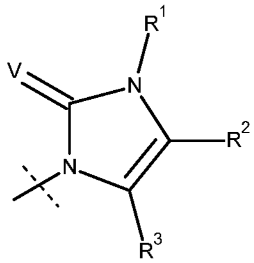
(I-E)

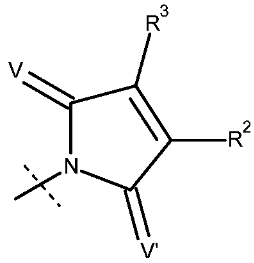
(I-F)

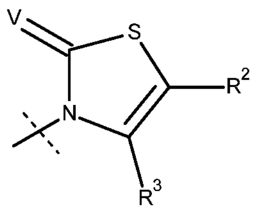
(I-G)

wobei
$R^1$ für Wasserstoff, Cyano oder Nitro steht; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl steht, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, steht; oder
für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl steht, wobei die vorgenannten Reste jeweils gesättigt oder ungesättigt und/oder gegebenenfalls substituiert sein können oder für Carbonyl oder Carboxyl steht; oder
für gegebenenfalls substituiertes Phenyl oder gegebenenfalls substituiertes Hetaryl steht; oder
für Alkoxy, Halogenalkoxy, Cycloalkyloxy, Aryloxy, Arylalkyloxy oder Carbonyloxy steht, wobei die vorgenannten Reste gegebenenfalls substituiert sein können, oder für Hydroxy steht; oder
für Alkylamino, Halogenalkylamino, Dihalogenalkylamino, Dialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino steht, wobei die vorgenannten Reste jerweils gesättigt oder ungesättigt und/oder gegebenenfalls substituiert sein können, oder Amino steht; oder
für Alkylsulfanyl, Alkylsulfinyl, Alkylsulfonyl, Halogenalkylsulfanyl, Halogenalkylsulfinyl, Halogenalkylsulfanyl, Cycloalkylsulfanyl, Cycloalkylsulfinyl, Cycloalkylsulfonyl, Cycloalkylalkylsulfanyl, Cycloalkylalkylsulfinyl, Cycloalkylalkylsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl steht, wobei die vorgenannten Reste jeweils gesättigt oder ungesättigt und/oder gegebenenfalls substituiert sein könnnen, oder für Sulfanyl steht; und

$R^2$, $R^3$, $R^4$, und $R^5$, jeweils unabhängig voneinander,
für Wasserstoff, Cyano, Halogen oder Nitro stehen; oder
für Alkyl, Cycloalkylalkyl, Halogenalkyl, Cyanoalkyl, Hydroxyalkyl, Alkoxyalkyl, Aminoalkyl, Alkoxycarbonylalkyl, Alkylsulfanylalkyl, Alkylsulfinylalkyl, Alkylsulfonylalkyl, Halogenalkylsulfanylalkyl, Halogenalkylsulfinylalkyl, Halogenalkylsulfonylalkyl, Alkoxyalkylsulfanylalkyl, Alkoxyalkylsulfinylalkyl, Alkoxyalkylsulfonylalkyl, Phenylalkyl, Phenoxyalkyl, Phenylsulfanylalkyl, Phenylsulfinylalkyl, Phenylsulfonylalkyl, Hetarylalkyl, Hetaryloxyalkyl, Hetarylthioalkyl stehen, wobei die vorgenannten Reste jeweils gesättigt oder ungesättigt und/oder gegebenenfalls substituiert sein können; oder
für gegebenenfalls substituiertes gesättigtes oder ungesättigtes Cycloalkyl, welches gegebenenfalls durch ein oder mehrere Heteroatome unterbrochen sein kann, stehen; oder
für Alkylcarbonyl, Halogenalkylcarbonyl, Hydroxyalkylcarbonyl, Alkoxyalkylcarbonyl, Phenylcarbonyl, Hetarylcarbonyl, Alkoxycarbonyl, Halogenalkoxycarbonyl, Aryloxycarbonyl, Aminocarbonyl, Alkylaminocarbonyl, Dialkylaminocarbonyl, Cycloalkylaminocarbonyl, Dicycloalkylaminocarbonyl, Cycloalkyl(alkyl)aminocarbonyl, Arylaminocarbonyl, Diarylaminocarbonyl, Alkyl(aryl)aminocarbonyl, Cycloalkyl(aryl)aminocarbonyl, Alkylaminothiocarbonyl, Dialkylaminothiocarbonyl oder Aminothiocarbonyl stehen, wobei die vorgenannten Reste jeweils gesättigt oder ungesättigt und/oder gegebenenfalls substituiert sein können oder für Carbonyl oder Carboxyl stehen; oder
für gegebenenfalls substituiertes Phenyl oder gegebenenfalls substituiertes Hetaryl stehen; oder
für Alkoxy, Halogenalkoxy, Alkoxyalkoxy, Aryloxy, Arylalkyloxy, Cycloalkyloxy, Cycloalkylalkyloxy oder Carbonyloxy stehen, wobei die vorgenannten Reste gesättigt oder ungesättigt und/oder gegebenenfalls substiuiert sein können, oder für Hydroxy stehen; oder
für Alkylamino, Dialkylamino, Halogenalkylamino, Dihalogendialkylamino, Cycloalkylamino, Dicycloalkylamino, Cycloalkyl(alkyl)amino, Arylamino, Diarylamino, Hetarylamino, Dihetarylamino, Alkyl(aryl)amino, Cycloalkyl(aryl)amino, Alkylcarbonylamino, Arylcarbonylamino, Alkoxycarbonylamino, Aryloxycarbonylamino, Alkylcarbamoylamino, Arylcarbamoylamino, Alkylsulfonylamino, oder Arylsulfonylamino stehen, wobei die vorgenannten Reste jeweils gesättigt oder ungesättigt und/oder gegebenenfalls substituiert sein können, oder für Amino stehen; oder
für Alkylsulfanyl, Alkylsulfinyl, Alkylsulfonyl, Halogenalkylsulfanyl, Halogenalkylsulfinyl, Halogenalkylsulfonyl, Cycloalkylsulfanyl, Cycloalkylsulfinyl, Cycloalkylsulfonyl, Cycloalkylalkylsulfanyl, Cycloalkylalkylsulfinyl, Cycloalkylalyklsulfonyl, Arylsulfanyl, Arylsulfinyl, Arylsulfonyl, Arylalkylsulfanyl, Arylalkylsulfinyl, Arylalkylsulfonyl, Aminosulfonyl, Alkylaminosulfonyl, Dialkylaminosulfonyl oder Arylaminosulfonyl stehen, wobei die vorgenannten Reste jeweils gesättigt oder ungesättigt und/oder gegebenenfalls substituiert sein könnnen, oder für Sulfanyl stehen; oder
$R^4$ und $R^5$ gemeinsam mit dem Atom, an das sie gebunden sind, einen gegebenenfalls substituierten und gegebenenfalls durch ein oder mehrere Heteroatome, welche unabhängig ausgewählt werden, aus der Gruppe, bestehend aus O, S und N, unterbrochenen gesättigten oder ungesättigten dreibis achtgliedrigen Ring bilden können; oder
oder $R^2$ für einen gesättigten oder ungesättigten gegebenenfalls durch ein oder mehrere Heteroatome, welche jeweils ausgewählt werden, aus der Gruppe, bestehend aus O, S und N, unterbrochenen Cyclus, der gegebenenfalls substituiert sein kann, steht;
W für Wasserstoff oder Halogen steht;

für den Fall, dass eine Substruktur gemäß einer der Formeln (I-B) bis (I-G) vorliegt,

V und V' jeweils unabhängig voneinander für Sauerstoff, Schwefel oder einen gegebenenfalls substituierten Stickstoff stehen;

und für den Fall, dass eine Substruktur gemäß Formel (I-A) vorliegt,

V für Sauerstoff oder Schwefel steht;
X, Y und Z, jeweils unabhängig voneinander,

für Wasserstoff, Halogen, Hydroxy, Amino, Cyano, Nitro, OCN, SCN, $SF_5$, stehen; oder
für Trialkylsilyl, Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy, Alkylcarbonyl, Halogenalkylcarbonyl, Carboxyl, Alkylcarbonyloxy, Alkoxycarbonyl, Halogenalkoxycarbonyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl oder Dialkylaminothiocarbonyl stehen, wobei alle vorgenannten Reste gegebenenfalls substituiert sein können; oder

für Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy, Hetarylthio, Hetarylsulfinyl oder Hetarylsulfonyl stehen, wobei alle vorgenannten Reste gegebenenfalls substituiert sein können; oder

für Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl oder Cycloalkenyl stehen, wobei alle vorgenannten Reste jeweils gegebenenfalls substituiert sein können; oder

für NR'R" stehen,

wobei R' und R" unabhängig voneinander

für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl oder Alkoxycarbonyl stehen; oder

R' und R" gemeinsam mit dem N-Atom, an das sie gebunden sind, einen gegebenenfalls substituierten und gegebenenfalls durch ein oder mehrere Heteroatome, welche unabhängig ausgewählt werden aus der Gruppe, bestehend aus O, S und N, unterbrochenen gesättigten oder ungesättigten fünf- bis achtgliedrigen Ring bilden können; oder

für einen 3- bis 6-gliedrigen gesättigten, teilgesättigten oder aromatischen Ring, der gegebenenfalls ein bis drei Heteroatome, welche unabhängig ausgewählt werden aus der Gruppe, bestehend aus O, S und N, enthalten kann und der gegebenenfalls substituiert sein kann, stehen;

oder X und Z, oder Y und Z, mit den C-Atomen, an die sie gebunden sind, einen 5 oder 6-gliedrigen Ring ausbilden, der gegebenenfalls substituiert ist und gegebenenfalls durch ein oder mehrere Heteroatome, welche unabhängig ausgewählt werden, aus der Gruppe, bestehend aus O, S, N und CO, unterbrochen ist; und

n für die Zahl 0 oder 1 steht.

**2.** Verbindung nach Anspruch 1, in welcher

A und B gemeinsam mit den Atomen, an die sie gebunden sind, für eine Substruktur stehen, die ausgewählt ist aus der Gruppe, bestehend aus I-A bis I-G,

$R^1$ für Wasserstoff, Cyano oder Nitro steht; oder

für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl$(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder

für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl$(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$, $R^3$, $R^4$, und $R^5$, jeweils unabhängig voneinander,

für Wasserstoff, Cyano, Halogen oder Nitro stehen; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder

für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl$(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl,

($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können; oder

oder $R^2$ für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring, steht;

W für Wasserstoff oder Halogen steht;

für den Fall, dass eine Substruktur gemäß einer der Formeln (I-B) bis (I-G) vorliegt,

V und V' jeweils unabhängig voneinander für Sauerstoff, Schwefel oder einen gegebenenfalls substituierten Stickstoff stehen;

und für den Fall, dass eine Substruktur gemäß Formel (I-A) vorliegt,

V für Sauerstoff oder Schwefel steht;

X, Y und Z, jeweils unabhängig voneinander, die zuvor genannten Bedeutungen haben;

und

n für die Zahl 0 oder 1 steht.

**3.** Verbindung nach Anspruch 2, in welcher

X, Y und Z, jeweils unabhängig voneinander,

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls

einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für $(C_3\text{-}C_6)$Cycloalkyl$(C_1\text{-}C_3)$alkyl, $(C_3\text{-}C_6)$Cycloalkyloxy, $(C_3\text{-}C_6)$Cycloalkyl$(C_1\text{-}C_3)$alkoxy, $(C_3\text{-}C_6)$Cycloalkylthio, $(C_3\text{-}C_6)$Cycloalkyl$(C_1\text{-}C_3)$alkylthio, $(C_3\text{-}C_6)$Cycloalkylsulfinyl, $(C_3\text{-}C_6)$Cycloalkyl$(C_1\text{-}C_3)$alkylsulfinyl, $(C_3\text{-}C_6)$Cycloalkylsulfonyl, $(C_3\text{-}C_6)$Cycloalkyl$(C_1\text{-}C_3)$alkylsulfonyl oder $(C_3\text{-}C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R" unabhängig voneinander

für Wasserstoff, Cyano, $(C_1\text{-}C_6)$Alkyl, Halogen$(C_1\text{-}C_6)$alkyl, Cyano$(C_1\text{-}C_6)$alkyl, Hydroxy$(C_1\text{-}C_6)$alkyl, $(C_1\text{-}C_6)$Alkoxy$(C_1\text{-}C_6)$alkyl, $(C_1\text{-}C_6)$Alkylthio$(C_1\text{-}C_6)$alkyl, $(C_2\text{-}C_6)$Alkenyl, Halogen$(C_2\text{-}C_6)$alkenyl, Cyano$(C_2\text{-}C_6)$alkenyl, $(C_2\text{-}C_6)$Alkinyl, Halogen$(C_2\text{-}C_6)$alkinyl, Cyano$(C_2\text{-}C_6)$alkinyl, Acyl oder $(C_1\text{-}C_6)$Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3\text{-}C_6)$Cycloalkyl, Oxetan, Oxolan, Oxan, $(C_3\text{-}C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

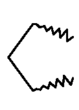 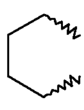 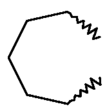 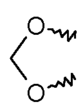 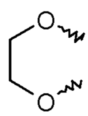 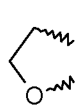 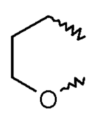

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

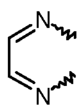 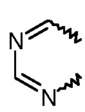 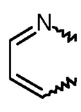 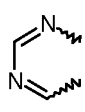 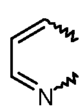 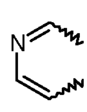

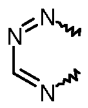
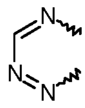
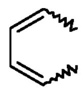
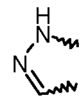
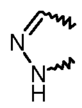
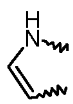
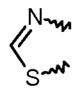
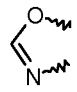
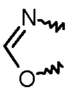
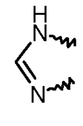
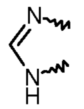
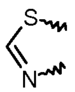
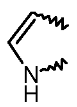
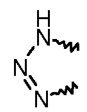
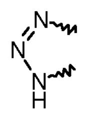
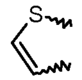
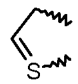
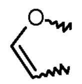
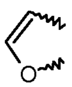

**4.** Verbindung nach einem der Ansprüche 1 bis 3, in welcher

A und B gemeinsam mit den Atomen, an die sie gebunden sind, für eine Substruktur der Formel (I-A) stehen

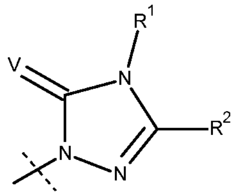

(I-A)

wobei
$R^1$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder

für gegebenenfalls substituiertes Phenyl oder gegebenenfalls substituiertes Hetaryl steht; oder

für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy steht, wobei die vorgenannten Reste jeweils gegebenenfalls substiuiert sein können, oder für Hydroxy steht; oder für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und

$R^2$ für Wasserstoff, Cyano, Halogen oder Nitro steht; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl,

$(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring steht,

W für Wasserstoff oder Halogen steht;

V für Sauerstoff oder Schwefel steht;

X, Y und Z, jeweils unabhängig voneinander, für Wasserstoff, Halogen, Hydroxy, Amino, Cyano, Nitro, OCN, SCN, $SF_5$, stehen; oder

für Tri$(C_1-C_6)$alkylsilyl, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxycarbonyl$(C_1-C_6)$alkyl, $(C_1-C_6)$Alkoxy$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, $(C_1-C_6)$Alkoxy, Halogen$(C_1-C_6)$alkoxy, Cyano$(C_1-C_6)$alkoxy, Hydroxycarbonyl$(C_1-C_6)$alkoxy, $(C_1-C_6)$Alkoxycarbonyl$(C_1-C_6)$alkoxy, $(C_1-C_6)$Alkoxy$(C_1-C_6)$alkoxy, $(C_1-C_6)$Alkylhydroxyimino, $(C_1-C_6)$Alkoxyimino, $(C_1-C_6)$Alkyl$(C_1-C_6)$alkoxyimino, Halogen$(C_1-C_6)$alkyl$(C_1-C_6)$alkoxyimino, $(C_1-C_6)$Alkylthio, Halogen$(C_1-C_6)$alkylthio, $(C_1-C_6)$Alkoxy$(C_1-C_6)$alkylthio, $(C_1-C_6)$Alkylthio$(C_1-C_6)$alkyl, $(C_1-C_6)$Alkylsulfinyl, Halogen$(C_1-C_6)$alkylsulfinyl, $(C_1-C_6)$Alkoxy$(C_1-C_6)$alkylsulfinyl, $(C_1-C_6)$Alkylsulfinyl$(C_1-C_6)$alkyl, $(C_1-C_6)$Alkylsulfonyl, Halogen$(C_1-C_6)$alkylsulfonyl, $(C_1-C_6)$Alkoxy$(C_1-C_6)$alkylsulfonyl, $(C_1-C_6)$Alkylsulfonyl$(C_1-C_6)$alkyl, $(C_1-C_6)$Alkylsulfonyloxy, $(C_1-C_6)$Alkylcarbonyl, Halogen$(C_1-C_6)$alkylcarbonyl, Carboxyl, $(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cyclo$(C_1-C_6)$alkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder

für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R'' stehen,

wobei R' und R'' unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder

R' und R'' gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen; oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituiert sind,

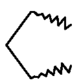 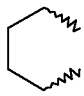 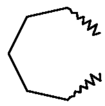 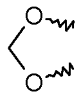 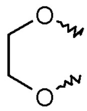 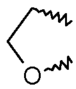 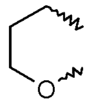

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

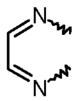 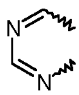 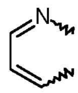 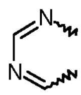 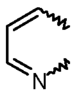 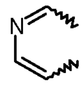

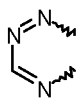 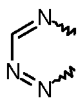 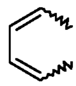

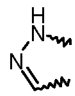 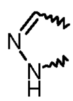 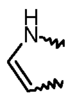

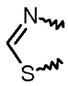 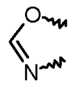 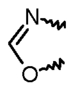

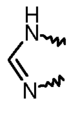 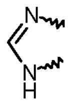 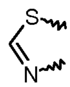

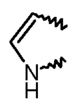 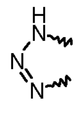 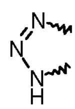

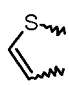 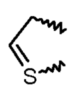 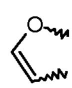 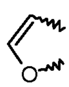

und

n für die Zahl 0 oder 1 steht.

**5.** Verbindung nach Anspruch 4, in welcher die Substruktur der Formel (I-A) für eine Substruktur steht, welche ausgewählt wird aus der Gruppe, bestehend aus

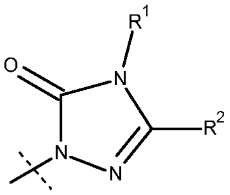
(I-A-1)

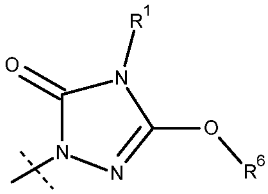
(I-A-2)

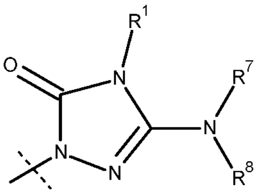
(I-A-3)

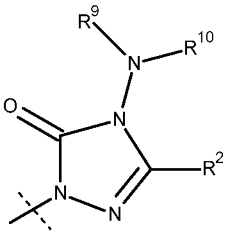
(I-A-4)

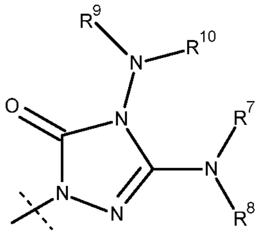
(I-A-5)

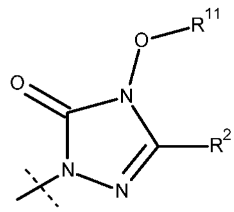
(I-A-6)

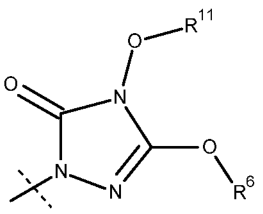
(I-A-7)

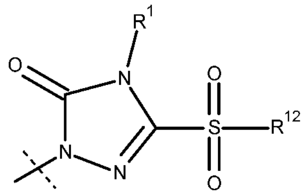
(I-A-8)

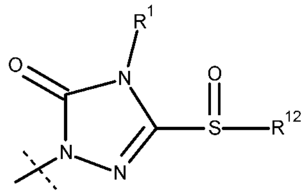
(I-A-9)

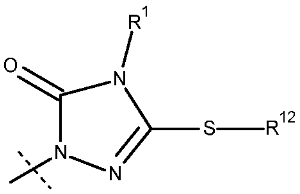
(I-A-10)

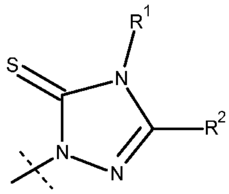
(I-A-11)

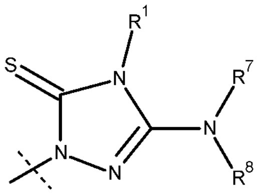
(I-A-12)

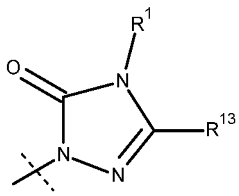
(I-A-13)

wobei

$R^1$ für Wasserstoff steht; oder
für $(C_1-C_6)$Alkyl, $(C_3-C_5)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Al-

kylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder

für ($C_3$-$C_6$)Cycloalkyl, ($C_3$-$C_6$)Cycloalkenyl, Oxetan, Oxolan, Oxan, Phenyl, Pyridyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^2$ für Wasserstoff steht; oder

für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder

für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht; oder

für Carboxyl steht;

$R^6$ für Wasserstoff steht; oder

für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)Alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder

für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;

$R^7$ und $R^8$ jeweils unabhängig voneinander für Wasserstoff, ($C_1$-$C_6$)Alkyl, Halogen($C_2$-$C_6$)Alkyl, Cyano($C_1$-$C_6$)Alkyl oder ($C_3$-$C_6$)Cycloalkyl stehen, wobei die vorgenannten Reste gegebenenfalls jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano oder Cyclopropyl substituiertes Cyclopropyl substituiert sein können;

oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, ($C_1$-$C_6$)Alkyl, Halogen($C_2$-$C_6$)Alkyl, Cyano($C_1$-$C_6$)Alkyl, ($C_3$-$C_6$)Cycloalkyl, oder gemeinsam für ($C_2$-$C_6$)Alkyliden stehen, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano oder Cyclopropyl substituiertes Cyclopropyl substituiert sein können;

oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^{12}$ für $(C_1-C_6)$Alkyl, $(C_3-C_6)$Cycloalkyl, $(C_2-C_6)$Alkenyl, Halogen$(C_1-C_6)$Alkyl, Cyano$(C_1-C_6)$Alkyl, Phenyl$(C_1-C_3)$alkyl oder $(C_3-C_6)$Cycloalkyl$(C_1-C_6)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;

$R^{13}$ für Halogen steht;

W für Wasserstoff oder Halogen (insbesondere Fluor oder Chlor) steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**6.** Verbindung nach Anspruch 4 oder 5, in welcher die Substruktur der Formel (I-A) für eine Substruktur steht, welche ausgewählt wird aus der Gruppe, bestehend aus

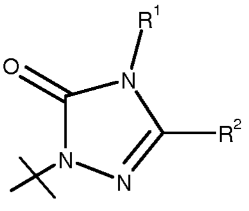

(I-A-1)

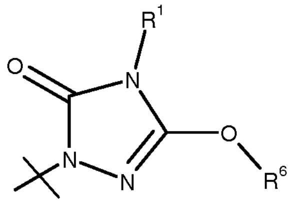

(I-A-2)

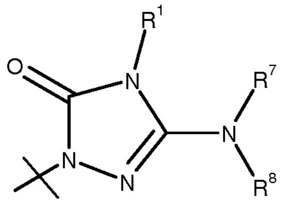

(I-A-3)

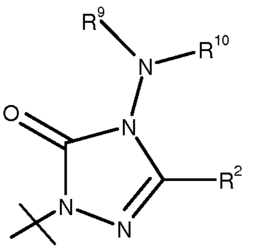

(I-A-4)

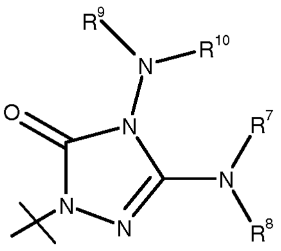

(I-A-5)

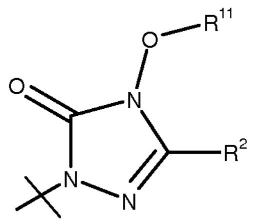

(I-A-6)

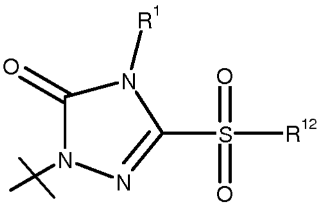

(I-A-8)

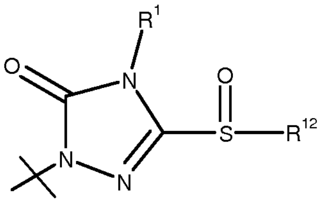

(I-A-9)

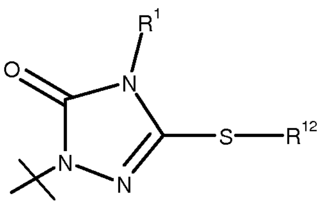

(I-A-10)

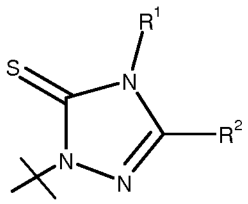

(I-A-11)

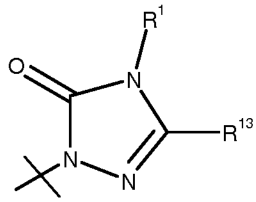

(I-A-13)

wobei

$R^1$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyridyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^2$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl,

Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht; oder

für Carboxyl steht;

$R^6$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^7$ und $R^8$ jeweils unabhängig voneinander für Wasserstoff, $(C_1-C_6)$Alkyl, Halogen$(C_2-C_6)$Alkyl, Cyano$(C_1-C_6)$Alkyl oder $(C_3-C_6)$Cycloalkyl, wobei die vorgenannten Reste gegebenenfalls jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können;

oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^9$ und $R^{10}$ jeweils unabhängig voneinander für Wasserstoff, $(C_1-C_6)$Alkyl, Halogen$(C_2-C_6)$Alkyl, Cyano$(C_1-C_6)$Alkyl, $(C_3-C_6)$Cycloalkyl, oder gemeinsam für $(C_2-C_6)$Alkyliden steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano oder Cyclopropyl substituiertes Cyclopropyl substituiert sein können;

oder gemeinsam zusammen mit dem Stickstoffatom, an das sie gebunden sind, für ein gegebenenfalls substituiertes Triazolinon, Triazolidinthion, Tetrazolinon, Tetrazolidinthion, Oxadiazolinon, Pyrrolidinon, Pyrrolidinthion, Imidazolinon, Imidazolidinthion, Pyrrolidindion, Thiazolidinon, Thiazolidinthion, Morpholin, Thiomorpholin, Thiomorpholin-1-oxid, Thiomorpholin-1,1-dioxid, Piperazin, *N*-Methylpiperazin oder *N*-Ethylpiperazin, stehen;

$R^{11}$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$Alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^{12}$ für $(C_1-C_6)$Alkyl, $(C_3-C_6)$Cycloalkyl, $(C_2-C_6)$Alkenyl, Halogen$(C_1-C_6)$Alkyl, Cyano$(C_1-C_6)$Alkyl, Phenyl$(C_1-C_3)$alkyl oder $(C_3-C_6)$Cycloalkyl$(C_1-C_6)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Ha-

lo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können;
$R^{13}$ für Halogen steht;
W für Wasserstoff oder Halogen, insbesondere Fluor oder Chlor, steht;
X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und
n für die Zahl 0 oder 1 steht.

**7.** Verbindung nach einem der Ansprüche 1 bis 3, in welcher

A und B gemeinsam mit den Atomen, an die sie gebunden sind, für eine Substruktur der Formel (I-B) stehen

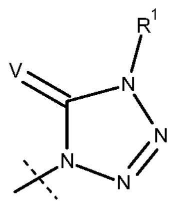

(I-B)

wobei
$R^1$ für Wasserstoff, Cyano oder Nitro steht; oder
für ($C_1$-$C_6$)Alkyl, ($C_3$-$C_6$)Cycloalkyl($C_1$-$C_6$)alkyl, Halogen($C_2$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylsulfanyl($C_1$-$C_6$)alkyl, Hetarylsulfinyl($C_1$-$C_6$)alkyl, Hetarylsulfonyl($C_1$-$C_6$)alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, steht; oder
für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für ($C_1$-$C_6$)Alkoxy, Halogen($C_1$-$C_6$)alkoxy, ($C_3$-$C_6$)Cycloalkyloxy, Aryloxy, Aryl($C_1$-$C_6$)alkyloxy oder Carbonyloxy

steht, wobei die vorgenannten Reste jeweils gegebenenfalls substiuiert sein können, oder für Hydroxy steht; oder für ($C_1$-$C_6$)Alkylamino, Halogen($C_1$-$C_6$)alkylamino, Dihalogen($C_1$-$C_6$)alkylamino, Di($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfanyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und
V für Sauerstoff, Schwefel oder einen gegebenenfalls substituierten Stickstoff stehen;
X, Y und Z, jeweils unabhängig voneinander,

für Wasserstoff, Halogen, Hydroxy, Amino, Cyano, Nitro, OCN, SCN, $SF_5$, stehen; oder
für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cyclo($C_1$-$C_6$)alkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder
für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,
wobei R' und R" unabhängig voneinander
für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder
R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl,

Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder
für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

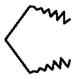 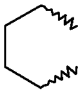 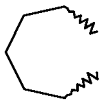 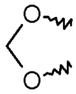 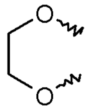 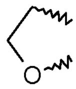 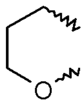

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

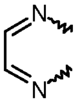 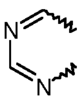 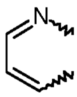 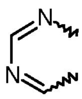 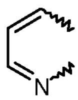 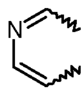

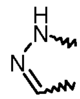 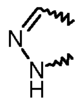 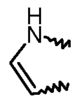 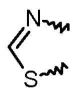 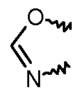 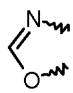

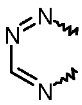 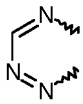 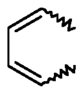

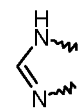 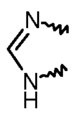 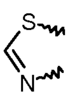 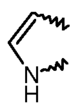 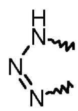 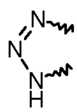

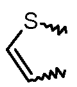 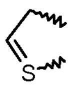 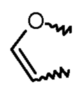 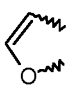

und
n für die Zahl 0 oder 1 steht.

**8.** Verbindung nach Anspruch 7, in welcher
die Substruktur der Formel (I-B) für eine Substruktur steht, welche ausgewählt wird aus der Gruppe, bestehend aus

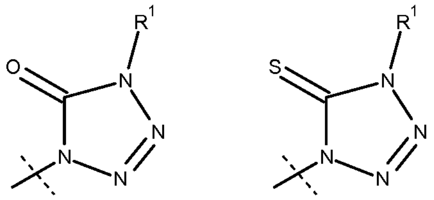

(I-B-1) (I-B-2)

wobei

$R^1$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

$(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**9.** Verbindung nach einem der Ansprüche 1 bis 3, in welcher

A und B gemeinsam mit den Atomen, an die sie gebunden sind, für eine Substruktur der Formel (I-C) stehen

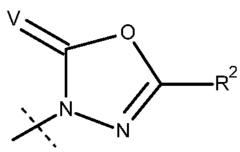

(I-C)

wobei

$R^2$ für Wasserstoff, Cyano, Halogen oder Nitro steht; oder

für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfa-

nyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring,

W für Wasserstoff oder Halogen steht;

V für Sauerstoff, Schwefel oder einen gegebenenefalls substituierten Stickstoff stehen;

X, Y und Z, jeweils unabhängig voneinander,

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Al-

kyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R" unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

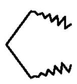 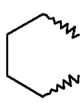 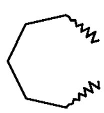 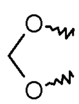 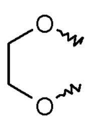 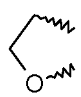 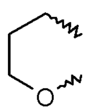

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

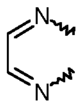 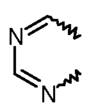 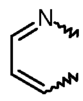 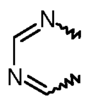 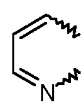 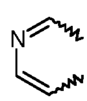

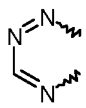 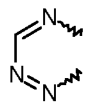 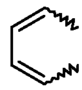 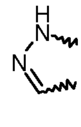 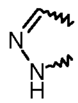 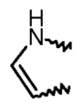 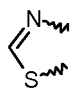 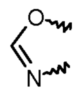 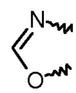 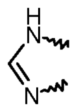 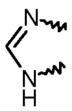 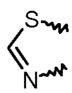 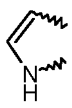 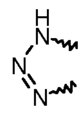 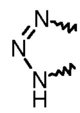

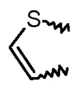 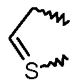 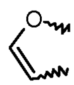 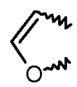

n für die Zahl 0 oder 1 steht.

**10.** Verbindung nach Anspruch 9, in welcher die Substruktur der Formel (I-C) für die Substruktur (I-C-1) steht,

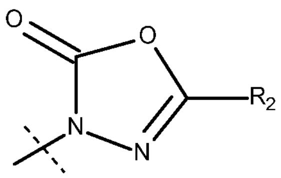

(I-C-1)

wobei

$R^2$ für Wasserstoff steht; oder
für $(C_1\text{-}C_6)$Alkyl, $(C_3\text{-}C_6)$Cycloalkyl$(C_1\text{-}C_6)$alkyl, $(C_2\text{-}C_6)$Alkenyl, $(C_2\text{-}C_6)$Alkinyl, Halogen$(C_1\text{-}C_6)$alkyl, Cyano$(C_1\text{-}C_6)$alkyl, $(C_1\text{-}C_6)$Alkoxy$(C_1\text{-}C_6)$alkyl oder Phenyl$(C_1\text{-}C_3)$alkyl, steht, wobei die vorgenannten Reste jeweils gegebenenfalls substituiert sein können; oder
für $(C_3\text{-}C_6)$Cycloalkyl, $(C_3\text{-}C_6)$Cycloalkenyl, Phenyl, Pyridyl, Pyrimidyl, Pyridazinyl, Pyrazinyl, Furyl, Thienyl, Triazinyl, Thiazolyl, Thiadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen$(C_1\text{-}C_3)$alkylsulfanyl oder gegebenenfalls durch Methyl, Fluor, Chlor, Cyano substituiertes Cyclopropyl substituiert sein können, steht;
W Wasserstoff oder Halogen, insbesondere F oder Cl, steht;
X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;
Z für Wasserstoff steht; und
n für die Zahl 0 oder 1 steht.

**Claims**

**11.** Verbindung nach einem der Ansprüche 1 bis 3, in welcher A und B gemeinsam mit den Atomen, an die sie gebunden sind, für eine Substruktur der Formel (I-D) stehen

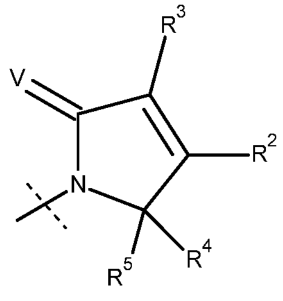

(I-D)

wobei

$R^2$, $R^3$, $R^4$, und $R^5$, jeweils unabhängig voneinander,
für Wasserstoff, Cyano, Halogen oder Nitro stehen; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halo-

gen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder $R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring bilden können;oder

oder $R^2$ für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring, steht;

W für Wasserstoff oder Halogen steht;

V für Sauerstoff, Schwefel oder einen gegebenenfalls substituierten Stickstoff stehen;

W für Wasserstoff oder Halogen steht;

X, Y und Z, jeweils unabhängig voneinander,

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R'' stehen,

wobei R' und R'' unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

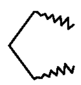 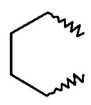 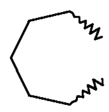 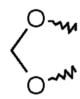 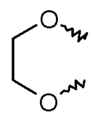 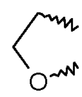 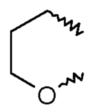

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

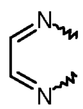 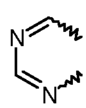 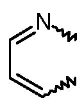 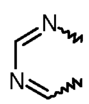 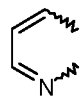 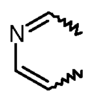

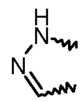 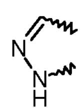 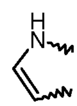

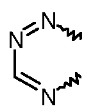 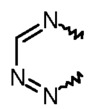 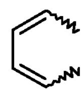

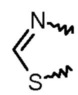 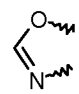 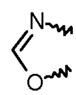

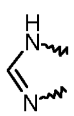 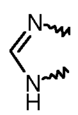 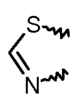

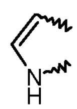 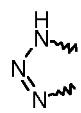 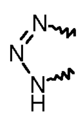

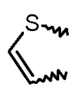 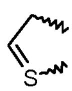 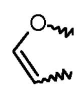 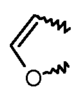

n für die Zahl 0 oder 1 steht.

**12.** Verbindung nach Anspruch 11, in welcher die Substruktur der Formel (I-D) für eine Substruktur steht, welche ausgewählt wird aus der Gruppe, bestehend aus

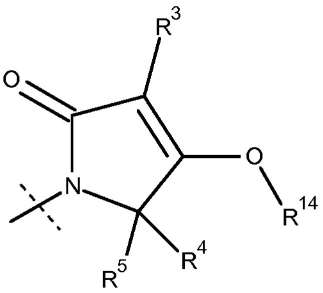

(I-D-1)

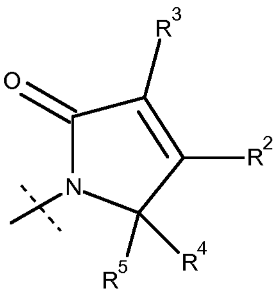

(I-D-2)

wobei

$R^2$, $R^3$, $R^4$, und $R^5$ unabhängig voneinander
für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht; oder
für Carboxyl steht; oder
$R^4$ und $R^5$ gemeinsam mit dem Atom, an das sie gebunden sind, einen gegebenenfalls einfach oder mehrfach, gleich oder verschieden durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring bilden können;
$R^{14}$ für $(C_1-C_6)$Alkyl, Halogen$(C_1-C_6)$alkyl, Cyano$(C_1-C_6)$alkyl, $(C_1-C_6)$Alkoxy$(C_1-C_3)$alkyl, Phenyl$(C_1-C_3)$alkyl, $(C_3-C_6)$Cycloalkyl oder $(C_3-C_6)$Cycloalkyl$(C_1-C_3)$alkyl stehen, wobei die vorgenannten Reste jeweils jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können;
X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
n für die Zahl 0 oder 1 steht.

**13.** Verbindung nach einem der Ansprüche 1 bis 3, in welcher

A und B gemeinsam mit den Atomen, an die sie gebunden sind, für eine Substruktur der Formel (I-E) stehen

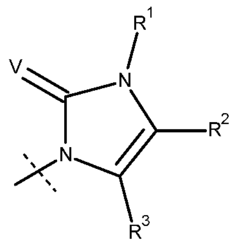

(I-E)

$R^1$ für Wasserstoff, Cyano oder Nitro steht; oder
für $(C_1-C_6)$Alkyl, $(C_3-C_6)$Cycloalkyl$(C_1-C_6)$alkyl, Halogen$(C_2-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylsulfanyl$(C_1-C_6)$alkyl, Hetarylsulfinyl$(C_1-C_6)$alkyl, Hetarylsulfonyl$(C_1-C_6)$alkyl, steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, steht; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl steht; oder
für gegebenenfalls substituiertes Phenyl oder gegebenenfalls substituiertes Hetaryl steht; oder
für $(C_1-C_6)$Alkoxy, Halogen$(C_1-C_6)$alkoxy, $(C_3-C_6)$Cycloalkyloxy, Aryloxy, Aryl$(C_1-C_6)$alkyloxy oder Carbonyloxy steht, wobei die vorgenannten Reste jeweils gegebenenfalls substiuiert sein können, oder für Hydroxy steht; oder
für $(C_1-C_6)$Alkylamino, Halogen$(C_1-C_6)$alkylamino, Dihalogen$(C_1-C_6)$alkylamino, Di$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können, oder Amino steht; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfanyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl steht, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl steht; und
$R^2$ und $R^3$, jeweils unabhängig voneinander,
für Wasserstoff, Cyano, Halogen oder Nitro steht; oder

für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder

für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder

für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder

für gegebenenfalls substituiertes Phenyl oder gegebenenfalls substituiertes Hetaryl stehen; oder

für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder

für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder

für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder

für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring,

W für Wasserstoff oder Halogen steht;

V für Sauerstoff, Schwefel oder einen gegebenenfalls substituierten Stickstoff stehen;

X, Y und Z, jeweils unabhängig voneinander,

für Wasserstoff, Halogen, Hydroxy, Amino, Cyano, Nitro, OCN, SCN, $SF_5$, stehen; oder

für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino,

Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cycloalkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl, ($C_1$-$C_6$)Alkylsulfoximino, Aminothiocarbonyl, ($C_1$-$C_6$)Alkylaminothiocarbonyl oder Di($C_1$-$C_6$)alkylaminothiocarbonyl stehen, wobei alle vorgenannten Reste gegebenenfalls durch Halogen substituiert sein können; oder

für Phenyl($C_1$-$C_3$)alkyl, Phenoxy, Phenyl($C_1$-$C_3$)alkyloxy, Phenoxy($C_1$-$C_3$)alkyl, Phenylthio, Phenylthio($C_1$-$C_3$)alkyl, Phenylsulfinyl, Phenylsulfonyl, Hetaryl($C_1$-$C_3$)alkyl, Hetaryloxy, Hetaryl($C_1$-$C_3$)alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für ($C_3$-$C_6$)Cycloalkyl($C_1$-$C_3$)alkyl, ($C_3$-$C_6$)Cycloalkyloxy, ($C_3$-$C_6$)Cycloalkyl($C_1$-$C_3$)alkoxy, ($C_3$-$C_6$)Cycloalkylthio, ($C_3$-$C_6$)Cycloalkyl($C_1$-$C_3$)alkylthio, ($C_3$-$C_6$)Cycloalkylsulfinyl, ($C_3$-$C_6$)Cycloalkyl($C_1$-$C_3$)alkylsulfinyl, ($C_3$-$C_6$)Cycloalkylsulfonyl, ($C_3$-$C_6$)Cycloalkyl($C_1$-$C_3$)alkylsulfonyl oder ($C_3$-$C_8$)Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder

für NR'R" stehen,

wobei R' und R" unabhängig voneinander

für Wasserstoff, Cyano, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, ($C_1$-$C_6$)Alkylthio($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, Acyl oder ($C_1$-$C_6$)Alkoxycarbonyl stehen; oder

R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder

für einen ($C_3$-$C_6$)Cycloalkyl, Oxetan, Oxolan, Oxan, ($C_3$-$C_8$)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

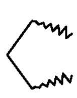 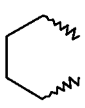 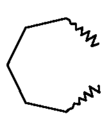 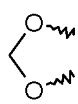 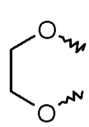 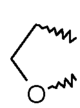 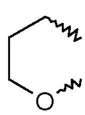

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

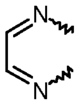 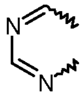 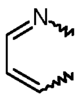 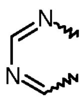 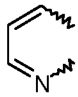 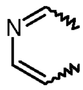

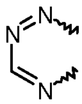 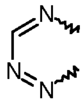 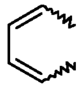 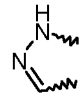 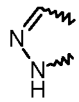 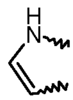

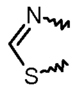 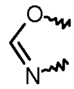 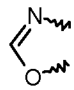

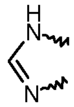 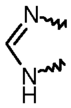 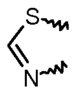 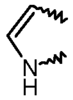 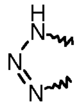 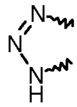

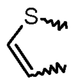 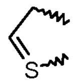 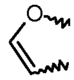 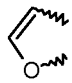

und
n für die Zahl 0 oder 1 steht.

**14.** Verbindung nach Anspruch 13, in welcher
die Substruktur der Formel (I-E) für eine Substruktur der Formel (I-E-1) steht,

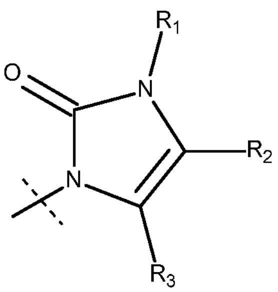

(I-E-1)

wobei

$R^1$ für Wasserstoff steht; oder
für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder
für $(C_3-C_6)$Cycloalkyl, $(C_3-C_6)$Cycloalkenyl, Oxetan, Oxolan, Oxan, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halo-

gen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht;
$R^2$ und $R^3$ unabhängig voneinander für Wasserstoff steht; oder
für ($C_1$-$C_6$)Alkyl, ($C_3$-$C_6$)Cycloalkyl($C_1$-$C_3$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl steht, wobei die vorgenannten Reste jeweils gegebenenfalls einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können; oder
für ($C_3$-$C_6$)Cycloalkyl, ($C_3$-$C_6$)Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls durch Methyl, Fluor, Chlor, Cyano substituiertes Cyclopropyl substituiert sein können, steht; ;
W für Wasserstoff oder Halogen, insbesondere Fluor oder Chlor, steht;
X, Y und Z unabhängig voneinander für Wasserstoff, Fluor, Chlor, Brom, Cyano, Nitro, Amino, Hydroxy, ($C_1$-$C_4$)Alkyl, ($C_1$-$C_4$)Halogenalkyl, ($C_2$-$C_4$)Alkenyl, ($C_2$-$C_4$)Alkinyl, ($C_1$-$C_4$)Alkoxy, ($C_1$-$C_4$)Halogenalkoxy oder Aminothiocarbonyl stehen;
oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;
oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und
n für die Zahl 0 oder 1 steht.

**15.** Verbindung nach einem der Ansprüche 1 bis 3, in welcher

A und B gemeinsam mit den Atomen, an die sie gebunden sind, für eine Substruktur der Formel (I-F) stehen

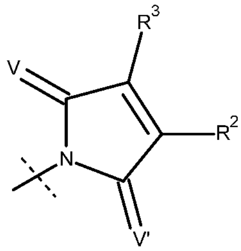

(I-F)

$R^2$ und $R^3$, jeweils unabhängig voneinander,
für Wasserstoff, Cyano, Halogen oder Nitro steht; oder
für ($C_1$-$C_6$)Alkyl, ($C_3$-$C_6$)Cycloalkyl($C_1$-$C_6$)alkyl, Halogen($C_1$-$C_6$)alkyl, Cyano($C_1$-$C_6$)alkyl, Hydroxy($C_1$-$C_6$)alkyl, ($C_1$-$C_6$)Alkoxy($C_1$-$C_6$)alkyl, Amino($C_1$-$C_6$)alkyl, ($C_1$-$C_6$)Alkoxycarbonyl($C_1$-$C_6$)alkyl, ($C_1$-$C_6$)Alkylsulfanyl($C_1$-$C_6$)alkyl, ($C_1$-$C_6$)Alkylsulfinyl($C_1$-$C_6$)alkyl, ($C_1$-$C_6$)Alkylsulfonyl($C_1$-$C_6$)alkyl, Halogen($C_1$-$C_6$)alkylsulfanyl($C_1$-$C_6$)alkyl, Halogen($C_1$-$C_6$)alkylsulfinyl($C_1$-$C_6$)alkyl, Halogen($C_1$-$C_6$)alkylsulfonyl($C_1$-$C_6$)alkyl, ($C_1$-$C_6$)Alkoxy($C_1$-$C_6$)alkylsulfanyl($C_1$-$C_6$)alkyl, ($C_1$-$C_6$)Alkoxy($C_1$-$C_6$)alkylsulfinyl($C_1$-$C_6$)alkyl, ($C_1$-$C_6$)Alkoxy($C_1$-$C_6$)alkylsulfonyl($C_1$-$C_6$)alkyl, Phenyl($C_1$-$C_6$)alkyl, Phenoxy($C_1$-$C_6$)alkyl, Phenylsulfanyl($C_1$-$C_6$)alkyl, Phenylsulfinyl($C_1$-$C_6$)alkyl, Phenylsulfonyl($C_1$-$C_6$)alkyl, Hetaryl($C_1$-$C_6$)alkyl, Hetaryloxy($C_1$-$C_6$)alkyl, Hetarylthio($C_1$-$C_6$)alkyl stehen, wobei die vorgenannten Reste jeweils gegebenenfalls substituiert sein können und/oder unter der Bedingung, dass im Falle eines ungesättigten Restes, die definierte

Mindestanzahl an Kohlenstoffatomen 2 beträgt, die Reste gesättigt oder ungesättigt sein können; oder
für gegebenenfalls substituiertes gesättigtes oder ungesättigtes ($C_3$-$C_6$)Cycloalkyl, welches gegebenenfalls durch ein oder mehrere Heteroatome unterbrochen sein kann, stehen; oder
für ($C_1$-$C_6$)Alkylcarbonyl, Halogen($C_1$-$C_6$)alkylcarbonyl, Hydroxy($C_1$-$C_6$)alkylcarbonyl, ($C_1$-$C_6$)Alkoxy($C_1$-$C_6$)alkylcarbonyl, Phenylcarbonyl, Hetarylcarbonyl, ($C_1$-$C_6$)Alkoxycarbonyl, Halogen($C_1$-$C_6$)alkoxycarbonyl, Aryloxycarbonyl, Aminocarbonyl, ($C_1$-$C_6$)Alkylaminocarbonyl, Di($C_1$-$C_6$)alkylaminocarbonyl, ($C_3$-$C_6$)Cycloalkylaminocarbonyl, Di($C_3$-$C_6$)cycloalkylaminocarbonyl, ($C_3$-$C_6$)Cycloalkyl(($C_1$-$C_6$)alkyl)aminocarbonyl, Arylaminocarbonyl, Diarylaminocarbonyl, ($C_1$-$C_6$)Alkyl(aryl)aminocarbonyl, ($C_3$-$C_6$)Cycloalkyl(aryl)aminocarbonyl, ($C_1$-$C_6$)Alkylaminothiocarbonyl, Di($C_1$-$C_6$)alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für ($C_1$-$C_6$)Alkoxy, Halogen($C_1$-$C_6$)alkoxy, ($C_1$-$C_6$)Alkoxy($C_1$-$C_6$)alkoxy, Aryloxy, Aryl($C_1$-$C_6$)alkyloxy, ($C_3$-$C_6$)Cycloalkyloxy, ($C_3$-$C_6$)Cycloalkyl($C_1$-$C_6$)alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für ($C_1$-$C_6$)Alkylamino, Di($C_1$-$C_6$)alkylamino, Halogen($C_1$-$C_6$)alkylamino, Dihalogen($C_1$-$C_6$)alkylamino, ($C_3$-$C_6$)Cycloalkylamino, Di($C_3$-$C_6$)cycloalkylamino, ($C_3$-$C_6$)Cycloalkyl(($C_1$-$C_6$)alkyl)amino, Arylamino, Diarylamino, Hetarylamino, Dihetarylamino, ($C_1$-$C_6$)Alkyl(aryl)amino, ($C_3$-$C_6$)Cycloalkyl(aryl)amino, ($C_1$-$C_6$)Alkylcarbonylamino, Arylcarbonylamino, ($C_1$-$C_6$)Alkoxycarbonylamino, Aryloxycarbonylamino, ($C_1$-$C_6$)Alkylcarbamoylamino, Arylcarbamoylamino, ($C_1$-$C_6$)Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für ($C_1$-$C_6$)Alkylsulfanyl, ($C_1$-$C_6$)Alkylsulfinyl, ($C_1$-$C_6$)Alkylsulfonyl, Halogen($C_1$-$C_6$)alkylsulfanyl, Halogen($C_1$-$C_6$)alkylsulfinyl, Halogen($C_1$-$C_6$)alkylsulfonyl, ($C_3$-$C_6$)Cycloalkylsulfanyl, ($C_3$-$C_6$)Cycloalkylsulfinyl, ($C_3$-$C_6$)Cycloalkylsulfonyl, ($C_3$-$C_6$)Cycloalkyl($C_1$-$C_6$)alkylsulfanyl, ($C_3$-$C_6$)Cycloalkyl($C_1$-$C_6$)alkylsulfinyl, ($C_3$-$C_6$)Cycloalkyl($C_1$-$C_6$)alkylsulfonyl, Arylsulfanyl, Arylsulfinyl, Arylsulfonyl, Aryl($C_1$-$C_6$)alkylsulfanyl, Aryl($C_1$-$C_6$)alkylsulfinyl, Aryl($C_1$-$C_6$)alkylsulfonyl, Aminosulfonyl, ($C_1$-$C_6$)Alkylaminosulfonyl, Di($C_1$-$C_6$)alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder
für einen gegebenenfalls einfach oder mehrfach, gleich oder verschieden durch Halogen, Cyano, Nitro, Hydroxy, Amino, ($C_1$-$C_6$)Alkyl, Halogen($C_1$-$C_6$)Alkyl, ($C_1$-$C_6$)Alkoxy, Halogen($C_1$-$C_6$)alkoxy, ($C_1$-$C_6$)Alkylsulfinyl, ($C_1$-$C_6$)Alkylsulfanyl, ($C_1$-$C_6$)Alkylsulfonyl, Halo($C_1$-$C_6$)alkylsulfinyl, Halo($C_1$-$C_6$)alkylsulfanyl, Halo($C_1$-$C_6$)alkylsulfonyl oder gegebenenfalls substituiertes ($C_3$-$C_6$)Cycloalkyl, substituierten und gegebenenfalls durch Heteroatome aus der Reihe O, S oder N unterbrochenen gesättigten oder ungesättigten drei- bis sechsgliedrigen Ring,
W für Wasserstoff oder Halogen steht;
V und V', jeweils unabhängig voneinander, für Sauerstoff, Schwefel oder einen gegebenenfalls substituierten Stickstoff stehen;
X, Y und Z, jeweils unabhängig voneinander,
für Wasserstoff, Halogen, Hydroxy, Amino, Cyano, Nitro, OCN, SCN, $SF_5$, stehen; oder
für Tri($C_1$-$C_6$)alkylsilyl, ($C_1$-$C_6$)Alkyl, Halogen($C_1$-$C_6$)alkyl, Cyano($C_1$-$C_6$)alkyl, Hydroxy($C_1$-$C_6$)alkyl, ($C_1$-$C_6$)Alkoxycarbonyl($C_1$-$C_6$)alkyl, ($C_1$-$C_6$)Alkoxy($C_1$-$C_6$)alkyl, ($C_2$-$C_6$)Alkenyl, Halogen($C_2$-$C_6$)alkenyl, Cyano($C_2$-$C_6$)alkenyl, ($C_2$-$C_6$)Alkinyl, Halogen($C_2$-$C_6$)alkinyl, Cyano($C_2$-$C_6$)alkinyl, ($C_1$-$C_6$)Alkoxy, Halogen($C_1$-$C_6$)alkoxy, Cyano($C_1$-$C_6$)alkoxy, Hydroxycarbonyl($C_1$-$C_6$)alkoxy, ($C_1$-$C_6$)Alkoxycarbonyl($C_1$-$C_6$)alkoxy, ($C_1$-$C_6$)Alkoxy($C_1$-$C_6$)alkoxy, ($C_1$-$C_6$)Alkylhydroxyimino, ($C_1$-$C_6$)Alkoxyimino, ($C_1$-$C_6$)Alkyl($C_1$-$C_6$)alkoxyimino, Halogen($C_1$-$C_6$)alkyl($C_1$-$C_6$)alkoxyimino, ($C_1$-$C_6$)Alkylthio, Halogen($C_1$-$C_6$)alkylthio, ($C_1$-$C_6$)Alkoxy($C_1$-$C_6$)alkylthio, ($C_1$-$C_6$)Alkylthio($C_1$-$C_6$)alkyl, ($C_1$-$C_6$)Alkylsulfinyl, Halogen($C_1$-$C_6$)alkylsulfinyl, ($C_1$-$C_6$)Alkoxy($C_1$-$C_6$)alkylsulfinyl, ($C_1$-$C_6$)Alkylsulfinyl($C_1$-$C_6$)alkyl, ($C_1$-$C_6$)Alkylsulfonyl, Halogen($C_1$-$C_6$)alkylsulfonyl, ($C_1$-$C_6$)Alkoxy($C_1$-$C_6$)alkylsulfonyl, ($C_1$-$C_6$)Alkylsulfonyl($C_1$-$C_6$)alkyl, ($C_1$-$C_6$)Alkylsulfonyloxy, ($C_1$-$C_6$)Alkylcarbonyl, Halogen($C_1$-$C_6$)alkylcarbonyl, Carboxyl, ($C_1$-$C_6$)Alkylcarbonyloxy, ($C_1$-$C_6$)Alkoxycarbonyl, Halogen($C_1$-$C_6$)alkoxycarbonyl, Aminocarbonyl, ($C_1$-$C_6$)Alkylaminocarbonyl, Di($C_1$-$C_6$)alkylaminocarbonyl, ($C_2$-$C_6$)Alkenylaminocarbonyl, Di($C_2$-$C_6$)alkenylaminocarbonyl, ($C_3$-$C_6$)Cyclo($C_1$-$C_6$)alkylaminocarbonyl, ($C_1$-$C_6$)Alkylsulfonylamino, Aminosulfonyl, ($C_1$-$C_6$)Alkylaminosulfonyl, Di($C_1$-$C_6$)alkylaminosulfonyl,

$(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,
wobei R' und R" unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder
R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder
für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

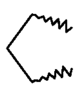 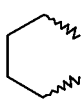 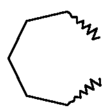 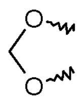 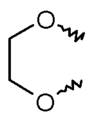 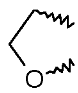 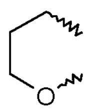

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

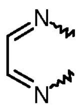 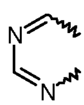 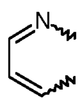 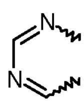 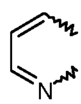 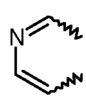

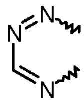
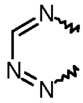
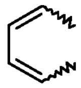
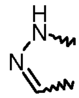
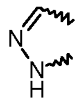
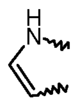
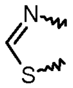
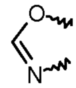
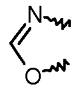
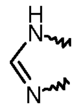
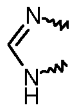
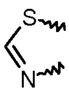
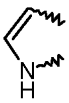
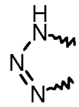
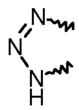
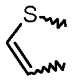
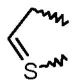
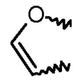
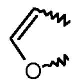

und
n für die Zahl 0 oder 1 steht.

**16.** Verbindung nach Anspruch 15, in welcher die Substruktur der Formel (I-F) für eine Substruktur der Formel (I-F-1) steht,

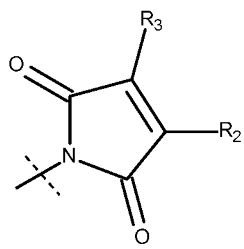

(I-F-1)

wobei

$R^2$ und $R^3$ unabhängig voneinander für Wasserstoff oder Halogen stehen; oder
für ($C_1$-$C_6$)Alkyl, ($C_3$-$C_6$)Cycloalkyl($C_1$-$C_6$)alkyl, ($C_2$-$C_6$)Alkenyl, ($C_2$-$C_6$)Alkinyl, Halogen($C_1$-$C_6$)alkyl, Cyano($C_1$-$C_6$)alkyl, ($C_1$-$C_6$)Alkoxy($C_1$-$C_6$)alkyl oder Phenyl($C_1$-$C_3$)alkyl, steht, wobei die vorgenannten Reste jeweils gegebenenfalls substituiert sein können; oder
für ($C_3$-$C_6$)Cycloalkyl, ($C_3$-$C_6$)Cycloalkenyl oder Phenyl, wobei die vorgenannten Reste jeweils einfach bis dreifach durch Halogen, Cyano, Nitro, Hydroxy, Amino, Methyl, Ethyl, Isopropyl, tert-Butyl, Trifluoromethyl, Difluoromethyl, Methoxy, Trifluoromethoxy, Difluoromethoxy, Cyano, Amino, Hydroxy, Nitro, Halogen($C_1$-$C_3$)alkylsulfanyl oder gegebenenfalls durch Methyl, Fluor, Chlor, Cyano substituiertes Cyclopropyl substituiert sein können, stehen;
W Wasserstoff oder Halogen, insbesondere F oder Cl, steht;
X und Y, unabhängig voneinander für Wasserstoff, Fluor, Chlor, Brom, Methyl, Ethyl, Trifluoromethyl, 2,2-Difluoromethyl, Difluoromethoxy, Trifluoromethoxy, Cyclopropyl, Cyano, Amino, Hydroxy oder Nitro stehen;
Z für Wasserstoff steht; und
n für die Zahl 0 oder 1 steht.

**17.** Verbindung nach einem der Ansprüche 1 bis 3, in welcher

A und B gemeinsam mit den Atomen, an die sie gebunden sind, für eine Substruktur der Formel (I-G) stehen

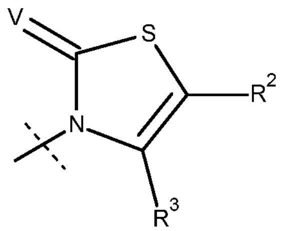

(I-G)

$R^2$ und $R^3$, jeweils unabhängig voneinander,
für Wasserstoff, Cyano, Halogen oder Nitro steht; oder
für $(C_1-C_6)$Alkyl, $(C_3-C_6)$Cycloalkyl$(C_1-C_6)$alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, Amino$(C_1-C_6)$alkyl, $(C_1-C_6)$Alkoxycarbonyl$(C_1-C_6)$alkyl, $(C_1-C_6)$Alkylsulfanyl$(C_1-C_6)$alkyl, $(C_1-C_6)$Alkylsulfinyl$(C_1-C_6)$alkyl, $(C_1-C_6)$Alkylsulfonyl$(C_1-C_6)$alkyl, Halogen$(C_1-C_6)$alkylsulfanyl$(C_1-C_6)$alkyl, Halogen$(C_1-C_6)$alkylsulfinyl$(C_1-C_6)$alkyl, Halogen$(C_1-C_6)$alkylsulfonyl$(C_1-C_6)$alkyl, $(C_1-C_6)$Alkoxy$(C_1-C_6)$alkylsulfanyl$(C_1-C_6)$alkyl, $(C_1-C_6)$Alkoxy$(C_1-C_6)$alkylsulfinyl$(C_1-C_6)$alkyl, $(C_1-C_6)$Alkoxy$(C_1-C_6)$alkylsulfonyl$(C_1-C_6)$alkyl, Phenyl$(C_1-C_6)$alkyl, Phenoxy$(C_1-C_6)$alkyl, Phenylsulfanyl$(C_1-C_6)$alkyl, Phenylsulfinyl$(C_1-C_6)$alkyl, Phenylsulfonyl$(C_1-C_6)$alkyl, Hetaryl$(C_1-C_6)$alkyl, Hetaryloxy$(C_1-C_6)$alkyl, Hetarylthio$(C_1-C_6)$alkyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können; oder
für gegebenenfalls substituiertes gesättigtes oder ungesättigtes $(C_3-C_6)$Cycloalkyl, welches gegebenenfalls durch ein oder mehrere Heteroatome unterbrochen sein kann, stehen; oder
für $(C_1-C_6)$Alkylcarbonyl, Halogen$(C_1-C_6)$alkylcarbonyl, Hydroxy$(C_1-C_6)$alkylcarbonyl, $(C_1-C_6)$Alkoxy$(C_1-C_6)$alkylcarbonyl, Phenylcarbonyl, Hetarylcarbonyl, $(C_1-C_6)$Alkoxycarbonyl, Halogen$(C_1-C_6)$alkoxycarbonyl, Aryloxycarbonyl, Aminocarbonyl, $(C_1-C_6)$Alkylaminocarbonyl, Di$(C_1-C_6)$alkylaminocarbonyl, $(C_3-C_6)$Cycloalkylaminocarbonyl, Di$(C_3-C_6)$cycloalkylaminocarbonyl, $(C_3-C_6)$Cycloalkyl($(C_1-C_6)$alkyl)aminocarbonyl, Arylaminocarbonyl, Diarylaminocarbonyl, $(C_1-C_6)$Alkyl(aryl)aminocarbonyl, $(C_3-C_6)$Cycloalkyl(aryl)aminocarbonyl, $(C_1-C_6)$Alkylaminothiocarbonyl, Di$(C_1-C_6)$alkylaminothiocarbonyl oder Aminothiocarbonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Carbonyl oder Carboxyl stehen; oder
für gegebenenfalls substituiertes Phenyl oder gegebenenfalls substituiertes Hetaryl stehen; oder
für $(C_1-C_6)$Alkoxy, Halogen$(C_1-C_6)$alkoxy, $(C_1-C_6)$Alkoxy$(C_1-C_6)$alkoxy, Aryloxy, Aryl$(C_1-C_6)$alkyloxy, $(C_3-C_6)$Cycloalkyloxy, $(C_3-C_6)$Cycloalkyl$(C_1-C_6)$alkyloxy oder Carbonyloxy stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Hydroxy stehen; oder
für $(C_1-C_6)$Alkylamino, Di$(C_1-C_6)$alkylamino, Halogen$(C_1-C_6)$alkylamino, Dihalogen$(C_1-C_6)$alkylamino, $(C_3-C_6)$Cycloalkylamino, Di$(C_3-C_6)$cycloalkylamino, $(C_3-C_6)$Cycloalkyl($(C_1-C_6)$alkyl)amino, Arylamino, Diarylamino, Hetarylamino, Dihetarylamino, $(C_1-C_6)$Alkyl(aryl)amino, $(C_3-C_6)$Cycloalkyl(aryl)amino, $(C_1-C_6)$Alkylcarbonylamino, Arylcarbonylamino, $(C_1-C_6)$Alkoxycarbonylamino, Aryloxycarbonylamino, $(C_1-C_6)$Alkylcarbamoylamino, Arylcarbamoylamino, $(C_1-C_6)$Alkylsulfonylamino, oder Arylsulfonylamino stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Amino stehen; oder
für $(C_1-C_6)$Alkylsulfanyl, $(C_1-C_6)$Alkylsulfinyl, $(C_1-C_6)$Alkylsulfonyl, Halogen$(C_1-C_6)$alkylsulfanyl, Halogen$(C_1-C_6)$alkylsulfinyl, Halogen$(C_1-C_6)$alkylsulfonyl, $(C_3-C_6)$Cycloalkylsulfanyl, $(C_3-C_6)$Cycloalkylsulfinyl, $(C_3-C_6)$Cycloalkylsulfonyl, $(C_3-C_6)$Cycloalkyl$(C_1-C_6)$alkylsulfanyl, $(C_3-C_6)$Cycloalkyl$(C_1-C_6)$alkylsulfinyl, $(C_3-C_6)$Cycloalkyl$(C_1-C_6)$alkylsulfonyl, Arylsulfanyl, Arylsulfinyl, Arylsulfonyl, Aryl$(C_1-C_6)$alkylsulfanyl, Aryl$(C_1-C_6)$alkylsulfinyl, Aryl$(C_1-C_6)$alkylsulfonyl, Aminosulfonyl, $(C_1-C_6)$Alkylaminosulfonyl, Di$(C_1-C_6)$alkylaminosulfonyl oder Arylaminosulfonyl stehen, wobei die vorgenannten Reste jeweils gegebenenfalls substituiert sein können und/oder unter der Bedingung, dass im Falle eines ungesättigten Restes, die definierte Mindestanzahl an Kohlenstoffatomen 2 beträgt, die Reste gesättigt oder ungesättigt sein können oder für Sulfanyl stehen; oder
für einen gegebenenfalls einfach oder mehrfach, gleich oder verschieden durch Halogen, Cyano, Nitro, Hydroxy,

Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls substituiertes $(C_3-C_6)$Cycloalkyl, substituierten und gegebenenfalls durch Heteroatome aus der Reihe O, S oder N unterbrochenen gesättigten oder ungesättigten drei- bis sechsgliedrigen Ring,
W für Wasserstoff oder Halogen steht;
V für Sauerstoff, Schwefel oder einen gegebenenfalls substituierten Stickstoff stehen;
X, Y und Z, jeweils unabhängig voneinander,
für Wasserstoff, Halogen, Hydroxy, Amino, Cyano, Nitro, OCN, SCN, $SF_5$, stehen; oder
für Tri$(C_1-C_6)$alkylsilyl, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxycarbonyl$(C_1-C_6)$alkyl, $(C_1-C_6)$Alkoxy$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, $(C_1-C_6)$Alkoxy, Halogen$(C_1-C_6)$alkoxy, Cyano$(C_1-C_6)$alkoxy, Hydroxycarbonyl$(C_1-C_6)$alkoxy, $(C_1-C_6)$Alkoxycarbonyl$(C_1-C_6)$alkoxy, $(C_1-C_6)$Alkoxy$(C_1-C_6)$alkoxy, $(C_1-C_6)$Alkylhydroxyimino, $(C_1-C_6)$Alkoxyimino, $(C_1-C_6)$Alkyl$(C_1-C_6)$alkoxyimino, Halogen$(C_1-C_6)$alkyl$(C_1-C_6)$alkoxyimino, $(C_1-C_6)$Alkylthio, Halogen$(C_1-C_6)$alkylthio, $(C_1-C_6)$Alkoxy$(C_1-C_6)$alkylthio, $(C_1-C_6)$Alkylthio$(C_1-C_6)$alkyl, $(C_1-C_6)$Alkylsulfinyl, Halogen$(C_1-C_6)$alkylsulfinyl, $(C_1-C_6)$Alkoxy$(C_1-C_6)$alkylsulfinyl, $(C_1-C_6)$Alkylsulfinyl$(C_1-C_6)$alkyl, $(C_1-C_6)$Alkylsulfonyl, Halogen$(C_1-C_6)$alkylsulfonyl, $(C_1-C_6)$Alkoxy$(C_1-C_6)$alkylsulfonyl, $(C_1-C_6)$Alkylsulfonyl$(C_1-C_6)$alkyl, $(C_1-C_6)$Alkylsulfonyloxy, $(C_1-C_6)$Alkylcarbonyl, Halogen$(C_1-C_6)$alkylcarbonyl, Carboxyl, $(C_1-C_6)$Alkylcarbonyloxy, $(C_1-C_6)$Alkoxycarbonyl, Halogen$(C_1-C_6)$alkoxycarbonyl, Aminocarbonyl, $(C_1-C_6)$Alkylaminocarbonyl, Di$(C_1-C_6)$alkylaminocarbonyl, $(C_2-C_6)$Alkenylaminocarbonyl, Di$(C_2-C_6)$alkenylaminocarbonyl, $(C_3-C_6)$Cyclo$(C_1-C_6)$alkylaminocarbonyl, $(C_1-C_6)$Alkylsulfonylamino, Aminosulfonyl, $(C_1-C_6)$Alkylaminosulfonyl, Di$(C_1-C_6)$alkylaminosulfonyl, $(C_1-C_6)$Alkylsulfoximino, Aminothiocarbonyl, $(C_1-C_6)$Alkylaminothiocarbonyl oder Di$(C_1-C_6)$alkylaminothiocarbonyl stehen, wobei alle vorgenannten Reste gegebenenfalls durch Halogen substituiert sein können; oder
für Phenyl$(C_1-C_3)$alkyl, Phenoxy, Phenyl$(C_1-C_3)$alkyloxy, Phenoxy$(C_1-C_3)$alkyl, Phenylthio, Phenylthio$(C_1-C_3)$alkyl, Phenylsulfinyl, Phenylsulfonyl, Hetaryl$(C_1-C_3)$alkyl, Hetaryloxy, Hetaryl$(C_1-C_3)$alkyloxy, Hetarylthio, Hetarylsulfinyl oder Hetarylsulfonyl stehen, wobei alle vorgenannten Reste gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für $(C_3-C_6)$Cycloalkyl$(C_1-C_3)$alkyl, $(C_3-C_6)$Cycloalkyloxy, $(C_3-C_6)$Cycloalkyl$(C_1-C_3)$alkoxy, $(C_3-C_6)$Cycloalkylthio, $(C_3-C_6)$Cycloalkyl$(C_1-C_3)$alkylthio, $(C_3-C_6)$Cycloalkylsulfinyl, $(C_3-C_6)$Cycloalkyl$(C_1-C_3)$alkylsulfinyl, $(C_3-C_6)$Cycloalkylsulfonyl, $(C_3-C_6)$Cycloalkyl$(C_1-C_3)$alkylsulfonyl oder $(C_3-C_8)$Cycloalkenyl stehen, wobei die vorgenannten Reste jeweils gegebenenfalls einfach bis dreifach durch Halogen, Cyano, Nitro, Amino, Hydroxy, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl substituiert sein können; oder
für NR'R" stehen,
wobei R' und R" unabhängig voneinander

für Wasserstoff, Cyano, $(C_1-C_6)$Alkyl, Halogen$(C_1-C_6)$alkyl, Cyano$(C_1-C_6)$alkyl, Hydroxy$(C_1-C_6)$alkyl, $(C_1-C_6)$Alkoxy$(C_1-C_6)$alkyl, $(C_1-C_6)$Alkylthio$(C_1-C_6)$alkyl, $(C_2-C_6)$Alkenyl, Halogen$(C_2-C_6)$alkenyl, Cyano$(C_2-C_6)$alkenyl, $(C_2-C_6)$Alkinyl, Halogen$(C_2-C_6)$alkinyl, Cyano$(C_2-C_6)$alkinyl, Acyl oder $(C_1-C_6)$Alkoxycarbonyl stehen; oder
R' und R" gemeinsam mit dem N-Atom, an dem sie gebunden sind, einen gegebenenfalls gleich oder verschieden durch Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, substituierten und gegebenenfalls durch Heteroatome aus der Reihe O, S oder N unterbrochenen gesättigten oder ungesättigten fünf- bis siebengliedrigen Ring bilden können; oder
für einen $(C_3-C_6)$Cycloalkyl, Oxetan, Oxolan, Oxan, $(C_3-C_8)$Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden mit einem Substituenten S1 substituiert sein kann, welcher ausgewählt wird aus der Gruppe, bestehend aus Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, stehen;

oder X und Z, oder Y und Z, können die folgenden 5 oder 6-gliedrigen Ring ausbilden, die gegebenenfalls gleich

oder verschieden durch Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl, subsitutiert sind,

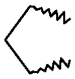 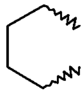 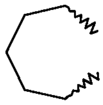 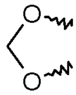 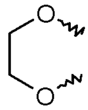 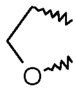 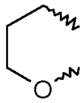

oder X und Z, oder Y und Z, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl,

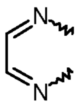 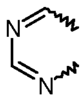 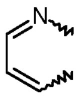 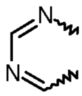 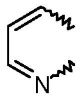 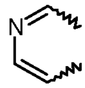

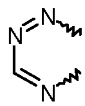 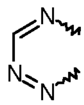 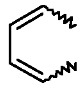

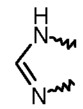 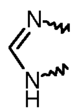 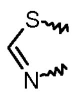

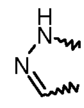 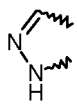 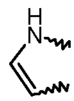

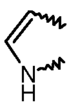 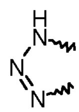 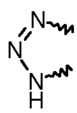

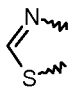 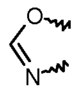 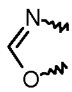

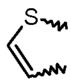 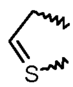 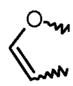 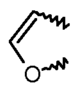

und
n für die Zahl 0 oder 1 steht.

**18.** Verbindung nach Anspruch 17, in welcher
die Substruktur der Formel (I-G) für eine Substruktur (I-G-1) steht,

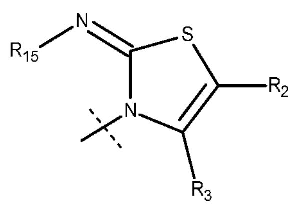

(I-G-1)

wobei

$R^2$ und $R^3$ unabhängig voneinander für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_1-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder für $(C_3-C_6)$Cycloalkyl, $(C_3-C_6)$Cycloalkenyl, Azetidinyl, Azolidinyl, Azinanyl, Oxetanyl, Oxolanyl, Oxanyl, Thiethanyl, Thiolanyl, Thianyl, Phenyl, Pyrimidyl, Pyridazinyl, Pyrazinyl, Triazinyl, Thiazolyl, Thiazadiazolyl, Oxazolyl, Oxadiazolyl, Pyrazolyl, Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

$R^{15}$ für Wasserstoff steht; oder

für $(C_1-C_6)$Alkyl, $(C_3-C_6)$Cycloalkyl$(C_1-C_3)$alkyl, $(C_2-C_6)$Alkenyl, $(C_2-C_6)$Alkinyl, Halogen$(C_2-C_6)$alkyl, Cyano$(C_1-C_6)$alkyl, $(C_1-C_6)$Alkoxy$(C_1-C_6)$alkyl oder Phenyl$(C_1-C_3)$alkyl steht, wobei die vorgenannten Reste jeweils gegebenenfalls einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können; oder

für $(C_3-C_6)$Cycloalkyl, $(C_3-C_6)$Cycloalkenyl, Oxetan, Oxolan, Oxan, Phenyl, Pyrimidyl, Pyridazinyl, Pyrazinyl, Triazinyl, Thiazolyl, Thiazadiazolyl, Oxazolyl, Oxadiazolyl, Pyrazolyl oder Triazolyl, wobei die vorgenannten Reste jeweils einfach bis dreifach durch Halogen, Cyano, Nitro, Hydroxy, Amino, $(C_1-C_6)$Alkyl, Halogen$(C_1-C_6)$Alkyl, $(C_1-C_6)$Alkoxy, Halogen$(C_1-C_6)$alkoxy, $(C_1-C_6)$Alkylsulfinyl, $(C_1-C_6)$Alkylsulfanyl, $(C_1-C_6)$Alkylsulfonyl, Halo$(C_1-C_6)$alkylsulfinyl, Halo$(C_1-C_6)$alkylsulfanyl, Halo$(C_1-C_6)$alkylsulfonyl oder gegebenenfalls durch Methyl, Fluor, Chlor, Cyano substituiertes Cyclopropyl substituiert sein können, steht;

W für Wasserstoff oder Halogen, insbesondere Fluor oder Chlor, steht;

X, Y und Z unabhängig voneinander für Wasserstoff, Fluor, Chlor, Brom, Cyano, Nitro, Amino, Hydroxy, $(C_1-C_4)$Alkyl, $(C_1-C_4)$Halogenalkyl, $(C_2-C_4)$Alkenyl, $(C_2-C_4)$Alkinyl, $(C_1-C_4)$Alkoxy, $(C_1-C_4)$Halogenalkoxy oder Aminothiocarbonyl stehen;

oder für ein Benzyl, Phenoxy, Phenylthio, Cyclopropylmethyl, Cyclopropyloxy oder Cyclopropylthio, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen;

oder für einen Cyclopropyl, Cyclobutyl, Cyclopentyl, Cyclohexyl oder Phenyl, der gegebenenfalls einfach oder mehrfach, gleich oder verschieden substituiert ist mit Fluor, Chlor, Brom, Cyano, Nitro, Hydroxy, Amino, Methyl, Ethyl, Trifluoromethyl, Difluoromethyl, Trifluoroethyl, Difluoroethyl, Methoxy, Ethoxy, Trifluoromethoxy, Trifluoroethoxy oder gegebenenfalls durch Methyl, Fluor, Chlor, Cyano substituiertes Cyclopropyl stehen; und

n für die Zahl 0 oder 1 steht.

**19.** Wirkstoffzusammensetzung enthaltend mindestens eine Verbindung der allgemeinen Formel (I) gemäß einem der Ansprüche 1 bis 18 und mindestens einen weiteren insektiziden, akariziden oder nematiziden Wirkstoff ausgewählt aus der Gruppe bestehend aus

(1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder

Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifosmethyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos,

Tetrachlorvinphos, Thiometon, Triazophos, Trichlorfon und Vamidothion.
(2) GABA-gesteuerte Chlorid-Kanal-Antagonisten, wie beispielsweise Cyclodien-organochlorine, z.B. Chlordane und Endosulfan; oder
Phenylpyrazole (Fiprole), z.B. Ethiprole und Fipronil.
(3) Natrium-Kanal-Modulatoren / Spannungsabhängige Natrium-Kanal-Blocker, wie beispielsweise
Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder
DDT; oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam; oder
Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
Avermectine/Milbemycine, z.B. Abamectin, Emamectin-benzoat, Lepimectin und Milbemectin.
(7) Juvenilhormon-Imitatoren, wie beispielsweise
Juvenilhormon-Analoge, z.B. Hydroprene, Kinoprene und Methoprene; oder Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
Organozinnverbindungen, z.B. Azocyclotin, Cyhexatin und Fenbutatin-oxid; oder
Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
METI-Akarizide, z.B. Fenazaquin, Fenpyroximate, Pyrimidifen, Pyridaben, Tebufenpyrad und Tolfenpyrad; oder Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise
Diamide, z.B. Chlorantraniliprole und Flubendiamide;

weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:

3-Brom-N-{2-brom-4-chlor-6-[(1-cyclopropylethyl)carbamoyl]phenyl}-1-(3-chlorpyridin-2-yl)-1H-pyrazol-5-carboxamid 4-{[(6-Brompyrid-3-yl)methyl](2-fluorethyl)amino}furan-2(5H)-on, 4-{[(6-Fluorpyrid-3-yl)methyl](2,2-difluorethyl)amino}furan-2(5H)-on, 4-{[(2-Chlor-1,3-thiazol-5-yl)methyl](2-fluorethyl)amino}furan-2(5H)-on, 4-{[(6-Chlorpyrid-3-yl)methyl](2-fluorethyl)amino}furan-2(5H)-on, Flupyradifurone, 4-{[(6-Chlor-5-fluorpyrid-3-yl)methyl](methyl)amino}furan-2(5H)-on, 4-{[(5,6-Dichlorpyrid-3-yl)methyl](2-fluorethyl)amino}furan-2(5H)-on, 4-{[(6-Chlor-5-fluorpyrid-3-yl)methyl](cyclopropyl)amino}furan-2(5H)-on, 4-{[(6-Chlorpyrid-3-yl)methyl](cyclopropyl)amino}furan-2(5H)-on, 4-{[(6-Chlorpyrid-3-yl)methyl](methyl)amino}furan-2(5H)-on, {[1-(6-Chlorpyridin-3-yl)ethyl](methyl)oxido-$\lambda^4$-sulfanyliden}cyanamid und seine Diastereomere {[(1R)-1-(6-Chlorpyridin-3-yl)ethyl](methyl)oxido-$\lambda^4$-sulfanyliden}cyanamid (A) und {[(1S)-1-(6-Chlorpyridin-3-yl)ethyl](methyl)oxido-$\lambda^4$-sulfanyliden}cyanamid (B) sowie Sulfoxaflor und seine Diastereomere [(R)-Methyl(oxido){(1R)-1-[6-(trifluormethyl)pyridin-3-yl]ethyl}-$\lambda^4$-sulfanyliden]cyanamid (A1) und [(S)-Methyl(oxido){(1S)-1-[6-(trifluormethyl)pyridin-3-yl]ethyl}-$\lambda^4$-sulfanyliden]cyanamid (A2), bezeichnet als Diastereomerengruppe A, [(R)-Methyl(oxido){(1S)-1-[6-(trifluormethyl)pyridin-3-yl]ethyl}-$\lambda^4$-sulfanyliden]cyanamid (B1) und [(S)-Methyl(oxido) {(1R)-1-[6-(trifluormethyl)pyridin-3-yl]ethyl}-$\lambda^4$-sulfanyliden]cyanamid (B2), bezeichnet als Diastereomerengruppe B und 11-(4-Chlor-2,6-dimethylphenyl)-12-hydroxy-1,4-dioxa-9-azadispiro[4.2.4.2]tetradec-11-en-10-on, 3-(4'-Fluor-2,4-dimethylbiphenyl-3-yl)-4-hydroxy-8-oxa-1-azaspiro[4.5]dec-3-en-2-on, 1-{2-Fluor-4-methyl-5-[(2,2,2-trifluorethyl)sulfinyl]phenyl}-3-(trifluormethyl)-1H-1,2,4-triazol-5-amin, [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat, 2-Cyan-3-(difluormethoxy)-N,N-dimethylbenzolsulfonamid, 2-Cyan-3-(difluormethoxy)-N-methylbenzolsulfonamid, 2-Cyan-3-(difluormethoxy)-N-ethylbenzolsulfonamid, 4-(Difluormethoxy)-N-ethyl-N-methyl-l,2-benzothiazol-3-amin-1,1-dioxid, N-[1-(2,3-Dimethylphenyl)-2-(3,5-dimethylphenyl)ethyl]-4,5-dihydro-1,3-thiazol-2-amin, {1'-[(2E)-3-(4-Chlorphenyl)prop-2-en-1-yl]-5-fluorspiro[indol-3,4'-piperidin]-1(2H)-yl}(2-chlorpyridin-4-yl)methanon, 3-(2,5-Dimethylphenyl)-4-hydroxy-8-methoxy-1,8-diazaspiro[4.5]dec-3-en-2-on, 3-(2,5-Dimethylphenyl)-8-methoxy-2-oxo-1,8-diazaspiro[4.5]dec-3-en-4-yl-ethylcarbonat, 4-(But-2-in-1-yloxy)-6-(3,5-dimethylpiperidin-1-yl)-5-fluorpyrimidin, (2,2,3,3,4,4,5,5-Octafluorpentyl)(3,3,3-trifluorpropyl)malononitril, (2,2,3,3,4,4,5,5-Octafluorpentyl)(3,3,4,4,4-pentafluorbutyl)malononitril, 8-[2-(Cyclopropylmethoxy)-4-(trifluormethyl)phenoxy]-3-[6-(trifluormethyl)pyridazin-3-yl]-3-azabicyclo[3.2.1]octan, Flometoquin, PF1364 (CAS-Reg.Nr. 1204776-60-2), 5-[5-(3,5-Dichlorphenyl)-5-(trifluormethyl)-4,5-dihydro-1,2-oxazol-3-yl]-2-(1H-1,2,4-triazol-1-yl)benzonitril, 5-[5-(2-Chlorpyridin-4-yl)-5-(trifluormethyl)-4,5-dihydro-1,2-oxazol-3-yl]-2-(1H-1,2,4-triazol-1-yl)benzonitril, 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid, 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino)-1,3-oxazol-2(5H)-on, NNI-0711, 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid, Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat, Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat, Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat, Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat, Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat, (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin, 2-{6-[2-(5-Fluorpyridin-3-yl)-1,3-thiazol-5-yl]pyridin-2-yl}pyrimidin, 2-{6-[2-(Pyridin-3-yl)-1,3-thiazol-5-yl]pyridin-2-yl}pyrimidin, 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid, 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid, N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid, N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid, (1E)-N-[(6-Chlorpyridin-3-yl)methyl]-N'-cyan-N-(2,2-difluorethyl)ethanimidamid, N-[2-(5-Amino-1,3,4-thiadiazol-2-yl)-4-chlor-6-methylphenyl]-3-brom-1-(3-chlorpyridin-2-yl)-1H-pyrazol-5-carboxamid und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat,

und/oder mindestens einen weiteren fungiziden Wirkstoff ausgewählt aus der Gruppe bestehend aus

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazolp (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).

(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30)N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.

(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2), (3.9) Famoxadon (131807-57-3), (3.10) Fenamidon (161326-34-7), (3.11) Fenoxystrobin (918162-02-4), (3.12) Fluoxastrobin (361377-29-9), (3.13) Kresoxim-Methyl (143390-89-0), (3.14) Metominostrobin (133408-50-1), (3.15) Orysastrobin (189892-69-1), (3.16) Picoxystrobin (117428-22-5), (3.17) Pyraclostrobin (175013-18-0), (3.18) Pyrametostrobin (915410-70-7), (3.19) Pyraoxystrobin (862588-11-2), (3.20) Pyribencarb (799247-52-2), (3.21) Triclopyricarb (902760-40-1), (3.22) Trifloxystrobin (141517-21-7), (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({ [(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).

(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).

(5) Verbindungen mit Multisite-Aktivität, wie beispielsweise (5.1) Bordeauxmischung (8011-63-0), (5.2) Captafol (2425-06-1), (5.3) Captan (133-06-2), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).

(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).

(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7).

(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).

(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).

(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).

(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6).

(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).

(13) Inhibitoren der Signaltransduktion, wie beispielsweise (13.1) Chlozolinat (84332-86-5), (13.2) Fenpiclonil

(74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).

(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).

(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4), (15.75) 5-Fluor-2-[(4-methylbenzyl)oxy]pyrimidin-4-amin (1174376-25-0), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl] -2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.

(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid, (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl{6-[({[(Z)-(1-methyl-1H-tetrazol-5-yl)(phenyl)methylen]amino}oxy)methyl]pyridin-2-yl}carbamat.

**20.** Agrochemische Zusammensetzungen, **dadurch gekennzeichnet, dass** sie mindestens eine Verbindung der Formel (I) gemäß den Ansprüchen 1 bis 18 oder eine Zusamensetzung gemäß Anspruch 19, sowie Streckmittel und/oder oberflächenaktive Stoffe enthalten.

**21.** Verfahren zur Herstellung agrochemischer Zusammensetzungen, **dadurch gekennzeichnet, dass** man Verbindungen der Formel (I) gemäß den Ansprüchen 1 bis 18 oder eine Zusammensetzung gemäß Anspruch 19 mit Streckmitteln und/oder oberflächenaktiven Stoffen vermischt.

**22.** Verfahren zur Bekämpfung tierischer Schädlinge, **dadurch gekennzeichnet, dass** man Verbindungen der Formel (I) gemäß den Ansprüchen 1 bis 18 oder eine Zusammensetzung gemäß Anspruch 19 auf tierische Schädlinge und/oder deren Lebensraum einwirken lässt, wobei Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

**23.** Verwendung von Verbindungen der Formel (I) gemäß einem der Ansprüche 1 bis 18 oder einer Zusammensetzung gemäß Anspruch 19 zur Bekämpfung tierischer Schädlinge im Pflanzenschutz, im Materialschutz und/oder im veterinärmedizinischen Sektor, wobei Verwendungen in Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

## Claims

**1.** Compounds of the formula (I)

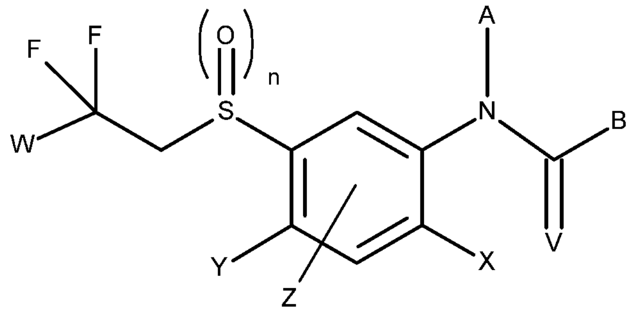

(I)

in which

A and B together with the atoms to which they are bonded are a substructure selected from the group consisting of

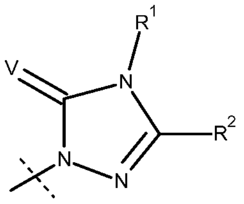

(I-A)

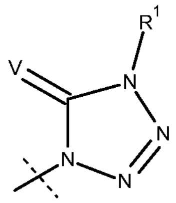

(I-B)

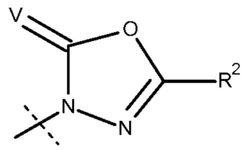

(I-C)

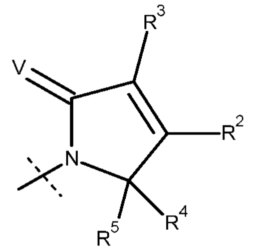

(I-D)

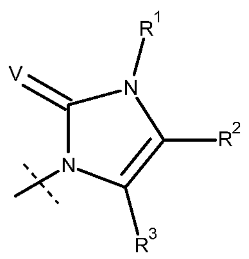

(I-E)

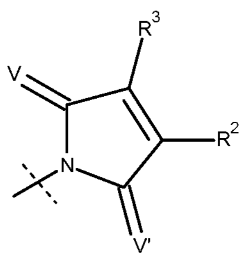

(I-F)

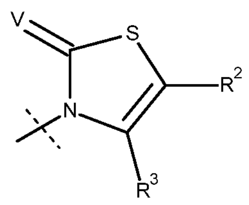

(I-G)

where

$R^1$ is hydrogen, cyano or nitro; or

is alkyl, cycloalkylalkyl, haloalkyl, cyanoalkyl, hydroxyalkyl, alkoxyalkyl, aminoalkyl, alkoxycarbonylalkyl, alkylsulphanylalkyl, alkylsulphinylalkyl, alkylsulphonylalkyl, haloalkylsulphanylalkyl, haloalkylsulphinylalkyl, haloalkylsulphonylalkyl, alkoxyalkylsulphanylalkyl, alkoxyalkylsulphinylalkyl, alkoxyalkylsulphonylalkyl, phenylalkyl, phenoxyalkyl, phenylsulphanylalkyl, phenylsulphinylalkyl, phenylsulphonylalkyl, hetarylalkyl, hetaryloxyalkyl, hetarylthioalkyl, where the aforementioned radicals may each be saturated or unsaturated and/or optionally substituted; or

is optionally substituted saturated or unsaturated cycloalkyl which may optionally be interrupted by one or more heteroatoms; or

is alkylcarbonyl, haloalkylcarbonyl, hydroxyalkylcarbonyl, alkoxyalkylcarbonyl, phenylcarbonyl, hetarylcarbonyl, alkoxycarbonyl, haloalkoxycarbonyl, aryloxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or is carbonyl or carboxyl; or

is optionally substituted phenyl or optionally substituted hetaryl; or

is alkoxy, haloalkoxy, cycloalkyloxy, aryloxy, arylalkyloxy or carbonyloxy, where the aforementioned radicals

may optionally be substituted, or is hydroxyl; or

is alkylamino, haloalkylamino, dihaloalkylamino, dialkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulphonylamino, or arylsulphonylamino, where the aforementioned radicals may each be saturated or unsaturated and/or optionally substituted, or is amino; or

is alkylsulphanyl, alkylsulphinyl, alkylsulphonyl, haloalkylsulphanyl, haloalkylsulphinyl, haloalkylsulphanyl, cycloalkylsulphanyl, cycloalkylsulphinyl, cycloalkylsulphonyl, cycloalkylalkylsulphanyl, cycloalkylalkylsulphinyl, cycloalkylalkylsulphonyl, arylsulphanyl, arylsulphinyl, arylsulphonyl, arylalkylsulphanyl, arylalkylsulphinyl, arylalkylsulphonyl, aminosulphonyl, alkylaminosulphonyl, dialkylaminosulphonyl or arylaminosulphonyl, where the aforementioned radicals may each be saturated or unsaturated and/or optionally substituted, or is sulphanyl; and

$R^2$, $R^3$, $R^4$ and $R^5$ each independently

are hydrogen, cyano, halogen or nitro; or

are alkyl, cycloalkylalkyl, haloalkyl, cyanoalkyl, hydroxyalkyl, alkoxyalkyl, aminoalkyl, alkoxycarbonylalkyl, alkylsulphanylalkyl, alkylsulphinylalkyl, alkylsulphonylalkyl, haloalkylsulphanylalkyl, haloalkylsulphinylalkyl, haloalkylsulphonylalkyl, alkoxyalkylsulphanylalkyl, alkoxyalkylsulphinylalkyl, alkoxyalkylsulphonylalkyl, phenylalkyl, phenoxyalkyl, phenylsulphanylalkyl, phenylsulphinylalkyl, phenylsulphonylalkyl, hetarylalkyl, hetaryloxyalkyl, hetarylthioalkyl, where the aforementioned radicals may each be saturated or unsaturated and/or optionally substituted; or

are optionally substituted saturated or unsaturated cycloalkyl which may optionally be interrupted by one or more heteroatoms; or

are alkylcarbonyl, haloalkylcarbonyl, hydroxyalkylcarbonyl, alkoxyalkylcarbonyl, phenylcarbonyl, hetarylcarbonyl, alkoxycarbonyl, haloalkoxycarbonyl, aryloxycarbonyl, aminocarbonyl, alkylaminocarbonyl, dialkylaminocarbonyl, cycloalkylaminocarbonyl, dicycloalkylaminocarbonyl, cycloalkyl(alkyl)aminocarbonyl, arylaminocarbonyl, diarylaminocarbonyl, alkyl(aryl)aminocarbonyl, cycloalkyl(aryl)aminocarbonyl, alkylaminothiocarbonyl, dialkylaminothiocarbonyl or aminothiocarbonyl, where the aforementioned radicals may each be saturated or unsaturated and/or optionally substituted, or are carbonyl or carboxyl; or

are optionally substituted phenyl or optionally substituted hetaryl; or

are alkoxy, haloalkoxy, alkoxyalkoxy, aryloxy, arylalkyloxy, cycloalkyloxy, cycloalkylalkyloxy or carbonyloxy, where the aforementioned radicals may be saturated or unsaturated and/or optionally substituted, or are hydroxyl; or

are alkylamino, dialkylamino, haloalkylamino, dihaloalkylamino, cycloalkylamino, dicycloalkylamino, cycloalkyl(alkyl)amino, arylamino, diarylamino, hetarylamino, dihetarylamino, alkyl(aryl)amino, cycloalkyl(aryl)amino, alkylcarbonylamino, arylcarbonylamino, alkoxycarbonylamino, aryloxycarbonylamino, alkylcarbamoylamino, arylcarbamoylamino, alkylsulphonylamino, or arylsulphonylamino, where the aforementioned radicals may each be saturated or unsaturated and/or optionally substituted, or are amino; or

are alkylsulphanyl, alkylsulphinyl, alkylsulphonyl, haloalkylsulphanyl, haloalkylsulphinyl, haloalkylsulphonyl, cycloalkylsulphanyl, cycloalkylsulphinyl, cycloalkylsulphonyl, cycloalkylalkylsulphanyl, cycloalkylalkylsulphinyl, cycloalkylalkylsulphonyl, arylsulphanyl, arylsulphinyl, arylsulphonyl, arylalkylsulphanyl, arylalkylsulphinyl, arylalkylsulphonyl, aminosulphonyl, alkylaminosulphonyl, dialkylaminosulphonyl or arylaminosulphonyl, where the aforementioned radicals may each be saturated or unsaturated and/or optionally substituted, or are sulphanyl; or

$R^4$ and $R^5$ together with the atom to which they are bonded may form an optionally substituted, saturated or unsaturated three- to eight-membered ring optionally interrupted by one or more heteroatoms which are selected independently from the group consisting of 0, S and N; or

or $R^2$ is a saturated or unsaturated cycle optionally interrupted by one or more heteroatoms which are each selected from the group consisting of O S and N, which may optionally be substituted;

W is hydrogen or halogen;

if a substructure of one of the formulae (I-B) to (I-G) is present,

V and V' are each independently oxygen, sulphur or an optionally substituted nitrogen;

and if a substructure of formula (I-A) is present,

V is oxygen or sulphur;

X, Y and Z, each independently,

are hydrogen, halogen, hydroxyl, amino, cyano, nitro, OCN, SCN, $SF_5$; or

are trialkylsilyl, alkyl, haloalkyl, cyanoalkyl, hydroxyalkyl, alkoxycarbonylalkyl, alkoxyalkyl, alkenyl, haloalkenyl, cyanoalkenyl, alkynyl, haloalkynyl, cyanoalkynyl, alkoxy, haloalkoxy, cyanoalkoxy, hydroxycarbonylalkoxy, alkoxycarbonylalkoxy, alkoxyalkoxy, alkylhydroxyimino, alkoxyimino, alkylalkoxyimino, haloalkylalkoxyimino, alkylthio, haloalkylthio, alkoxyalkylthio, alkylthioalkyl, alkylsulphinyl, haloalkylsulphinyl, alkoxyalkylsulphinyl, alkylsulphinylalkyl, alkylsulphonyl, haloalkylsulphonyl, alkoxyalkylsulphonyl, alkylsulphonylalkyl, alkylsulphonyloxy, alkylcarbonyl, haloalkylcarbonyl, carboxyl, alkylcarbonyloxy, alkoxycarbonyl, haloalkoxycarbonyl, aminocarbonyl, alkylaminocarbonyl, dialkylaminocarbonyl, alkenylaminocarbonyl, dialkenylaminocarbonyl, cycloalkylaminocarbonyl, alkylsulphonylamino, aminosulphonyl, alkylaminosulphonyl, dialkylaminosulphonyl, alkylsulphoximino, aminothiocarbonyl, alkylaminothiocarbonyl or dialkylaminothiocarbonyl, where all the aforementioned radicals may optionally be substituted; or

are phenylalkyl, phenoxy, phenylalkyloxy, phenoxyalkyl, phenylthio, phenylthioalkyl, phenylsulphinyl, phenylsulphonyl, hetarylalkyl, hetaryloxy, hetarylalkyloxy, hetarylthio, hetarylsulphinyl or hetarylsulphonyl, where all the aforementioned radicals may optionally be substituted; or

are cycloalkylalkyl, cycloalkyloxy, cycloalkylalkoxy, cycloalkylthio, cycloalkylalkylthio, cycloalkylsulphinyl, cycloalkylalkylsulphinyl, cycloalkylsulphonyl, cycloalkylalkylsulphonyl or cycloalkenyl, where all the aforementioned radicals may each optionally be substituted; or

are NR’R",

where R’ and R" each independently

are hydrogen, cyano, alkyl, haloalkyl, cyanoalkyl, hydroxyalkyl, alkoxyalkyl, alkylthioalkyl, alkenyl, haloalkenyl, cyanoalkenyl, alkynyl, haloalkynyl, cyanoalkynyl, acyl or alkoxycarbonyl; or

R’ and R" together with the nitrogen atom to which they are bonded may form an optionally substituted, saturated or unsaturated five- to eight-membered ring optionally interrupted by one or more heteroatoms which are selected independently from the group consisting of O S and N; or

are a 3- to 6-membered saturated, partly saturated or aromatic ring which may optionally contain one to three heteroatoms which are selected independently from the group consisting of O, S and N, and which may optionally be substituted;

or X and Z, or Y and Z, together with the carbon atoms to which they are bonded, form a 5- or 6-membered ring which is optionally substituted and optionally interrupted by one or more heteroatoms which are selected independently from the group consisting of 0, S, N and CO;

and

n is the number 0 or 1.

**2.** Compound according to Claim 1 in which

A and B together with the atoms to which they are bonded are a substructure selected from the group consisting of I-A to I-G;

$R^1$ is hydrogen, cyano or nitro; or

is $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulphanyl$(C_1-C_6)$alkyl, $(C_1-C_6)$alkylsulphinyl$(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl$(C_1-C_6)$alkyl, $(C_1-C_6)$alkoxy$(C_1-C_6)$alkylsulphanyl$(C_1-C_6)$alkyl, $(C_1-C_6)$alkoxy$(C_1-C_6)$alkylsulphinyl$(C_1-C_6)$alkyl, $(C_1-C_6)$alkoxy $(C_1-C_6)$alkylsulphonyl$(C_1-C_6)$alkyl, phenyl$(C_1-C_6)$alkyl, phenoxy$(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl$(C_1-C_6)$alkyl, hetaryloxy$(C_1-C_6)$alkyl, hetarylsulphanyl$(C_1-C_6)$alkyl, hetarylsulphinyl$(C_1-C_6)$alkyl, hetarylsulphonyl$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated $(C_3-C_6)$cycloalkyl which may optionally be interrupted by one or more heteroatoms; or

is $(C_1-C_6)$alkylcarbonyl, halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl$(C_1-C_6)$alkylaminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

is optionally substituted phenyl or optionally substituted hetaryl; or

is $(C_1-C_6)$alkoxy, halo $(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or is hydroxyl; or

is $(C_1-C_6)$alkylamino, halo $(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl$(C_1-C_6)$alkylamino, arylamino, diarylamino, hetarylamino, dihetarylamino, $(C_1-C_6)$alkyl(aryl)amino, $(C_3-C_6)$cycloalkyl (aryl) amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino,

$(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or

is $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphanyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl$(C_1-C_6)$alkylsulphinyl, $(C_3-C_6)$cycloalkyl$(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di$(C_1-C_6)$alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; and

$R^2$, $R^3$, $R^4$ and $R^5$ each independently

are hydrogen, cyano, halogen or nitro; or

are $(C_1-C_6)$alkyl, $(C_3-C_6)$cycloalkyl$(C_1-C_6)$alkyl, halo$(C_1-C_6)$alkyl, cyano$(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulphanyl$(C_1-C_6)$alkyl, $(C_1-C_6)$alkylsulphinyl$(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl$(C_1-C_6)$alkyl, $(C_1-C_6)$alkoxy$(C_1-C_6)$alkylsulphanyl$(C_1-C_6)$alkyl, $(C_1-C_6)$alkoxy$(C_1-C_6)$alkylsulphinyl$(C_1-C_6)$alkyl, $(C_1-C_6)$alkoxy$(C_1-C_6)$alkylsulphonyl$(C_1-C_6)$alkyl, phenyl$(C_1-C_6)$alkyl, phenoxy$(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl$(C_1-C_6)$alkyl, hetaryloxy$(C_1-C_6)$alkyl, hetarylthio$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

are optionally substituted saturated or unsaturated $(C_3-C_6)$cycloalkyl which may optionally be interrupted by one or more heteroatoms; or

are $(C_1-C_6)$alkylcarbonyl, halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl$(C_1-C_6)$alkylaminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or are carbonyl or carboxyl; or

are optionally substituted phenyl or optionally substituted hetaryl; or

are $(C_1-C_6)$alkoxy, halo$(C_1-C_6)$alkoxy, $(C_1-C_6)$alkoxy$(C_1-C_6)$alkoxy, aryloxy, aryl$(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or are hydroxyl; or

are $(C_1-C_6)$alkylamino, di$(C_1-C_6)$alkylamino, halo$(C_1-C_6)$alkylamino, dihalo$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl) amino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or are amino; or

are $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphonyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl $(C_1-C_6)$alkylsulphinyl, $(C_3-C_6)$cycloalkyl $(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di$(C_1-C_6)$alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or are sulphanyl; or

$R^4$ and $R^5$ together with the atom to which they are bonded may form an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo$(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo $(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of O, S and

N; or

or $R^2$ is an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo $(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo $(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of O, S and N;

W is hydrogen or halogen;

if a substructure of one of the formulae (I-B) to (I-G) is present,

V and V' are each independently oxygen, sulphur or an optionally substituted nitrogen;

if a substructure of formula (I-A) is present,

V is oxygen or sulphur;

X, Y and Z are each independently as defined above; and

n is the number 0 or 1.

**3.** Compound according to Claim 2 in which

X, Y and Z each independently are hydrogen, halogen, hydroxyl, amino, cyano, nitro, OCN, SCN, $SF_5$; or

are tri$(C_1-C_6)$alkylsilyl, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxycarbonyl$(C_1-C_6)$alkyl, $(C_1-C_6)$alkoxy$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, $(C_1-C_6)$alkoxy, halo$(C_1-C_6)$alkoxy, cyano$(C_1-C_6)$alkoxy, hydroxycarbonyl$(C_1-C_6)$alkoxy, $(C_1-C_6)$alkoxycarbonyl$(C_1-C_6)$alkoxy, $(C_1-C_6)$alkoxy$(C_1-C_6)$alkoxy, $(C_1-C_6)$alkylhydroxyimino, $(C_1-C_6)$alkoxyimino, $(C_1-C_6)$alkyl$(C_1-C_6)$alkoxyimino, halo$(C_1-C_6)$alkyl$(C_1-C_6)$alkoxyimino, $(C_1-C_6)$alkylthio, halo$(C_1-C_6)$alkylthio, $(C_1-C_6)$alkoxy$(C_1-C_6)$alkylthio, $(C_1-C_6)$alkylthio$(C_1-C_6)$alkyl, $(C_1-C_6)$alkylsulphinyl, halo$(C_1-C_6)$alkylsulphinyl, $(C_1-C_6)$alkoxy$(C_1-C_6)$alkylsulphinyl, $(C_1-C_6)$alkylsulphinyl$(C_1-C_6)$alkyl, $(C_1-C_6)$alkylsulphonyl, halo$(C_1-C_6)$alkylsulphonyl, $(C_1-C_6)$alkoxy$(C_1-C_6)$alkylsulphonyl, $(C_1-C_6)$alkylsulphonyl$(C_1-C_6)$alkyl, $(C_1-C_6)$alkylsulphonyloxy, $(C_1-C_6)$alkylcarbonyl, halo$(C_1-C_6)$alkylcarbonyl, carboxyl, $(C_1-C_6)$alkylcarbonyloxy, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_2-C_6)$alkenylaminocarbonyl, di$(C_2-C_6)$alkenylaminocarbonyl, $(C_3-C_6)$cycloalkylaminocarbonyl, $(C_1-C_6)$alkylsulphonylamino, aminosulphonyl, $(C_1-C_6)$alkylaminosulphonyl, di$(C_1-C_6)$alkylaminosulphonyl, $(C_1-C_6)$alkylsulphoximino, aminothiocarbonyl, $(C_1-C_6)$alkylaminothiocarbonyl or di $(C_1-C_6)$alkylaminothiocarbonyl, where all the aforementioned radicals may optionally be substituted by halogen; or

are phenyl$(C_1-C_3)$alkyl, phenoxy, phenyl$(C_1-C_3)$alkyloxy, phenoxy$(C_1-C_3)$alkyl, phenylthio, phenylthio$(C_1-C_3)$alkyl, phenylsulphinyl, phenylsulphonyl, hetaryl$(C_1-C_3)$alkyl, hetaryloxy, hetaryl$(C_1-C_3)$alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are $(C_3-C_6)$cycloalkyl$(C_1-C_3)$alkyl, $(C_3-C_6)$cycloalkyloxy, $(C_3-C_6)$cycloalkyl$(C_1-C_3)$alkoxy, $(C_3-C_6)$cycloalkylthio, $(C_3-C_6)$cycloalkyl$(C_1-C_3)$alkylthio, $(C_3-C_6)$cycloalkylsulphinyl, $(C_3-C_6)$cycloalkyl$(C_1-C_3)$alkylsulphinyl, $(C_3-C_6)$cycloalkylsulphonyl, $(C_3-C_6)$cycloalkyl$(C_1-C_3)$alkylsulphonyl or $(C_3-C_8)$cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are NR'R"

where R' and R" each independently

are hydrogen, cyano, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxy$(C_1-C_6)$alkyl, $(C_1-C_6)$alkylthio$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, acyl or $(C_1-C_6)$alkoxycarbonyl; or

R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five-to seven-membered ring optionally interrupted by heteroatoms from the group of O, S and N; or

are a $(C_3-C_6)$cycloalkyl, oxetanyl, oxolanyl, oxanyl, $(C_3-C_8)$cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

or X and Z, or Y and Z, may form the following 5 or 6-membered rings which are optionally substituted identically

or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

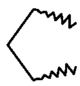 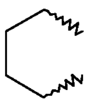 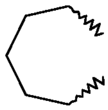 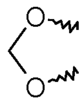 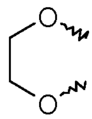 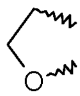 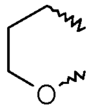

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

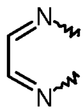 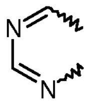 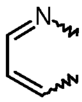 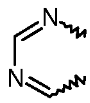 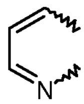 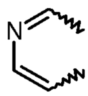

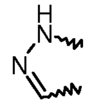 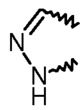 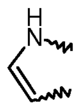

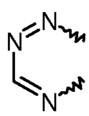 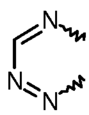 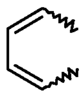

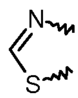 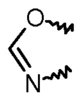 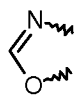

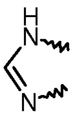 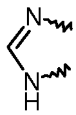 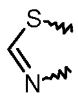

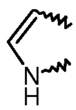 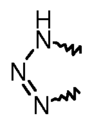 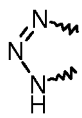

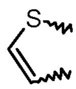 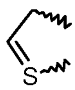 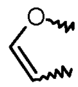 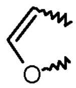

4. Compound according to any of Claims 1 to 3, in which A and B together with the atoms to which they are bonded are a substructure of the formula (I-A)

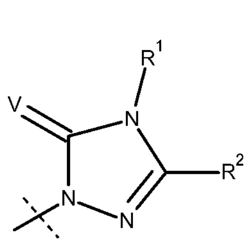

(I-A)

where

$R^1$ is hydrogen, cyano or nitro; or is $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl,

$(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulphanyl$(C_1-C_6)$alkyl, $(C_1-C_6)$alkylsulphinyl$(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl$(C_1-C_6)$alkyl, $(C_1-C_6)$alkoxy$(C_1-C_6)$alkylsulphanyl$(C_1-C_6)$alkyl, $(C_1-C_6)$alkoxy$(C_1-C_6)$alkylsulphinyl$(C_1-C_6)$alkyl, $(C_1-C_6)$alkoxy$(C_1-C_6)$alkylsulphonyl$(C_1-C_6)$alkyl, phenyl$(C_1-C_6)$alkyl, phenoxy$(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl$(C_1-C_6)$alkyl, hetaryloxy$(C_1-C_6)$alkyl, hetarylsulphanyl$(C_1-C_6)$alkyl, hetarylsulphinyl$(C_1-C_6)$alkyl, hetarylsulphonyl$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated $(C_3-C_6)$cycloalkyl which may optionally be interrupted by one or more heteroatoms; or

is $(C_1-C_6)$alkylcarbonyl, halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

is optionally substituted phenyl or optionally substituted hetaryl; or

is $(C_1-C_6)$alkoxy, halo$(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or is hydroxyl; or

is $(C_1-C_6)$alkylamino, halo$(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl) amino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or

is $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphanyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl $(C_1-C_6)$alkylsulphinyl, $(C_3-C_6)$cycloalkyl $(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di$(C_1-C_6)$alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; and

$R^2$ is hydrogen, cyano, halogen or nitro; or

is $(C_1-C_6)$alkyl, $(C_3-C_6)$cycloalkyl $(C_1-C_6)$ alkyl, halo$(C_1-C_6)$alkyl, cyano $(C_1-C_6)$alkyl, hydroxy $(C_1-C_6)$alkyl, $(C_1-C_6)$alkoxy $(C_1-C_6)$ alkyl, amino $(C_1-C_6)$ alkyl, $(C_1-C_6)$alkoxycarbonyl $(C_1-C_6)$alkyl, $(C_1-C_6)$alkylsulphanyl $(C_1-C_6)$alkyl, $(C_1-C_6)$ alkylsulphinyl $(C_1-C_6)$ alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$alkyl, $(C_1-C_6)$alkoxy $(C_1-C_6)$alkylsulphanyl $(C_1-C_6)$alkyl, $(C_1-C_6)$alkoxy $(C_1-C_6)$alkylsulphinyl $(C_1-C_6)$alkyl, $(C_1-C_6)$alkoxy $(C_1-C_6)$alkylsulphonyl $(C_1-C_6)$alkyl, phenyl $(C_1-C_6)$alkyl, phenoxy $(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl $(C_1-C_6)$alkyl, hetaryloxy $(C_1-C_6)$alkyl, hetarylthio$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated $(C_3-C_6)$cycloalkyl which may optionally be interrupted by one or more heteroatoms; or

is $(C_1-C_6)$ alkylcarbonyl, halo $(C_1-C_6)$ alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, $(C_1-C_6)$alkoxycarbonyl, halo $(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl ($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined

minimum number of carbon atoms is 2, the radicals may be saturated or unsaturated, or is carbonyl or carboxyl; or is optionally substituted phenyl or optionally substituted hetaryl; or

is ($C_1$-$C_6$) alkoxy, halo ($C_1$-$C_6$) alkoxy, ($C_1$-$C_6$) alkoxy ($C_1$-$C_6$)alkoxy, aryloxy, aryl ($C_1$-$C_6$)alkyloxy, ($C_3$-$C_6$)cycloalkyloxy, ($C_3$-$C_6$)cycloalkyl ($C_1$-$C_6$) alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or is hydroxyl; or

is ($C_1$-$C_6$) alkylamino, di($C_1$-$C_6$) alkylamino, halo($C_1$-$C_6$)alkylamino, dihalo ($C_1$-$C_6$) alkylamino, ($C_3$-$C_6$)cycloalkylamino, di($C_3$-$C_6$)cycloalkylamino, ($C_3$-$C_6$)cycloalkyl (($C_1$-$C_6$)alkyl) amino, arylamino, diarylamino, hetarylamino, dihetarylamino, ($C_1$-$C_6$)alkyl (aryl) amino, ($C_3$-$C_6$)cycloalkyl (aryl) amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or

is ($C_1$-$C_6$) alkylsulphanyl, ($C_1$-$C_6$) alkylsulphinyl, ($C_1$-$C_6$)alkylsulphonyl, halo ($C_1$-$C_6$) alkylsulphanyl, halo($C_1$-$C_6$)alkylsulphinyl, halo ($C_1$-$C_6$) alkylsulphonyl, ($C_3$-$C_6$)cycloalkylsulphanyl, ($C_3$-$C_6$)cycloalkylsulphinyl, ($C_3$-$C_6$)cycloalkylsulphonyl, ($C_3$-$C_6$)cycloalkyl($C_1$-$C_6$)alkylsulphanyl, ($C_3$-$C_6$)cycloalkyl ($C_1$-$C_6$)alkylsulphinyl, ($C_3$-$C_6$)cycloalkyl ($C_1$-$C_6$)alkylsulphonyl, arylsulphanyl, arylsulphinyl, arylsulphonyl, aryl($C_1$-$C_6$)alkylsulphanyl, aryl($C_1$-$C_6$)alkylsulphinyl, aryl ($C_1$-$C_6$)alkylsulphonyl, aminosulphonyl, ($C_1$-$C_6$)alkylaminosulphonyl, di($C_1$-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; or

is an optionally singly or multiply, identically or differently halogen-, cyano-, nitro-, hydroxyl-, amino-, ($C_1$-$C_6$)alkyl-, halo ($C_1$-$C_6$)alkyl-, ($C_1$-$C_6$)alkoxy-, halo ($C_1$-$C_6$) alkoxy-, ($C_1$-$C_6$) alkylsulphinyl-, ($C_1$-$C_6$)alkylsulphanyl-, ($C_1$-$C_6$) alkylsulphonyl-, halo($C_1$-$C_6$)alkylsulphinyl-, halo ($C_1$-$C_6$)alkylsulphanyl-, halo($C_1$-$C_6$)alkylsulphonyl- or optionally substituted ($C_3$-$C_6$)cycloalkyl-substituted, saturated or unsaturated three- to six-membered ring optionally interrupted by heteroatoms from the group of 0, S and N;

W is hydrogen or halogen;

V is oxygen or sulphur;

X, Y and Z each independently are hydrogen, halogen, hydroxyl, amino, cyano, nitro, OCN, SCN, $SF_5$; or

are tri($C_1$-$C_6$)alkylsilyl, ($C_1$-$C_6$)alkyl, halo ($C_1$-$C_6$)alkyl, cyano ($C_1$-$C_6$) alkyl, hydroxy ($C_1$-$C_6$) alkyl, ($C_1$-$C_6$)alkoxycarbonyl ($C_1$-$C_6$)alkyl, ($C_1$-$C_6$)alkoxy ($C_1$-$C_6$)alkyl, ($C_2$-$C_6$)alkenyl, halo ($C_2$-$C_6$)alkenyl, cyano ($C_2$-$C_6$)alkenyl, ($C_2$-$C_6$)alkynyl, halo ($C_2$-$C_6$)alkynyl, cyano ($C_2$-$C_6$)alkynyl, ($C_1$-$C_6$) alkoxy, halo ($C_1$-$C_6$) alkoxy, cyano ($C_1$-$C_6$) alkoxy, hydroxycarbonyl ($C_1$-$C_6$)alkoxy, ($C_1$-$C_6$)alkoxycarbonyl ($C_1$-$C_6$)alkoxy, ($C_1$-$C_6$) alkoxy ($C_1$-$C_6$) alkoxy, ($C_1$-$C_6$)alkylhydroxyimino, ($C_1$-$C_6$) alkoxyimino, ($C_1$-$C_6$)alkyl ($C_1$-$C_6$)alkoxyimino, halo ($C_1$-$C_6$)alkyl ($C_1$-$C_6$)alkoxyimino, ($C_1$-$C_6$) alkylthio, halo ($C_1$-$C_6$) alkylthio, ($C_1$-$C_6$) alkoxy ($C_1$-$C_6$) alkylthio, ($C_1$-$C_6$) alkylthio ($C_1$-$C_6$)alkyl, ($C_1$-$C_6$) alkylsulphinyl, halo($C_1$-$C_6$)alkylsulphinyl, ($C_1$-$C_6$) alkoxy ($C_1$-$C_6$)alkylsulphinyl, ($C_1$-$C_6$)alkylsulphinyl ($C_1$-$C_6$)alkyl, ($C_1$-$C_6$)alkylsulphonyl, halo ($C_1$-$C_6$) alkylsulphonyl, ($C_1$-$C_6$) alkoxy ($C_1$-$C_6$)alkylsulphonyl, ($C_1$-$C_6$) alkylsulphonyl ($C_1$-$C_6$) alkyl, ($C_1$-$C_6$) alkylsulphonyloxy, ($C_1$-$C_6$) alkylcarbonyl, halo($C_1$-$C_6$)alkylcarbonyl, carboxyl, ($C_1$-$C_6$) alkylcarbonyloxy, ($C_1$-$C_6$)alkoxycarbonyl, halo ($C_1$-$C_6$)alkoxycarbonyl, aminocarbonyl, ($C_1$-$C_6$)alkylaminocarbonyl, di($C_1$-$C_6$)alkylaminocarbonyl, ($C_2$-$C_6$)alkenylaminocarbonyl, di($C_2$-$C_6$)alkenylaminocarbonyl, ($C_3$-$C_6$)cyclo ($C_1$-$C_6$)alkylaminocarbonyl, ($C_1$-$C_6$)alkylsulphonylamino, aminosulphonyl, ($C_1$-$C_6$)alkylaminosulphonyl, di($C_1$-$C_6$)alkylaminosulphonyl, ($C_1$-$C_6$)alkylsulphoximino, aminothiocarbonyl, ($C_1$-$C_6$)alkylaminothiocarbonyl or di($C_1$-$C_6$)alkylaminothiocarbonyl, where all the aforementioned radicals may optionally be substituted by halogen; or

are phenyl ($C_1$-$C_3$)alkyl, phenoxy, phenyl ($C_1$-$C_3$)alkyloxy, phenoxy($C_1$-$C_3$)alkyl, phenylthio, phenylthio($C_1$-$C_3$)alkyl, phenylsulphinyl, phenylsulphonyl, hetaryl($C_1$-$C_3$)alkyl, hetaryloxy, hetaryl($C_1$-$C_3$)alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are ($C_3$-$C_6$)cycloalkyl ($C_1$-$C_3$)alkyl, ($C_3$-$C_6$)cycloalkyloxy, ($C_3$-$C_6$)cycloalkyl ($C_1$-$C_3$)alkoxy, ($C_3$-$C_6$)cycloalkylthio, ($C_3$-$C_6$)cycloalkyl ($C_1$-$C_3$)alkylthio, ($C_3$-$C_6$)cycloalkylsulphinyl, ($C_3$-$C_6$)cycloalkyl ($C_1$-$C_3$)alkylsulphinyl, ($C_3$-$C_6$)cycloalkylsulphonyl, ($C_3$-$C_6$)cycloalkyl ($C_1$-$C_3$)alkylsulphonyl or ($C_3$-$C_8$)cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are NR'R''

where R' and R'' each independently

are hydrogen, cyano, $(C_1-C_6)$alkyl, halo $(C_1-C_6)$alkyl, cyano $(C_1-C_6)$alkyl, hydroxy $(C_1-C_6)$alkyl, $(C_1-C_6)$alkoxy $(C_1-C_6)$alkyl, $(C_1-C_6)$alkylthio $(C_1-C_6)$alkyl, $(C_2-C_6)$alkenyl, halo $(C_2-C_6)$alkenyl, cyano $(C_2-C_6)$alkenyl, $(C_2-C_6)$alkynyl, halo $(C_2-C_6)$alkynyl, cyano $(C_2-C_6)$alkynyl, acyl or $(C_1-C_6)$alkoxycarbonyl; or
R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five-to seven-membered ring optionally interrupted by heteroatoms from the group of 0, S and N; or
are a $(C_3-C_6)$cycloalkyl, oxetanyl, oxolanyl, oxanyl, $(C_3-C_8)$cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or X and Z, or Y and Z, may form the following 5 or 6-membered rings which are optionally substituted identically or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

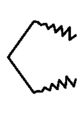 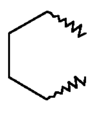 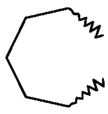 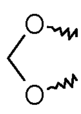 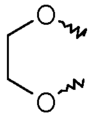 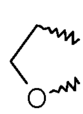 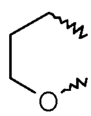

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

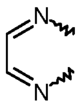 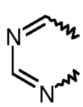 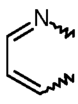 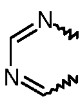 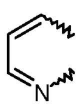 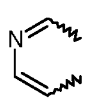

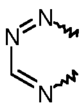 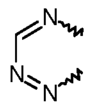 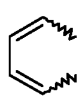

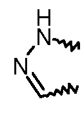 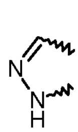 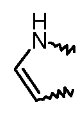

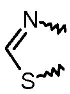 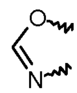 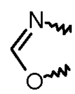

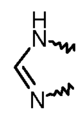 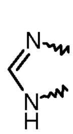 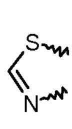

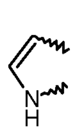 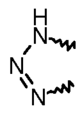 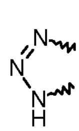

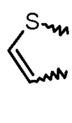 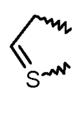 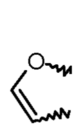 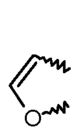

and
n is the number 0 or 1.

**5.** Compound according to Claim 4 in which the substructure of the formula (I-A) is a substructure which is selected

from the group consisting of

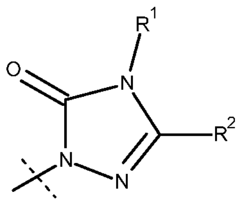

(I-A-1)

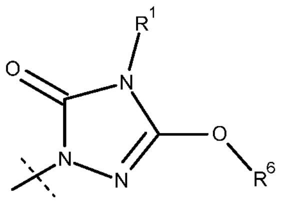

(I-A-2)

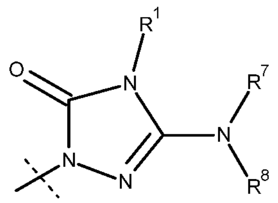

(I-A-3)

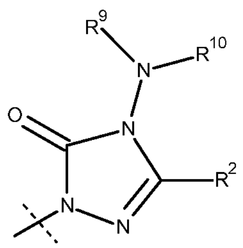

(I-A-4)

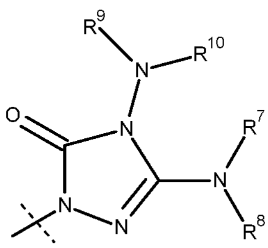

(I-A-5)

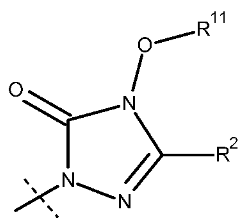

(I-A-6)

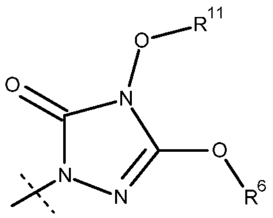

(I-A-7)

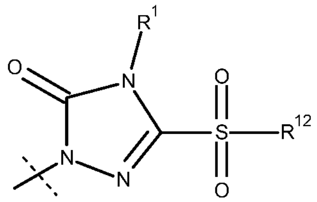

(I-A-8)

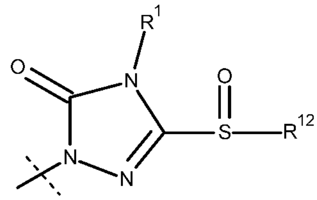

(I-A-9)

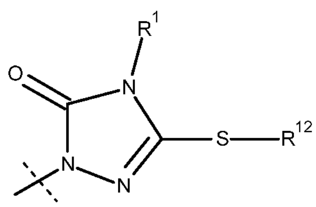

(I-A-10)

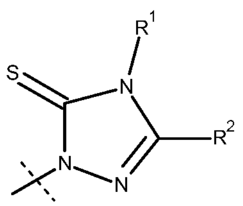

(I-A-11)

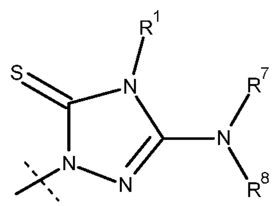

(I-A-12)

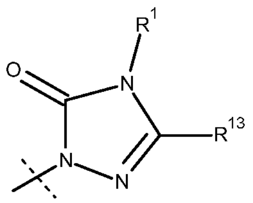

(I-A-13)

where

$R^1$ is hydrogen; or

is $(C_1-C_6)$alkyl, $(C_3-C_5)$cycloalkyl $(C_1-C_3)$alkyl, $(C_2-C_6)$alkenyl, $(C_2-C_6)$alkynyl, halo $(C_2-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or

is $(C_3-C_6)$cycloalkyl, $(C_3-C_6)$cycloalkenyl, oxetanyl, oxolanyl, oxanyl, phenyl, pyridyl, pyrimidyl, pyridazinyl, pyrazinyl, triazinyl, thiazolyl, thiazadiazolyl, oxazolyl, oxadiazolyl, pyrazolyl or triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl;

$R^2$ is hydrogen; or

is $(C_1-C_6)$alkyl, $(C_3-C_6)$cycloalkyl $(C_1-C_3)$alkyl, $(C_2-C_6)$alkenyl, $(C_2-C_6)$alkynyl, halo $(C_1-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or

is $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or

is carboxyl;

$R^6$ is hydrogen; or

is $(C_1-C_6)$alkyl, $(C_3-C_6)$cycloalkyl $(C_1-C_3)$alkyl, $(C_2-C_6)$alkenyl, $(C_2-C_6)$alkynyl, halo $(C_1-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or

is $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl;

$R^7$ and $R^8$ are each independently hydrogen, $(C_1-C_6)$alkyl, halo $(C_2-C_6)$alkyl, cyano $(C_1-C_6)$alkyl or $(C_3-C_6)$cycloalkyl, where the aforementioned radicals may optionally each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano- or cyclopropyl-substituted cyclopropyl; or both together with the nitrogen atom to which they are bonded are an optionally substituted triazolinone, triazolidinethione, tetrazolinone, tetrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine 1-oxide, thiomorpholine 1,1-dioxide, piperazine, N-methylpiperazine or N-ethylpiperazine;

$R^9$ and $R^{10}$ are each independently hydrogen, $(C_1-C_6)$alkyl, halo $(C_2-C_6)$alkyl, cyano $(C_1-C_6)$alkyl, $(C_3-C_6)$cycloalkyl, or together are $(C_2-C_6)$alkylidene, where the aforementioned radicals may each optionally be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano- or cyclopropyl-substituted cyclopropyl;

or both together with the nitrogen atom to which they are bonded are an optionally substituted triazolinone, triazolidinethione, tetrazolinone, tetrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine 1-oxide, thiomorpholine 1,1-dioxide, piperazine, N-methylpiperazine or N-ethylpiperazine;

$R^{11}$ is hydrogen; or
is $(C_1-C_6)$alkyl, $(C_3-C_6)$cycloalkyl $(C_1-C_3)$alkyl, $(C_2-C_6)$alkenyl, $(C_2-C_6)$alkynyl, halo $(C_1-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or
is $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl;
$R^{12}$ is $(C_1-C_6)$alkyl, $(C_3-C_6)$cycloalkyl, $(C_2-C_6)$alkenyl, halo $(C_1-C_6)$alkyl, cyano $(C_1-C_6)$alkyl, phenyl $(C_1-C_3)$alkyl or $(C_3-C_6)$cycloalkyl $(C_1-C_6)$alkyl, where the aforementioned radicals may each optionally be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl;
$R^{13}$ is halogen;
W is hydrogen or halogen (especially fluorine or chlorine);
X, Y and Z are each independently hydrogen, fluorine, chlorine, bromine, cyano, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;
or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or are a cyclopropyl, cyclobutyl, cyclopentyl,
cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; and
n is the number 0 or 1.

**6.** Compound according to Claim 4 or 5 in which
the substructure of the formula (I-A) is a substructure which is selected from the group consisting of

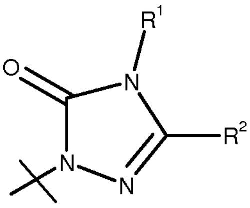
(I-A-1)

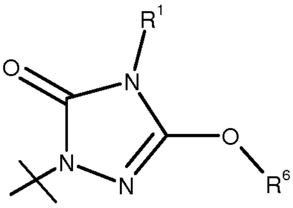
(I-A-2)

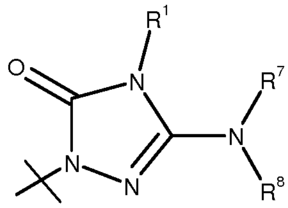
(I-A-3)

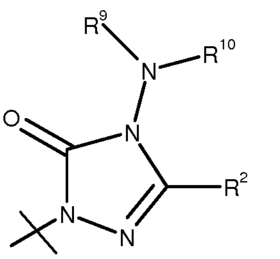

(I-A-4)

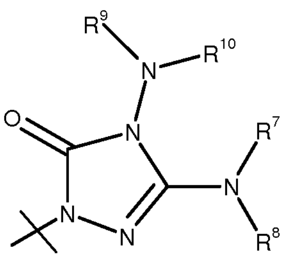

(I-A-5)

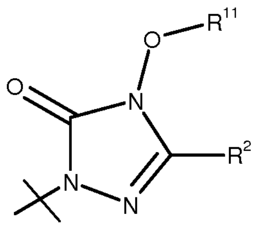

(I-A-6)

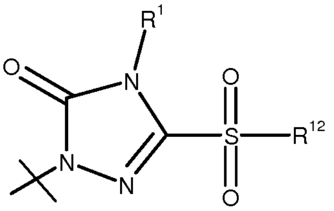

(I-A-8)

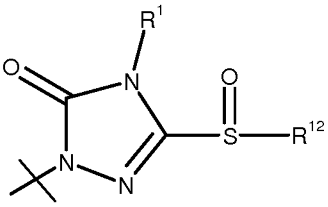

(I-A-9)

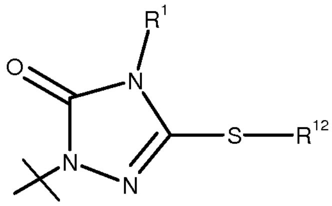

(I-A-10)

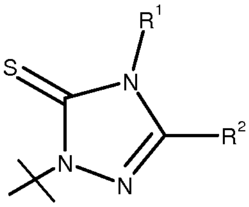

(I-A-11)

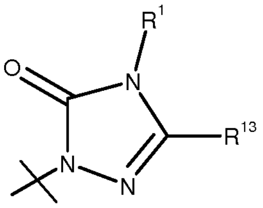

(I-A-13)

where

$R^1$ is hydrogen; or

is $(C_1\text{-}C_6)$alkyl, $(C_3\text{-}C_6)$cycloalkyl $(C_1\text{-}C_3)$alkyl, $(C_2\text{-}C_6)$alkenyl, $(C_2\text{-}C_6)$alkynyl, halo $(C_2\text{-}C_6)$alkyl, cyano $(C_1\text{-}C_6)$alkyl, $(C_1\text{-}C_6)$alkoxy $(C_1\text{-}C_6)$alkyl or phenyl $(C_1\text{-}C_3)$alkyl, where the aforementioned radicals may each optionally be mono- to trisubstituted by halogen, cyano, nitro, hydroxyl, amino, $(C_1\text{-}C_6)$ alkyl, halo $(C_1\text{-}C_6)$ alkyl, $(C_1\text{-}C_6)$alkoxy, halo $(C_1\text{-}C_6)$alkoxy, $(C_1\text{-}C_6)$alkylsulphinyl, $(C_1\text{-}C_6)$alkylsulphanyl, $(C_1\text{-}C_6)$ alkylsulphonyl, halo$(C_1\text{-}C_6)$alkylsulphinyl, halo $(C_1\text{-}C_6)$ alkylsulphanyl, halo$(C_1\text{-}C_6)$alkylsulphonyl or optionally methyl-, fluorine-, chlorine-, cyano-substituted cyclopropyl; or

is $(C_3\text{-}C_6)$cycloalkyl, $(C_3\text{-}C_6)$cycloalkenyl, azetidinyl, azolidinyl, azinanyl, oxetanyl, oxolanyl, oxanyl, thiethanyl, thiolanyl, thianyl, phenyl, pyridyl, pyrimidyl, pyridazinyl, pyrazinyl, triazinyl, thiazolyl, thiazadiazolyl, oxazolyl, oxadiazolyl, pyrazolyl or triazolyl, where the aforementioned radicals may each be mono- to trisubstituted by halogen, cyano, nitro, hydroxyl, amino, $(C_1\text{-}C_6)$alkyl, halo $(C_1\text{-}C_6)$alkyl, $(C_1\text{-}C_6)$alkoxy, halo $(C_1\text{-}C_6)$alkoxy, $(C_1\text{-}C_6)$alkylsulphinyl, $(C_1\text{-}C_6)$ alkylsulphanyl, $(C_1\text{-}C_6)$alkylsulphonyl, halo $(C_1\text{-}C_6)$ alkylsulphinyl, halo$(C_1\text{-}C_6)$alkylsulphanyl, halo$(C_1\text{-}C_6)$alkylsulphonyl or optionally methyl-, fluorine-, chlorine-, cyano-substituted cyclopropyl;

$R^2$ is hydrogen; or

is $(C_1\text{-}C_6)$alkyl, $(C_3\text{-}C_6)$cycloalkyl $(C_1\text{-}C_3)$alkyl, $(C_2\text{-}C_6)$alkenyl, $(C_2\text{-}C_6)$alkynyl, halo $(C_1\text{-}C_6)$alkyl, cyano $(C_1\text{-}C_6)$alkyl, $(C_1\text{-}C_6)$alkoxy $(C_1\text{-}C_6)$alkyl or phenyl $(C_1\text{-}C_3)$alkyl, where the aforementioned radicals may each optionally be mono- to trisubstituted by halogen, cyano, nitro, hydroxyl, amino, $(C_1\text{-}C_6)$ alkyl, halo $(C_1\text{-}C_6)$ alkyl, $(C_1\text{-}C_6)$alkoxy, halo $(C_1\text{-}C_6)$alkoxy, $(C_1\text{-}C_6)$alkylsulphinyl, $(C_1\text{-}C_6)$alkylsulphanyl, $(C_1\text{-}C_6)$ alkylsulphonyl, halo$(C_1\text{-}C_6)$alkylsulphinyl, halo $(C_1\text{-}C_6)$ alkylsulphanyl, halo$(C_1\text{-}C_6)$alkylsulphonyl or optionally methyl-, fluorine-, chlorine-, cyano-substituted cyclopropyl; or

is $(C_3\text{-}C_6)$cycloalkyl, $(C_3\text{-}C_6)$cycloalkenyl, azetidinyl, azolidinyl, azinanyl, oxetanyl, oxolanyl, oxanyl, thiethanyl,

thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or
is carboxyl;
$R^6$ is hydrogen; or
is $(C_1-C_6)$alkyl, $(C_3-C_6)$cycloalkyl $(C_1-C_3)$alkyl, $(C_2-C_6)$alkenyl, $(C_2-C_6)$alkynyl, halo $(C_1-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or
is $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl;
$R^7$ and $R^8$ are each independently hydrogen, $(C_1-C_6)$alkyl, halo $(C_2-C_6)$alkyl, cyano $(C_1-C_6)$alkyl or $(C_3-C_6)$cycloalkyl, where the aforementioned radicals may optionally each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano- or cyclopropyl-substituted cyclopropyl;
or both together with the nitrogen atom to which they are bonded are an optionally substituted triazolinone, triazolidinethione, tetrazolinone, tetrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine 1-oxide, thiomorpholine 1,1-dioxide, piperazine, N-methylpiperazine or N-ethylpiperazine;
$R^9$ and $R^{10}$ are each independently hydrogen, $(C_1-C_6)$alkyl, halo $(C_2-C_6)$alkyl, cyano $(C_1-C_6)$alkyl, $(C_3-C_6)$cycloalkyl, or together are $(C_2-C_6)$alkylidene, where the aforementioned radicals may each optionally be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano- or cyclopropyl-substituted cyclopropyl;
or both together with the nitrogen atom to which they are bonded are an optionally substituted triazolinone, triazolidinethione, tetrazolinone, tetrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine 1-oxide, thiomorpholine 1,1-dioxide, piperazine, N-methylpiperazine or N-ethylpiperazine;
$R^{11}$ is hydrogen; or
is $(C_1-C_6)$alkyl, $(C_3-C_6)$cycloalkyl $(C_1-C_3)$alkyl, $(C_2-C_6)$alkenyl, $(C_2-C_6)$alkynyl, halo $(C_1-C_6)$alkyl, cyano $(C_1-C_6)$alkyl, $(C_1-C_6)$alkoxy $(C_1-C_6)$alkyl or phenyl $(C_1-C_3)$alkyl, where the aforementioned radicals may each optionally be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl; or
is $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$ alkyl, halo $(C_1-C_6)$ alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl;
$R^{12}$ is $(C_1-C_6)$alkyl, $(C_3-C_6)$cycloalkyl, $(C_2-C_6)$alkenyl, halo $(C_1-C_6)$alkyl, cyano $(C_1-C_6)$alkyl, phenyl $(C_1-C_3)$alkyl or $(C_3-C_6)$cycloalkyl $(C_1-C_6)$alkyl, where the aforementioned radicals may each optionally be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$ alkylsulphonyl, halo$(C_1-C_6)$alkylsulphinyl, halo $(C_1-C_6)$ alkylsulphanyl, halo$(C_1-C_6)$alkylsulphonyl or optionally methyl-, fluorine-, chlorine-, cyano-substituted cyclopropyl;
$R^{13}$ is halogen;

W is hydrogen or halogen, especially fluorine or chlorine;

X, Y and Z are each independently hydrogen, fluorine, chlorine, bromine, cyano, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;

or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

or are a cyclopropyl, cyclobutyl, cyclopentyl, cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; and

n is the number 0 or 1.

**7.** Compound according to any of Claims 1 to 3, in which
A and B together with the atoms to which they are bonded are a substructure of the formula (I-B)

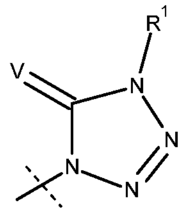

(I-B)

where

$R^1$ is hydrogen, cyano or nitro; or

is $(C_1-C_6)$alkyl, $(C_3-C_6)$cycloalkyl $(C_1-C_6)$alkyl, halo$(C_2-C_6)$alkyl, cyano $(C_1-C_6)$alkyl, hydroxy $(C_1-C_6)$alkyl, $(C_1-C_6)$alkoxy $(C_1-C_6)$alkyl, amino $(C_1-C_6)$alkyl, $(C_1-C_6)$alkoxycarbonyl $(C_1-C_6)$alkyl, $(C_1-C_6)$alkylsulphanyl $(C_1-C_6)$alkyl, $(C_1-C_6)$ alkylsulphinyl $(C_1-C_6)$ alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$ alkyl, $(C_1-C_6)$alkoxy $(C_1-C_6)$alkylsulphanyl $(C_1-C_6)$ alkyl, $(C_1-C_6)$alkoxy $(C_1-C_6)$alkylsulphinyl $(C_1-C_6)$ alkyl, $(C_1-C_6)$alkoxy $(C_1-C_6)$alkylsulphonyl $(C_1-C_6)$ alkyl, phenyl $(C_1-C_6)$ alkyl, phenoxy $(C_1-C_6)$ alkyl, phenylsulphanyl$(C_1-C_6)$ alkyl, phenylsulphinyl$(C_1-C_6)$alkyl, phenylsulphonyl$(C_1-C_6)$alkyl, hetaryl $(C_1-C_6)$ alkyl, hetaryloxy $(C_1-C_6)$ alkyl, hetarylsulphanyl$(C_1-C_6)$alkyl, hetarylsulphinyl$(C_1-C_6)$alkyl, hetarylsulphonyl$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated $(C_3-C_6)$cycloalkyl which may optionally be interrupted by one or more heteroatoms; or

is $(C_1-C_6)$ alkylcarbonyl, halo $(C_1-C_6)$ alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, $(C_1-C_6)$alkoxycarbonyl, halo $(C_1-C_6)$alkoxycarbonyl, aryloxycarbonyl, aminocarbonyl, $(C_1-C_6)$alkylaminocarbonyl, di$(C_1-C_6)$ alkylaminocarbonyl, $(C_3-C_6)$cycloalkylaminocarbonyl, di$(C_3-C_6)$cycloalkylaminocarbonyl, $(C_3-C_6)$cycloalkyl $((C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or

is optionally substituted phenyl or optionally substituted hetaryl; or

is $(C_1-C_6)$ alkoxy, halo $(C_1-C_6)$ alkoxy, $(C_3-C_6)$cycloalkyloxy, aryloxy, aryl$(C_1-C_6)$alkyloxy or carbonyloxy, where the aforementioned radicals may each optionally be substituted, or is hydroxyl; or

is $(C_1-C_6)$ alkylamino, halo $(C_1-C_6)$ alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl $((C_1-C_6)$alkyl) amino, arylamino, diarylamino, hetarylamino, dihetarylamino, $(C_1-C_6)$alkyl (aryl) amino, $(C_3-C_6)$cycloalkyl (aryl) amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcar-

bamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or
is $(C_1-C_6)$alkylsulphanyl, $(C_1-C_6)$alkylsulphinyl, $(C_1-C_6)$alkylsulphonyl, halo $(C_1-C_6)$ alkylsulphanyl, halo$(C_1-C_6)$alkylsulphinyl, halo $(C_1-C_6)$ alkylsulphanyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl $(C_1-C_6)$alkylsulphinyl, $(C_3-C_6)$cycloalkyl $(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl $(C_1-C_6)$alkylsulphonyl, aminosulphonyl, $(C_1-C_6)$alkylaminosulphonyl, di(Ci-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; and
V is oxygen, sulphur or an optionally substituted nitrogen;
X, Y and Z each independently
are hydrogen, halogen, hydroxyl, amino, cyano, nitro, OCN, SCN, $SF_5$; or
are tri $(C_1-C_6)$alkylsilyl, $(C_1-C_6)$alkyl, halo $(C_1-C_6)$alkyl, cyano $(C_1-C_6)$alkyl, hydroxy $(C_1-C_6)$alkyl, $(C_1-C_6)$alkoxycarbonyl $(C_1-C_6)$alkyl, $(C_1-C_6)$alkoxy $(C_1-C_6)$alkyl, $(C_2-C_6)$alkenyl, halo $(C_2-C_6)$alkenyl, cyano $(C_2-C_6)$alkenyl, $(C_2-C_6)$alkynyl, halo $(C_2-C_6)$alkynyl, cyano $(C_2-C_6)$alkynyl, $(C_1-C_6)$alkoxy, halo $(C_1-C_6)$alkoxy, cyano $(C_1-C_6)$alkoxy, hydroxycarbonyl $(C_1-C_6)$alkoxy, $(C_1-C_6)$alkoxycarbonyl $(C_1-C_6)$alkoxy, $(C_1-C_6)$alkoxy $(C_1-C_6)$alkoxy, $(C_1-C_6)$alkylhydroxyimino, $(C_1-C_6)$ alkoxyimino, $(C_1-C_6)$alkyl $(C_1-C_6)$alkoxyimino, halo $(C_1-C_6)$alkyl $(C_1-C_6)$alkoxyimino, $(C_1-C_6)$alkylthio, halo $(C_1-C_6)$alkylthio, $(C_1-C_6)$alkoxy $(C_1-C_6)$alkylthio, $(C_1-C_6)$alkylthio $(C_1-C_6)$alkyl, $(C_1-C_6)$ alkylsulphinyl, halo$(C_1-C_6)$alkylsulphinyl, $(C_1-C_6)$alkoxy $(C_1-C_6)$alkylsulphinyl, $(C_1-C_6)$alkylsulphinyl $(C_1-C_6)$alkyl, $(C_1-C_6)$alkylsulphonyl, halo $(C_1-C_6)$ alkylsulphonyl, $(C_1-C_6)$alkoxy $(C_1-C_6)$alkylsulphonyl, $(C_1-C_6)$ alkylsulphonyl $(C_1-C_6)$ alkyl, $(C_1-C_6)$alkylsulphonyloxy, $(C_1-C_6)$alkylcarbonyl, halo$(C_1-C_6)$alkylcarbonyl, carboxyl, $(C_1-C_6)$alkylcarbonyloxy, $(C_1-C_6)$alkoxycarbonyl, halo $(C_1-C_6)$alkoxycarbonyl, aminocarbonyl, $(C_1-C_6)$alkylaminocarbonyl, di(Ci-$C_6$)alkylaminocarbonyl, $(C_2-C_6)$alkenylaminocarbonyl, di$(C_2-C_6)$alkenylaminocarbonyl, $(C_3-C_6)$cyclo $(C_1-C_6)$alkylaminocarbonyl, $(C_1-C_6)$alkylsulphonylamino, aminosulphonyl, $(C_1-C_6)$alkylaminosulphonyl, di$(C_1-C_6)$alkylaminosulphonyl, $(C_1-C_6)$alkylsulphoximino, aminothiocarbonyl, $(C_1-C_6)$alkylaminothiocarbonyl or di$(C_1-C_6)$alkylaminothiocarbonyl, where all the aforementioned radicals may optionally be substituted by halogen; or
are phenyl $(C_1-C_3)$alkyl, phenoxy, phenyl $(C_1-C_3)$alkyloxy, phenoxy$(C_1-C_3)$alkyl, phenylthio, phenylthio$(C_1-C_3)$alkyl, phenylsulphinyl, phenylsulphonyl, hetaryl$(C_1-C_3)$alkyl, hetaryloxy, hetaryl$(C_1-C_3)$alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or
are $(C_3-C_6)$cycloalkyl $(C_1-C_3)$alkyl, $(C_3-C_6)$cycloalkyloxy, $(C_3-C_6)$cycloalkyl $(C_1-C_3)$alkoxy, $(C_3-C_6)$cycloalkylthio, $(C_3-C_6)$cycloalkyl $(C_1-C_3)$alkylthio, $(C_3-C_6)$cycloalkylsulphinyl, $(C_3-C_6)$cycloalkyl $(C_1-C_3)$alkylsulphinyl, $(C_3-C_6)$cycloalkylsulphonyl, $(C_3-C_6)$cycloalkyl $(C_1-C_3)$alkylsulphonyl or $(C_3-C_8)$cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or
are NR'R''
where R' and R" each independently
are hydrogen, cyano, $(C_1-C_6)$alkyl, halo $(C_1-C_6)$alkyl, cyano $(C_1-C_6)$alkyl, hydroxy $(C_1-C_6)$alkyl, $(C_1-C_6)$alkoxy $(C_1-C_6)$alkyl, $(C_1-C_6)$alkylthio $(C_1-C_6)$alkyl, $(C_2-C_6)$alkenyl, halo $(C_2-C_6)$alkenyl, cyano $(C_2-C_6)$alkenyl, $(C_2-C_6)$alkynyl, halo $(C_2-C_6)$alkynyl, cyano $(C_2-C_6)$alkynyl, acyl or $(C_1-C_6)$alkoxycarbonyl; or
R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five-to seven-membered ring optionally interrupted by heteroatoms from the group of O, S and N; or
are a $(C_3-C_6)$cycloalkyl, oxetanyl, oxolanyl, oxanyl, $(C_3-C_8)$cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or X and Z, or Y and Z, may form the following 5 or 6-membered rings which are optionally substituted identically

or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

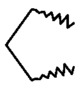 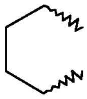 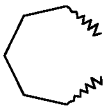 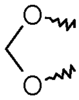 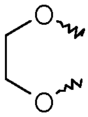 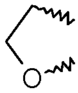 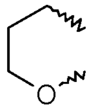

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

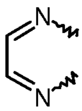 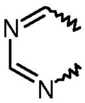 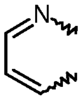 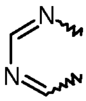 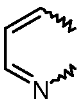 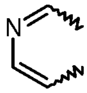

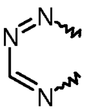 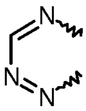 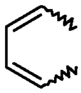 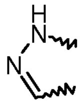 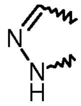 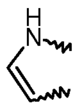

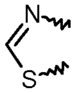 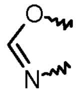 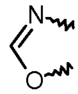

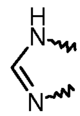 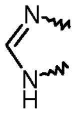 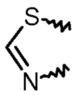

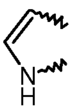 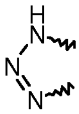 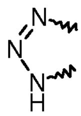

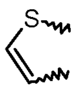 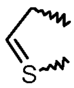 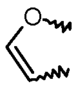 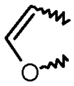

and
n is the number 0 or 1.

**8.** Compound according to Claim 7 in which
the substructure of the formula (I-B) is a substructure which is selected from the group consisting of

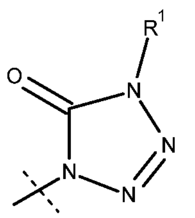

(I-B-1)

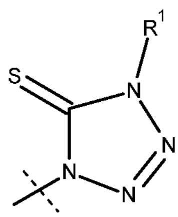

(I-B-2)

where

$R^1$ is hydrogen; or

is ($C_1$-$C_6$) alkyl, ($C_3$-$C_6$)cycloalkyl ($C_1$-$C_3$)alkyl, ($C_2$-$C_6$)alkenyl, ($C_2$-$C_6$)alkynyl, halo ($C_2$-$C_6$)alkyl, cyano ($C_1$-$C_6$)alkyl, ($C_1$-$C_6$)alkoxy ($C_1$-$C_6$)alkyl or phenyl ($C_1$-$C_3$) alkyl, where the aforementioned radicals may each optionally be mono- to trisubstituted by halogen, cyano, nitro, hydroxyl, amino, ($C_1$-$C_6$)alkyl, halo ($C_1$-$C_6$)alkyl, ($C_1$-$C_6$)alkoxy, halo ($C_1$-$C_6$) alkoxy, ($C_1$-$C_6$) alkylsulphinyl, ($C_1$-$C_6$)alkylsulphanyl, ($C_1$-$C_6$)alkylsulphonyl, halo($C_1$-$C_6$)alkylsulphinyl, halo ($C_1$-$C_6$)alkylsulphanyl, halo($C_1$-$C_6$)alkylsulphonyl or optionally methyl-, fluorine-, chlorine-, cyano-substituted cyclopropyl; or

is ($C_3$-$C_6$)cycloalkyl, ($C_3$-$C_6$)cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl or triazolyl, where the aforementioned radicals may each be mono- to trisubstituted by halogen, cyano, nitro, hydroxyl, amino, ($C_1$-$C_6$)alkyl, halo ($C_1$-$C_6$)alkyl, ($C_1$-$C_6$)alkoxy, halo ($C_1$-$C_6$)alkoxy, ($C_1$-$C_6$)alkylsulphinyl, ($C_1$-$C_6$)alkylsulphanyl, ($C_1$-$C_6$)alkylsulphonyl, halo($C_1$-$C_6$)alkylsulphinyl, halo ($C_1$-$C_6$)alkylsulphanyl, halo($C_1$-$C_6$)alkylsulphonyl or optionally methyl-, fluorine-, chlorine-, cyano-substituted cyclopropyl;

W is hydrogen or halogen, especially fluorine or chlorine;

X, Y and Z are each independently hydrogen, fluorine, chlorine, bromine, cyano, nitro, amino, hydroxyl, ($C_1$-$C_4$)alkyl, ($C_1$-$C_4$)haloalkyl, ($C_2$-$C_4$)alkenyl, ($C_2$-$C_4$)alkynyl, ($C_1$-$C_4$)alkoxy, ($C_1$-$C_4$)haloalkoxy or aminothiocarbonyl;

or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

or are a cyclopropyl, cyclobutyl, cyclopentyl, cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; and

n is the number 0 or 1.

**9.** Compound according to any of Claims 1 to 3, in which
A and B together with the atoms to which they are bonded are a substructure of the formula (I-C)

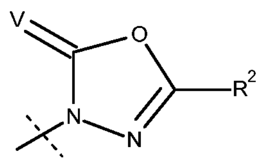

(I-C)

where

$R^2$ is hydrogen, cyano, halogen or nitro; or

is ($C_1$-$C_6$)alkyl, ($C_3$-$C_6$)cycloalkyl ($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl ($C_1$-$C_6$)alkyl, ($C_1$-$C_6$)alkylsulphanyl ($C_1$-$C_6$)alkyl, ($C_1$-$C_6$)alkylsulphinyl ($C_1$-$C_6$)alkyl, ($C_1$-$C_6$)alkylsulphonyl($C_1$-$C_6$)alkyl, halo($C_1$-$C_6$)alkylsulphanyl($C_1$-$C_6$)alkyl, halo($C_1$-$C_6$)alkylsulphinyl($C_1$-$C_6$)alkyl, halo($C_1$-$C_6$)alkylsulphonyl ($C_1$-$C_6$)alkyl, ($C_1$-$C_6$)alkoxy($C_1$-$C_6$)alkylsulphanyl ($C_1$-$C_6$)alkyl, ($C_1$-$C_6$)alkoxy($C_1$-$C_6$)alkylsulphinyl ($C_1$-$C_6$)alkyl, ($C_1$-$C_6$)alkoxy ($C_1$-$C_6$)alkylsulphonyl ($C_1$-$C_6$)alkyl, phenyl ($C_1$-$C_6$)alkyl, phenoxy ($C_1$-$C_6$)alkyl, phenylsulphanyl($C_1$-$C_6$)alkyl, phenylsulphinyl($C_1$-$C_6$)alkyl, phenylsulphonyl($C_1$-$C_6$)alkyl, hetaryl ($C_1$-$C_6$)alkyl, hetaryloxy ($C_1$-$C_6$)alkyl, hetarylthio ($C_1$-$C_6$)alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated ($C_3$-$C_6$)cycloalkyl which may optionally be interrupted by one or more heteroatoms; or

is ($C_1$-$C_6$)alkylcarbonyl, halo ($C_1$-$C_6$)alkylcarbonyl, hydroxy ($C_1$-$C_6$)alkylcarbonyl, ($C_1$-$C_6$)alkoxy ($C_1$-$C_6$)alkylcarbonyl, phenylcarbonyl, hetarylcarbonyl, ($C_1$-$C_6$)alkoxycarbonyl, halo ($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl,

di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl (aryl) aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or is optionally substituted phenyl or optionally substituted hetaryl; or

is ($C_1$-$C_6$)alkoxy, halo($C_1$-$C_6$)alkoxy, ($C_1$-$C_6$)alkoxy ($C_1$-$C_6$)alkoxy, aryloxy, aryl($C_1$-$C_6$)alkyloxy, ($C_3$-$C_6$)cycloalkyloxy, ($C_3$-$C_6$)cycloalkyl($C_1$-$C_6$)alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or is hydroxyl; or

is ($C_1$-$C_6$)alkylamino, di($C_1$-$C_6$)alkylamino, halo($C_1$-$C_6$)alkylamino, dihalo($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hetarylamino, dihetarylamino, ($C_1$-$C_6$)alkyl (aryl) amino, ($C_3$-$C_6$)cycloalkyl (aryl) amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or

is ($C_1$-$C_6$)alkylsulphanyl, ($C_1$-$C_6$)alkylsulphinyl, ($C_1$-$C_6$)alkylsulphonyl, halo ($C_1$-$C_6$)alkylsulphanyl, halo($C_1$-$C_6$)alkylsulphinyl, halo ($C_1$-$C_6$)alkylsulphonyl, ($C_3$-$C_6$)cycloalkylsulphanyl, ($C_3$-$C_6$)cycloalkylsulphinyl, ($C_3$-$C_6$)cycloalkylsulphonyl, ($C_3$-$C_6$)cycloalkyl($C_1$-$C_6$)alkylsulphanyl, ($C_3$-$C_6$)cycloalkyl ($C_1$-$C_6$)alkylsulphinyl, ($C_3$-$C_6$)cycloalkyl ($C_1$-$C_6$)alkylsulphonyl, arylsulphanyl, arylsulphinyl, arylsulphonyl, aryl($C_1$-$C_6$)alkylsulphanyl, aryl($C_1$-$C_6$)alkylsulphinyl, aryl ($C_1$-$C_6$)alkylsulphonyl, aminosulphonyl, ($C_1$-$C_6$)alkylaminosulphonyl, di(Ci-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; or

is an optionally singly or multiply, identically or differently halogen-, cyano-, nitro-, hydroxyl-, amino-, ($C_1$-$C_6$)alkyl-, halo($C_1$-$C_6$)alkyl-, ($C_1$-$C_6$)alkoxy-, halo ($C_1$-$C_6$)alkoxy-, ($C_1$-$C_6$)alkylsulphinyl-, ($C_1$-$C_6$)alkylsulphanyl-, ($C_1$-$C_6$)alkylsulphonyl-, halo($C_1$-$C_6$)alkylsulphinyl-, halo ($C_1$-$C_6$)alkylsulphanyl-, halo($C_1$-$C_6$)alkylsulphonyl- or optionally substituted ($C_3$-$C_6$)cycloalkyl-substituted, saturated or unsaturated three- to six-membered ring optionally interrupted by heteroatoms from the group of 0, S and N;

W is hydrogen or halogen;

V is oxygen, sulphur or an optionally substituted nitrogen;

X, Y and Z each independently

are hydrogen, halogen, hydroxyl, amino, cyano, nitro, OCN, SCN, $SF_5$; or

are tri ($C_1$-$C_6$)alkylsilyl, ($C_1$-$C_6$)alkyl, halo ($C_1$-$C_6$)alkyl, cyano ($C_1$-$C_6$)alkyl, hydroxy($C_1$-$C_6$)alkyl, ($C_1$-$C_6$)alkoxycarbonyl ($C_1$-$C_6$)alkyl, ($C_1$-$C_6$)alkoxy($C_1$-$C_6$)alkyl, ($C_2$-$C_6$)alkenyl, halo($C_2$-$C_6$)alkenyl, cyano($C_2$-$C_6$)alkenyl, ($C_2$-$C_6$)alkynyl, halo($C_2$-$C_6$)alkynyl, cyano($C_2$-$C_6$)alkynyl, ($C_1$-$C_6$)alkoxy, halo($C_1$-$C_6$)alkoxy, cyano ($C_1$-$C_6$)alkoxy, hydroxycarbonyl ($C_1$-$C_6$)alkoxy, ($C_1$-$C_6$)alkoxycarbonyl ($C_1$-$C_6$)alkoxy, ($C_1$-$C_6$)alkoxy ($C_1$-$C_6$)alkoxy, ($C_1$-$C_6$)alkylhydroxyimino, ($C_1$-$C_6$)alkoxyimino, ($C_1$-$C_6$)alkyl($C_1$-$C_6$)alkoxyimino, halo($C_1$-$C_6$)alkyl($C_1$-$C_6$)alkoxyimino, ($C_1$-$C_6$)alkylthio, halo($C_1$-$C_6$)alkylthio, ($C_1$-$C_6$)alkoxy($C_1$-$C_6$)alkylthio, ($C_1$-$C_6$)alkylthio($C_1$-$C_6$)alkyl, ($C_1$-$C_6$)alkylsulphinyl, halo($C_1$-$C_6$)alkylsulphinyl, ($C_1$-$C_6$)alkoxy($C_1$-$C_6$)alkylsulphinyl, ($C_1$-$C_6$) alkylsulphinyl ($C_1$-$C_6$)alkyl, ($C_1$-$C_6$)alkylsulphonyl, halo ($C_1$-$C_6$)alkylsulphonyl, ($C_1$-$C_6$)alkoxy($C_1$-$C_6$)alkylsulphonyl, ($C_1$-$C_6$)alkylsulphonyl($C_1$-$C_6$)alkyl, ($C_1$-$C_6$)alkylsulphonyloxy, ($C_1$-$C_6$)alkylcarbonyl, halo($C_1$-$C_6$)alkylcarbonyl, carboxyl, ($C_1$-$C_6$)alkylcarbonyloxy, ($C_1$-$C_6$)alkoxycarbonyl, halo ($C_1$-$C_6$)alkoxycarbonyl, aminocarbonyl, ($C_1$-$C_6$)alkylaminocarbonyl, di($C_1$-$C_6$)alkylaminocarbonyl, ($C_2$-$C_6$)alkenylaminocarbonyl, di($C_2$-$C_6$)alkenylaminocarbonyl, ($C_3$-$C_6$)cycloalkylaminocarbonyl, ($C_1$-$C_6$)alkylsulphonylamino, aminosulphonyl, ($C_1$-$C_6$)alkylaminosulphonyl, di($C_1$-$C_6$)alkylaminosulphonyl, ($C_1$-$C_6$)alkylsulphoximino, aminothiocarbonyl, ($C_1$-$C_6$)alkylaminothiocarbonyl or di ($C_1$-$C_6$)alkylaminothiocarbonyl, where all the aforementioned radicals may optionally be substituted by halogen; or

are phenyl($C_1$-$C_3$)alkyl, phenoxy, phenyl($C_1$-$C_3$)alkyloxy, phenoxy($C_1$-$C_3$)alkyl, phenylthio, phenylthio($C_1$-$C_3$)alkyl, phenylsulphinyl, phenylsulphonyl, hetaryl($C_1$-$C_3$)alkyl, hetaryloxy, hetaryl($C_1$-$C_3$)alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are ($C_3$-$C_6$)cycloalkyl($C_1$-$C_3$)alkyl, ($C_3$-$C_6$)cycloalkyloxy, ($C_3$-$C_6$)cycloalkyl($C_1$-$C_3$)alkoxy, ($C_3$-$C_6$)cycloalkylthio, ($C_3$-$C_6$)cycloalkyl($C_1$-$C_3$)alkylthio, ($C_3$-$C_6$)cycloalkylsulphinyl, ($C_3$-$C_6$)cycloalkyl ($C_1$-$C_3$)alkylsulphinyl, ($C_3$-$C_6$)cycloalkylsulphonyl, ($C_3$-$C_6$)cycloalkyl($C_1$-$C_3$)alkylsulphonyl or ($C_3$-$C_8$)cycloalkenyl, where the afore-

mentioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or
are NR'R"
where R' and R" each independently
are hydrogen, cyano, $(C_1-C_6)$alkyl, halo$(C_1-C_6)$alkyl, cyano$(C_1-C_6)$ alkyl, hydroxy$(C_1-C_6)$alkyl, $(C_1-C_6)$alkoxy$(C_1-C_6)$alkyl, $(C_1-C_6)$alkylthio$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, acyl or $(C_1-C_6)$alkoxycarbonyl; or
R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five-to seven-membered ring optionally interrupted by heteroatoms from the group of 0, S and N; or
are a $(C_3-C_6)$cycloalkyl, oxetanyl, oxolanyl, oxanyl, $(C_3-C_8)$cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or X and Z, or Y and Z, may form the following 5 or 6-membered rings which are optionally substituted identically or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

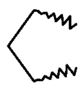 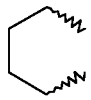 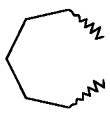 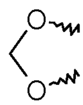 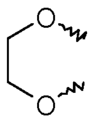 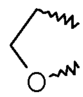 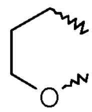

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

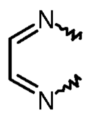 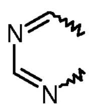 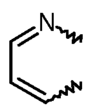 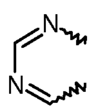 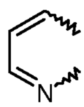 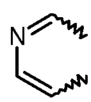

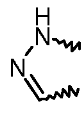 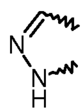 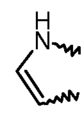 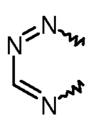 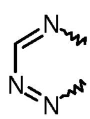 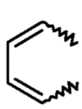

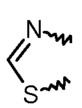 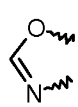 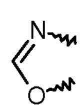

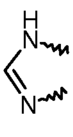 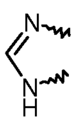 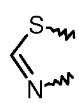

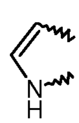 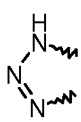 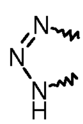

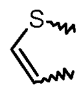 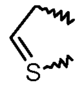 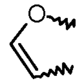 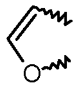

n is the number 0 or 1.

**10.** Compound according to Claim 9 in which the substructure of the formula (I-C) is the substructure (I-C-1)

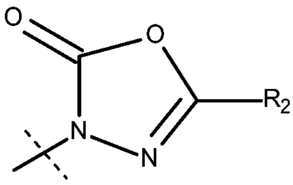

(I-C-1)

where

$R^2$ is hydrogen; or
is ($C_1$-$C_6$)alkyl, ($C_3$-$C_6$) cycloalkyl ($C_1$-$C_6$) alkyl, ($C_2$-$C_6$)alkenyl, ($C_2$-$C_6$)alkynyl, halo($C_1$-$C_6$)alkyl, cyano($C_1$-$C_6$)alkyl, ($C_1$-$C_6$)alkoxy ($C_1$-$C_6$)alkyl or phenyl($C_1$-$C_3$)alkyl, where the aforementioned radicals may each optionally be substituted; or
is ($C_3$-$C_6$)cycloalkyl, ($C_3$-$C_6$)cycloalkenyl, phenyl, pyridyl, pyrimidyl, pyridazinyl, pyrazinyl, furyl, thienyl, triazinyl, thiazolyl, thiadiazolyl, oxazolyl, oxadiazolyl, pyrazolyl or triazolyl, where the aforementioned radicals may each be mono- to trisubstituted by halogen, cyano, nitro, hydroxyl, amino, methyl, ethyl, isopropyl, tert-butyl, trifluoromethyl, difluoromethyl, methoxy, trifluoromethoxy, difluoromethoxy, cyano, amino, hydroxyl, nitro, halo($C_1$-$C_3$)alkylsulphanyl or optionally methyl-, fluorine-, chlorine-, cyano-substituted cyclopropyl;
W is hydrogen or halogen, especially F or Cl;
X and Y are each independently hydrogen, fluorine, chlorine, bromine, methyl, ethyl, trifluoromethyl, 2,2-difluoromethyl, difluoromethoxy, trifluoromethoxy, cyclopropyl, cyano, amino, hydroxyl or nitro;
Z is hydrogen; and
n is the number 0 or 1.

**11.** Compound according to any of Claims 1 to 3, in which A and B together with the atoms to which they are bonded are a substructure of the formula (I-D)

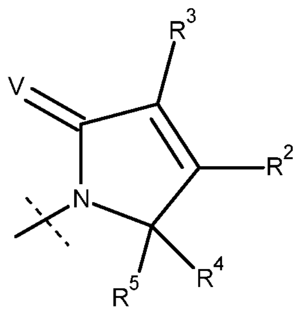

(I-D)

where

$R^2$, $R^3$, $R^4$ and $R^5$ each independently

are hydrogen, cyano, halogen or nitro; or

are $(C_1-C_6)$alkyl, $(C_3-C_6)$cycloalkyl $(C_1-C_6)$alkyl, halo$(C_1-C_6)$alkyl, cyano $(C_1-C_6)$alkyl, hydroxy $(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulphanyl $(C_1-C_6)$alkyl, $(C_1-C_6)$alkylsulphinyl $(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$alkyl, $(C_1-C_6)$alkoxy $(C_1-C_6)$alkylsulphanyl $(C_1-C_6)$alkyl, $(C_1-C_6)$alkoxy $(C_1-C_6)$alkylsulphinyl $(C_1-C_6)$alkyl, $(C_1-C_6)$alkoxy $(C_1-C_6)$alkylsulphonyl $(C_1-C_6)$alkyl, phenyl $(C_1-C_6)$alkyl, phenoxy $(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl $(C_1-C_6)$alkyl, hetaryloxy $(C_1-C_6)$alkyl, hetarylthio $(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

are optionally substituted saturated or unsaturated $(C_3-C_6)$cycloalkyl which may optionally be interrupted by one or more heteroatoms; or

are $(C_1-C_6)$alkylcarbonyl, halo$(C_1-C_6)$alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl ($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or are carbonyl or carboxyl; or

are optionally substituted phenyl or optionally substituted hetaryl; or

are $(C_1-C_6)$alkoxy, halo$(C_1-C_6)$alkoxy, $(C_1-C_6)$alkoxy $(C_1-C_6)$alkoxy, aryloxy, aryl$(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or are hydroxyl; or

are $(C_1-C_6)$alkylamino, di$(C_1-C_6)$alkylamino, halo$(C_1-C_6)$alkylamino, dihalo $(C_1-C_6)$alkylamino, $(C_3-C_6)$cycloalkylamino, di$(C_3-C_6)$cycloalkylamino, $(C_3-C_6)$cycloalkyl ($(C_1-C_6)$alkyl)amino, arylamino, diarylamino, hetarylamino, dihetarylamino, $(C_1-C_6)$alkyl (aryl) amino, $(C_3-C_6)$cycloalkyl (aryl) amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or are amino; or

are $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphonyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl $(C_1-C_6)$alkylsulphinyl, $(C_3-C_6)$cycloalkyl $(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di$(C_1-C_6)$alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or are sulphanyl; or

$R^4$ and $R^5$ together with the atom to which they are bonded may form an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo $(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo $(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of 0, S and N; or

or $R^2$ is an optionally singly or multiply, identically or or differently halogen-, cyano-, nitro-, hydroxyl-, amino-, $(C_1-C_6)$alkyl-, halo$(C_1-C_6)$alkyl-, $(C_1-C_6)$alkoxy-, halo $(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo $(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of 0, S and N;

W is hydrogen or halogen;

V is oxygen, sulphur or an optionally substituted nitrogen;

W is hydrogen or halogen;

X, Y and Z each independently

are hydrogen, halogen, hydroxyl, amino, cyano, nitro, OCN, SCN, $SF_5$; or

are tri($C_1$-$C_6$)alkylsilyl, ($C_1$-$C_6$)alkyl, halo($C_1$-$C_6$)alkyl, cyano($C_1$-$C_6$)alkyl, hydroxy ($C_1$-$C_6$)alkyl, ($C_1$-$C_6$)alkoxycarbonyl($C_1$-$C_6$)alkyl, ($C_1$-$C_6$)alkoxy ($C_1$-$C_6$)alkyl, ($C_2$-$C_6$)alkenyl, halo ($C_2$-$C_6$)alkenyl, cyano($C_2$-$C_6$)alkenyl, ($C_2$-$C_6$)alkynyl, halo($C_2$-$C_6$)alkynyl, cyano($C_2$-$C_6$)alkynyl, ($C_1$-$C_6$)alkoxy, halo($C_1$-$C_6$)alkoxy, cyano($C_1$-$C_6$)alkoxy, hydroxycarbonyl ($C_1$-$C_6$)alkoxy, ($C_1$-$C_6$)alkoxycarbonyl ($C_1$-$C_6$)alkoxy, ($C_1$-$C_6$)alkoxy($C_1$-$C_6$)alkoxy, ($C_1$-$C_6$)alkylhydroxyimino, ($C_1$-$C_6$)alkoxyimino, ($C_1$-$C_6$)alkyl($C_1$-$C_6$)alkoxyimino, halo($C_1$-$C_6$)alkyl ($C_1$-$C_6$)alkoxyimino, ($C_1$-$C_6$)alkylthio, halo($C_1$-$C_6$)alkylthio, ($C_1$-$C_6$)alkoxy ($C_1$-$C_6$)alkylthio, ($C_1$-$C_6$)alkylthio ($C_1$-$C_6$)alkyl, ($C_1$-$C_6$)alkylsulphinyl, halo($C_1$-$C_6$)alkylsulphinyl, ($C_1$-$C_6$)alkoxy ($C_1$-$C_6$)alkylsulphinyl, ($C_1$-$C_6$)alkylsulphinyl ($C_1$-$C_6$)alkyl, ($C_1$-$C_6$)alkylsulphonyl, halo ($C_1$-$C_6$)alkylsulphonyl, ($C_1$-$C_6$)alkoxy ($C_1$-$C_6$)alkylsulphonyl, ($C_1$-$C_6$)alkylsulphonyl ($C_1$-$C_6$)alkyl, ($C_1$-$C_6$)alkylsulphonyloxy, ($C_1$-$C_6$)alkylcarbonyl, halo($C_1$-$C_6$)alkylcarbonyl, carboxyl, ($C_1$-$C_6$)alkylcarbonyloxy, ($C_1$-$C_6$)alkoxycarbonyl, halo ($C_1$-$C_6$)alkoxycarbonyl, aminocarbonyl, ($C_1$-$C_6$)alkylaminocarbonyl, di(Ci-$C_6$)alkylaminocarbonyl, ($C_2$-$C_6$)alkenylaminocarbonyl, di($C_2$-$C_6$)alkenylaminocarbonyl, ($C_3$-$C_6$)cycloalkylaminocarbonyl, ($C_1$-$C_6$)alkylsulphonylamino, aminosulphonyl, ($C_1$-$C_6$)alkylaminosulphonyl, di(Ci-$C_6$)alkylaminosulphonyl, ($C_1$-$C_6$)alkylsulphoximino, aminothiocarbonyl, ($C_1$-$C_6$)alkylaminothiocarbonyl or di($C_1$-$C_6$)alkylaminothiocarbonyl, where all the aforementioned radicals may optionally be substituted by halogen; or

are phenyl ($C_1$-$C_3$)alkyl, phenoxy, phenyl ($C_1$-$C_3$)alkyloxy, phenoxy($C_1$-$C_3$)alkyl, phenylthio, phenylthio($C_1$-$C_3$)alkyl, phenylsulphinyl, phenylsulphonyl, hetaryl($C_1$-$C_3$)alkyl, hetaryloxy, hetaryl($C_1$-$C_3$)alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are ($C_3$-$C_6$) cycloalkyl ($C_1$-$C_3$)alkyl, ($C_3$-$C_6$) cycloalkyloxy, ($C_3$-$C_6$)cycloalkyl ($C_1$-$C_3$)alkoxy, ($C_3$-$C_6$)cycloalkylthio, ($C_3$-$C_6$)cycloalkyl ($C_1$-$C_3$)alkylthio, ($C_3$-$C_6$)cycloalkylsulphinyl, ($C_3$-$C_6$)cycloalkyl ($C_1$-$C_3$)alkylsulphinyl, ($C_3$-$C_6$) cycloalkylsulphonyl, ($C_3$-$C_6$)cycloalkyl ($C_1$-$C_3$)alkylsulphonyl or ($C_3$-$C_8$)cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are NR'R"

where R' and R" each independently

are hydrogen, cyano, ($C_1$-$C_6$)alkyl, halo ($C_1$-$C_6$)alkyl, cyano($C_1$-$C_6$)alkyl, hydroxy($C_1$-$C_6$)alkyl, ($C_1$-$C_6$)alkoxy ($C_1$-$C_6$)alkyl, ($C_1$-$C_6$)alkylthio ($C_1$-$C_6$)alkyl, ($C_2$-$C_6$)alkenyl, halo($C_2$-$C_6$)alkenyl, cyano($C_2$-$C_6$)alkenyl, ($C_2$-$C_6$)alkynyl, halo($C_2$-$C_6$)alkynyl, cyano($C_2$-$C_6$)alkynyl, acyl or ($C_1$-$C_6$)alkoxycarbonyl; or

R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five-to seven-membered ring optionally interrupted by heteroatoms from the group of O, S and N; or

are a ($C_3$-$C_6$)cycloalkyl, oxetanyl, oxolanyl, oxanyl, ($C_3$-$C_8$)cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyanosubstituted cyclopropyl;

or X and Z, or Y and Z, may form the following 5 or 6-membered rings which are optionally substituted identically or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

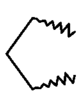 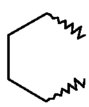 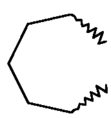 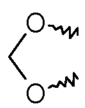 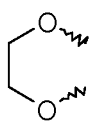 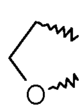 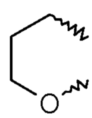

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

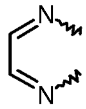 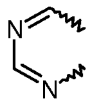 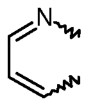 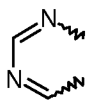 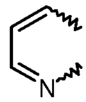 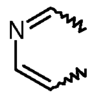

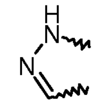 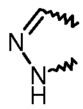 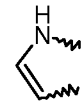 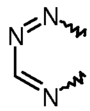 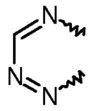 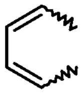

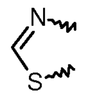 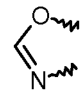 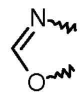

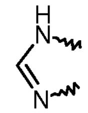 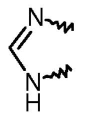 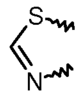

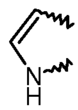 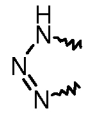 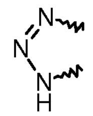

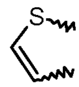 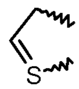 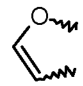 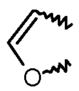

n is the number 0 or 1.

**12.** Compound according to Claim 11 in which the substructure of the formula (I-D) is a substructure which is selected from the group consisting of

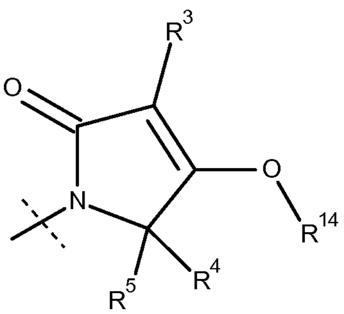

(I-D-1)

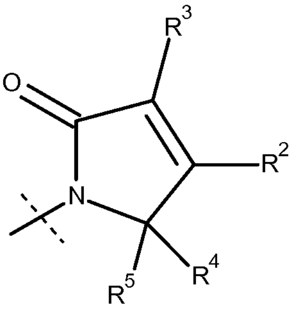

(I-D-2)

where

$R^2$, $R^3$, $R^4$ and $R^5$ each independently are hydrogen; or are $(C_1-C_6)$alkyl, $(C_3-C_6)$cycloalkyl $(C_1-C_3)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy $(C_1-C_6)$alkyl or phenyl$(C_1-C_3)$alkyl, where the aforementioned radicals may each optionally be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$ alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl; or are $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano,

nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl; or are carboxyl; or

$R^4$ and $R^5$ together with the atom to which they are bonded may form an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo $(C_1-C_6)$alkoxy-, $(C_1-C_6)$alkylsulphinyl-, $(C_1-C_6)$ alkylsulphanyl-, $(C_1-C_6)$alkylsulphonyl-, halo$(C_1-C_6)$alkylsulphinyl-, halo $(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of O, S and N;

$R^{14}$ is $(C_1-C_6)$alkyl, halo$(C_1-C_6)$ alkyl, cyano$(C_1-C_6)$alkyl, $(C_1-C_6)$alkoxy $(C_1-C_3)$alkyl, phenyl $(C_1-C_3)$alkyl, $(C_3-C_6)$cycloalkyl or $(C_3-C_6)$cycloalkyl $(C_1-C_3)$alkyl, where the aforementioned radicals may each be mono- to trisubstituted by halogen, cyano, nitro, hydroxyl, amino, $(C_1-C_6)$alkyl, halo $(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl;

X, Y and Z are each independently hydrogen, fluorine, chlorine, bromine, cyano, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;

or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

or are a cyclopropyl, cyclobutyl, cyclopentyl, cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

n is the number 0 or 1.

**13.** Compound according to any of Claims 1 to 3, in which
A and B together with the atoms to which they are bonded are a substructure of the formula (I-E)

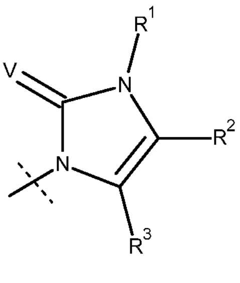

(I-E)

$R^1$ is hydrogen, cyano or nitro; or

is $(C_1-C_6)$alkyl, $(C_3-C_6)$cycloalkyl$(C_1-C_6)$alkyl, halo$(C_2-C_6)$alkyl, cyano$(C_1-C_6)$alkyl, hydroxy$(C_1-C_6)$alkyl, $(C_1-C_6)$alkoxy$(C_1-C_6)$ alkyl, amino$(C_1-C_6)$alkyl, $(C_1-C_6)$alkoxycarbonyl$(C_1-C_6)$alkyl, $(C_1-C_6)$alkylsulphanyl $(C_1-C_6)$alkyl, $(C_1-C_6)$alkylsulphinyl $(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$alkyl, $(C_1-C_6)$alkoxy$(C_1-C_6)$alkylsulphanyl $(C_1-C_6)$alkyl, $(C_1-C_6)$alkoxy$(C_1-C_6)$alkylsulphinyl $(C_1-C_6)$alkyl, $(C_1-C_6)$alkoxy$(C_1-C_6)$alkylsulphonyl $(C_1-C_6)$alkyl, phenyl $(C_1-C_6)$alkyl, phenoxy $(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl $(C_1-C_6)$alkyl, hetaryloxy $(C_1-C_6)$alkyl, hetarylsulphanyl$(C_1-C_6)$alkyl, hetarylsulphinyl$(C_1-C_6)$alkyl, hetarylsulphonyl$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or

is optionally substituted saturated or unsaturated $(C_3-C_6)$cycloalkyl which may optionally be interrupted by one or more heteroatoms; or

is $(C_1-C_6)$alkylcarbonyl, halo $(C_1-C_6)$alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, $(C_1-C_6)$alkoxycarbonyl, halo $(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl $((C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or is carbonyl or carboxyl; or
is optionally substituted phenyl or optionally substituted hetaryl; or
is $(C_1-C_6)$alkoxy, halo $(C_1-C_6)$alkoxy, $(C_3-C_6)$cycloalkyloxy, aryloxy, aryl$(C_1-C_6)$alkyloxy or carbonyloxy, where the aforementioned radicals may each optionally be substituted, or is hydroxyl; or
is $(C_1-C_6)$alkylamino, halo $(C_1-C_6)$alkylamino, dihalo$(C_1-C_6)$alkylamino, di$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl)amino, arylamino, diarylamino, hetarylamino, dihetarylamino, $(C_1-C_6)$alkyl (aryl) amino, $(C_3-C_6)$cycloalkyl (aryl) amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or is amino; or
is $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphanyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl$(C_1-C_6)$alkylsulphinyl, $(C_3-C_6)$cycloalkyl$(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl $(C_1-C_6)$alkylsulphonyl, aminosulphonyl, $(C_1-C_6)$alkylaminosulphonyl, di(Ci-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or is sulphanyl; and
$R^2$ and $R^3$ each independently
are hydrogen, cyano, halogen or nitro; or
are $(C_1-C_6)$alkyl, $(C_3-C_6)$cycloalkyl $(C_1-C_6)$alkyl, halo$(C_1-C_6)$alkyl, cyano$(C_1-C_6)$alkyl, hydroxy$(C_1-C_6)$alkyl, $(C_1-C_6)$alkoxy $(C_1-C_6)$alkyl, amino$(C_1-C_6)$alkyl, $(C_1-C_6)$alkoxycarbonyl $(C_1-C_6)$alkyl, $(C_1-C_6)$alkylsulphanyl$(C_1-C_6)$alkyl, $(C_1-C_6)$alkylsulphinyl$(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$alkyl, $(C_1-C_6)$alkoxy$(C_1-C_6)$alkylsulphanyl $(C_1-C_6)$alkyl, $(C_1-C_6)$alkoxy$(C_1-C_6)$alkylsulphinyl $(C_1-C_6)$alkyl, $(C_1-C_6)$alkoxy$(C_1-C_6)$alkylsulphonyl $(C_1-C_6)$alkyl, phenyl$(C_1-C_6)$alkyl, phenoxy $(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl $(C_1-C_6)$alkyl, hetaryloxy$(C_1-C_6)$alkyl, hetarylthio $(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or
are optionally substituted saturated or unsaturated $(C_3-C_6)$cycloalkyl which may optionally be interrupted by one or more heteroatoms; or
are $(C_1-C_6)$alkylcarbonyl, halo$(C_1-C_6)$alkylcarbonyl, hydroxy $(C_1-C_6)$alkylcarbonyl, $(C_1-C_6)$alkoxy $(C_1-C_6)$alkylcarbonyl, phenylcarbonyl, hetarylcarbonyl, $(C_1-C_6)$alkoxycarbonyl, halo $(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl $((C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or are carbonyl or carboxyl; or
are optionally substituted phenyl or optionally substituted hetaryl; or
are $(C_1-C_6)$alkoxy, halo$(C_1-C_6)$alkoxy, $(C_1-C_6)$alkoxy$(C_1-C_6)$alkoxy, aryloxy, aryl$(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or are hydroxyl; or
are $(C_1-C_6)$alkylamino, di$(C_1-C_6)$alkylamino, halo$(C_1-C_6)$alkylamino, dihalo $(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl)amino, arylamino, diarylamino, hetarylamino, dihetarylamino, $(C_1-C_6)$alkyl (aryl) amino, $(C_3-C_6)$cycloalkyl (aryl) amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcar-

bamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or are amino; or

are $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphonyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl $(C_1-C_6)$alkylsulphinyl, $(C_3-C_6)$cycloalkyl $(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di(Ci-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or are sulphanyl; or

are an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo$(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo$(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of O, S and N;

W is hydrogen or halogen;

V is oxygen, sulphur or an optionally substituted nitrogen;

X, Y and Z each independently

are hydrogen, halogen, hydroxyl, amino, cyano, nitro, OCN, SCN, $SF_5$; or

are tri$(C_1-C_6)$alkylsilyl, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxycarbonyl$(C_1-C_6)$alkyl, $(C_1-C_6)$alkoxy$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, $(C_1-C_6)$alkoxy, halo$(C_1-C_6)$alkoxy, cyano$(C_1-C_6)$alkoxy, hydroxycarbonyl$(C_1-C_6)$alkoxy, $(C_1-C_6)$alkoxycarbonyl$(C_1-C_6)$alkoxy, $(C_1-C_6)$alkoxy$(C_1-C_6)$alkoxy, $(C_1-C_6)$alkylhydroxyimino, $(C_1-C_6)$alkoxyimino, $(C_1-C_6)$alkyl$(C_1-C_6)$alkoxyimino, halo$(C_1-C_6)$alkyl $(C_1-C_6)$alkoxyimino, $(C_1-C_6)$alkylthio, halo$(C_1-C_6)$alkylthio, $(C_1-C_6)$alkoxy$(C_1-C_6)$alkylthio, $(C_1-C_6)$alkylthio $(C_1-C_6)$alkyl, $(C_1-C_6)$alkylsulphinyl, halo$(C_1-C_6)$alkylsulphinyl, $(C_1-C_6)$alkoxy $(C_1-C_6)$alkylsulphinyl, $(C_1-C_6)$alkylsulphinyl$(C_1-C_6)$alkyl, $(C_1-C_6)$alkylsulphonyl, halo$(C_1-C_6)$alkylsulphonyl, $(C_1-C_6)$alkoxy$(C_1-C_6)$alkylsulphonyl, $(C_1-C_6)$alkylsulphonyl$(C_1-C_6)$alkyl, $(C_1-C_6)$alkylsulphonyloxy, $(C_1-C_6)$alkylcarbonyl, halo$(C_1-C_6)$alkylcarbonyl, carboxyl, $(C_1-C_6)$alkylcarbonyloxy, $(C_1-C_6)$alkoxycarbonyl, halo$(C_1-C_6)$alkoxycarbonyl, aminocarbonyl, $(C_1-C_6)$alkylaminocarbonyl, di$(C_1-C_6)$alkylaminocarbonyl, $(C_2-C_6)$alkenylaminocarbonyl, di$(C_2-C_6)$alkenylaminocarbonyl, $(C_3-C_6)$cycloalkylaminocarbonyl, $(C_1-C_6)$alkylsulphonylamino, aminosulphonyl, $(C_1-C_6)$alkylaminosulphonyl, di$(C_1-C_6)$alkylaminosulphonyl, $(C_1-C_6)$alkylsulphoximino, aminothiocarbonyl, $(C_1-C_6)$alkylaminothiocarbonyl or di$(C_1-C_6)$alkylaminothiocarbonyl, where all the aforementioned radicals may optionally be substituted by halogen; or

are phenyl$(C_1-C_3)$alkyl, phenoxy, phenyl$(C_1-C_3)$alkyloxy, phenoxy$(C_1-C_3)$alkyl, phenylthio, phenylthio$(C_1-C_3)$alkyl, phenylsulphinyl, phenylsulphonyl, hetaryl$(C_1-C_3)$alkyl, hetaryloxy, hetaryl$(C_1-C_3)$alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are $(C_3-C_6)$cycloalkyl $(C_1-C_3)$alkyl, $(C_3-C_6)$cycloalkyloxy, $(C_3-C_6)$cycloalkyl$(C_1-C_3)$alkoxy, $(C_3-C_6)$cycloalkylthio, $(C_3-C_6)$cycloalkyl$(C_1-C_3)$alkylthio, $(C_3-C_6)$cycloalkylsulphinyl, $(C_3-C_6)$cycloalkyl$(C_1-C_3)$alkylsulphinyl, $(C_3-C_6)$cycloalkylsulphonyl, $(C_3-C_6)$cycloalkyl$(C_1-C_3)$alkylsulphonyl or $(C_3-C_8)$cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are NR'R''

where R' and R" each independently are hydrogen, cyano, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxy$(C_1-C_6)$alkyl, $(C_1-C_6)$alkylthio$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, acyl or $(C_1-C_6)$alkoxycarbonyl; or

R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five- to seven-membered ring optionally interrupted by heteroatoms from the group of O, S and N; or

are a $(C_3-C_6)$cycloalkyl, oxetanyl, oxolanyl, oxanyl, $(C_3-C_8)$cycloalkenyl, phenyl, pyridyl, pyrimidyl, pyridazinyl,

pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy and optionally methyl-, fluorine-, chlorine-, cyano-substituted cyclopropyl;

or X and Z, or Y and Z, may form the following 5 or 6-membered rings which are optionally substituted identically or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

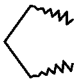 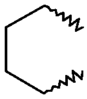 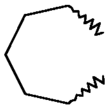 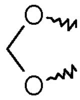 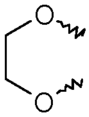 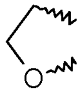 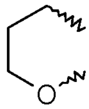

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

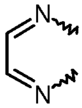 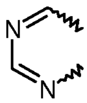 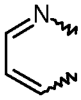 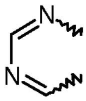 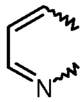 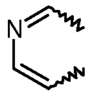

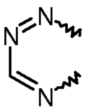 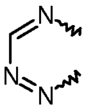 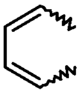 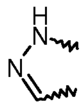 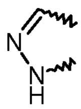 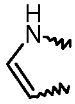

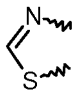 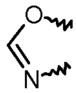 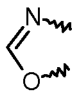

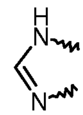 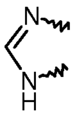 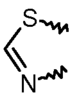 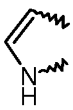 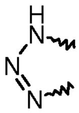 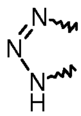

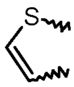 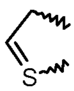 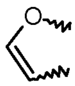 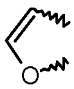

and

n is the number 0 or 1.

**14.** Compound according to Claim 13 in which the substructure of the formula (I-E) is a substructure of the formula (I-E-1)

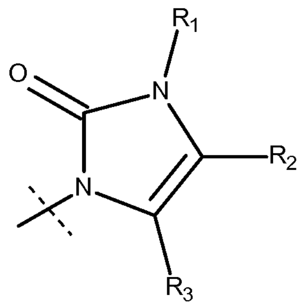

(I-E-1)

where

$R^1$ is hydrogen; or
is $(C_1-C_6)$alkyl, $(C_3-C_6)$cycloalkyl$(C_1-C_3)$alkyl, $(C_2-C_6)$alkenyl, $(C_2-C_6)$alkynyl, halo $(C_2-C_6)$alkyl, cyano$(C_1-C_6)$alkyl, $(C_1-C_6)$alkoxy$(C_1-C_6)$alkyl or phenyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl; or
is $(C_3-C_6)$cycloalkyl, $(C_3-C_6)$cycloalkenyl, oxetanyl, oxolanyl, oxanyl, phenyl, pyrimidyl, pyridazinyl, pyrazinyl, triazinyl, thiazolyl, thiazadiazolyl, oxazolyl, oxadiazolyl, pyrazolyl or triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo$(C_1-C_6)$alkylsulphanyl, halo$(C_1-C_6)$alkylsulphonyl or optionally methyl-, fluorine-, chlorine-, cyano-substituted cyclopropyl;
$R^2$ and $R^3$ each independently are hydrogen; or
are $(C_1-C_6)$alkyl, $(C_3-C_6)$cycloalkyl $(C_1-C_3)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy$(C_1-C_6)$alkyl or phenyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo $(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl; or
are $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl;
W is hydrogen or halogen, especially fluorine or chlorine;
X, Y and Z are each independently hydrogen, fluorine, chlorine, bromine, cyano, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;
or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or are a cyclopropyl, cyclobutyl, cyclopentyl, cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; and
n is the number 0 or 1.

**15.** Compound according to any of Claims 1 to 3, in which
A and B together with the atoms to which they are bonded are a substructure of the formula (I-F)

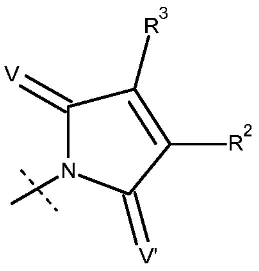

(I-F)

$R^2$ and $R^3$ each independently

are hydrogen, cyano, halogen or nitro; or are ($C_1$-$C_6$)alkyl, ($C_3$-$C_6$)cycloalkyl($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxy($C_1$-$C_6$)alkyl, amino($C_1$-$C_6$)alkyl, ($C_1$-$C_6$)alkoxycarbonyl($C_1$-$C_6$)alkyl, ($C_1$-$C_6$)alkylsulphanyl($C_1$-$C_6$)alkyl, ($C_1$-$C_6$)alkylsulphinyl($C_1$-$C_6$)alkyl, ($C_1$-$C_6$)alkylsulphonyl($C_1$-$C_6$)alkyl, halo($C_1$-$C_6$)alkylsulphanyl($C_1$-$C_6$)alkyl, halo($C_1$-$C_6$)alkylsulphinyl($C_1$-$C_6$)alkyl, halo($C_1$-$C_6$)alkylsulphonyl($C_1$-$C_6$)alkyl, ($C_1$-$C_6$)alkoxy($C_1$-$C_6$)alkylsulphanyl($C_1$-$C_6$)alkyl, ($C_1$-$C_6$)alkoxy($C_1$-$C_6$)alkylsulphinyl($C_1$-$C_6$)alkyl, ($C_1$-$C_6$)alkoxy($C_1$-$C_6$)alkylsulphonyl($C_1$-$C_6$)alkyl, phenyl($C_1$-$C_6$)alkyl, phenoxy($C_1$-$C_6$)alkyl, phenylsulphanyl($C_1$-$C_6$)alkyl, phenylsulphinyl($C_1$-$C_6$)alkyl, phenylsulphonyl($C_1$-$C_6$)alkyl, hetaryl($C_1$-$C_6$)alkyl, hetaryloxy($C_1$-$C_6$)alkyl, hetarylthio($C_1$-$C_6$)alkyl, where the aforementioned radicals may each optionally be substituted and/or, under the condition that, in the case of an unsaturated radical, the defined minimum number of carbon atoms is 2, the radicals may be saturated or unsaturated; or

are optionally substituted saturated or unsaturated ($C_3$-$C_6$)cycloalkyl which may optionally be interrupted by one or more heteroatoms; or

are ($C_1$-$C_6$)alkylcarbonyl, halo ($C_1$-$C_6$)alkylcarbonyl, hydroxy ($C_1$-$C_6$)alkylcarbonyl, ($C_1$-$C_6$)alkoxy ($C_1$-$C_6$)alkylcarbonyl, phenylcarbonyl, hetarylcarbonyl, ($C_1$-$C_6$)alkoxycarbonyl, halo($C_1$-$C_6$)alkoxycarbonyl, aryloxycarbonyl, aminocarbonyl, ($C_1$-$C_6$)alkylaminocarbonyl, di($C_1$-$C_6$)alkylaminocarbonyl, ($C_3$-$C_6$)cycloalkylaminocarbonyl, di($C_3$-$C_6$)cycloalkylaminocarbonyl, ($C_3$-$C_6$)cycloalkyl(($C_1$-$C_6$)alkyl)aminocarbonyl, arylaminocarbonyl, diarylaminocarbonyl, ($C_1$-$C_6$)alkyl(aryl)aminocarbonyl, ($C_3$-$C_6$)cycloalkyl(aryl)aminocarbonyl, ($C_1$-$C_6$)alkylaminothiocarbonyl, di($C_1$-$C_6$)alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or are carbonyl or carboxyl; or

are optionally substituted phenyl or optionally substituted hetaryl; or

are ($C_1$-$C_6$)alkoxy, halo($C_1$-$C_6$)alkoxy, ($C_1$-$C_6$)alkoxy ($C_1$-$C_6$)alkoxy, aryloxy, aryl($C_1$-$C_6$)alkyloxy, ($C_3$-$C_6$)cycloalkyloxy, ($C_3$-$C_6$)cycloalkyl($C_1$-$C_6$)alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or are hydroxyl; or

are ($C_1$-$C_6$)alkylamino, di($C_1$-$C_6$)alkylamino, halo($C_1$-$C_6$)alkylamino, dihalo($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hetarylamino, dihetarylamino, ($C_1$-$C_6$)alkyl (aryl) amino, ($C_3$-$C_6$)cycloalkyl (aryl) amino, ($C_1$-$C_6$)alkylcarbonylamino, arylcarbonylamino, ($C_1$-$C_6$)alkoxycarbonylamino, aryloxycarbonylamino, ($C_1$-$C_6$)alkylcarbamoylamino, arylcarbamoylamino, ($C_1$-$C_6$)alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or are amino; or

are ($C_1$-$C_6$)alkylsulphanyl, ($C_1$-$C_6$)alkylsulphinyl, ($C_1$-$C_6$)alkylsulphonyl, halo ($C_1$-$C_6$)alkylsulphanyl, halo($C_1$-$C_6$)alkylsulphinyl, halo ($C_1$-$C_6$)alkylsulphonyl, ($C_3$-$C_6$)cycloalkylsulphanyl, ($C_3$-$C_6$)cycloalkylsulphinyl, ($C_3$-$C_6$)cycloalkylsulphonyl, ($C_3$-$C_6$)cycloalkyl($C_1$-$C_6$)alkylsulphanyl, ($C_3$-$C_6$)cycloalkyl($C_1$-$C_6$)alkylsulphinyl, ($C_3$-$C_6$)cycloalkyl($C_1$-$C_6$)alkylsulphonyl, arylsulphanyl, arylsulphinyl, arylsulphonyl, aryl($C_1$-$C_6$)alkylsulphanyl, aryl($C_1$-$C_6$)alkylsulphinyl, aryl($C_1$-$C_6$)alkylsulphonyl, aminosulphonyl, ($C_1$-$C_6$)alkylaminosulphonyl, di($C_1$-$C_6$)alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or are sulphanyl; or

are an optionally singly or multiply, identically or differently halogen-, cyano-, nitro-, hydroxyl-, amino-, ($C_1$-$C_6$)alkyl-, halo($C_1$-$C_6$)alkyl-, ($C_1$-$C_6$)alkoxy-, halo ($C_1$-$C_6$)alkoxy-, ($C_1$-$C_6$)alkylsulphinyl-, ($C_1$-$C_6$)alkylsulphanyl-, ($C_1$-$C_6$)alkylsulphonyl-, halo($C_1$-$C_6$)alkylsulphinyl-, halo($C_1$-$C_6$)alkylsulphanyl-, halo($C_1$-$C_6$)alkylsulphonyl- or optionally substituted ($C_3$-$C_6$)cycloalkyl-substituted, saturated or unsaturated three- to six-membered

ring optionally interrupted by heteroatoms from the group of O, S and N;
W is hydrogen or halogen;
V and V' each independently
are oxygen, sulphur or an optionally substituted nitrogen;
X, Y and Z each independently
are hydrogen, halogen, hydroxyl, amino, cyano, nitro, OCN, SCN, $SF_5$; or are tri($C_1$-$C_6$)alkylsilyl, ($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxycarbonyl ($C_1$-$C_6$)alkyl, ($C_1$-$C_6$)alkoxy ($C_1$-$C_6$)alkyl, ($C_2$-$C_6$)alkenyl, halo($C_2$-$C_6$)alkenyl, cyano($C_2$-$C_6$)alkenyl, ($C_2$-$C_6$)alkynyl, halo($C_2$-$C_6$)alkynyl, cyano($C_2$-$C_6$)alkynyl, ($C_1$-$C_6$)alkoxy, halo($C_1$-$C_6$)alkoxy, cyano($C_1$-$C_6$)alkoxy, hydroxycarbonyl($C_1$-$C_6$)alkoxy, ($C_1$-$C_6$)alkoxycarbonyl($C_1$-$C_6$)alkoxy, ($C_1$-$C_6$)alkoxy($C_1$-$C_6$)alkoxy, ($C_1$-$C_6$)alkylhydroxyimino, ($C_1$-$C_6$)alkoxyimino, ($C_1$-$C_6$)alkyl($C_1$-$C_6$)alkoxyimino, halo($C_1$-$C_6$)alkyl($C_1$-$C_6$)alkoxyimino, ($C_1$-$C_6$)alkylthio, halo($C_1$-$C_6$)alkylthio, ($C_1$-$C_6$)alkoxy($C_1$-$C_6$)alkylthio, ($C_1$-$C_6$)alkylthio ($C_1$-$C_6$)alkyl, ($C_1$-$C_6$)alkylsulphinyl, halo($C_1$-$C_6$)alkylsulphinyl, ($C_1$-$C_6$)alkoxy($C_1$-$C_6$)alkylsulphinyl, ($C_1$-$C_6$)alkylsulphinyl($C_1$-$C_6$)alkyl, ($C_1$-$C_6$)alkylsulphonyl, halo($C_1$-$C_6$)alkylsulphonyl, ($C_1$-$C_6$)alkoxy ($C_1$-$C_6$)alkylsulphonyl, ($C_1$-$C_6$)alkylsulphonyl($C_1$-$C_6$)alkyl, ($C_1$-$C_6$)alkylsulphonyloxy, ($C_1$-$C_6$)alkylcarbonyl, halo($C_1$-$C_6$)alkylcarbonyl, carboxyl, ($C_1$-$C_6$)alkylcarbonyloxy, ($C_1$-$C_6$)alkoxycarbonyl, halo($C_1$-$C_6$)alkoxycarbonyl, aminocarbonyl, ($C_1$-$C_6$)alkylaminocarbonyl, di($C_1$-$C_6$)alkylaminocarbonyl, ($C_2$-$C_6$)alkenylaminocarbonyl, di($C_2$-$C_6$)alkenylaminocarbonyl, ($C_3$-$C_6$)cyclo($C_1$-$C_6$)alkylaminocarbonyl, ($C_1$-$C_6$)alkylsulphonylamino, aminosulphonyl, ($C_1$-$C_6$)alkylaminosulphonyl, di($C_1$-$C_6$)alkylaminosulphonyl, ($C_1$-$C_6$)alkylsulphoximino, aminothiocarbonyl, ($C_1$-$C_6$)alkylaminothiocarbonyl or di($C_1$-$C_6$)alkylaminothiocarbonyl, where all the aforementioned radicals may optionally be substituted by halogen; or
are phenyl($C_1$-$C_3$)alkyl, phenoxy, phenyl($C_1$-$C_3$)alkyloxy, phenoxy($C_1$-$C_3$)alkyl, phenylthio, phenylthio($C_1$-$C_3$)alkyl, phenylsulphinyl, phenylsulphonyl, hetaryl($C_1$-$C_3$)alkyl, hetaryloxy, hetaryl($C_1$-$C_3$)alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or
are ($C_3$-$C_6$)cycloalkyl($C_1$-$C_3$)alkyl, ($C_3$-$C_6$)cycloalkyloxy, ($C_3$-$C_6$)cycloalkyl($C_1$-$C_3$)alkoxy, ($C_3$-$C_6$)cycloalkylthio, ($C_3$-$C_6$)cycloalkyl($C_1$-$C_3$)alkylthio, ($C_3$-$C_6$)cycloalkylsulphinyl, ($C_3$-$C_6$)cycloalkyl($C_1$-$C_3$)alkylsulphinyl, ($C_3$-$C_6$)cycloalkylsulphonyl, ($C_3$-$C_6$)cycloalkyl($C_1$-$C_3$)alkylsulphonyl or ($C_3$-$C_8$)cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or
are NR'R''
where R' and R'' each independently
are hydrogen, cyano, ($C_1$-$C_6$)alkyl, halo($C_1$-$C_6$)alkyl, cyano($C_1$-$C_6$)alkyl, hydroxy($C_1$-$C_6$)alkyl, ($C_1$-$C_6$)alkoxy ($C_1$-$C_6$)alkyl, ($C_1$-$C_6$)alkylthio ($C_1$-$C_6$)alkyl, ($C_2$-$C_6$)alkenyl, halo($C_2$-$C_6$)alkenyl, cyano($C_2$-$C_6$)alkenyl, ($C_2$-$C_6$)alkynyl, halo($C_2$-$C_6$)alkynyl, cyano($C_2$-$C_6$)alkynyl, acyl or ($C_1$-$C_6$)alkoxycarbonyl; or
R' and R'' together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five- to seven-membered ring optionally interrupted by heteroatoms from the group of O, S and N; or
are a ($C_3$-$C_6$)cycloalkyl, oxetanyl, oxolanyl, oxanyl, ($C_3$-$C_8$)cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or X and Z, or Y and Z, may form the following 5 or 6-membered rings which are optionally substituted identically or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

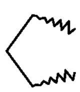 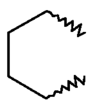 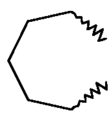 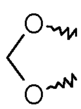 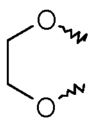 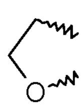 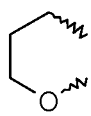

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

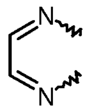 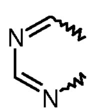 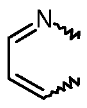 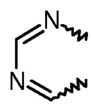 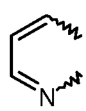 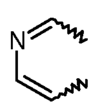

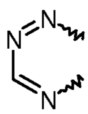 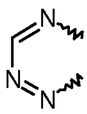 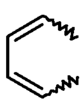

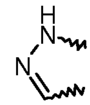 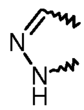 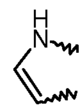

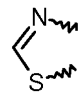 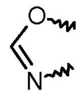 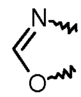

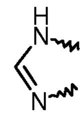 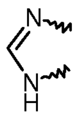 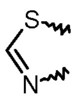

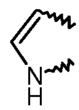 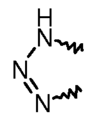 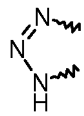

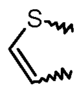 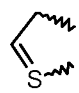 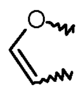 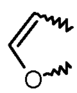

and

n is the number 0 or 1.

**16.** Compound according to Claim 15 in which the substructure of the formula (I-F) is a substructure of the formula (I-F-1)

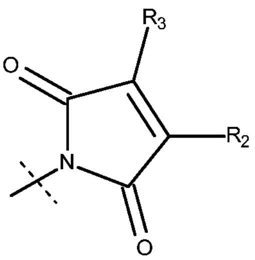

(I-F-1)

where

$R^2$ and $R^3$ each independently are hydrogen or halogen; or

are $(C_1-C_6)$alkyl, $(C_3-C_6)$cycloalkyl$(C_1-C_6)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy$(C_1-C_6)$alkyl or phenyl$(C_1-C_3)$alkyl, where the aforementioned radicals may each optionally be substituted; or

are $(C_3-C_6)$cycloalkyl, $(C_3-C_6)$cycloalkenyl or phenyl, where the aforementioned radicals may each be mono- to trisubstituted by halogen, cyano, nitro, hydroxyl, amino, methyl, ethyl, isopropyl, tert-butyl, trifluoromethyl, difluoromethyl, methoxy, trifluoromethoxy, difluoromethoxy, cyano, amino, hydroxyl, nitro, halo$(C_1-C_3)$alkylsul-

phanyl or optionally methyl-, fluorine-, chlorine-, cyano-substituted cyclopropyl;
W is hydrogen or halogen, especially F or Cl;
X and Y are each independently hydrogen, fluorine, chlorine, bromine, methyl, ethyl, trifluoromethyl, 2,2-difluoromethyl, difluoromethoxy, trifluoromethoxy, cyclopropyl, cyano, amino, hydroxyl or nitro;
Z is hydrogen; and
n is the number 0 or 1.

**17.** Compound according to any of Claims 1 to 3, in which
A and B together with the atoms to which they are bonded are a substructure of the formula (I-G)

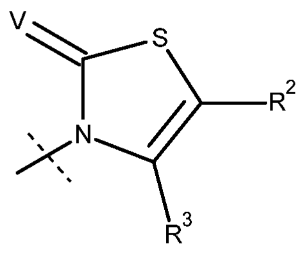

(I-G)

$R^2$ and $R^3$ each independently are hydrogen, cyano, halogen or nitro; or
are $(C_1-C_6)$alkyl, $(C_3-C_6)$cycloalkyl$(C_1-C_6)$alkyl, halo$(C_1-C_6)$alkyl, cyano$(C_1-C_6)$alkyl, hydroxy$(C_1-C_6)$alkyl, $(C_1-C_6)$alkoxy$(C_1-C_6)$alkyl, amino$(C_1-C_6)$alkyl, $(C_1-C_6)$alkoxycarbonyl$(C_1-C_6)$alkyl, $(C_1-C_6)$alkylsulphanyl $(C_1-C_6)$alkyl, $(C_1-C_6)$alkylsulphinyl$(C_1-C_6)$alkyl, $(C_1-C_6)$alkylsulphonyl$(C_1-C_6)$alkyl, halo$(C_1-C_6)$alkylsulphanyl$(C_1-C_6)$alkyl, halo$(C_1-C_6)$alkylsulphinyl$(C_1-C_6)$alkyl, halo$(C_1-C_6)$alkylsulphonyl $(C_1-C_6)$alkyl, $(C_1-C_6)$alkoxy$(C_1-C_6)$alkylsulphanyl$(C_1-C_6)$alkyl, $(C_1-C_6)$alkoxy$(C_1-C_6)$alkylsulphinyl$(C_1-C_6)$alkyl, $(C_1-C_6)$alkoxy$(C_1-C_6)$alkylsulphonyl$(C_1-C_6)$alkyl, phenyl$(C_1-C_6)$alkyl, phenoxy$(C_1-C_6)$alkyl, phenylsulphanyl$(C_1-C_6)$alkyl, phenylsulphinyl$(C_1-C_6)$alkyl, phenylsulphonyl$(C_1-C_6)$alkyl, hetaryl$(C_1-C_6)$alkyl, hetaryloxy$(C_1-C_6)$alkyl, hetarylthio$(C_1-C_6)$alkyl, where the aforementioned radicals may each optionally be substituted and/or, under the condition that, in the case of an unsaturated radical, the defined minimum number of carbon atoms is 2, the radicals may be saturated or unsaturated; or
are optionally substituted saturated or unsaturated $(C_3-C_6)$cycloalkyl which may optionally be interrupted by one or more heteroatoms; or
are $(C_1-C_6)$alkylcarbonyl, halo$(C_1-C_6)$alkylcarbonyl, hydroxy$(C_1-C_6)$alkylcarbonyl, $(C_1-C_6)$alkoxy$(C_1-C_6)$alkylcarbonyl, phenylcarbonyl, hetarylcarbonyl, $(C_1-C_6)$alkoxycarbonyl, halo$(C_1-C_6)$alkoxycarbonyl, aryloxycarbonyl, aminocarbonyl, $(C_1-C_6)$alkylaminocarbonyl, di$(C_1-C_6)$alkylaminocarbonyl, $(C_3-C_6)$cycloalkylaminocarbonyl, di$(C_3-C_6)$cycloalkylaminocarbonyl, $(C_3-C_6)$cycloalkyl($(C_1-C_6)$alkyl)aminocarbonyl, arylaminocarbonyl, diarylaminocarbonyl, $(C_1-C_6)$alkyl(aryl)aminocarbonyl, $(C_3-C_6)$cycloalkyl (aryl) aminocarbonyl, $(C_1-C_6)$alkylaminothiocarbonyl, di$(C_1-C_6)$alkylaminothiocarbonyl or aminothiocarbonyl, where the aforementioned radicals may each optionally be substituted and/or, under the condition that, in the case of an unsaturated radical, the defined minimum number of carbon atoms is 2, the radicals may be saturated or unsaturated, or are carbonyl or carboxyl; or
are optionally substituted phenyl or optionally substituted hetaryl; or
are $(C_1-C_6)$alkoxy, halo$(C_1-C_6)$alkoxy, $(C_1-C_6)$alkoxy$(C_1-C_6)$alkoxy, aryloxy, aryl$(C_1-C_6)$alkyloxy, $(C_3-C_6)$cycloalkyloxy, $(C_3-C_6)$cycloalkyl$(C_1-C_6)$alkyloxy or carbonyloxy, where the aforementioned radicals may each optionally be substituted and/or, under the condition that, in the case of an unsaturated radical, the defined minimum number of carbon atoms is 2, the radicals may be saturated or unsaturated, or are hydroxyl; or
are $(C_1-C_6)$alkylamino, di$(C_1-C_6)$alkylamino, halo$(C_1-C_6)$alkylamino, dihalo$(C_1-C_6)$alkylamino, $(C_3-C_6)$cycloalkylamino, di$(C_3-C_6)$cycloalkylamino, $(C_3-C_6)$cycloalkyl($(C_1-C_6)$alkyl)amino, arylamino, diarylamino, hetarylamino, dihetarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alkoxycarbonylamino, aryloxycarbonylamino, $(C_1-C_6)$alkylcarbamoylamino, arylcarbamoylamino, $(C_1-C_6)$alkylsulphonylamino, or arylsulphonylamino, where the aforementioned radicals may each optionally be substituted and/or, under the condition that, in the case of an unsaturated radical, the defined minimum number of carbon atoms is 2, the radicals may be saturated or unsaturated, or are amino; or
are $(C_1-C_6)$alkylsulphanyl, $(C_1-C_6)$alkylsulphinyl, $(C_1-C_6)$alkylsulphonyl, halo$(C_1-C_6)$alkylsulphanyl, halo$(C_1-C_6)$alkylsulphinyl, halo$(C_1-C_6)$alkylsulphonyl, $(C_3-C_6)$cycloalkylsulphanyl, $(C_3-C_6)$cycloalkylsulphinyl, $(C_3-C_6)$cycloalkylsulphonyl, $(C_3-C_6)$cycloalkyl$(C_1-C_6)$alkylsulphanyl, $(C_3-C_6)$cycloalkyl$(C_1-C_6)$alkylsulphinyl,

$(C_3-C_6)$cycloalkyl$(C_1-C_6)$alkylsulphonyl, arylsulphanyl, arylsulphinyl, arylsulphonyl, aryl$(C_1-C_6)$alkylsulphanyl, aryl$(C_1-C_6)$alkylsulphinyl, aryl$(C_1-C_6)$alkylsulphonyl, aminosulphonyl, $(C_1-C_6)$alkylaminosulphonyl, di$(C_1-C_6)$alkylaminosulphonyl or arylaminosulphonyl, where the aforementioned radicals may each optionally be substituted and/or, under the condition that, in the case of an unsaturated radical, the defined minimum number of carbon atoms is 2, the radicals may be saturated or unsaturated, or are sulphanyl; or

are an optionally singly or multiply, identically or differently halogen-, cyano-, nitro-, hydroxyl-, amino-, $(C_1-C_6)$alkyl-, halo$(C_1-C_6)$alkyl-, $(C_1-C_6)$alkoxy-, halo$(C_1-C_6)$alkoxy-, $(C_1-C_6)$alkylsulphinyl-, $(C_1-C_6)$alkylsulphanyl-, $(C_1-C_6)$alkylsulphonyl-, halo$(C_1-C_6)$alkylsulphinyl-, halo$(C_1-C_6)$alkylsulphanyl-, halo$(C_1-C_6)$alkylsulphonyl- or optionally substituted $(C_3-C_6)$cycloalkyl-substituted, saturated or unsaturated three- to six-membered ring optionally interrupted by heteroatoms from the group of O, S and N;

W is hydrogen or halogen;

V is oxygen, sulphur or an optionally substituted nitrogen;

X, Y and Z each independently

are hydrogen, halogen, hydroxyl, amino, cyano, nitro, OCN, SCN, $SF_5$; or

are tri$(C_1-C_6)$alkylsilyl, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxycarbonyl$(C_1-C_6)$alkyl, $(C_1-C_6)$alkoxy$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, $(C_1-C_6)$alkoxy, halo$(C_1-C_6)$alkoxy, cyano$(C_1-C_6)$alkoxy, hydroxycarbonyl$(C_1-C_6)$alkoxy, $(C_1-C_6)$alkoxycarbonyl $(C_1-C_6)$alkoxy, $(C_1-C_6)$alkoxy$(C_1-C_6)$alkoxy, $(C_1-C_6)$alkylhydroxyimino, $(C_1-C_6)$alkoxyimino, $(C_1-C_6)$alkyl$(C_1-C_6)$alkoxyimino, halo$(C_1-C_6)$alkyl$(C_1-C_6)$alkoxyimino, $(C_1-C_6)$alkylthio, halo$(C_1-C_6)$alkylthio, $(C_1-C_6)$alkoxy$(C_1-C_6)$alkylthio, $(C_1-C_6)$alkylthio $(C_1-C_6)$alkyl, $(C_1-C_6)$alkylsulphinyl, halo$(C_1-C_6)$alkylsulphinyl, $(C_1-C_6)$alkoxy$(C_1-C_6)$alkylsulphinyl, $(C_1-C_6)$alkylsulphinyl$(C_1-C_6)$alkyl, $(C_1-C_6)$alkylsulphonyl, halo$(C_1-C_6)$alkylsulphonyl, $(C_1-C_6)$alkoxy$(C_1-C_6)$alkylsulphonyl, $(C_1-C_6)$alkylsulphonyl$(C_1-C_6)$alkyl, $(C_1-C_6)$alkylsulphonyloxy, $(C_1-C_6)$alkylcarbonyl, halo$(C_1-C_6)$alkylcarbonyl, carboxyl, $(C_1-C_6)$alkylcarbonyloxy, $(C_1-C_6)$alkoxycarbonyl, halo $(C_1-C_6)$alkoxycarbonyl, aminocarbonyl, $(C_1-C_6)$alkylaminocarbonyl, di$(C_1-C_6)$alkylaminocarbonyl, $(C_2-C_6)$alkenylaminocarbonyl, di$(C_2-C_6)$alkenylaminocarbonyl, $(C_3-C_6)$cyclo$(C_1-C_6)$alkylaminocarbonyl, $(C_1-C_6)$alkylsulphonylamino, aminosulphonyl, $(C_1-C_6)$alkylaminosulphonyl, di$(C_1-C_6)$alkylaminosulphonyl, $(C_1-C_6)$alkylsulphoximino, aminothiocarbonyl, $(C_1-C_6)$alkylaminothiocarbonyl or di$(C_1-C_6)$alkylaminothiocarbonyl, where all the aforementioned radicals may optionally be substituted by halogen; or

are phenyl$(C_1-C_3)$alkyl, phenoxy, phenyl$(C_1-C_3)$alkyloxy, phenoxy$(C_1-C_3)$alkyl, phenylthio, phenylthio$(C_1-C_3)$alkyl, phenylsulphinyl, phenylsulphonyl, hetaryl$(C_1-C_3)$alkyl, hetaryloxy, hetaryl$(C_1-C_3)$alkyloxy, hetarylthio, hetarylsulphinyl or hetarylsulphonyl, where all the aforementioned radicals may optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are $(C_3-C_6)$cycloalkyl$(C_1-C_3)$alkyl, $(C_3-C_6)$cycloalkyloxy, $(C_3-C_6)$cycloalkyl$(C_1-C_3)$alkoxy, $(C_3-C_6)$cycloalkylthio, $(C_3-C_6)$cycloalkyl$(C_1-C_3)$alkylthio, $(C_3-C_6)$cycloalkylsulphinyl, $(C_3-C_6)$cycloalkyl$(C_1-C_3)$alkylsulphinyl, $(C_3-C_6)$cycloalkylsulphonyl, $(C_3-C_6)$cycloalkyl$(C_1-C_3)$alkylsulphonyl or $(C_3-C_8)$cycloalkenyl, where the aforementioned radicals may each optionally be mono- to trisubstituted by halogen, cyano, nitro, amino, hydroxyl, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; or

are NR'R"

where R' and R" each independently

are hydrogen, cyano, $(C_1-C_6)$alkyl, halo$(C_1-C_6)$alkyl, cyano$(C_1-C_6)$alkyl, hydroxy$(C_1-C_6)$alkyl, $(C_1-C_6)$alkoxy $(C_1-C_6)$alkyl, $(C_1-C_6)$alkylthio$(C_1-C_6)$alkyl, $(C_2-C_6)$alkenyl, halo$(C_2-C_6)$alkenyl, cyano$(C_2-C_6)$alkenyl, $(C_2-C_6)$alkynyl, halo$(C_2-C_6)$alkynyl, cyano$(C_2-C_6)$alkynyl, acyl or $(C_1-C_6)$alkoxycarbonyl; or

R' and R" together with the nitrogen atom to which they are bonded may form an optionally identically or differently halogen-, cyano-, nitro-, hydroxyl-, amino-, methyl-, ethyl-, trifluoromethyl-, difluoromethyl-, trifluoroethyl-, difluoroethyl-, methoxy-, ethoxy-, trifluoromethoxy-, trifluoroethoxy- or optionally methyl-, fluorine-, chlorine-, cyano-substituted cyclopropyl-substituted, saturated or unsaturated five- to seven-membered ring optionally interrupted by heteroatoms from the group of O, S and N; or

are a $(C_3-C_6)$cycloalkyl, oxetanyl, oxolanyl, oxanyl, $(C_3-C_8)$cycloalkenyl, phenyl, pyridyl, pyrimidyl, pyridazinyl, pyrazinyl, triazinyl, thiazolyl, thiadiazolyl, oxazolyl, oxadiazolyl, pyrazolyl or triazolyl, which may optionally be singly or multiply, identically or differently substituted by a substituent S1 which is selected from the group consisting of halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;

or X and Z, or Y and Z, may form the following 5 or 6-membered rings which are optionally substituted identically

or differently by hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

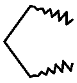 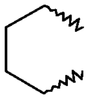 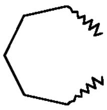 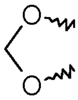 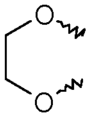 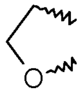 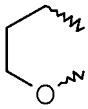

or X and Z, or Y and Z, may form the following fused rings which are optionally mono- or polysubstituted identically or differently, where the substituents may each independently be selected from hydrogen, halogen, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl:

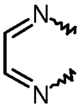 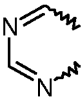 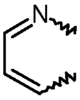 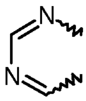 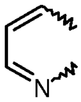 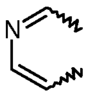

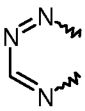 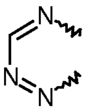 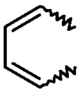 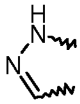 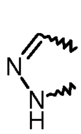 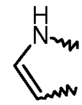

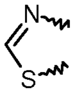 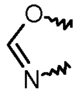 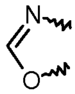

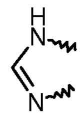 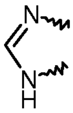 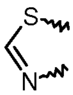

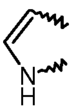 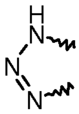 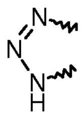

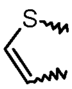 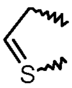 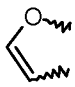 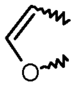

and
n is the number 0 or 1.

**18.** Compound according to Claim 17 in which the substructure of the formula (I-G) is a substructure (I-G-1)

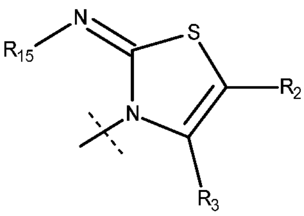

(I-G-1)

where

$R^2$ and $R^3$ each independently are hydrogen; or
are $(C_1-C_6)$alkyl, $(C_3-C_6)$cycloalkyl$(C_1-C_3)$alkyl, $(C_2-C_6)$alkenyl, $(C_2-C_6)$alkynyl, halo$(C_1-C_6)$alkyl, cyano$(C_1-C_6)$alkyl, $(C_1-C_6)$alkoxy$(C_1-C_6)$alkyl or phenyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl; or
are $(C_3-C_6)$cycloalkyl, $(C_3-C_6)$cycloalkenyl, azetidinyl, azolidinyl, azinanyl, oxetanyl, oxolanyl, oxanyl, thiethanyl, thiolanyl, thianyl, phenyl, pyrimidyl, pyridazinyl, pyrazinyl, triazinyl, thiazolyl, thiazadiazolyl, oxazolyl, oxadiazolyl, pyrazolyl, triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo $(C_1-C_6)$alkylsulphanyl, halo$(C_1-C_6)$alkylsulphonyl or optionally methyl-, fluorine-, chlorine-, cyano-substituted cyclopropyl;
$R^{15}$ is hydrogen; or
is $(C_1-C_6)$alkyl, $(C_3-C_6)$cycloalkyl$(C_1-C_3)$alkyl, $(C_2-C_6)$alkenyl, $(C_2-C_6)$alkynyl, halo$(C_2-C_6)$alkyl, cyano$(C_1-C_6)$alkyl, $(C_1-C_6)$alkoxy$(C_1-C_6)$alkyl or phenyl$(C_1-C_3)$alkyl, where the aforementioned radicals may each optionally be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo$(C_1-C_6)$alkylsulphanyl, halo$(C_1-C_6)$alkylsulphonyl or optionally methyl-, fluorine-, chlorine-, cyano-substituted cyclopropyl; or
is $(C_3-C_6)$cycloalkyl, $(C_3-C_6)$cycloalkenyl, oxetanyl, oxolanyl, oxanyl, phenyl, pyrimidyl, pyridazinyl, pyrazinyl, triazinyl, thiazolyl, thiazadiazolyl, oxazolyl, oxadiazolyl, pyrazolyl or triazolyl, where the aforementioned radicals may each be mono- to trisubstituted by halogen, cyano, nitro, hydroxyl, amino, $(C_1-C_6)$alkyl, halo$(C_1-C_6)$alkyl, $(C_1-C_6)$alkoxy, halo$(C_1-C_6)$alkoxy, $(C_1-C_6)$alkylsulphinyl, $(C_1-C_6)$alkylsulphanyl, $(C_1-C_6)$alkylsulphonyl, halo$(C_1-C_6)$alkylsulphinyl, halo$(C_1-C_6)$alkylsulphanyl, halo$(C_1-C_6)$alkylsulphonyl or optionally methyl-, fluorine-, chlorine-, cyano-substituted cyclopropyl;
W is hydrogen or halogen, especially fluorine or chlorine;
X, Y and Z are each independently hydrogen, fluorine, chlorine, bromine, cyano, nitro, amino, hydroxyl, $(C_1-C_4)$alkyl, $(C_1-C_4)$haloalkyl, $(C_2-C_4)$alkenyl, $(C_2-C_4)$alkynyl, $(C_1-C_4)$alkoxy, $(C_1-C_4)$haloalkoxy or aminothiocarbonyl;
or are a benzyl, phenoxy, phenylthio, cyclopropylmethyl, cyclopropyloxy or cyclopropylthio, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl;
or are a cyclopropyl, cyclobutyl, cyclopentyl, cyclohexyl or phenyl, which is optionally mono- or polysubstituted identically or differently by fluorine, chlorine, bromine, cyano, nitro, hydroxyl, amino, methyl, ethyl, trifluoromethyl, difluoromethyl, trifluoroethyl, difluoroethyl, methoxy, ethoxy, trifluoromethoxy, trifluoroethoxy or optionally methyl-, fluorine-, chlorine-, cyano-substituted cyclopropyl; and
n is the number 0 or 1.

**19.** Active ingredient composition comprising at least one compound of the general formula (I) according to any of Claims 1 to 18 and at least one further insecticidally, acaricidally or nematicidally active ingredient selected from the group consisting of

(1) Acetylcholinesterase (AChE) inhibitors, for example
carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or
organophosphates, for example acephate, azamethiphos, azinphos-ethyl, azinphos-methyl, cadusafos, chlorethoxyfos, chlorfenvinphos, chlormephos, chloropyrifos, chloropyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon and vamidothion.

(2) GABA-gated chloride channel antagonists, for example
cyclodiene organochlorines, e.g. chlordane and endosulfan; or
phenylpyrazoles (fiproles), e.g. ethiprole and fipronil.
(3) Sodium channel modulators/voltage-dependent sodium channel blockers, for example
pyrethroids, for example acrinathrin, allethrin, d-cis-trans allethrin, d-trans allethrin, bifenthrin, bioallethrin, bioallethrin S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomers], deltamethrin, empenthrin [(EZ)-(1R) isomers], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrine (pyrethrum), resmethrin, silafluofen, tefluthrin, tetramethrin, tetramethrin [(1R) isomers], tralomethrin and transfluthrin; or
DDT; or methoxychlor.
(4) Nicotinergic acetylcholine receptor (nAChR) agonists, for example
neonicotinoids, for example acetamiprid, clothianidin, dinotefuran, imidacloprid, nitenpyram, thiacloprid and thiamethoxam; or nicotine.
(5) Nicotinergic acetylcholine receptor (nAChR) allosteric activators, for example
spinosyns, for example spinetoram and spinosad.
(6) Chloride channel activators, for example
avermectins/milbemycins, for example abamectin, emamectin benzoate, lepimectin and milbemectin.
(7) Juvenile hormone imitators, for example
juvenile hormone analogs, for example hydroprene, kinoprene and methoprene; or
fenoxycarb; or pyriproxyfen.
(8) Active ingredients with unknown or nonspecific mechanisms of action, for example
alkyl halides, for example methyl bromide and other alkyl halides; or
chloropicrin; or sulphuryl fluoride; or borax; or tartar emetic.
(9) Selective antifeedants, for example pymetrozine; or flonicamid.
(10) Mite growth inhibitors, for example clofentezine, hexythiazox and diflovidazin; or etoxazole.
(11) Microbial disruptors of the insect gut membrane, for example Bacillus thuringiensis subspecies israelensis, Bacillus sphaericus, Bacillus thuringiensis subspecies aizawai, Bacillus thuringiensis subspecies kurstaki, Bacillus thuringiensis subspecies tenebrionis, and BT plant proteins: Cry1Ab, Cry1Ac, Cry1Fa, Cry2Ab, mCry3A, Cry3Ab, Cry3Bb, Cry34/35Ab1.
(12) Oxidative phosphorylation inhibitors, ATP disruptors, for example diafenthiuron; or
organotin compounds, e.g. azocyclotin, cyhexatin and fenbutatin oxide; or
propargite; or tetradifon.
(13) Oxidative phosphorylation decouplers that interrupt the H proton gradient, for example chlorfenapyr, DNOC and sulfluramid.
(14) Nicotinergic acetylcholine receptor antagonists, for example bensultap, cartap hydrochloride, thiocyclam, and thiosultap-sodium.
(15) Chitin biosynthesis inhibitors, type 0, for example bistrifluron, chlorfluazuron, diflubenzuron, flucycloxuron, flufenoxuron, hexaflumuron, lufenuron, novaluron, noviflumuron, teflubenzuron and triflumuron.
(16) Chitin biosynthesis inhibitors, type 1, for example buprofezin.
(17) Molting disruptors, dipteran, for example cyromazine.
(18) Ecdysone receptor agonists, for example chromafenozide, halofenozide, methoxyfenozide and tebufenozide.
(19) Octopaminergic agonists, for example amitraz.
(20) Complex-III electron transport inhibitors, for example hydramethylnon; or acequinocyl; or fluacrypyrim.
(21) Complex-I electron transport inhibitors, for example
METI acaricides, for example fenazaquin, fenpyroximate, pyrimidifen, pyridaben, tebufenpyrad and tolfenpyrad; or
rotenone (Derris).
(22) Voltage-dependent sodium channel blockers, for example indoxacarb; or metaflumizone.
(23) Inhibitors of acetyl-CoA carboxylase, for example
tetronic and tetramic acid derivatives, for example spirodiclofen, spiromesifen and spirotetramat.
(24) Complex-IV electron transport inhibitors, for example
phosphines, for example aluminium phosphide, calcium phosphide, phosphine and zinc phosphide; or
cyanide.
(25) Complex-II electron transport inhibitors, for example cyenopyrafen.
(28) Ryanodine receptor effectors, for example

diamides, for example chlorantraniliprole and flubendiamide;

further active ingredients having an unknown mechanism of action, for example amidoflumet, azadirachtin, benclothiaz, benzoximate, bifenazate, bromopropylate, chinomethionat, cryolite, cyantraniliprole (Cyazypyr), cyflumetofen, dicofol, diflovidazin, fluensulfone, flufenerim, flufiprole, fluopyram, fufenozide, imidaclothiz, iprodione, meperfluthrin, pyridalyl, pyrifluquinazon, tetramethylfluthrin and iodomethane; and additionally preparations based on Bacillus firmus (particularly strain CNCM 1-1582, for example VOTiVO™, BioNem), and the following known active compounds:

3-bromo-N-{2-bromo-4-chloro-6-[(1-cyclopropylethyl)carbamoyl]phenyl}-1-(3-chloropyridin-2-yl)-1H-pyrazole-5-carboxamide, 4-{[(6-bromopyrid-3-yl)methyl](2-fluoroethyl)amino}furan-2(5H)-one, 4-{[(6-fluoropyrid-3-yl)methyl](2,2-difluoroethyl)amino}furan-2(5H)-one, 4-{[(2-chloro-1,3-thiazol-5-yl)methyl](2-fluoroethyl)amino}furan-2(5H)-one, 4-{[(6-chloropyrid-3-yl)methyl](2-fluoroethyl)amino}furan-2(5H)-one, flupyradifurone, 4-{[(6-chloro-5-fluoropyrid-3-yl)methyl](methyl)amino}furan-2(5H)-one, 4-{[(5,6-dichloropyrid-3-yl)methyl](2-fluoroethyl)amino}furan-2(5H)-one, 4-{[(6-chloro-5-fluoropyrid-3-yl)methyl](cyclopropyl)amino}furan-2(5H)-one, 4-{[(6-chloropyrid-3-yl)methyl](cyclopropyl)amino}furan-2(5H)-one, 4-{[(6-chloropyrid-3-yl)methyl](methyl)amino}furan-2(5H)-one, {[1-(6-chloropyridin-3-yl)ethyl](methyl)oxido-$\lambda^4$-sulphanylidene}cyanamide and its diastereomers {[(1R)-1-(6-chloropyridin-3-yl)ethyl](methyl)oxido-$\lambda^4$-sulphanylidene}cyanamide (A) and {[(1S)-1-(6-chloropyridin-3-yl)ethyl] (methyl)oxido-$\lambda^4$-sulphanylidene}cyanamide (B) and sulfoxaflor and its diastereomers [(R)-methyl(oxido){(1R)-1-[6-(trifluoromethyl)pyridin-3-yl]ethyl}-$\lambda^4$-sulphanylidene]cyanamide (A1) and [(S)-methyl(oxido){(1S)-1-[6-(trifluoromethyl)pyridin-3-yl]ethyl}-$\lambda^4$-sulphanylidene]cyanamide (A2), identified as diastereomer group A, [(R)-methyl(oxido){(1S)-1-[6-(trifluoromethyl)pyridin-3-yl]ethyl}-$\lambda^4$-sulphanylidene]cyanamide (B1) and [(S)-methyl(oxido){(1R)-1-[6-(trifluoromethyl)pyridin-3-yl]ethyl}-$\lambda^4$-sulphanylidene]cyanamide (B2), identified as diastereomer group B and 11-(4-chloro-2,6-dimethylphenyl)-12-hydroxy-1,4-dioxa-9-azadispiro[4.2.4.2]tetradec-11-en-10-one, 3-(4'-fluoro-2,4-dimethylbiphenyl-3-yl)-4-hydroxy-8-oxa-1-azaspiro[4.5]dec-3-en-2-one, 1-{2-fluoro-4-methyl-5-[(2,2,2-trifluoroethyl)sulphinyl]phenyl}-3-(trifluoromethyl)-1H-1,2,4-triazol-5-amine, [(3S,4aR,12R,12aS,12bS)-3-[(cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methyl cyclopropanecarboxylate, 2-cyano-3-(difluoromethoxy)-N,N-dimethylbenzenesulphonamide, 2-cyano-3-(difluoromethoxy)-N-methylbenzenesulphonamide, 2-cyano-3-(difluoromethoxy)-N-ethylbenzenesulphonamide, 4-(difluoromethoxy)-N-ethyl-N-methyl-1,2-benzothiazol-3-amine-1,1-dioxide, N-[1-(2,3-dimethylphenyl)-2-(3,5-dimethylphenyl)ethyl]-4,5-dihydro-1,3-thiazol-2-amine, {1'-[(2E)-3-(4-chlorophenyl)prop-2-en-1-yl]-5-fluorospiro[indol-3,4'-piperidine]-1(2H)-yl} (2-chloropyridin-4-yl)methanone, 3-(2,5-dimethylphenyl)-4-hydroxy-8-methoxy-1,8-diazaspiro[4.5]dec-3-en-2-one, 3-(2,5-dimethylphenyl)-8-methoxy-2-oxo-1,8-diazaspiro[4.5]dec-3-en-4-yl ethyl carbonate, 4-(but-2-yn-1-yloxy)-6-(3,5-dimethylpiperidin-1-yl)-5-fluoropyrimidine, (2,2,3,3,4,4,5,5-octafluoropentyl)(3,3,3-trifluoropropyl)malononitrile, (2,2,3,3,4,4,5,5-octafluoropentyl)(3,3,4,4,4-pentafluorobutyl)malononitrile, 8-[2-(cyclopropylmethoxy)-4-(trifluoromethyl)phenoxy]-3-[6-(trifluoromethyl)pyridazin-3-yl]-3-azabicyclo[3.2.1]octane, flometoquin, PF1364 (CAS Reg. No. 1204776-60-2), 5-[5-(3,5-dichlorophenyl)-5-(trifluoromethyl)-4,5-dihydro-1,2-oxazol-3-yl]-2-(1H-1,2,4-triazol-1-yl)benzonitrile, 5-[5-(2-chloropyridin-4-yl)-5-(trifluoromethyl)-4,5-dihydro-1,2-oxazol-3-yl]-2-(1H-1,2,4-triazol-1-yl)benzonitrile, 4-[5-(3,5-dichlorophenyl)-5-(trifluoromethyl)-4,5-dihydro-1,2-oxazol-3-yl]-2-methyl-N-{2-oxo-2-[(2,2,2-trifluoroethyl)amino]ethyl}benzamide, 4-{[(6-chloropyridin-3-yl)methyl](cyclopropyl)amino}-1,3-oxazol-2(5H)-one, 4-{[(6-chloropyridin-3-yl)methyl](2,2-difluoroethyl)amino}-1,3-oxazol-2(5H)-one, 4-{[(6-chloropyridin-3-yl)methyl](ethyl)amino}-1,3-oxazol-2(5H)-one, 4-{[(6-chloropyridin-3-yl)methyl](methyl)amino}-1,3-oxazol-2(5H)-one, NNI-0711, 1-acetyl-N-[4-(1,1,1,3,3,3-hexafluoro-2-methoxypropan-2-yl)-3-isobutylphenyl]-N-isobutyryl-3,5-dimethyl-1H-pyrazole-4-carboxamide, methyl 2-[2-({[3-bromo-1-(3-chloropyridin-2-yl)-1H-pyrazol-5-yl]carbonyl}amino)-5-chloro-3-methylbenzoyl]-2-methylhydrazinecarboxylate, methyl 2-[2-({[3-bromo-1-(3-chloropyridin-2-yl)-1H-pyrazol-5-yl]carbonyl}amino)-5-cyano-3-methylbenzoyl]-2-ethylhydrazinecarboxylate, methyl 2-[2-({[3-bromo-1-(3-chloropyridin-2-yl)-1H-pyrazol-5-yl]carbonyl}amino)-5-cyano-3-methylbenzoyl]-2-methylhydrazinecarboxylate, methyl 2-[3,5-dibromo-2-({[3-bromo-1-(3-chloropyridin-2-yl)-1H-pyrazol-5-yl]carbonyl}amino)benzoyl]-1,2-diethylhydrazinecarboxylate, methyl 2-[3,5-dibromo-2-({[3-bromo-1-(3-chloropyridin-2-yl)-1H-pyrazol-5-yl]carbonyl}amino)benzoyl]-2-ethylhydrazinecarboxylate, (5RS,7RS;5RS,7SR)-1-(6-chloro-3-pyridylmethyl)-1,2,3,5,6,7-hexahydro-7-methyl-8-nitro-5-propoxyimidazo[1,2-a]pyridine, 2-{6-[2-(5-fluoropyridin-3-yl)-1,3-thiazol-5-yl]pyridin-2-yl}pyrimidine, 2-{6-[2-(pyridin-3-yl)-1,3-thiazol-5-yl]pyridin-2-yl}pyrimidine, 1-(3-chloropyridin-2-yl)-N-[4-cyano-2-methyl-6-(methylcarbamoyl)phenyl]-3-{[5-(trifluoromethyl)-1H-tetrazol-1-yl]methyl}-1H-pyrazole-5-carboxamide, 1-(3-chloropyridin-2-yl)-N-[4-cyano-2-methyl-6-(methylcarbamoyl)phenyl]-3-{[5-(trifluoromethyl)-2H-tetrazol-2-yl]methyl}-1H-pyrazole-5-carboxamide, N-[2-(tert-butylcarbamoyl)-4-cyano-6-methylphenyl]-1-(3-chloropyridin-2-yl)-3-{[5-(trifluoromethyl)-1H-tetrazol-1-yl]methyl}-1H-pyrazole-5-carboxamide, N-[2-(tert-butylcarbamoyl)-4-cyano-6-methylphenyl]-1-(3-chloropyridin-2-yl)-3-{[5-(trifluoromethyl)-2H-tetrazol-2-yl]methyl}-1H-pyrazole-5-carboxamide,

(1E)-N-[(6-chloropyridin-3-yl)methyl]-N'-cyano-N-(2,2-difluoroethyl)ethanimideamide, N-[2-(5-amino-1,3,4-thiadiazol-2-yl)-4-chloro-6-methylphenyl]-3-bromo-1-(3-chloropyridin-2-yl)-1H-pyrazole-5-carboxamide and methyl 2-[3,5-dibromo-2-({[3-bromo-1-(3-chloropyridin-2-yl)-1H-pyrazol-5-yl]carbonyl}amino)benzoyl]-2-ethyl-1-methylhydrazinecarboxylate,

and/or at least one further active fungicidal ingredient selected from the group consisting of

(1) Inhibitors of ergosterol biosynthesis, for example (1.1) aldimorph (1704-28-5), (1.2) azaconazole (60207-31-0), (1.3) bitertanol (55179-31-2), (1.4) bromuconazole (116255-48-2), (1.5) cyproconazole (113096-99-4), (1.6) diclobutrazole (75736-33-3), (1.7) difenoconazole (119446-68-3), (1.8) diniconazole (83657-24-3), (1.9) diniconazole-M (83657-18-5), (1.10) dodemorph (1593-77-7), (1.11) dodemorph acetate (31717-87-0), (1.12) epoxiconazole (106325-08-0), (1.13) etaconazole (60207-93-4), (1.14) fenarimol (60168-88-9), (1.15) fenbuconazole (114369-43-6), (1.16) fenhexamid (126833-17-8), (1.17) fenpropidin (67306-00-7), (1.18) fenpropimorph (67306-03-0), (1.19) fluquinconazole (136426-54-5), (1.20) flurprimidol (56425-91-3), (1.21) flusilazole (85509-19-9), (1.22) flutriafol (76674-21-0), (1.23) furconazole (112839-33-5), (1.24) furconazole-cis (112839-32-4), (1.25) hexaconazole (79983-71-4), (1.26) imazalil (60534-80-7), (1.27) imazalil sulfate (58594-72-2), (1.28) imibenconazole (86598-92-7), (1.29) ipconazole (125225-28-7), (1.30) metconazole (125116-23-6), (1.31) myclobutanil (88671-89-0), (1.32) naftifin (65472-88-0), (1.33) nuarimol (63284-71-9), (1.34) oxpoconazole (174212-12-5), (1.35) paclobutrazole (76738-62-0), (1.36) pefurazoate (101903-30-4), (1.37) penconazole (66246-88-6), (1.38) piperalin (3478-94-2), (1.39) prochloraz (67747-09-5), (1.40) propiconazole (60207-90-1), (1.41) prothioconazole (178928-70-6), (1.42) pyributicarb (88678-67-5), (1.43) pyrifenox (88283-41-4), (1.44) quinconazole (103970-75-8), (1.45) simeconazole (149508-90-7), (1.46) spiroxamine (118134-30-8), (1.47) tebuconazole (107534-96-3), (1.48) terbinafin (91161-71-6), (1.49) tetraconazole (112281-77-3), (1.50) triadimefon (43121-43-3), (1.51) triadimenol (89482-17-7), (1.52) tridemorph (81412-43-3), (1.53) triflumizole (68694-11-1), (1.54) triforine (26644-46-2), (1.55) triticonazole (131983-72-7), (1.56) uniconazole (83657-22-1), (1.57) uniconazole-p (83657-17-4), (1.58) viniconazole (77174-66-4), (1.59) voriconazole (137234-62-9), (1.60) 1-(4-chlorophenyl)-2-(1H-1,2,4-triazol-1-yl)cycloheptanol (129586-32-9), (1.61) methyl 1-(2,2-dimethyl-2,3-dihydro-1H-inden-1-yl)-1H-imidazole-5-carboxylate (110323-95-0), (1.62) N'-{5-(difluoromethyl)-2-methyl-4-[3-(trimethylsilyl)propoxy]phenyl}-N-ethyl-N-methylimidoformamide, (1.63) N-ethyl-N-methyl-N'-{2-methyl-5-(trifluoromethyl)-4-[3-(trimethylsilyl)propoxy]phenyl}imidoformamide and (1.64) O-[1-(4-methoxyphenoxy)-3,3-dimethylbutan-2-yl] 1H-imidazole-1-carbothioate (111226-71-2).

(2) Respiration inhibitors (respiratory chain inhibitors), for example (2.1) bixafen (581809-46-3), (2.2) boscalid (188425-85-6), (2.3) carboxin (5234-68-4), (2.4) diflumetorim (130339-07-0), (2.5) fenfuram (24691-80-3), (2.6) fluopyram (658066-35-4), (2.7) flutolanil (66332-96-5), (2.8) fluxapyroxad (907204-31-3), (2.9) furametpyr (123572-88-3), (2.10) furmecyclox (60568-05-0), (2.11) isopyrazam mixture of the syn-epimeric racemate 1RS,4SR,9RS and the anti-empimeric racemate 1RS,4SR,9SR (881685-58-1), (2.12) isopyrazam (anti-epimeric racemate), (2.13) isopyrazam (anti-epimeric enantiomer 1R,4S,9S), (2.14) isopyrazam (anti-epimeric enantiomer 1S,4R,9R), (2.15) isopyrazam (syn-epimeric racemate 1RS,4SR,9RS), (2.16) isopyrazam (syn-epimeric enantiomer 1R,4S,9R), (2.17) isopyrazam (syn-epimeric enantiomer 1S,4R,9S), (2.18) mepronil (55814-41-0), (2.19) oxycarboxin (5259-88-1), (2.20) penflufen (494793-67-8), (2.21) penthiopyrad (183675-82-3), (2.22) sedaxane (874967-67-6), (2.23) thifluzamid (130000-40-7), (2.24) 1-methyl-N-[2-(1,1,2,2-tetrafluoroethoxy)phenyl]-3-(trifluoromethyl)-1H-pyrazole-4-carboxamide, (2.25) 3-(difluoromethyl)-1-methyl-N-[2-(1,1,2,2-tetrafluoroethoxy)phenyl]-1H-pyrazole-4-carboxamide, (2.26) 3-(difluoromethyl)-N-[4-fluoro-2-(1,1,2,3,3,3-hexafluoropropoxy)phenyl]-1-methyl-1H-pyrazole-4-carboxamide, (2.27) N-[1-(2,4-dichlorophenyl)-1-methoxypropan-2-yl]-3-(difluoromethyl)-1-methyl-1H-pyrazole-4-carboxamide (1092400-95-7), (2.28) 5,8-difluoro-N-[2-(2-fluoro-4-{[4-(trifluoromethyl)pyridin-2-yl]oxy}phenyl)ethyl]quinazoline-4-amine (1210070-84-0), (2.29) N-[9-(dichloromethylene)-1,2,3,4-tetrahydro-1,4-methanonaphthalen-5-yl]-3-(difluoromethyl)-1-methyl-1H-pyrazole-4-carboxamide, (2.30) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide and (2.31) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide.

(3) Respiration inhibitors (respiratory chain inhibitors) that act on complex III of the respiratory chain, for example (3.1) ametoctradin (865318-97-4), (3.2) amisulbrom (348635-87-0), (3.3) azoxystrobin (131860-33-8), (3.4) cyazofamid (120116-88-3), (3.5) coumethoxystrobin (850881-30-0), (3.6) coumoxystrobin (850881-70-8), (3.5) dimoxystrobin (141600-52-4), (3.6) enestroburin (238410-11-2), (3.9) famoxadone (131807-57-3), (3.10) fenamidone (161326-34-7), (3.11) fenoxystrobin (918162-02-4), (3.12) fluoxastrobin (361377-29-9), (3.13) kresoxim-methyl (143390-89-0), (3.14) metominostrobin (133408-50-1),

(3.15) orysastrobin (189892-69-1), (3.16) picoxystrobin (117428-22-5), (3.17) pyraclostrobin (175013-18-0), (3.18) pyrametostrobin (915410-70-7), (3.19) pyraoxystrobin (862588-11-2), (3.20) pyribencarb (799247-52-2), (3.21) triclopyricarb (902760-40-1), (3.22) trifloxystrobin (141517-21-7), (3.23) (2E)-2-(2-{[6-(3-chloro-2-methylphenoxy)-5-fluoropyrimidin-4-yl]oxy}phenyl)-2-(methoxyimino)-N-methylethanamide, (3.24) (2E)-2-(methoxyimino)-N-methyl-2-(2-{[({(1E)-1-[3-(trifluoromethyl)phenyl]ethylidene}amino)oxy]methyl} phenyl)ethanamide, (3.25) (2E)-2-(methoxyimino)-N-methyl-2-{2-[(E)-({1-[3-(trifluoromethyl)phenyl]ethoxy}imino)methyl]phenyl} ethanamide (158169-73-4), (3.26) (2E)-2-{2-[({[(1E)-1-(3-{[(E)-1-fluoro-2-phenylethenyl]oxy}phenyl)ethylidene]amino}oxy)methyl] phenyl}-2-(methoxyimino)-N-methylethanamide (326896-28-0), (3.27) (2E)-2-{2-[({[(2E,3E)-4-(2,6-dichlorophenyl)but-3-en-2-ylidene]amino}oxy)methyl]phenyl}-2-(methoxyimino)-N-methylethanamide, (3.28) 2-chloro-N-(1,1,3-trimethyl-2,3-dihydro-1H-inden-4-yl)pyridine-3-carboxamide (119899-14-8), (3.29) 5-methoxy-2-methyl-4-(2-{[({(1E)-1-[3-(trifluoromethyl)phenyl]ethylidene}amino)oxy]methyl} phenyl)-2,4-dihydro-3H-1,2,4-triazol-3-one, (3.30) methyl (2E)-2-{2-[({cyclopropyl[(4-methoxyphenyl)imino]methyl}sulphanyl)methyl]phenyl}-3-methoxyprop-2-enoate (149601-03-6), (3.31) N-(3-ethyl-3,5,5-trimethylcyclohexyl)-3-(formylamino)-2-hydroxybenzamide (226551-21-9), (3.32) 2-{2-[(2,5-dimethylphenoxy)methyl]phenyl}-2-methoxy-N-methylacetamide (173662-97-0) and (3.33) (2R)-2-{2-[(2,5-dimethylphenoxy)methyl]phenyl}-2-methoxy-N-methylacetamide (394657-24-0).

(4) Inhibitors of mitosis and cell division, for example (4.1) benomyl (17804-35-2), (4.2) carbendazim (10605-21-7), (4.3) chlorfenazole (3574-96-7), (4.4) diethofencarb (87130-20-9), (4.5) ethaboxam (162650-77-3), (4.6) fluopicolid (239110-15-7), (4.7) fuberidazole (3878-19-1), (4.8) pencycuron (66063-05-6), (4.9) thiabendazole (148-79-8), (4.10) thiophanate-methyl (23564-05-8), (4.11) thiophanate (23564-06-9), (4.12) zoxamide (156052-68-5), (4.13) 5-chloro-7-(4-methylpiperidin-1-yl)-6-(2,4,6-trifluorophenyl)[1,2,4]triazolo[1,5-a]pyrimidine (214706-53-3) and (4.14) 3-chloro-5-(6-chloropyridin-3-yl)-6-methyl-4-(2,4,6-trifluorophenyl)pyridazine (1002756-87-7).

(5) Compounds having multisite activity, for example (5.1) Bordeaux mixture (8011-63-0), (5.2) captafol (2425-06-1), (5.3) captan (133-06-2), (5.4) chlorothalonil (1897-45-6), (5.5) copper preparations such as copper hydroxide (20427-59-2), (5.6) copper naphthenate (1338-02-9), (5.7) copper oxide (1317-39-1), (5.8) copper oxychloride (1332-40-7), (5.9) copper sulphate (7758-98-7), (5.10) dichlofluanid (1085-98-9), (5.11) dithianon (3347-22-6), (5.12) dodine (2439-10-3), (5.13) dodine free base, (5.14) ferbam (14484-64-1), (5.15) fluorofolpet (719-96-0), (5.16) folpet (133-07-3), (5.17) guazatine (108173-90-6), (5.18) guazatine acetate, (5.19) iminoctadine (13516-27-3), (5.20) iminoctadine albesilate (169202-06-6), (5.21) iminoctadine triacetate (57520-17-9), (5.22) mancopper (53988-93-5), (5.23) mancozeb (8018-01-7), (5.24) maneb (12427-38-2), (5.25) metiram (9006-42-2), (5.26) zinc metiram (9006-42-2), (5.27) copper-oxine (10380-28-6), (5.28) propamidine (104-32-5), (5.29) propineb (12071-83-9), (5.30) sulphur and sulphur preparations, for example calcium polysulphide (7704-34-9), (5.31) thiram (137-26-8), (5.32) tolylfluanid (731-27-1), (5.33) zineb (12122-67-7) and (5.34) ziram (137-30-4).

(6) Resistance inducers, for example (6.1) acibenzolar-S-methyl (135158-54-2), (6.2) isotianil (224049-04-1), (6.3) probenazole (27605-76-1) and (6.4) tiadinil (223580-51-6).

(7) Inhibitors of amino acid and protein biosynthesis, for example (7.1) andoprim (23951-85-1), (7.2) blasticidin-S (2079-00-7), (7.3) cyprodinil (121552-61-2), (7.4) kasugamycin (6980-18-3), (7.5) kasugamycin hydrochloride hydrate (19408-46-9), (7.6) mepanipyrim (110235-47-7), (7.7) pyrimethanil (53112-28-0) and (7.8) 3-(5-fluoro-3,3,4,4-tetramethyl-3,4-dihydroisoquinolin-1-yl)quinoline (861647-32-7).

(8) ATP production inhibitors, for example (8.1) fentin acetate (900-95-8), (8.2) fentin chloride (639-58-7), (8.3) fentin hydroxide (76-87-9) and (8.4) silthiofam (175217-20-6).

(9) Inhibitors of cell wall synthesis, for example (9.1) benthiavalicarb (177406-68-7), (9.2) dimethomorph (110488-70-5), (9.3) flumorph (211867-47-9), (9.4) iprovalicarb (140923-17-7), (9.5) mandipropamid (374726-62-2), (9.6) polyoxins (11113-80-7), (9.7) polyoxorim (22976-86-9), (9.8) validamycin A (37248-47-8) and (9.9) valifenalate (283159-94-4; 283159-90-0).

(10) Inhibitors of lipid and membrane synthesis, for example (10.1) biphenyl (92-52-4), (10.2) chloroneb (2675-77-6), (10.3) dicloran (99-30-9), (10.4) edifenphos (17109-49-8), (10.5) etridiazole (2593-15-9), (10.6) iodocarb (55406-53-6), (10.7) iprobenfos (26087-47-8), (10.8) isoprothiolane (50512-35-1), (10.9) propamocarb (25606-41-1), (10.10) propamocarb hydrochloride (25606-41-1), (10.11) prothiocarb (19622-08-3), (10.12) pyrazophos (13457-18-6), (10.13) quintozene (82-68-8), (10.14) tecnazene (117-18-0) and (10.15) tolclofos-methyl (57018-04-9).

(11) Melanin biosynthesis inhibitors, for example (11.1) carpropamid (104030-54-8), (11.2) diclocymet (139920-32-4), (11.3) fenoxanil (115852-48-7), (11.4) fthalide (27355-22-2), (11.5) pyroquilon (57369-32-1), (11.6) tricyclazole (41814-78-2) and (11.7) 2,2,2-trifluoroethyl {3-methyl-1-[(4-methylbenzoyl)amino]butan-2-yl}carbamate (851524-22-6).

(12) Inhibitors of nucleic acid synthesis, for example (12.1) benalaxyl (71626-11-4), (12.2) benalaxyl-M (kiralaxyl) (98243-83-5), (12.3) bupirimate (41483-43-6), (12.4) clozylacon (67932-85-8), (12.5) dimethirimol (5221-53-4), (12.6) ethirimol (23947-60-6), (12.7) furalaxyl (57646-30-7), (12.8) hymexazole (10004-44-1), (12.9) metalaxyl (57837-19-1), (12.10) metalaxyl-M (mefenoxam) (70630-17-0), (12.11) ofurace (58810-48-3), (12.12) oxadixyl (77732-09-3) and (12.13) oxolinic acid (14698-29-4).

(13) Signal transduction inhibitors, for example (13.1) chlozolinate (84332-86-5), (13.2) fenpiclonil (74738-17-3), (13.3) fludioxonil (131341-86-1), (13.4) iprodione (36734-19-7), (13.5) procymidone (32809-16-8), (13.6) quinoxyfen (124495-18-7) and (13.7) vinclozolin (50471-44-8).

(14) Decouplers, for example (14.1) binapacryl (485-31-4), (14.2) dinocap (131-72-6), (14.3) ferimzone (89269-64-7), (14.4) fluazinam (79622-59-6) and (14.5) meptyldinocap (131-72-6).

(15) Further compounds, for example (15.1) benthiazole (21564-17-0), (15.2) bethoxazine (163269-30-5), (15.3) capsimycin (70694-08-5), (15.4) carvone (99-49-0), (15.5) chinomethionat (2439-01-2), (15.6) pyriofenone (chlazafenone) (688046-61-9), (15.7) cufraneb (11096-18-7), (15.8) cyflufenamid (180409-60-3), (15.9) cymoxanil (57966-95-7), (15.10) cyprosulfamide (221667-31-8), (15.11) dazomet (533-74-4), (15.12) debacarb (62732-91-6), (15.13) dichlorophen (97-23-4), (15.14) diclomezine (62865-36-5), (15.15) difenzoquat (49866-87-7), (15.16) difenzoquat methylsulphate (43222-48-6), (15.17) diphenylamine (122-39-4), (15.18) EcoMate, (15.19) fenpyrazamine (473798-59-3), (15.20) flumetover (154025-04-4), (15.21) fluoromid (41205-21-4), (15.22) flusulfamide (106917-52-6), (15.23) flutianil (304900-25-2), (15.24) fosetyl-aluminium (39148-24-8), (15.25) fosetyl-calcium, (15.26) fosetyl-sodium (39148-16-8), (15.27) hexachlorobenzene (118-74-1), (15.28) irumamycin (81604-73-1), (15.29) methasulfocarb (66952-49-6), (15.30) methyl isothiocyanate (556-61-6), (15.31) metrafenone (220899-03-6), (15.32) mildiomycin (67527-71-3), (15.33) natamycin (7681-93-8), (15.34) nickel dimethyldithiocarbamate (15521-65-0), (15.35) nitrothal-isopropyl (10552-74-6), (15.36) octhilinone (26530-20-1), (15.37) oxamocarb (917242-12-7), (15.38) oxyfenthiin (34407-87-9), (15.39) pentachlorophenol and its salts (87-86-5), (15.40) phenothrin, (15.41) phosphoric acid and its salts (13598-36-2), (15.42) propamocarb-fosetylate, (15.43) propanosine-sodium (88498-02-6), (15.44) proquinazid (189278-12-4), (15.45) pyrimorph (868390-90-3), (15.45e) (2E)-3-(4-tert-butylphenyl)-3-(2-chloropyridin-4-yl)-1-(morpholin-4-yl)prop-2-en-1-one (1231776-28-5), (15.45z) (2Z)-3-(4-tert-butylphenyl)-3-(2-chloropyridin-4-yl)-1-(morpholin-4-yl)prop-2-en-1-one (1231776-29-6), (15.46) pyrrolnitrin (1018-71-9), (15.47) tebufloquin (376645-78-2), (15.48) tecloftalam (76280-91-6), (15.49) tolnifanide (304911-98-6), (15.50) triazoxide (72459-58-6), (15.51) trichlamide (70193-21-4), (15.52) zarilamid (84527-51-5), (15.53) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate (517875-34-2), (15.54) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-79-6), (15.55) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-80-9), (15.56) 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003318-67-9), (15.57) 1-(4-methoxyphenoxy)-3,3-dimethylbutan-2-yl 1H-imidazole-1-carboxylate (111227-17-9), (15.58) 2,3,5,6-tetrachloro-4-(methylsulphonyl)pyridine (13108-52-6), (15.59) 2,3-dibutyl-6-chlorothieno[2,3-d]pyrimidin-4(3H)-one (221451-58-7), (15.60) 2,6-dimethyl-1H,5H-[1,4]dithiino[2,3-c:5,6-c']dipyrrole-1,3,5,7(2H,6H)-tetrone, (15.61) 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone (1003316-53-7), (15.62) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone (1003316-54-8), (15.63) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone (1003316-51-5), (15.64) 2-butoxy-6-iodo-3-propyl-4H-chromen-4-one, (15.65) 2-chloro-5-[2-chloro-1-(2,6-difluoro-4-methoxyphenyl)-4-methyl-1H-imidazol-5-yl]pyridine, (15.66) 2-phenylphenol and its salts (90-43-7), (15.67) 3-(4,4,5-trifluoro-3,3-dimethyl-3,4-dihydroisoquinolin-1-yl)quinoline (861647-85-0), (15.68) 3,4,5-trichloropyridine-2,6-dicarbonitrile (17824-85-0), (15.69) 3-[5-(4-chlorophenyl)-2,3-dimethyl-1,2-oxazolidin-3-yl]pyridine, (15.70) 3-chloro-5-(4-chlorophenyl)-4-(2,6-difluorophenyl)-6-methylpyridazine, (15.71) 4-(4-chlorophenyl)-5-(2,6-difluorophenyl)-3,6-dimethylpyridazine, (15.72) 5-amino-1,3,4-thiadiazole-2-thiol, (15.73) 5-chloro-N'-phenyl-N'-(prop-2-yn-1-yl)thiophene-2-sulphonohydrazide (134-31-6), (15.74) 5-fluoro-2-[(4-fluorobenzyl)oxy]pyrimidine-4-amine (1174376-11-4), (15.75) 5-fluoro-2-[(4-methylbenzyl)oxy]pyrimidine-4-amine (1174376-25-0), (15.76) 5-methyl-6-octyl[1,2,4]triazolo[1,5-a]pyrimidine-7-amine, (15.77) ethyl-(2Z)-3-amino-2-cyano-3-phenylprop-2-enoate, (15.78) N'-(4-{[3-(4-chlorobenzyl)-1,2,4-thiadiazol-5-yl]oxy}-2,5-dimethylphenyl)-N-ethyl-N-methylimidoformamide, (15.79) N-(4-chlorobenzyl)-3-[3-methoxy-4-(prop-2-yn-1-yloxy)phenyl]propanamide, (15.80) N-[(4-chlorophenyl)(cyano)methyl]-3-[3-methoxy-4-(prop-2-yn-1-yloxy)phenyl]propanamide, (15.81) N-[(5-bromo-3-chloropyridin-2-yl)methyl]-2,4-dichloropyridine-3-carboxamide, (15.82) N-[1-(5-bromo-3-chloropyridin-2-yl)ethyl]-2,4-dichloropyridine-3-carboxamide, (15.83) N-[1-(5-bromo-3-chloropyridin-2-yl)ethyl]-2-fluoro-4-iodopyridine-3-carboxamide, (15.84) N-{(E)-[(cyclopropylmethoxy)imino][6-(difluoromethoxy)-2,3-difluorophenyl]methyl}-2-phenylacetamide (221201-92-9), (15.85) N-{(Z)-[(cyclopropylmethoxy)imino] [6-(difluoromethoxy)-2,3-difluorophenyl]methyl}-2-phenylacetamide (221201-92-9), (15.86) N'-{4-[(3-tert-butyl-4-cyano-1,2-thiazol-5-yl)oxy]-2-chloro-5-methylphenyl}-N-ethyl-N-methylimidoformamide, (15.87) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide (922514-49-6), (15.88) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide (922514-07-6), (15.89) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide (922514-48-5), (15.90) pentyl-{6-[({[(1-methyl-1H-tetrazol-5-yl)(phenyl)methylidene]amino}oxy)methyl]pyridin-2-yl}carbamate, (15.91) phenazine-1-carboxylic acid, (15.92) quinolin-8-ol (134-31-6), (15.93) quinolin-8-ol sulphate (2:1) (134-31-6) and (15.94) tert-butyl {6-[({[(1-methyl-1H-tetrazol-5-yl)(phenyl)methylene]amino}oxy)methyl]pyridin-2-yl}carbamate.

(16) Further compounds, for example (16.1) 1-methyl-3-(trifluoromethyl)-N-[2'-(trifluoromethyl)biphenyl-2-yl]-1H-pyrazole-4-carboxamide, (16.2) N-(4'-chlorobiphenyl-2-yl)-3-(difluoromethyl)-1-methyl-1H-pyrazole-4-carboxamide, (16.3) N-(2',4'-dichlorobiphenyl-2-yl)-3-(difluoromethyl)-1-methyl-1H-pyrazole-4-carboxamide, (16.4) 3-(difluoromethyl)-1-methyl-N-[4'-(trifluoromethyl)biphenyl-2-yl]-1H-pyrazole-4-carboxamide, (16.5) N-(2',5'-difluorobiphenyl-2-yl)-1-methyl-3-(trifluoromethyl)-1H-pyrazole-4-carboxamide, (16.6) 3-(difluoromethyl)-1-methyl-N-[4'-(prop-1-yn-1-yl)biphenyl-2-yl]-1H-pyrazole-4-carboxamide, (16.7) 5-fluoro-1,3-dimethyl-N-[4'-(prop-1-yn-1-yl)biphenyl-2-yl]-1H-pyrazole-4-carboxamide, (16.8) 2-chloro-N-[4'-(prop-1-yn-1-yl)biphenyl-2-yl]pyridine-3-carboxamide, (16.9) 3-(difluoromethyl)-N-[4'-(3,3-dimethylbut-1-yn-1-yl)biphenyl-2-yl]-1-methyl-1H-pyrazole-4-carboxamide, (16.10) N-[4'-(3,3-dimethylbut-1-yn-1-yl)biphenyl-2-yl]-5-fluoro-1,3-dimethyl-1H-pyrazole-4-carboxamide, (16.11) 3-(difluoromethyl)-N-(4'-ethynylbiphenyl-2-yl)-1-methyl-1H-pyrazole-4-carboxamide, (16.12) N-(4'-ethynylbiphenyl-2-yl)-5-fluoro-1,3-dimethyl-1H-pyrazole-4-carboxamide, (16.13) 2-chloro-N-(4'-ethynylbiphenyl-2-yl)pyridine-3-carboxamide, (16.14) 2-chloro-N-[4'-(3,3-dimethylbut-1-yn-1-yl)biphenyl-2-yl]pyridine-3-carboxamide, (16.15) 4-(difluoromethyl)-2-methyl-N-[4'-(trifluoromethyl)biphenyl-2-yl]-1,3-thiazole-5-carboxamide, (16.16) 5-fluoro-N-[4'-(3-hydroxy-3-methylbut-1-yn-1-yl)biphenyl-2-yl]-1,3-dimethyl-1H-pyrazole-4-carboxamide, (16.17) 2-chloro-N-[4'-(3-hydroxy-3-methylbut-1-yn-1-yl)biphenyl-2-yl]pyridine-3-carboxamide, (16.18) 3-(difluoromethyl)-N-[4'-(3-methoxy-3-methylbut-1-yn-1-yl)biphenyl-2-yl]-1-methyl-1H-pyrazole-4-carboxamide, (16.19) 5-fluoro-N-[4'-(3-methoxy-3-methylbut-1-yn-1-yl)biphenyl-2-yl]-1,3-dimethyl-1H-pyrazole-4-carboxamide, (16.20) 2-chloro-N-[4'-(3-methoxy-3-methylbut-1-yn-1-yl)biphenyl-2-yl]pyridine-3-carboxamide, (16.21) (5-bromo-2-methoxy-4-methylpyridin-3-yl) (2,3,4-trimethoxy-6-methylphenyl)methanone, (16.22) N-[2-(4-{[3-(4-chlorophenyl)prop-2-yn-1-yl]oxy}-3-methoxyphenyl)ethyl]-N2-(methylsulphonyl)valinamide (220706-93-4), (16.23) 4-oxo-4-[(2-phenylethyl)amino]butanoic acid and (16.24) but-3-yn-1-yl{6-[({[(Z)-(1-methyl-1H-tetrazol-5-yl)(phenyl)methylene]amino}oxy)methyl]pyridin-2-yl}carbamate.

**20.** Agrochemical composition, **characterized in that** it comprises at least one compound of the formula (I) according to Claims 1 to 18 or a composition according to Claim 19, and extenders and/or surfactants.

**21.** Process for producing agrochemical compositions, **characterized in that** compounds of the formula (I) according to Claims 1 to 18 or a composition according to Claim 19 are/is mixed with extenders and/or surfactants.

**22.** Method for controlling animal pests, **characterized in that** compounds of the formula (I) according to Claims 1 to 18 or a composition according to Claim 19 are/is allowed to act on animal pests and/or their habitat, excluding methods of surgical or therapeutic treatment of the human or animal body and diagnostic methods that are implemented on the human or animal body.

**23.** Use of compounds of the formula (I) according to any of Claims 1 to 18 or a composition according to Claim 19 for controlling animal pests in crop protection, in the protection of materials and/or in the veterinary sector, excluding use in methods of surgical or therapeutic treatment of the human or animal body and diagnostic methods that are implemented on the human or animal body.

## Revendications

**1.** Composés de formule (I)

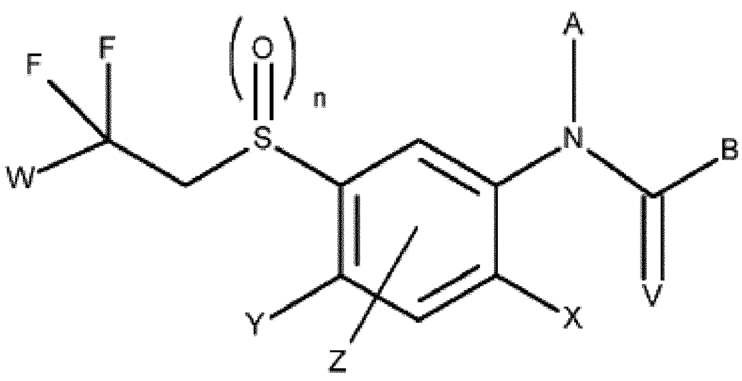

(I)

dans laquelle

A et B conjointement avec les atomes auxquels ils sont liés, représentent une sous-structure, qui est choisie dans le groupe constitué par

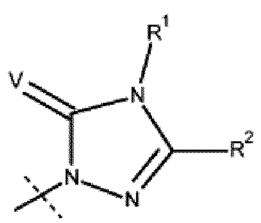

(I-A)

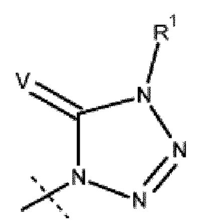

(I-B)

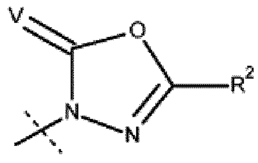

(I-C)

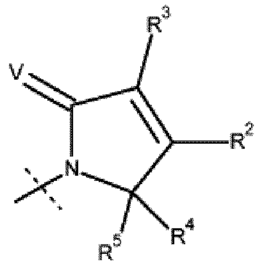

(I-D)

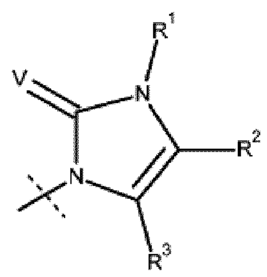

(I-E)

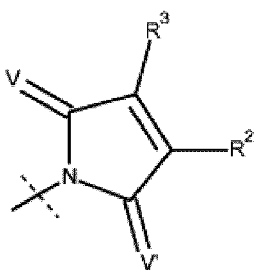

(I-F)

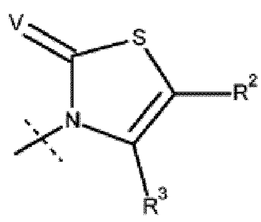

(I-G)

$R^1$ représentant hydrogène, cyano ou nitro ; ou

représentant alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétarylalkyle, hétaryloxyalkyle, hétarylthioalkyle, lesdits radicaux pouvant à chaque fois être saturés ou insaturés et/ou éventuellement substitués ; ou
représentant cycloalkyle saturé ou insaturé éventuellement substitué, qui peut éventuellement être interrompu par un ou plusieurs hétéroatomes ; ou
représentant alkylcarbonyle, halogénoalkylcarbonyle, hydroxyalkylcarbonyle, alcoxyalkylcarbonyle, phénylcarbonyle, hétarylcarbonyle, alcoxycarbonyle,
halogénoalcoxycarbonyle, aryloxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)aminocarbonyle, alkylaminothiocarbonyle, dialkylaminothiocarbonyle ou aminothiocarbonyle, lesdits radicaux pouvant à chaque fois être saturés ou insaturés et/ou éventuellement substitués ou représentant carbonyle ou carboxyle ; ou
représentant phényle éventuellement substitué ou hétaryle éventuellement substitué ; ou
représentant alcoxyle, halogénoalcoxyle, cycloalkyloxyle, aryloxyle, arylalkyloxyle ou carbonyloxyle, lesdits radicaux pouvant éventuellement être substitués, ou représentant hydroxyle ; ou
représentant alkylamino, halogénoalkylamino, dihalogénoalkylamino, dialkylamino, cycloalkylamino, dicycloalkylamino, cycloalkyl(alkyl)amino, arylamino, diarylamino, hétarylamino, dihétarylamino, alkyl(aryl)amino, cycloalkyl(aryl)amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, lesdits radicaux pouvant à chaque fois être saturés ou insaturés et/ou éventuellement substitués, ou représentant amino ; ou
représentant alkylsulfanyle, alkylsulfinyle, alkysulfonyle, halogénoalkylsulfanyle, halogénoalkylsulfinyle, halogénoalkylsulfanyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, lesdits radicaux pouvant à chaque fois être saturés ou insaturés et/ou éventuellement substitués, ou représentant sulfanyle ; et

$R^2$, $R^3$, $R^4$, et $R^5$, à chaque fois indépendamment les uns des autres,

représentant hydrogène, cyano, halogène ou nitro ; ou
représentant alkyle, cycloalkylalkyle, halogénoalkyle, cyanoalkyle, hydroxyalkyle, alcoxyalkyle, aminoalkyle, alcoxycarbonylalkyle, alkylsulfanylalkyle, alkylsulfinylalkyle, alkylsulfonylalkyle, halogénoalkylsulfanylalkyle, halogénoalkylsulfinylalkyle, halogénoalkylsulfonylalkyle, alcoxyalkylsulfanylalkyle, alcoxyalkylsulfinylalkyle, alcoxyalkylsulfonylalkyle, phénylalkyle, phénoxyalkyle, phénylsulfanylalkyle, phénylsulfinylalkyle, phénylsulfonylalkyle, hétarylalkyle, hétaryloxyalkyle, hétarylthioalkyle, lesdits radicaux pouvant à chaque fois être saturés ou insaturés et/ou éventuellement substitués ; ou
représentant cycloalkyle saturé ou insaturé éventuellement substitué, qui peut éventuellement être interrompu par un ou plusieurs hétéroatomes ; ou
représentant alkylcarbonyle, halogénoalkylcarbonyle, hydroxyalkylcarbonyle, alcoxyalkylcarbonyle, phénylcarbonyle, hétarylcarbonyle, alcoxycarbonyle, halogénoalcoxycarbonyle, aryloxycarbonyle, aminocarbonyle, alkylaminocarbonyle, dialkylaminocarbonyle, cycloalkylaminocarbonyle, dicycloalkylaminocarbonyle, cycloalkyl(alkyl)aminocarbonyle, arylaminocarbonyle, diarylaminocarbonyle, alkyl(aryl)aminocarbonyle, cycloalkyl(aryl)aminocarbonyle, alkylaminothiocarbonyle, dialkylaminothiocarbonyle ou aminothiocarbonyle, lesdits radicaux pouvant à chaque fois être saturés ou insaturés et/ou éventuellement substitués ou représentant carbonyle ou carboxyle ; ou
représentant phényle éventuellement substitué ou hétaryle éventuellement substitué ; ou
représentant alcoxyle, halogénoalcoxyle, alcoxyalcoxyle, aryloxyle, arylalkyloxyle, cycloalkyloxyle, cycloalkylalkyloxyle ou carbonyloxyle, lesdits radicaux pouvant être saturés ou insaturés et/ou éventuellement substitués, ou représentant hydroxyle ; ou
représentant alkylamino, dialkylamino, halogénoalkylamino, dihalogénoalkylamino, cycloalkylamino, dicycloalkylamino, cycloalkyl(alkyl)amino, arylamino, diarylamino, hétarylamino, dihétarylamino, alkyl(aryl)amino, cycloalkyl(aryl)amino, alkylcarbonylamino, arylcarbonylamino, alcoxycarbonylamino, aryloxycarbonylamino, alkylcarbamoylamino, arylcarbamoylamino, alkylsulfonylamino, ou arylsulfonylamino, lesdits radicaux pouvant à chaque fois être saturés ou insaturés et/ou éventuellement substitués, ou représentant amino ; ou
représentant alkylsulfanyle, alkylsulfinyle, alkysulfonyle, halogénoalkylsulfanyle, halogénoalkylsulfinyle, halogénoalkylsulfonyle, cycloalkylsulfanyle, cycloalkylsulfinyle, cycloalkylsulfonyle, cycloalkylalkylsulfanyle, cycloalkylalkylsulfinyle, cycloalkylalkylsulfonyle, arylsulfanyle, arylsulfinyle, arylsulfonyle, arylalkylsulfanyle, arylalkylsulfinyle, arylalkylsulfonyle, aminosulfonyle, alkylaminosulfonyle, dialkylaminosulfonyle ou arylaminosulfonyle, lesdits radicaux pouvant à chaque fois être saturés ou insaturés et/ou éventuellement substitués, ou représentant sulfanyle ;
$R^4$ et $R^5$, conjointement avec l'atome auquel ils sont liés, pouvant former un cycle saturé ou insaturé, éventuellement substitué et éventuellement interrompu par un ou plusieurs hétéroatomes, qui sont indépendamment choisis dans le groupe constitué par O, S ou N, à trois à huit chaînons ; ou
ou $R^2$ représentant un cycle saturé ou insaturé, éventuellement interrompu par un ou plusieurs hétéroatomes, qui sont indépendamment choisis dans le groupe constitué par O, S ou N, qui peut éventuellement être substitué ;

W représentant hydrogène ou halogène ;
dans le cas où une sous-structure selon l'une des formules (I-B) à (I-G) est présente,
V et V' représentent à chaque fois indépendamment l'un de l'autre oxygène, soufre ou un azote éventuellement substitué ;
et dans le cas, où une sous-structure selon la formule (I-A) est présente,
V représente oxygène ou soufre ;
X, Y et Z à chaque fois indépendamment les uns des autres,

représentant hydrogène, halogène, hydroxyle, amino, cyano, nitro, OCN, SCN, $SF_5$; ou
représentant trialkylsilyle, alkyle, halogénoalkyle, cyanoalkyle, hydroxyalkyle, alcoxycarbonylalkyle, alcoxyalkyle, alcényle, halogénoalcényle, cyanoalcényle, alcynyle, halogénoalcynyle, cyanoalcynyle, alcoxyle, halogénoalcoxyle, cyanoalcoxyle, hydroxycarbonylalcoxyle, alcoxycarbonylalcoxyle, alcoxyalcoxyle, alkylhydroxyimino, alcoxyimino, alkylalcoxyimino, halogénoalkylalcoxyimino, alkylthio, halogénoalkylthio, alcoxyalkylthio, alkylthioalkyle, alkylsulfinyle, halogénoalkylsulfinyle, alcoxyalkylsulfinyle, alkylsulfinylalkyle, alkylsulfonyle, halogénoalkylsulfonyle, alcoxyalkylsulfonyle, alkylsulfonylalkyle, alkylsulfonyloxyle, alkylcarbonyle, halogénoalkylcarbonyle, carboxyle, alkylcarbonyloxyle, alcoxycarbonyle, halogénoalcoxycarbonyle, aminocarbonyle, alkylaminocarbonyle, dialkylaminocarbonyle, alcénylaminocarbonyle, dialcénylaminocarbonyle, cycloalkylaminocarbonyle, alkylsulfonylamino, aminosulfonyle, alkylaminosulfonyle, dialkylaminosulfonyle, alkylsulfoxymino, aminothiocarbonyle, alkylaminothiocarbonyle, ou dialkylaminothiocarbonyle, tous lesdits radicaux pouvant éventuellement être substitués ; ou
représentant phénylalkyle, phénoxyle, phénylalkyloxyle, phénoxyalkyle, phénylthio, phénylthioalkyle, phénylsulfinyle, phénylsulfonyle, hétarylalkyle, hétaryloxyle, hétarylalkyloxyle, hétarylthio, hétarylsulfinyle ou hétarylsulfonyle, tous lesdits radicaux pouvant éventuellement être substitués ; ou
représentant cycloalkylalkyle, cycloalkyloxyle, cycloalkylalcoxyle, cycloalkylthio, cycloalkylalkylthio, cycloalkylsulfinyle, cycloalkylalkylsulfinyle, cycloalkylsulfonyle, cycloalkylalkylsulfonyle ou cycloalcényle, tous lesdits radicaux pouvant éventuellement être substitués ; ou
représentant NR'R",
R' et R" représentant indépendamment l'un de l'autre
hydrogène, cyano, alkyle, halogénoalkyle, cyanoalkyle, hydroxyalkyle, alcoxyalkyle, alkylthioalkyle, alcényle, halogénoalcényle, cyanoalcényle, alcynyle, halogénoalcynyle, cyanoalcynyle, acyle ou alcoxycarbonyle ; ou
R' et R", conjointement avec l'atome N auquel ils sont liés, pouvant former un cycle saturé ou insaturé, éventuellement substitué et éventuellement interrompu par un ou plusieurs hétéroatomes, qui sont indépendamment choisis dans le groupe constitué par O, S ou N, à cinq à huit chaînons ; ou
représentant un cycle saturé, partiellement saturé ou aromatique, à 3 à 6 chaînons, qui peut éventuellement contenir un à trois hétéroatomes, qui sont indépendamment choisis dans le groupe constitué par O, S ou N et qui peut éventuellement être substitué ;
ou X et Z, ou Y et Z, avec les atomes C, auxquels ils sont liés, formant un cycle à 5 ou 6 chaînons, qui est éventuellement substitué et est éventuellement interrompu par un ou plusieurs hétéroatomes, qui sont indépendamment choisis dans le groupe constitué par O, S, N et CO ;

et
n représentant le nombre 0 ou 1.

**2.** Composé selon la revendication 1, dans lequel

A et B conjointement avec les atomes auxquels ils sont liés, représentent une sous-structure, qui est choisie dans le groupe constitué par I-A à I-G,
$R^1$ représentant hydrogène, cyano ou nitro ; ou
représentant ($C_1$-$C_6$) alkyle, ($C_3$-$C_6$) cycloalkyl ($C_1$-$C_6$) alkyle, halogéno ($C_2$-$C_6$) alkyle, cyano ($C_1$-$C_6$) alkyle, hydroxy ($C_1$-$C_6$) alkyle, ($C_1$-$C_6$) alcoxy ($C_1$-$C_6$) alkyle, amino ($C_1$-$C_6$) alkyle, ($C_1$-$C_6$) alcoxycarbonyl ($C_1$-$C_6$) alkyle, ($C_1$-$C_6$) alkylsulfanyl ($C_1$-$C_6$) alkyle, ($C_1$-$C_6$) alkylsulfinyl ($C_1$-$C_6$) alkyle, ($C_1$-$C_6$) alkylsulfonyl ($C_1$-$C_6$) alkyle, halogéno ($C_1$-$C_6$) alkylsulfanyl ($C_1$-$C_6$) alkyle, halogéno ($C_1$-$C_6$) alkylsulfinyl ($C_1$-$C_6$) alkyle, halogéno ($C_1$-$C_6$) alkylsulfonyl ($C_1$-$C_6$) alkyle, ($C_1$-$C_6$) alcoxy ($C_1$-$C_6$) alkylsulfanyl ($C_1$-$C_6$) alkyle, ($C_1$-$C_6$) alcoxy ($C_1$-$C_6$) alkylsulfinyl ($C_1$-$C_6$) alkyle, ($C_1$-$C_6$) alcoxy ($C_1$-$C_6$) alkylsulfonyl ($C_1$-$C_6$) alkyle, phényl ($C_1$-$C_6$) alkyle, phé-

noxy($C_1$-$C_6$)alkyle, phénylsulfanyl($C_1$-$C_6$) alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylsulfanyl($C_1$-$C_6$)alkyle, hétarylsulfinyl($C_1$-$C_6$)alkyle, hétarylsulfonyl($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou
représentant ($C_3$-$C_6$)cycloalkyle saturé ou insaturé éventuellement substitué, qui peut être interrompu par un ou plusieurs hétéroatomes ; ou
représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxy($C_1$-$C_6$)alkylcarbonyle, phénylcarbonyle, hétarylcarbonyle, ($C_1$-$C_6$)alcoxycarbonyle, halogèno($C_1$-$C_6$)alcoxycarbonyle, aryloxycarbonyle, aminocarbonyle, ($C_1$-$C_6$)alkylaminocarbonyle, di($C_1$-$C_6$) alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou
représentant phényle éventuellement substitué ou hétaryle éventuellement substitué ; ou
représentant ($C_1$-$C_6$)alcoxyle, halogéno($C_1$-$C_6$)alcoxyle, ($C_3$-$C_6$)cycloalkyloxyle, aryloxyle, aryl($C_1$-$C_6$)alkyloxyle ou carbonyloxyle, lesdits radicaux pouvant éventuellement être substitués, ou représentant hydroxyle ; ou
représentant ($C_1$-$C_6$)alkylamino, halogéno($C_1$-$C_6$)alkylamino, dihalogéno($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl (aryl) amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou
représentant ($C_1$-$C_6$)alkylsulfanyle, ($C_1$-$C_6$) alkylsulfinyle, ($C_1$-$C_6$)alkysulfonyle, halogéno($C_1$-$C_6$)alkylsulfanyle, halogéno($C_1$-$C_6$)alkylsulfinyle,
halogéno($C_1$-$C_6$)alkylsulfanyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl($C_1$-$C_6$)alkylsulfanyle, ($C_3$-$C_6$)cycloalkyl($C_1$-$C_6$)alkylsulfinyle, ($C_3$-$C_6$)cycloalkyl($C_1$-$C_6$)alkylsulfonyle, arylsulfanyle, arylsulfinyle, arylsulfonyle, aryl($C_1$-$C_6$)alkylsulfanyle, aryl($C_1$-$C_6$)alkylsulfinyle, aryl($C_1$-$C_6$) alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; et
$R^2$, $R^3$, $R^4$, et $R^5$, à chaque fois indépendamment les uns des autres,

représentant hydrogène, cyano, halogène ou nitro ; ou
représentant ($C_1$-$C_6$)alkyle, ($C_3$-$C_6$)cycloalkyl($C_1$-$C_6$)alkyle, halogéno($C_1$-$C_6$)alkyle, cyano($C_1$-$C_6$)alkyle, hydroxy ($C_1$-$C_6$)alkyle, ($C_1$-$C_6$)alcoxy($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy ($C_1$-$C_6$)alkylsulfonyl($C_1$-$C_6$) alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$) alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou
représentant ($C_3$-$C_6$)cycloalkyle saturé ou insaturé éventuellement substitué, qui peut éventuellement être interrompu par un ou plusieurs hétéroatomes ; ou
représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxyalkyl($C_1$-$C_6$)carbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl) aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués

et/ou à condition que dans le cas d'un radical insaturé, le nombre minimal défini d'atomes de carbone est de 2, les radicaux pouvant être saturés ou insaturés ou représentant carbonyle ou carboxyle ; ou représentant phényle éventuellement substitué ou hétaryle éventuellement substitué ; ou représentant $(C_1-C_6)$alcoxyle, halogéno$(C_1-C_6)$ alcoxyle, $(C_1-C_6)$alcoxy$(C_1-C_6)$alcoxyle, aryloxyle, aryl$(C_1-C_6)$alkyloxyle, $(C_3-C_6)$cycloalkyloxyle, $(C_3-C_6)$cycloalkyl$(C_1-C_6)$ alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou

représentant $(C_1-C_6)$alkylamino, di$(C_1-C_6)$alkylamino, halogéno$(C_1-C_6)$alkylamino, dihalogéno$(C_1-C_6)$alkylamino, $(C_3-C_6)$cycloalkylamino, di$(C_3-C_6)$cycloalkylamino, $(C_3-C_6)$ cycloalkyl $((C_1-C_6)$alkyl)amino, arylamino, diarylamino, hétarylamino, dihétarylamino, $(C_1-C_6)$ alkyl (aryl) amino, $(C_3-C_6)$cycloalkyl(aryl)amino, $(C_1-C_6)$alkylcarbonylamino, arylcarbonylamino, $(C_1-C_6)$alcoxycarbonylamino, aryloxycarbonylamino, $(C_1-C_6)$alkylcarbamoylamino, arylcarbamoylamino, $(C_1-C_6)$alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant $(C_1-C_6)$ alkylsulfanyle, $(C_1-C_6)$alkylsulfinyle, $(C_1-C_6)$alkysulfonyle, halogéno$(C_1-C_6)$alkylsulfanyle, halogéno$(C_1-C_6)$alkylsulfinyle, halogéno$(C_1-C_6)$alkylsulfonyle, $(C_3-C_6)$cycloalkylsulfanyle, $(C_3-C_6)$cycloalkylsulfinyle, $(C_3-C_6)$cycloalkylsulfonyle, $(C_3-C_6)$cycloalkyl$(C_1-C_6)$alkylsulfanyle, $(C_3-C_6)$cycloalkyl$(C_1-C_6)$alkylsulfinyle, $(C_3-C_6)$cycloalkyl$(C_1-C_6)$alkylsulfonyle, arylsulfanyle, arylsulfinyle, arylsulfonyle, aryl$(C_1-C_6)$alkylsulfanyle, aryl$(C_1-C_6)$alkylsulfinyle, aryl$(C_1-C_6)$ alkylsulfonyle, aminosulfonyle, $(C_1-C_6)$alkylaminosulfonyle, di$(C_1-C_6)$alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou

$R^4$ et $R^5$, conjointement avec l'atome auquel ils sont liés, pouvant former un cycle saturé ou insaturé, éventuellement substitué une ou plusieurs fois de manière identique ou différente par halogène, cyano, nitro, hydroxyle, amino, $(C_1-C_6)$ alkyle, halogéno$(C_1-C_6)$alkyle, $(C_1-C_6)$ alcoxyle, halogéno$(C_1-C_6)$alcoxyle, $(C_1-C_6)$ alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle, ou $(C_3-C_6)$ cycloalkyle éventuellement substitué et éventuellement interrompu par des hétéroatomes, de la série O, S ou N, à trois à six chaînons ; ou

ou $R^2$ représentant un cycle saturé ou insaturé, éventuellement substitué une ou plusieurs fois de manière identique ou différente par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle, ou $(C_3-C_6)$ cycloalkyle éventuellement substitué et éventuellement interrompu par des hétéroatomes, de la série O, S ou N, à trois à six chaînons ;

W représentant hydrogène ou halogène ;
dans le cas où une sous-structure selon l'une des formules (I-B) à (I-G) est présente,
V et V' représentent à chaque fois indépendamment l'un de l'autre oxygène, soufre ou un azote éventuellement substitué ;
et dans le cas, où une sous-structure selon la formule (I-A) est présente,
V représente oxygène ou soufre ;
X, Y et Z à chaque fois indépendamment les uns des autres, possédant les significations indiquées précédemment ;
et
n représentant le nombre 0 ou 1.

**3.** Composé selon la revendication 2, dans lequel

X, Y et Z à chaque fois indépendamment les uns des autres,

représentant hydrogène, halogène, hydroxyle, amino, cyano, nitro, OCN, SCN, $SF_5$; ou
représentant tri$(C_1-C_6)$alkylsilyle, $(C_1-C_6)$alkyle, halogéno$(C_1-C_6)$alkyle, cyano $(C_1-C_6)$alkyle, hydroxy$(C_1-C_6)$alkyle, $(C_1-C_6)$alcoxycarbonyl$(C_1-C_6)$alkyle, $(C_1-C_6)$alcoxy$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, $(C_1-C_6)$alcoxyle, halogéno$(C_1-C_6)$alcoxyle, cyano$(C_1-C_6)$alcoxyle, hydroxycarbo-

nyl($C_1$-$C_6$)alcoxyle, ($C_1$-$C_6$)alcoxycarbonyl($C_1$-$C_6$)alcoxyle, ($C_1$-$C_6$)alcoxy($C_1$-$C_6$)alcoxyle, ($C_1$-$C_6$)alkylhydroxyimino, ($C_1$-$C_6$)alcoxyimino, ($C_1$-$C_6$)alkyl($C_1$-$C_6$)alcoxyimino, halogéno($C_1$-$C_6$)alkyl($C_1$-$C_6$)alcoxyimino, ($C_1$-$C_6$)alkylthio, halogéno($C_1$-$C_6$)alkylthio, ($C_1$-$C_6$)alcoxy($C_1$-$C_6$)alkylthio, ($C_1$-$C_6$)alkylthio($C_1$-$C_6$)alkyle, ($C_1$-$C_6$)alkylsulfinyle, halogéno($C_1$-$C_6$)alkylsulfinyle, ($C_1$-$C_6$)alcoxy($C_1$-$C_6$)alkylsulfinyle, ($C_1$-$C_6$)alkylsulfinyl($C_1$-$C_6$)alkyle, ($C_1$-$C_6$)alkylsulfonyle, halogéno($C_1$-$C_6$)alkylsulfonyle, ($C_1$-$C_6$)alcoxy($C_1$-$C_6$)alkylsulfonyle, ($C_1$-$C_6$)alkylsulfonyl($C_1$-$C_6$)alkyle, ($C_1$-$C_6$)alkylsulfonyloxyle, ($C_1$-$C_6$)alkylcarbonyle, halogéno($C_1$-$C_6$)alkylcarbonyle, carboxyle, ($C_1$-$C_6$)alkylcarbonyloxyle, ($C_1$-$C_6$)alcoxycarbonyle, halogéno($C_1$-$C_6$)alcoxycarbonyle, aminocarbonyle, ($C_1$-$C_6$)alkylaminocarbonyle, di($C_1$-$C_6$)alkylaminocarbonyle, ($C_2$-$C_6$)alcénylaminocarbonyle, di($C_2$-$C_6$)alcénylaminocarbonyle, ($C_3$-$C_6$)cycloalkylaminocarbonyle, ($C_1$-$C_6$)alkylsulfonylamino, aminosulfonyle, ($C_1$-$C_6$)alkylaminosulfonyle, di($C_1$-$C_6$)alkylaminosulfonyle, ($C_1$-$C_6$)alkylsulfoxymino, aminothiocarbonyle, ($C_1$-$C_6$) alkylaminothiocarbonyle, ou di($C_1$-$C_6$)alkylaminothiocarbonyle, tous lesdits radicaux pouvant éventuellement être substitués par halogène ; ou

représentant phényl($C_1$-$C_3$)alkyle, phénoxyle, phényl($C_1$-$C_3$)alkyloxyle, phénoxy($C_1$-$C_3$)alkyle, phénylthio, phénylthio($C_1$-$C_3$)alkyle, phénylsulfinyle, phénylsulfonyle, hétaryl($C_1$-$C_3$)alkyle, hétaryloxyle, hétaryl($C_1$-$C_3$)alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant ($C_3$-$C_6$)cycloalkyl($C_1$-$C_3$)alkyle, ($C_3$-$C_6$)cycloalkyloxyle, ($C_3$-$C_6$)cycloalkyl($C_1$-$C_3$)alcoxyle, ($C_3$-$C_6$)cycloalkylthio, ($C_3$-$C_6$)cycloalkyl($C_1$-$C_3$)alkylthio, ($C_3$-$C_6$)cycloalkylsulfinyle, ($C_3$-$C_6$)cycloalkyl($C_1$-$C_3$)alkylsulfinyle, ($C_3$-$C_6$)cycloalkylsulfonyle, ($C_3$-$C_6$) cycloalkyl ($C_1$-$C_3$)alkylsulfonyle ou ($C_3$-$C_8$)cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R", R' et R" représentant indépendamment l'un de l'autre hydrogène, cyano, ($C_1$-$C_6$)alkyle, halogéno($C_1$-$C_6$)alkyle, cyano($C_1$-$C_6$)alkyle, hydroxy($C_1$-$C_6$)alkyle, ($C_1$-$C_6$)alcoxy($C_1$-$C_6$)alkyle, ($C_1$-$C_6$)alkylthio($C_1$-$C_6$)alkyle, ($C_2$-$C_6$)alcényle, halogéno($C_2$-$C_6$)alcényle, cyano($C_2$-$C_6$)alcényle, ($C_2$-$C_6$)alcynyle, halogéno($C_2$-$C_6$)alcynyle, cyano($C_2$-$C_6$)alcynyle, acyle ou ($C_1$-$C_6$)alcoxycarbonyle ; ou R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série O, S ou N, à cinq à sept chaînons ; ou

représentant ($C_3$-$C_6$)cycloalkyle, oxétane, oxolane, oxane, ($C_3$-$C_8$) cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

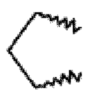 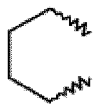 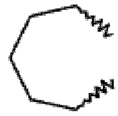 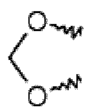 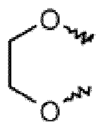 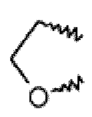 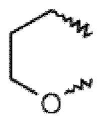

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

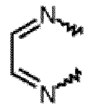 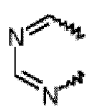 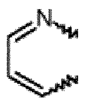 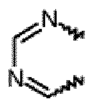 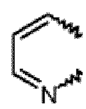 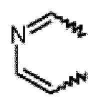

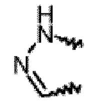 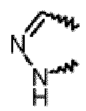 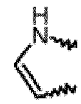 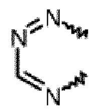 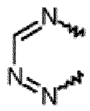 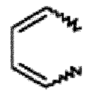

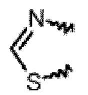 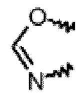 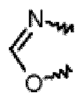

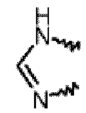 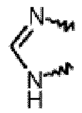 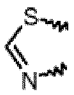

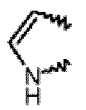 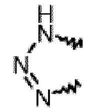 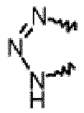

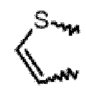 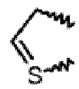 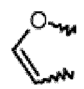 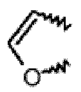

.

**4.** Composé selon l'une quelconque des revendications 1 à 3, dans lequel A et B conjointement avec les atomes, auxquels ils sont liés, représentent une sous-structure de formule (I-A)

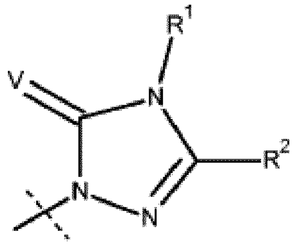

(I-A)

$R^1$ représentant hydrogène, cyano ou nitro ; ou

représentant ($C_1$-$C_6$)alkyle, ($C_3$-$C_6$)cycloalkyl($C_1$-$C_6$)alkyle, halogéno($C_2$-$C_6$)alkyle, cyano($C_1$-$C_6$)alkyle, hydroxy ($C_1$-$C_6$)alkyle, ($C_1$-$C_6$)alcoxy($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylsulfanyl($C_1$-$C_6$)alkyle, hétarylsulfinyl($C_1$-$C_6$)alkyle, hétarylsulfonyl($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant ($C_3$-$C_6$)cycloalkyle saturé ou insaturé éventuellement substitué, qui peut être interrompu par un ou plusieurs hétéroatomes ; ou

représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxy($C_1$-$C_6$)alkylcarbonyle, phénylcarbonyle, hétarylcarbonyle, ($C_1$-$C_6$)alcoxycarbonyle, halogène($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cy-

cloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogéno($C_1$-$C_6$)alcoxyle, ($C_3$-$C_6$)cycloalkyloxyle, aryloxyle, aryl($C_1$-$C_6$)alkyloxyle ou carbonyloxyle, lesdits radicaux pouvant éventuellement être substitués, ou représentant hydroxyle ; ou

représentant ($C_1$-$C_6$)alkylamino, halogéno($C_1$-$C_6$)alkylamino, dihalogéno($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$) cycloalkyl(($C_1$-$C_6$)alkyl)amino, arylamino, diarylamino, hétarylamino, dihétarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogéno($C_1$-$C_6$)alkylsulfanyle, halogéno($C_1$-$C_6$)alkylsulfinyle, halogéno($C_1$-$C_6$)alkylsulfanyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl($C_1$-$C_6$)alkylsulfanyle, ($C_3$-$C_6$)cycloalkyl($C_1$-$C_6$)alkylsulfinyle, ($C_3$-$C_6$)cycloalkyl($C_1$-$C_6$)alkylsulfonyle, arylsulfanyle, arylsulfinyle, arylsulfonyle, aryl($C_1$-$C_6$)alkylsulfanyle, aryl($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; et

$R^2$ représentant hydrogène, cyano, halogène ou nitro ; ou

représentant ($C_1$-$C_6$)alkyle, ($C_3$-$C_6$)cycloalkyl($C_1$-$C_6$)alkyle, halogéno($C_1$-$C_6$)alkyle, cyano($C_1$-$C_6$) alkyle, hydroxy($C_1$-$C_6$)alkyle, ($C_1$-$C_6$)alcoxy($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy ($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant($C_3$-$C_6$)cycloalkyle saturé ou insaturé éventuellement substitué, qui peut éventuellement être interrompu par un ou plusieurs hétéroatomes ; ou

représentant ($C_1$-$C_6$)alkylcarbonyle, halogéno($C_1$-$C_6$)alkylcarbonyle, hydroxy ($C_1$-$C_6$)alkylcarbonyle, ($C_1$-$C_6$)alcoxyalkyl($C_1$-$C_6$)carbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant($C_1$-$C_6$)alcoxyle, halogéno($C_1$-$C_6$)alcoxyle, ($C_1$-$C_6$)alcoxy ($C_1$-$C_6$)alcoxyle, aryloxyle, aryl($C_1$-$C_6$)alkyloxyle($C_3$-$C_6$)cycloalkyloxyle, ($C_3$-$C_6$)cycloalkyl($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou

représentant($C_1$-$C_6$)alkylamino, di($C_1$-$C_6$)alkylamino, halogéno($C_1$-$C_6$)alkylamino, dihalogéno($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hétarylamino, dihétarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogéno($C_1$-$C_6$)alkylsulfanyle, halogéno($C_1$-$C_6$)alkylsulfinyle, halogéno($C_1$-$C_6$)alkylsulfonyle, ($C_3$-$C_6$)cycloalkylsulfanyle, ($C_3$-$C_6$)cycloalkylsulfinyle,

$(C_3-C_6)$cycloalkylsulfonyle, $(C_3-C_6)$cycloalkyl$(C_1-C_6)$alkylsulfanyle, $(C_3-C_6)$cycloalkyl$(C_1-C_6)$alkylsulfinyle, $(C_3-C_6)$cycloalkyl$(C_1-C_6)$alkylsulfonyle, arylsulfanyle, arylsulfinyle, arylsulfonyle, aryl$(C_1-C_6)$alkylsulfanyle, aryl$(C_1-C_6)$alkylsulfinyle, aryl$(C_1-C_6)$alkylsulfonyle, aminosulfonyle, $(C_1-C_6)$alkylaminosulfonyle, di$(C_1-C_6)$alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou

représentant un cycle saturé ou insaturé, éventuellement substitué une ou plusieurs fois de manière identique ou différente par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle, ou $(C_3-C_6)$cycloalkyle éventuellement substitué et éventuellement interrompu par des hétéroatomes, de la série O, S ou N, à trois à six chaînons,

W représentant hydrogène ou halogène ;

V représentant oxygène ou soufre ;

X, Y et Z à chaque fois indépendamment les uns des autres, représentant hydrogène, halogène, hydroxyle, amino, cyano, nitro, OCN, SCN, $SF_5$; ou

représentant tri$(C_1-C_6)$alkylsilyle, $(C_1-C_6)$alkyle, halogéno$(C_1-C_6)$alkyle, cyano$(C_1-C_6)$alkyle, hydroxy$(C_1-C_6)$alkyle, $(C_1-C_6)$alcoxycarbonyl$(C_1-C_6)$alkyle, $(C_1-C_6)$alcoxy$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, $(C_1-C_6)$alcoxyle, halogéno$(C_1-C_6)$alcoxyle, cyano$(C_1-C_6)$alcoxyle, hydroxycarbonyl$(C_1-C_6)$alcoxyle, $(C_1-C_6)$alcoxycarbonyl$(C_1-C_6)$alcoxyle, $(C_1-C_6)$alcoxy $(C_1-C_6)$alcoxyle, $(C_1-C_6)$alkylhydroxyimino, $(C_1-C_6)$alcoxyimino, $(C_1-C_6)$alkyl $(C_1-C_6)$alcoxyimino, halogéno$(C_1-C_6)$alkyl$(C_1-C_6)$alcoxyimino, $(C_1-C_6)$alkylthio, halogéno$(C_1-C_6)$alkylthio, $(C_1-C_6)$alcoxy$(C_1-C_6)$alkylthio, $(C_1-C_6)$alkylthio$(C_1-C_6)$alkyle, $(C_1-C_6)$alkylsulfinyle, halogéno$(C_1-C_6)$alkylsulfinyle, $(C_1-C_6)$alcoxy$(C_1-C_6)$alkylsulfinyle, $(C_1-C_6)$alkylsulfinyl$(C_1-C_6)$alkyle, $(C_1-C_6)$alkylsulfonyle, halogéno$(C_1-C_6)$alkylsulfonyle, $(C_1-C_6)$alcoxy $(C_1-C_6)$alkylsulfonyle, $(C_1-C_6)$alkylsulfonyl$(C_1-C_6)$alkyle, $(C_1-C_6)$alkylsulfonyloxyle, $(C_1-C_6)$alkylcarbonyle, halogéno$(C_1-C_6)$alkylcarbonyle, carboxyle, $(C_1-C_6)$alkylcarbonyloxyle, $(C_1-C_6)$alcoxycarbonyle, halogéno$(C_1-C_6)$alcoxycarbonyle, aminocarbonyle, $(C_1-C_6)$alkylaminocarbonyle, di$(C_1-C_6)$alkylaminocarbonyle, $(C_2-C_6)$alcénylaminocarbonyle, di$(C_2-C_6)$alcénylaminocarbonyle, $(C_3-C_6)$cyclo$(C_1-C_6)$alkylaminocarbonyle, $(C_1-C_6)$alkylsulfonylamino, aminosulfonyle, $(C_1-C_6)$alkylaminosulfonyle, di$(C_1-C_6)$alkylaminosulfonyle, $(C_1-C_6)$alkylsulfoxymino, aminothiocarbonyle, $(C_1-C_6)$alkylaminothiocarbonyle, ou di$(C_1-C_6)$alkylaminothiocarbonyle, tous lesdits radicaux pouvant éventuellement être substitués par halogène ; ou

représentant phényl$(C_1-C_3)$alkyle, phénoxyle, phényl$(C_1-C_3)$alkyloxyle, phénoxy$(C_1-C_3)$alkyle, phénylthio, phénylthio$(C_1-C_3)$alkyle, phénylsulfinyle, phénylsulfonyle, hétaryl$(C_1-C_3)$alkyle, hétaryloxyle, hétaryl$(C_1-C_3)$alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant$(C_3-C_6)$cycloalkyl$(C_1-C_3)$alkyle, $(C_3-C_6)$cycloalkyloxyle, $(C_3-C_6)$cycloalkyl$(C_1-C_3)$alcoxyle, $(C_3-C_6)$cycloalkylthio, $(C_3-C_6)$cycloalkyl$(C_1-C_3)$alkylthio, $(C_3-C_6)$cycloalkylsulfinyle, $(C_3-C_6)$cycloalkyl$(C_1-C_3)$alkylsulfinyle, $(C_3-C_6)$cycloalkylsulfonyle, $(C_3-C_6)$cycloalkyl$(C_1-C_3)$alkylsulfonyle ou $(C_3-C_8)$cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R",

R' et R" représentant indépendamment l'un de l'autre hydrogène, cyano, $(C_1-C_6)$alkyle, halogéno$(C_1-C_6)$alkyle, cyano$(C_1-C_6)$alkyle, hydroxy$(C_1-C_6)$alkyle, $(C_1-C_6)$alcoxy$(C_1-C_6)$alkyle, $(C_1-C_6)$alkylthio$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, acyle ou $(C_1-C_6)$alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série O, S ou N, à cinq à sept chaînons ; ou

représentant $(C_3-C_6)$cycloalkyle, oxétane, oxolane, oxane, $(C_3-C_8)$cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle

éventuellement substitué par méthyle, fluor, chlore, cyano ;

ou X et Z, ou Y et Z, pouvant former les cycles suivants à 5 ou 6 chaînons, qui sont éventuellement substitués par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

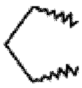 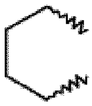 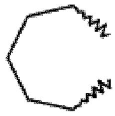 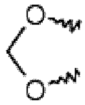 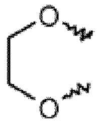 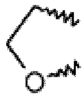 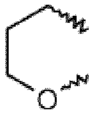

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

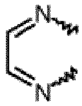 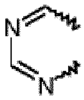 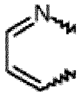 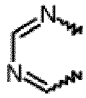 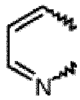 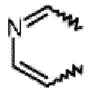

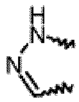 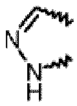 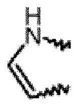

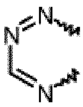 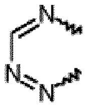 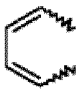

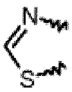 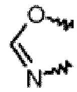 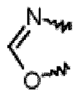

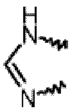 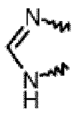 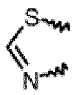

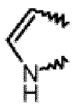 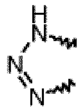 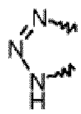

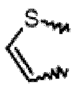 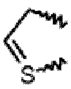 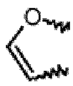 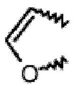

et

n représentant le nombre 0 ou 1.

**5.** Composé selon la revendication 4, dans lequel la sous-structure de formule (I-A) représente une sous-structure, qui est choisie dans le groupe constitué par

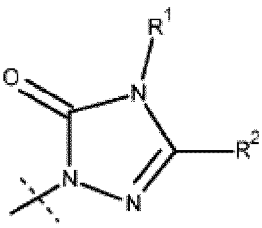

(I-A-1)

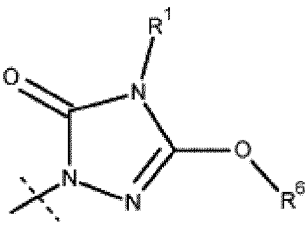

(I-A-2)

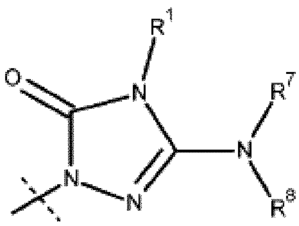

(I-A-3)

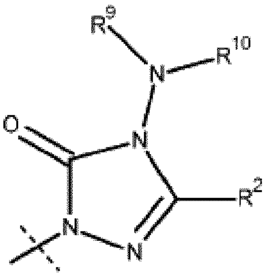

(I-A-4)

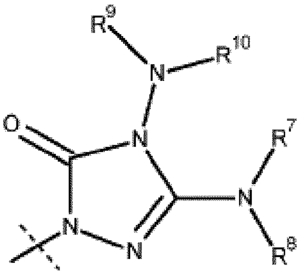

(I-A-5)

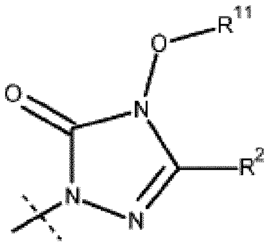

(I-A-6)

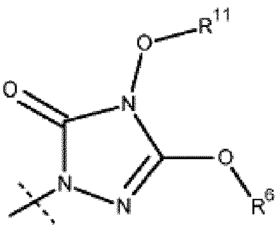

(I-A-7)

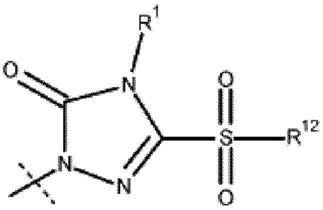

(I-A-8)

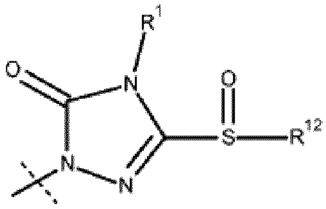

(I-A-9)

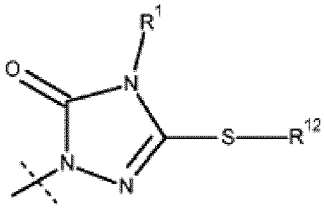

(I-A-10)

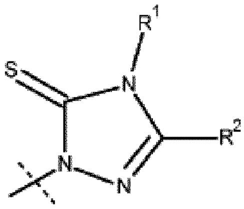

(I-A-11)

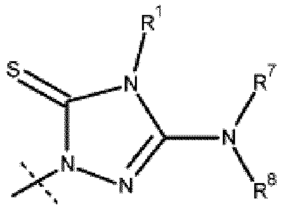

(I-A-12)

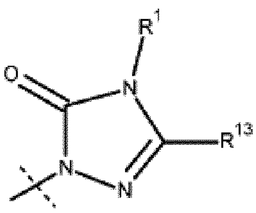

(I-A-13)

$R^1$ représentant hydrogène ; ou

représentant $(C_1-C_6)$alkyle, $(C_3-C_5)$cycloalkyl$(C_1-C_3)$alkyle, $(C_2-C_6)$alcényle, $(C_2-C_6)$alcynyle, halogéno$(C_2-C_6)$alkyle, cyano$(C_1-C_6)$alkyle, $(C_1-C_6)$alcoxy$(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou

représentant $(C_3-C_6)$cycloalkyle, $(C_3-C_6)$cycloalcényle, oxétane, oxolane, oxane, phényle, pyridinyle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^2$ représentant hydrogène ; ou

représentant $(C_1-C_6)$alkyle, $(C_3-C_6)$cycloalkyl$(C_1-C_3)$alkyle, $(C_2-C_6)$alcényle, $(C_2-C_6)$alcynyle, halogéno$(C_2-C_6)$alkyle, cyano$(C_1-C_6)$alkyle, $(C_1-C_6)$alcoxy$(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou

représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle, triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou

représentant carboxyle ;

$R^6$ représentant hydrogène ; ou

représentant $(C_1-C_6)$alkyle, $(C_3-C_6)$cycloalkyl$(C_1-C_3)$alkyle, $(C_2-C_6)$alcényle, $(C_2-C_6)$alcynyle, halogéno$(C_1-C_6)$alkyle, cyano $(C_1-C_6)$alkyle, $(C_1-C_6)$alcoxy$(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou

représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^7$ et $R^8$ représentant à chaque fois indépendamment l'un de l'autre hydrogène, $(C_1-C_6)$alkyle, halogéno$(C_2-C_6)$alkyle, cyano $(C_1-C_6)$alkyle ou $(C_3-C_6)$cycloalkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou conjointement avec l'atome d'azote auquel ils sont liés, formant une triazolinone, triazolidinethione, tétrazolinone, tétrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine-1-oxyde, thiomorpholine-1,1-dioxyde, pipérazine, *N*-méthylpipérazine ou *N*-éthylpipérazine éventuellement substituée ;

$R^9$ et $R^{10}$ représentant à chaque fois indépendamment l'un de l'autre hydrogène, $(C_1-C_6)$alkyle, halogéno $(C_2-C_6)$alkyle, cyano $(C_1-C_6)$alkyle, $(C_3-C_6)$cycloalkyle ou conjointement $(C_2-C_6)$alkylidène, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

ou conjointement avec l'atome d'azote auquel ils sont liés, formant une triazolinone, triazolidinethione, tétrazolinone,

tétrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine-1-oxyde, thiomorpholine-1,1-dioxyde, pipérazine, *N*-méthylpipérazine ou *N*-éthylpipérazine, éventuellement substituée ;

$R^{11}$ représentant hydrogène ; ou

représentant $(C_1\text{-}C_6)$alkyle, $(C_3\text{-}C_6)$cycloalkyl $(C_1\text{-}C_3)$alkyle, $(C_2\text{-}C_6)$alcényle, $(C_2\text{-}C_6)$alcynyle, halogéno$(C_1\text{-}C_6)$alkyle, cyano $(C_1\text{-}C_6)$alkyle, $(C_1\text{-}C_6)$alcoxy $(C_1\text{-}C_6)$alkyle ou phényl$(C_1\text{-}C_3)$alkyle, lesdits radicaux pouvant à chaque fois éventuellement être substitués une à trois fois par halogène, cyano, nitro, hydroxyle, amino, $(C_1\text{-}C_6)$alkyle, halogéno$(C_1\text{-}C_6)$alkyle, $(C_1\text{-}C_6)$alcoxyle, halogéno $(C_1\text{-}C_6)$alcoxyle, $(C_1\text{-}C_6)$alkylsulfinyle, $(C_1\text{-}C_6)$alkylsulfanyle, $(C_1\text{-}C_6)$alkylsulfonyle, halogéno$(C_1\text{-}C_6)$alkylsulfinyle, halogéno$(C_1\text{-}C_6)$alkylsulfanyle, halogéno$(C_1\text{-}C_6)$alkylsulfonyle ou cyclopropyle éventuellement substitué par méthyle, fluor, chlore, cyano ; ou

représentant $(C_3\text{-}C_6)$cycloalkyle, $(C_3\text{-}C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle, triazolyle, lesdits radicaux pouvant à chaque fois éventuellement être substitués une à trois fois par halogène, cyano, nitro, hydroxyle, amino, $(C_1\text{-}C_6)$alkyle, halogéno$(C_1\text{-}C_6)$alkyle, $(C_1\text{-}C_6)$alcoxyle, halogéno$(C_1\text{-}C_6)$alcoxyle, $(C_1\text{-}C_6)$alkylsulfinyle, $(C_1\text{-}C_6)$alkylsulfanyle, $(C_1\text{-}C_6)$alkylsulfonyle, halogéno$(C_1\text{-}C_6)$alkylsulfinyle, halogéno$(C_1\text{-}C_6)$alkylsulfanyle, halogéno$(C_1\text{-}C_6)$alkylsulfonyle ou cyclopropyle éventuellement substitué par méthyle, fluor, chlore, cyano ;

$R^{12}$ représentant $(C_1\text{-}C_6)$alkyle, $(C_3\text{-}C_6)$cycloalkyle, $(C_2\text{-}C_6)$alcényle, halogéno$(C_1\text{-}C_6)$alkyle, cyano$(C_1\text{-}C_6)$alkyle, phényl$(C_1\text{-}C_3)$alkyle ou $(C_3\text{-}C_6)$cyclo$(C_1\text{-}C_6)$alkyle, lesdits radicaux pouvant à chaque fois éventuellement être substitués une à trois fois par halogène, cyano, nitro, hydroxyle, amino, $(C_1\text{-}C_6)$alkyle, halogéno$(C_1\text{-}C_6)$alkyle, $(C_1\text{-}C_6)$alcoxyle, halogéno$(C_1\text{-}C_6)$alcoxyle, $(C_1\text{-}C_6)$alkylsulfinyle, $(C_1\text{-}C_6)$alkylsulfanyle, $(C_1\text{-}C_6)$alkylsulfonyle, halogéno$(C_1\text{-}C_6)$alkylsulfinyle, halogéno$(C_1\text{-}C_6)$alkylsulfanyle, halogène$(C_1\text{-}C_6)$alkylsulfonyle ou cyclopropyle éventuellement substitué par méthyle, fluor, chlore, cyano ;

$R^{13}$ représentant halogène ;

W représentant hydrogène ou halogène (en particulier fluor ou chlore) ;

X, Y et Z indépendamment les uns des autres représentant hydrogène, fluor, chlore, brome, cyano, nitro, amino, hydroxyle, $(C_1\text{-}C_4)$alkyle, halogéno$(C_1\text{-}C_4)$alkyle, $(C_2\text{-}C_4)$alcényle, $(C_2\text{-}C_4)$alcynyle, $(C_1\text{-}C_4)$alcoxyle, halogéno$(C_1\text{-}C_4)$alcoxyle ou aminothiocarbonyle ;

ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; et

n représentant le nombre 0 ou 1.

6. Composé selon la revendication 4 ou 5, dans lequel la sous-structure de formule (1-A) représente une sous-structure, qui est choisie dans le groupe constitué par

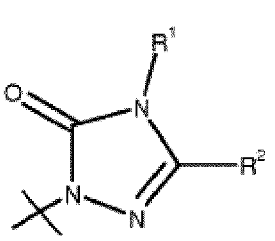

(I-A-1)

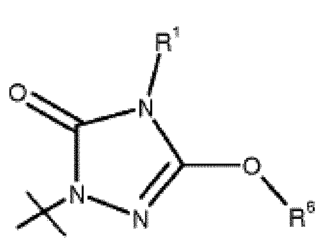

(I-A-2)

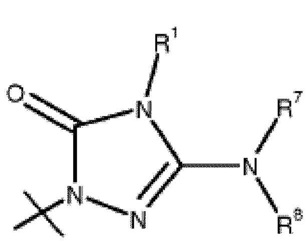

(I-A-3)

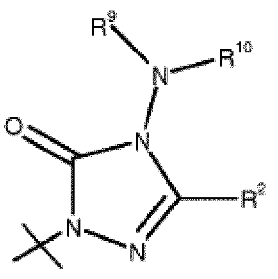

(I-A-4)

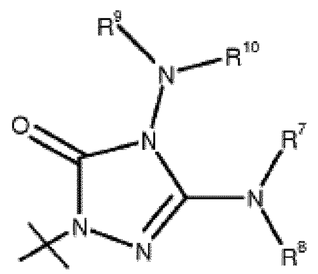

(I-A-5)

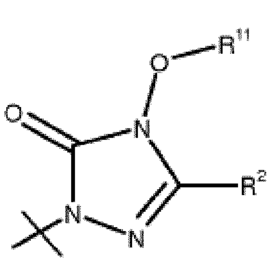

(I-A-6)

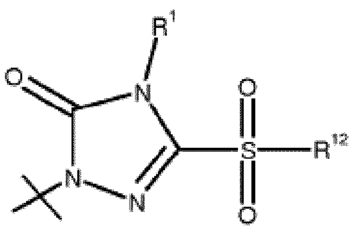

(I-A-8)

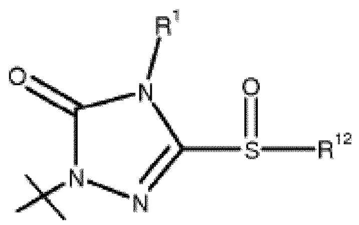

(I-A-9)

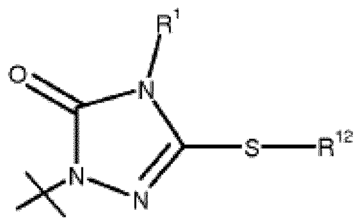

(I-A-10)

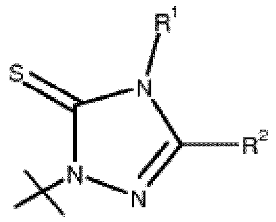

(I-A-11)

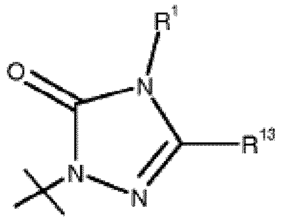

(I-A-13)

$R^1$ représentant hydrogène ; ou
représentant $(C_1-C_6)$alkyle, $(C_3-C_5)$cycloalkyl $(C_1-C_3)$alkyle, $(C_2-C_6)$alcényle, $(C_2-C_6)$alcynyle, halogéno$(C_2-C_6)$alkyle, cyano $(C_1-C_6)$alkyle, $(C_1-C_6)$alcoxy $(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyridinyle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^2$ représentant hydrogène ; ou
représentant $(C_1-C_6)$alkyle, $(C_3-C_6)$cycloalkyl $(C_1-C_3)$alkyle, $(C_2-C_6)$alcényle, $(C_2-C_6)$alcynyle, halogéno$(C_2-C_6)$alkyle, cyano$(C_1-C_6)$alkyle, $(C_1-C_6)$alcoxy$(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant carboxyle ;

$R^6$ représentant hydrogène ; ou
représentant $(C_1-C_6)$alkyle, $(C_3-C_6)$cycloalkyl$(C_1-C_3)$alkyle, $(C_2-C_6)$alcényle, $(C_2-C_6)$alcynyle, halogéno$(C_2-C_6)$alkyle, cyano $(C_1-C_6)$alkyle, $(C_1-C_6)$alcoxy $(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno $(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^7$ et $R^8$ représentant à chaque fois indépendamment l'un de l'autre hydrogène, $(C_1-C_6)$alkyle, halogéno$(C_2-C_6)$alkyle, cyano $(C_1-C_6)$alkyle ou $(C_3-C_6)$cycloalkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;
ou conjointement avec l'atome d'azote auquel ils sont liés, formant une triazolinone, triazolidinethione, tétrazolinone, tétrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine-1-oxyde, thiomorpholine-1,1-dioxyde, pipérazine, *N*-méthylpipérazine ou *N*-éthylpipérazine, éventuellement substituée ;

$R^9$ et $R^{10}$ représentant à chaque fois indépendamment l'un de l'autre hydrogène, $(C_1-C_6)$alkyle, halogéno $(C_2-C_6)$alkyle, cyano $(C_1-C_6)$alkyle, $(C_3-C_6)$cycloalkyle ou conjointement $(C_2-C_6)$alkylidène, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou conjointement avec l'atome d'azote auquel ils sont liés, formant une triazolinone, triazolidinethione, tétrazolinone, tétrazolidinethione, oxadiazolinone, pyrrolidinone, pyrrolidinethione, imidazolinone, imidazolidinethione, pyrrolidinedione, thiazolidinone, thiazolidinethione, morpholine, thiomorpholine, thiomorpholine-1-oxyde, thiomorpholine-1,1-dioxyde, pipérazine, *N*-méthylpipérazine ou *N*-éthylpipérazine éventuellement substituée ;

$R^{11}$ représentant hydrogène ; ou
représentant $(C_1-C_6)$alkyle, $(C_3-C_6)$cycloalkyl$(C_1-C_3)$alkyle, $(C_2-C_6)$alcényle, $(C_2-C_6)$alcynyle, halogéno$(C_2-C_6)$alkyle, cyano $(C_1-C_6)$alkyle, $(C_1-C_6)$alcoxy $(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $(C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle, triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^{12}$ représentant $(C_1-C_6)$alkyle, $(C_3-C_6)$cycloalkyle, $(C_2-C_6)$alcényle, halogéno$(C_1-C_6)$alkyle, cyano $(C_1-C_6)$alkyle, phényl $(C_1-C_3)$alkyle ou $(C_3-C_6)$cyclo$(C_1-C_6)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno $(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfi-

nyle, halogéno($C_1$-$C_6$)alkylsulfanyle, halogéno($C_1$-$C_6$)alkylsulfonyle ou cyclopropyle éventuellement substitué par méthyle, fluor, chlore, cyano ;

$R^{13}$ représentant halogène ;

W représentant hydrogène ou halogène en particulier fluor ou chlore ;

X, Y et Z indépendamment les uns des autres représentant hydrogène, fluor, chlore, brome, cyano, nitro, amino, hydroxyle, ($C_1$-$C_4$)alkyle, halogéno($C_1$-$C_4$)alkyle, ($C_2$-$C_4$)alcényle, ($C_2$-$C_4$)alcynyle, ($C_1$-$C_4$)alcoxyle, halogéno($C_1$-$C_4$)alcoxyle ou aminothiocarbonyle ;

ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; et

n représentant le nombre 0 ou 1.

**7.** Composé selon l'une quelconque des revendications 1 à 3, dans lequel
A et B conjointement avec les atomes, auxquels ils sont liés, représentent une sous-structure de formule (I-B)

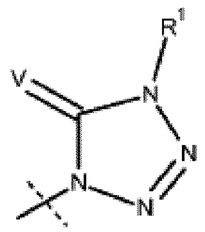

(I-B)

$R^1$ représentant hydrogène, cyano ou nitro ; ou

représentant ($C_1$-$C_6$)alkyle, ($C_3$-$C_6$)cycloalkyl($C_1$-$C_6$)alkyle, halogéno($C_2$-$C_6$)alkyle, cyano($C_1$-$C_6$)alkyle, hydroxy($C_1$-$C_6$)alkyle, ($C_1$-$C_6$)alcoxy ($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl ($C_1$-$C_6$)alkyle, halogéno($C_1$-$C_6$)alkylsulfinyl($C_1$-$C_6$)alkyle, halogéno($C_1$-$C_6$)alkylsulfonyl ($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylsulfanyl($C_1$-$C_6$)alkyle, hétarylsulfinyl($C_1$-$C_6$)alkyle, hétarylsulfonyl($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant ($C_3$-$C_6$)cycloalkyle saturé ou insaturé éventuellement substitué, qui peut éventuellement être interrompu par un ou plusieurs hétéroatomes ; ou

représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxy($C_1$-$C_6$)alkylcarbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou représentant phényle éventuellement substitué ou hétaryle éventuellement substitué ; ou

représentant ($C_1$-$C_6$) alcoxyle, halogéno ($C_1$-$C_6$) alcoxyle, ($C_3$-$C_6$)cycloalkyloxyle, aryloxyle, aryl($C_1$-$C_6$)alkyloxyle ou carbonyloxyle, lesdits radicaux pouvant à chaque fois éventuellement être substitués, ou représentant hydroxyle ; ou

représentant ($C_1$-$C_6$)alkylamino, halogéno($C_1$-$C_6$)alkylamino, dihalogéno($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hé-

tarylamino, dihétarylamino, $(C_1-C_6)$alkyl(aryl)amino, $(C_3-C_6)$cycloalkyl(aryl)amino, $(C_1-C_6)$alkylcarbonylamino, arylcarbonylamino, $(C_1-C_6)$alcoxycarbonylamino, aryloxycarbonylamino, $(C_1-C_6)$alkylcarbamoylamino, arylcarbamoylamino, $(C_1-C_6)$alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant $(C_1-C_6)$alkylsulfanyle, $(C_1-C_6)$alkylsulfinyle, $(C_1-C_6)$alkysulfonyle, halogéno$(C_1-C_6)$alkylsulfanyle, halogéno $(C_1-C_6)$alkylsulfinyle, halogéno $(C_1-C_6)$alkylsulfanyle, $(C_3-C_6)$cycloalkylsulfanyle, $(C_3-C_6)$cycloalkylsulfinyle, $(C_3-C_6)$cycloalkylsulfonyle, $(C_3-C_6)$cycloalkyl$(C_1-C_6)$alkylsulfanyle, $(C_3-C_6)$cycloalkyl$(C_1-C_6)$alkylsulfinyle, $(C_3-C_6)$cycloalkyl$(C_1-C_6)$alkylsulfonyle, arylsulfanyle, arylsulfinyle, arylsulfonyle, aryl$(C_1-C_6)$alkylsulfanyle, aryl$(C_1-C_6)$alkylsulfinyle, aryl$(C_1-C_6)$alkylsulfonyle, aminosulfonyle, $(C_1-C_6)$alkylaminosulfonyle, di$(C_1-C_6)$alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; et

V représentant oxygène, soufre ou un azote éventuellement substitué ;

X, Y et Z à chaque fois indépendamment les uns des autres,

représentant hydrogène, halogène, hydroxyle, amino, cyano, nitro, OCN, SCN, $SF_5$ ; ou

représentant tri$(C_1-C_6)$alkylsilyle, $(C_1-C_6)$alkyle, halogéno$(C_1-C_6)$alkyle, cyano $(C_1-C_6)$alkyle, hydroxy$(C_1-C_6)$alkyle, $(C_1-C_6)$alcoxycarbonyl$(C_1-C_6)$alkyle, $(C_1-C_6)$alcoxy$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno $(C_2-C_6)$alcynyle, cyano$(C_2-C_6)$alcynyle, $(C_1-C_6)$alcoxyle, halogéno $(C_1-C_6)$alcoxyle, cyano$(C_1-C_6)$alcoxyle, hydroxycarbonyl$(C_1-C_6)$alcoxyle, $(C_1-C_6)$alcoxycarbonyl$(C_1-C_6)$alcoxyle, $(C_1-C_6)$alcoxy$(C_1-C_6)$alcoxyle, $(C_1-C_6)$alkylhydroxyimino, $(C_1-C_6)$alcoxyimino, $(C_1-C_6)$alkyl$(C_1-C_6)$alcoxyimino, halogéno$(C_1-C_6)$alkyl $(C_1-C_6)$alcoxyimino, $(C_1-C_6)$alkylthio, halogéno $(C_1-C_6)$alkylthio, $(C_1-C_6)$ alcoxy $(C_1-C_6)$alkylthio, $(C_1-C_6)$alkylthio $(C_1-C_6)$alkyle, $(C_1-C_6)$alkylsulfinyle, halogéno$(C_1-C_6)$alkylsulfinyle, $(C_1-C_6)$alcoxy $(C_1-C_6)$alkylsulfinyle, $(C_1-C_6)$alkylsulfinyl$(C_1-C_6)$alkyle, $(C_1-C_6)$alkylsulfonyle, halogéno$(C_1-C_6)$alkylsulfonyle, $(C_1-C_6)$alcoxy$(C_1-C_6)$alkylsulfonyle, $(C_1-C_6)$alkylsulfonyl$(C_1-C_6)$alkyle, $(C_1-C_6)$alkylsulfonyloxyle, $(C_1-C_6)$alkylcarbonyle, halogéno$(C_1-C_6)$alkylcarbonyle, carboxyle, $(C_1-C_6)$alkylcarbonyloxyle, $(C_1-C_6)$alcoxycarbonyle, halogéno$(C_1-C_6)$alcoxycarbonyle, aminocarbonyle, $(C_1-C_6)$alkylaminocarbonyle, di$(C_1-C_6)$alkylaminocarbonyle, $(C_2-C_6)$alcénylaminocarbonyle, di$(C_2-C_6)$alcénylaminocarbonyle, $(C_3-C_6)$cyclo$(C_1-C_6)$alkylaminocarbonyle, $(C_1-C_6)$alkylsulfonylamino, aminosulfonyle, $(C_1-C_6)$alkylaminosulfonyle, di$(C_1-C_6)$alkylaminosulfonyle, $(C_1-C_6)$alkylsulfoxymino, aminothiocarbonyle, $(C_1-C_6)$ alkylaminothiocarbonyle, ou di$(C_1-C_6)$alkylaminothiocarbonyle, tous lesdits radicaux pouvant éventuellement être substitués par halogène ; ou

représentant phényl$(C_1-C_3)$alkyle, phénoxyle, phényl$(C_1-C_3)$alkyloxyle, phénoxy$(C_1-C_3)$alkyle, phénylthio, phénylthio$(C_1-C_3)$alkyle, phénylsulfinyle, phénylsulfonyle, hétaryl$(C_1-C_3)$alkyle, hétaryloxyle, hétaryl$(C_1-C_3)$alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant $(C_3-C_6)$cycloalkyl$(C_1-C_3)$alkyle, $(C_3-C_6)$cycloalkyloxyle, $(C_3-C_6)$cycloalkyl $(C_1-C_3)$alcoxyle, $(C_3-C_6)$cycloalkylthio, $(C_3-C_6)$cycloalkyl$(C_1-C_3)$alkylthio, $(C_3-C_6)$cycloalkylsulfinyle, $(C_3-C_6)$cycloalkyl $(C_1-C_3)$alkylsulfinyle, $(C_3-C_6)$cycloalkylsulfonyle, $(C_3-C_6)$cycloalkyl$(C_1-C_3)$alkylsulfonyle ou $(C_3-C_8)$cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R",

R' et R" représentant indépendamment l'un de l'autre

hydrogène, cyano, $(C_1-C_6)$alkyle, halogéno$(C_1-C_6)$alkyle, cyano$(C_1-C_6)$alkyle, hydroxy$(C_1-C_6)$alkyle, $(C_1-C_6)$alcoxy$(C_1-C_6)$alkyle, $(C_1-C_6)$alkylthio$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, acyle ou $(C_1-C_6)$alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série 0, S ou N, à cinq à sept chaînons ; ou

représentant $(C_3-C_6)$cycloalkyle, oxétane, oxolane, oxane, $(C_3-C_8)$cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle,

difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués de manière identique ou différente par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

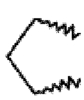 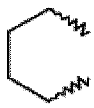 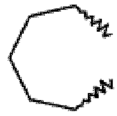 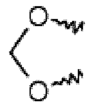 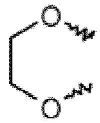 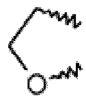 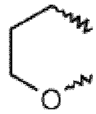

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

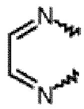 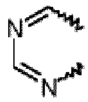 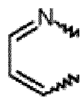 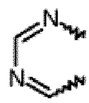 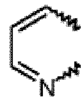 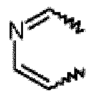

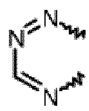 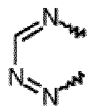 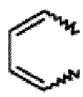

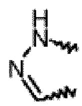 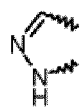 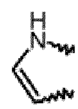

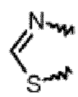 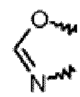 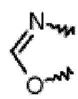

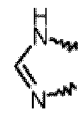 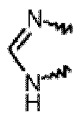 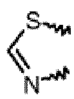

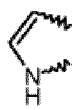 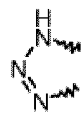 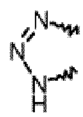

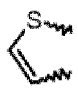 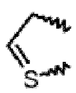 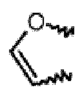 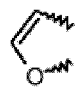

et

n représentant le nombre 0 ou 1.

**8.** Composé selon la revendication 7, dans lequel la sous-structure de formule (I-B) représente une sous-structure, qui est choisie dans le groupe constitué par

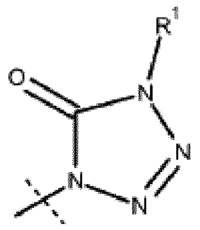

(I-B-1)

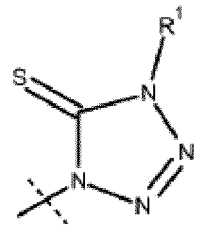

(I-B-2)

$R^1$ représentant hydrogène ; ou
représentant ($C_1$-$C_6$)alkyle, ($C_3$-$C_5$)cycloalkyl($C_1$-$C_3$)alkyle, ($C_2$-$C_6$)alcényle, ($C_2$-$C_6$)alcynyle, halogéno($C_2$-$C_6$)alkyle, cyano($C_1$-$C_6$)alkyle, ($C_1$-$C_6$)alcoxy($C_1$-$C_6$)alkyle ou phényl($C_1$-$C_3$)alkyle, lesdits radicaux pouvant à chaque fois éventuellement être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogène($C_1$-$C_6$)alkylsulfonyle ou cyclopropyle éventuellement substitué par méthyle, fluor, chlore, cyano ; ou
représentant ($C_3$-$C_6$)cycloalkyle, ($C_3$-$C_6$)cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ;

W représentant hydrogène ou halogène, en particulier fluor ou chlore ;

X, Y et Z indépendamment les uns des autres représentant hydrogène, fluor, chlore, brome, cyano, nitro, amino, hydroxyle, ($C_1$-$C_4$)alkyle, halogéno($C_1$-$C_4$)alkyle, ($C_2$-$C_4$)alcényle, ($C_2$-$C_4$)alcynyle, ($C_1$-$C_4$)alcoxyle, halogéno($C_1$-$C_4$)alcoxyle ou aminothiocarbonyle ;
ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;
ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; et

n représentant le nombre 0 ou 1.

**9.** Composé selon l'une quelconque des revendications 1 à 3, dans lequel
A et B conjointement avec les atomes, auxquels ils sont liés, représentent une sous-structure de formule (I-C)

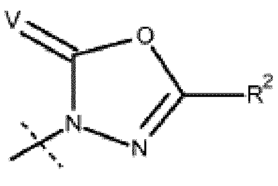

(I-C)

$R^2$ représentant hydrogène, cyano, halogène ou nitro ; ou
représentant ($C_1$-$C_6$)alkyle, ($C_3$-$C_6$)cycloalkyl($C_1$-$C_6$)alkyle, halogéno($C_1$-$C_6$)alkyle, cyano ($C_1$-$C_6$)alkyle, hydroxy($C_1$-$C_6$)alkyle, ($C_1$-$C_6$)alcoxy($C_1$-$C_6$)alkyle, amino ($C_1$-$C_6$)alkyle, ($C_1$-$C_6$)alcoxycarbonyl ($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfo-

nyl($C_1$-$C_6$)alkyle, ($C_1$-$C_6$)alcoxy ($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant($C_3$-$C_6$)cycloalkyle saturé ou insaturé éventuellement substitué, qui peut être interrompu par un ou plusieurs hétéroatomes ; ou

représentant ($C_1$-$C_6$)alkylcarbonyle, halogéno ($C_1$-$C_6$)alkylcarbonyle, hydroxy ($C_1$-$C_6$)alkylcarbonyle, ($C_1$-$C_6$)alcoxy($C_1$-$C_6$)alkylcarbonyle, phénylcarbonyle, hétarylcarbonyle, ($C_1$-$C_6$)alcoxycarbonyle, halogène($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogéno ($C_1$-$C_6$)alcoxyle, ($C_1$-$C_6$)alcoxy ($C_1$-$C_6$)alcoxyle, aryloxyle, aryl($C_1$-$C_6$)alkyloxyle, ($C_3$-$C_6$)cycloalkyloxyle, ($C_3$-$C_6$)cycloalkyl ($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou

représentant ($C_1$-$C_6$)alkylamino, di($C_1$-$C_6$)alkylamino, halogéno($C_1$-$C_6$) alkylamino, dihalogéno($C_1$-$C_6$) alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl (aryl) amino, ($C_3$-$C_6$)cycloalkyl(aryl)amino, ($C_1$-$C_6$)alkylcarbonylamino, arylcarbonylamino, ($C_1$-$C_1$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogéno ($C_1$-$C_6$)alkylsulfanyle, halogéno ($C_1$-$C_6$)alkylsulfinyle, halogéno ($C_1$-$C_6$) alkylsulfonyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl ($C_1$-$C_6$)alkylsulfanyle, ($C_3$-$C_6$)cycloalkyl ($C_1$-$C_6$)alkylsulfinyle, ($C_3$-$C_6$)cycloalkyl ($C_1$-$C_6$)alkylsulfonyle, arylsulfanyle, arylsulfinyle, arylsulfonyle, aryl($C_1$-$C_6$)alkylsulfanyle, aryl ($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou

représentant un cycle saturé ou insaturé, éventuellement substitué, de manière identique ou différente, une ou plusieurs fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogène($C_1$-$C_6$)alkylsulfanyle, halogéno($C_1$-$C_6$)alkylsulfonyle, ou ($C_3$-$C_6$)cycloalkyle éventuellement substitué et éventuellement interrompu par des hétéroatomes, de la série 0, S ou N, à trois à six chaînons,

W représentant hydrogène ou halogène ;

V représentant oxygène, soufre ou un azote éventuellement substitué ;

X, Y et Z à chaque fois indépendamment les uns des autres,

représentant hydrogène, halogène, hydroxyle, amino, cyano, nitro, OCN, SCN, $SF_5$ ; ou

représentant tri ($C_1$-$C_6$)alkylsilyle, ($C_1$-$C_6$)alkyle, halogéno($C_1$-$C_6$)alkyle, cyano($C_1$-$C_6$)alkyle, hydroxy($C_1$-$C_6$)alkyle, ($C_1$-$C_6$)alcoxycarbonyl($C_1$-$C_6$)alkyle, ($C_1$-$C_6$)alcoxy($C_1$-$C_6$)alkyle, ($C_2$-$C_6$)alcényle, halogéno($C_2$-$C_6$)alcényle, cyano($C_2$-$C_6$)alcényle, ($C_2$-$C_6$)alcynyle, halogéno($C_2$-$C_6$)alcynyle, cyano ($C_2$-$C_6$)alcynyle, ($C_1$-$C_6$)alcoxyle, halogéno ($C_1$-$C_6$)alcoxyle, cyano ($C_1$-$C_6$)alcoxyle, hydroxycarbonyl($C_1$-$C_6$)alcoxyle, ($C_1$-$C_6$)alcoxycarbonyl ($C_1$-$C_6$)alcoxyle, ($C_1$-$C_6$)alcoxy($C_1$-$C_6$)alcoxyle, ($C_1$-$C_6$)alkylhydroxyimino, ($C_1$-$C_6$)alcoxyimino, ($C_1$-$C_6$)alkyl($C_1$-$C_6$)alcoxyimino, halogéno($C_1$-$C_6$)alkyl($C_1$-$C_6$)alcoxyimino, ($C_1$-$C_6$)alkylthio, halogéno($C_1$-$C_6$)alkylthio, ($C_1$-$C_6$)alcoxy($C_1$-$C_6$)alkylthio, ($C_1$-$C_6$)alkylthio ($C_1$-$C_6$)alkyle, ($C_1$-$C_6$)alkylsulfinyle, halogéno($C_1$-$C_6$)alkylsulfinyle, ($C_1$-$C_6$)alcoxy ($C_1$-$C_6$)alkylsulfinyle, ($C_1$-$C_6$)alkylsulfinyl($C_1$-$C_6$)alkyle, ($C_1$-$C_6$)alkylsulfonyle, halogéno($C_1$-$C_6$)alkylsulfonyle, ($C_1$-$C_6$)alcoxy($C_1$-$C_6$)alkylsulfonyle, ($C_1$-$C_6$)alkylsulfonyl($C_1$-$C_6$) alkyle, ($C_1$-$C_6$)alkylsulfonyloxyle, ($C_1$-$C_6$)alkylcarbonyle, halogéno($C_1$-$C_6$)alkylcarbonyle, carboxyle, ($C_1$-$C_6$)alkylcarbonyloxyle, ($C_1$-$C_6$)alcoxycarbonyle, halogéno($C_1$-$C_6$)alcoxycarbonyle, aminocarbonyle, ($C_1$-$C_6$)alkylaminocarbonyle, di($C_1$-$C_6$)alkylaminocarbonyle,

($C_2$-$C_6$)alcénylaminocarbonyle, di($C_2$-$C_6$)alcénylaminocarbonyle, ($C_3$-$C_6$)cycloalkylaminocarbonyle, ($C_1$-$C_6$)alkylsulfonylamino, aminosulfonyle, ($C_1$-$C_6$)alkylaminosulfonyle, di($C_1$-$C_6$)alkylaminosulfonyle, ($C_1$-$C_6$)alkylsulfoxymino, aminothiocarbonyle, ($C_1$-$C_6$) alkylaminothiocarbonyle, ou di($C_1$-$C_6$)alkylaminothiocarbonyle, tous lesdits radicaux pouvant éventuellement être substitués par halogène ; ou

représentant phényl($C_1$-$C_3$)alkyle, phénoxyle, phényl($C_1$-$C_3$)alkyloxyle, phénoxy($C_1$-$C_3$)alkyle, phénylthio, phénylthio($C_1$-$C_3$)alkyle, phénylsulfinyle, phénylsulfonyle, hétaryl($C_1$-$C_3$)alkyle, hétaryloxyle, hétaryl($C_1$-$C_3$)alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou représentant ($C_3$-$C_6$)cycloalkyl($C_1$-$C_3$)alkyle, ($C_3$-$C_6$)cycloalkyloxyle, ($C_3$-$C_6$)cycloalkyl($C_1$-$C_3$)alcoxyle, ($C_3$-$C_6$)cycloalkylthio, ($C_3$-$C_6$)cycloalkyl ($C_1$-$C_3$)alkylthio, ($C_3$-$C_6$)cycloalkylsulfinyle, ($C_3$-$C_6$)cycloalkyl($C_1$-$C_3$)alkylsulfinyle, ($C_3$-$C_6$)cycloalkylsulfonyle, ($C_3$-$C_6$)cycloalkyl ($C_1$-$C_3$)alkylsulfonyle ou ($C_3$-$C_8$)cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R",

R' et R" représentant indépendamment l'un de l'autre

hydrogène, cyano, ($C_1$-$C_6$)alkyle, halogéno($C_1$-$C_6$)alkyle, cyano($C_1$-$C_6$)alkyle, hydroxy($C_1$-$C_6$)alkyle, ($C_1$-$C_6$)alcoxy($C_1$-$C_6$)alkyle, ($C_1$-$C_6$)alkylthio($C_1$-$C_6$)alkyle, ($C_2$-$C_6$)alcényle, halogéno($C_2$-$C_6$)alcényle, cyano($C_2$-$C_6$)alcényle, ($C_2$-$C_6$)alcynyle, halogéno($C_2$-$C_6$)alcynyle, cyano($C_2$-$C_6$)alcynyle, acyle ou ($C_1$-$C_6$)alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série 0, S ou N, à cinq à sept chaînons ; ou

représentant ($C_3$-$C_6$)cycloalkyle, oxétane, oxolane, oxane, ($C_3$-$C_8$)cycloalcényle, phényle, pyridinyle, pyrimidinyle, pyridazinyle, pyrazinyle, triazinyle, thiazolyle, thiazadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

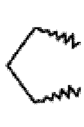 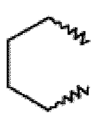 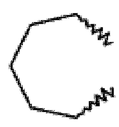 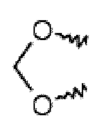 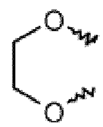 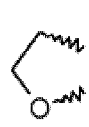 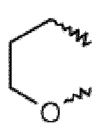

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

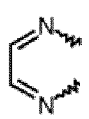 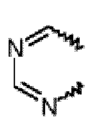 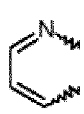 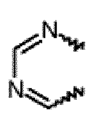 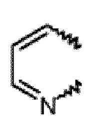 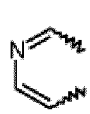

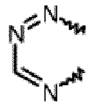
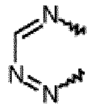
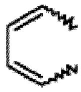
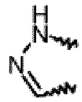
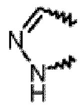
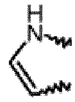
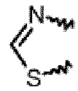
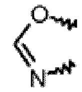
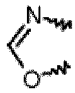
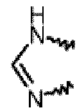
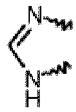
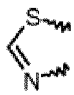
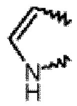
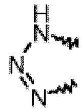
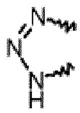
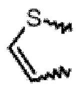
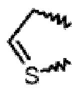
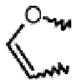
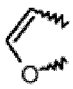

et

n représentant le nombre 0 ou 1.

**10.** Composé selon la revendication 9, dans lequel la sous-structure de formule (I-C) représente la sous-structure (I-C-1),

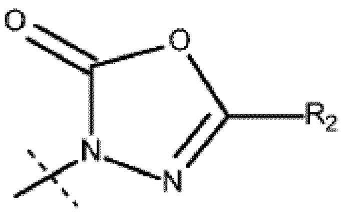

(I-C-1)

$R^2$ représentant hydrogène ; ou
représentant $(C_1-C_6)$alkyle, $(C_3-C_6)$cycloalkyl$(C_1-C_6)$alkyle, $(C_2-C_6)$alcényle, $(C_2-C_6)$alcynyle, halogéno$(C_1-C_6)$alkyle, cyano$(C_1-C_6)$alkyle, $(C_1-C_6)$alcoxy$(C_1-C_6)$alkyle ou phényl$(C_1-C_3)$alkyle, lesdits radicaux pouvant éventuellement être substitués ; ou
représentant $(C_3-C_6)$cycloalkyle, $(C_3-C_6)$cycloalcényle, phényle, pyridinyle, pyrimidinyle, pyridazinyle, pyrazinyle, furyle, thiényle, triazinyle, thiazolyle, thiadiazolyle, oxazolyle, oxadiazolyle, pyrazolyle ou triazolyle, lesdits radicaux pouvant à chaque fois être substitués une à trois fois par halogène, cyano, nitro, hydroxyle, amino, méthyle, éthyle, isopropyle, tert-butyle, trifluorométhyle, difluorométhyle, méthoxyle, trifluorométhoxyle, difluorométhoxyle, cyano, amino, hydroxyle, nitro, halogéno$(C_3-C_6)$alkylsulfanyle ou cyclopropyle éventuellement substitué par méthyle, fluor, chlore, cyano ;

W représentant hydrogène ou halogène, en particulier F ou Cl ;

X et Y représentant indépendamment l'un de l'autre hydrogène, fluor, chlore, brome, méthyle, éthyle, trifluorométhyle, 2,2-difluorométhyle, difluorométhoxyle, trifluorométhoxyle, cyclopropyle, cyano, amino, hydroxyle ou nitro ;

Z représentant hydrogène ; et

n représentant le nombre 0 ou 1.

**11.** Composé selon l'une quelconque des revendications 1 à 3, dans lequel
A et B conjointement avec les atomes, auxquels ils sont liés, représentent une sous-structure de formule (I-D)

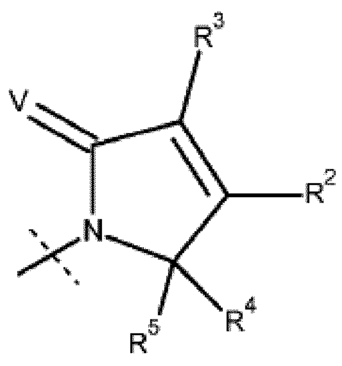

(I-D)

$R^2$, $R^3$, $R^4$, et $R^5$, à chaque fois indépendamment les uns des autres,
représentant hydrogène, cyano, halogène ou nitro ; ou
représentant ($C_1$-$C_6$)alkyle, ($C_3$-$C_6$)cycloalkyl($C_1$-$C_6$)alkyle, halogéno($C_1$-$C_6$)alkyle, cyano($C_1$-$C_6$)alkyle, hydroxy($C_1$-$C_6$)alkyle, ($C_1$-$C_6$)alcoxy($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$) alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou
représentant($C_3$-$C_6$)cycloalkyle saturé ou insaturé éventuellement substitué, qui peut éventuellement être interrompu par un ou plusieurs hétéroatomes ; ou
représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxyalkyl($C_1$-$C_6$)carbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl (($C_1$-$C_6$)alkyl) aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou
représentant phényle éventuellement substitué ou hétaryle éventuellement substitué ; ou
représentant ($C_1$-$C_6$)alcoxyle, halogéno($C_1$-$C_6$)alcoxyle, ($C_1$-$C_6$)alcoxy ($C_1$-$C_6$)alcoxyle, aryloxyle, aryl ($C_1$-$C_6$)alkyloxyle($C_3$-$C_6$)cycloalkyloxyle, ($C_3$-$C_6$)cycloalkyl($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou
représentant ($C_1$-$C_6$)alkylamino, di($C_1$-$C_6$)alkylamino, halogéno($C_1$-$C_6$)alkylamino, dihalogéno($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl(aryl)amino, (C3-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou
représentant ($C_1$-$C_6$)alkylsulfanyle, ($C_1$-$C_6$)alkylsulfinyle, ($C_1$-$C_6$)alkysulfonyle, halogéno($C_1$-$C_6$) alkylsulfanyle, halogéno($C_1$-$C_6$)alkylsulfinyle, halogéno($C_1$-$C_6$)alkylsulfonyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl($C_1$-$C_6$)alkylsulfanyle, ($C_3$-$C_6$)cycloalkyl($C_1$-$C_6$)alkylsulfinyle, ($C_3$-$C_6$)cycloalkyl($C_1$-$C_6$)alkylsulfonyle, arylsulfanyle, arylsulfinyle, arylsulfonyle, aryl($C_1$-$C_6$)alkylsulfanyle, aryl ($C_1$-$C_6$) alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou
$R^4$ et $R^5$, conjointement avec l'atome auquel ils sont liés, pouvant former un cycle saturé ou insaturé, éventuellement substitué une ou plusieurs fois de manière identique ou différente par halogène, cyano, nitro, hydroxyle, amino,

$(C_1-C_6)$alkyle, halogéno $(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle, ou $(C_3-C_6)$cycloalkyle éventuellement substitué et éventuellement interrompu par des hétéroatomes, de la série 0, S ou N, à trois à six chaînons ; ou

ou $R^2$ représentant un cycle saturé ou insaturé, éventuellement substitué une ou plusieurs fois de manière identique ou différente par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle, ou $(C_3-C_6)$cycloalkyle éventuellement substitué et éventuellement interrompu par des hétéroatomes, de la série 0, S ou N, à trois à six chaînons ;

W représentant hydrogène ou halogène ;

V représentant oxygène, soufre ou un azote éventuellement substitué ;

W représentant hydrogène ou halogène ;

X, Y et Z, à chaque fois indépendamment les uns des autres,

représentant hydrogène, halogène, hydroxyle, amino, cyano, nitro, OCN, SCN, $SF_5$ ; ou

représentant tri$(C_1-C_6)$alkylsilyle, $(C_1-C_6)$alkyle, halogéno$(C_1-C_6)$alkyle, cyano $(C_1-C_6)$alkyle, hydroxy$(C_1-C_6)$alkyle, $(C_1-C_6)$alcoxycarbonyl$(C_1-C_6)$alkyle, $(C_1-C_6)$alcoxy$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano $(C_2-C_6)$alcynyle, $(C_1-C_6)$alcoxyle, halogéno$(C_1-C_6)$alcoxyle, cyano$(C_1-C_6)$alcoxyle, hydroxycarbonyl$(C_1-C_6)$alcoxyle, $(C_1-C_6)$alcoxycarbonyl$(C_1-C_6)$alcoxyle, $(C_1-C_6)$alcoxy$(C_1-C_6)$alcoxyle, $(C_1-C_6)$alkylhydroxyimino, $(C_1-C_6)$alcoxyimino, $(C_1-C_6)$alkyl$(C_1-C_6)$alcoxyimino, halogéno$(C_1-C_6)$alkyl$(C_1-C_6)$alcoxyimino, $(C_1-C_6)$alkylthio, halogéno$(C_1-C_6)$alkylthio, $(C_1-C_6)$alcoxy$(C_1-C_6)$alkylthio, $(C_1-C_6)$alkylthio$(C_1-C_6)$alkyle, $(C_1-C_6)$alkylsulfinyle, halogéno$(C_1-C_6)$alkylsulfinyle, $(C_1-C_6)$alcoxy$(C_1-C_6)$alkylsulfinyle, $(C_1-C_6)$alkylsulfinyl$(C_1-C_6)$alkyle, $(C_1-C_6)$alkylsulfonyle, halogéno$(C_1-C_6)$alkylsulfonyle, $(C_1-C_6)$alcoxy$(C_1-C_6)$alkylsulfonyle, $(C_1-C_6)$alkylsulfonyl$(C_1-C_6)$alkyle, $(C_1-C_6)$alkylsulfonyloxyle, $(C_1-C_6)$alkylcarbonyle, halogéno$(C_1-C_6)$alkylcarbonyle, carboxyle, $(C_1-C_6)$alkylcarbonyloxyle, $(C_1-C_6)$alcoxycarbonyle, halogéno$(C_1-C_6)$alcoxycarbonyle, aminocarbonyle, $(C_1-C_6)$alkylaminocarbonyle, di$(C_1-C_6)$alkylaminocarbonyle, $(C_2-C_6)$alcénylaminocarbonyle, di$(C_2-C_6)$alcénylaminocarbonyle, $(C_3-C_6)$cycloalkylaminocarbonyle, $(C_1-C_6)$alkylsulfonylamino, aminosulfonyle, $(C_1-C_6)$alkylaminosulfonyle, di$(C_1-C_6)$alkylaminosulfonyle, $(C_1-C_6)$alkylsulfoxymino, aminothiocarbonyle, $(C_1-C_6)$alkylaminothiocarbonyle, ou di$(C_1-C_6)$alkylaminothiocarbonyle, tous lesdits radicaux pouvant éventuellement être substitués par halogène ; ou

représentant phényl$(C_1-C_3)$alkyle, phénoxyle, phényl$(C_1-C_3)$alkyloxyle, phénoxy$(C_1-C_3)$alkyle, phénylthio, phénylthio$(C_1-C_3)$alkyle, phénylsulfinyle, phénylsulfonyle, étaryl$(C_1-C_3)$alkyle, hétaryloxyle, hétaryl$(C_1-C_3)$alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant $(C_3-C_6)$cycloalkyl $(C_1-C_3)$alkyle, $(C_3-C_6)$cycloalkyloxyle, $(C_3-C_6)$cycloalkyl $(C_1-C_3)$alcoxyle, $(C_3-C_6)$cycloalkylthio, $(C_3-C_6)$cycloalkyl $(C_1-C_3)$alkylthio, $(C_3-C_6)$cycloalkylsulfinyle, $(C_3-C_6)$cycloalkyl$(C_1-C_3)$alkylsulfinyle, $(C_3-C_6)$cycloalkylsulfonyle, $(C_3-C_6)$cycloalkyl $(C_1-C_3)$alkylsulfonyle ou $(C_3-C_8)$ cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R",

R' et R" représentant indépendamment l'un de l'autre

hydrogène, cyano, $(C_1-C_6)$alkyle, halogéno$(C_1-C_6)$alkyle, cyano$(C_1-C_6)$alkyle, hydroxy$(C_1-C_6)$alkyle, $(C_1-C_6)$ alcoxy$(C_1-C_6)$alkyle, $(C_1-C_6)$alkylthio$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, acyle ou $(C_1-C_6)$alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série 0, S ou N, à cinq à sept chaînons ; ou

représentant $(C_3-C_6)$cycloalkyle, oxétane, oxolane, oxane, $(C_3-C_8)$cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués de manière identique ou différente par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

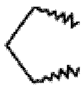 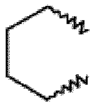 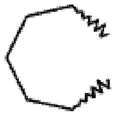 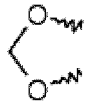 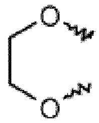 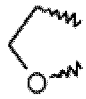 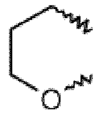

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

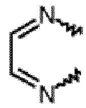 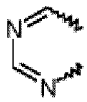 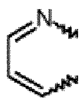 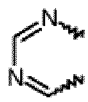 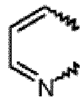 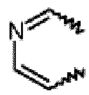

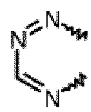 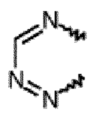 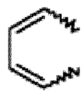 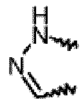 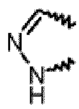 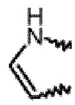

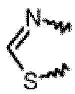 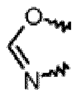 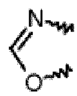

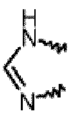 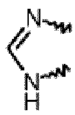 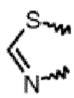

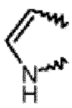 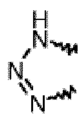 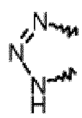

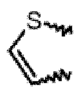 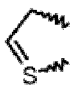 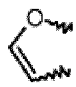 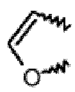

et

n représentant le nombre 0 ou 1.

**12.** Composé selon la revendication 11, dans lequel la sous-structure de formule (I-D) représente une sous-structure, qui est choisie dans le groupe constitué par

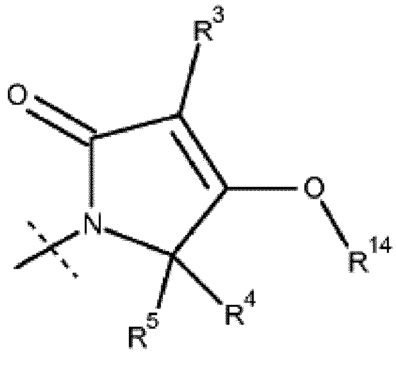
(I-D-1)

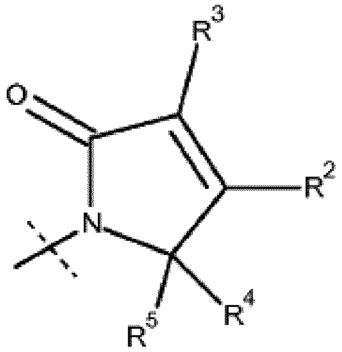
(I-D-2)

$R^2$, $R^3$, $R^4$ et $R^5$ indépendamment les uns des autres
représentant hydrogène ; ou
représentant ($C_1$-$C_6$)alkyle, ($C_3$-$C_6$)cycloalkyl ($C_1$-$C_3$)alkyle, ($C_2$-$C_6$)alcényle, ($C_2$-$C_6$)alcynyle, halogéno($C_1$-$C_6$)alkyle, cyano($C_1$-$C_6$)alkyle, ($C_1$-$C_6$)alcoxy ($C_1$-$C_6$)alkyle ou phényl($C_1$-$C_3$)alkyle, lesdits radicaux pouvant à chaque fois éventuellement être substitués une à trois fois par halogène, cyano, nitro, hydroxyle, amino, ($C_1$-$C_6$)alkyle, halogène($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ; ou
représentant ($C_3$-$C_6$)cycloalkyle, ($C_3$-$C_6$)cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle, triazolyle, lesdits radicaux pouvant à chaque fois éventuellement être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogène($C_1$-$C_6$)alkylsulfinyle, halogène($C_1$-$C_6$)alkylsulfanyle, halogène($C_1$-$C_6$)alkylsulfonyle ou cyclopropyle éventuellement substitué par méthyle, fluor, chlore, cyano ; ou
représentant carboxyle ; ou

$R^4$ et $R^5$ conjointement avec l'atome auquel ils sont liés, pouvant former un cycle saturé ou insaturé, éventuellement substitué une ou plusieurs fois de manière identique ou différente par halogène, cyano, nitro, hydroxyle, amino, ($C_1$-$C_6$)alkyle, halogène($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno ($C_1$-$C_6$)alkylsulfonyle, ou ($C_3$-$C_6$)cycloalkyle éventuellement substitué et éventuellement interrompu par des hétéroatomes, de la série 0, S ou N, à trois à six chaînons ;

$R^{14}$ représentant ($C_1$-$C_6$)alkyle, halogéno($C_1$-$C_6$)alkyle, cyano ($C_1$-$C_6$)alkyle, ($C_1$-$C_6$)alcoxy ($C_1$-$C_3$)alkyle, phényl ($C_1$-$C_3$)alkyle, ($C_3$-$C_6$)cycloalkyle ou ($C_3$-$C_6$)cycloalkyl ($C_1$-$C_3$)alkyle, lesdits radicaux pouvant à chaque fois éventuellement être substitués une à trois fois par halogène, cyano, nitro, hydroxyle, amino, ($C_1$-$C_6$)alkyle, halogène($C_1$-$C_6$)alkyle, ($C_1$-$C_6$)alcoxyle, halogène($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ;

X, Y et Z indépendamment les uns des autres représentant hydrogène, fluor, chlore, brome, cyano, nitro, amino, hydroxyle, ($C_1$-$C_4$)alkyle, halogéno($C_1$-$C_4$)alkyle, ($C_2$-$C_4$)alcényle, ($C_2$-$C_4$)alcynyle, ($C_1$-$C_4$)alcoxyle, halogéno ($C_1$-$C_4$)alcoxyle ou aminothiocarbonyle ;
ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;
ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

n représentant le nombre 0 ou 1.

**13.** Composé selon l'une quelconque des revendications 1 à 3, dans lequel

A et B conjointement avec les atomes, auxquels ils sont liés, représentent une sous-structure de formule (I-E)

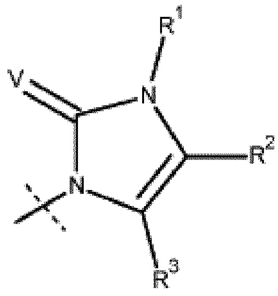

(I-E)

$R^1$ représentant hydrogène, cyano ou nitro ; ou

représentant ($C_1$-$C_6$)alkyle, ($C_3$-$C_6$)cycloalkyl ($C_1$-$C_6$)alkyle, halogéno($C_2$-$C_6$)alkyle, cyano ($C_1$-$C_6$)alkyle, hydroxy ($C_1$-$C_6$)alkyle, ($C_1$-$C_6$)alcoxy ($C_1$-$C_6$)alkyle, amino ($C_1$-$C_6$)alkyle, ($C_1$-$C_6$)alcoxycarbonyl ($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogène($C_1$-$C_6$)alkylsulfanyl($C_1$-$C_6$)alkyle, halogène($C_1$-$C_6$)alkylsulfinyl($C_1$-$C_6$)alkyle, halogène($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylsulfanyl($C_1$-$C_6$)alkyle, hétarylsulfinyl($C_1$-$C_6$)alkyle, hétarylsulfonyl($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant ($C_3$-$C_6$)cycloalkyle saturé ou insaturé éventuellement substitué, qui peut éventuellement être interrompu par un ou plusieurs hétéroatomes ; ou

représentant ($C_1$-$C_6$)alkylcarbonyle, halogène($C_1$-$C_6$)alkylcarbonyle, hydroxy ($C_1$-$C_6$)alkylcarbonyle, ($C_1$-$C_6$)alcoxy($C_1$-$C_6$)alkylcarbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_1$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogène($C_1$-$C_6$)alcoxyle, ($C_3$-$C_6$)cycloalkyloxyle, aryloxyle, aryl($C_1$-$C_6$)alkyloxyle ou carbonyloxyle, lesdits radicaux pouvant éventuellement être substitués, ou représentant hydroxyle ; ou

représentant ($C_1$-$C_6$)alkylamino, halogène($C_1$-$C_6$)alkylamino, dihalogéno($C_1$-$C_6$)alkylamino, di($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl (aryl) amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogène($C_1$-$C_6$)alkylsulfanyle, halogène($C_1$-$C_6$)alkylsulfinyle, halogène($C_1$-$C_6$)alkylsulfanyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl ($C_1$-$C_6$)alkylsulfanyle, ($C_3$-$C_6$)cycloalkyl ($C_1$-$C_6$)alkylsulfinyle, ($C_3$-$C_6$)cycloalkyl ($C_1$-$C_6$)alkylsulfonyle, arylsulfanyle, arylsulfinyle, arylsulfonyle, aryl ($C_1$-$C_6$)alkylsulfanyle, aryl ($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; et

$R^2$ et $R^3$ à chaque fois indépendamment l'un de l'autre,

représentant hydrogène, cyano, halogène ou nitro ; ou

représentant ($C_1$-$C_6$)alkyle, ($C_3$-$C_6$)cycloalkyl ($C_1$-$C_6$)alkyle, halogène($C_1$-$C_6$)alkyle, cyano ($C_1$-$C_6$)alkyle, hy-

droxy($C_1$-$C_6$)alkyle, ($C_1$-$C_6$)alcoxy ($C_1$-$C_6$)alkyle, amino ($C_1$-$C_6$)alkyle, ($C_1$-$C_6$)alcoxycarbonyl ($C_1$-$C_6$)alkyle, ($C_1$-$C_6$)alkylsulfanyl ($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy ($C_1$-$C_6$)alkylsulfanyl($C_1$-$C_6$)alkyle, ($C_1$-$C_6$)alcoxy ($C_1$-$C_6$)alkylsulfinyl($C_1$-$C_6$)alkyle, ($C_1$-$C_6$)alcoxy ($C_1$-$C_6$)alkylsulfonyl($C_1$-$C_6$)alkyle, phényl ($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant ($C_3$-$C_6$)cycloalkyle saturé ou insaturé éventuellement substitué, qui peut éventuellement être interrompu par un ou plusieurs hétéroatomes ; ou

représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxyalkyl($C_1$-$C_6$)carbonyle, phénylcarbonyle, hétarylcarbonyle, ($C_1$-$C_6$)alcoxycarbonyle, halogène($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl (($C_1$-$C_6$)alkyl) aminocarbonyle, arylaminocarbonyle, diarylaminocarbonyle, ($C_1$-$C_6$)alkyl (aryl) aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogéno($C_1$-$C_6$)alcoxyle, ($C_1$-$C_6$)alcoxy ($C_1$-$C_6$)alcoxyle, aryloxyle, aryl ($C_1$-$C_6$)alkyloxyle ($C_3$-$C_6$)cycloalkyloxyle, ($C_3$-$C_6$)cycloalkyl ($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant ou représentant hydroxyle ; ou

représentant ($C_1$-$C_6$)alkylamino, di($C_1$-$C_6$)alkylamino, halogène($C_1$-$C_6$)alkylamino, dihalogéno($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogéno($C_1$-$C_6$)alkylsulfanyle, halogéno($C_1$-$C_6$)alkylsulfinyle, halogéno($C_1$-$C_6$)alkylsulfonyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl ($C_1$-$C_6$)alkylsulfanyle, ($C_3$-$C_6$)cycloalkyl ($C_1$-$C_6$)alkylsulfinyle, ($C_3$-$C_6$)cycloalkyl ($C_1$-$C_6$)alkylsulfonyle, arylsulfanyle, arylsulfinyle, arylsulfonyle, aryl($C_1$-$C_6$)alkylsulfanyle, aryl ($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou

représentant un cycle saturé ou insaturé, éventuellement substitué une ou plusieurs fois de manière identique ou différente par halogène, cyano, nitro, hydroxyle, amino, ($C_1$-$C_6$)alkyle, halogéno ($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle, ou ($C_3$-$C_6$)cycloalkyle éventuellement substitué et éventuellement interrompu par des hétéroatomes, de la série 0, S ou N, à trois à six chaînons,

W représentant hydrogène ou halogène ;

V représentant oxygène, soufre ou un azote éventuellement substitué ;

X, Y et Z, à chaque fois indépendamment les uns des autres,

représentant hydrogène, halogène, hydroxyle, amino, cyano, nitro, OCN, SCN, $SF_5$ ; ou

représentant tri ($C_1$-$C_6$)alkylsilyle, ($C_1$-$C_6$)alkyle, halogéno($C_1$-$C_6$)alkyle, cyano($C_1$-$C_6$)alkyle, hydroxy ($C_1$-$C_6$)alkyle, ($C_1$-$C_6$)alcoxycarbonyl ($C_1$-$C_6$)alkyle, ($C_1$-$C_6$)alcoxy ($C_1$-$C_6$)alkyle, ($C_1$-$C_6$)alcényle, halogène($C_2$-$C_6$)alcényle, cyano ($C_2$-$C_6$)alcényle, ($C_2$-$C_6$)alcynyle, halogéno($C_2$-$C_6$)alcynyle, cyano ($C_2$-$C_6$)alcynyle, ($C_1$-$C_6$)alcoxyle, halogéno ($C_1$-$C_6$)alcoxyle, cyano ($C_1$-$C_6$)alcoxyle, hydroxycarbonyl($C_1$-$C_6$)alcoxyle, ($C_1$-$C_6$)alcoxycarbonyl ($C_1$-$C_6$)alcoxyle, ($C_1$-$C_6$)alcoxy ($C_1$-$C_6$)alcoxyle, ($C_1$-$C_6$)alkylhydroxyimino, ($C_1$-$C_6$)alcoxyimino, ($C_1$-$C_6$)alkyl ($C_1$-$C_6$)alcoxyimino, halogéno($C_1$-$C_6$)alkyl ($C_1$-$C_6$)alcoxyimino, ($C_1$-$C_6$)alkylthio, halogéno ($C_1$-$C_6$)alkylthio, ($C_1$-$C_6$)alcoxy ($C_1$-$C_6$)alkylthio, ($C_1$-$C_6$)alkylthio ($C_1$-$C_6$)alkyle, ($C_1$-$C_6$)alkylsulfinyle, halogéno($C_1$-$C_6$)alkylsulfinyle, ($C_1$-$C_6$)alcoxy($C_1$-$C_6$)alkylsulfinyle, ($C_1$-$C_6$)alkylsulfinyl($C_1$-$C_6$)alkyle, ($C_1$-$C_6$)alkylsulfonyle, halogène($C_1$-$C_6$)alkylsulfonyle, ($C_1$-$C_6$)alcoxy ($C_1$-$C_6$)alkylsulfonyle, ($C_1$-$C_6$)alkylsulfonyl($C_1$-$C_6$)alkyle, ($C_1$-$C_6$)alkylsulfonyloxyle, ($C_1$-$C_6$)alkylcarbonyle, halogéno($C_1$-$C_6$)alkylcarbonyle, carboxyle, ($C_1$-$C_6$)alkylcarbonyloxyle, ($C_1$-$C_6$)alcoxycarbonyle, halogéno($C_1$-$C_6$)alcoxycarbonyle, aminocarbo-

nyle, ($C_1$-$C_6$)alkylaminocarbonyle, di($C_1$-$C_6$)alkylaminocarbonyle, ($C_2$-$C_6$)alcénylaminocarbonyle, di($C_2$-$C_6$)alcénylaminocarbonyle, ($C_3$-$C_6$)cycloalkylaminocarbonyle, ($C_1$-$C_6$)alkylsulfonylamino, aminosulfonyle, ($C_1$-$C_6$)alkylaminosulfonyle, di($C_1$-$C_6$)alkylaminosulfonyle, ($C_1$-$C_6$)alkylsulfoxymino, aminothiocarbonyle, ($C_1$-$C_6$)alkylaminothiocarbonyle, ou di($C_1$-$C_6$)alkylaminothiocarbonyle, tous lesdits radicaux pouvant éventuellement être substitués par halogène ; ou

représentant phényl($C_1$-$C_3$)alkyle, phénoxyle, phényl($C_1$-$C_3$)alkyloxyle, phénoxy($C_1$-$C_3$)alkyle, phénylthio, phénylthio($C_1$-$C_3$)alkyle, phénylsulfinyle, phénylsulfonyle, hétaryl($C_1$-$C_3$)alkyle, hétaryloxyle, hétaryl($C_1$-$C_3$)alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant ($C_3$-$C_6$)cycloalkyl ($C_1$-$C_3$)alkyle, ($C_3$-$C_6$)cycloalkyloxyle, ($C_3$-$C_6$)cycloalkyl ($C_1$-$C_3$)alcoxyle, ($C_3$-$C_6$)cycloalkylthio, ($C_3$-$C_6$)cycloalkyl ($C_1$-$C_3$)alkylthio, ($C_3$-$C_6$)cycloalkylsulfinyle, ($C_3$-$C_6$)cycloalkyl ($C_1$-$C_3$)alkylsulfinyle, ($C_3$-$C_6$)cycloalkylsulfonyle, ($C_3$-$C_6$)cycloalkyl($C_1$-$C_3$)alkylsulfonyle ou ($C_3$-$C_8$)cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R", R' et R" représentant indépendamment l'un de l'autre hydrogène, cyano, ($C_1$-$C_6$)alkyle, halogène($C_1$-$C_6$)alkyle, cyano ($C_1$-$C_6$)alkyle, hydroxy ($C_1$-$C_6$)alkyle, ($C_1$-$C_6$)alcoxy ($C_1$-$C_6$)alkyle, ($C_1$-$C_6$)alkylthio ($C_1$-$C_6$)alkyle, ($C_2$-$C_6$)alcényle, halogène($C_2$-$C_6$)alcényle, cyano ($C_2$-$C_6$)alcényle, ($C_2$-$C_6$)alcynyle, halogène($C_2$-$C_6$)alcynyle, cyano ($C_2$-$C_6$)alcynyle, acyle ou ($C_1$-$C_6$)alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série 0, S ou N, à cinq à sept chaînons ; ou

représentant un ($C_3$-$C_6$)cycloalkyle, oxétane, oxolane, oxane, ($C_3$-$C_8$)cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

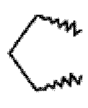 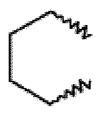 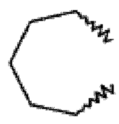 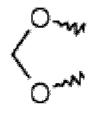 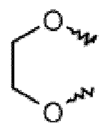 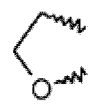 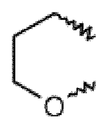

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

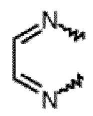 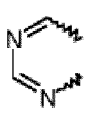 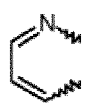 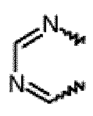 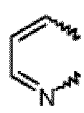 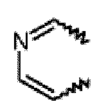

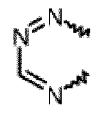
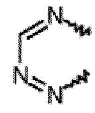
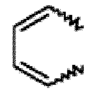
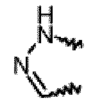
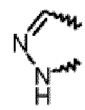
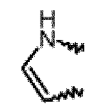
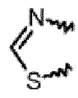
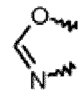
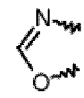
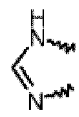
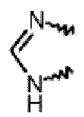
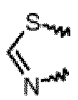
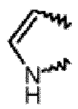
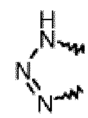
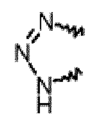
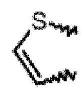
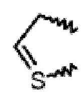
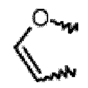
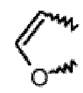

et

n représentant le nombre 0 ou 1.

**14.** Composé selon la revendication 13, dans lequel la sous-structure de formule (I-E) représente une sous-structure de formule (I-E-1),

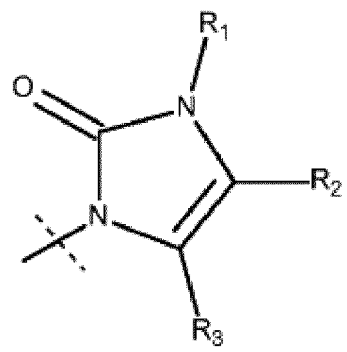

(I-E-1)

$R^1$ représentant hydrogène ; ou
représentant $(C_1-C_6)$alkyle, $(C_3-C_5)$cycloalkyl $(C_1-C_3)$ alkyle, $(C_2-C_6)$alcényle, $(C_2-C_6)$alcynyle, halogéno$(C_2-C_6)$alkyle, cyano $(C_1-C_6)$alkyle, $(C_1-C_6)$alcoxy $(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $(C_3-C_6)$cycloalcényle, oxétane, oxolane, oxane, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$ alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

$R^2$ et $R^3$ représentant indépendamment l'un de l'autre hydrogène ; ou
représentant $(C_1-C_6)$alkyle, $(C_3-C_6)$cycloalkyl $(C_1-C_3)$alkyle, $(C_2-C_6)$alcényle, $(C_2-C_6)$alcynyle, halogène$(C_2-C_6)$alkyle, cyano $(C_1-C_6)$alkyle, $(C_1-C_6)$alcoxy $(C_1-C_6)$alkyle ou phényl$(C_1-C_3)$alkyle, lesdits radicaux pouvant à chaque fois éventuellement être substitués une à trois fois par halogène, cyano, nitro, hydroxyle, amino, $(C_1-C_6)$alkyle, halogène$(C_1-C_6)$alkyle, $(C_1-C_6)$alcoxyle, halogéno $(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ; ou
représentant $(C_3-C_6)$cycloalkyle, $C_3-C_6)$cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, $(C_1-C_6)$alkyle, halogéno$(C_1-C_6)$alkyle, $(C_1-C_6)$alcoxyle, halogéno$(C_1-C_6)$alcoxyle, $(C_1-C_6)$alkylsulfinyle, $(C_1-C_6)$alkylsulfanyle, $(C_1-C_6)$alkylsulfonyle, halogéno$(C_1-C_6)$alkylsulfinyle, halogéno$(C_1-C_6)$alkylsulfanyle, halogéno$(C_1-C_6)$alkylsulfonyle ou cyclopropyle éventuellement substitué par méthyle, fluor, chlore, cyano ;

W représentant hydrogène ou halogène en particulier fluor ou chlore ;

X, Y et Z indépendamment les uns des autres représentant hydrogène, fluor, chlore, brome, cyano, nitro, amino, hydroxyle, $(C_1-C_4)$alkyle, halogéno$(C_1-C_4)$alkyle, $(C_2-C_4)$alcényle, $(C_2-C_4)$alcynyle, $(C_1-C_4)$alcoxyle, halogéno$(C_1-C_4)$alcoxyle ou aminothiocarbonyle ;
ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;
ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; et

n représentant le nombre 0 ou 1.

**15.** Composé selon l'une quelconque des revendications 1 à 3, dans lequel
A et B conjointement avec les atomes, auxquels ils sont liés, représentent une sous-structure de formule (I-F)

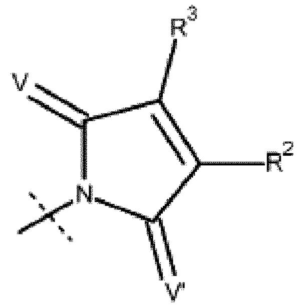

(I-F)

$R^2$ et $R^3$, à chaque fois indépendamment l'un de l'autre,

représentant hydrogène, cyano, halogène ou nitro ; ou

représentant $(C_1-C_6)$alkyle, $(C_3-C_6)$cycloalkyl $(C_1-C_6)$alkyle, halogéno$(C_1-C_6)$alkyle, cyano $(C_1-C_6)$alkyle, hydroxy $(C_1-C_6)$alkyle, $(C_1-C_6)$alcoxy $(C_1-C_6)$alkyle, amino $(C_1-C_6)$ alkyle, $(C_1-C_6)$alcoxycarbonyl $(C_1-C_6)$alkyle, $(C_1-C_6)$alkylsulfanyl$(C_1-C_6)$alkyle, $(C_1-C_6)$alkylsulfinyl$(C_1-C_6)$alkyle, $(C_1-C_6)$alkylsulfonyl$(C_1-C_6)$alkyle, halogéno$(C_1-C_6)$alkylsulfanyl$(C_1-C_6)$alkyle, halogéno$(C_1-C_6)$alkylsulfinyl$(C_1-C_6)$alkyle, halogéno$(C_1-C_6)$alkylsulfonyl$(C_1-C_6)$alkyle, $(C_1-C_6)$alcoxy$(C_1-C_6)$alkylsulfanyl$(C_1-C_6)$alkyle, $(C_1-C_6)$alcoxy$(C_1-C_6)$alkylsulfinyl$(C_1-C_6)$alkyle, $(C_1-C_6)$alcoxy$(C_1-C_6)$alkylsulfonyl$(C_1-C_6)$alkyle, phényl$(C_1-C_6)$alkyle, phénoxy$(C_1-C_6)$alkyle, phénylsulfanyl$(C_1-C_6)$alkyle, phénylsulfinyl$(C_1-C_6)$alkyle, phénylsulfonyl$(C_1-C_6)$alkyle, hétaryl$(C_1-C_6)$alkyle, hétaryloxy$(C_1-C_6)$alkyle, hétarylthio$(C_1-C_6)$alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant $(C_3-C_6)$cycloalkyle saturé ou insaturé éventuellement substitué, qui peut éventuellement être interrompu par un ou plusieurs hétéroatomes ; ou

représentant $(C_1-C_6)$alkylcarbonyle, halogéno$(C_1-C_6)$alkylcarbonyle, hydroxy$(C_1-C_6)$alkylcarbonyle, $(C_1-C_6)$al-

coxyalkyl($C_1$-$C_6$)carbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl) aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogéno($C_1$-$C_6$)alcoxyle, ($C_1$-$C_6$)alcoxy ($C_1$-$C_6$)alcoxyle, aryloxyle, aryl ($C_1$-$C_6$)alkyloxyle ($C_3$-$C_6$)cycloalkyloxyle, ($C_3$-$C_6$)cycloalkyl ($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou

représentant ($C_1$-$C_6$)alkylamino, di($C_1$-$C_6$)alkylamino, halogéno($C_1$-$C_6$)alkylamino, dihalogéno($C_1$-$C_6$)alkylamino, ($C_3$-$C_6$)cycloalkylamino, di($C_3$-$C_6$)cycloalkylamino, ($C_3$-$C_6$)cycloalkyl (($C_1$-$C_6$)alkyl)amino, arylamino, diarylamino, hétarylamino, dihétarylamino, ($C_1$-$C_6$)alkyl (aryl) amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou

représentant ($C_1$-$C_6$)alkylsulfanyle, ($C_1$-$C_6$)alkylsulfinyle, ($C_1$-$C_6$)alkysulfonyle, halogéno($C_1$-$C_6$)alkylsulfanyle, halogéno($C_1$-$C_6$)alkylsulfinyle, halogéno($C_1$-$C_6$)alkylsulfonyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl($C_1$-$C_6$)alkylsulfanyle, ($C_3$-$C_6$)cycloalkyl ($C_1$-$C_6$)alkylsulfinyle, ($C_3$-$C_6$)cycloalkyl ($C_1$-$C_6$)alkylsulfonyle, arylsulfanyle, arylsulfinyle, arylsulfonyle, aryl($C_1$-$C_6$)alkylsulfanyle, aryl($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou représentant un cycle saturé ou insaturé, éventuellement substitué une ou plusieurs fois de manière identique ou différente par halogène, cyano, nitro, hydroxyle, amino, ($C_1$-$C_6$)alkyle, halogéno ($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle, ou ($C_3$-$C_6$)cycloalkyle éventuellement substitué et éventuellement interrompu par des hétéroatomes, de la série 0, S ou N, à trois à six chaînons,

W représentant hydrogène ou halogène ;

V et V', à chaque fois indépendamment l'un de l'autre,

représentant oxygène, soufre ou un azote éventuellement substitué ;

X, Y et Z, à chaque fois indépendamment les uns des autres,

représentant hydrogène, halogène, hydroxyle, amino, cyano, nitro, OCN, SCN, $SF_5$ ; ou

représentant tri ($C_1$-$C_6$)alkylsilyle, ($C_1$-$C_6$)alkyle, halogéno($C_1$-$C_6$)alkyle, cyano ($C_1$-$C_6$)alkyle, hydroxy ($C_1$-$C_6$)alkyle, ($C_1$-$C_6$)alcoxycarbonyl ($C_1$-$C_6$)alkyle, ($C_1$-$C_6$)alcoxy ($C_1$-$C_6$)alkyle, ($C_2$-$C_6$)alcényle, halogène($C_2$-$C_6$)alcényle, cyano($C_2$-$C_6$)alcényle, ($C_2$-$C_6$)alcynyle, halogéno ($C_2$-$C_6$)alcynyle, cyano ($C_2$-$C_6$)alcynyle, ($C_1$-$C_6$)alcoxyle, halogéno($C_1$-$C_6$)alcoxyle, cyano($C_1$-$C_6$)alcoxyle, hydroxycarbonyl($C_1$-$C_6$)alcoxyle, ($C_1$-$C_6$)alcoxycarbonyl ($C_1$-$C_6$)alcoxyle, ($C_1$-$C_6$)alcoxy ($C_1$-$C_6$)alcoxyle, ($C_1$-$C_6$)alkylhydroxyimino, ($C_1$-$C_6$)alcoxyimino, ($C_1$-$C_6$)alkyl ($C_1$-$C_6$)alcoxyimino, halogéno($C_1$-$C_6$)alkyl ($C_1$-$C_6$)alcoxyimino, ($C_1$-$C_6$)alkylthio, halogène($C_1$-$C_6$)alkylthio, ($C_1$-$C_6$)alcoxy ($C_1$-$C_6$)alkylthio, ($C_1$-$C_6$)alkylthio ($C_1$-$C_6$)alkyle, ($C_1$-$C_6$)alkylsulfinyle, halogéno($C_1$-$C_6$)alkylsulfinyle, ($C_1$-$C_6$)alcoxy ($C_1$-$C_6$)alkylsulfinyle, ($C_1$-$C_6$)alkylsulfinyl($C_1$-$C_6$)alkyle, ($C_1$-$C_6$)alkylsulfonyle, halogène($C_1$-$C_6$)alkylsulfonyle, ($C_1$-$C_6$)alcoxy ($C_1$-$C_6$)alkylsulfonyle, ($C_1$-$C_6$)alkylsulfonyl($C_1$-$C_6$)alkyle, ($C_1$-$C_6$)alkylsulfonyloxyle, ($C_1$-$C_6$)alkylcarbonyle, halogéno($C_1$-$C_6$)alkylcarbonyle, carboxyle, ($C_1$-$C_6$)alkylcarbonyloxyle, ($C_1$-$C_6$)alcoxycarbonyle, halogéno($C_1$-$C_6$)alcoxycarbonyle, aminocarbonyle, ($C_1$-$C_6$)alkylaminocarbonyle, di($C_1$-$C_6$)alkylaminocarbonyle, ($C_2$-$C_6$)alcénylaminocarbonyle, di($C_2$-$C_6$)alcénylaminocarbonyle, ($C_3$-$C_6$)cyclo($C_1$-$C_6$)alkylaminocarbonyle, ($C_1$-$C_6$)alkylsulfonylamino, aminosulfonyle, ($C_1$-$C_6$)alkylaminosulfonyle, di($C_1$-$C_6$)alkylaminosulfonyle, ($C_1$-$C_6$)alkylsulfoxymino, aminothiocarbonyle, ($C_1$-$C_6$)alkylaminothiocarbonyle, ou di($C_1$-$C_6$)alkylaminothiocarbonyle, tous lesdits radicaux pouvant éventuellement être substitués par halogène ; ou

représentant phényl($C_1$-$C_3$)alkyle, phénoxyle, phényl($C_1$-$C_3$)alkyloxyle, phénoxy($C_1$-$C_3$)alkyle, phénylthio, phénylthio($C_1$-$C_3$)alkyle, phénylsulfinyle, phénylsulfonyle, hétaryl($C_1$-$C_3$)alkyle, hétaryloxyle, hétaryl($C_1$-$C_3$)alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par

méthyle, fluor, chlore, cyano ; ou

représentant $(C_3-C_6)$cycloalkyl $(C_1-C_3)$alkyle, $(C_3-C_6)$cycloalkyloxyle, $(C_3-C_6)$cycloalkyl $(C_1-C_3)$alcoxyle, $(C_3-C_6)$cycloalkylthio, $(C_3-C_6)$cycloalkyl $(C_1-C_3)$alkylthio, $(C_3-C_6)$cycloalkylsulfinyle, $(C_3-C_6)$cycloalkyl $(C_1-C_3)$alkylsulfinyle, $(C_3-C_6)$cycloalkylsulfonyle, $(C_3-C_6)$cycloalkyl $(C_1-C_3)$alkylsulfonyle ou $(C_3-C_8)$cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R'',

R' et R'' représentant indépendamment l'un de l'autre

hydrogène, cyano, $(C_1-C_6)$alkyle, halogéno$(C_1-C_6)$alkyle, cyano$(C_1-C_6)$alkyle, hydroxy$(C_1-C_6)$alkyle, $(C_1-C_6)$alcoxy$(C_1-C_6)$alkyle, $(C_1-C_6)$alkylthio$(C_1-C_6)$alkyle, $(C_2-C_6)$alcényle, halogéno$(C_2-C_6)$alcényle, cyano$(C_2-C_6)$alcényle, $(C_2-C_6)$alcynyle, halogéno$(C_2-C_6)$alcynyle, cyano$(C_2-C_6)$alcynyle, acyle ou $(C_1-C_6)$alcoxycarbonyle ; ou

R' et R'', conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série 0, S ou N, à cinq à sept chaînons ; ou

représentant un $(C_3-C_6)$cycloalkyle, oxétane, oxolane, oxane, $(C_3-C_8)$cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués de manière identique ou différente par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

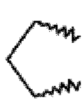 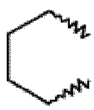 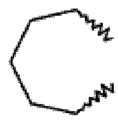 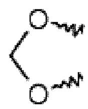 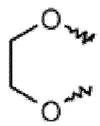 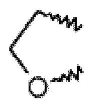 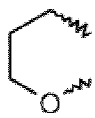

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

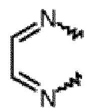 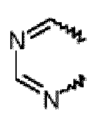 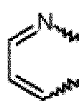 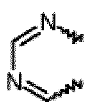 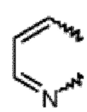 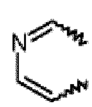

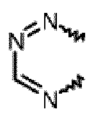 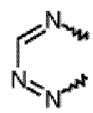 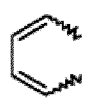 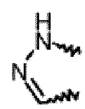 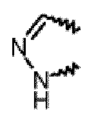 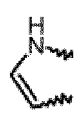

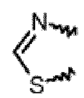 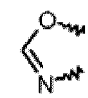 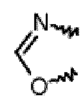 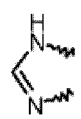 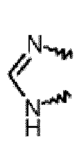 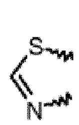

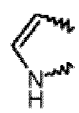 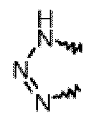 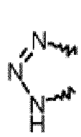

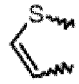 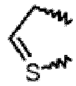 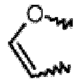 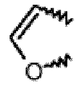

et

n représentant le nombre 0 ou 1.

**16.** Composé selon la revendication 15, dans lequel
la sous-structure de formule (I-F) représente une sous-structure de formule (I-F-1),

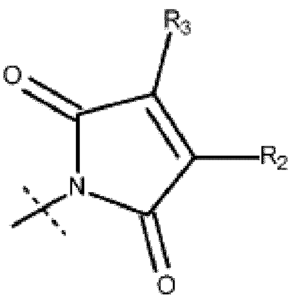

(I-F-1)

$R^2$ et $R^3$ représentant indépendamment l'un de l'autre hydrogène ou halogène ; ou

représentant ($C_1$-$C_6$)alkyle, ($C_3$-$C_6$)cycloalkyl($C_1$-$C_6$)alkyle, ($C_2$-$C_6$)alcényle, ($C_2$-$C_6$)alcynyle, halogéno($C_1$-$C_6$)alkyle, cyano($C_1$-$C_6$)alkyle, ($C_1$-$C_6$)alcoxy($C_1$-$C_6$)alkyle ou phényl($C_1$-$C_3$)alkyle, lesdits radicaux pouvant éventuellement être substitués ; ou

représentant ($C_3$-$C_6$)cycloalkyle, ($C_3$-$C_6$)cycloalcényle ou phényle, lesdits radicaux pouvant être substitués une à trois fois par halogène, cyano, nitro, hydroxyle, amino, méthyle, éthyle, isopropyle, tert-butyle, trifluorométhyle, difluorométhyle, méthoxyle, trifluorométhoxyle, difluorométhoxyle, cyano, amino, hydroxyle, nitro, halogéno($C_1$-$C_3$)alkylsulfanyle ou cyclopropyle éventuellement substitué par méthyle, fluor, chlore, cyano ;

W représentant hydrogène ou halogène, en particulier F ou Cl ;

X et Y représentant indépendamment l'un de l'autre hydrogène, fluor, chlore, brome, méthyle, éthyle, trifluorométhyle, 2,2-difluorométhyle, difluorométhoxyle, trifluorométhoxyle, cyclopropyle, cyano, amino, hydroxyle ou nitro ;

Z représentant hydrogène ; et

n représentant le nombre 0 ou 1.

**17.** Composé selon l'une quelconque des revendications 1 à 3, dans lequel
A et B conjointement avec les atomes, auxquels ils sont liés, représentent une sous-structure de formule (I-G)

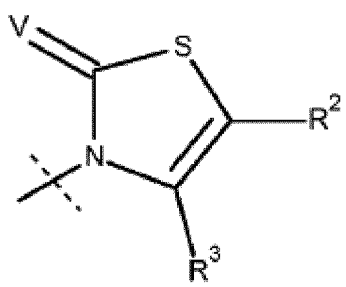

(I-G)

$R^2$ et $R^3$, à chaque fois indépendamment l'un de l'autre,

représentant hydrogène, cyano, halogène ou nitro ; ou

représentant ($C_1$-$C_6$)alkyle, ($C_3$-$C_6$)cycloalkyl($C_1$-$C_6$)alkyle, halogéno($C_1$-$C_6$)alkyle, cyano($C_1$-$C_6$)alkyle, hydroxy ($C_1$-$C_6$)alkyle, ($C_1$-$C_6$)alcoxy($C_1$-$C_6$)alkyle, amino($C_1$-$C_6$)alkyle, ($C_1$-$C_6$)alcoxycarbonyl($C_1$-$C_6$)alkyle, ($C_1$-$C_6$)alkylsulfanyl($C_1$-$C_6$)alkyle, ($C_1$-$C_6$)alkylsulfinyl($C_1$-$C_6$)alkyle, ($C_1$-$C_6$)alkylsulfonyl($C_1$-$C_6$)alkyle, halogéno($C_1$-$C_6$)alkylsulfanyl($C_1$-$C_6$)alkyle, halogéno($C_1$-$C_6$)alkylsulfinyl($C_1$-$C_6$)alkyle, halogéno($C_1$-$C_6$)alkylsulfonyl($C_1$-$C_6$)alkyle, ($C_1$-$C_6$)alcoxy($C_1$-$C_6$)alkylsulfanyl($C_1$-$C_6$)alkyle, ($C_1$-$C_6$)alcoxy($C_1$-$C_6$)alkylsulfinyl($C_1$-$C_6$)alkyle, ($C_1$-$C_6$)alcoxy($C_1$-$C_6$)alkylsulfonyl($C_1$-$C_6$)alkyle, phényl($C_1$-$C_6$)alkyle, phénoxy($C_1$-$C_6$)alkyle, phénylsulfanyl($C_1$-$C_6$)alkyle, phénylsulfinyl($C_1$-$C_6$)alkyle, phénylsulfonyl($C_1$-$C_6$)alkyle, hétaryl($C_1$-$C_6$)alkyle, hétaryloxy($C_1$-$C_6$)alkyle, hétarylthio($C_1$-$C_6$)alkyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ; ou

représentant ($C_3$-$C_6$)cycloalkyle saturé ou insaturé éventuellement substitué, qui peut éventuellement être interrompu par un ou plusieurs hétéroatomes ; ou

représentant ($C_1$-$C_6$)alkylcarbonyle, halogéno($C_1$-$C_6$)alkylcarbonyle, hydroxy($C_1$-$C_6$)alkylcarbonyle, ($C_1$-$C_6$)alcoxyalkyl($C_1$-$C_6$)carbonyle, phénylcarbonyle, hétarylcarbonyle, ($C_1$-$C_6$)alcoxycarbonyle, halogéno($C_1$-$C_6$)alcoxycarbonyle, aryloxycarbonyle, aminocarbonyle, ($C_1$-$C_6$)alkylaminocarbonyle, di($C_1$-$C_6$)alkylaminocarbonyle, ($C_3$-$C_6$)cycloalkylaminocarbonyle, di($C_3$-$C_6$)cycloalkylaminocarbonyle, ($C_3$-$C_6$)cycloalkyl(($C_1$-$C_6$)alkyl)aminocarbonyle, arylaminocarbonyle, diarylaminocarbonyle, ($C_1$-$C_6$)alkyl(aryl)aminocarbonyle, ($C_3$-$C_6$)cycloalkyl(aryl)aminocarbonyle, ($C_1$-$C_6$)alkylaminothiocarbonyle, di($C_1$-$C_6$)alkylaminothiocarbonyle ou aminothiocarbonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant carbonyle ou carboxyle ; ou

représentant phényle éventuellement substitué ou hétaryle éventuellement substitué ; ou

représentant ($C_1$-$C_6$)alcoxyle, halogéno($C_1$-$C_6$)alcoxyle, ($C_1$-$C_6$)alcoxy($C_1$-$C_6$)alcoxyle, aryloxyle, aryl($C_1$-$C_6$)alkyloxyle ($C_3$-$C_6$)cycloalkyloxyle, ($C_3$-$C_6$)cycloalkyl($C_1$-$C_6$)alkyloxyle ou carbonyloxyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant hydroxyle ; ou

représentant ($C_1$-$C_6$)alkylamino, di($C_1$-$C_6$)alkylamino, halogéno($C_1$-$C_6$)alkylamino, dihalogéno($C_1$-$C_6$)alkylamino, ($C_3$-$C_6$)cycloalkylamino, di($C_3$-$C_6$)cycloalkylamino, ($C_3$-$C_6$)cycloalkyl(($C_1$-$C_6$)alkyl)amino, arylamino, diarylamino, hétarylamino, dihétarylamino, ($C_1$-$C_6$)alkyl(aryl)amino, ($C_3$-$C_6$)cycloalkyl(aryl)amino, ($C_1$-$C_6$)alkylcarbonylamino, arylcarbonylamino, ($C_1$-$C_6$)alcoxycarbonylamino, aryloxycarbonylamino, ($C_1$-$C_6$)alkylcarbamoylamino, arylcarbamoylamino, ($C_1$-$C_6$)alkylsulfonylamino, ou arylsulfonylamino, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant amino ; ou repésentant ($C_1$-$C_6$)alkylsulfanyle, ($C_1$-$C_6$)alkylsulfinyle, ($C_1$-$C_6$)alkysulfonyle, halogéno($C_1$-$C_6$)alkylsulfanyle, halogéno($C_1$-$C_6$)alkylsulfinyle, halogéno($C_1$-$C_6$)alkylsulfonyle, ($C_3$-$C_6$)cycloalkylsulfanyle, ($C_3$-$C_6$)cycloalkylsulfinyle, ($C_3$-$C_6$)cycloalkylsulfonyle, ($C_3$-$C_6$)cycloalkyl ($C_1$-$C_6$)alkylsulfanyle, ($C_3$-$C_6$)cycloalkyl($C_1$-$C_6$)alkylsulfinyle, ($C_3$-$C_6$)cycloalkyl($C_1$-$C_6$)alkylsulfonyle, arylsulfanyle, arylsulfinyle, arylsulfonyle, aryl($C_1$-$C_6$)alkylsulfanyle, aryl($C_1$-$C_6$)alkylsulfinyle, aryl($C_1$-$C_6$)alkylsulfonyle, aminosulfonyle, ($C_1$-$C_6$)alkylaminosulfonyle, di($C_1$-$C_6$)alkylaminosulfonyle ou arylaminosulfonyle, lesdits radicaux pouvant à chaque fois éventuellement être substitués et/ou à condition que dans le cas d'un radical insaturé, le nombre minimal défini d'atomes de carbone est de 2, les radicaux pouvant être saturés ou insaturés ou représentant sulfanyle ; ou

représentant un cycle saturé ou insaturé, éventuellement substitué de manière identique ou différente, une ou plusieurs fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle, ou ($C_3$-$C_6$)cycloalkyle éventuellement substitué et éventuellement interrompu par des hétéroatomes, de la série 0, S ou N, à trois à six chaînons,

W représentant hydrogène ou halogène ;

V représentant oxygène, soufre ou un azote éventuellement substitué ;

X, Y et Z, à chaque fois indépendamment les uns des autres,

représentant hydrogène, halogène, hydroxyle, amino, cyano, nitro, OCN, SCN, $SF_5$ ; ou

représentant tri($C_1$-$C_6$)alkylsilyle, ($C_1$-$C_6$)alkyle, halogéno($C_1$-$C_6$)alkyle, cyano ($C_1$-$C_6$)alkyle, hydroxy($C_1$-$C_6$)alkyle, ($C_1$-$C_6$)alcoxycarbonyl($C_1$-$C_6$)alkyle, ($C_1$-$C_6$)alcoxy($C_1$-$C_6$)alkyle, ($C_2$-$C_6$) alcényle, halogéno($C_2$-$C_6$)alcényle, cyano($C_2$-$C_6$)alcényle, ($C_2$-$C_6$)alcynyle, halogéno($C_2$-$C_6$)alcynyle, cyano($C_2$-$C_6$)alcynyle, ($C_1$-$C_6$)alcoxyle, halogéno($C_1$-$C_6$)alcoxyle, cyano($C_1$-$C_6$)alcoxyle, hydroxycarbonyl($C_1$-$C_6$)alcoxyle, ($C_1$-$C_6$)alcoxycarbonyl($C_1$-$C_6$)alcoxyle, ($C_1$-$C_6$)alcoxy($C_1$-$C_6$)alcoxyle, ($C_1$-$C_6$)alkylhydroxyimino, ($C_1$-$C_6$)alcoxyimino, ($C_1$-$C_6$)alkyl($C_1$-$C_6$)alcoxyimino, halogéno($C_1$-$C_6$)alkyl($C_1$-$C_6$)alcoxyimino, ($C_1$-$C_6$)alkylthio, halogéno($C_1$-$C_6$)alkylthio, ($C_1$-$C_6$)alcoxy($C_1$-$C_6$)alkylthio, ($C_1$-$C_6$)alkylthio($C_1$-$C_6$)alkyle, ($C_1$-$C_6$)alkylsulfinyle, halogéno($C_1$-$C_6$)alkylsulfinyle,

$(C_1-C_6)$alcoxy$(C_1-C_6)$alkylsulfinyle, $(C_1-C_6)$alkylsulfinyl$(C_1-C_6)$alkyle, $(C_1-C_6)$alkylsulfonyle, halogéno$(C_1-C_6)$alkylsulfonyle, $(C_1-C_6)$alcoxy$(C_1-C_6)$alkylsulfonyle, $(C_1-C_6)$alkylsulfonyl$(C_1-C_6)$alkyle, $(C_1-C_6)$alkylsulfonyloxyle, $(C_1-C_6)$alkylcarbonyle, halogéno$(C_1-C_6)$alkylcarbonyle, carboxyle, $(C_1-C_6)$alkylcarbonyloxyle, $(C_1-C_6)$alcoxycarbonyle, halogéno$(C_1-C_6)$alcoxycarbonyle, aminocarbonyle, $(C_1-C_6)$alkylaminocarbonyle, di$(C_1-C_6)$alkylaminocarbonyle, $(C_2-C_6)$alcénylaminocarbonyle, di$(C_2-C_6)$alcénylaminocarbonyle, $(C_3-C_6)$cyclo$(C_1-C_6)$alkylaminocarbonyle, $(C_1-C_6)$alkylsulfonylamino, aminosulfonyle, $(C_1-C_6)$alkylaminosulfonyle, di$(C_1-C_6)$alkylaminosulfonyle, $(C_1-C_6)$alkylsulfoxymino, aminothiocarbonyle, $(C_1-C_6)$ alkylaminothiocarbonyle, ou di$(C_1-C_6)$alkylaminothiocarbonyle, tous lesdits radicaux pouvant éventuellement être substitués par halogène ; ou

représentant phényl$(C_1-C_3)$alkyle, phénoxyle, phényl$(C_1-C_3)$alkyloxyle, phénoxy$(C_1-C_3)$alkyle, phénylthio, phénylthio$(C_1-C_3)$alkyle, phénylsulfinyle, phénylsulfonyle, hétaryl$(C_1-C_3)$alkyle, hétaryloxyle, hétaryl$(C_1-C_3)$alkyloxyle, hétarylthio, hétarylsulfinyle ou hétarylsulfonyle, tous lesdits radicaux pouvant éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant $(C_3-C_6)$cycloalkyl$(C_1-C_3)$alkyle, $(C_3-C_6)$cycloalkyloxyle, $(C_3-C_6)$cycloalkyl$(C_1-C_3)$alcoxyle, $(C_3-C_6)$cycloalkylthio, $(C_3-C_6)$cycloalkyl$(C_1-C_3)$alkylthio, $(C_3-C_6)$cycloalkylsulfinyle, $(C_3-C_6)$cycloalkyl$(C_1-C_3)$alkylsulfinyle, $(C_3-C_6)$cycloalkylsulfonyle, $(C_3-C_6)$cycloalkyl$(C_1-C_3)$alkylsulfonyle ou $(C_3-C_8)$cycloalcényle, lesdits radicaux pouvant à chaque fois éventuellement être substitués une à trois fois par halogène, cyano, nitro, amino, hydroxyle, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; ou

représentant NR'R",

R' et R" représentant indépendamment l'un de l'autre

hydrogène, cyano, $(C_1-C_6)$alkyle, halogéno$(C_1-C_6)$alkyle, cyano$(C_1-C_6)$alkyle, hydroxy$(C_1-C_6)$alkyle, $(C_1-C_6)$alcoxy$(C_1-C_6)$alkyle, $(C_1-C_6)$alkylthio$(C_1-C_6)$alkyle, $(C_2-C_6)$alcényle, halogéno$(C_2-C_6)$alcényle, cyano$(C_2-C_6)$alcényle, $(C_2-C_6)$ alcynyle, halogéno$(C_2-C_6)$ alcynyle, cyano $(C_2-C_6)$alcynyle, acyle ou $(C_1-C_6)$alcoxycarbonyle ; ou

R' et R", conjointement avec l'atome N auquel ils sont liés, pouvant former un cycle saturé ou insaturé, éventuellement substitué de manière identique ou différente par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano et éventuellement interrompu par des hétéroatomes de la série O, S ou N, à cinq à sept chaînons ; ou

représentant un $(C_3-C_6)$cycloalkyle, oxétane, oxolane, oxane, $(C_3-C_8)$cycloalcényle, phényle, pyridinyle, pyrimidinyle, pyridazinyle, pyrazinyle, triazinyle, thiazolyle, thiadiazolyle, oxazolyle, oxadiazolyle, pyrazolyle ou triazolyle, qui éventuellement peut être substitué une ou plusieurs fois, de manière identique ou différente, par un substituant S1, qui est choisi dans le groupe constitué par halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou X et Z, ou Y et Z, pouvant former les cycles suivants à 5 ou 6 chaînons, qui sont éventuellement substitués par hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

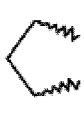 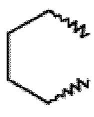 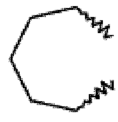 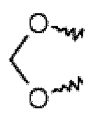 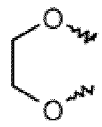 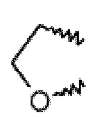 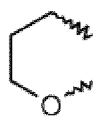

ou X et Z, ou Y et Z, pouvant former les cycles annelés suivants, qui sont éventuellement substitués une ou plusieurs fois, de manière identique ou différente, les substituants pouvant être choisis indépendamment les uns des autres parmi hydrogène, halogène, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano,

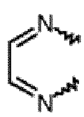 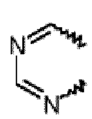 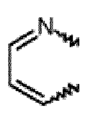 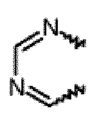 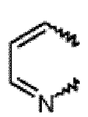 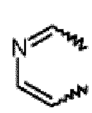

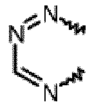
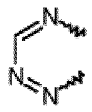
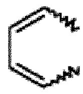
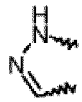
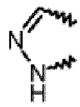
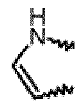
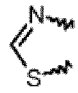
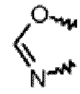
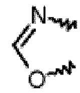
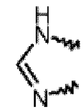
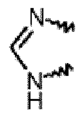
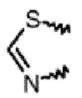
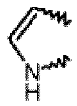
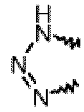
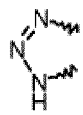
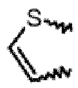
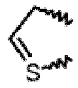
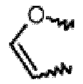
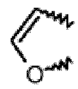

et

n représentant le nombre 0 ou 1.

**18.** Composé selon la revendication 17, dans lequel la sous-structure de formule (I-G) représente une sous-structure de formule (I-G-1),

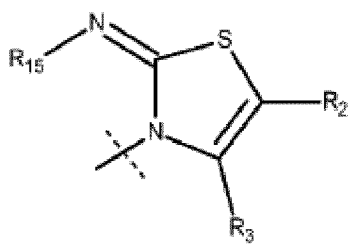

(I-G-1)

$R^2$ et $R^3$ indépendamment l'un de l'autre représentant hydrogène ; ou

représentant ($C_1$-$C_6$)alkyle, ($C_3$-$C_6$)cycloalkyl($C_1$-$C_3$)alkyle, ($C_2$-$C_6$)alcényle, ($C_2$-$C_6$)alcynyle, halogéno($C_1$-$C_6$)alkyle, cyano($C_1$-$C_6$)alkyle, ($C_1$-$C_6$)alcoxy($C_1$-$C_6$)alkyle ou phényl($C_1$-$C_3$)alkyle, lesdits radicaux pouvant à chaque fois éventuellement être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ; ou

représentant ($C_3$-$C_6$)cycloalkyle, ($C_3$-$C_6$)cycloalcényle, azétidinyle, azolidinyle, azinanyle, oxétanyle, oxolanyle, oxanyle, thiétanyle, thiolanyle, thianyle, phényle, pyrimidinyle, pyridazinyle, pyrazinyle, triazinyle, thiazolyle, thiazadiazolyle, oxazolyle, oxadiazolyle, pyrazolyle, triazolyle, lesdits radicaux pouvant à chaque fois être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ;

$R^{15}$ représentant hydrogène ; ou

représentant ($C_1$-$C_6$)alkyle, ($C_3$-$C_6$)cycloalkyl($C_1$-$C_3$)alkyle, ($C_2$-$C_6$)alcényle, ($C_2$-$C_6$)alcynyle, halogéno($C_2$-$C_6$)alkyle, cyano($C_1$-$C_6$)alkyle, ($C_1$-$C_6$)alcoxy($C_1$-$C_6$)alkyle ou phényl($C_1$-$C_3$)alkyle, lesdits radicaux pouvant à chaque fois éventuellement être substitués une à trois fois par halogène, cyano, nitro, hydroxyle, amino,

($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ; ou

représentant ($C_3$-$C_6$)cycloalkyle, ($C_3$-$C_6$)cycloalcényle, oxétane, oxolane, oxane, phényle, pyrimidinyle, pyridazinyle, pyrazinyle, triazinyle, thiazolyle, thiazadiazolyle, oxazolyle, oxadiazolyle, pyrazolyle ou triazolyle, lesdits radicaux pouvant à chaque fois éventuellement être substitués une à trois fois par halogène, cyano, nitro, hydroxyle, amino, ($C_1$-$C_6$)alkyle, halogéno($C_1$-$C_6$)alkyle, ($C_1$-$C_6$)alcoxyle, halogéno($C_1$-$C_6$)alcoxyle, ($C_1$-$C_6$)alkylsulfinyle, ($C_1$-$C_6$)alkylsulfanyle, ($C_1$-$C_6$)alkylsulfonyle, halogéno($C_1$-$C_6$)alkylsulfinyle, halogéno($C_1$-$C_6$)alkylsulfanyle, halogéno($C_1$-$C_6$)alkylsulfonyle ou cyclopropyle éventuellement substitué par méthyle, fluor, chlore, cyano ;

W représentant hydrogène ou halogène en particulier fluor ou chlore ;

X, Y et Z indépendamment les uns des autres représentant hydrogène, fluor, chlore, brome, cyano, nitro, amino, hydroxyle, ($C_1$-$C_4$)alkyle, halogéno($C_1$-$C_4$)alkyle, ($C_2$-$C_4$)alcényle, ($C_2$-$C_4$)alcynyle, ($C_1$-$C_4$)alcoxyle, halogéno($C_1$-$C_4$)alcoxyle ou aminothiocarbonyle ;

ou représentant un benzyle, phénoxyle, phénylthio, cyclopropylméthyle, cyclopropyloxyle ou cyclopropylthio, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ;

ou représentant un cyclopropyle, cyclobutyle, cyclopentyle, cyclohexyle ou phényle, qui peut éventuellement être substitué une fois ou plusieurs fois, de manière identique ou différente, par fluor, chlore, brome, cyano, nitro, hydroxyle, amino, méthyle, éthyle, trifluorométhyle, difluorométhyle, trifluoroéthyle, difluoroéthyle, méthoxyle, éthoxyle, trifluorométhoxyle, trifluoroéthoxyle ou cyclopropyle éventuellement substitué par méthyle, fluor, chlore, cyano ; et

n représentant le nombre 0 ou 1.

**19.** Composition de principes actifs contenant au moins un composé de formule générale (I) selon l'une quelconque des revendications 1 à 18 et au moins un autre principe actif insecticide, acaricide ou nématicide choisi dans le groupe constitué par

(1) des inhibiteurs de l'acétylcholinestérase (AChE), comme par exemple

des carbamates, par exemple alanycarbe, aldicarbe, bendiocarbe, benfuracarbe, butocarboxime, butoxycarboxime, carbaryl, carbofuran, carbosulfan, éthiophéncarbe, fénobucarbe, formétanate, furathiocarbe, isoprocarbe, méthiocarbe, méthomyle, métolcarbe, oxamyle, pirimicarbe, propoxur, thiodicarbe, thiofanox, triazamate, triméthacarbe, XMC et xylylcarbe ; ou

des organophosphates, par exemple acéphate, azaméthiphos, azinphos-éthyl, azinphos-méthyl, cadusafos, chloréthoxyphos, chlorfenvinphos, chlorméphos, chloropyrifos, chloropyrifos-méthyl, coumaphos, cyanophos, déméton-S-méthyl, diazinone, dichlorvos/DDVP, dicrotophos, diméthoate, diméthylvinphos, disulfotone, EPN, éthione, éthoprophos, famphur, fenamiphos, fénitrothione, fenthione, fosthiazate, hepténophos, imicyafos, isofenphos, isopropyl O-(méthoxyaminothio-phosphoryl) salicylate, isoxathione, malathione, mecarbam, méthamidophos, méthidathione, mevinphos, monocrotophos, naled, ométhoate, oxydéméton-méthyl, parathione, parathion-méthyl, phénthoate, phorate, phosalone, phosmet, phosphamidone, phoxime, pirimiphos-méthyl, profénophos, propétamphos, prothiofos, pyraclofos, pyridaphénthione, quinalphos, sulfotep, tébupirimfos, téméphos, terbufos, tétrachlorvinphos, thiométone, triazophos, trichlorfon et vamidothion.

(2) des antagonistes du canal chlorure GABAergiques, comme par exemple

les cyclodiènes/hydrocarbures chlorés, par exemple le chlordane et l'endosulfan ; ou les phénylpyrazole (fiproles), par exemple l'éthiprole et le finopril.

(3) des modulateurs du canal sodium/les bloqueurs du canal sodium dépendant de la tension, comme par exemple

les pyréthroïdes, par exemple Acrinathrine, Alléthrine, d-cis-trans-Alléthrine, d-trans-Alléthrine, Bifenthrine, Bioalléthrine, Bioalléthrine isomère S-Cyclopentényle, Bioresméthrine, Cycloprothrine, Cyfluthrine, bêta-Cyfluthrine, Cyhalothrine, lambda-Cyhalothrine, gamma-Cyhalothrine, Cyperméthrine, alpha-Cyperméthrine, bêta-Cyperméthrine, thêta-Cyperméthrine, zêta-Cyperméthrine, Cyphénothrine [isomère (1*R*)-trans-], Deltaméthrine, empenthrine [isomères (EZ)-(1*R*)), Esfenvalérate, Etofenprox, Fenpropathrin, Fenvalérate, Flucythrinate, Fluméthrine, tau-Fluvalinate, Halfenprox, Imiprothrine, Kadéthrine, Perméthrin, phénothrine [isomère (1*R*)-trans], Pralléthrine, Pyréthrine (Pyréthrum), Resméthrine, Silafluofène, Téfluthrine, Tétraméthrine, Tétramethrine [isomère (1*R*)], Tralométhrine et Transfluthrine ; ou

DDT ; Méthoxychlor.

(4) des agonistes des récepteurs nicotiniques de l'acétylcholine (nAChR), comme par exemple

les néonicotinoïdes, par exemple Acétamipride, Clothianidine, Dinotéfuran, Imidaclopride, Nitenpyram, Thiaclopride et Thiaméthoxame ; ou
Nicotine.

(5) des activateurs allostériques des récepteurs nicotiniques de l'acétylcholine (nAChR), comme par exemple les spinosynes, par exemple Spinétoram et Spinosad.
(6) des activateurs du canal chlorure, comme par exemple
les avermectines/milbémycines, par exemple Abamectine, Benzoate d'Émamectine, Lépimectine et Milbémectine.
(7) des imitateurs d'hormones juvéniles, comme par exemple

les analogues d'hormones juvéniles, par exemple Hydroprène, Kinoprène et Méthoprène ; ou
Fénoxycarbe ; ou Pyriproxyfène.

(8) des principes actifs présentant des mécanismes d'action inconnus ou non spécifiques, comme par exemple

les halogénures d'alkyle, par exemple le bromure de méthyle et d'autres halogénures d'alkyle ; ou
Chloropicrine ; ou Fluorure de sulfuryle ; ou Borax ; ou Tartrate de potassium et d'antimoine.

(9) des inhibiteurs d'alimentation, par exemple Pymétrozine ; ou Flonicamide.
(10) des inhibiteurs de croissance des acariens, par exemple Clofentézine, Hexythiazox et Diflovidazine ; ou Étoxazole.
(11) des perturbateurs microbiens de la membrane de l'intestin des insectes, par exemple Bacillus thuringiensis Subspezies israelensis, Bacillus sphaericus, Bacillus thuringiensis sous-espèce aizawai, Bacillus thuringiensis sous-espèce kurstaki, Bacillus thuringiensis sous-espèce tenebrionis et les protéines végétales BT : Cry1Ab, Cry1Ac, Cry1Fa, Cry2Ab, mCry3A, Cry3Ab, Cry3Bb, Cry34/35Ab1.
(12) des inhibiteurs de la phosphorylation oxydative, des perturbateurs de l'ATP, comme par exemple Diafenthurion ; ou

des composés organostanniques, par exemple Azocyclotine, Cyhexatine et Fenbutatine-oxyde ; ou
Propargite ; ou Tétradifon.

(13) des découpleurs de la phosphorylation oxydative par interruption du gradient de proton H, comme par exemple Chlorfénapyr, DNOC et Sulfuramide.
(14) des antagonistes des récepteurs nicotiniques de l'acétylcholine, comme par exemple Bensutalp, Chlorhydrate de Cartap, Thiocyclame et Thiosultap-sodium.
(15) des inhibiteurs de la biosynthèse de la chitine, Type 0, comme par exemple Bistriflurone, Chlorfluazurone, Diflubenzurone, Flucycloxurone, Flufénoxurone, Hexaflumurone, Lufénurone, Novalurone, Noviflumurone, Teflubenzuron et Triflumuron.
(16) des inhibiteurs de la biosynthèse de la chitine, Type 1, comme par exemple Buprofézine.
(17) des perturbateurs de mue, diptères, comme par exemple Cyromazine.
(18) des agonistes des récepteurs de l'ecdysone, comme par exemple Chromafénozide, Halofénozide, Méthoxyfénozide et Tébufénozide.
(19) des agonistes octopaminiques, comme par exemple Amitraze.
(20) des inhibiteurs du transport d'électrons du complexe III, comme par exemple Hydraméthylnon ; ou Acéquinocyle ; ou Fluacrypine.
(21) des inhibiteurs du transport d'électrons du complexe I, comme par exemple

des acaricides METI (Mitochondrial Electron Transport Inhibitors, inhibiteurs du transport d'électrons dans les mitochondries), par exemple Fénazaquin, Fenpyroximate, Pyrimidifène, Pyridabène, Tébufenpyrade et Tolfenpyrade ; ou
Roténone (Derris).

(22) des bloqueurs du canal sodium dépendant de la tension, par exemple Indoxacarbe ; ou Métaflumizone.
(23) des inhibiteurs de l'acétyl-CoA-carboxylase, comme par exemple
des dérivés des acides tétronique et tétramique, par exemple Spirodiclofène, Spiromésifène et Spirotétramate.
(24) des inhibiteurs du transport d'électrons du complexe IV, comme par exemple
des phosphines, par exemple Phosphure d'aluminium, Phosphure de calcium, Phosphine et Phosphure de zinc ; ou Cyanure.

(25) des inhibiteurs du transport d'électrons du complexe II, comme par exemple Cyenopyrafène.

(28) des modulateurs des récepteurs de la ryanodine, comme par exemple des diamides, par exemple Chlorantraniliprole et Flubendiamide ;

d'autres principes actifs présentant des mécanismes d'action inconnus, comme par exemple Amidoflumet, Azadirachtine, Benclothiaz, Benzoximate, Bifénazate, Bromopropylate, Chinométhionat, Cryolite, Cyantraniliprole (Cyazypyr), Cyflumétofène, Dicofol, Diflovidazine, Fluensulfone, Flufénérim, Flufiprole, Fluopyram, Fufénozide, Imidaclothiz, Iprodione, Meperfluthrin, Pyridalyl, Pyrifluquinazone, Tétraméthylfluthrin et Iodométhane ; en outre des préparations à base de Bacillus firmus (en particulier de la souche CNCM I-1582, par exemple VOTiVOTm, BioNem) ainsi que les composés suivants connus pour être efficaces :
3-bromo-N-{2-bromo-4-chloro-6-[(1-cyclopropyléthyl)carbamoyl]phényl}-1-(3-chloropyridin-2-yl)-1H-pyrazol-5-carboxamide, 4-{[(6-bromopyridin-3-yl)méthyl](2-fluoréthyl)amino}furan-2(5H)-one, 4-{[(6-fluoropyridin-3-yl)méthyl] (2,2-difluoréthyl)amino}furan-2(5H)-one, 4-{[(2-chloro-1,3-thiazol-5-yl)méthyl](2-fluoréthyl)amino}furan-2(5H)-one, 4-{[(6-chloropyridin-3-yl)méthyl] (2-fluoréthyl)amino}furan-2(5H)-one, flupyradifurone, 4-{[(6-chloro-5-fluoropyridin-3-yl)méthyl](méthyl)amino}furan-2(5H)-one, 4-{[(5,6-dichloropyridin-3-yl)méthyl](2-fluoréthyl)amino}furan-2(5H)-one, 4-{[(6-chloro-5-fluoropyridin-3-yl)méthyl](cyclopropyl)amino}furan-2(5H)-one, 4-{[(6-chloropyridin-3-yl)méthyl](cyclopropyl)amino}furan-2(5H)-one, 4-{[(6-chloropyridin-3-yl)méthyl](méthyl)amino}furan-2(5H)-one, {[1-(6-chloropyridin-3-yl)éthyl](méthyl)oxydo-$\lambda^4$-sulfanylidène}cyanamide et ses diastéréoisomères {[(1R)-1-(6-chloropyridin-3-yl)éthyl](méthyl)oxydo-$\lambda^4$-sulfanylidène}cyanamide (A) et {[(1S)-1-(6-chloropyridin-3-yl)éthyl](méthyl)oxydo-$\lambda^4$-sulfanylidène}cyanamide (B) ainsi que Sulfoxaflor et ses diastéréoisomères [(R)-méthyl(oxydo){(1R)-1-[6-(trifluorométhyl)pyridin-3-yl]éthyl}-$\lambda^4$-sulfanylidène]cyanamide (A1) et [(S)-méthyl(oxydo){(1S)-1-[6-(trifluorométhyl)pyridin-3-yl]éthyl}-$\lambda^4$-sulfanylidène]cyanamide (A2), désigné comme groupe de diastéréoisomères A, [(R)-méthyl(oxydo){(1S)-1-[6-(trifluorométhyl)pyridin-3-yl]éthyl}-$\lambda^4$-sulfanylidène]cyanamide (B1) et [(S)-méthyl(oxydo){(1R)-1-[6-(trifluorométhyl)pyridin-3-yl]éthyl}-$\lambda^4$-sulfanylidène]cyanamide (B2), désigné comme groupe de diastéréoisomères B et 11-(4-chloro-2,6-diméthylphényl)-12-hydroxy-1,4-dioxa-9-azadispiro[4.2.4.2]tétradéc-11-èn-10-one, 3-(4'-fluoro-2,4-diméthylbiphényl-3-yl)-4-hydroxy-8-oxa-1-azaspiro[4.5]déc-3-èn-2-one, 1-{2-fluoro-4-méthyl-5-[(2,2,2-trifluoréthyl)sulfinyl]phényl}-3-(trifluorométhyl)-1H-1,2,4-triazol-5-amine, [(3S,4aR,12R,12aS,12bS)-3-[(cyclopropylcarbonyl)oxy]-6,12-dihydroxy-4,12b-diméthyl-11-oxo-9-(pyridin-3-yl)-1,3,4,4a,5,6,6a,12,12a,12b-décahydro-2H,11H-benzo[f]pyrano[4,3-b]chromén-4-yl]méthylcyclopropancarboxylate, 2-cyano-3-(difluorométhoxy)-N,N-diméthylbenzosulfonamide, 2-cyano-3-(difluorométhoxy)-N-méthylbenzolsulfonamide, 2-cyano-3-(difluorométhoxy)-N-éthylbenzosulfonamide, 4-(difluorométhoxy)-N-éthyl-N-méthyl-1,2-benzothiazol-3-amin-1,1-dioxyde, N-[1-(2,3-diméthylphényl)-2-(3,5-diméthylphényl)éthyl]-4,5-dihydro-1,3-thiazol-2-amine, {1'-[(2E)-3-(4-chlorophényl)prop-2-én-1-yl]-5-fluorspiro[indolo-3,4'-pipéridin]-1(2H)-yl}(2-chloropyridin-4-yl)méthanone, 3-(2,5-diméthylphényl)-4-hydroxy-8-méthoxy-1,8-diazaspiro[4.5]déc-3-èn-2-one, 3-(2,5-diméthylphényl)-8-méthoxy-2-oxo-1,8-diazaspiro[4.5]déc-3-èn-4-yl-éthylcarbonate, 4-(but-2-in-1-yloxy)-6-(3,5-diméthylpipéridin-1-yl)-5-fluoropyrimidine, (2,2,3,3,4,4,5,5-octafluoropentyl)(3,3,3-trifluoropropyl)malononitrile, (2,2,3,3,4,4,5,5-octafluoropentyl)(3,3,4,4,4-pentafluorbutyl)malononitrile, 8-[2-(cyclopropylméthoxy)-4-(trifluorométhyl)phénoxy]-3-[6-(trifluorométhyl)pyridazin-3-yl]-3-azabicyclo[3.2.1]octane, flométoquin, PF1364 (CAS-Reg.Nr. 1204776-60-2), 5-[5-(3,5-dichlorophényl)-5-(trifluorométhyl)-4,5-dihydro-1,2-oxazol-3-yl]-2-(1H-1,2,4-triazol-1-yl)benzonitrile, 5-[5-(2-chloropyridin-4-yl)-5-(trifluorométhyl)-4,5-dihydro-1,2-oxazol-3-yl]-2-(1H-1,2,4-triazol-1-yl)benzonitrile,4-[5-(3,5-dichlorophényl)-5-(trifluorométhyl)-4,5-dihydro-1,2-oxazol-3-yl]-2-méthyl-N-{2-oxo-2-[(2,2,2-trifluoréthyl)amino]éthyl}benzamide, 4-{[(6-chloropyridin-3-yl)méthyl](cyclopropyl)amino}-1,3-oxazol-2(5H)-one, 4-{[(6-Chloropyridin-3-yl)méthyl](2,2-difluoréthyl)amino}-1,3-oxazol-2(5H)-one,4-{[(6-chloropyridin-3-yl)méthyl](éthyl)amino}-1,3-oxazol-2(5H)-one, 4-{[(6-chloropyridin-3-yl)méthyl](méthyl)amino}-1,3-oxazol-2(5H)-one, NNI-0711, 1-acétyl-N-[4-(1,1,1,3,3,3-hexafluoro-2-méthoxypropan-2-yl)-3-isobutylphényl]-N-isobutyryl-3,5-diméthyl-1H-pyrazol-4-carboxamide, méthyl-2-[2-({[3-bromo-1-(3-chloropyridin-2-yl)-1H-pyrazol-5-yl]carbonyl}amino)-5-chloro-3-méthylbenzoyl]-2-méthylhydrazinecarboxylate, méthyl-2-[2-({[3-bromo-1-(3-chloropyridin-2-yl)-1H-pyrazol-5-yl]carbonyl}amino)-5-cyano-3-méthylbenzoyl]-2-éthylhydrazinecarboxylate, méthyl-2-[2-({[3-bromo-1-(3-chloropyridin-2-yl)-1H-pyrazol-5-yl]carbonyl}amino)-5-cyano-3-méthylbenzoyl]-2-méthylhydrazinecarboxylate, méthyl-2-[3,5-dibromo-2-({[3-bromo-1-(3-chloropyridin-2-yl)-1H-pyrazol-5-yl]carbonyl}amino)benzoyl]-1,2-diéthylhydrazinecarboxylate, méthyl-2-[3,5-dibromo-2-({[3-bromo-1-(3-chloropyridin-2-yl)-1H-pyrazol-5-yl]carbonyl}amino)benzoyl]-2-éthylhydrazinecarboxylate, (5RS,7RS ;5RS,7SR)-1-(6-chloro-3-pyridinylméthyl)-1,2,3,5,6,7-hexahydro-7-méthyl-8-nitro-5-propoxyimidazo[1,2-a]pyridine, 2-{6-[2-(5-fluoropyridin-3-yl)-1,3-thiazol-5-yl]pyridin-2-yl}pyrimidine, 2-{6-[2-(pyridin-3-yl)-1,3-thiazol-5-yl]pyridin-2-yl}pyrimidine, 1-(3-chloropyridin-2-yl)-N-[4-cyano-2-méthyl-6-(méthylcarbamoyl)phényl]-3-{[5-(trifluorométhyl)-1H-tétrazol-1-yl]méthyl}-1H-pyrazol-5-carboxamide, 1-(3-chloropyridin-2-yl)-N-[4-cyano-2-méthyl-6-(méthylcarbamoyl)phényl]-3-{[5-(trifluorométhyl)-2H-tétrazol-2-yl]méthyl}-1H-pyrazol-5-carboxamide, N-[2-(tert-butylcarbamoyl)-4-cyano-6-méthylphényl]-1-(3-chloropyridin-2-yl)-3-{[5-(trifluorométhyl)-1H-tétrazol-1-yl]méthyl}-1H-pyrazol-5-carboxamide, N-[2-(tert-butylcarbamoyl)-4-cyano-6-méthylphényl]-1-(3-chloropyridin-2-yl)-3-{[5-(trifluorométhyl)-2H-tétrazol-2-yl]méthyl}-1H-pyrazol-5-carboxamide, (1E)-N-[(6-chloropyridin-3-yl)méthyl]-N'-cyano-N-(2,2-difluoréthyl)éthanimidamide, N-[2-(5-amino-1,3,4-thiadiazol-2-yl)-4-chloro-6-méthylphényl]-3-bromo-1-(3-chloropyridin-2-yl)-1H-pyrazol-5-carboxamide et Méthyl-2-[3,5-dibromo-2-({[3-bromo-1-(3-chloropyridin-2-yl)-1H-pyrazol-5-yl]carbonyl}amino)benzoyl]-2-éthyl-1-méthylhydrazinecarboxylate,
et/ou au moins un autre principe actif fongicide choisi dans le groupe constitué par

(1) des inhibiteurs de la biosynthèse de l'ergostérol, comme par exemple (1.1) Aldimorph (1704-28-5), (1.2) Azaconazole (60207-31-0), (1.3) Bitertanol (55179-31-2), (1.4) Bromuconazole (116255-48-2), (1.5) Cyproconazole (113096-99-4), (1.6) Diclobutrazole (75736-33-3), (1.7) Difenoconazole (119446-68-3), (1.8) Diniconazole (83657-24-3), (1.9) Diniconazole-M (83657-18-5), (1.10) Dodemorph (1593-77-7), (1.11) Acétate de Dodémorphe (31717-87-0), (1.12) Epoxiconazole (106325-08-0), (1.13) Etaconazole (60207-93-4), (1.14) Fenarimol (60168-88-9), (1.15) Fenbuconazole (114369-43-6), (1.16) Fenhexamide(126833-17-8), (1.17) Fenpropidin (67306-00-7), (1.18) Fenpropimorph (67306-03-0), (1.19) Fluquinconazole (136426-54-5), (1.20) Flurprimidol (56425-91-3), (1.21) Flusilazole (85509-19-9), (1.22) Flutriafol (76674-21-0), (1.23) Furconazole (112839-33-5), (1.24) Furconazole-Cis (112839-32-4), (1.25) Hexaconazole (79983-71-4), (1.26) Imazalil (60534-80-7), (1.27) Sulfate d'Imazalil (58594-72-2), (1.28) Imibenconazole (86598-92-7), (1.29) Ipconazole (125225-28-7), (1.30) Metconazole (125116-23-6), (1.31) Myclobutanil (88671-89-0), (1.32) Naftifin (65472-88-0), (1.33) Nuarimol (63284-71-9), (1.34) Oxpoconazole (174212-12-5), (1.35) Paclobutrazole (76738-62-0), (1.36) Pefurazoat (101903-30-4), (1.37) Penconazole (66246-88-6), (1.38) Pipéraline (3478-94-2), (1.39) Prochloraz (67747-09-5), (1.40) Propiconazole (60207-90-1), (1.41) Prothioconazole (178928-70-6), (1.42) Pyributicarb (88678-67-5), (1.43) Pyrifenox (88283-41-4), (1.44) Quinconazole (103970-75-8), (1.45) Simeconazole (149508-90-7), (1.46) Spiroxamin (118134-30-8), (1.47) Tebuconazole (107534-96-3), (1.48) Terbinafin (91161-71-6), (1.49) Tétraconazole (112281-77-3), (1.50) Triadimefon (43121-43-3), (1.51) Triadimenol (89482-17-7), (1.52) Tridemorph (81412-43-3), (1.53) Triflumizole (68694-11-1), (1.54) Triforin (26644-46-2), (1.55) Triticonazole (131983-72-7), (1.56) Uniconazole (83657-22-1), (1.57) Uniconazole-p (83657-17-4), (1.58) Viniconazole (77174-66-4), (1.59) Voriconazole (137234-62-9), (1.60) 1-(4-Chlorophényl)-2-(1H-1,2,4-triazol-1-yl)cycloheptanol (129586-32-9), (1.61) Méthyl-1-(2,2-diméthyl-2,3-dihydro-1H-indén-1-yl)-1H-imidazol-5-carboxylate (110323-95-0), (1.62) N'-{5-(Difluorométhyl)-2-méthyl-4-[3-(triméthylsilyl)propoxy]phényl}-N-éthyl-N-méthylimidoformamide, (1.63) N-éthyl-N-méthyl-N'-{2-méthyl-5-(trifluorométhyl)-4-[3-(triméthylsilyl)propoxy]phényl}imidoform amide et (1.64) O-[1-(4-Méthoxyphénoxy)-3,3-diméthylbutan-2-yl]-1H-imidazol-1-carbothioate (111226-71-2).

(2) des inhibiteurs de la respiration (inhibiteurs de la chaîne respiratoire), comme par exemple (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam en mélange du racémique syn-épimère 1RS,4SR,9RS et du racémique anti-épimère 1RS,4SR,9SR (881685-58-1), (2.12) Isopyrazam (racémique anti-épimère), (2.13) Isopyrazam (énantiomère anti-épimère 1R,4S,9S), (2.14) Isopyrazam (énantiomère anti-épimère 1S,4R,9R), (2.15) Isopyrazam (racémique syn-épimère 1RS,4SR,9RS), (2.16) Isopyrazam (énantiomère syn-épimère 1R,4S,9R), (2.17) Isopyrazam (énantiomère syn-épimère 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamide(130000-40-7), (2.24) 1-Méthyl-N-[2-(1,1,2,2-tétrafluoréthoxy)phényl]-3-(trifluorométhyl)-1H-pyrazol-4-carboxamide, (2.25) 3-(Difluorométhyl)-1-méthyl-N-[2-(1,1,2,2-tétrafluoréthoxy)phényl]-1H-pyrazol-4-carboxamide, (2.26) 3-(Difluorométhyl)-N-[4-fluoro-2-(1,1,2,3,3,3-hexafluoropropoxy)phényl]-1-méthyl-1H-pyrazol-4-carboxamide, (2.27) N-[1-(2,4-Dichlorophényl)-1-méthoxypropan-2-yl]-3-(difluorométhyl)-1-méthyl-1H-pyrazol-4-carboxamide(1092400-95-7), (2.28) 5,8-Difluoro-N-[2-(2-fluoro-4-{[4-(trifluorométhyl)pyridin-2-yl]oxy}phényl)éthyl]quinazolin-4-amine (1210070-84-0), (2.29) N-[9-(Dichlorométhylèn)-1,2,3,4-tétrahydro-1,4-méthanonaphtalén-5-yl]-3-(difluorométhyl)-1-méthyl-1H-pyrazol-4-carboxamide, (2.30) N-[(1S,4R)-9-(dichlorméthylèn)-1,2,3,4-tétrahydro-1,4-méthanonaphtalén-5-yl]-3-(difluorométhyl)-1-méthyl-1H-pyrazol-4-carboxamide et (2.31) N-[(1R,4S)-9-(Dichlorméthylèn)-1,2,3,4-tétrahydro-1,4-méthanonaphtalen-5-yl]-3-(difluorométhyl)-1-méthyl-1H-pyrazol-4-carboxamide.

(3) des inhibiteurs de la respiration (inhibiteurs de la chaîne respiratoire) au complexe III de la chaîne respiratoire, comme par exemple (3.1) Ametoctradin (865318-97-4), (3.2) Amisulbrom (348635-87-0), (3.3) Azoxystrobin (131860-33-8), (3.4) Cyazofamide(120116-88-3), (3.5) Couméthoxystrobin (850881-30-0), (3.6) Coumoxystrobin (850881-70-8), (3.5) Dimoxystrobin (141600-52-4), (3.6) Enestroburin (238410-11-2), (3.9) Famoxadon (131807-57-3), (3.10) Fenamidon (161326-34-7), (3.11) Fenoxystrobin (918162-02-4), (3.12) Fluoxastrobin (361377-29-9), (3.13) Kresoxim-Méthyl (143390-89-0), (3.14) Metominostrobin (133408-50-1), (3.15) Orysastrobin (189892-69-1), (3.16) Picoxystrobin (117428-22-5), (3.17) Pyraclostrobin (175013-18-0), (3.18) Pyrametostrobin (915410-70-7), (3.19) Pyraoxystrobin (862588-11-2), (3.20) Pyribencarb (799247-52-2), (3.21) Triclopyricarb (902760-40-1), (3.22) Trifloxystrobin (141517-21-7), (3.23) (2E)-2-(2-{[6-(3-Chloro-2-méthylphénoxy)-5-fluoropyrimidin-4-yl]oxy}phényl)-2-(méthoxyimino)-N-méthyléthanamide, (3.24) (2E)-2-(Méthoxyimino)-N-méthyl-2-(2-{[({(1E)-1-[3-(trifluorométhyl)phényl]éthylidèn)amino) oxy]méthyl}phényl)éthanamide, (3.25) (2E)-2-(Méthoxyimino)-N-méthyl-2-{2-[(E)-({1-[3-(trifluorométhyl)phényl]éthoxy}imino)mét hyl]phényl}éthanamide(158169-73-4), (3.26) (2E)-2-{2[({[(1E)-1-(3-{[(E)-1-Fluoro-2-phénylethényl]oxy}phény)éthylidèn]amino} oxy)méthyl]phényl}-2-(méthoxyimino)-N-méthyléthanamide (326896-28-0), (3.27) (2E)-2-{2-[({[(2E,3E)-4-(2,6-Dichlorophényl)but-3-én-2-ylidèn]amino}oxy)méthyl]phényl}-2-(méthoxyimino)-N-méthyléthanamide, (3.28) 2-Chloro-N-(1,1,3-triméthyl-2,3-dihydro-1H-indén-4-yl)pyridine-3-carboxamide (119899-14-8), (3.29) 5-Méthoxy-2-méthyl-4-(2-{[({(1E)-1-[3-(trifluorométhyl)phényl]éthylidèn}amino) oxy]méthyl}phényl)-2,4-dihydro-3H-1,2,4-triazol-3-one, (3.30) Méthyl-(2E)-2-{2-[({cyclopropyl[(4-méthoxyphényl)imino]méthyl}sulfanyl)méth yl]phényl}-3-méthoxyprop-2-énoate (149601-03-6), (3.31) N-(3-éthyl-3,5,5-triméthylcyclohexyl)-3-(formylamino)-2-hydroxybenzamide (226551-21-9), (3.32) 2-{2-[(2,5-Diméthylphénoxy)méthyl]phényl}-2-méthoxy-N-méthylacetamide (173662-97-0) et (3.33) (2R)-2-{2-[(2,5-Diméthylphénoxy)méthyl]phényl}-2-méthoxy-N-méthylacetamide(394657-24-0) .

(4) des inhibiteurs de la mitose et de la division cellulaire, comme par exemple (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-méthyl (23564-05-8), (4.11) Thiophanat (23564-06-9), (4.12) Zoxamide(156052-68-5), (4.13) 5-Chloro-7-(4-méthylpipéridin-1-yl)-6-(2,4,6-trifluorophényl)[1,2,4]triazolo[1,5-a]pyrimidine (214706-53-3) et (4.14) 3-Chloro-5-(6-chloropyridin-3-yl)-6-méthyl-4-(2,4,6-trifluorophényl)pyridazine (1002756-87-7).

(5) Composés présentant une activité multisite, comme par exemple (5.1) bouillie bordelaise (8011-63-0), (5.2) Captafol (2425-06-1), (5.3) Captan (133-06-2), (5.4) Chlorothalonil (1897-45-6), (5.5) des préparations à base de cuivre comme l'hydroxyde de cuivre (20427-59-2), (5.6) naphténate de cuivre (1338-02-9), (5.7) oxyde de cuivre (1317-39-1), (5.8) oxychlorure de cuivre (1332-40-7), (5.9) sulfate de cuivre (7758-98-7), (5.10) Dichlofluanid (1085-98-9), (5.11) Dithianon (3347-22-6), (5.12) Dodine (2439-10-3), (5.13) Dodine base libre, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Métiram-zinc (9006-42-2), (5.27) Oxine-cuivre (10380-28-6), (5.28) Propamidin (104-32-5), (5.29) Propineb (12071-83-9), (5.30) Soufre et des préparation à base de soufre comme par exemple le polysulfure de calcium (7704-34-9), (5.31) Thiram (137-26-8), (5.32) Tolylfluanid (731-27-1), (5.33) Zineb (12122-67-7) et (5.34) Ziram (137-30-4).

(6) des inducteurs de résistance, comme par exemple (6.1) Acibenzolar-S-Méthyl (135158-54-2), (6.2) Isotianil (224049-04-1), (6.3) Probenazol (27605-76-1) et (6.4) Tiadinil (223580-51-6).

(7) des inhibiteurs de la biosynthèse des acides aminés et des protéines, comme par exemple (7.1) Andoprim (23951-85-1), (7.2) Blasticidin-S (2079-00-7), (7.3) Cyprodinil (121552-61-2), (7.4) Kasugamycin (6980-18-3), (7.5) Chlorhydrate hydrate de Kasugamycin (19408-46-9), (7.6) Mepanipyrim (110235-47-7), (7.7) Pyriméthanil (53112-28-0) et (7.8) 3-(5-Fluoro-3,3,4,4-tétraméthyl-3,4-dihydroisoquinoléin-1-yl)quinoléine (861647-32-7).

(8) des inhibiteurs de la production de l'ATP, comme par exemple (8.1) Acétate de Fentine (900-95-8), (8.2) Chlorure de Fentine (639-58-7), (8.3) Hydroxyde de Fentine (76-87-9) et (8.4) Silthiofam (175217-20-6).

(9) des inhibiteurs de la synthèse de la paroi cellulaire, comme par exemple (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamide(374726-62-2), (9.6) Polyoxins (11113-80-7), (9.7) Polyoxorim (22976-86-9), (9.8) Validamycin A (37248-47-8) et (9.9) Valifenalat (283159-94-4 ; 283159-90-0).

(10) des inhibiteurs de la synthèse des lipides et de la synthèse membranaire, comme par exemple (10.1) Biphényle (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Chlorhydrate de Propamocarb (25606-41-1), (10.11) Prothiocarb (19622-08-3), (10.12) Pyrazophos (13457-18-6), (10.13) Quintozen (82-68-8), (10.14) Tecnazene (117-18-0) et (10.15) Tolclofos-Méthyl (57018-04-9).

(11) des inhibiteurs de la biosynthèse de la mélanine, comme par exemple (11.1) Carpropamide(104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) et (11.7) 2,2,2-Trifluoréthyl{3-méthyl-1-[(4-méthylbenzoyl)amino]butan-2-yl}carbamate (851524-22-6).

(12) des inhibiteurs de la synthèse des acides nucléiques, comme par exemple (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace

(58810-48-3), (12.12) Oxadixyl (77732-09-3) et (12.13) Acide Oxolinique (14698-29-4).

(13) des inhibiteurs de la transduction du signal, comme par exemple (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) et (13.7) Vinclozolin (50471-44-8).

(14) des découpleurs, comme par exemple (14.1) Binapacryl (485-31-4), (14.2) Dinocap (131-72-6), (14.3) Ferimzon (89269-64-7), (14.4) Fluazinam (79622-59-6) et (14.5) Meptyldinocap (131-72-6).

(15) d'autres composés, comme par exemple (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamide(180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Méthylsulfate de Difenzoquat (43222-48-6), (15.17) Diphénylamine (122-39-4), (15.18) Ecomat, (15.19) Fenpyrazamin (473798-59-3), (15.20) Flumetover (154025-04-4), (15.21) Fluoromid (41205-21-4), (15.22) Flusulfamide(106917-52-6), (15.23) Flutianil (304900-25-2), (15.24) Fosetyl-Aluminium (39148-24-8), (15.25) Fosetyl-Calcium, (15.26) Fosetyl-Sodium (39148-16-8), (15.27) Hexachlorbenzène (118-74-1), (15.28) Irumamycin (81604-73-1), (15.29) Methasulfocarb (66952-49-6), (15.30) Méthylisothiocyanate (556-61-6), (15.31) Metrafenon (220899-03-6), (15.32) Mildiomycin (67527-71-3), (15.33) Natamycin (7681-93-8), (15.34) Diméthyldithiocarbamat de Nickel (15521-65-0), (15.35) Nitrothal-Isopropyle (10552-74-6), (15.36) Octhilinone (26530-20-1), (15.37) Oxamocarb (917242-12-7), (15.38) Oxyfenthiin (34407-87-9), (15.39) Pentachlorophenol et ses sels (87-86-5), (15.40) Phenothrin, (15.41) Acides Phosphoriques et leurs sels (13598-36-2), (15.42) Propamocarb-Fosetylat, (15.43) Propanosin-Sodium (88498-02-6), (15.44) Proquinazid (189278-12-4), (15.45) Pyrimorph (868390-90-3), (15.45e) (2E)-3-(4-tert-butylphényl)-3-(2-chloropyridin-4-yl)-1-(morpholin-4-yl)prop-2-én-1-one (1231776-28-5), (15.45z) (2Z)-3-(4-tert-butylphényl)-3-(2-chloropyridin-4-yl)-1-(morpholin-4-yl)prop-2-én-1-one (1231776-29-6), (15.46) Pyrrolnitrin (1018-71-9), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamide(70193-21-4), (15.52) Zarilamide(84527-51-5), (15.53) (3S,6S,7R,8R)-8-benzyl-3-[({3-[(isobutyryloxy)méthoxy]-4-méthoxypyridin-2-yl}carbonyl)amino]-6-méthyl-4,9-dioxo-1,5-dioxonan-7-yl 2-méthylpropanoate (517875-34-2), (15.54) 1-(4-{4-[(5R)-5-(2,6-Difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone (1003319-79-6), (15.55) 1-(4-{4-[(5S)-5-(2,6-Difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone (1003319-80-9), (15.56) 1-(4-{4-[5-(2,6-Difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone (1003318-67-9), (15.57) 1-(4-Méthoxyphénoxy)-3,3-diméthylbutan-2-yl-1H-imidazol-1-carboxylate (111227-17-9), (15.58) 2,3,5,6-tétrachloro-4-(méthylsulfonyl)pyridine (13108-52-6), (15.59) 2,3-Dibutyl-6-chlorthiéno[2,3-d]pyrimidin-4(3H)-one (221451-58-7), (15.60) 2,6-Diméthyl-1H,5H-[1,4]dithiino[2,3-c :5,6-c']dipyrrol-1,3,5,7 (2H,6H)-tétrone, (15.61) 2-[5-Méthyl-3-(trifluorométhyl)-1H-pyrazol-1-yl]-1-(4-{4-[(5R)-5-phényl-4,5-dihydro-1,2-oxazol-3-yl]-1,3-thiazol-2-yl}pipéridin-1-yl)éthanone (1003316-53-7), (15.62) 2-[5-Méthyl-3-(trifluorométhyl)-1H-pyrazol-1-yl]-1-(4-{4-[(5S)-5-phényl-4,5-dihydro-1,2-oxazol-3-yl]-1,3-thiazol-2-yl}pipéridin-1-yl)éthanone (1003316-54-8), (15.63) 2-[5-Méthyl-3-(trifluorométhyl)-1H-pyrazol-1-yl]-1-{4-[4-(5-phényl-4,5-dihydro-1,2-oxazol-3-yl)-1,3-thiazol-2-yl]pipéridin-1-yl}éthanone (1003316-51-5), (15.64) 2-Butoxy-6-iodo-3-propyl-4H-chromén-4-one, (15.65) 2-Chloro-5-[2-chloro-1-(2,6-difluoro-4-méthoxyphényl)-4-méthyl-1H-imidazol-5-yl]pyridine, (15.66) 2-Phénylphénol et ses sels (90-43-7), (15.67) 3-(4,4,5-Trifluoro-3,3-diméthyl-3,4-dihydroisoquinolin-1-yl)quinoléine (861647-85-0), (15.68) 3,4,5-Trichloropyridin-2,6-dicarbonitrile (17824-85-0), (15.69) 3-[5-(4-Chlorophényl)-2,3-diméthyl-1,2-oxazolidin-3-yl]pyridine, (15.70) 3-Chloro-5-(4-chlorophényl)-4-(2,6-difluorophényl)-6-méthylpyridazine, (15.71) 4-(4-Chlorophényl)-5-(2,6-difluorophényl)-3,6-diméthylpyridazine, (15.72) 5-Amino-1,3,4-thiadiazol-2-thiol, (15.73) 5-Chloro-N'-phényl-N'-(prop-2-yn-1-yl)thiophén-2-sulfonohydrazide (134-31-6), (15.74) 5-Fluoro-2-[(4-fluorobenzyl)oxy]pyrimidin-4-amine (1174376-11-4), (15.75) 5-Fluoro-2-[(4-méthylbenzyl)oxy]pyrimidin-4-amine (1174376-25-0), (15.76) 5-Méthyl-6-octyl[1,2,4]triazolo[1,5-a]pyrimidin-7-amine, (15.77) Éthyl-(2Z)-3-amino-2-cyano-3-phénylprop-2-énoate, (15.78) N'-(4-{[3-(4-Chlorobenzyl)-1,2,4-thiadiazol-5-yl]oxy}-2,5-diméthylphényl)-N-éthyl-N-méthylimidoformamide, (15.79) N-(4-Chlorobenzyl)-3-[3-méthoxy-4-(prop-2-yn-1-yloxy)phényl]propanamide, (15.80) N-[(4-Chlorophényl)(cyano)méthyl]-3-[3-méthoxy-4-(prop-2-yn-1-yloxy)phényl]propanamide, (15.81) N-[(5-Bromo-3-chloropyridin-2-yl)méthyl]-2,4-dichloropyridine-3-carboxamide, (15.82) N-[1-(5-Bromo-3-chloropyridin-2-yl)éthyl]-2,4-dichloropyridine-3-carboxamide, (15.83) N-[1-(5-Bromo-3-chloropyridin-2-yl)éthyl]-2-fluoro-4-iodopyridine-3-carboxamide, (15.84) N-{(E)-[(Cyclopropylméthoxy)imino] [6-(difluorométhoxy)-2,3-difluorophényl]méthyl}-2-phénylacétamide(221201-92-9), (15.85) N-{(Z)-[(Cyclopropylméthoxy)imino][6-(difluorométhoxy)-2,3-difluorophényl]méthyl}-2-phénylacétamide(221201-92-9), (15.86) N'-{4-[(3-Tert-butyl-4-cyano-1,2-thiazol-5-yl)oxy]-2-chloro-5-méthylphényl}-N-éthyl-N-méthylimidoformamide, (15.87) N-Méthyl-2-(1-{[5-méthyl-3-(trifluorométhyl)-1H-pyrazol-1-yl]acétyl}pipéridin-4-yl)-N-(1,2,3,4-tétrahydronaphtalén-1-yl)-1,3-thiazol-4-carboxamide (922514-49-6), (15.88) N-Méthyl-2-(1-{[5-méthyl-3-(trifluorométhyl)-1H-pyrazol-1-yl]acétyl}pipéridin-4-yl)-N-[(1R)-1,2,3,4-tétrahydronaphtalen-1-yl]-1,3-thiazol-4-carboxamide(922514-07-6), (15.89) N-Méthyl-2-(1-{[5-méthyl-3-(trifluorométhyl)-1H-pyrazol-1-yl]acétyl}pipéridin-4-yl)-N-[(1S)-1,2,3,4-tétrahydronaphtalén-1-yl]-1,3-thiazol-4-carboxamide (922514-48-5), (15.90) Pentyl-{6-[({[(1-méthyl-1H-tétrazol-5-yl)(phényl)méthylidèn]amino}oxy)méthyl]p yridin-2-yl}carbamate, (15.91) Acide Phénazin-1-carboxylique, (15.92) quinoléin-8-ol (134-31-6), (15.93) sulfate de quinoléin-8-ol (2 :1) (134-31-6) et (15.94) Tert-butyl{6-[({[(1-méthyl-1H-tétrazol-5-yl)(phényl)méthylèn]amino}oxy)méthyl]pyr idin-2-yl}carbamate.

(16) d'autres composés, comme par exemple (16.1) 1-Méthyl-3-(trifluorométhyl)-N-[2'-(trifluorométhyl)biphényl-2-yl]-1H-pyrazol-4-carboxamide, (16.2) N-(4'-Chlorobiphényl-2-yl)-3-(difluorométhyl)-1-méthyl-1H-pyrazol-4-carboxamide, (16.3) N-(2',4'-Dichlorobiphényl-2-yl)-3-(difluorométhyl)-1-méthyl-1H-pyrazol-4-carboxamide, (16.4) 3-(Difluorométhyl)-1-méthyl-N-[4'-(trifluorométhyl)biphényl-2-yl]-1H-pyrazol-4-carboxamide, (16.5) N-(2',5'-Difluorobiphényl-2-yl)-1-méthyl-3-(trifluorométhyl)-1H-pyrazol-4-carboxamide, (16.6) 3-(Difluorométhyl)-1-méthyl-N-[4'-(prop-1-yn-1-yl)biphényl-2-yl]-1H-pyrazol-4-carboxamide, (16.7) 5-Fluoro-1,3-diméthyl-N-[4'-(prop-1-yn-1-yl)biphényl-2-yl]-1H-pyrazol-4-carboxamide, (16.8) 2-Chloro-N-[4'-(prop-1-yn-1-yl)biphényl-2-yl]pyridine-3-carboxamide, (16.9) 3-(Difluorométhyl)-N-[4'-(3,3-diméthylbut-1-yn-1-yl)biphényl-2-yl]-1-méthyl-1H-pyrazol-4-carboxamide, (16.10) N-[4'-(3,3-Diméthylbut-1-yn-1-yl)biphényl-2-yl]-5-fluoro-1,3-diméthyl-1H-pyrazol-4-carboxamide, 16.11) 3-(Difluorométhyl)-N-(4'-éthynylbiphényl-2-yl)-1-méthyl-1H-pyrazol-4-carboxamide, (16.12) N-(4'-éthynylbiphényl-2-yl)-5-fluoro-1,3-diméthyl-1H-pyrazol-4-carboxamide, (16.13) 2-Chloro-N-(4'-éthynylbiphényl-2-yl)pyridine-3-carboxamide, (16.14) 2-Chloro-N-[4'-(3,3-diméthylbut-1-yn-1-yl)biphényl-2-yl]pyridine-3-carboxamide, (16.15) 4-(Difluorométhyl)-2-méthyl-N-[4'-(trifluorométhyl)biphényl-2-yl]-1,3-thiazol-5-carboxamide, (16.16) 5-Fluoro-N-[4'-(3-hydroxy-3-méthylbut-1-yn-1-yl)biphényl-2-yl]-1,3-diméthyl-1H-pyrazol-4-carboxamide, (16.17) 2-Chloro-N-[4'-(3-hydroxy-3-méthylbut-1-yn-1-yl)biphényl-2-yl]pyridine-3-carboxamide, (16.18) 3-(Difluorométhyl)-N-[4'-(3-méthoxy-3-méthylbut-1-yn-1-yl)biphényl-2-yl]-1-méthyl-1H-pyrazol-4-carboxamide, (16.19) 5-Fluoro-N-[4'-(3-méthoxy-3-méthylbut-1-yn-1-yl)biphényl-2-yl]-1,3-diméthyl-1H-pyrazol-4-carboxamide, (16.20) 2-Chloro-N-[4'-(3-méthoxy-3-méthylbut-1-yn-1-yl)biphényl-2-yl]pyridine-3-carboxamide, (16.21) (5-Bromo-2-méthoxy-4-méthylpyridin-3-yl)(2,3,4-triméthoxy-6-méthylphényl)méthanone, (16.22) N-[2-(4-{[3-(4-Chlorophényl)prop-2-yn-1-yl]oxy}-3-méthoxyphényl)éthyl]-N2-(méthylsulfonyl)va1inamide (220706-93-4), (16.23) acide 4-oxo-4-[(2-phényléthyl)amino]butyrique et (16.24) But-3-yn-1-yl{6-[({[(Z)-(1-méthyl-1H-tétrazol-5-yl)(phényl)méthylèn]amino}oxy)méthyl]pyr idin-2-yl}carbamate.

**20.** Compositions agrochimiques, **caractérisées en ce qu'**elles contiennent au moins un composé de formule (I) selon les revendications 1 à 18 ou une composition selon la revendication 19, ainsi que des diluants et/ou des tensioactifs.

**21.** Procédé pour la préparation de compositions agrochimiques, **caractérisé en ce que** l'on mélange des composés de formule (I) selon les revendications 1 à 18 ou une composition selon la revendication 19 avec des diluants et/ou des tensioactifs.

**22.** Procédé pour la lutte contre des parasites animaux, **caractérisé en ce que** l'on laisse agir des composés de formule (I) selon les revendications 1 à 18 ou une composition selon la revendication 19 sur des parasites animaux et/ou sur leur habitat, des procédés pour le traitement chirurgical ou thérapeutique du corps humain ou animal et des procédés de diagnostic, qui sont effectués sur le corps humain ou animal, étant exclus.

**23.** Utilisation de composés de formule (I) selon l'une quelconque des revendications 1 à 18 ou d'une composition selon la revendication 19 pour la lutte contre des parasites animaux dans le domaine phytosanitaire, dans le domaine de la protection des matériaux et/ou dans le domaine vétérinaire, des utilisations dans des procédés pour le traitement chirurgical ou thérapeutique du corps humain ou animal et des procédés de diagnostic, qui sont effectués sur le corps humain ou animal, étant exclus.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 1999055668 A
- WO 2007131680 A
- WO 2010100189 A
- JP 2011042611 A
- EP 0597360 A1
- US 2009076282 A1
- EP 2604118 A1
- US 6906006 B
- DE 19500439
- WO 20100119194 A
- DE 4431218
- WO 2009012275 A
- WO 2008155034 A
- DE 19528305
- WO 2008086226 A
- WO 2006117657 A1
- US 201099725 A1
- WO 201047956 A1
- WO 200585226 A1
- WO 200862905 A2
- EP 301946 A
- EP 597360 A
- WO 2002012203 A
- WO 2009088025 A
- US 2011130415 A1
- JP 46037339 B
- JP 6145142 B
- US 3133079 A
- WO 2004011438 A
- US 4279637 A
- US 3853912 A
- EP 260228 A
- US 4079144 A
- US 3007927 A
- DE 2554866
- WO 2000026194 A
- JP 2007284356 A
- WO 2007034755 A
- JP 2007081019 A
- JP 2007284385 A
- JP 2008260706 A
- JP 2008308448 A
- JP 2009023910 A
- WO 2012176856 A
- EP 1803712 A1
- WO 2006043635 A
- EP 1183229 B1
- US 2010160388 A1
- EP 422469 A2
- EP 657437 A1
- EP 507171 A1
- DE 19508118 A1
- DE 19525973 A1
- DE 4339412 A1
- EP 726258 A1
- EP 415196 A2
- DE 4239296 A1
- EP 534266 A1
- DE 3936623 A1
- US 5599944 A
- DE 19508119 A1
- EP 425948 A2
- EP 711761 A1
- EP 643049 A1
- US 5502204 A1
- US 20110130415 A
- EP 638561 A1
- DE 2725148
- WO 2005077934 A
- WO 2007115644 A
- WO 2007115643 A
- WO 2007115646 A
- EP 0539588 A
- WO 2007149134 A
- WO 2010074747 A
- WO 2010074751 A
- WO 2006089633 A
- WO 2008067911 A
- WO 2008066153 A
- WO 2006056433 A
- WO 2006100288 A
- WO 2005035486 A
- WO 2007057407 A
- WO 2008104503 A
- WO 2003106457 A
- WO 2009049851 A
- WO 2004099160 A
- WO 2005063094 A
- WO 2007040280 A
- JP 2010018586 A
- WO 2007075459 A
- WO 2005085216 A
- WO 2010005692 A
- WO 2002096882 A
- WO 2007101369 A
- WO 2010006713 A
- WO 2010069502 A
- WO 2008009360 A

- CN 102057925
- WO 2011049233 A
- WO 2010025451 A
- WO 2004058723 A
- WO 0212172 A
- WO 2005070917 A
- WO 2005042474 A
- EP 1559320 A
- WO 2003035617 A
- WO 2009094442 A
- DE 4339412
- US 5599945 A
- US 5516749 A
- DE 2250572

**In der Beschreibung aufgeführte Nicht-Patentliteratur**

- *Chem. Pharm. Bull.,* 1997, vol. 45 (4), 719-721
- *J. Amer. Chem. Soc.,* 2003, vol. 125 (37), 11253-11258
- *Bull. Korean Chem. Soc.,* 2010, vol. 31 (8), 2143-2146
- *Chem. Sci.,* 2010, vol. 1, 13-31
- *Tetrahedron Lett.,* 2009, vol. 50, 7293-7296
- *Bioorg. Med. Chem. Lett.,* 2010, vol. 20 (13), 3920-3924
- *Proc. Natl. Acad. Sci. USA,* 2011, vol. 108 (17), 6781-6786
- *Chem. Eur. J.,* 2009, vol. 15 (29), 7044-7047
- *Synlett,* 2004, vol. 6, 1095-97
- *J. Org. Chem.,* 2005, vol. 70 (4), 1486-1489
- *Tetrahedron Lett.,* 1998, vol. 39, 2933-2936
- *Synthesis,* 2011, 829-856
- *Tetrahedron,* 2012, vol. 68, 7735-7754
- *Synth. Comm.,* 1986, vol. 16 (2), 163-167
- *Eur. J. Med. Chem.,* 2011, vol. 46 (10), 5039-5045
- *J. Med. Chem.,* 2007, vol. 50 (3), 528-542
- *Tetrahedron Lett.,* 1990, vol. 31 (19), 2717-2718
- *J. Med. Chem.,* 1993, vol. 36, 2558-2568
- *J. Heterocycl. Chem.,* 1983, vol. 20, 1533-1537
- *J. Org. Chem.,* 1976, vol. 41 (20), 3233-3237
- *J. Phys. Org. Chem.,* 1993, vol. 6, 601-608
- *Chem. Ber.,* 1903, vol. 36, 3139-3154
- *Liebigs Ann.,* 1959, vol. 627, 162-165
- *Ind. J. Chem. Sect. B,* 1980, vol. 19 (9), 805-809
- *Liebigs Ann.,* 1982, vol. 5, 994-1000
- *Tetrahedron,* 2010, vol. 66 (13), 2427-2432
- *J. Computational Chem.,* 2012, vol. 33 (7), 715-722
- *J. Chem. Res. Synopses,* 2003, vol. 5, 275-278
- *Arch. Pharm.,* 1987, vol. 320 (11), 1167-1173
- *J. Amer. Chem. Soc.,* 1915, vol. 37, 183-189
- *J. Amer. Chem. Soc.,* 1917, vol. 39, 950-961
- *Z. Chem.,* 1980, vol. 20 (10), 371-372
- *J. Chem. Soc.,* 1954, 3319-3324
- *Chem. Ber.,* 1958, vol. 91, 2578-2580
- **M. YOKOYAMA.** *J. Chem. Soc. (Perkin Trans. 1),* 1982, 1059-1062
- **M. YOKOMOTO.** *J. Med. Chem.,* 2012, vol. 55, 7772-7785
- **K. SCHULZE.** *J. Het. Chem.,* 1995, vol. 32, 275-281
- *J. Heterocycl. Chem.,* 1998, vol. 35, 29-32
- *J. Chem. Soc.,* 1959, 3789-3794
- *Helv. Chim. Acta,* 1996, vol. 79, 61-83
- *J. Heterocycl. Chem.,* 1999, vol. 36, 667-674
- *J. Heterocycl. Chem.,* 1991, vol. 28, 1421-1427
- *Tetrahedron Lett.,* 1989, vol. 30, 2369-2370
- *J. Org. Chem.,* 2011, vol. 76, 216-222
- *J. Org. Chem.,* 1980, vol. 45 (25), 5130-5136
- *J. Am. Chem. Soc.,* 1959, vol. 81 (12), 3076-3079
- *J. Heterocycl. Chem.,* 2007, vol. 44 (4), 937-943
- *Tetrahedron,* 1975, vol. 31 (7), 765-775
- *Bioorg. Med. Chem. Lett.,* 1999, vol. 9, 1251-1254
- *Bioorg. Med. Chem. Lett.,* 2010, vol. 20, 3920-3924
- *Chem. Ber.,* 1985, vol. 118, 526-540
- *J. Fluor. Chem.,* 2008, vol. 129 (11), 1073-1075
- *Tetrahedron Lett.,* 1999, vol. 40 (37), 6739-6744
- *J. Mol. Struct.,* 2009, vol. 933, 38-45
- *Angew. Chem.,* 1967, vol. 79, 663-680
- *Synth. Commun.,* 1997, vol. 27, 2645-2650
- *J. Amer. Chem. Soc.,* 1922, vol. 44, 2896-2903
- *J. Het. Chem.,* 1968, vol. 5, 165-177
- *J. Med. Chem.,* 2001, vol. 44 (12), 1972-1985
- *Tetrahedron,* 2002, vol. 58, 2101-2116
- *Can. J. Chem.,* 1957, vol. 37, 101-105
- *Bull. Kor. Chem. Soc.,* 2012, vol. 33, 55-59
- *Inorg. Chem.,* 1987, vol. 26, 2969-2974
- *J. Fluor. Chem.,* 2003, vol. 122 (2), 165-170
- *Synthesis and Reactivity in Inorganic and Metal-Organic Chemistry,* 1999, vol. 29, 1579-1592
- *Synthesis and Reactivity in Inorganic and Metal-Organic Chemistry,* 1984, vol. 14, 477-484
- *Org. Lett.,* 2011, vol. 13, 336-339
- *Russ. J. Appl. Chem.,* 1996, vol. 69, 1841-1848
- *Heteroatom Chem.,* 2007, vol. 18, 637-643

- *Monatsh. Chem.,* 1995, vol. 126, 1035-1044
- *J. Org. Chem.,* 1985, vol. 50, 980-987
- *J. Med. Chem.,* 2007, vol. 50, 528-542
- *J. Heterocycl. Chem.,* 1982, vol. 19, 823-828
- *Bioorg. Med. Chem.,* 2001, vol. 9, 1307-1323
- *J. Agric. Food Chem.,* 2010, vol. 58, 2643-2651
- *Pharmazie,* 1990, vol. 45, 138-139
- *Synthesis,* 1992, 391-394
- *J. Med. Chem.,* 2006, vol. 49, 1855-1866
- *Tetrahedron Lett.,* 1984, vol. 25, 1871-1874
- **JORDAN.** *J. Chem.,* 2010, vol. 5, 13-21
- *Synthesis,* 2002, 869-874
- *J. Org. Chem.,* 2008, vol. 73, 2345-2356
- *J. Med. Chem.,* 2006, vol. 49, 6015-6026
- *Bioscience, Biotechnology, and Biochemistry,* 1992, vol. 56 (7), 1164-1154
- *Chem. Pharm. Bull.,* 1985, vol. 33 (9), 4026-4029
- *Can. J. Chem.,* 2004, vol. 82, 1649-1661
- *J. Med. Chem.,* 1966, vol. 9 (6), 858-860
- *Angewandte Makromolekulare Chemie,* 1988, vol. 157, 59-78
- *J. Amer. Chem. Soc.,* 1956, 1938-1941
- *Chem. Pharm. Bull.,* 1962, vol. 10, 647-652
- **A.R. MAGUIRE.** *ARKIVOC,* 2011, vol. i, 1-110
- **H. SUZUKI.** *Chem. Let.,* 1985, vol. 3, 411-412
- **S. L. BUCHWALD.** *J. Amer. Chem. Soc.,* 2002, vol. 124 (50), 14844-14845
- **E. B. MERKUSHEV.** *Synthesis,* 1988, vol. 12, 923-937
- *J. Med. Chem.,* 2003, vol. 46, 4405-4418
- *J. Amer. Chem. Soc.,* 1940, vol. 62, 2965-2966
- *Angew. Chem.,* 1995, vol. 107, 2746-2749
- *Chem. Ber.,* 1965, vol. 98, 3025-3034
- *J. Med. Chem.,* 1990, vol. 33, 2772-2777
- *Synthesis,* 1987, vol. 10, 912-914
- *Tetrahedron,* 2001, vol. 57, 2003-2010
- *J. Org. Chem.,* 1980, vol. 45, 5130-5136
- *Chem. Lett.,* 2011, vol. 40, 1149-1151
- *J. Heterocycl. Chem.,* 2007, vol. 44, 937-943
- *J. Amer. Chem. Soc.,* 1959, vol. 81, 3076-3079
- *J. Med. Chem.,* 1986, vol. 29, 2290-2297
- *J. Amer. Chem. Soc.,* 1963, vol. 85, 749-754
- *Phosp., Sulf., Sil. and rel. Elem.,* 1996, vol. 119, 161-168
- FAO Plant Production and Protection Papers - 173, prepared by the FAO/WHO Joint Meeting on Pesticide Specifications. Pesticide Specifications, Manual on development and use of FAO and WHO specifications for pesticides. Crop Life International, 2004
- **BAUR et al.** *Pesticide Science,* 1997, vol. 51, 131-152
- The Pesticide Manual. British Crop Protection Council, 2006
- *CHEMICAL ABSTRACTS,* 1204776-60-2
- *Bull. Soc. Chim. Fr.,* 1975, 1191-1194